# EUROPEAN PATENT SPECIFICATION

(11) **EP 3 397 756 B1**
(45) Date of publication and mention of the grant of the patent: **08.03.2023**
(21) Application number: 16881286.5
(22) Date of filing: 30.12.2016
(51) Int. Cl.: C12N 5/0783, C12N 15/113, C07D 295/00, C07D 401/14, A61K 35/17, C07D 207/34, C07K 16/24, C07K 16/28, C07K 16/30, A61P 35/00

(54) **IMMUNE EFFECTOR CELL THERAPIES WITH ENHANCED EFFICACY**
IMMUNEFFEKTORZELLTHERAPIEN MIT VERBESSERTER WIRKSAMKEIT
THÉRAPIES À BASE DE CELLULES EFFECTRICES IMMUNITAIRES DOTÉES D'UNE EFFICACITÉ ACCRUE

(30) Priority: 30.12.2015 WO PCT/CN2015/099882
(43) Date of publication of application: 07.11.2018
(60) Divisional application: 23152887.8
(73) Proprietor: Novartis AG, 4056 Basel (CH); The Trustees of The University of Pennsylvania, Philadelphia, PA 19104 (US)
(72) Inventor: MOTZ, Gregory, Quincy Massachusetts 02170 (US); MAVRAKIAS, Konstantinos John, Cambridge Massachusetts 02139 (US); LIU, Jinbiao, Shanghai 201203 (CN); LIU, Lei, Shanghai 201203 (CN); ZHENG, Qiangang, Shanghai 201318 (CN); XUN, Guoliang, Shanghai 201203 (CN); XIAO, Qitao, Shanghai 201203 (CN)
(74) Representative: Wilding, James Roger
(86) International application number: PCT/CN2016/113612
(87) International publication number: WO 2017/114497

(56) References cited:
- WO-A1-2012/107499
- WO-A1-2015/153514
- WO-A1-2015/181380
- WO-A1-2016/210292
- WO-A1-2017/161001
- WO-A1-2018/059549
- WO-A2-2014/165707
- US-A1- 2015 307 617
- J. WANG ET AL: "Novel Histone Demethylase LSD1 Inhibitors Selectively Target Cancer Cells with Pluripotent Stem Cell Properties", CANCER RESEARCH, vol. 71, no. 23, 5 October 2011 (2011-10-05), pages 7238-7249, XP055303191, US ISSN: 0008-5472, DOI: 10.1158/0008-5472.CAN-11-0896
- Y LI ET AL.: 'Dynamic interaction between TALI oncoprotein and LSD1 regulates TALI function in hematopoiesis and leukemogenesis' ONCOGENE vol. 31, no. 48, 06 February 2012, ISSN 0950-9232 pages 5007 - 5018, XP055399301
- SHIN-TANG SU ET AL.: 'Involvement of Histone Demethylase LSD1 in Blimp-1-Mediated Gene Repression during Plasma Cell Differentiation' MOLECULAR AND CELLULAR BIOLOGY vol. 29, no. 6, 31 March 2009, ISSN 0270-7306 pages 1421 - 1431, XP055399305
- JINFANG ZHU ET AL.: 'Down-regulation of Gfi-1 expression by TGF-beta is important for differentiation of Th17 and CD 103 + inducible regulatory T cells' THE JOURNAL OF EXPERIMENTAL MEDICINE vol. 206, no. 2, 02 February 2009, ISSN 0022-1007 pages 329 - 341, XP055399356
- HELAI P. MOHAMMAD ET AL.: 'A DNA Hypomethylation Signature Predicts Antitumor Activity of LSD1 Inhibitors in SCLC' CANCER CELL vol. 28, no. 1, 13 July 2015, ISSN 1535-6108 pages 57 - 69, XP055228761
- DATABASE GENBANK [Online] 15 March 2015 XP055399361 Database accession no. NM_001009999.2
- DATABASE GENBANK [Online] 25 September 2015 XP055399363 Database accession no. NM_015013.3

## Description

### RELATED APPLICATIONS

This application claims priority to PCT Patent Application Number PCT/CN2015/099882, filed December 30, 2015.

### SEQUENCE LISTING

The instant application contains a Sequence Listing which has been submitted electronically in ASCII format. Said ASCII copy, created on December 29, 2016, is named N2067-7105WO3_SL.txt and is 1,118,768 bytes in size.

### FIELD OF THE INVENTION

The present invention relates generally to the use LSD1 inhibitors in connection with use and manufacture of immune effector cells (e.g., T cells, NK cells), e.g., engineered to express a chimeric antigen receptor (CAR), to treat a subject having a disease, e.g., a disease associated with expression of a tumor antigen.

### BACKGROUND OF THE INVENTION

Adoptive cell transfer (ACT) therapy, for example, with T-cells transduced with Chimeric Antigen Receptors (CARs), has shown promise in cancer trials. There is a medical need for T cell therapies, especially CAR T cell therapies with improved efficacy.

### SUMMARY OF THE INVENTION

Methods and compositions disclosed herein relate to the use of an inhibitor of Lysine-specific demethylase 1 (LSD1) in connection with the use and/or manufacture of immune effector cells (e.g., T cells or NK cells), for example, immune effector cells engineered to express a Chimeric Antigen Receptor (CAR), to treat a disease, e.g., a disease associated with expression of a cancer associated antigen (or tumor marker).

In particular, the invention provides a composition comprising a population of immune effector cells engineered to express a CAR for use, in combination with an LSD1 inhibitor, in a method of treating a disease associated with the expression of a tumor antigen in a subject, said method comprising administering to said subject an LSD1 inhibitor and a population of immune effector cells engineered to express a CAR, wherein the population of immune effector cells comprises, e.g., consists of, T cells, wherein the disease associated with expression of a tumor antigen is chosen from a proliferative disease, a precancerous condition, and a cancer.

The invention also provides a composition comprising a population of immune effector cells engineered to express a CAR for use, in combination with an LSD1 inhibitor, in a method of increasing the therapeutic efficacy of a population of immune effector cells engineered to express a CAR, e.g., a CART19 (e.g., CTL019) in a subject having a disease associated with expression of a tumor antigen, said method comprising a step of decreasing the activity or expression of LSD1 in said cells, transiently or permanently, optionally wherein the step of decreasing the activity or expression of LSD1 in said cells comprises contacting the cells with an LSD1 inhibitor, wherein the population of immune effector cells comprises, e.g., consists of, T cells, wherein the disease associated with expression of a tumor antigen is chosen from a proliferative disease, a precancerous condition, and a cancer.

Also provided is a method of making a population of immune effector cells comprising contacting a population of immune effector cells with an LSD1 inhibitor, ex vivo thereby making a population of immune effector cells, wherein the population of immune effector cells comprises, e.g., consists of, T cells, wherein the contacting with the LSD1 inhibitor causes one or more of the following to occur:
1) an increase in the proportion of naive T cells, e.g., TSCM cells;
2) an increase in the number of naive T cells, e.g., TSCM cells;
3) a decrease in the number of TEM cells;
4) a decrease in the proportion of TEM cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14) an increase in the production of cytokines (e.g., IFNg and/or IL-2) from said population of immune effector cells; or
15) a combination of two or more of the above;
as compared to a non-contacted population of immune effector cells.

The invention also provides a population of immune effector cells, which is engineered to express a chimeric antigen receptor (CAR), wherein the CAR comprises an antigen-binding domain, a transmembrane domain, and an intracellular signaling domain, and wherein expression and/or function of LSD1 in said cell has been reduced or eliminated, wherein the population of immune effector cells further comprises an LSD1 inhibitor, wherein the population of immune effector cells comprises, e.g., consists of, T cells.

The invention also provides a method of manufacturing a population of immune effector cells comprising introducing into said cells nucleic acid encoding a CAR, wherein the nucleic acid integrates into the genome of said cell within the LSD1 gene, such that LSD1 expression and/or function is reduced, wherein the population of immune effector cells comprises, e.g., consists of, T cells.

### DETAILED DESCRIPTION

The technical information set out below may in some respects go beyond the scope of the invention, which is defined by the appended claims. The additional technical information is provided to place the actual invention in a broader technical context and to illustrate possible related technical developments.

In addition, incidental references to methods for treatment of the human or animal body by surgery or therapy and diagnostic methods practised on the human or animal body are not to be construed as claiming protection for such method as such, but are instead to be construed as referring to products, in particular substances or compositions, for use in any of these methods.

It has been discovered that inhibition of LSD1 is effective in improving the function of immune effector cells, e.g., engineered to express a CAR molecule, e.g., as described herein, and can be combined with T cell, e.g., CAR T cell, therapy and/or manufacturing.

While not wishing to be bound by theory, it is believed that contacting a population of immune effector cells, e.g., engineered to express a CAR molecule, e.g., as described herein, with an LSD1 inhibitor is accompanied by an increase in the proportion of naive T cells (e.g., CD45RA+CD62L+ T cells, e.g., T_{SCM} cells), at least transiently, relative to an uncontacted population, for example, when such cells are stimulated (e.g., with anti-CD3 and/or anti-CD28 stimulation) or induced to proliferate (e.g., in response to antigen recognition, e.g., antigen recognition through a CAR molecule, e.g., as described herein).

While not wishing to be bound by theory, it is believed that contacting a population of immune effector cells, e.g., engineered to express a CAR molecule, e.g., as described herein, with an LSD1 inhibitor is accompanied by an increase in the number of naive T cells (e.g., CD45RA+CD62L+ T cells, e.g., T_{SCM} cells), at least transiently, relative to an uncontacted population, for example, when such cells are stimulated (e.g., with anti-CD3 and/or anti-CD28 stimulation) or induced to proliferate (e.g., in response to antigen recognition, e.g., antigen recognition through a CAR).

While not wishing to be bound by theory, it is believed that contacting a population of immune effector cells, e.g., engineered to express a CAR molecule, e.g., as described herein, with an LSD1 inhibitor is accompanied by a decrease in the number of T_{EM} cells, at least transiently, relative to an uncontacted population, for example, when such cells are stimulated (e.g., with anti-CD3 and/or anti-CD28 stimulation) or induced to proliferate (e.g., in response to antigen recognition, e.g., antigen recognition through a CAR).

While not wishing to be bound by theory, it is believed that contacting a population of immune effector cells, e.g., engineered to express a CAR molecule, e.g., as described herein, with an LSD1 inhibitor is accompanied by a decrease in the proportion of T_{EM} cells, at least transiently, relative to an uncontacted population, for example, when such cells are stimulated (e.g., with anti-CD3 and/or anti-CD28 stimulation) or induced to proliferate (e.g., in response to antigen recognition, e.g., antigen recognition through a CAR molecule, e.g., as described herein).

While not wishing to be bound by theory, it is believed that contacting a population of immune effector cells, e.g., engineered to express a CAR molecule, e.g., as described herein, with an LSD1 inhibitor is accompanied by an increase in the production of cytokines (e.g., IFNg and/or IL-2) from said population of immune effector cells, at least transiently, relative to an uncontacted population, for example, when such cells are stimulated (e.g., with anti-CD3 and/or anti-CD28 stimulation) or induced to proliferate (e.g., in response to antigen recognition, e.g., antigen recognition through a CAR molecule, e.g., as described herein).

While not wishing to be bound by theory, it is believed that contacting a population of immune effector cells, e.g., engineered to express a CAR molecule, e.g., as described herein, with an LSD1 inhibitor is accompanied by a decrease in the proportion of PD-1 positive immune effector cells, at least transiently, relative to an uncontacted population, for example, when such cells are stimulated (e.g., with anti-CD3 and/or anti-CD28 stimulation) or induced to proliferate (e.g., in response to antigen recognition, e.g., antigen recognition through a CAR molecule, e.g., as described herein).

While not wishing to be bound by theory, it is believed that contacting a population of immune effector cells, e.g., engineered to express a CAR molecule, e.g., as described herein, with an LSD1 inhibitor is accompanied by an increase in the ratio of PD-1 negative immune effector cells / PD-1 positive immune effector cells, at least transiently, relative to an uncontacted population, for example, when such cells are stimulated (e.g., with anti-CD3 and/or anti-CD28 stimulation) or induced to proliferate (e.g., in response to antigen recognition, e.g., antigen recognition through a CAR molecule, e.g., as described herein).

While not wishing to be bound by theory, it is believed that contacting a population of immune effector cells, e.g., engineered to express a CAR molecule, e.g., as described herein, with an LSD1 inhibitor is accompanied by a decrease in the proportion of PD-1+/Lag3+/Tim3+ immune effector cells, at least transiently, relative to an uncontacted population, for example, when such cells are stimulated (e.g., with anti-CD3 and/or anti-CD28 stimulation) or induced to proliferate (e.g., in response to antigen recognition, e.g., antigen recognition through a CAR molecule, e.g., as described herein).

While not wishing to be bound by theory, it is believed that contacting a population of immune effector cells, e.g., engineered to express a CAR molecule, e.g., as described herein, with an LSD1 inhibitor is accompanied by an increase in the ratio of PD-1-/Lag3-/Tim3- immune effector cells to PD-1+/Lag3+/Tim3+ immune effector cells, at least transiently, relative to an uncontacted population, for example, when such cells are stimulated (e.g., with anti-CD3 and/or anti-CD28 stimulation) or induced to proliferate (e.g., in response to antigen recognition, e.g., antigen recognition through a CAR molecule, e.g., as described herein).

While not wishing to be bound by theory, it is believed that contacting a population of immune effector cells, e.g., engineered to express a CAR molecule, e.g., as described herein, with an LSD1 inhibitor is accompanied by an increase in the proliferation of the immune effector cells, at least transiently, relative to an uncontacted population, for example, when such cells are stimulated (e.g., with anti-CD3 and/or anti-CD28 stimulation) or induced to proliferate (e.g., in response to antigen recognition, e.g., antigen recognition through a CAR molecule, e.g., as described herein). Again, without being bound by theory, it is believed that T cells can be exhausted by, for example, stimulation with CD3/CD28 stimulation or antigen stimulation (e.g., by induced signaling through a CAR). Such exhaustion can lead to decreased efficacy or function (e.g., decreased proliferation, persistence, and/or anti-tumor efficacy) of such immune effector cells. As described herein, the inventors have discovered that inhibiting LSD1 increases the proliferation and/or survival of more naive T cells, e.g., T_{SCM} cells, which in turn have better efficacy and function. Thus, embodiments of the invention are based, at least in part, on the recognition that LSD1 inhibition, is associated with improved T cell function and/or phenotype.

These approaches can be used to optimize the performance of immune effector cells, e.g., T cells, in the subject. While not wishing to be bound by theory, it is believed that, in some instances disclosed herein, the performance of endogenous, non-modified immune effector cells, e.g., T cells, is improved. While not wishing to be bound by theory, it is believed that, in some instances, the performance of immune effector cells, e.g., T cells, harvested (e.g., from a subject administered an LSD1 inhibitor) and engineered to express a CAR molecule, e.g., as described herein, is improved. In other instances, a population of immune effector cells, e.g., T cells, which have been, or will be engineered to express a CAR molecule, e.g., as described herein, can be treated ex vivo by contact with an amount of an LSD1 inhibitor that improves the number or ratio of naive T cells, e.g., T_{SCM} cells, and/or improves the number or ratio of PD-1 negative, e.g., PD-1-/Tim3-/Lag3- T cells, relative to an uncontacted population.

The invention provides a composition comprising a population of immune effector cells engineered to express a CAR for use, in combination with an LSD1 inhibitor, in a method of treating a disease associated with the expression of a tumor antigen in a subject, said method comprising administering to said subject an LSD1 inhibitor and a population of immune effector cells engineered to express a CAR, wherein the population of immune effector cells comprises, e.g., consists of, T cells, wherein the disease associated with expression of a tumor antigen is chosen from a proliferative disease, a precancerous condition, and a cancer.

In an instance of the disclosure, the LSD1 inhibitor is administered for an amount of time sufficient to decrease the proportion of PD-1 positive T cells, increase the proportion of PD-1 negative T cells, or increase the ratio of PD-1 negative T cells/PD-1 positive T cells, in the peripheral blood of the subject (or in a preparation of T cells isolated from the subject).

In an instance, treatment, e.g., promoting an immune response in, a subject, e.g., a human subject, comprises inhibiting a negative immune response mediated by the engagement of PD-1 with PD-L1 or PD-L2, e.g., relative to a T cell not contacted with an LSD1 inhibitor.

In an instance, treatment, e.g., promoting an immune response in, a subject, e.g., a human subject, comprises increasing the number of T cells capable of proliferation, e.g., relative to a T cell not contacted with an LSD1 inhibitor.

In an instance, treatment, e.g., promoting an immune response in, a subject, e.g., a human subject, comprises increasing the number of T cells capable of cytotoxic function, secreting cytokines, or activation, e.g., relative to a T cell not contacted with an LSD1 inhibitor.

In an instance, treatment, e.g., promoting an immune response in, a subject, e.g., a human subject, comprises increasing the amount of cytokine secretion (e.g., interferon gamma (IFN-g) and/or interleukin 2 (IL-2)) in response to stimulation and/or activation of the T cell, e.g., relative to a T cell not contacted with an LSD1 inhibitor.

The invention provides a composition comprising a population of immune effector cells engineered to express a CAR for use, in combination with an LSD1 inhibitor, in a method of increasing the therapeutic efficacy of a population of immune effector cells engineered to express a CAR, e.g., a CART19 (e.g., CTL019) in a subject having a disease associated with expression of a tumor antigen, said method comprising a step of decreasing the activity or expression of LSD1 in said cells, transiently or permanently, optionally wherein the step of decreasing the activity or expression of LSD1 in said cells comprises contacting the cells with an LSD1 inhibitor, wherein the population of immune effector cells comprises, e.g., consists of, T cells, wherein the disease associated with expression of a tumor antigen is chosen from a proliferative disease, a precancerous condition, and a cancer.

In an embodiment, the LSD1 inhibitor is administered (in vivo or ex vivo) prior to administration of immune effector cells, e.g., T cells to be engineered to express a CAR molecule, e.g., as described herein, (e.g., prior to or after harvest of the immune effector cells) for an amount of time sufficient for one or more of the following to occur:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14 an increase in the production of cytokines (e.g., IFNg and/or IL-2) from said population of immune effector cells; or
15) a combination of two, three, four, five, six, seven, eight, nine, ten, eleven, twelve, thirteen, or more (e.g., all) of the above;
and wherein 1), 2), 3), 4), 5), 6), 7), 8), 9), 10), 11), 12) 13), 14) or 15) occurs e.g., at least transiently, e.g., permanently, e.g., as compared to a non-treated subject. In an embodiment, the immune effector cell, e.g., T cell, to be engineered to express a CAR molecule, e.g., as described herein, is harvested at least 5, 10, 15, 20, 25, 30, 35, 40, 45, 50, 55, or 60 days after initiation, or completion, of dosing with the LSD1 inhibitor.

In the invention, the population of immune effector cells comprises, e.g., consists of, T cells.

In an embodiment, the LSD1 inhibitor is administered to a subject prior to harvest of immune effector cells, e.g., T cells to be engineered to express an CAR molecule, e.g., as described herein, for an amount of time sufficient for one or more of the following to occur e.g., to occur in the harvested cells or in the engineered cells (or in non-harvested cells, or in both):
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14 an increase in the production of cytokines (e.g., IFNg and/or IL-2) from said population of immune effector cells; or
15) a combination of two, three, four, five, six, seven, eight, nine, ten, eleven, twelve, thirteen, or more (e.g., all) of the above;
and wherein 1), 2), 3), 4), 5), 6), 7), 8), 9), 10), 11), 12) 13), 14) or 15) occurs e.g., at least transiently, e.g., permanently, e.g., as compared to a non-treated subject. In an embodiment, the immune effector cell, e.g., T cell, to be engineered to express a CAR molecule, e.g., as described herein, is harvested at least 5, 10, 15, 20, 25, 30, 35, 40, 45, 50, 55, or 60 days after initiation, or completion, of dosing with the LSD1 inhibitor.

In an embodiment, the LSD1 inhibitor is administered after harvest of immune effector cells, e.g., T cells to be engineered to express an CAR molecule, e.g., as described herein, for an amount of time sufficient for one or more of the following to occur:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14 an increase in the production of cytokines (e.g., IFNg and/or IL-2) from said population of immune effector cells; or
15) a combination of two, three, four, five, six, seven, eight, nine, ten, eleven, twelve, thirteen, or more (e.g., all) of the above;
and wherein 1), 2), 3), 4), 5), 6), 7), 8), 9), 10), 11), 12) 13), 14) or 15) occurs e.g., at least transiently, e.g., permanently, e.g., as compared to a non-treated subject.

In an embodiment, the LSD1 inhibitor is administered after administration of immune effector cells, e.g., T cells to be engineered to express an CAR molecule, e.g., as described herein, for an amount of time sufficient for one or more of the following to occur:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14 an increase in the production of cytokines (e.g., IFNg and/or IL-2) from said population of immune effector cells; or
15) a combination of two, three, four, five, six, seven, eight, nine, ten, eleven, twelve, thirteen, or more (e.g., all) of the above;
and wherein 1), 2), 3), 4), 5), 6), 7), 8), 9), 10), 11), 12) 13), 14) or 15) occurs e.g., at least transiently, e.g., permanently, e.g., as compared to a non-treated subject.

In an embodiment, LSD1 inhibitor is administered to immune effector cells, e.g., T cells, which have, or will be engineered to express a CAR molecule, e.g., as described herein, ex vivo for an amount of time sufficient for one or more of the following to occur:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14 an increase in the production of cytokines (e.g., IFNg and/or IL-2) from said population of immune effector cells; or
15) a combination of two, three, four, five, six, seven, eight, nine, ten, eleven, twelve, thirteen, or more (e.g., all) of the above;
and wherein 1), 2), 3), 4), 5), 6), 7), 8), 9), 10), 11), 12) 13), 14) or 15) occurs e.g., at least transiently, e.g., permanently, e.g., as compared to a non-treated cell.

Without being bound by theory, it is believed that LSD1 may also directly demethylate p53 (Nature Reviews Molecular Cell Biology 13, 297-311 (May 2012) doi:10.1038/nrm3327). Thus, in an embodiment, the compounds and methods disclosed herein may be used to inhibit demethylation of p53.

In an embodiment, the subject has cancer and the treatment comprises promoting the subject's immune response to the cancer. In an embodiment, the subject was selected on the basis of having cancer. In an embodiment, a cell of the cancer expresses PD-L1 or PD-L2. In an embodiment, a cell in the cancer microenvironment expresses PD-L1 or PD-L2.

In an embodiment, the cancer comprises a solid tumor. In an embodiment, the cancer is a hematological cancer. In an embodiment, the cancer is a leukemia. In an embodiment, the cancer is a chronic lymphocytic leukemia (CLL). In an embodiment, the cancer is CLL and wherein the antigen binding domain of the CAR targets CD19. In an embodiment, the cancer is melanoma.

In an embodiment, the treatment further comprises administering an additional treatment, e.g., a chemotherapeutic, radiation, a cellular therapy, or bone marrow transplant to the subject. In an embodiment, the treatment further comprises administering an additional treatment that kills T cells, e.g., radiation or cytotoxic chemotherapy. In an embodiment, the treatment further comprises administering to the subject an mTOR pathway inhibitor, such as vitamin E, vitamin A, an antibacterial antibiotic, an antioxidant, L- carnitine, lipoic acid, metformin, resveratrol, leptine, a non-steroid antiinflammatory drug, or a COX inhibitor. In an embodiment, the treatment further comprises administering metformin to the subject. In an embodiment, the LSD1 inhibitor is administered prior to or after the initiation of the additional treatment. In an embodiment, the treatment further comprises administering an additional treatment for the cancer.

In an embodiment, the treatment further comprises administering the immune effector cell, e.g., T cell, engineered to express a CAR molecule, e.g., as described herein, in combination with another agent (in addition to the LSD1 inhibitor). In one embodiment, the agent can be a kinase inhibitor, e.g., a CDK4/6 inhibitor, a BTK inhibitor, an mTOR inhibitor, a MNK inhibitor, or a dual mTOR/PI3K kinase inhibitor, and combinations thereof.

In an embodiment, the treatment comprises providing an anti-tumor immunity in a mammal. In one embodiment, the cell is an autologous T cell or an autologous NK cell. In one embodiment, the cell is an allogeneic T cell or an allogeneic NK cell. In one embodiment, the mammal is a human.

In an embodiment the treatment comprises treating a mammal having a disease associated with expression of a cancer associated antigen or tumor marker.

In one embodiment, the treatment comprises administering an agent that increases the efficacy of the immune effector cell, e.g., T cell or NK cell, engineered to express a CAR molecule, e.g., as described herein, e.g., an agent described herein.

In one embodiment, the treatment comprises administering an agent that ameliorates one or more side effect associated with administration of a cell expressing a CAR molecule, e.g., as described herein, the immune effector cell, e.g., T cell or NK cell, engineered to express a CAR molecule, e.g., as described herein, e.g., an agent described herein.

In one embodiment, the treatment comprises administering an agent that treats the disease associated with a cancer associated antigen as described herein, e.g., an agent described herein.

In one embodiment, the immune effector cell, e.g., T cell or NK cell, engineered to express a CAR molecule, e.g., as described herein, expresses two or more CAR molecules and, e.g., is administered to a subject in need thereof to treat cancer.

In one embodiment, the CAR molecule is introduced into immune effector cells (e.g., T cells, NK cells), e.g., using in vitro transcription, and the subject (e.g., human) receives an initial administration of cells comprising a CAR molecule, and one or more subsequent administrations of cells comprising a CAR molecule, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of cells comprising a CAR molecule are administered to the subject (e.g., human) per week, e.g., 2, 3, or 4 administrations of cells comprising a CAR molecule are administered per week. In one embodiment, the subject (e.g., human subject) receives more than one administration of cells comprising a CAR molecule per week (e.g., 2, 3 or 4 administrations per week) (also referred to herein as a cycle), followed by a week of no administration of cells comprising a CAR molecule, and then one or more additional administrations of cells comprising a CAR molecule (e.g., more than one administration of the cells comprising a CAR molecule per week) are administered to the subject. In another embodiment, the subject (e.g., human subject) receives more than one cycle of cells comprising a CAR molecule, and the time between each cycle is less than 10, 9, 8, 7, 6, 5, 4, or 3 days. In one embodiment, the cells comprising a CAR molecule are administered every other day for 3 administrations per week. In one embodiment, the cells comprising a CAR molecule are administered for at least two, three, four, five, six, seven, eight or more weeks.

In one embodiment, the immune effector cell, e.g., T cell or NK cell, engineered to express a CAR, e.g., a CAR molecule described herein, is administered as a first line treatment for the disease, e.g., the cancer, e.g., the cancer described herein. In another embodiment, the immune effector cell, e.g., T cell, engineered to express a CAR, e.g., a CAR molecule described herein, is administered as a second, third, fourth line treatment for the disease, e.g., the cancer, e.g., the cancer described herein.

In one embodiment, a population of cells as defined in the claims is administered.

In one embodiment, the LSD1 inhibitor and the immune effector cell, e.g., a T cell, engineered to express a CAR molecule, e.g., as described herein, are present in a single composition, e.g., are administered as a single composition. In one embodiment, LSD1 inhibitor and the immune effector cell, e.g., a T cell, engineered to express a CAR molecule, e.g., as described herein, are present in separate compositions, e.g., are administered as separate compositions.

In certain aspects, the disclosure provides an LSD1 inhibitor for use in treating a subject, wherein said LSD1 inhibitor enhances an immune response of said subject, and wherein said subject has received, is receiving or is about to receive an immune effector cell engineered to express a CAR molecule, e.g., as described herein.

In certain aspects, the disclosure provides an immune effector cell engineered to express a CAR molecule, e.g., as described herein for use in treating a subject, wherein said subject has received, is receiving, or is about to receive, an LSD1 inhibitor, e.g., one that enhances an immune response of said subject.

In certain aspects, the disclosure provides an immune effector cell engineered to express a CAR molecule, e.g., as described herein for use in treating a subject, wherein said immune effector cell engineered to express a CAR molecule, e.g., as described herein has been contacted with an LSD1 inhibitor, e.g., contacted ex vivo with an LSD1 inhibitor.

In one embodiment, the population of autologous or allogeneic immune effector cells are transfected or transduced with a vector comprising a nucleic acid molecule encoding a CAR molecule, e.g., as described herein. In one embodiment, the vector is a retroviral vector. In one embodiment, the vector is a self-inactivating lentiviral vector as described elsewhere herein. In one embodiment, the vector is delivered (e.g., by transfecting or electroporating) to a cell, e.g., a T cell or a NK cell, wherein the vector comprises a nucleic acid molecule encoding a CAR molecule, e.g., as described herein, which is transcribed as an mRNA molecule, and the CAR molecule is translated from the RNA molecule and expressed on the surface of the cell.

In an embodiment, a population of CAR-expressing cells, e.g., CAR-expressing T cells (CART cells) or CAR-expressing NK cells, is administered. In some embodiments, the population of CAR-expressing cells comprises a mixture of cells expressing different CARs. For example, in one embodiment, the population of CAR-expressing cells can include a first cell expressing a CAR having an antigen binding domain that binds to a first tumor marker as described herein, and a second cell expressing a CAR having a different antigen binding domain that binds to a second tumor marker as described herein. As another example, the population of CAR-expressing cells can include a first cell expressing a CAR that includes an antigen binding domain that binds to a tumor marker as described herein, and a second cell expressing a CAR that includes an antigen binding domain to a target other than a tumor marker as described herein. In one embodiment, the population of CAR-expressing cells includes, e.g., a first cell expressing a CAR that includes a primary intracellular signaling domain, and a second cell expressing a CAR that includes a secondary signaling domain.

In one aspect, the invention relates to treating a subject (e.g., a subject suffering from a disease, e.g., a disease associated with expression of a tumor antigen, e.g., a cancer), that includes administering an LSD1 inhibitor and a population of immune effector cells engineered to express a chimeric antigen receptor (CAR). In the treatments of the invention, the population of immune effector cells comprises, e.g., consists of, T cells, and the treatment is of a disease associated with expression of a tumor antigen, chosen from a proliferative disease, a precancerous condition, and a cancer. In embodiments, the treating includes administering the LSD1 inhibitor before the population of immune effector cells. In embodiments, the treating includes administering the LSD1 inhibitor concurrently with the population of immune effector cells. In embodiments, the treating includes administering the LSD1 inhibitor after the population of immune effector cells. In embodiments, the treating includes administering the LSD1 inhibitor (e.g., for an interval) before and after the population of immune effector cells is administered.

In one aspect, the invention relates to treating a subject (e.g., a subject suffering from a disease, e.g., a disease associated with expression of a tumor antigen, e.g., a cancer), that includes administering an LSD1 inhibitor to the subject, wherein said subject has received, is receiving or is about to receive a population of immune effector cells engineered to express a chimeric antigen receptor (CAR). In embodiments, the treating includes administering to a subject an LSD1 inhibitor and a population of immune effector cells engineered to express a CAR molecule, e.g., as described herein. In embodiments, the LSD1 inhibitor is administered before the population of immune effector cells engineered to express a CAR molecule, e.g., as described herein, and wherein said administration of the LSD1 inhibitor is continued for a period of time after the administration of the population of immune effector cells engineered to express a CAR molecule, e.g., as described herein. In other embodiments, the administration of the LSD1 inhibitor after the administration of the population of immune effector cells engineered to express a CAR molecule, e.g., as described herein is in an amount sufficient to increase an anti-tumor effect of the population of immune effector cells engineered to express a CAR molecule, e.g., as described herein relative to an equivalent population of immune effector cells engineered to express a CAR molecule, e.g., as described herein administered in the absence of said LSD1 inhibitor.

In another aspect, the invention relates to increasing the therapeutic efficacy in a subject of a population of immune effector cells engineered to express a CAR molecule, e.g., as described herein, e.g., a CAR19 (e.g., CTL019), including a step of decreasing the activity or expression of LSD1 in said cell, at least transiently. In embodiments, the step of decreasing the activity or expression of LSD1 in said cell includes contacting the cell with an LSD1 inhibitor. In embodiments, the contacting is done ex vivo. In embodiments, the contacting is done in vivo (e.g., the population of immune effector cells and the LSD1 inhibitor are coadministered to the subject).

In embodiments of any of the foregoing aspects, the administration or the contacting of the LSD1 inhibitor results in:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14 an increase in the production of cytokines (e.g., IFNg and/or IL-2) from said population of immune effector cells; or
15) a combination of two, three, four, five, six, seven, eight, nine, ten, eleven, twelve, thirteen, or more (e.g., all) of the above.

In embodiments, the effect is transient. In embodiments, the effect is permanent. In embodiments, the effect is as compared to cells not contacted with the LSD1 inhibitor. In embodiments, the effect is as compared to cells of the same subject not contacted with the LSD1 inhibitor.

In another aspect, disclosed herein is a method of treating a subject that includes:
a) administering an LSD1 inhibitor to a subject;
b) collecting a population of immune effector cells from the subject of a), after said administration of the LSD1 inhibitor;
c) providing said population of immune effector cells *ex vivo*;
d) contacting said *ex vivo* population of immune effector cells with the LSD1 inhibitor, wherein the contacting with the LSD1 inhibitor causes one or more of the following to occur:
   1) an increase in the proportion of naive T cells, e.g., T_{SCM} cells;
   2) an increase in the number of naive T cells, e.g., T_{SCM} cells;
   3) a decrease in the number of T_{EM} cells;
   4) a decrease in the proportion of T_{EM} cells;
   5) an increase in the proportion of CD45RA+CD62L+ T cells;
   6) an increase in the number of CD45RA+CD62L+ T cells;
   7) an increase in the proportion of CD45RA+CCR7+ T cells;
   8) an increase in the number of CD45RA+CCR7+ T cells;
   9) a decrease in the proportion of PD-1 positive immune effector cells;
   10) an increase in the ratio of PD-1 negative immune effector cells / PD-1 positive immune effector cells;
   11) a decrease in the proportion of PD-1+/Lag3+/Tim3+ immune effector cells;
   12) an increase in the ratio of PD-1-/Lag3-/Tim3- immune effector cells to PD-1+/Lag3+/Tim3+ immune effector cells;
   13) an increase in the proliferation of the immune effector cells;
   14 an increase in the production of cytokines (e.g., IFNg and/or IL-2) from said population of immune effector cells; or
   15) a combination of two, three, four, five, six, seven, eight, nine, ten, eleven, twelve, thirteen, or more (e.g., all) of the above;
and e) administering the population of immune effector cells to a subject.

In instances, the effect of d) is transient. In instances, the effect of d) is permanent. In instances, the effect of d) is as compared to cells not contacted with the LSD1 inhibitor. In instances, the administering of step e) is to the same subject as the subject of step b) (e.g., relates to a method of treatment using a population of autologous immune effector cells). In instances, the administering of step e) is to a different subject, e.g., of the same species, as the subject of step b) (e.g., relates to treatment using a population of allogeneic immune effector cells).

In instances, step of e) further includes administering the LSD1 inhibitor to the subject. In instances, the method further includes the step of inserting nucleic acid that encodes a CAR molecule, e.g., as described herein into cells of the *ex vivo* population of immune effector cells.

In another aspect, the invention relates to the use of LSD1 inhibitors in the manufacture of a population of immune effector cells, e.g., engineered to express a CAR molecule, e.g., as described herein. In one aspect, the invention provides a method of making a population of immune effector cells, which is optionally a population of T cells, including the steps of:
a) contacting a population of immune effector cells with an LSD1 inhibitor;
   thereby making a population of immune effector cells, which is optionally a population of T cells,
   wherein the contacting with the LSD1 inhibitor causes one or more of the following to occur:
      1) an increase in the proportion of naive T cells, e.g., T_{SCM} cells;
      2) an increase in the number of naive T cells, e.g., T_{SCM} cells;
      3) a decrease in the number of T_{EM} cells;
      4) a decrease in the proportion of T_{EM} cells;
      5) an increase in the proportion of CD45RA+CD62L+ T cells;
      6) an increase in the number of CD45RA+CD62L+ T cells;
      7) an increase in the proportion of CD45RA+CCR7+ T cells;
      8) an increase in the number of CD45RA+CCR7+ T cells;
      9) a decrease in the proportion of PD-1 positive immune effector cells;
      10) an increase in the ratio of PD-1 negative immune effector cells / PD-1 positive immune effector cells;
      11) a decrease in the proportion of PD-1+/Lag3+/Tim3+ immune effector cells;
      12) an increase in the ratio of PD-1-/Lag3-/Tim3- immune effector cells to PD-1+/Lag3+/Tim3+ immune effector cells;
      13) an increase in the proliferation of the immune effector cells;
      14 an increase in the production of cytokines (e.g., IFNg and/or IL-2) from said population of immune effector cells; or
      15) a combination of two, three, four, five, six, seven, eight, nine, ten, eleven, twelve, thirteen, or more (e.g., all) of the above.

In the methods of making a population of immune effector cells of the invention, the population of immune effector cells comprises, e.g., consists of, T cells.

In embodiments, the effect of 1)-15) is transient. In embodiments, the effect of 1)-15) is permanent. In embodiments, the effect of 1)-15) is as compared to cells not contacted with the LSD1 inhibitor.

In embodiments, the method further includes the step of b) inserting nucleic acid that encodes a CAR molecule, e.g., as described herein, into cells of the population of immune effector cells. In embodiments, the contacting of step a) occurs
1) prior to;
2) concurrently with;
3) after; or
4) both before and after;
said inserting of step b). In embodiments, the contacting of step a), and optionally the inserting of step b), is *ex vivo.*

In another aspect, the disclosure features cells, e.g., immune effector cells, e.g., a population of immune effector cells, e.g., engineered to express a CAR molecule, e.g., as described herein, made by any of the methods described in the foregoing aspects.

In another aspect, the invention features a population of immune effector cells engineered to express a CAR molecule, e.g., as described herein, wherein the CAR includes an antigen-binding domain, a transmembrane domain, and an intracellular signaling domain, and wherein expression and/or function of LSD1 in said cell has been reduced or eliminated. In the population of immune effector cells of the invention, the population of immune effector cells further comprises an LSD1 inhibitor, and the population of immune effector cells comprises, e.g., consists of, T cells. In an embodiment, the reduction or elimination is at least transient. In embodiments, the population of immune effector cells has been contacted with an LSD1 inhibitor. In embodiments the invention features a composition comprising the population of immune effector cells described above and an LSD1 inhibitor.

In any of the foregoing aspects, instances and embodiments, the cells and/or population of cells are (or include) immune effector cells, e.g., the population of immune effector cells includes, e.g., consists of, T cells or NK cells. In the invention, the population of immune effector cells comprises, e.g., consists of, T cells. In embodiments, the cells are T cells, e.g., CD8+ T cells, CD4+ T cells, or a combination thereof. In embodiments, the cells are NK cells.

In embodiments, the cells are human cells. In embodiments, the cells are autologous, e.g., to the subject to be administered the cells. In embodiments, the cells are allogeneic, e.g., to the subject to be administered the cells.

In embodiments, the cells are, or include, cells engineered to express a CAR molecule, e.g., as described herein.

In any of the foregoing aspects and embodiments involving a CAR, the CAR includes an antigen binding domain (which is optionally an antibody or antibody fragment, TCR or TCR fragment), a transmembrane domain, and an intracellular signaling domain (which is optionally an intracellular signaling domain including a costimulatory domain and/or a primary signaling domain). In embodiments, the antigen-binding domain binds to a tumor antigen is selected from a group consisting of: TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, PRSS21, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1. In one embodiment, the antigen binding domain binds CD19, e.g., is an antigen binding domain as described in, e.g., WO2012/079000 or WO2014/153270. In one embodiment, the antigen binding domain binds BCMA, e.g., is an antigen binding domain as described in, e.g., WO2016/014565, e.g., is the antigen binding domain of CAR BCMA-10 (139109) from WO2016/014565.

In embodiments, the antigen-binding domain is an antibody or antibody fragment comprising:
(i) the amino acid sequence of a CD19 binding domain according to Tables 6-9, e.g., the amino acid sequence of CTL019 scFv domain according to Table 9 or an amino acid sequence according to SEQ ID NO: 957, or an amino acid sequence at least 95% identical thereto;
(ii) the amino acid sequence of a humanized CD19 binding domain according to Tables 6-9, e.g., the amino acid sequence of CAR2 scFv domain according to Table 9 or an amino acid sequence according to SEQ ID NO: 898, or an amino acid sequence at least 95% identical thereto; or
(iii) the amino acid sequence of a BCMA binding domain according to Tables 11A-11B, e.g., the amino acid sequence of 139109 scFv domain according to Table 11A or an amino acid sequence according to SEQ ID NO: 967, or an amino acid sequence at least 95% identical thereto.

In embodiments, the transmembrane domain includes:
(i) an amino acid sequence having at least one, two or three modifications but not more than 20, 10 or 5 modifications of an amino acid sequence of SEQ ID NO: 12, or a sequence with 95-99% identity to an amino acid sequence of SEQ ID NO: 12; or
(ii) the sequence of SEQ ID NO: 12.

In embodiments, the antigen binding domain is connected to the transmembrane domain by a hinge region, wherein said hinge region includes SEQ ID NO: 2 or SEQ ID NO: 6, or a sequence with 95-99% identity thereof.

In embodiments, the intracellular signaling domain includes a primary signaling domain and/or a costimulatory signaling domain, wherein the primary signaling domain includes a functional signaling domain of a protein chosen from CD3 zeta, CD3 gamma, CD3 delta, CD3 epsilon, common FcR gamma (FCER1G), FcR beta (Fc Epsilon R1b), CD79a, CD79b, Fcgamma RIIa, DAP10, or DAP12.

In embodiments, the primary signaling domain includes:
(i) an amino acid sequence having at least one, two or three modifications but not more than 20, 10 or 5 modifications of an amino acid sequence of SEQ ID NO: 18 or SEQ ID NO: 20, or a sequence with 95-99% identity to an amino acid sequence of SEQ ID NO: 18 or SEQ ID NO: 20; or
(ii) the amino acid sequence of SEQ ID NO:18 or SEQ ID NO: 20.

In embodiments, the intracellular signaling domain includes a costimulatory signaling domain, or a primary signaling domain and a costimulatory signaling domain, wherein the costimulatory signaling domain includes a functional signaling domain of a protein selected from the group consisting of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, a ligand that specifically binds with CD83, CDS, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, and NKG2D, e.g., the costimulatory signaling domain includes an amino acid sequence having at least one, two or three modifications but not more than 20, 10 or 5 modifications of an amino acid sequence of SEQ ID NO:14 or SEQ ID NO: 16, or a sequence with 95-99% identity to an amino acid sequence of SEQ ID NO:14 or SEQ ID NO: 16, e.g., the costimulatory signaling domain includes a sequence of SEQ ID NO: 14 or SEQ ID NO: 16, e.g., the intracellular domain includes the sequence of SEQ ID NO: 14 or SEQ ID NO: 16, and the sequence of SEQ ID NO: 18 or SEQ ID NO: 20, wherein the sequences including the intracellular signaling domain are expressed in the same frame and as a single polypeptide chain.

In embodiments, the CAR includes a leader sequence including, e.g., consisting of, SEQ ID NO: 2.

In embodiments, the CAR comprises:
(i) the amino acid sequence of a CD19 CAR according to Tables 6-9, e.g., the amino acid sequence of CTL019 according to Table 9 or an amino acid sequence according to SEQ ID NO: 956 or an amino acid sequence at least 95% identical thereto;
(ii) the amino acid sequence of a humanized CD19 CAR according to Tables 6-9, e.g., the amino acid sequence of CAR2 according to Table 9 or an amino acid sequence according to SEQ ID NO: 902, or an amino acid sequence at least 95% identical thereto; or
(iii) the amino acid sequence of a BCMA CAR according to Tables 11A-11B, e.g., the amino acid sequence of 139109 CAR according to Table 11A or an amino acid sequence according to SEQ ID NO: 971, or an amino acid sequence at least 95% identical thereto.

In any of the foregoing aspects and embodiments, the LSD1 inhibitor may be: (1) a gene editing system targeted to one or more sites of the LSD1 gene, or its corresponding regulatory elements; (2) a nucleic acid (e.g., an siRNA or shRNA, or antisense oligonucleotide) including sequence complementary to a target sequence of the LSD1 gene; (3) a protein (e.g., a dominant negative LSD1, e.g., catalytically inactive LSD1, or a dominant negative binding partner of LSD1); (4) a small molecule; (5) a nucleic acid encoding any of (1)-(3); or (6) any combination of (1)-(5).

In one aspect, the LSD1 inhibitor is an shRNA or siRNA. In embodiments, the LSD1 inhibitor is a shRNA. In embodiments, the LSD1 inhibitor is as siRNA. In embodiments, the shRNA or siRNA includes sequence complementary to a target sequence of the LSD1 gene (KDM 1A), e.g., listed in Table 1, e.g., selected from SEQ ID NO: [43] to SEQ ID NO: [82].

In another aspect, the LSD1 inhibitor is an shRNA encoded by nucleic acid including any sequence encoding an anti-LSD1 shRNA of Table 1, e.g., encoded by nucleic acid including a sequence selected from SEQ ID NO: [83] to SEQ ID NO: [122].

In another aspect, the LSD1 inhibitor is nucleic acid including any sequence encoding an anti-LSD1 shRNA of Table 1, e.g., a sequence selected from SEQ ID NO: [83] to SEQ ID NO: [122].

In another aspect, the LSD1 inhibitor is an antisense oligonucleotide. In embodiments, the antisense oligonucleotide includes sequence that is complementary to a sequence of an LSD1 mRNA. In embodiments, the antisense oligonucleotide includes sequence that is complementary to a sequence of an LSD1 pre-mRNA.

In embodiments, the nucleic acid encoding the LSD1 inhibitor is disposed on a vector, e.g., a vector further including a U6 or H1 promoter operably linked to said nucleic acid, e.g., a retroviral vector, a lentiviral vector, an adenoviral vector, an adeno-associated viral (AAV) vector, a herpes simplex virus (HSV) vector, a plasmid, a minicircle, a nanoplasmid, or an RNA vector. In embodiments the vector further includes sequence encoding a CAR molecule.

In another aspect, the LSD1 inhibitor is a genome editing system specific for a sequence of the LSD1 gene (KDM1A) or its regulatory elements selected from a CRISPR genome editing system, a zinc finger nuclease genome editing system, a TALEN genome editing system and a meganuclease genome editing system.

In another aspect, the LSD1 inhibitor is a CRISPR genome editing system including a gRNA molecule including a targeting domain complementary to a sequence of the LSD1 gene (KDM1A) or its regulatory elements, e.g., including any one of SEQ ID NO: [132] to [862].

In other aspect, the LSD1 inhibitor is a small molecule. In embodiments, the small molecule is a reversible LSD1 inhibitor. In embodiments, the small molecule is an irreversible LSD1 inhibitor. In embodiments, the small molecule LSD1 inhibitor is:
a) GSK2699537;
b) rel- 2-[[(1R,2S)-2-[4-[(4-chlorophenyl)methoxy]phenyl]cyclopropyl]amino]-1-(4-methyl-1-piperazinyl)-ethanone (described in PCT Publication No. WO 2010043721);
c) (R)-4-(5-(pyrrolidin-3-ylmethoxy)-2-(p-tolyl)pyridin-3-yl)benzonitrile;
d) (1S,2R)-N-((2-methoxypyridin-3-yl)methyl)-2-phenylcyclopropan-1-amine;
e) N,N-dimethyl-1-((4-(4-(4-(piperidin-4-yl)phenyl)-1H-indazol-1-yl)phenyl)sulfonyl)piperidin-4-amine;
f) 5-(6-chloro-4'-(methylsulfonyl)-[1,1'-biphenyl]-3-yl)-2-(piperazin-1-yl)-1H-pyrrole-3-carbonitrile;
g) rel- N-[(1R,2S)-2-Phenylcyclopropyl]-4-Piperidinamine; or
h) 2-(1R,2S)-2-(4-(Benzyloxy)phenyl)cyclopropylamino)-1-(4-methylpiperazin-1-yl)ethanone;
i) Trans-3-(3-amino-2-methylphenyl)-1-(4-hydroxycyclohexyl)-6-methyl-1H-indole-5-carbonitrile; or
j) a pharmaceutically acceptable salt of any of the foregoing. In embodiments, the LSD1 inhibitor is a small molecule and said LSD1 inhibitor is conjugated to an antibody or antigen-binding fragment thereof, e.g., an antibody or antigen-binding fragment thereof that recognizes an antigen on the surface of a T cell, e.g., CD3.

In another aspect, the LSD1 inhibitor is a protein, e.g., is a dominant negative binding partner of LSD1 (e.g., a histone deacetylase (HDAC) that interacts with LSD1 or other member of the Co-REST or AR co-activator complex), or nucleic acid encoding said dominant negative binding partner of LSD1.

In another aspect, the inhibitor of LSD1 is a protein, e.g., is a dominant negative (e.g., catalytically inactive) LSD1, or nucleic acid encoding said dominant negative LSD1.

In another aspect, the present disclosure provides for treating a subject in need thereof, including administering to said subject an effective amount of the population of immune effector cells of any of the previous aspects and embodiments. In embodiments, the treating further includes administering to said subject an LSD1 inhibitor. In embodiments, the subject receives a pre-treatment of an LSD1 inhibitor, prior to the administration of the population of immune effector cells. In embodiments, the subject receives concurrent treatment with an LSD1 inhibitor and the population of immune effector cells. In embodiments, the subject receives treatment with an LSD1 inhibitor after administration of the population of immune effector cells; In embodiments, the subject receives a combination of any of the foregoing.

In an aspect, including in the previous aspects relating to treatment, the disclosure relates to treating a subject, wherein the subject has a disease associated with expression of a tumor antigen, e.g., a proliferative disease, a precancerous condition, a cancer, or a non-cancer related indication associated with expression of the tumor antigen. In the invention, the disease associated with expression of a tumor antigen is chosen from a proliferative disease, a precancerous condition, and a cancer. In embodiments, the cancer is a hematologic cancer chosen from one or more of chronic lymphocytic leukemia (CLL), acute leukemias, acute lymphoid leukemia (ALL), acute myeloid leukemia (AML), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, or pre-leukemia. In embodiments, the cancer is selected from the group consisting of colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.

In another aspect, the disclosure provides novel compounds. Such compounds are useful, for example, in the methods and compositions described herein, but such uses and compositions are not intended to be limiting. The compound may be selected from N,N-dimethyl-1-((4-(4-(4-(piperidin-4-yl)phenyl)-1H-indazol-1-yl)phenyl)sulfonyl)piperidin-4-amine and 5-(6-chloro-4'-(methylsulfonyl)biphenyl-3-yl)-2-(piperazin-1-yl)-1H-pyrrole-3-carbonitrile. In an instance, the disclosure provides N,N-dimethyl-1-((4-(4-(4-(piperidin-4-yl)phenyl)-1H-indazol-1-yl)phenyl)sulfonyl)piperidin-4-amine. In an instance, the disclosure provides 5-(6-chloro-4'-(methylsulfonyl)biphenyl-3-yl)-2-(piperazin-1-yl)-1H-pyrrole-3-carbonitrile. The disclosure further provides a pharmaceutically acceptable salt of any of the foregoing. The disclosure further provides a compound described above, for use in the manufacture of a medicament. The disclosure further provides a compound described above, for use as a medicament. The disclosure further provides a compound described above, for use in the manufacture of a medicament for use as an LSD1 inhibitor, e.g., for use as an LSD1 inhibitor in any of the methods described herein. In an instance, the disclosure provides a compound described above, for use in therapy, alone, or optionally in combination with at least another agent.

In another aspect, the disclosure provides a composition for use in *ex vivo* manufacturing a population of immune effector cells, that includes an LSD1 inhibitor, e.g., a small molecule LSD1 inhibitor. In instances, the composition includes the small molecule LSD1 inhibitor at a concentration of ranges from about 0.001 nM to about 10 mM, e.g., from about 0.001 nM to about 100 nM, or from, e.g., about 0.1 uM to about 10 uM.

In an aspect, the disclosure provides an LSD1 inhibitor, for use in treating a subject, wherein said subject has received, is receiving, or is about to receive therapy including an immune effector cell, e.g., an immune effector cell engineered to express a CAR molecule, e.g., as described herein.

In an aspect, the disclosure provides an LSD1 inhibitor, for use in the manufacture of an immune effector cell, e.g., an immune effector cell engineered to express a CAR molecule, e.g., as described herein.

In an aspect, the invention provides a method of manufacturing an immune effector cell, e.g., a population of immune effector cells, that includes introducing into said cells nucleic acid encoding a CAR molecule, e.g., as described herein, wherein the nucleic acid integrates into the genome of said cell within the LSD1 gene, such that LSD1 expression and/or function is reduced. In the invention, the population of immune effector cells comprises, e.g., consists of, T cells.

### BRIEF DESCRIPTION OF THE DRAWINGS

Figure 1 depicts the percentage of CD8+ T cells from human donors that are CD45RA+CD62L+ after activation using CD3/CD28 in the presence of an LSD1 inhibitor shRNA (molecules 1A, 1B, 2, 3A, 3B, 4 or 6A) compared to control.
Figure 2 depicts the percentage of CD4+ T cells from human donors that are CD45RA+CD62L+ after activation using CD3/CD28 in the presence of an LSD1 inhibitor shRNA (molecules 1A, 1B, 2, 3A, 3B, 4 or 6A) compared to control.

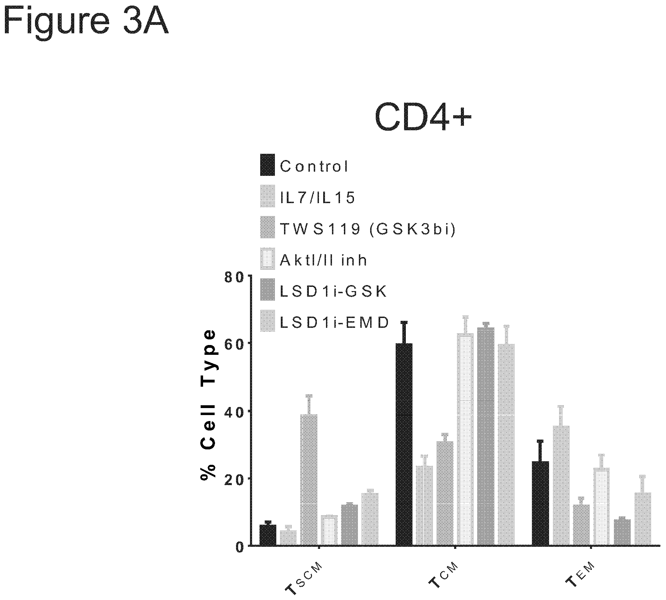

Figure 3A shows the ability of the indicated compounds to produce T cells of a given phenotype was assessed. Naive human T cells (peripheral pan CD3+ T cells, pooled population) were isolated by negative selection and expanded with anti-CD3/CD28 beads at a 3:1 ratio for 10 days in the presence of the indicated compounds. Compounds were refreshed every 2 days. Following expansion, T cell phenotypes were determined by FACS staining. LSD1 inhibition significantly enhanced the percentage of Tscm cells while reducing the percentage of Tem cells in CD4+ T cells relative to controls and relative to other known conditions.

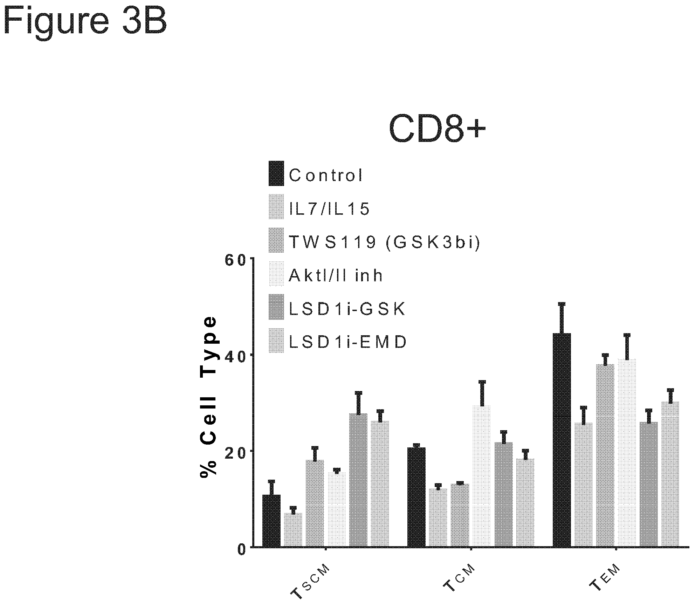

Figure 3B shows the ability of the indicated compounds to produce T cells of a given phenotype was assessed. Naive human T cells (peripheral pan CD3+ T cells, pooled population) were isolated by negative selection and expanded with anti-CD3/CD28 beads at a 3:1 ratio for 10 days in the presence of the indicated compounds. Compounds were refreshed every 2 days. Following expansion, T cell phenotypes were determined by FACS staining. LSD1 inhibition significantly enhanced the percentage of T_{SCM} cells while reducing the percentage of Tem cells in CD8+ T cells relative to controls and relative to other known conditions.
Figure 4A shows the effect of LSD1 inhibition in comparison to other compounds believed to affect T cell phenotype on T_{SCM} to T_{EM} ratio in CD4+ cells.
Figure 4B shows the effect of LSD1 inhibition in comparison to other compounds believed to affect T cell phenotype on T_{SCM} to T_{EM} ratio in CD8+ cells.
Figure 5 shows the expansion of T cells using CD3/CD28 stimulation in the presence of LSD1 inhibitors.
Figure 6 shows the expression of checkpoint proteins PD1, Tim3 and Lag3 on T cells expanded in the presence of LSD1 inhibitors.
Figure 7 shows the level of CAR expression on T cells expanded in the presence of LSD1 inhibitors.
Figure 8 shows the proportion of CD4+ and CD8+ CART and untransduced T cells in the presence of LSD1 inhibitors.
Figure 9 shows the expansion of CART cells and untransduced T cells in the presence of LSD1 inhibitors.
Figure 10 shows the cytokine production from CART cells expanded in the presence of LSD1 inhibitors, and then exposed to CD19+ or CD19- tumor cells.

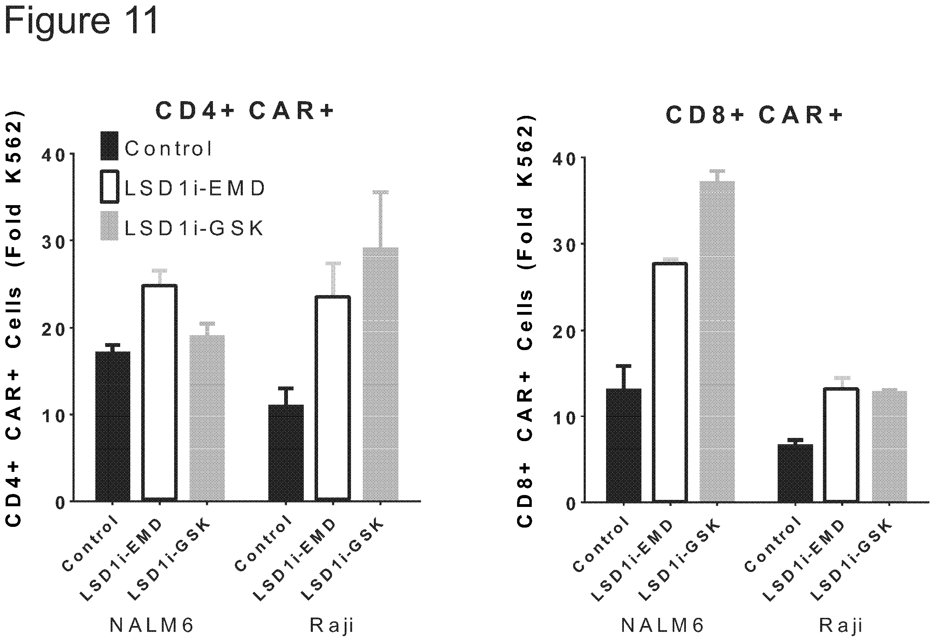

Figure 11 shows the effect of LSD1 inhibition in significantly increased proliferative capacity following CAR stimulation. Naive human T cells (peripheral pan CD3+ T cells, pooled population) were isolated from PBMCs by negative selection and expanded with anti-CD3/CD28 beads at a 3:1 ratio for 10 days in the presence of the indicated compounds. T cells were transduced with an anti-CD19 scFV on day 1. Compounds were refreshed every 2 days until day 10 was washed out prior to functional assays. Following expansion, T cells were mixed with CD19+ tumor cells lines NALM6 and Raji, as well as CD19-tumor cell line K562. Tumor cells were irradiated and T cells and tumor cells were mixed at a 1:1 ratio. On day 4 following incubation, T cells were stained for CAR using Protein L and CAR+ T cell numbers were determined by FACS using countbright beads. Proliferation was measured as the number of FACS positive cells detected in the period of time used to count 2500 beads. Data expressed as fold no target (CD19-) control (K562).

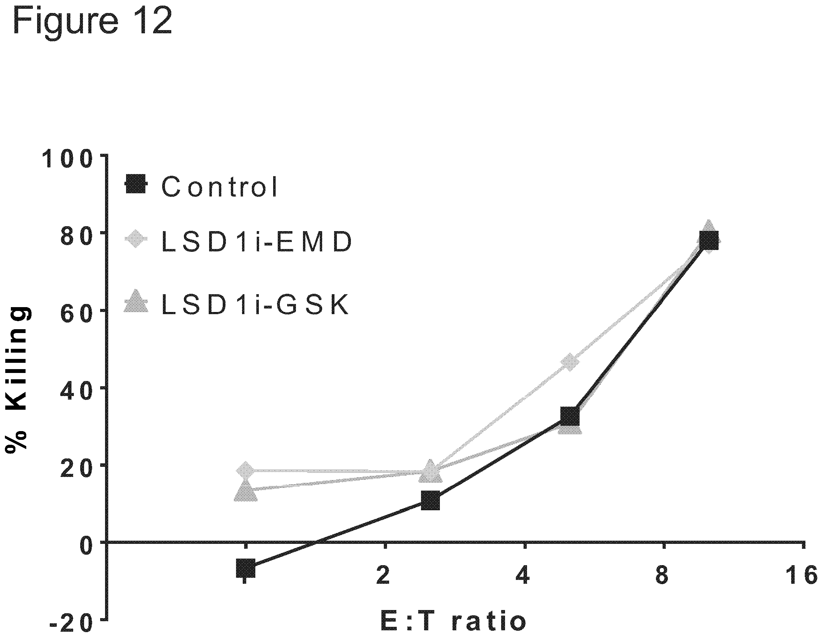

Figure 12 shows the effects of the indicated compounds on naive human T cells (peripheral pan CD3+ T cells, pooled population) isolated by negative selection from PBMCs and expanded with anti-CD3/CD28 beads at a 3:1 ratio for 10 days in the presence of the indicated compounds. T cells were transduced with an anti-CD19 scFV on day 1. Compounds were refreshed every 2 days until day 10 was washed out prior to functional assays. Following expansion, T cell killing of the luciferized CD19+ NALM6 tumor cell line was assessed. After 20 hours luciferase signal was measured using the Bright-Glo^{™} Luciferase Assay on the EnVision instrument.
Figure 13 shows the in vivo anti-tumor efficacy of CART cells expanded ex vivo in the presence of LSD1 inhibitors.
Figure 14A shows the level of expansion of CD4+ T cells (e.g., T_{SCM}) cells in the presence of LSD1 inhibitors.
Figure 14B shows the level of expansion of CD8+ T cells (e.g., T_{SCM}) cells in the presence of LSD1 inhibitors.
Figure 15A shows the level of expression of checkpoint proteins PD1, Tim3 and Lag3 on CD4+ T cells expanded in the presence of LSD1 inhibitors.
Figure 15B shows the level of expression of checkpoint proteins PD1, Tim3 and Lag3 on CD8+ T cells expanded in the presence of LSD1 inhibitors.
Figure 16 depicts the percentage of total T cells expressing PD1, Tim3 or Lag3 (left panel) or co-expressing PD1/Lag3 or PD1/Lag3/Tim3 after expansion in the presence or absence of LSD1 inhibitor.
Figure 17 depicts the percentage of CD4+ T cells (left panel) and percentage of CD8+ T cells (right panel) which are Tscm after expansion in the presence or absence of LSD1 inhibitor.
Figure 18 depicts the percentage of CD4+ T cells (left panel) and percentage of CD8+ T cells (right panel) which are positive for co-expression of Tim3/Lag3/PD-1 after expansion in the presence or absence of LSD1 inhibitor.
Figure 19 depicts an ilustration of gating strategy for Tscm, Tcm and Tem by flow cytometry analysis.
Figure 20 depicts the effects of different concentrations of LSD1 inhibitors on the T cell phenotypic changes after 10 day activation, treatment and expansion in culture. Percentage of Tscm, Tem and Tcm of CD3+ T cells, as well as the ratio of subset CD8+/CD4+, Tscm/Tem, and Tscm/Tcm are shown.
Figure 21 depicts a dose response curve of the compound 93 (NVS Compound 1) on the subset of CD8+ and CD4+ T cells on induction of Tscm, Tem and Tcm.
Figure 22 depicts a dose response curve of the LSD1i-GSK on the subset of CD8+ and CD4+ T cells on induction of Tscm, Tem and Tcm.
Figure 23 depicts a dose response curve of the compound 93 and LSD1i-GSK on the CD3+ and CD8+ T cells on induction of Tscm (EC50 shown).
Figure 24 shows that Compound A and Compound B showed similar effects on inducing Tscm and reducing Tem at 100nM started dosing 24h after activation, when compared with Compound 93.
Figure 25 depicts an ilustration of gating strategy for Tscm, Tcm and Tem in total T cell and CAR+ T cells by flow cytometry analysis.
Figure 26 shows FACS analyses for total CD3+ T cells, CAR expression, and CD8+ CAR+ vs CD4+ CAR+ ratio in the final CART product in response to different concentrations of LSD1 inhibition by Compound 93.
Figure 27 shows FACS analyses for Tscm, Tem, Tcm in the total CD8+ T cells of the final CART products in response to different concentrations of LSD1 inhibition by Compound 93.
Figure 28 shows FACS analyses for the percentage of Tscm, Tem, Tcm in the CAR+CD8+ T cells in response to different concentrations of LSD1 inhibition by Compound 93.
Figure 29 shows results from in vitro cytokine assay for IFNg secretion by CART cells with or without LSD1 inhibitor treatment in response to their specific tumor target cells line at an effector to target cells ratio 1.25:1 incubation for 20 hours.
Figure 30 shows in vitro CD3+ (total) and CD3+CAR+ cell proliferation levels in response to their specific irradiated tumor cells lines at an effector to target cells ratio 1:1 for 4 days.
Figure 31 shows in vitro CD8+ and CD8+CAR+ T cell proliferation levels in response to their specific irradiated tumor cells lines at an effector to target cells ratio 1:1 for 4 days.
Figure 32 shows in vitro CD4+ and CD4+CAR+ T cell proliferation levels in response to their specific irradiated tumor cells lines at an effector to target cells ratio 1:1 for 4 days.
Figure 33 shows in vivo anti-tumor efficacy of BCMA CART cells against BCMA+ tumor line. UTD = T cells that were not transduced with the CAR gene; LSDi = indicates those populations that were expanded ex vivo in the presence of Compound 93; BCMA 0.167 million = indicates those populations which were transduced with the CAR gene, and the number of CAR+ cells in the population; PBS = no cell control (phosphate buffered saline injection only).

### FURTHER DETAILED DESCRIPTION

### Definitions

Unless defined otherwise, all technical and scientific terms used herein have the same meaning as commonly understood by one of ordinary skill in the art to which the invention pertains.

The term "a" and "an" refers to one or to more than one (i.e., to at least one) of the grammatical object of the article. By way of example, "an element" means one element or more than one element.

The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

The term "Chimeric Antigen Receptor" or alternatively a "CAR" refers to a set of polypeptides, typically two in the simplest embodiments, which when in an immune effector cell, provides the cell with specificity for a target cell, typically a cancer cell, and with intracellular signal generation. In some embodiments, a CAR comprises at least an extracellular antigen binding domain, a transmembrane domain and a cytoplasmic signaling domain (also referred to herein as "an intracellular signaling domain") comprising a functional signaling domain derived from a stimulatory molecule and/or costimulatory molecule as defined below. In some aspects, the set of polypeptides are contiguous with each other. In some embodiments, the set of polypeptides includes a dimerization switch that, upon the presence of a dimerization molecule, can couple the polypeptides to one another, e.g., can couple an antigen binding domain to an intracellular signaling domain. In one aspect, the stimulatory molecule is the zeta chain associated with the T cell receptor complex. In one aspect, the cytoplasmic signaling domain further comprises one or more functional signaling domains derived from at least one costimulatory molecule as defined below. In one aspect, the costimulatory molecule is chosen from the costimulatory molecules described herein, e.g., 4-1BB (i.e., CD137), CD27 and/or CD28. In one aspect, the CAR comprises a chimeric fusion protein comprising an extracellular antigen binding domain, a transmembrane domain and an intracellular signaling domain comprising a functional signaling domain derived from a stimulatory molecule. In one aspect, the CAR comprises a chimeric fusion protein comprising an extracellular antigen binding domain, a transmembrane domain and an intracellular signaling domain comprising a functional signaling domain derived from a costimulatory molecule and a functional signaling domain derived from a stimulatory molecule. In one aspect, the CAR comprises a chimeric fusion protein comprising an extracellular antigen binding domain, a transmembrane domain and an intracellular signaling domain comprising two functional signaling domains derived from one or more costimulatory molecule(s) and a functional signaling domain derived from a stimulatory molecule. In one aspect, the CAR comprises a chimeric fusion protein comprising an extracellular antigen binding domain, a transmembrane domain and an intracellular signaling domain comprising at least two functional signaling domains derived from one or more costimulatory molecule(s) and a functional signaling domain derived from a stimulatory molecule. In one aspect the CAR comprises an optional leader sequence at the amino-terminus (N-ter) of the CAR fusion protein. In one aspect, the CAR further comprises a leader sequence at the N-terminus of the extracellular antigen binding domain, wherein the leader sequence is optionally cleaved from the antigen binding domain (e.g., a scFv) during cellular processing and localization of the CAR to the cellular membrane.

A CAR that comprises an antigen binding domain (e.g., a scFv, or TCR) that targets a specific tumor marker X, such as those described herein, is also referred to as XCAR. For example, a CAR that comprises an antigen binding domain that targets CD19 is referred to as CD19CAR.

The term "signaling domain" refers to the functional portion of a protein which acts by transmitting information within the cell to regulate cellular activity via defined signaling pathways by generating second messengers or functioning as effectors by responding to such messengers.

The term "antibody," as used herein, refers to a protein, or polypeptide sequence derived from an immunoglobulin molecule which specifically binds with an antigen. Antibodies can be polyclonal or monoclonal, multiple or single chain, or intact immunoglobulins, and may be derived from natural sources or from recombinant sources. Antibodies can be tetramers of immunoglobulin molecules.

The term "antibody fragment" refers to at least one portion of an antibody, that retains the ability to specifically interact with (e.g., by binding, steric hinderance, stabilizing/destabilizing, spatial distribution) an epitope of an antigen. Examples of antibody fragments include, but are not limited to, Fab, Fab', F(ab')₂, Fv fragments, scFv antibody fragments, disulfide-linked Fvs (sdFv), a Fd fragment consisting of the VH and CH1 domains, linear antibodies, single domain antibodies such as sdAb (either VL or VH), camelid VHH domains, multi-specific antibodies formed from antibody fragments such as a bivalent fragment comprising two Fab fragments linked by a disulfide bridge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can also be grafted into scaffolds based on polypeptides such as a fibronectin type III (Fn3) (see U.S. Patent No.: 6,703,199, which describes fibronectin polypeptide minibodies).

The term "scFv" refers to a fusion protein comprising at least one antibody fragment comprising a variable region of a light chain and at least one antibody fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

The portion of the CAR used in the invention comprising an antibody or antibody fragment thereof may exist in a variety of forms where the antigen binding domain is expressed as part of a contiguous polypeptide chain including, for example, a single domain antibody fragment (sdAb), a single chain antibody (scFv), a humanized antibody or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition used in the invention comprises an antibody fragment. In a further aspect, the CAR comprises an antibody fragment that comprises a scFv. The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof.

As used herein, the term "binding domain" or "antibody molecule" refers to a protein, e.g., an immunoglobulin chain or fragment thereof, comprising at least one immunoglobulin variable domain sequence. The term "binding domain" or "antibody molecule" encompasses antibodies and antibody fragments. In an embodiment, an antibody molecule is a multispecific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

The term "antibody heavy chain," refers to the larger of the two types of polypeptide chains present in antibody molecules in their naturally occurring conformations, and which normally determines the class to which the antibody belongs.

The term "antibody light chain," refers to the smaller of the two types of polypeptide chains present in antibody molecules in their naturally occurring conformations. Kappa (κ) and lambda (λ) light chains refer to the two major antibody light chain isotypes.

The term "recombinant antibody" refers to an antibody which is generated using recombinant DNA technology, such as, for example, an antibody expressed by a bacteriophage or yeast expression system. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using recombinant DNA or amino acid sequence technology which is available and well known in the art.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response, therefore encodes an "antigen" as that term is used herein. Furthermore, one skilled in the art will understand that an antigen need not be encoded solely by a full length nucleotide sequence of a gene. It is readily apparent that the present disclosure includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be synthesized or can be derived from a biological sample, or might be a macromolecule besides a polypeptide. Such a biological sample can include, but is not limited to a tissue sample, a tumor sample, a cell or a fluid with other biological components.

The term "anti-cancer effect" refers to a biological effect which can be manifested by various means, including but not limited to, e.g., a decrease in tumor volume, a decrease in the number of cancer cells, a decrease in the number of metastases, an increase in life expectancy, decrease in cancer cell proliferation, decrease in cancer cell survival, or amelioration of various physiological symptoms associated with the cancerous condition. An "anti-cancer effect" can also be manifested by the ability of the peptides, polynucleotides, cells and antibodies in prevention of the occurrence of cancer in the first place. The term "anti-tumor effect" refers to a biological effect which can be manifested by various means, including but not limited to, e.g., a decrease in tumor volume, a decrease in the number of tumor cells, a decrease in tumor cell proliferation, an increase in tumor cell death, an increase in tumor cell apoptosis, or a decrease in tumor cell survival.

The term "autologous" refers to any material derived from the same individual to whom it is later to be re-introduced into the individual.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically.

The term "xenogeneic" refers to a graft derived from an animal of a different species.

The term "cancer" refers to a disease characterized by the uncontrolled growth of aberrant cells. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

"Derived from" as that term is used herein, indicates a relationship between a first and a second molecule. It generally refers to structural similarity between the first molecule and a second molecule and does not connotate or include a process or source limitation on a first molecule that is derived from a second molecule. For example, in the case of an intracellular signaling domain that is derived from a CD3zeta molecule, the intracellular signaling domain retains sufficient CD3zeta structure such that it has the required function, namely, the ability to generate a signal under the appropriate conditions. It does not connotate or include a limitation to a particular process of producing the intracellular signaling domain, e.g., it does not mean that, to provide the intracellular signaling domain, one must start with a CD3zeta sequence and delete unwanted sequence, or impose mutations, to arrive at the intracellular signaling domain.

The phrase "disease associated with expression of a tumor antigen as described herein" includes, but is not limited to, a disease associated with expression of a tumor antigen as described herein or condition associated with cells which express a tumor antigen as described herein including, e.g., proliferative diseases such as a cancer or malignancy or a precancerous condition such as a myelodysplasia, a myelodysplastic syndrome or a preleukemia; or a noncancer related indication associated with cells which express a tumor antigen as described herein. In one aspect, a cancer associated with expression of a tumor antigen as described herein is a hematological cancer. In one aspect, a cancer associated with expression of a tumor antigen as described herein is a solid cancer. Further diseases associated with expression of a tumor antigen described herein include, but not limited to, e.g., atypical and/or non-classical cancers, malignancies, precancerous conditions or proliferative diseases associated with expression of a tumor antigen as described herein. Non-cancer related indications associated with expression of a tumor antigen as described herein include, but are not limited to, e.g., autoimmune disease, (e.g., lupus), inflammatory disorders (allergy and asthma) and transplantation. In some embodiments, the tumor antigen-expressing cells express, or at any time expressed, mRNA encoding the tumor antigen. In an instance, the tumor antigen -expressing cells produce the tumor antigen protein (e.g., wild-type or mutant), and the tumor antigen protein may be present at normal levels or reduced levels. In an instance, the tumor antigen -expressing cells produced detectable levels of a tumor antigen protein at one point, and subsequently produced substantially no detectable tumor antigen protein.

The term "conservative sequence modifications" refers to amino acid modifications that do not significantly affect or alter the binding characteristics of the antibody or antibody fragment containing the amino acid sequence. Such conservative modifications include amino acid substitutions, additions and deletions. Modifications can be introduced into an antibody or antibody fragment of the disclosure by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within a CAR of the invention can be replaced with other amino acid residues from the same side chain family and the altered CAR can be tested using the functional assays described herein.

The term "stimulation," refers to a primary response induced by binding of a stimulatory molecule (e.g., a TCR/CD3 complex or CAR) with its cognate ligand (or tumor antigen in the case of a CAR) thereby mediating a signal transduction event, such as, but not limited to, signal transduction via the TCR/CD3 complex or signal transduction via the appropriate NK receptor or signaling domains of the CAR. Stimulation can mediate altered expression of certain molecules.

The term "stimulatory molecule," refers to a molecule expressed by an immune cell (e.g., T cell, NK cell, B cell) that provides the cytoplasmic signaling sequence(s) that regulate activation of the immune cell in a stimulatory way for at least some aspect of the immune cell signaling pathway. In one aspect, the-signal is a primary signal that is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyrosine-based activation motif or ITAM. Examples of an ITAM containing-cytoplasmic signaling sequence that is of particular use in the invention include, but are not limited to, those derived from CD3 zeta, common FcR gamma (FCER1G), Fc gamma Rlla, FcR beta (Fc Epsilon R1b), CD3 gamma, CD3 delta, CD3 epsilon, CD79a, CD79b, DAP10, and DAP12. In a specific CAR used in the invention, the intracellular signaling domain in any one or more CARS of the invention comprises an intracellular signaling sequence, e.g., a primary signaling sequence of CD3-zeta. In a specific CAR used in the invention, the primary signaling sequence of CD3-zeta is the sequence provided as SEQ ID NO: 18, or the equivalent residues from a non-human species, e.g., mouse, rodent, monkey, ape and the like. In a specific CAR used in the invention, the primary signaling sequence of CD3-zeta is the sequence as provided in SEQ ID NO:20, or the equivalent residues from a non-human species, e.g., mouse, rodent, monkey, ape and the like.

The term "antigen presenting cell" or "APC" refers to an immune system cell such as an accessory cell (e.g., a B-cell, a dendritic cell, and the like) that displays a foreign antigen complexed with major histocompatibility complexes (MHC's) on its surface. T-cells may recognize these complexes using their T-cell receptors (TCRs). APCs process antigens and present them to T-cells.

An "intracellular signaling domain," as the term is used herein, refers to an intracellular portion of a molecule. The intracellular signaling domain generates a signal that promotes an immune effector function of the CAR containing cell, e.g., a CART cell. Examples of immune effector function, e.g., in a CART cell, include cytolytic activity and helper activity, including the secretion of cytokines.

In an embodiment, the intracellular signaling domain can comprise a primary intracellular signaling domain. Exemplary primary intracellular signaling domains include those derived from the molecules responsible for primary stimulation, or antigen dependent simulation. In an embodiment, the intracellular signaling domain can comprise a costimulatory intracellular domain. Exemplary costimulatory intracellular signaling domains include those derived from molecules responsible for costimulatory signals, or antigen independent stimulation. For example, in the case of a CART, a primary intracellular signaling domain can comprise a cytoplasmic sequence of a T cell receptor, and a costimulatory intracellular signaling domain can comprise cytoplasmic sequence from co-receptor or costimulatory molecule.

A primary intracellular signaling domain can comprise a signaling motif which is known as an immunoreceptor tyrosine-based activation motif or ITAM. Examples of ITAM containing primary cytoplasmic signaling sequences include, but are not limited to, those derived from CD3 zeta, common FcR gamma (FCER1G), Fc gamma Rlla, FcR beta (Fc Epsilon R1b), CD3 gamma, CD3 delta, CD3 epsilon, CD79a, CD79b, DAP10, and DAP12.

The term "zeta" or alternatively "zeta chain", "CD3-zeta" (or "CD3zeta , CD3 zeta or CD3z) or "TCR-zeta" is defined as the protein provided as GenBan Acc. No. BAG36664.1, or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain, or functional derivatives thereof, that are sufficient to functionally transmit an initial signal necessary for T cell activation. In one aspect the cytoplasmic domain of zeta comprises residues 52 through 164 of GenBank Acc. No. BAG36664.1 or the equivalent residues from a non-human species, e.g., mouse, rodent, monkey, ape and the like, that are functional orthologs thereof. In one aspect, the "zeta stimulatory domain" or a "CD3-zeta stimulatory domain" is the sequence provided as SEQ ID NO:18. In one aspect, the "zeta stimulatory domain" or a "CD3-zeta stimulatory domain" is the sequence provided as SEQ ID NO:20.

The term a "costimulatory molecule" refers to a cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are contribute to an efficient immune response. Costimulatory molecules include, but are not limited to an MHC class I molecule, BTLA and a Toll ligand receptor, as well as OX40, CD27, CD28, CDS, ICAM-1, LFA-1 (CD11a/CD18), ICOS (CD278), and 4-1BB (CD137). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83.

A costimulatory intracellular signaling domain can be the intracellular portion of a costimulatory molecule. A costimulatory molecule can be represented in the following protein families: TNF receptor proteins, Immunoglobulin-like proteins, cytokine receptors, integrins, signaling lymphocytic activation molecules (SLAM proteins), and activating NK cell receptors. Examples of such molecules include CD27, CD28, 4-1BB (CD137), OX40, GITR, CD30, CD40, ICOS, BAFFR, HVEM, ICAM-1, lymphocyte function-associated antigen-1 (LFA-1), CD2, CDS, CD7, CD287, LIGHT, NKG2C, NKG2D, SLAMF7, NKp80, NKp30, NKp44, NKp46, CD160, B7-H3, and a ligand that specifically binds with CD83, and the like.

The intracellular signaling domain can comprise the entire intracellular portion, or the entire native intracellular signaling domain, of the molecule from which it is derived, or a functional fragment or derivative thereof.

The term "4-1BB" refers to a member of the TNFR superfamily with an amino acid sequence provided as GenBank Acc. No. AAA62478.2, or the equivalent residues from a non-human species, e.g., mouse, rodent, monkey, ape and the like; and a "4-1BB costimulatory domain" is defined as amino acid residues 214-255 of GenBank Acc. No. AAA62478.2, or the equivalent residues from a non-human species, e.g., mouse, rodent, monkey, ape and the like. In one aspect, the "4-1BB costimulatory domain" is the sequence provided as SEQ ID NO:14 or the equivalent residues from a non-human species, e.g., mouse, rodent, monkey, ape and the like.

"Immune effector cell," as that term is used herein, refers to a cell that is involved in an immune response, e.g., in the promotion of an immune effector response. Examples of immune effector cells include T cells, e.g., alpha/beta T cells and gamma/delta T cells, B cells, natural killer (NK) cells, natural killer T (NKT) cells, mast cells, and myeloic-derived phagocytes.

"Immune effector function or immune effector response," as that term is used herein, refers to function or response, e.g., of an immune effector cell, that enhances or promotes an immune attack of a target cell. E.g., an immune effector function or response refers a property of a T or NK cell that promotes killing or inhibition of growth or proliferation, of a target cell. In the case of a T cell, primary stimulation and co-stimulation are examples of immune effector function or response.

The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "effective amount" or "therapeutically effective amount" are used interchangeably herein, and refer to an amount of a compound, formulation, material, or composition, as described herein effective to achieve a particular biological result.

The term "endogenous" refers to any material from or produced inside an organism, cell, tissue or system.

The term "exogenous" refers to any material introduced from or produced outside an organism, cell, tissue or system.

The term "expression" refers to the transcription and/or translation of a particular nucleotide sequence driven by a promoter.

The term "transfer vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "transfer vector" includes an autonomously replicating plasmid or a virus. The term should also be construed to further include non-plasmid and non-viral compounds which facilitate transfer of nucleic acid into cells, such as, for example, a polylysine compound, liposome, and the like. Examples of viral transfer vectors include, but are not limited to, adenoviral vectors, adeno-associated virus vectors, retroviral vectors, lentiviral vectors, and the like.

The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.

The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses.

The term "lentiviral vector" refers to a vector derived from at least a portion of a lentivirus genome, including especially a self-inactivating lentiviral vector as provided in Milone et al., Mol. Ther. 17(8): 1453-1464 (2009). Other examples of lentivirus vectors that may be used in the clinic, include but are not limited to, e.g., the LENTIVECTOR^{®} gene delivery technology from Oxford BioMedica, the LENTIMAX^{™} vector system from Lentigen and the like. Nonclinical types of lentiviral vectors are also available and would be known to one skilled in the art.

The term "homologous" or "identity" refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer ten subunits in length) of the positions in two sequences are homologous, the two sequences are 50% homologous; if 90% of the positions (e.g., 9 of 10), are matched or homologous, the two sequences are 90% homologous.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. For the most part, humanized antibodies and antibody fragments thereof are human immunoglobulins (recipient antibody or antibody fragment) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, a humanized antibody/antibody fragment can comprise residues which are found neither in the recipient antibody nor in the imported CDR or framework sequences. These modifications can further refine and optimize antibody or antibody fragment performance. In general, the humanized antibody or antibody fragment thereof will comprise substantially all of at least one, and typically two, variable domains, in which all or substantially all of the CDR regions correspond to those of a non-human immunoglobulin and all or a significant portion of the FR regions are those of a human immunoglobulin sequence. The humanized antibody or antibody fragment can also comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

"Fully human" refers to an immunoglobulin, such as an antibody or antibody fragment, where the whole molecule is of human origin or consists of an amino acid sequence identical to a human form of the antibody or immunoglobulin.

The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

The term "operably linked" or "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.

The term "parenteral" administration of an immunogenic composition includes, e.g., subcutaneous (s.c.), intravenous (i.v.), intramuscular (i.m.), or intrasternal injection, intratumoral, or infusion techniques.

The term "nucleic acid" or "polynucleotide" refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.

The term "promoter" refers to a DNA sequence recognized by the synthetic machinery of the cell, or introduced synthetic machinery, required to initiate the specific transcription of a polynucleotide sequence.

The term "promoter/regulatory sequence" refers to a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

The term "constitutive" promoter refers to a nucleotide sequence which, when operably linked with a polynucleotide which encodes or specifies a gene product, causes the gene product to be produced in a cell under most or all physiological conditions of the cell.

The term "inducible" promoter refers to a nucleotide sequence which, when operably linked with a polynucleotide which encodes or specifies a gene product, causes the gene product to be produced in a cell substantially only when an inducer which corresponds to the promoter is present in the cell.

The term "tissue-specific" promoter refers to a nucleotide sequence which, when operably linked with a polynucleotide encodes or specified by a gene, causes the gene product to be produced in a cell substantially only if the cell is a cell of the tissue type corresponding to the promoter.

The terms "cancer associated antigen" or "tumor antigen" interchangeably refers to a molecule (typically a protein, carbohydrate or lipid) that is expressed on the surface of a cancer cell, either entirely or as a fragment (e.g., MHC/peptide), and which is useful for the preferential targeting of a pharmacological agent to the cancer cell. In some embodiments, a tumor antigen is a marker expressed by both normal cells and cancer cells, e.g., a lineage marker, e.g., CD19 on B cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some embodiments, a tumor antigen is a cell surface molecule that is inappropriately synthesized in the cancer cell, for instance, a molecule that contains deletions, additions or mutations in comparison to the molecule expressed on a normal cell. In some embodiments, a tumor antigen will be expressed exclusively on the cell surface of a cancer cell, entirely or as a fragment (e.g., MHC/peptide), and not synthesized or expressed on the surface of a normal cell. In some embodiments, the CARs used in the present invention includes CARs comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5):1935-1942; Sergeeva et al., Blood, 2011 117(16):4262-4272; Verma et al., J Immunol 2010 184(4):2156-2165; Willemsen et al., Gene Ther 2001 8(21) :1601-1608; Dao et al., Sci Transl Med 2013 5(176) :176ra33; Tassev et al., Cancer Gene Ther 2012 19(2):84-100). For example, TCR-like antibody can be identified from screening a library, such as a human scFv phage displayed library.

The term "tumor-supporting antigen" or "cancer-supporting antigen" interchangeably refer to a molecule (typically a protein, carbohydrate or lipid) that is expressed on the surface of a cell that is, itself, not cancerous, but supports the cancer cells, e.g., by promoting their growth or survival e.g., resistance to immune cells. Exemplary cells of this type include stromal cells and myeloid-derived suppressor cells (MDSCs). The tumor-supporting antigen itself need not play a role in supporting the tumor cells so long as the antigen is present on a cell that supports cancer cells.

The term "flexible polypeptide linker" or "linker" as used in the context of a scFv refers to a peptide linker that consists of amino acids such as glycine and/or serine residues used alone or in combination, to link variable heavy and variable light chain regions together. In one embodiment, the flexible polypeptide linker is a Gly/Ser linker and comprises the amino acid sequence (Gly-Gly-Gly-Ser)n, where n is a positive integer equal to or greater than 1. For example, n=1, n=2, n=3. n=4, n=5 and n=6, n=7, n=8, n=9 and n=10 (SEQ ID NO:28). In one embodiment, the flexible polypeptide linkers include, but are not limited to, (Gly4 Ser)4 (SEQ ID NO:29) or (Gly4 Ser)3 (SEQ ID NO:30). In another embodiment, the linkers include multiple repeats of (Gly2Ser), (GlySer) or (Gly3Ser) (SEQ ID NO:31). Also included within the scope of the disclosure are linkers described in WO2012/138475.

As used herein, a 5' cap (also termed an RNA cap, an RNA 7-methylguanosine cap or an RNA m⁷G cap) is a modified guanine nucleotide that has been added to the "front" or 5' end of a eukaryotic messenger RNA shortly after the start of transcription. The 5' cap consists of a terminal group which is linked to the first transcribed nucleotide. Its presence is critical for recognition by the ribosome and protection from RNases. Cap addition is coupled to transcription, and occurs co-transcriptionally, such that each influences the other. Shortly after the start of transcription, the 5' end of the mRNA being synthesized is bound by a cap-synthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.

As used herein, "in vitro transcribed RNA" refers to RNA, preferably mRNA, that has been synthesized in vitro. Generally, the in vitro transcribed RNA is generated from an in vitro transcription vector. The in vitro transcription vector comprises a template that is used to generate the in vitro transcribed RNA.

As used herein, a "poly(A)" is a series of adenosines attached by polyadenylation to the mRNA. In a preferred embodiment of a construct for transient expression, the polyA is between 50 and 5000 (SEQ ID NO: 34), preferably greater than 64, more preferably greater than 100, most preferably greater than 300 or 400 poly(A) sequences can be modified chemically or enzymatically to modulate mRNA functionality such as localization, stability or efficiency of translation.

As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly(A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

As used herein in connection with expression, e.g., expression of a CAR molecule, "transient" refers to expression of a non-integrated transgene for a period of hours, days or weeks, wherein the period of time of expression is less than the period of time for expression of the gene if integrated into the genome or contained within a stable plasmid replicon in the host cell.

As used herein in connection with an effect, e.g., an effect of an LSD1 inhibitor, "transient" means the effect is present for a period of, for example, hours, days, weeks or months, but diminishes (e.g., until the effect is no longer measurable) over a period of time. In embodiments the effect is as measured according to the assays described herein, e.g., in the examples.

As used herein, the terms "treat", "treatment" and "treating" refer to the reduction or amelioration of the progression, severity and/or duration of a proliferative disorder, or the amelioration of one or more symptoms (preferably, one or more discernible symptoms) of a proliferative disorder resulting from the administration of one or more therapies (e.g., one or more therapeutic agents such as a CAR used in the invention). In specific embodiments, the terms "treat", "treatment" and "treating" refer to the amelioration of at least one measurable physical parameter of a proliferative disorder, such as growth of a tumor, not necessarily discernible by the patient. In other embodiments the terms "treat", "treatment" and "treating" -refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

The term "signal transduction pathway" refers to the biochemical relationship between a variety of signal transduction molecules that play a role in the transmission of a signal from one portion of a cell to another portion of a cell. The phrase "cell surface receptor" includes molecules and complexes of molecules capable of receiving a signal and transmitting signal across the membrane of a cell.

The term "subject" is intended to include living organisms in which an immune response can be elicited (e.g., mammals, human).

The term, a "substantially purified" cell refers to a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some aspects, the cells are cultured in vitro. In other aspects, the cells are not cultured in vitro.

The term "therapeutic" as used herein means a treatment. A therapeutic effect is obtained by reduction, suppression, remission, or eradication of a disease state.

The term "prophylaxis" as used herein means the prevention of or protective treatment for a disease or disease state.

The term "transfected" or "transformed" or "transduced" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The term "specifically binds," refers to an antibody, or a ligand, which recognizes and binds with a binding partner (e.g., a tumor antigen) protein present in a sample, but which antibody or ligand does not substantially recognize or bind other molecules in the sample.

"Regulatable chimeric antigen receptor (RCAR),"as that term is used herein, refers to a set of polypeptides, typically two in the simplest embodiments, which when in a RCARX cell, provides the RCARX cell with specificity for a target cell, typically a cancer cell, and with regulatable intracellular signal generation or proliferation, which can optimize an immune effector property of the RCARX cell. An RCARX cell relies at least in part, on an antigen binding domain to provide specificity to a target cell that comprises the antigen bound by the antigen binding domain. In an embodiment, an RCAR includes a dimerization switch that, upon the presence of a dimerization molecule, can couple an intracellular signaling domain to the antigen binding domain.

"Membrane anchor" or "membrane tethering domain", as that term is used herein, refers to a polypeptide or moiety, e.g., a myristoyl group, sufficient to anchor an extracellular or intracellular domain to the plasma membrane.

"Switch domain," as that term is used herein, e.g., when referring to an RCAR, refers to an entity, typically a polypeptide-based entity, that, in the presence of a dimerization molecule, associates with another switch domain. The association results in a functional coupling of a first entity linked to, e.g., fused to, a first switch domain, and a second entity linked to, e.g., fused to, a second switch domain. A first and second switch domain are collectively referred to as a dimerization switch. In embodiments, the first and second switch domains are the same as one another, e.g., they are polypeptides having the same primary amino acid sequence, and are referred to collectively as a homodimerization switch. In embodiments, the first and second switch domains are different from one another, e.g., they are polypeptides having different primary amino acid sequences, and are referred to collectively as a heterodimerization switch. In embodiments, the switch is intracellular. In embodiments, the switch is extracellular. In embodiments, the switch domain is a polypeptide-based entity, e.g., FKBP or FRB-based, and the dimerization molecule is small molecule, e.g., a rapalogue. In embodiments, the switch domain is a polypeptide-based entity, e.g., an scFv that binds a myc peptide, and the dimerization molecule is a polypeptide, a fragment thereof, or a multimer of a polypeptide, e.g., a myc ligand or multimers of a myc ligand that bind to one or more myc scFvs. In embodiments, the switch domain is a polypeptide-based entity, e.g., myc receptor, and the dimerization molecule is an antibody or fragments thereof, e.g., myc antibody.

"Dimerization molecule," as that term is used herein, e.g., when referring to an RCAR, refers to a molecule that promotes the association of a first switch domain with a second switch domain. In embodiments, the dimerization molecule does not naturally occur in the subject, or does not occur in concentrations that would result in significant dimerization. In embodiments, the dimerization molecule is a small molecule, e.g., rapamycin or a rapalogue, e.g, RAD001.

The term "bioequivalent" refers to an amount of an agent other than the reference compound, required to produce an effect equivalent to the effect produced by the reference dose or reference amount of the reference compound. In an embodiment the effect is the level of LSD1 inhibition, e.g., as measured by LSD1 protein levels, e.g., as evaluated in an in vivo or in vitro assay, e.g., as measured by an assay described herein, e.g., flow cytometry. In an embodiment, the effect is enhanced proliferation of T_{SCM} cells, e.g., CD45RA+CD62L+ cells, e.g., e.g., enhanced proliferation relative to other T cell phenotypes, e.g., T_{CM} (e.g., CD45RA-CD62L+), T_{EM} (e.g., CD45RA-CD62L-), T_{EFF} or T_{REG} cells, e.g., as measured by cell sorting. In an embodiment, the effect is enhanced proliferation of T_{SCM} cells, e.g., CD45RA+CCR7+ cells, e.g., e.g., enhanced proliferation relative to other T cell phenotypes, e.g., CD45RA-CCR7+, CD45RA-CCR7-, CD45RA+CCR7-, T_{EFF} or T_{REG} cells, e.g., as measured by cell sorting.

"Refractory" as used herein refers to a disease, e.g., cancer, that does not respond to a treatment. In embodiments, a refractory cancer can be resistant to a treatment before or at the beginning of the treatment. In other embodiments, the refractory cancer can become resistant during a treatment. A refractory cancer is also called a resistant cancer.

"Relapsed" as used herein refers to the return of a disease (e.g., cancer) or the signs and symptoms of a disease such as cancer after a period of improvement, e.g., after prior treatment of a therapy, e.g., cancer therapy

"LSD1," "lysine-specific demethylase 1A," "Lysine-specific histone demethylase 1A," "KDM1A," "AOF2," "KIAA0601" and "BHC110" are used interchangeably herein, and refer to the gene KDM1A (lysine-specific demethylase 1A) and the protein encoded by said gene, lysine-specific demethylase 1A (LSD1). This gene encodes a nuclear protein containing a SWIRM domain, a FAD-binding motif, and an amine oxidase domain. This protein is a component of several histone deacetylase complexes, through it silences genes by functioning as a histone demethylase. In the human genome, KDM1A is located on chromosome 1 Chr1:23030596 (on Assembly GRCh38). Currently two isoforms of LSD1 are known, and the isoforms are described in GeneBank number NM_001009999.2 and NM_015013.3.

Examples of the protein sequence of human LSD1 is provided as UniProt accession number 060341-1 having an amino acid sequence as follows:

10 20 30 40 50
MLSGKKAAAA AAAAAAAATG TEAGPGTAGG SENGSEVAAQ PAGLSGPAEV
60 70 80 90 100
GPGAVGERTP RKKEPPRASP PGGLAEPPGS AGPQAGPTVV PGSATPMETG
110 120 130 140 150
IAETPEGRRT SRRKRAKVEY REMDESLANL SEDEYYSEEE RNAKAEKEKK
160 170 180 190 200
LPPPPPQAPP EEENESEPEE PSGVEGAAFQ SRLPHDRMTS QEAACFPDII
210 220 230 240 250
SGPQQTQKVF LFIRNRTLQL WLDNPKIQLT FEATLQQLEA PYNSDTVLVH
260 270 280 290 300
RVHSYLERHG LINFGIYKRI KPLPTKKTGK VIIIGSGVSG LAAARQLQSF
310 320 330 340 350
GMDVTLLEAR DRVGGRVATF RKGNYVADLG AMVVTGLGGN PMAVVSKQVN
360 370 380 390 400
MELAKIKQKC PLYEANGQAV PKEKDEMVEQ EFNRLLEATS YLSHQLDFNV
410 420 430 440 450
LNNKPVSLGQ ALEVVIQLQE KHVKDEQIEH WKKIVKTQEE LKELLNKMVN
460 470 480 490 500
LKEKIKELHQ QYKEASEVKP PRDITAEFLV KSKHRDLTAL CKEYDELAET
510 520 530 540 550
QGKLEEKLQE LEANPPSDVY LSSRDRQILD WHFANLEFAN ATPLSTLSLK
560 570 580 590 600
HWDQDDDFEF TGSHLTVRNG YSCVPVALAE GLDIKLNTAV RQVRYTASGC
610 620 630 640 650
EVIAVNTRST SQTFIYKCDA VLCTLPLGVL KQQPPAVQFV PPLPEWKTSA
660 670 680 690 700
VQRMGFGNLN KVVLCFDRVF WDPSVNLFGH VGSTTASRGE LFLFWNLYKA

710 720 730 740 750
PILLALVAGE AAGIMENISD DVIVGRCLAI LKGIFGSSAV PQPKETVVSR
760 770 780 790 800
WRADPWARGS YSYVAAGSSG NDYDLMAQPI TPGPSIPGAP QPIPRLFFAG
810 820 830 840 850
EHTIRNYPAT VHGALLSGLR EAGRIADQFL GAMYTLPRQA TPGVPAQQSP
852
SM
SEQ ID NO: 40.

The disclosure also includes other isoforms of LSD1, including Isoform 2, provided as UniProt accession number 060341-2.

Examples of nucleic acid sequences encoding LSD1 are provided below. There are 2 identified isoforms of human LSD1. The mRNA sequences are provided below (In embodiments, in each sequence, T may be replaced with U). In embodiments, LSD1 includes the proteins encoded by each of the sequences below:

| **Gene ID** | **Variant** | **Sequence** |
|---|---|---|
| KDM1A/LSD1/AOF 2; Gene ID: 23028 | Isoform A; NCBI Reference Sequence: NM_001009999.2 | GGCGCGGCGGGAGCGCGCTTGGCGCGTGCGTACGCG ACGGCGGTTGGCGGCGCGCGGGCAGCGTGAAGCG AGGCGAGGCAAGGCTTTTCGGACCCACGGAGCGACA GAGCGAGCGGCCCCTACGGCCGTCGGCGGCCCGG CGGCCCGAGATGTTATCTGGGAAGAAGGCGGCAGCC GCGGCGGCGGCGGCTGCAGCGGCAGCAACCGGGA CGGAGGCTGGCCCTGGGACAGCAGGCGGCTCCGAGA ACGGGTCTGAGGTGGCCGCGCAGCCCGCGGGCCT GTCGGGCCCAGCCGAGGTCGGGCCGGGGGCGGTGG GGGAGCGCACACCCCGCAAGAAAGAGCCTCCGCGG GCCTCGCCCCCCGGGGGCCTGGCGGAACCGCCGGGG TCCGCAGGGCCTCAGGCCGGCCCTACTGTCGTGC CTGGGTCTGCGACCCCCATGGAAACTGGAATAGCAGA GACTCCGGAGGGGCGTCGGACCAGCCGGCGCAA GCGGGCGAAGGTAGAGTACAGAGAGATGGATGAAA GCTTGGCCAACCTCTCAGAAGATGAGTATTATTCA GAAGAAGAGAGAAATGCCAAAGCAGAGAAGGAAAA GAAGCTTCCCCCACCACCCCCTCAAGCCCCACCTG AGGAAGAAAATGAAAGTGAGCCTGAAGAACCATCGG |
| SEQ ID NO: 41 | | |
| | | GGCAAGCAGGAGGACTTCAAGACGACAGTTCTGG AGGGTATGGAGACGGCCAAGCATCAGGTGTGGAGG GCGCAGCTTTCCAGAGCCGACTTCCTCATGACCGG ATGACTTCTCAAGAAGCAGCCTGTTTTCCAGATATTA TCAGTGGACCACAACAGACCCAGAAGGTTTTTC TTTTCATTAGAAACCGCACACTGCAGTTGTGGTTGGA TAATCCAAAGATTCAGCTGACATTTGAGGCTAC TCTCCAACAATTAGAAGCACCTTATAACAGTGATACT GTGCTTGTCCACCGAGTTCACAGTTATTTAGAG CGTCATGGTCTTATCAACTTCGGCATCTATAAGAGGA TAAAACCCCTACCAACTAAAAAGACAGGAAAGG TAATTATTATAGGCTCTGGGGTCTCAGGCTTGGCAG CAGCTCGACAGTTACAAAGTTTTGGAATGGATGT CACACTTTTGGAAGCCAGGGATCGTGTGGGTGGAC GAGTTGCCACATTTCGCAAAGGAAACTATGTAGCT GATCTTGGAGCCATGGTGGTAACAGGTCTTGGAGG GAATCCTATGGCTGTGGTCAGCAAACAAGTAAATA TGGAACTGGCCAAGATCAAGCAAAAATGCCCACTTT ATGAAGCCAACGGACAAGCTGACACTGTCAAGGT TCCTAAAGAGAAAGATGAAATGGTAGAGCAAGAGT TTAACCGGTTGCTAGAAGCTACATCTTACCTTAGT CATCAACTAGACTTCAATGTCCTCAATAATAAGCCTG TGTCCCTTGGCCAGGCATTGGAAGTTGTCATTC AGTTACAAGAGAAGCATGTCAAAGATGAGCAGATT GAACATTGGAAGAAGATAGTGAAAACTCAGGAAGA ATTGAAAGAACTTCTTAATAAGATGGTAAATTTGAA AGAGAAAATTAAAGAACTCCATCAGCAATACAAA GAAGCATCTGAAGTAAAGCCACCCAGAGATATTACT GCCGAGTTCTTAGTGAAAAGCAAACACAGGGATC TGACCGCCCTATGCAAGGAATATGATGAATTAGCTG AAACACAAGGAAAGCTAGAAGAAAAACTTCAGGA GTTGGAAGCGAATCCCCCAAGTGATGTATATCTCTC ATCAAGAGACAGACAAATACTTGATTGGCATTTT GCAAATCTTGAATTTGCTAATGCCACACCTCTCTCAA CTCTCTCCCTTAAGCACTGGGATCAGGATGATG ACTTTGAGTTCACTGGCAGCCACCTGACAGTAAGGA ATGGCTACTCGTGTGTGCCTGTGGCTTTAGCAGA AGGCCTAGACATTAAACTGAATACAGCAGTGCGACA GGTTCGCTACACGGCTTCAGGATGTGAAGTGATA GCTGTGAATACCCGCTCCACGAGTCAAACCTTTATTT ATAAATGCGACGCAGTTCTCTGTACCCTTCCCC |
| | | TGGGTGTGCTGAAGCAGCAGCCACCAGCCGTTCAGTTT GTGCCACCTCTCCCTGAGTGGAAAACATCTGC AGTCCAAAGGATGGGATTTGGCAACCTTAACAAGGTG GTGTTGTGTTTTGATCGGGTGTTCTGGGATCCA AGTGTCAATTTGTTCGGGCATGTTGGCAGTACGACTGC CAGCAGGGGTGAGCTCTTCCTCTTCTGGAACC TCTATAAAGCTCCAATACTGTTGGCACTAGTGGCAGGA GAAGCTGCTGGTATCATGGAAAACATAAGTGA CGATGTGATTGTTGGCCGATGCCTGGCCATTCTCAAAG GGATTTTTGGTAGCAGTGCAGTACCTCAGCCC AAAGAAACTGTGGTGTCTCGTTGGCGTGCTGATCCCTG GGCTCGGGGCTCTTATTCCTATGTTGCTGCAG GATCATCTGGAAATGACTATGATTTAATGGCTCAGCCA ATCACTCCTGGCCCCTCGATTCCAGGTGCCCC ACAGCCGATTCCACGACTCTTCTTTGCGGGAGAACATA CGATCCGTAACTACCCAGCCACAGTGCATGGT GCTCTGCTGAGTGGGCTGCGAGAAGCGGGAAGAATTG CAGACCAGTTTTTGGGGGCCATGTATACGCTGC CTCGCCAGGCCACACCAGGTGTTCCTGCACAGCAGTCC CCAAGCATGTGAGACAGATGCATTCTAAGGGA AGAGGCCCATGTGCCTGTTTCTGCCATGTAAGGAAGGC TCTTCTAGCAATACTAGATCCCACTGAGAAAA TCCACCCTGGCATCTGGGCTCCTGATCAGCTGATGGAG CTCCTGATTTGACAAAGGAGCTTGCCTCCTTT GAATGACCTAGAGCACAGGGAGGAACTTGTCCATTAGT TTGGAATTGTGTTCTTCGTAAAGACTGAGGCA AGCAAGTGCTGTGAAATAACATCATCTTAGTCCCTTGGT GTGTGGGGTTTTTGTTTTTTTTTTATATTTT GAGAATAAAACTTCATATAAAATTGGCAAAAAAAAAAA AAAAAAA |
| KDM1A/LSD1/AOF 2; Gene ID: 23028 | Isoform B; NCBI Reference Sequence: NM_015013.3 | GGCGCGGCGGGAGCGCGCTTGGCGCGTGCGTACGCGA CGGCGGTTGGCGGCGCGCGGGCAGCGTGAAGCG AGGCGAGGCAAGGCTTTTCGGACCCACGGAGCGACAG AGCGAGCGGCCCCTACGGCCGTCGGCGGCCCGG CGGCCCGAGATGTTATCTGGGAAGAAGGCGGCAGCCG CGGCGGCGGCGGCTGCAGCGGCAGCAACCGGGA CGGAGGCTGGCCCTGGGACAGCAGGCGGCTCCGAGAA CGGGTCTGAGGTGGCCGCGCAGCCCGCGGGCCT GTCGGGCCCAGCCGAGGTCGGGCCGGGGGCGGTGGG GGAGCGCACACCCCGCAAGAAAGAGCCTCCGCGG GCCTCGCCCCCCGGGGGCCTGGCGGAACCGCCGGGG |
| SEQ ID NO: 42 | | |
| | | TCCGCAGGGCCTCAGGCCGGCCCTACTGTCGTGC CTGGGTCTGCGACCCCCATGGAAACTGGAATAGCAG AGACTCCGGAGGGGCGTCGGACCAGCCGGCGCAA GCGGGCGAAGGTAGAGTACAGAGAGATGGATGAAA GCTTGGCCAACCTCTCAGAAGATGAGTATTATTCA GAAGAAGAGAGAAATGCCAAAGCAGAGAAGGAAAA GAAGCTTCCCCCACCACCCCCTCAAGCCCCACCTG AGGAAGAAAATGAAAGTGAGCCTGAAGAACCATCG GGTGTGGAGGGCGCAGCTTTCCAGAGCCGACTTCC TCATGACCGGATGACTTCTCAAGAAGCAGCCTGTTTT CCAGATATTATCAGTGGACCACAACAGACCCAG AAGGTTTTTCTTTTCATTAGAAACCGCACACTGCAGTT GTGGTTGGATAATCCAAAGATTCAGCTGACAT TTGAGGCTACTCTCCAACAATTAGAAGCACCTTATAA CAGTGATACTGTGCTTGTCCACCGAGTTCACAG TTATTTAGAGCGTCATGGTCTTATCAACTTCGGCATCT ATAAGAGGATAAAACCCCTACCAACTAAAAAG ACAGGAAAGGTAATTATTATAGGCTCTGGGGTCTCA GGCTTGGCAGCAGCTCGACAGTTACAAAGTTTTG GAATGGATGTCACACTTTTGGAAGCCAGGGATCGTG TGGGTGGACGAGTTGCCACATTTCGCAAAGGAAA CTATGTAGCTGATCTTGGAGCCATGGTGGTAACAGGT CTTGGAGGGAATCCTATGGCTGTGGTCAGCAAA CAAGTAAATATGGAACTGGCCAAGATCAAGCAAAAA TGCCCACTTTATGAAGCCAACGGACAAGCTGTTC CTAAAGAGAAAGATGAAATGGTAGAGCAAGAGTTTA ACCGGTTGCTAGAAGCTACATCTTACCTTAGTCA TCAACTAGACTTCAATGTCCTCAATAATAAGCCTGTGT CCCTTGGCCAGGCATTGGAAGTTGTCATTCAG TTACAAGAGAAGCATGTCAAAGATGAGCAGATTGAA CATTGGAAGAAGATAGTGAAAACTCAGGAAGAAT TGAAAGAACTTCTTAATAAGATGGTAAATTTGAAAGA GAAAATTAAAGAACTCCATCAGCAATACAAAGA AGCATCTGAAGTAAAGCCACCCAGAGATATTACTGCC GAGTTCTTAGTGAAAAGCAAACACAGGGATCTG ACCGCCCTATGCAAGGAATATGATGAATTAGCTGAA ACACAAGGAAAGCTAGAAGAAAAACTTCAGGAGT TGGAAGCGAATCCCCCAAGTGATGTATATCTCTCATC AAGAGACAGACAAATACTTGATTGGCATTTTGC AAATCTTGAATTTGCTAATGCCACACCTCTCTCAACTC TCTCCCTTAAGCACTGGGATCAGGATGATGAC |
| | | TTTGAGTTCACTGGCAGCCACCTGACAGTAAGGAA TGGCTACTCGTGTGTGCCTGTGGCTTTAGCAGAAG GCCTAGACATTAAACTGAATACAGCAGTGCGACAG GTTCGCTACACGGCTTCAGGATGTGAAGTGATAGC TGTGAATACCCGCTCCACGAGTCAAACCTTTATTTA TAAATGCGACGCAGTTCTCTGTACCCTTCCCCTG GGTGTGCTGAAGCAGCAGCCACCAGCCGTTCAGTT TGTGCCACCTCTCCCTGAGTGGAAAACATCTGCAG TCCAAAGGATGGGATTTGGCAACCTTAACAAGGTG GTGTTGTGTTTTGATCGGGTGTTCTGGGATCCAAG TGTCAATTTGTTCGGGCATGTTGGCAGTACGACTGC CAGCAGGGGTGAGCTCTTCCTCTTCTGGAACCTC TATAAAGCTCCAATACTGTTGGCACTAGTGGCAGG AGAAGCTGCTGGTATCATGGAAAACATAAGTGACG ATGTGATTGTTGGCCGATGCCTGGCCATTCTCAAAG GGATTTTTGGTAGCAGTGCAGTACCTCAGCCCAA AGAAACTGTGGTGTCTCGTTGGCGTGCTGATCCCTG GGCTCGGGGCTCTTATTCCTATGTTGCTGCAGGA TCATCTGGAAATGACTATGATTTAATGGCTCAGCCA ATCACTCCTGGCCCCTCGATTCCAGGTGCCCCAC AGCCGATTCCACGACTCTTCTTTGCGGGAGAACATA CGATCCGTAACTACCCAGCCACAGTGCATGGTGC TCTGCTGAGTGGGCTGCGAGAAGCGGGAAGAATT GCAGACCAGTTTTTGGGGGCCATGTATACGCTGCCT CGCCAGGCCACACCAGGTGTTCCTGCACAGCAGTC CCCAAGCATGTGAGACAGATGCATTCTAAGGGAAG AGGCCCATGTGCCTGTTTCTGCCATGTAAGGAAGG CTCTTCTAGCAATACTAGATCCCACTGAGAAAATC CACCCTGGCATCTGGGCTCCTGATCAGCTGATGGA GCTCCTGATTTGACAAAGGAGCTTGCCTCCTTTGA ATGACCTAGAGCACAGGGAGGAACTTGTCCATTAG TTTGGAATTGTGTTCTTCGTAAAGACTGAGGCAAG CAAGTGCTGTGAAATAACATCATCTTAGTCCCTTGG TGTGTGGGGTTTTTGTTTTTTTTTTATATTTTGA GAATAAAACTTCATATAAAATTGGCAAAAAAAAAA AAAAAAAA |

"LSD1 inhibitor" as the term is used herein, refers to a molecule, or a group of molecules (e.g., a system) that reduces or eliminates the function and/or expression of LSD1. In embodiments, an LSD1 inhibitor is a molecule that inhibits the expression of LSD1 e.g., reduces or eliminates expression of LSD1. In embodiments, the LSD1 inhibitor is a molecule that inhibits the function of LSD1. An example of an LSD1 inhibitor that inhibits the expression of LSD1 is a gene editing system, e.g., as described herein, that is targeted to nucleic acid within the LSD1 gene (e.g., within the KDM1A gene), or its regulatory elements, such that modification of the nucleic acid at or near the gene editing system binding site(s) is modified to reduce or eliminate expression of LSD1. Another example of an LSD1 inhibitor that inhibits the expression of LSD1 is a nucleic acid molecule, e.g., RNA molecule, e.g., a short hairpin RNA (shRNA) or short interfering RNA (siRNA), capable of hybridizing with LSD1 mRNA and causing a reduction or elimination of LSD1 translation. Another example of an LSD1 inhibitor that inhibits the expression of LSD1 is an antisense oligonucleotide. LSD1 inhibitors also include nucleic acids encoding molecules which inhibit LSD1 expression (e.g., nucleic acid encoding an anti-LSD1 shRNA or siRNA, or nucleic acid encoding one or more, e.g., all, components of an anti-LSD1 gene editing system). An example of a molecule that inhibits the function of LSD1 is a molecule, e.g., a protein or small molecule which inhibits one or more activities of LSD1. An example is a small molecule inhibitor of LSD1, e.g., as described herein. In an exemplary embodiment, a small molecule LSD1 inhibitor is a reversible LSD1 inhibitor. In another exemplary embodiment, a small molecule LSD1 inhibitor is an irreversible LSD1 inhibitor. A small molecule LSD1 inhibitor may bind LSD1 at the catalytic site or at a site other than the catalytic site. Another example is a dominant negative LSD1 protein. Another example is an anti-LSD1 antibody or antigen-binding fragment thereof. Another example is a molecule, e.g., a small molecule, which inhibits an LSD1 binding partner. LSD1 inhibitors also include nucleic acids encoding inhibitors of LSD1 function. Further description of LSD1 inhibitors is provided below in the section titled "LSD1 inhibitors."

A "binding partner" as the term is used herein in the context of an LSD1 binding partner, refers to a molecule, e.g., a protein, which interacts, e.g., binds to, LSD1 protein. Without being bound by theory, it is believed that LSD1 binds to one or more HDAC proteins, e.g., HDAC1. Such HDAC proteins are considered examples of LSD1 binding partners. Other LSD1 binding partners include, for example, proteins of the Co-REST/REST complex, e.g., HDAC1, HDAC2, p40, p80, Co-REST and ZNF217 (Lee, M. G., Wynder, C., Cooch, N. & Shiekhattar, R. An essential role for CoREST in nucleosomal histone 3 lysine 4 demethylation. Nature 437, 432-435 (2005)); proteins of the Blimp1 complex (Mol Cell Biol. 2009 Mar;29(6):1421-31. doi: 10.1128/MCB.01158-08. Epub 2009 Jan 5); proteins of the NuRD complex (Cell. 2009 Aug. 21;138(4):660-72. doi: 10.1016/j.cell.2009.05.050); and the androgen receptor (Nature. 2005 Sep 15;437(7057):436-9. Epub 2005 Aug 3).

A "system" as the term is used herein in connection with, for example, gene editing, refers to a group of molecules, e.g., one or more molecules, which together act to produce a desired function.

A "gene editing system" as the term is used herein, refers to a system, e.g., one or more molecules, that direct and effect an alteration, e.g., a deletion, of one or more nucleic acids at or near a site of genomic DNA targeted by said system. Gene editing systems are known in the art, and are described more fully below.

A "dominant negative" gene product or protein is one that interferes with the function of a gene product or protein. The gene product affected can be the same or different from the dominant negative protein. Dominant negative gene products can be of many forms, including truncations, full length proteins with point mutations or fragments thereof, or fusions of full length wild type or mutant proteins or fragments thereof with other proteins. The level of inhibition observed can be very low. For example, it may require a large excess of the dominant negative protein compared to the functional protein or proteins involved in a process in order to see an effect. It may be difficult to see effects under normal biological assay conditions. In one embodiment, a dominant negative LSD1 is a catalytically inactive LSD1.

The term "proportion" refers to the ratio of the specified molecule to the total number of molecules in a population. In an exemplary embodiment, a proportion of T cells having a specific phenotype (e.g., T_{SCM} cells) refers to the ratio of the number of T cells having that phenotype relative to the total number of T cells in a population. In an exemplary embodiment, a proportion of T cells having a specific phenotype (e.g., CD45RA+CD62L+ cells) refers to the ratio of the number of T cells having that phenotype relative to the total number of T cells in a population. It will be understood that such proportions may be measured against certain subsets of cells, where indicted. For example, the proportion of CD4+ T_{SCM} cells may be measured against the total number of CD4+ T cells.

The term "population of immune effector cells" as used herein refers to a composition comprising at least two, e.g., two or more, e.g., more than one, immune effector cell, and does not denote any level of purity or the presence or absence of other cell types. In an exemplary embodiment, the population is substantially free of other cell types. In another exemplary embodiment, the population comprises at least two cells of the specified cell type, or having the specified function or property.

The terms "T_{SCM}-like cell," "naive T Cell' and "naive T cell" are used interchangeably and refer to a less differentiated T cell state, that is characterized by surface expression of CD45RA and CD62L (e.g., is CD45RA positive and CD62L positive (sometimes written as CD45RA+CD62L+)). In general, T cell differentiation proceeds, from most "naive" to most "exhausted," T_{SCMm}-like (e.g., a CD45RA+CD62L+ cell) >T_{CM} (e.g., a CD45RA-CD62L+ cell)>T_{EM} (e.g., a CD45RA-CD62L- cell)>T_{EFF}. Naive T cells may be characterized, for example, as having increased self-renewal, anti-tumor efficacy, proliferation and/or survival, relative to a more exhausted T cell phenotype. In an exemplary embodiment, a naive T cell refers to a CD45RA+CD62L+ T cell. In another exemplary embodiment, a naive T cell refers to a T_{SCM} cell, e.g., a CD45RA+CD62L+CCR7+CD27+CD95+ T cell.

The term "T_{SCM}" refers to a T cell having a stem cell memory phenotype, characterized in that it expresses CD45RA, CD62L, CCR7, CD27 and CD95 on its cell surface (e.g., is CD45RA positive, CD62L positive, CCR7 positive, CD27 positive and CD95 positive (sometimes written as CD45RA+CD62L+CCR7+CD27+CD95+)). A T_{SCM} cell is an example of a naive T cell. The T cell may be CD4+ and/or CD8+ T cell.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes subranges such as 96-99%, 96-98%, 96-97%, 97-99%, 97-98% and 98-99% identity. This applies regardless of the breadth of the range.

Headings, sub-headings or numbered or lettered elements, e.g., (a), (b), (i) etc, are presented merely for ease of reading. The use of headings or numbered or lettered elements in this document does not require the steps or elements be performed in alphabetical order or that the steps or elements are necessarily discrete from one another.

Other features, objects, and advantages of the invention will be apparent from the description and drawings, and from the claims.

### LSD1 Inhibitors

Any molecule that inhibits LSD1 may be useful in the aspects of the invention described herein, e.g., in connection with the cells, compositions and methods disclosed herein. The following sections provide exemplary LSD1 inhibitors, and are not intended to be limiting.

In embodiments, the LSD1 inhibitor is a molecule or system that results in increased or prolonged proliferation or persistence of CAR-expressing cells with a naive phenotype (e.g., T_{SCM} cells), e.g., in culture or in a subject, e.g., as compared to non-treated CAR-expressing cells or a non-treated subject. In embodiments, increased proliferation or persistence is associated with in an increase in the number of CAR-expressing cells. Methods for measuring increased or prolonged proliferation are described in Example 4. In another embodiment, administration or contacting with an LSD1 inhibitor results in increased cytokine release or increased killing of cancer cells by CAR-expressing cells, e.g., in culture or in a subject, e.g., as compared to non-treated CAR-expressing cells or a non-treated subject. Methods for measuring increased cytokine release are described in, e.g., Example 4. In embodiments, increased killing of cancer cells is associated with in a decrease in tumor volume. Methods for measuring increased killing of cancer cells are described in Example 4 and, e.g., in International Application WO2014/153270.

### Nucleic acid inhibitors

In one aspect the LSD1 inhibitor is a nucleic acid molecule. In embodiments, the nucleic acid is a DNA molecule, e.g., an antisense oligonucleotide (e.g., Watts et al., J. Pathol., 2012, 226(2), pp. 365-379). In an embodiment, the antisense oligonucleotide is complementary to an LSD1 mRNA or pre-mRNA molecule. In another aspect, the LSD1 inhibitor includes nucleic acid encoding said antisense oligonucleotide.

In embodiments, the nucleic acid LSD1 inhibitor is an interfering RNA molecule, e.g., a shRNA or siRNA, that inhibits expression, e.g., translation, of LSD1. In another aspect, the LSD1 inhibitor includes nucleic acid encoding said interfering RNA molecule. In embodiments, the interfering RNA molecule, e.g., a shRNA or siRNA, that inhibits expression, e.g., translation, of LSD1 comprises a domain complementary to a sequence of an LSD1 mRNA (such sequence referred to herein in relation to an interfering RNA molecule, e.g., a shRNA or siRNA, as a "target sequence"). Examples of such target sequences are provided in Table 1.

Exemplary Target Sequences for shRNA and siRNA LSD1 inhibitors, and exemplary nucleic acids encoding shRNA LSD1 inhibitors are provided in Table 1 below,

**Table 1**

| **TARGET** | **SHRNA_ NAME** | **Target Sequence** | **SEQ ID NO:** | **Sequence encoding anti-LSD1 shRNA** | **SEQ ID NO:** |
|---|---|---|---|---|---|
| AOF2 | KDM1A-917_582 63 | CCGAGTTCACAGTTATTT AGA | 43 | CCGAGTTCACAGTTATTTAGACTCGAGTCTA AATAACTGTGAACTCGGTTTTTTGAATTCGCA CCAGCACGCTACGCAACACGTTGACGTTGAC CACATGTTCGCCGTCTTC | 83 |
| AOF2 | KDM1A-1265_58 264 | CGGACAAGCTGTTCCTAA AGA | 44 | CGGACAAGCTGTTCCTAAAGACTCGAGTCTT TAGGAACAGCTTGTCCGTTTTTTGAATTCGCA CCAGCACGCTACGCAGTCAGTTGACGTTGAC CAACGTTTCGCCGTCTTC | 84 |
| AOF2 | KDM1A-2928_58 265 | GAATTGTGTTCTTCGTAA AGA | 45 | GAATTGTGTTCTTCGTAAAGACTCGAGTCTTT ACGAAGAACACAATTCTTTTTTGAATTCGCAC CAGCACGCTACGCATGACGTTGACGTTGACC AACCATTCGCCGTCTTC | 85 |
| AOF2 | KDM1A-914_582 66 | CCACCGAGTTCACAGTTA TTT | 46 | CCACCGAGTTCACAGTTATTTCTCGAGAAAT AACTGTGAACTCGGTGGTTTTTTGAATTCGC ACCAGCACGCTACGCAACGTGTTGACGTTGA CCAACACTTCGCCGTCTTC | 86 |
| AOF2 | KDM1A-1000_58 267 | CAGGAAAGGTAATTATT ATAG | 47 | CAGGAAAGGTAATTATTATAGCTCGAGCTAT AATAATTACCTTTCCTGTTTTTTGAATTCGCA CCAGCACGCTACGCACATGGTTGACGTTGAC CAACTGTTCGCCGTCTTC | 87 |
| AOF2 | KDM1A-2759_58 268 | GCCTGTTTCTGCCATGTA AGG | 48 | GCCTGTTTCTGCCATGTAAGGCTCGAGCCTT ACATGGCAGAAACAGGCTTTTTTGAATTCGC ACCAGCACGCTACGCATGACGTTGACGTTGA CCATGGTTTCGCCGTCTTC | 88 |
| AOF2 | KDM1A-998_582 69 | GACAGGAAAGGTAATTA TTAT | 49 | GACAGGAAAGGTAATTATTATCTCGAGATAA TAATTACCTTTCCTGTCTTTTTTGAATTCGCAC CAGCACGCTACGCAACTGGTTGACGTTGACC ATGCATTCGCCGTCTTC | 89 |
| AOF2 | KDM1A-1271_58 270 | AGCTGTTCCTAAAGAGA | 50 | AGCTGTTCCTAAAGAGAAAGACTCGAGTCTT TCTCTTTAGGAACAGCTTTTTTTGAATTCGCA | 90 |
| | | AAGA | | CCAGCACGCTACGCACACAGTTGACGTTGAC CATGACTTCGCCGTCTTC | |
| AOF2 | KDM1A-1996_58 271 | CGAGTCAAACCTTTATTT ATA | 51 | CGAGTCAAACCTTTATTTATACTCGAGTATAA ATAAAGGTTTGACTCGTTTTTTGAATTCGCAC CAGCACGCTACGCAGTGTGTTGACGTTGACC ATGTGTTCGCCGTCTTC | 91 |
| AOF2 | KDM1A-863_582 72 | GGCTACTCTCCAACAATT AGA | 52 | GGCTACTCTCCAACAATTAGACTCGAGTCTA ATTGTTGGAGAGTAGCCTTTTTTGAATTCGC ACCAGCACGCTACGCAGTACGTTGACGTTGA CACGTGTTTCGCCGTCTTC | 92 |
| AOF2 | KDM1A-1044_58 273 | GCAGCTCGACAGTTACA AAGT | 53 | GCAGCTCGACAGTTACAAAGTCTCGAGACTT TGTAACTGTCGAGCTGCTTTTTTGAATTCGCA CCAGCACGCTACGCATGTGGTTGACGTTGAC ACGTCATTCGCCGTCTTC | 93 |
| AOF2 | KDM1A-1903_58 274 | CAGAAGGCCTAGACATT AAAC | 54 | CAGAAGGCCTAGACATTAAACCTCGAGGTTT AATGTCTAGGCCTTCTGTTTTTTGAATTCGCA CCAGCACGCTACGCAACCAGTTGACGTTGAC ACGTACTTCGCCGTCTTC | 94 |
| AOF2 | KDM1A-563_582 75 | AGATGAGTATTATTCAGA AGA | 55 | AGATGAGTATTATTCAGAAGACTCGAGTCTT CTGAATAATACTCATCTTTTTTTGAATTCGCA CCAGCACGCTACGCACAGTGTTGACGTTGAC ACGTTGTTCGCCGTCTTC | 95 |
| AOF2 | KDM1A-876_582 76 | CAATTAGAAGCACCTTAT AAC | 56 | CAATTAGAAGCACCTTATAACCTCGAGGTTA TAAGGTGCTTCTAATTGTTTTTTGAATTCGCA CCAGCACGCTACGCATGTGGTTGACGTTGAC ACCAGTTTCGCCGTCTTC | 96 |
| AOF2 | KDM1A-2908_58 277 | GGAACTTGTCCATTAGTT TGG | 57 | GGAACTTGTCCATTAGTTTGGCTCGAGCCAA ACTAATGGACAAGTTCCTTTTTTGAATTCGCA CCAGCACGCTACGCAACGTGTTGACGTTGAC ACCACATTCGCCGTCTTC | 97 |
| AOF2 | KDM1A-885_582 78 | GCACCTTATAACAGTGAT ACT | 58 | GCACCTTATAACAGTGATACTCTCGAGAGTA TCACTGTTATAAGGTGCTTTTTTGAATTCGCA CCAGCACGCTACGCACAACGTTGACGTTGAC ACCAACTTCGCCGTCTTC | 98 |
| AOF2 | KDM1A-960_582 79 | GGCATCTATAAGAGGAT AAAA | 59 | GGCATCTATAAGAGGATAAAACTCGAGTTTT ATCCTCTTATAGATGCCTTTTTTGAATTCGCA CCAGCACGCTACGCAGTCAGTTGACGTTGAC ACCATGTTCGCCGTCTTC | 99 |
| AOF2 | KDM1A-1399_58 280 | CATTGGAAGTTGTCATTC | 60 | CATTGGAAGTTGTCATTCAGTCTCGAGACTG AATGACAACTTCCAATGTTTTTTGAATTCGCA | 100 |
| | | AGT | | CCAGCACGCTACGCAACGTGTTGACGTTGAC ACACGTTTCGCCGTCTTC | |
| AOF2 | KDM1A-2474_58 281 | TGGAAATGACTATGATTT AAT | 61 | TGGAAATGACTATGATTTAATCTCGAGATTA AATCATAGTCATTTCCATTTTTTGAATTCGCA CCAGCACGCTACGCACACAGTTGACGTTGAC ACACCATTCGCCGTCTTC | 101 |
| AOF2 | KDM1A_ NM_015 013.3_21 174 | CGGTTGCTAGAAGCTAC ATCT | 62 | GAAGACGCACCGGCGGTTGCTAGAAGTTAC ATTTGTTAATATTCATAGCAGATGTAGCTTCT AGCAACCGTTTTTTGAATTCGCACCAGCACG CTACGCAACGTGTCACAGTACACTGCAACTT CGCCGT | 102 |
| AOF2 | KDM1A_ NM_015 013.3_21 173 | GCCACATTTCGCAAAGG AAAC | 63 | GAAGACGCACCGGGCCACATTTCGTAAAGG AAATGTTAATATTCATAGCGTTTCCTTTGCGA AATGTGGCTTTTTTGAATTCGCACCAGCACG CTACGCATGACGTCACAGTACACTGCACATT CGCCGT | 103 |
| AOF2 | KDM1A_ NM_015 013.3_21 172 | CCTCATGACCGGATGACT TCT | 64 | GAAGACGCACCGGCCTCATGACTGGATGATT TCTGTTAATATTCATAGCAGAAGTCATCCGGT CATGAGGTTTTTTGAATTCGCACCAGCACGC TACGCAGTCAGTCACAGTACACTGCAGTTTC GCCGT | 104 |
| AOF2 | KDM1A_ NM_015 013.3_21 171 | CGAGTTGCCACATTTCGC AAA | 65 | GAAGACGCACCGGCGAGTTGCCATATTTCGT AAAGTTAATATTCATAGCTTTGCGAAATGTG GCAACTCGTTTTTTGAATTCGCACCAGCACGC TACGCAACACGTCACAGTACACTGGTTGTTC GCCGT | 105 |
| AOF2 | KDM1A_ NM_015 013.3_21 170 | CGTCATGGTCTTATCAAC TTC | 66 | GAAGACGCACCGGCGTCATGGTCTTATCAAT TTCGTTAATATTCATAGCGAAGTTGATAAGA CCATGACGTTTTTTGAATTCGCACCAGCACGC TACGCATGTGGTCACAGTACACTGGTACTTC GCCGT | 106 |
| AOF2 | KDM1A_ NM_015 013.3_21 169 | GAGCGTCATGGTCTTATC AAC | 67 | GAAGACGCACCGGGAGCGTTATGGTCTTATT AACGTTAATATTCATAGCGTTGATAAGACCA TGACGCTCTTTTTTGAATTCGCACCAGCACGC TACGCAGTCAGTCACAGTACACTGGTCATTC GCCGT | 107 |
| AOF2 | KDM1A_ NM_015 013.3_21 168 | CCGGATGACTTCTCAAGA AGC | 68 | GAAGACGCACCGGCCGGATGACTTCTTAAG AAGTGTTAATATTCATAGCGCTTCTTGAGAA GTCATCCGGTTTTTTGAATTCGCACCAGCAC GCTACGCACAGTGTCACAGTACACTGGTGTT TCGCCGT | 108 |
| AOF2 | KDM1A_ NM_015 013.3_21 167 | CACAGGGAGGAACTTGT CCAT | 69 | GAAGACGCACCGGCACAGGGAGGAATTTGT CTATGTTAATATTCATAGCATGGACAAGTTCC TCCCTGTGTTTTTTGAATTCGCACCAGCACGC TACGCAGTACGTCACAGTACACACTGTGTTC GCCGT | 109 |
| AOF2 | KDM1A_ NM_015 013.3_21 166 | GCCTCCTTTGAATGACCT AGA | 70 | GAAGACGCACCGGGCCTTCTTTGAATGACTT AGAGTTAATATTCATAGCTCTAGGTCATTCAA AGGAGGCTTTTTTGAATTCGCACCAGCACGC TACGCACATGGTCACAGTACACACTGACTTC GCCGT | 110 |
| AOF2 | KDM1A_ NM_015 013.3_21 165 | CCTATGGCTGTGGTCAGC AAA | 71 | GAAGACGCACCGGCCTATGGTTGTGGTCAG TAAAGTTAATATTCATAGCTTTGCTGACCACA GCCATAGGTTTTTTGAATTCGCACCAGCACG CTACGCAACCAGTCACAGTACACACTGCATT CGCCGT | 111 |
| AOF2 | KDM1A_ NM_015 013.3_21 164 | GAGCTTGCCTCCTTTGAA TGA | 72 | GAAGACGCACCGGGAGCTTGTGTTCTTTGAA TGAGTTAATATTCATAGCTCATTCAAAGGAG GCAAGCTCTTTTTTGAATTCGCACCAGCACGC TACGCATGGTGTCACAGTACACACTGGTTTC GCCGT | 112 |
| AOF2 | KDM1A_ NM_015 013.3_21 163 | CAGGTCTTGGAGGGAAT CCTA | 73 | GAAGACGCACCGGCAGGTCTTGGAGGGGAT CTTAGTTAATATTCATAGCTAGGATTCCCTCC AAGACCTGTTTTTTGAATTCGCACCAGCACG CTACGCACACAGTCACAGTACACACACTGTT CGCCGT | 113 |
| AOF2 | KDM1A_ NM_015 013.3_21 162 | CAGTACCTCAGCCCAAAG AAA | 74 | GAAGACGCACCGGCAGTACTTCAGCCTAAA GAAAGTTAATATTCATAGCTTTCTTTGGGCTG AGGTACTGTTTTTTGAATTCGCACCAGCACG CTACGCAACACGTCACAGTACACACACACTT CGCCGT | 114 |
| AOF2 | KDM1A_ NM_015 013.3_21 161 | CTGGCCATTCTCAAAGGG ATT | 75 | GAAGACGCACCGGCTGGCTATTCTTAAAGG GATTGTTAATATTCATAGCAATCCCTTTGAGA ATGGCCAGTTTTTTGAATTCGCACCAGCACG CTACGCATGGTGTCACAGTACACACACCATT CGCCGT | 115 |
| AOF2 | KDM1A_ NM_015 013.3_21 160 | GCCATGGTGGTAACAGG TCTT | 76 | GAAGACGCACCGGGCCATGGTGGTAATAGG TCTTGTTAATATTCATAGCAAGACCTGTTACC ACCATGGCTTTTTTGAATTCGCACCAGCACGC TACGCAGTTGGTCACAGTACACACACGTTTC GCCGT | 116 |
| AOF2 | KDM1A_ | CAGGAGAAGCTGCTGGT | 77 | GAAGACGCACCGGCAGGAGAAGCTGTTGGT | 117 |
| | | ATCA | | ATTAGTTAATATTCATAGCTGATACCAGCAG CTTCTCCTGTTTTTTGAATTCGCACCAGCACG CTACGCAACGTGTCACAGTACACACCATGTT CGCCGT | |
| AOF2 | KDM1A_ NM_015 013.3_15 448 | CCACGAGTCAAACCTTTA TTT | 78 | GAAGACGCACCGGCCACGAGTCAAATCTTTA TTTGTTAATATTCATAGCAAATAAAGGTTTGA CTCGTGGTTTTTTGAATTCGCACCAGCACGCT ACGCACACAGTGTTGTGGTCACAACGTTTCG CCGT | 118 |
| AOF2 | KDM1A_ NM_015 013.3_15 447 | GCTACATCTTACCTTAGT CAT | 79 | GAAGACGCACCGGGCTACATCTTACCTTAGT TATGTTAATATTCATAGCATGACTAAGGTAA GATGTAGCTTTTTTGAATTCGCACCAGCACG CTACGCATGACGTGTTGTGGTCACACATGTT CGCCGT | 119 |
| AOF2 | KDM1A-725_582 82 | CCGGATGACTTCTCAAGA AGC | 80 | CCGGATGACTTCTCAAGAAGCCTCGAGGCTT CTTGAGAAGTCATCCGGTTTTTTGAATTCGCA CCAGCACGCTACGCAGTTGGTTGACGTTGAC ACACACTTCGCCGTCTTC | 120 |
| AOF2 | KDM1A_ NM_015 013.3_15 445 | GCTCCAATACTGTTGGCA CTA | 81 | GAAGACGCACCGGGCTCCAATATTGTTGGCA TTAGTTAATATTCATAGCTAGTGCCAACAGTA TTGGAGCTTTTTTGAATTCGCACCAGCACGCT ACGCACACAGTGTTGTGGTCACACACATTCG CCGT | 121 |
| AOF2 | KDM1A_ NM_015 013.3_15 446 | CCAACAATTAGAAGCACC TTA | 82 | GAAGACGCACCGGCCAACAATTAGAAGCAT CTTAGTTAATATTCATAGCTAAGGTGCTTCTA ATTGTTGGTTTTTTGAATTCGCACCAGCACGC TACGCAGTTGGTGTTGTGGTCACACAACTTC GCCGT | 122 |

Nucleic acid LSD1 inhibitor molecules include nucleic acid molecules comprising chemical modifications, e.g., modifications to the nucleic acid base, the sugar and/or the phosphate backbone, including, for example, peptide nucleic acids, phospho morpholino backbones, phosphorothioate backbones, 5' and 3' end caps, 2'-Omethyl modification, 2'-F modifications, and other modifications known in the art.

### Genome Editing System LSD1 inhibitors

Genome editing systems are known in the art, and include zinc finger nuclease gene editing systems, TALEN gene editing systems, meganuclease gene editing systems, and CRISPR gene editing systems. As used herein, the term "genome editing system" (used herein synonymously with "gene editing system") refers to a molecule or set of molecules necessary and sufficient to direct modification, e.g., insertion or deletion, of nucleic acids, at or near a site targeted by said system. As the term is used herein, the term "genome editing system" also refers to nucleic acid encoding one or more components (e.g., molecules) of the genome editing system. Exemplary gene editing systems are known in the art, and are described more fully below.

### CRISPR Gene Editing Systems

As used herein, the terms "CRISPR System" "CRISPR/Cas System", "CRISPR/Cas gene editing system", "CRISPR/Cas genome editing system", "CRISPR genome editing system" and "CRISPR gene editing system" are used synonymously herein. Naturally-occurring CRISPR systems are found in approximately 40% of sequenced eubacteria genomes and 90% of sequenced archaea. Grissa et al. (2007) BMC Bioinformatics 8: 172. This system is a type of prokaryotic immune system that confers resistance to foreign genetic elements such as plasmids and phages and provides a form of acquired immunity. Barrangou et al. (2007) Science 315: 1709-1712; Marragini et al. (2008) Science 322: 1843-1845.

The CRISPR system has been modified for use in gene editing (silencing, enhancing or changing specific genes) in eukaryotes such as mice, primates and humans. Wiedenheft et al. (2012) Nature 482: 331-8. This is accomplished by, for example, introducing into the eukaryotic cell one or more vectors encoding a specifically engineered guide RNA (gRNA) (e.g., a gRNA comprising sequence complementary to sequence of a eukaryotic genome) and one or more appropriate RNA-guided nucleases, e.g., Cas proteins. The RNA guided nuclease forms a complex with the gRNA, which is then directed to the target DNA site by hybridization of the gRNA's sequence to complementary sequence of a eukaryotic genome, where the RNA-guided nuclease then induces a double or single-strand break in the DNA. Insertion or deletion of nucleotides at or near the strand break creates the modified genome.

As these naturally occur in many different types of bacteria, the exact arrangements of the CRISPR and structure, function and number of Cas genes and their product differ somewhat from species to species. Haft et al. (2005) PLoS Comput. Biol. 1: e60; Kunin et al. (2007) Genome Biol. 8: R61; Mojica et al. (2005) J. Mol. Evol. 60: 174-182; Bolotin et al. (2005) Microbiol. 151: 2551-2561; Pourcel et al. (2005) Microbiol. 151: 653-663; and Stern et al. (2010) Trends. Genet. 28: 335-340. For example, the Cse (Cas subtype, E. coli) proteins (e.g., CasA) form a functional complex, Cascade, that processes CRISPR RNA transcripts into spacer-repeat units that Cascade retains. Brouns et al. (2008) Science 321: 960-964. In other prokaryotes, Cas6 processes the CRISPR transcript. The CRISPR-based phage inactivation in E. coli requires Cascade and Cas3, but not Cas1 or Cas2. The Cmr (Cas RAMP module) proteins in Pyrococcus furiosus and other prokaryotes form a functional complex with small CRISPR RNAs that recognizes and cleaves complementary target RNAs. A simpler CRISPR system relies on the protein Cas9, which is a nuclease with two active cutting sites, one for each strand of the double helix. Combining Cas9 and modified CRISPR locus RNA can be used in a system for gene editing. Pennisi (2013) Science 341: 833-836.

With respect to general information on CRISPR-Cas Systems, components thereof, and delivery of such components, including methods, materials, delivery vehicles, vectors, particles, AAV, and making and using thereof, including as to amounts and formulations, all useful in the practice of the instant invention, reference is made to: US Patents Nos. 8,697,359, 8,771,945, 8,795,965, 8,865,406, 8,871,445, 8,889,356, 8,889,418 and 8,895,308; US Patent Publications US 2014-0310830 (US APP. Ser. No. 14/105,031), US 2014-0287938 A1 (U.S. App. Ser. No. 14/213,991), US 2014-0273234 A1 (U.S. App. Ser. No. 14/293,674), US2014-0273232 A1 (U.S. App. Ser. No. 14/290,575), US 2014-0273231 (U.S. App. Ser. No. 14/259,420), US 2014-0256046 A1 (U.S. App. Ser. No. 14/226,274), US 2014-0248702 A1 (U.S. App. Ser. No. 14/258,458), US 2014-0242700 A1 (U.S. App. Ser. No. 14/222,930), US 2014-0242699 A1 (U.S. App. Ser. No. 14/183,512), US 2014-0242664 A1 (U.S. App. Ser. No. 14/104,990), US 2014-0234972 A1 (U.S. App. Ser. No. 14/183,471), US 2014-0227787 A1 (U.S. App. Ser. No. 14/256,912), US 2014-0189896 A1 (U.S. App. Ser. No. 14/105,035), US 2014-0186958 (U.S. App. Ser. No. 14/105,017), US 2014-0186919 A1 (U.S. App. Ser. No. 14/104,977), US 2014-0186843 A1 (U.S. App. Ser. No. 14/104,900), US 2014-0179770 A1 (U.S. App. Ser. No. 14/104,837) and US 2014-0179006 A1 (U.S. App. Ser. No. 14/183,486), US 2014-0170753 (US App Ser No 14/183,429); European Patent Applications EP 2 771 468 (EP13818570.7), EP 2 764 103 (EP13824232.6), and EP 2 784 162 (EP14170383.5); and PCT Patent Publications WO 2014/093661 (PCT/US2013/074743), WO 2014/093694 (PCT/US2013/074790), WO 2014/093595 (PCT/US2013/074611), WO 2014/093718 (PCT/US2013/074825), WO 2014/093709 (PCT/US2013/074812), WO 2014/093622 (PCT/US2013/074667), WO 2014/093635 (PCT/US2013/074691), WO 2014/093655 (PCT/US2013/074736), WO 2014/093712 (PCT/US2013/074819), WO 2014/093701 (PCT7US2013/074800), WO 2014/018423 (PCT/US2013/051418) ,WO 2014/204723 (PCT/US2014/041790), WO 2014/204724 (PCT/US2014/041800), WO 2014/204725 (PCT/US2014/041803), WO 2014/204726 (PCT US2014/041804), WO 2014/204727 (PCT US2014/041806), WO 2014/204728 (PCT/US2014/041808), and WO 2014/204729 (PCT US2014/041809). Reference is also made to US provisional patent applications 61/758,468; 61/802,174; 61/806,375; 61/814,263; 61/819,803 and 61/828,130, filed on January 30, 2013; March 15, 2013; March 28, 2013; April 20, 2013; May 6, 2013 and May 28, 2013 respectively. Reference is also made to US provisional patent application 61/836,123, filed on June 17, 2013. Reference is additionally made to US provisional patent applications 61/835,931, 61/835,936, 61/836,127, 61/836, 101, 61/836,080 and 61/835,973, each filed June 17, 2013. Further reference is made to US provisional patent applications 61/862,468 and 61/862,355 filed on August 5, 2013; 61/871,301 filed on August 28, 2013; 61/960,777 filed on September 25, 2013 and 61/961 ,980 filed on October 28, 2013. Reference is yet further made to: PCT Patent applications Nos: PCT/US2014/041803, PCT/US2014/041800, PCT/US2014/041809, PCT/US2014/041804 and PCT US2014/041806, each filed June 10, 2014 6/10/14; PCT US2014/041808 filed June 11, 2014; and PCT/US2014/62558 filed October 28, 2014, and US Provisional Patent Applications Serial Nos.: 61/915,150, 61/915,301, 61/915,267 and 61/915,260, each filed December 12, 2013; 61/757,972 and 61/768,959, filed on January 29, 2013 and February 25, 2013; 61/835,936, 61/836,127, 61/836,101, 61/836,080, 61/835,973, and 61/835,931, filed June 17, 2013; 62/010,888 and 62/010,879, both filed June 11, 2014; 62/010,329 and 62/010,441, each filed June 10, 2014; 61/939,228 and 61/939,242, each filed February 12, 2014; 61/980,012, filed April 15,2014; 62/038,358, filed August 17, 2014; 62/054,490, 62/055,484, 62/055,460 and 62/055,487, each filed September 25, 2014; and 62/069,243, filed October 27, 2014. Reference is also made to US provisional patent applications Nos. 62/055,484, 62/055,460, and 62/055,487, filed September 25, 2014; US provisional patent application 61/980,012, filed April 15, 2014; and US provisional patent application 61/939,242 filed February 12, 2014. Reference is made to PCT application designating, inter alia, the United States, application No. PCT/US 14/41806, filed June 10, 2014. Reference is made to US provisional patent application 61/930,214 filed on January 22, 2014. Reference is made to US provisional patent applications 61/915,251, 61/915,260 and 61/915,267, each filed on December 12, 2013. Reference is made to US provisional patent application USSN 61/980,012 filed April 15, 2014. Reference is made to PCT application designating, inter alia, the United States, application No. PCT/US 14/41806, filed June 10, 2014. Reference is made to US provisional patent application 61/930,214 filed on January 22, 2014. Reference is made to US provisional patent applications 61/915,251; 61/915,260 and 61/915,267, each filed on December 12, 2013. [0054] Mention is also made of US application 62/091,455, filed, 12-Dec-14, PROTECTED GUIDE RNAS (PGRNAS); US application 62/096,708, 24-Dec-14, PROTECTED GUIDE RNAS (PGRNAS); US application 62/091,462, 12-Dec-14, DEAD GUIDES FOR CRISPR TRANSCRIPTION FACTORS; US application 62/096,324, 23-Dec-14, DEAD GUIDES FOR CRISPR TRANSCRIPTION FACTORS; US application 62/091,456, 12-Dec-14, ESCORTED AND FUNCTI ON AL IZED GUIDES FOR CRISPR-CAS SYSTEMS; US application 62/091,461, 12-Dec-14, DELIVERY, USE AND THERAPEUTIC APPLICATIONS OF THE CRISPR-CAS SYSTEMS AND COMPOSITIONS FOR GENOME EDITING AS TO HEMATOPOETIC STEM CELLS (HSCs); US application 62/094,903, 19-Dec-14, UNBIASED IDENTIFICATION OF DOUBLE-STRAND BREAKS AND GENOMIC REARRANGEMENT BY GENOME- WISE INSERT CAPTURE SEQUENCING; US application 62/096,761, 24-Dec-14, ENGINEERING OF SYSTEMS, METHODS AND OPTIMIZED ENZYME AND GUIDE SCAFFOLDS FOR SEQUENCE MANIPULATION; US application 62/098,059, 30-Dec-14, RNA-TARGETING SYSTEM; US application 62/096,656, 24-Dec-14, CRISPR HAVING OR ASSOCIATED WITH DESTABILIZATION DOMAINS; US application 62/096,697, 24-Dec-14, CRISPR HAVING OR ASSOCIATED WITH AAV; US application 62/098,158, 30-Dec-14, ENGINEERED CRISPR COMPLEX INSERTIONAL TARGETING SYSTEMS; US application 62/151,052, 22-Apr-15, CELLULAR TARGETING FOR EXTRACELLULAR EXOSOMAL REPORTING; US application 62/054,490, 24-Sep-14, DELIVERY, USE AND THERAPEUTIC APPLICATIONS OF THE CRISPR-CAS SYSTEMS AND COMPOSITIONS FOR TARGETING DISORDERS AND DISEASES USING PARTICLE DELIVERY COMPONENTS; US application 62/055,484, 25-Sep-14, SYSTEMS, METHODS AND COMPOSITIONS FOR SEQUENCE MANIPULATION WITH OPTIMIZED FUNCTIONAL CRISPR-CAS SYSTEMS; US application 62/087,537, 4-Dec-14, SYSTEMS, METHODS AND COMPOSITIONS FOR SEQUENCE MANIPULATION WITH OPTIMIZED FUNCTIONAL CRISPR-CAS SYSTEMS; US application 62/054,651, 24-Sep-14, DELIVERY, USE AND THERAPEUTIC APPLICATIONS OF THE CRISPR-CAS SYSTEMS AND COMPOSITIONS FOR MODELING COMPETITION OF MULTIPLE CANCER MUTATIONS IN VIVO; US application 62/067,886, 23-Oct-14, DELIVERY, USE AND THERAPEUTIC APPLICATIONS OF THE CRISPR-CAS SYSTEMS AND COMPOSITIONS FOR MODELING COMPETITION OF MULTIPLE CANCER MUTATIONS IN VIVO; US application 62/054,675, 24-Sep-14, DELIVERY, USE AND THERAPEUTIC APPLICATIONS OF THE CRISPR-CAS SYSTEMS AND COMPOSITIONS IN NEURONAL CELLS/TISSUES; US application 62/054,528, 24-Sep-14, DELIVERY, USE AND THERAPEUTIC APPLICATIONS OF THE CRISPR-CAS SYSTEMS AND COMPOSITIONS IN IMMUNE DISEASES OR DISORDERS; US application 62/055,454, 25-Sep-14, DELIVERY, USE AND THERAPEUTIC APPLICATIONS OF THE CRISPR-CAS SYSTEMS AND COMPOSITIONS FOR TARGETING DISORDERS AND DISEASES USING CELL PENETRATION PEPTIDES (CPP); US application 62/055,460, 25-Sep-14, MULTIFUNCTIONAL-CRISPR COMPLEXES AND/OR OPTIMIZED ENZYME LINKED FUNCTION AL-CRISPR COMPLEXES; US application 62/087,475, 4-Dec-14, FUNCTIONAL SCREENING WITH OPTIMIZED FUNCTIONAL CRISPR-CAS SYSTEMS; US application 62/055,487, 25-Sep-14, FUNCTIONAL SCREENING WITH OPTIMIZED FUNCTIONAL CRISPR-CAS SYSTEMS; US application 62/087,546, 4-Dec-14, MULTIFUNCTIONAL CRISPR COMPLEXES AND/OR OPTIMIZED ENZYME LINKED FUNCTIONAL-CRISPR COMPLEXES; and US application 62/098,285, 30-Dec-14, CRISPR MEDIATED IN VIVO MODELING AND GENETIC SCREENING OF TUMOR GROWTH AND METASTASIS.

Each of these patents, patent publications, and applications, and all documents cited therein or during their prosecution ("appln cited documents") and all documents cited or referenced in the appln cited documents, together with any instructions, descriptions, product specifications, and product sheets for any products mentioned therein or in any document therein may be employed in the practice of the invention.

Also with respect to general information on CRISPR-Cas Systems, mention is made of the following:
Multiplex genome engineering using CRISPR/Cas systems, Cong, L., Ran, F.A., Cox, D., Lin, S., Barretto, R., Habib, N., Hsu, P.D., Wu, X., Jiang, W., Marraffini, L.A., & Zhang, F. Science Feb 15;339(6121):819-23 (2013);RNA-gided editing of bacterial genomes using CRISPR-Cas systems. Jiang W., Bikard D., Cox D., Zhang F, Marraffini LA. Nat Biotechnol Mar;31(3):233-9 (2013); One-Step Generation of Mice Carrying Mutations in Multiple Genes by CRISPR/Cas-Mediated Genome Engineering. Wang H., Yang H., Shivalila CS., Dawlaty MM., Cheng AW., Zhang F., Jaenisch R. Cell May 9;153(4):910-8 (2013); Optical control of mammalian endogenous transcription and epigenetic states. Konermann S, Brigham MD, Trevino AE, Hsu PD, Heidenreich M, Cong L, Piatt RJ, Scott DA, Church GM, Zhang F. Nature. 2013 Aug 22;500(7463):472-6. doi: 10.1038/Nature 12466. Epub 2013 Aug 23;
Double Nicking by RNA-Guided CRISPR Cas9 for Enhanced Genome Editing Specificity. Ran, FA., Hsu, PD., Lin, CY., Gootenberg, JS., Konermann, S., Trevino, AE,, Scott, DA., Inoue, A., Matoba, S., Zhang, Y,, & Zhang, F. Cell Aug 28. pii: S0092-8674( 13)01015-5. (2013
DNA targeting specificity of RNA-guided Cas9 nucleases. Hsu, P., Scott, D., Weinstein, J., Ran, FA., Konermann, S., Agarwala, V., Li, Y., Fine, E., Wu, X., Shalem, O., Cradick, TJ., Marraffini, LA., Bao, G., & Zhang, F. Nat Biotechnol doi:10.1038/nbt.2647 (2013);
Genome engineering using the CRISPR-Cas9 system. Ran, FA., Hsu, PD., Wright, J., Agarwala, V., Scott, DA., Zhang, F. Nature Protocols Nov;8(II):2281-308. (2013); Genome-Scale CRISPR-Cas9 Knockout Screening in Human Cells. Shalem, O., Sanjana, NE., Hartenian, E., Shi, X., Scott, DA., Mikkelson, T., Heckl, D., Ebert, BL., Root, DE., Doench, JG., Zhang, F. Science Dec 12. (2013). [Epub ahead of print]; Crystal structure of cas9 in complex with guide RNA and target DNA. Nishimasu, H., Ran, FA., Hsu, PD., Konermann, S., Shehata, SI., Dohmae, N., Ishitani, R., Zhang, F., Nureki, O. Cell Feb 27. (2014). 156(5):935-49;
Genome-wide binding of the CRISPR endonuclease Cas9 in mammalian cells. Wu X., Scott DA., Kriz AJ., Chiu AC, Hsu PD., Dadon DB., Cheng AW., Trevino AE., Konermann S., Chen S., Jaenisch R., Zhang F., Sharp PA. Nat Biotechnol. (2014) Apr 20. doi: 10.1038/nbt.2889,
CRISPR-Cas9 Knockin Mice for Genome Editing and Cancer Modeling, Piatt et al., Cell 159(2): 440-455 (2014) DOI: 10.1016/j.cell.2014.09.014,
Development and Applications of CRISPR-Cas9 for Genome Engineering, Hsu et al. Cell 157, 1262-1278 (June 5, 2014) (Hsu 2014),
Genetic screens in human cells using the CRISPR/Cas9 system, Wang et al., Science. 2014 January 3; 343(6166): 80-84. doi: 10.1126/science.1246981,
Rational design of highly active sgRNAs for CRISPR-Cas9-mediated gene inactivation, Doench et al., Nature Biotechnology published online 3 September 2014; doi: 10.1038/nbt.3026, and In vivo interrogation of gene function in the mammalian brain using CRISPR-Cas9, Swiech et al, Nature Biotechnology ; published online 19 October 2014; doi:10.1038/nbt.3055.

Genome-scale transcriptional activation by an engineered CRISPR-Cas9 complex, onermann S, Brigham MD, Trevino AE, Joung J, Abudayyeh 00, Barcena C, Hsu PD, Habib N, Gootenberg JS, Nishimasu H, Nureki O, Zhang F., Nature. Jan 29;517(7536):583-8 (2015).
A split-Cas9 architecture for inducible genome editing and transcription modulation, Zetsche B, Volz SE, Zhang F., (published online 02 February 2015) Nat Biotechnol. Feb;33(2): 139-42 (2015);
Genome-wide CRISPR Screen in a Mouse Model of Tumor Growth and Metastasis, Chen S, Sanjana NE, Zheng, Shalem O, Lee, Shi X, Scott DA, Song J, Pan JQ, Weissleder R, Lee H, Zhang F, Sharp PA. Cell 160, 1246-1260, March 12, 2015 (multiplex screen in mouse), and
In vivo genome editing using Staphylococcus aureus Cas9, Ran FA, Cong L, Yan WX, Scott DA, Gootenberg JS, Kriz AJ, Zetsche B, Shalem O, Wu X, Makarova KS, oonin EV, Sharp PA, Zhang F., (published online 01 April 2015), Nature. Apr 9;520(7546): 186-91 (2015).

High-throughput functional genomics using CRISPR-Cas9, Shalem et al,, Nature Reviews Genetics 16, 299-311 (May 2015).

Sequence determinants of improved CRISPR sgRNA design, Xu et al., Genome Research 25, 1 147-1 157 (August 2015).

A Genome-wide CRISPR Screen in Primary Immune Cells to Dissect Regulatory Networks, Parnas et al., Cell 162, 675-686 (July 30, 2015).

CRISPR/Cas9 cleavage of viral DNA efficiently suppresses hepatitis B virus, Ramanan et al., Scientific Reports 5:10833. doi: 10.1038/srepI0833 (June 2, 2015).

Crystal Structure of Staphylococcus aureus Cas9, Nishimasu et al., Cell 162, 1113-1126 (Aug. 27, 2015).

BCL11 A enhancer dissection by Cas9-mediated in situ saturating mutagenesis, Canver et al., Nature 527(7577): 192-7 (Nov. 12, 2015) doi: 10.1038/naturel 5521. Epub 2015 Sep 16. each of which is discussed briefly below:
Cong et al. engineered type II CRISPR/Cas systems for use in eukaryotic cells based on both Streptococcus thermophilus Cas9 and also Streptoccocus pyogenes Cas9 and demonstrated that Cas9 nucleases can be directed by short RNAs to induce precise cleavage of DNA in human and mouse cells. Their study further showed that Cas9 as converted into a nicking enzyme can be used to facilitate homology-directed repair in eukaryotic cells with minimal mutagenic activity. Additionally, their study demonstrated that multiple guide sequences can be encoded into a single CRISPR array to enable simultaneous editing of several at endogenous genomic loci sites within the mammalian genome, demonstrating easy programmability and wide applicability of the RNA-guided nuclease technology. This ability to use RNA to program sequence specific DNA cleavage in cells defined a new class of genome engineering tools. These studies further showed that other CRISPR loci are likely to be transplantable into mammalian cells and can also mediate mammalian genome cleavage. Importantly, it can be envisaged that several aspects of the CRISPR/Cas system can be further improved to increase its efficiency and versatility.

Jiang et al. used the clustered, regularly interspaced, short palindromic repeats (CRISPR)-associated Cas9 endonuclease complexed with dual-RNAs to introduce precise mutations in the genomes of Streptococcus pneumoniae and Escherichia coli. The approach relied on dual-RNA:Cas9-directed cleavage at the targeted genomic site to kill unmutated cells and circumvents the need for selectable markers or counter-selection systems, The study reported reprogramming dual-RNA:Cas9 specificity by changing the sequence of short CRISPR RNA (crRNA) to make single- and multinucleotide changes carried on editing templates. The study showed that simultaneous use of two crRNAs enabled multiplex mutagenesis. Furthermore, when the approach was used in combination with recombineering, in S. pneumoniae, nearly 100% of cells that were recovered using the described approach contained the desired mutation, and in E. coli, 65% that were recovered contained the mutation.

Wang et al. (2013) used the CRISPR/Cas system for the one-step generation of mice carrying mutations in multiple genes which were traditionally generated in multiple steps by sequential recombination in embryonic stem cells and/or time-consuming intercrossing of mice with a single mutation. The CRISPR/Cas system will greatly accelerate the in vivo study of functionally redundant genes and of epistatic gene interactions.

Konermann et al. addressed the need in the art for versatile and robust technologies that enable optical and chemical modulation of DNA-binding domains based CRISPR Cas9 enzyme and also Transcriptional Activator Like Effectors.

Ran et al. (2013-A) described an approach that combined a Cas9 nickase mutant with paired guide RNAs to introduce targeted double-strand breaks. This addresses the issue of the Cas9 nuclease from the microbial CRISPR-Cas system being targeted to specific genomic loci by a guide sequence, which can tolerate certain mismatches to the DNA target and thereby promote undesired off-target mutagenesis. Because individual nicks in the genome are repaired with high fidelity, simultaneous nicking via appropriately offset guide RNAs is required for double-stranded breaks and extends the number of specifically recognized bases for target cleavage. The authors demonstrated that using paired nicking can reduce off-target activity by 50- to 1,500-fold in cell lines and to facilitate gene knockout in mouse zygotes without sacrificing on-target cleavage efficiency. This versatile strategy enables a wide variety of genome editing applications that require high specificity. Hsu et al. (2013) characterized SpCas9 targeting specificity in human cells to inform the selection of target sites and avoid off-target effects. The study evaluated >700 guide RNA variants and SpCas9-induced indel mutation levels at > 100 predicted genomic off-target loci in 293T and 293FT cells. The authors that SpCas9 tolerates mismatches between guide RNA and target DNA at different positions in a sequence-dependent manner, sensitive to the number, position and distribution of mismatches. The authors further showed that SpCas9-mediated cleavage is unaffected by DNA methylation and that the dosage of SpCas9 and sgRNA can be titrated to minimize off-target modification. Additionally, to facilitate mammalian genome engineering applications, the authors reported providing a web-based software tool to guide the selection and validation of target sequences as well as off-target analyses.

Ran et al. (2013-B) described a set of tools for Cas9-mediated genome editing via non-homologous end joining (NHEJ) or homology-directed repair (HDR) in mammalian cells, as well as generation of modified cell lines for downstream functional studies. To minimize off-target cleavage, the authors further described a double-nicking strategy using the Cas9 nickase mutant with paired guide RNAs. The protocol provided by the authors experimentally derived guidelines for the selection of target sites, evaluation of cleavage efficiency and analysis of off-target activity. The studies showed that beginning with target design, gene modifications can be achieved within as little as 1-2 weeks, and modified clonal cell lines can be derived within 2-3 weeks.

Shaiem et al. described a new way to interrogate gene function on a genome-wide scale. Their studies showed that delivery of a genome-scale CRISPR-Cas9 knockout (GeC O) library targeted 18,080 genes with 64,751 unique guide sequences enabled both negative and positive selection screening in human cells. First, the authors showed use of the GeCKO library to identify genes essential for cell viability in cancer and pluripotent stem cells. Next, in a melanoma model, the authors screened for genes whose loss is involved in resistance to vemurafenib, a therapeutic that inhibits mutant protein kinase BRAF. Their studies showed that the highest-ranking candidates included previously validated genes NF1 and MED 12 as well as novel hits NF2, CUL3, TADA2B, and TADAL The authors observed a high level of consistency between independent guide RNAs targeting the same gene and a high rate of hit confirmation, and thus demonstrated the promise of genome-scale screening with Cas9.

Nishimasu et al. reported the crystal structure of Streptococcus pyogenes Cas9 in complex with sgRNA and its target DNA at 2.5 A° resolution. The structure revealed a bilobed architecture composed of target recognition and nuclease lobes, accommodating the sgRNA:DNA heteroduplex in a positively charged groove at their interface. Whereas the recognition lobe is essential for binding sgRNA and DNA, the nuclease lobe contains the HNH and RuvC nuclease domains, which are properly positioned for cleavage of the complementary and non-complementary strands of the target DNA, respectively. The nuclease lobe also contains a carboxyl-terminal domain responsible for the interaction with the protospacer adjacent motif (PAM). This high-resolution structure and accompanying functional analyses have revealed the molecular mechanism of RNA-guided DNA targeting by Cas9, thus paving the way for the rational design of new, versatile genome-editing technologies.

Wu et al. mapped genome-wide binding sites of a catalytically inactive Cas9 (dCas9) from Streptococcus pyogenes loaded with single guide RNAs (sgRNAs) in mouse embryonic stem cells (mESCs). The authors showed that each of the four sgRNAs tested targets dCas9 to between tens and thousands of genomic sites, frequently characterized by a 5-nucleotide seed region in the sgRNA and an NGG protospacer adjacent motif (PAM). Chromatin inaccessibility decreases dCas9 binding to other sites with matching seed sequences; thus 70% of off-target sites are associated with genes. The authors showed that targeted sequencing of 295 dCas9 binding sites in mESCs transfected with catalytically active Cas9 identified only one site mutated above background levels. The authors proposed a two-state model for Cas9 binding and cleavage, in which a seed match triggers binding but extensive pairing with target DNA is required for cleavage.

Piatt et al. established a Cre-dependent Cas9 knockin mouse. The authors demonstrated in vivo as well as ex vivo genome editing using adeno-associated virus (AAV)-, lentivirus-, or particle-mediated delivery of guide RNA in neurons, immune cells, and endothelial cells.

Hsu et al. (2014) is a review article that discusses generally CRISPR-Cas9 history from yogurt to genome editing, including genetic screening of cells.

Wang et al, (2014) relates to a pooled, loss-of-function genetic screening approach suitable for both positive and negative selection that uses a genome-scale lentiviral single guide RNA (sgRNA) library.

Doench et al. created a pool of sgRNAs, tiling across all possible target sites of a panel of six endogenous mouse and three endogenous human genes and quantitatively assessed their ability to produce null alleles of their target gene by antibody staining and flow cytometry. The authors showed that optimization of the PAM improved activity and also provided an on-line tool for designing sgRNAs.

Swiech et al. demonstrate that AAV-mediated SpCas9 genome editing can enable reverse genetic studies of gene function in the brain.

Konermann et al. (2015) discusses the ability to attach multiple effector domains, e.g., transcriptional activator, functional and epigenomic regulators at appropriate positions on the guide such as stem or tetraloop with and without linkers.

Zetsche et al. demonstrates that the Cas9 enzyme can be split into two and hence the assembly of Cas9 for activation can be controlled.

Chen et al relates to multiplex screening by demonstrating that a genome- wide in vivo CRISPR-Cas9 screen in mice reveals genes regulating lung metastasis. > Ran et al. (2015) relates to SaCas9 and its ability to edit genomes and demonstrates that one cannot extrapolate from biochemical assays. Shalem et al. (2015) described ways in which catalytically inactive Cas9 (dCas9) fusions are used to synthetically repress (CRISPRi) or activate (CRISPRa) expression, showing, advances using Cas9 for genome-scale screens, including arrayed and pooled screens, knockout approaches that inactivate genomic loci and strategies that modulate transcriptional activity.

Shalem et al. (2015) described ways in which catalytically inactive Cas9 (dCas9) fusions are used to synthetically repress (CRISPRi) or activate (CRISPRa) expression, showing, advances using Cas9 for genome-scale screens, including arrayed and pooled screens, knockout approaches that inactivate genomic loci and strategies that modulate transcriptional activity.

Xu et al. (2015) assessed the DNA sequence features that contribute to single guide RNA (sgRNA) efficiency in CRISPR-based screens. The authors explored efficiency of CRISPR/Cas9 knockout and nucleotide preference at the cleavage site. The authors also found that the sequence preference for CRISPRi/a is substantially different from that for CRISPR Cas9 knockout.

Parnas et al. (2015) introduced genome- wide pooled CRISPR-Cas9 libraries into dendritic cells (DCs) to identify genes that control the induction of tumor necrosis factor (Tnf) by bacterial lipopolysaccharide (LPS). Known regulators of TIr4 signaling and previously unknown candidates were identified and classified into three functional modules with distinct effects on the canonical responses to LPS.

Ramanan et al (2015) demonstrated cleavage of viral episomal DNA (cccDNA) in infected cells. The HBV genome exists in the nuclei of infected hepatocytes as a 3.2kb double-stranded episomal DNA species called covalently closed circular DNA (cccDNA), which is a key component in the HBV life cycle whose replication is not inhibited by current therapies. The authors showed that sgRNAs specifically targeting highly conserved regions of HBV robustly suppresses viral replication and depleted cccDNA. Nishimasu et al. (2015) reported the crystal structures of SaCas9 in complex with a single guide RNA (sgRNA) and its double-stranded DNA targets, containing the 5'-TTGAAT-3' PAM and the 5'-TTGGGT-3' PAM. A structural comparison of SaCas9 with SpCas9 highlighted both structural conservation and divergence, explaining their distinct PAM specificities and orthologous sgRNA recognition.

Slaymaker et al (2015) reported the use of structure-guided protein engineering to improve the specificity of Streptococcus pyogenes Cas9 (SpCas9). The authors developed "enhanced specificity" SpCas9 (eSpCas9) variants which maintained robust on-target cleavage with reduced off-target effects.

Tsai et al, "Dimeric CRISPR A-guided Fokl nucleases for highly specific genome editing," Nature Biotechnology 32(6): 569-77 (2014) which is not believed to be prior art to the instant invention or application, but which may be considered in the practice of the instant invention. Mention is also made of Konermann et al., "Genome-scale transcription activation by an engineered CRISPR-Cas9 complex," doi:10.1038/naturel4136.

In general, the CRISPR-Cas or CRISPR system is as used in the foregoing documents, such as WO 2014/093622 (PCT/US2013/074667) and refers collectively to transcripts and other elements involved in the expression of or directing the activity of CRISPR-associated (Cas) genes, including sequences encoding a Cas gene, a tracr (trans-activating CRISPR) sequence (e.g. tracrRNA or an active partial tracrRNA), a tracr-mate sequence (encompassing a direct repeat and a tracrRNA-processed partial direct repeat in the context of an endogenous CRISPR system), a guide sequence (also referred to as a spacer in the context of an endogenous CRISPR system), or gRNA(s) as that term is herein used (including, e.g., single guide RNA (sgRNA) (chimeric RNA) and dual guide RNAs (dgRNAs)). In the context CRISPR systems a, "target sequence" refers to a sequence to which the targeting domain sequence of a gRNA molecule is designed to have complementarity, where hybridization between a target sequence and a targeting domain sequence of a gRNA directs the CRISPR system to the locus comprising the target sequence. A target sequence may comprise any polynucleotide, such as DNA or RNA polynucleotides. In some embodiments, a target sequence is located in the nucleus or cytoplasm of a cell. In some embodiments, direct repeats may be identified in silico by searching for repetitive motifs that fulfill any or all of the following criteria: 1. found in a 2Kb window of genomic sequence flanking the type II CRISPR locus; 2. span from 20 to 50 bp; and 3. interspaced by 20 to 50 bp. In some embodiments, 2 of these criteria may be used, for instance 1 and 2, 2 and 3, or 1 and 3. In some embodiments, all 3 criteria may be used. In some embodiments it may be preferred in a CRISPR system that the tracr sequence has one or more hairpins and is 30 or more nucleotides in length, 40 or more nucleotides in length, or 50 or more nucleotides in length; the guide sequence is between 10 to 30 nucleotides in length, the CRISPR/Cas enzyme is a Type II Cas9 enzyme. In embodiments of the invention the terms guide sequence and guide RNA are used interchangeably as in foregoing cited documents such as WO 2014/093622 (PCT US2013/074667). In general, a guide sequence is any polynucleotide sequence having sufficient complementarity with a target sequence to hybridize with the target sequence and direct sequence-specific binding of a CRISPR system to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith- Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and aq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. Preferably the guide sequence is 10 - 30 nucleotides long. The ability of a guide sequence to direct sequence- specific binding of a CRISPR system to a target sequence may be assessed by any suitable assay. For example, the components of a CRISPR system sufficient to form a CRISPR system, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described in the literature and known to those skilled in the art. Similarly, cleavage of a target polynucleotide sequence may be evaluated in a test tube by providing the target sequence, components of a CRISPR system, including the guide sequence to be tested and a control guide sequence different from the test guide sequence, and comparing binding or rate of cleavage at the target sequence between the test and control guide sequence reactions. Other assays are possible, and will occur to those skilled in the art. A guide sequence may be selected to target any target sequence. In some embodiments, the target sequence is a sequence within a genome of a cell. Exemplary target sequences include those that are unique in the target genome. For example, for the S. pyogenes Cas9, a unique sequence in a genome may include a Cas9 target sequence of the form NNNNNNNNNNNNNNNNNNNNXGG (where N is A, G, T, or C; and X can be anything), wherein the sequence has a single occurrence in the genome. For the S. thermophilus CRISPR/Cas9 system, a unique sequence in a genome may include a Cas9 target site of the form NNNNNNNNNNNNNNNNNNNNXXAGAAW (SEQ ID NO: 863) (where N is A, G, T, or C; X can be anything; and W is A or T), wherein the sequence has a single occurrence in the genome. For the S. pyogenes Cas9 or the S. Thermophilius Cas9, a unique sequence in a genome may include a Cas9 target site of the form NNNNNNNNNNNNNNNNNNNNXGGXG (where N is A, G, T, or C; and X can be anything), wherein the sequence has a single occurrence in the genome. In some embodiments, a guide RNA sequence is selected to reduce the degree secondary structure within the guide RNA sequence. In some embodiments, about or less than about 75%, 50%, 40%, 30%, 25%, 20%, 15%, 10%, 5%, 1%, or fewer of the nucleotides of the guide sequence participate in self-complementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

In some embodiments, the RNA-guided nuclease is a Cas molecule, e.g., a Cas9 molecule. Cas9 molecules of a variety of species can be used in the methods and compositions described herein. In preferred embodiments, the Cas9 molecule is a S. pyogenes Cas9 molecule. In embodiments, the Cas9 molecule is derived from a S. pyogenes Cas9 molecule (e.g., UniProt Q99ZW2). While the S. pyogenes Cas9 molecule are the subject of much of the disclosure herein, Cas9 molecules of, derived from, or based on the Cas9 proteins of other species listed herein can be used as well. In other words, other Cas9 molecules, e.g., S. thermophilus, Staphylococcus aureus and/or Neisseria meningitidis Cas9 molecules, may be used in the systems, methods and compositions described herein. Additional Cas9 species include: Acidovorax avenae, Actinobacillus pleuropneumoniae, Actinobacillus succinogenes, Actinobacillus suis, Actinomyces sp., cycliphilus denitrificans, Aminomonas paucivorans, Bacillus cereus, Bacillus smithii, Bacillus thuringiensis, Bacteroides sp., Blastopirellula marina, Bradyrhiz' obium sp., Brevibacillus latemsporus, Campylobacter coli, Campylobacter jejuni, Campylobacter lad, Candidatus Puniceispirillum, Clostridiu cellulolyticum, Clostridium perfringens, Corynebacterium accolens, Corynebacterium diphtheria, Corynebacterium matruchotii, Dinoroseobacter sliibae, Eubacterium dolichum, gamma proteobacterium, Gluconacetobacler diazotrophicus, Haemophilus parainfluenzae, Haemophilus sputorum, Helicobacter canadensis, Helicobacter cinaedi, Helicobacter mustelae, Ilyobacler polytropus, Kingella kingae, Lactobacillus crispatus, Listeria ivanovii, Listeria monocytogenes, Listeriaceae bacterium, Methylocystis sp., Methylosinus trichosporium, Mobiluncus mulieris, Neisseria bacilliformis, Neisseria cinerea, Neisseria flavescens, Neisseria lactamica. Neisseria sp., Neisseria wadsworthii, Nitrosomonas sp., Parvibaculum lavamentivorans, Pasteurella multocida, Phascolarctobacterium succinatutens, Ralstonia syzygii, Rhodopseudomonas palustris, Rhodovulum sp., Simonsiella muelleri, Sphingomonas sp., Sporolactobacillus vineae, Staphylococcus lugdunensis, Streptococcus sp., Subdoligranulum sp., Tislrella mobilis, Treponema sp., or Verminephrobacter eiseniae.

A Cas9 molecule, as that term is used herein, refers to a molecule that can interact with a gRNA molecule (e.g., sequence of a domain of a tracr) and, in concert with the gRNA molecule, localize (e.g., target or home) to a site which comprises a target sequence and PAM sequence.

In embodiments, the ability of an active Cas9 molecule to interact with and cleave a target nucleic acid is PAM sequence dependent. A PAM sequence is a sequence in the target nucleic acid. In an embodiment, cleavage of the target nucleic acid occurs upstream from the PAM sequence. Active Cas9 molecules from different bacterial species can recognize different sequence motifs (e.g., PAM sequences). In an embodiment, an active Cas9 molecule of S. pyogenes recognizes the sequence motif NGG and directs cleavage of a target nucleic acid sequence 1 to 10, e.g., 3 to 5, base pairs upstream from that sequence. See, e.g., Mali el al, SCIENCE 2013; 339(6121): 823- 826. In an embodiment, an active Cas9 molecule of S. thermophilus recognizes the sequence motif NGGNG and NNAGAAW (W = A or T) and directs cleavage of a core target nucleic acid sequence 1 to 10, e.g., 3 to 5, base pairs upstream from these sequences. See, e.g., Horvath et al., SCIENCE 2010; 327(5962): 167- 170, and Deveau et al, J BACTERIOL 2008; 190(4): 1390- 1400. In an embodiment, an active Cas9 molecule of S. mutans recognizes the sequence motif NGG or NAAR (R - A or G) and directs cleavage of a core target nucleic acid sequence 1 to 10, e.g., 3 to 5 base pairs, upstream from this sequence. See, e.g., Deveau et al., J BACTERIOL 2008; 190(4): 1390- 1400.

In an embodiment, an active Cas9 molecule of S. aureus recognizes the sequence motif NNGRR (R = A or G) and directs cleavage of a target nucleic acid sequence 1 to 10, e.g., 3 to 5, base pairs upstream from that sequence. See, e.g., Ran F. et al., NATURE, vol. 520, 2015, pp. 186-191. In an embodiment, an active Cas9 molecule of N. meningitidis recognizes the sequence motif NNNNGATT and directs cleavage of a target nucleic acid sequence 1 to 10, e.g., 3 to 5, base pairs upstream from that sequence. See, e.g., Hou et al., PNAS EARLY EDITION 2013, 1 -6. The ability of a Cas9 molecule to recognize a PAM sequence can be determined, e.g., using a transformation assay described in Jinek et al, SCIENCE 2012, 337:816.

Exemplary naturally occurring Cas9 molecules are described in Chylinski et al , RNA Biology 2013; 10:5, 727-737. Such Cas9 molecules include Cas9 molecules of a cluster 1 bacterial family, cluster 2 bacterial family, cluster 3 bacterial family, cluster 4 bacterial family, cluster 5 bacterial family, cluster 6 bacterial family, a cluster 7 bacterial family, a cluster 8 bacterial family, a cluster 9 bacterial family, a cluster 10 bacterial family, a cluster 11 bacterial family, a cluster 12 bacterial family, a cluster 13 bacterial family, a cluster 14 bacterial family, a cluster 1 bacterial family, a cluster 16 bacterial family, a cluster 17 bacterial family, a cluster 18 bacterial family, a cluster 19 bacterial family, a cluster 20 bacterial family, a cluster 21 bacterial family, a cluster 22 bacterial family, a cluster 23 bacterial family, a cluster 24 bacterial family, a cluster 25 bacterial family, a cluster 26 bacterial family, a cluster 27 bacterial family, a cluster 28 bacterial family, a cluster 29 bacterial family, a cluster 30 bacterial family, a cluster 31 bacterial family, a cluster 32 bacterial family, a cluster 33 bacterial family, a cluster 34 bacterial family, a cluster 35 bacterial family, a cluster 36 bacterial family, a cluster 37 bacterial family, a cluster 38 bacterial family, a cluster 39 bacterial family, a cluster 40 bacterial family, a cluster 41 bacterial family, a cluster 42 bacterial family, a cluster 43 bacterial family, a cluster 44 bacterial family, a cluster 45 bacterial family, a cluster 46 bacterial family, a cluster 47 bacterial family, a cluster 48 bacterial family,. a cluster 49 bacterial family, a cluster 50 bacterial family, a cluster 5 1 bacterial family, a cluster 52 bacterial family, a cluster 53 bacterial family, a cluster 54 bacterial family, a cluster 55 bacterial family, a cluster 56 bacterial family, a cluster 57 bacterial family, a cluster 58 bacterial family, a cluster 59 bacterial family, a cluster 60 bacterial family, a cluster 61 bacterial family, a cluster 62 bacterial family, a cluster 63 bacterial family, a cluster 64 bacterial family, a cluster 65 bacterial family, a cluster 66 bacterial family, a cluster 67 bacterial family, a cluster 68 bacterial family, a cluster 69 bacterial family, a cluster 70 bacterial family, a cluster 71 bacterial family, a cluster 72 bacterial family, a cluster 73 bacterial family, a cluster 74 bacterial family, a cluster 75 bacterial family, a cluster 76 bacterial family, a cluster 77 bacterial family, or a cluster 78 bacterial family.

Exemplary naturally occurring Cas9 molecules include a Cas9 molecule of a cluster 1 bacterial family. Examples include a Cas9 molecule of: S. pyogenes (e.g., strain SF370, MGAS 10270, MGAS 10750, MGAS2096, MGAS315, MGAS5005, MGAS6180, MGAS9429, NZ131 and SSI-1), S. thermophilus (e.g., strain LMD-9), S. pseudoporcinus (e.g., strain SPIN 20026), S. mutans (e.g., strain UA 159, NN2025), S. macacae (e.g., strain NCTC1 1558), S. gallolylicus (e.g., strain UCN34, ATCC BAA-2069), S. equines (e.g., strain ATCC 9812, MGCS 124), S. dysdalactiae (e.g., strain GGS 124), S. bovis (e.g., strain ATCC 700338), S. cmginosus (e.g.; strain F021 1 ), S. agalactia* (e.g., strain NEM316, A909), Listeria monocytogenes (e.g., strain F6854), Listeria innocua (L. innocua, e.g., strain Clip 11262), EtUerococcus italicus (e.g., strain DSM 15952), or Enterococcus faecium (e.g., strain 1,23 ,408). Additional exemplary Cas9 molecules are a Cas9 molecule of Neisseria meningitidis (Hou et al. PNAS Early Edition 2013, 1-6) and a S. aureus Cas9 molecule.

In an embodiment, a Cas9 molecule, e.g., an active Cas9 molecule or inactive Cas9 molecule, comprises an amino acid sequence: having 60%, 65%, 70%, 75%, 80%, 85%, 90%, 95%, 96%, 97%, 98%, or 99% homology with; differs at no more than 1%, 2%, 5%, 10%, 15%, 20%, 30%, or 40% of the amino acid residues when compared with; differs by at least 1, 2, 5, 10 or 20 amino acids but by no more than 100, 80, 70, 60, 50, 40 or 30 amino acids from; or is identical to; any Cas9 molecule sequence described herein or a naturally occurring Cas9 molecule sequence, e.g., a Cas9 molecule from a species listed herein or described in Chylinski et al., RNA Biology 2013, 10:5, 'I2'I-T,1 Hou et al. PNAS Early Edition 2013, 1-6.

In an embodiment, a Cas9 molecule comprises an amino acid sequence having 60%, 65%, 70%, 75%, 80%, 85%, 90%, 95%, 96%, 97%, 98%, or 99% homology with; differs at no more than 1%, 2%, 5%, 10%, 15%, 20%, 30%, or 40% of the amino acid residues when compared with; differs by at least 1, 2, 5, 10 or 20 amino acids but by no more than 100, 80, 70, 60, 50, 40 or 30 amino acids from; or is identical to; *S. pyogenes* Cas9 (UniProt Q99ZW2). In embodiments, the Cas9 molecule is a *S. pyogenes* Cas9 variant, such as a variant described in Slaymaker et al., Science Express, available online December 1, 2015 at Science DOI: 10.1126/science.aad5227; Kleinstiver et al., Nature, 529, 2016, pp. 490-495, available online January 6, 2016 at doi:10.1038/nature16526; or US2016/0102324. In an embodiment, the Cas9 molecule is catalytically inactive, e.g., dCas9. Tsai et al. (2014), Nat. Biotech. 32:569-577; U.S. Patent No.: 8,871,445; 8,865,406; 8,795,965; 8,771,945; and 8,697,359. A catalytically inactive Cas9, e.g., dCas9, molecule may be fused with a transcription modulator, e.g., a transcription repressor or transcription activator.

In embodiments, the Cas9 molecule, e.g, a Cas9 of S. *pyogenes,* may additionally comprise one or more amino acid sequences that confer additional activity. In some aspects, the Cas9 molecule may comprise one or more nuclear localization sequences (NLSs), such as at least 1, 2, 3, 4, 5, 6, 7, 8, 9, 10, or more NLSs. Typically, an NLS consists of one or more short sequences of positively charged lysines or arginines exposed on the protein surface, but other types of NLS are known. Non-limiting examples of NLSs include an NLS sequence comprising or derived from: the NLS of the SV40 virus large T-antigen, having the amino acid sequence PKKKRKV (SEQ ID NO: 864). Other suitable NLS sequences are known in the art (e.g., Sorokin, Biochemistry (Moscow) (2007) 72:13, 1439-1457; Lange J Biol Chem. (2007) 282:8, 5101-5). In any of the aforementioned embodiments, the Cas9 molecule may additionally (or alternatively) comprise a tag, e.g., a His tag, e.g., a His(6) tag (SEQ ID NO: 865) or His(8) tag (SEQ ID NO: 866), e.g., at the N terminus and/or the C terminus.

Thus, engineered CRISPR gene editing systems, e.g., for gene editing in eukaryotic cells, typically involve (1) a guide RNA molecule (gRNA) comprising a targeting domain (which is capable of hybridizing to the genomic DNA target sequence), and sequence which is capable of binding to a Cas, e.g., Cas9 enzyme, and (2) a Cas, e.g., Cas9, protein. This second domain may comprise a domain referred to as a tracr domain. The targeting domain and the sequence which is capable of binding to a Cas, e.g., Cas9 enzyme, may be disposed on the same (sometimes referred to as a single gRNA, chimeric gRNA or sgRNA) or different molecules (sometimes referred to as a dual guide RNA, dual gRNA or dgRNA). If disposed on different molecules, each includes a hybridization domain which allows the molecules to associate, e.g., through hybridization.
gRNA molecule formats are known in the art. An exemplary gRNA molecule, e.g., dgRNA molecule, of the present invention comprises, e.g., consists of, a first nucleic acid having the sequence: nnnnnnnnnnnnnnnnnnnnGUUUUAGAGCUAUGCUGUUUUG (SEQ ID NO: 867),
where the "n"'s refer to the residues of the targeting domain, e.g., a targeting domain to KDM1A, e.g., as described herein, and may consist of 15-25 nucleotides, e.g., consists of 20 nucleotides;
and a second nucleic acid sequence having the exemplary sequence:
AACUUACCAAGGAACAGCAUAGCAAGUUAAAAUAAGGCUAGUCCGUUAUCAACUUGAAAAAGUGGCACCGA GUCGGUGC, optionally with 1, 2, 3, 4, 5, 6, or 7 (e.g., 4 or 7, e.g., 7) additional U nucleotides at the 3' end (SEQ ID NO: 868).

The second nucleic acid molecule may alternatively consist of a fragment of the sequence above, wherein such fragment is capable of hybridizing to the first nucleic acid. An example of such second nucleic acid molecule is:
AACAGCAUAGCAAGUUAAAAUAAGGCUAGUCCGUUAUCAACUUGAAAAAGUGGCACCGAGUCGGU GC, optionally with 1, 2, 3, 4, 5, 6, or 7 (e.g., 4 or 7, e.g., 7) additional U nucleotides at the 3' end (SEQ ID NO: 869).

Another exemplary gRNA molecule, e.g., a sgRNA molecule, in the present invention comprises, e.g., consists of a first nucleic acid having the sequence:
nnnnnnnnnnnnnnnnnnnGUUUUAGAGCUAGAAAUAGCAAGUUAAAAUAAGGCUAGUCCGUUAUCA ACUUGAAAAAGUGGCACCGAGUCGGUGC (SEQ ID NO: 870), where the "n"'s refer to the residues of the targeting domain, e.g., a targeting domain to KDM1A, e.g., as described herein, and may consist of 15-25 nucleotides, e.g., consist of 20 nucleotides, optionally with 1, 2, 3, 4, 5, 6, or 7 (e.g., 4 or 7, e.g., 4) additional U nucleotides at the 3' end.

Exemplary sequences of gRNA molecule targeting domains useful in the present invention (e.g., which target an LSD1 gene, e.g., KDM1A) are provided in Table 2.

Additional components and/or elements of CRISPR gene editing systems known in the art, e.g., are described in U.S. Publication No.2014/0068797, WO2015/048577, and Cong (2013) Science 339: 819-823. Such systems can be generated which inhibit a target gene, by, for example, engineering a CRISPR gene editing system to include a gRNA molecule comprising a targeting domain that hybridizes to a sequence of the target gene. In embodiments, the gRNA comprises a targeting domain which is fully complementarity to 15-25 nucleotides, e.g., 20 nucleotides, of a target gene, e.g., KDM1A or its regulatory elements. In embodiments, the 15-25 nucleotides, e.g., 20 nucleotides, of the target gene, are disposed immediately 5' to a protospacer adjacent motif (PAM) sequence recognized by the RNA-guided nuclease, e.g., Cas protein, of the CRISPR gene editing system (e.g., where the system comprises a *S. pyogenes* Cas9 protein, the PAM sequence comprises NGG, where N can be any of A, T, G or C).

In embodiments, the gRNA molecule and RNA-guided nuclease, e.g., Cas protein, of the CRISPR gene editing system can be complexed to form a RNP complex. In other embodiments, nucleic acid encoding one or more components of the CRISPR gene editing system may be used.

In embodiments, foreign DNA can be introduced into the cell along with the CRISPR gene editing system, e.g., DNA encoding a desired transgene, with or without a promoter active in the target cell type. Depending on the sequences of the foreign DNA and target sequence of the genome, this process can be used to integrate the foreign DNA into the genome, at or near the site targeted by the CRISPR gene editing system. For example, 3' and 5' sequences flanking the transgene may be included in the foreign DNA which are homologous to the gene sequence 3' and 5' (respectively) of the site in the genome cut by the gene editing system. Such foreign DNA molecule can be referred to "template DNA."

In an embodiment, the CRISPR gene editing system in the present invention comprises Cas9, e.g., S. pyogenes Cas9, and a gRNA comprising a targeting domain which hybridizes to a sequence of a gene of interest, e.g., KDM1A. In an embodiment, the gRNA and Cas9 are complexed to form a RNP. In an embodiment, the CRISPR gene editing system comprises nucleic acid encoding a gRNA and nucleic acid encoding a Cas protein, e.g., Cas9, e.g., S. pyogenes Cas9. In an embodiment, the CRISPR gene editing system comprises a gRNA and nucleic acid encoding a Cas protein, e.g., Cas9, e.g., S. pyogenes Cas9.

In an exemplary embodiment, the genome editing system LSD1 inhibitor is a CRISPR system. CRISPR genome editing systems useful in the practice of this invention are described in, for example, Artificial CRISPR/Cas systems can be generated which inhibit LSD1, using technology known in the art, e.g., that are described in U.S. Publication No. 20140068797, and Cong (2013) Science 339: 819-823. Other artificial CRISPR/Cas systems that are known in the art may also be generated which inhibit TCR and/or HLA, e.g., that described in Tsai (2014) Nature Biotechnol., 32:6 569-576, U.S. Patent No.: 8,871,445; 8,865,406; 8,795,965; 8,771,945; and 8,697,359.

**Table 2: Exemplary LSD1 gRNA targeting domain sequences.**

| **Target Gene** | **gRNA Targeting Domain Sequence** | **SEQ ID NO:** |
|---|---|---|
| KDM1A | AGUGCGACAGGUUCGCUACA | 132 |
| KDM1A | UGGAAUAGCAGAGACUCCGG | 133 |
| KDM1A | CUAAAUAACUGUGAACUCGG | 134 |
| KDM1A | UGCUAUUCCAGUUUCCAUGG | 135 |
| KDM1A | CAGACCCAGGCACGACAGUA | 136 |
| KDM1A | UUUCUGAAACAGGAUCGUGU | 137 |
| KDM1A | UGAGAAGUCAUCCGGUCAUG | 138 |
| KDM1A | GAAUUGCAGACCAGUUUUUG | 139 |
| KDM1A | CGAGUUGCCACAUUUCGCAA | 140 |
| KDM1A | CUGAAACAGGAUCGUGUGGG | 141 |
| KDM1A | GGCGGCCCGAGAUGUUAUCU | 142 |
| KDM1A | UGUAUACCACACCUUGCAUA | 143 |
| KDM1A | AUGUAUACCACACCUUGCAU | 144 |
| KDM1A | UCUUAGUGAAAAGCAAACAC | 145 |
| KDM1A | GGGAUUUGGCAACCUUAACA | 146 |
| KDM1A | UUGUGCCACCUCUCCCUGAG | 147 |
| KDM1A | ACCAAGACCUGUUACCACCA | 148 |
| KDM1A | UCGGCAGUAAUAUCUCUGGG | 149 |
| KDM1A | GGAUCUGACCGCCCUAUGCA | 150 |
| KDM1A | CGUCAUGGUCUUAUCAACUU | 151 |
| KDM1A | UUCAAGACGACAGUUCUGGA | 152 |
| KDM1A | UGAAGUCUAGUUGAUGACUA | 153 |
| KDM1A | CACAGUUAUUUAGAGCGUCA | 154 |
| KDM1A | UGCCAACAGUAUUGGAGCUG | 155 |
| KDM1A | AGUGCCAACAGUAUUGGAGC | 156 |
| KDM1A | GCAUCUGUCUCACAUGCUUG | 157 |
| KDM1A | ACAUCUGCAGUCCAAAGGAU | 158 |
| KDM1A | GGCACAAACUGAACGGCUGG | 159 |
| KDM1A | GGUAAUUAUUAUAGGCUCUG | 160 |
| KDM1A | AAGAUGAGCAGAUUGAACAU | 161 |
| KDM1A | UGGCAGUACGACUGCCAGCA | 162 |
| KDM1A | CGCGGAGGCUCUUUCUUGCG | 163 |
| KDM1A | AUAAGUGACGAUGUGAUUGU | 164 |
| KDM1A | GGCAGUACGACUGCCAGCAG | 165 |
| KDM1A | GGAAAACAUCUGCAGUCCAA | 166 |
| KDM1A | UGCCCGAACAAAUUGACACU | 167 |
| KDM1A | ACUGAAUACAGCAGUGCGAC | 168 |
| KDM1A | CUGGCAGCCACCUGACAGUA | 169 |
| KDM1A | GCAGACCCAGGCACGACAGU | 170 |
| KDM1A | UUUGCUUUUCACUAAGAACU | 171 |
| KDM1A | GGGACACAGGCUUAUUAUUG | 172 |
| KDM1A | UGGCUACUCGUGUGUGCCUG | 173 |
| KDM1A | UCUCCCGCAAAGAAGAGUCG | 174 |
| KDM1A | AACAUCUGCAGUCCAAAGGA | 175 |
| KDM1A | CUUAGUGAAAAGCAAACACA | 176 |
| KDM1A | CCCAGGCACGACAGUAGGGC | 177 |
| KDM1A | GUCAAUUUGUUCGGGCAUGU | 178 |
| KDM1A | GAAUAAGAGCCCCGAGCCCA | 179 |
| KDM1A | CUGCUAUUCCAGUUUCCAUG | 180 |
| KDM1A | GAGAGGUGGCACAAACUGAA | 181 |
| KDM1A | AAGUGAGCCUGAAGAACCAU | 182 |
| KDM1A | UUUGCUUUCUUCGUUAGGUG | 183 |
| KDM1A | AUUUCUGAAACAGGAUCGUG | 184 |
| KDM1A | ACGACAGUAGGGCCGGCCUG | 185 |
| KDM1A | GUCCGUUGGCUUCAUAAAGU | 186 |
| KDM1A | AUACCACACCUUGCAUAGGG | 187 |
| KDM1A | GCUCUAAAUAACUGUGAACU | 188 |
| KDM1A | AGGAGGGAAUCCUAUGGCUG | 189 |
| KDM1A | AUAAAUAAAGGUUUGACUCG | 190 |
| KDM1A | AGCUGAUCUUGGAGCCAUGG | 191 |
| KDM1A | UAAAGGUUUGACUCGUGGAG | 192 |
| KDM1A | UGGACCACAACAGACCCAGA | 193 |
| KDM1A | GAUGAAUUAGCUGAAACACA | 194 |
| KDM1A | AGUAGCCAUUCCUUACUGUC | 195 |
| KDM1A | CUGGCGAGGCAGCGUAUACA | 196 |
| KDM1A | GUUUUCCACUCAGGGAGAGG | 197 |
| KDM1A | CGGAACCGCCGGGGUCCGCA | 198 |
| KDM1A | AGUGAGCCUGAAGAACCAUC | 199 |
| KDM1A | UCAACUUCGGCAUCUAUAAG | 200 |
| KDM1A | GUGGGGCACCUGGAAUCGAG | 201 |
| KDM1A | GGAAUAGCAGAGACUCCGGA | 202 |
| KDM1A | AGCCACCUGACAGUAAGGAA | 203 |
| KDM1A | AUAGUUUCCUUUGCGAAAUG | 204 |
| KDM1A | AGUUGGAAGCGAAUCCCCCA | 205 |
| KDM1A | AUGGCUCAGCCAAUCACUCC | 206 |
| KDM1A | AGCGGCAGCAACCGGGACGG | 207 |
| KDM1A | CUUUUAGCCCAAAGAAACUG | 208 |
| KDM1A | AAGAUGUAGCUUCUAGCAAC | 209 |
| KDM1A | AAUAAUACUCAUCUUCUGAG | 210 |
| KDM1A | CCGGCCCUACUGUCGUGCCU | 211 |
| KDM1A | CUGCUUCUUGAGAAGUCAUC | 212 |
| KDM1A | AGAGCCGACUUCCUCAUGAC | 213 |
| KDM1A | AGGCCUUCUGCUAAAGCCAC | 214 |
| KDM1A | GAAUGACAACUUCCAAUGCC | 215 |
| KDM1A | UGGUUAAAAGGACACUGUCA | 216 |
| KDM1A | UGCUGCUUCAGCACACCCAG | 217 |
| KDM1A | CCGCAAGAAAGAGCCUCCGC | 218 |
| KDM1A | AACUUCCAAUGCCUGGCCAA | 219 |
| KDM1A | AAAGGUUUGACUCGUGGAGC | 220 |
| KDM1A | GUGCCAACAGUAUUGGAGCU | 221 |
| KDM1A | UCAUCCGGUCAUGAGGAAGU | 222 |
| KDM1A | GCCACUAGUGCCAACAGUAU | 223 |
| KDM1A | UAGGGCAAGCUACCUUGUUA | 224 |
| KDM1A | GAUGCCUGGCCAUUCUCAAA | 225 |
| KDM1A | CAACUCUCUCCCUUAAGCAC | 226 |
| KDM1A | UGUUUUCCAGAUAUUAUCAG | 227 |
| KDM1A | CUUCAAGACGACAGUUCUGG | 228 |
| KDM1A | GAUUCCACGACUCUUCUUUG | 229 |
| KDM1A | AUGCAUCUGUCUCACAUGCU | 230 |
| KDM1A | AACUCUCUCCCUUAAGCACU | 231 |
| KDM1A | UCGACUUACAGCUUGUCCGU | 232 |
| KDM1A | UGGUAGCAGUGCAGUACCUC | 233 |
| KDM1A | AGGAGGUCCUUACUUGGUAG | 234 |
| KDM1A | AUAGG UAGAG UACAGAGAGA | 235 |
| KDM1A | GCACUGUGGCUGGGUAGUUA | 236 |
| KDM1A | UUUUCUAGGAGGGAAUCCUA | 237 |
| KDM1A | CGGACCCCGGCGGUUCCGCC | 238 |
| KDM1A | UGCCCACUUUAUGAAGCCAA | 239 |
| KDM1A | GAGCACCAUGCACUGUGGCU | 240 |
| KDM1A | GCAAAGAAGAGUCGUGGAAU | 241 |
| KDM1A | CUCCCUUAAGCACUGGGAUC | 242 |
| KDM1A | AGAUGAUCCUGCAGCAACAU | 243 |
| KDM1A | CCCGCGGAGGCUCUUUCUUG | 244 |
| KDM1A | UCUGCUAUUCCAGUUUCCAU | 245 |
| KDM1A | UGUGGUCCACUGAUAAUAUC | 246 |
| KDM1A | AAUUGCAGACCAGUUUUUGG | 247 |
| KDM1A | UUUAGCUAAAAAGACAGGAA | 248 |
| KDM1A | ACAGCAGGCGGCUCCGAGAA | 249 |
| KDM1A | AUGUCACACUUUUGGAAGCC | 250 |
| KDM1A | UUCUUCCCAGAUAACAUCUC | 251 |
| KDM1A | UCGUUGGCGUGCUGAUCCCU | 252 |
| KDM1A | GCUGAGUGGGCUGCGAGAAG | 253 |
| KDM1A | UCGGACCAGCCGGCGCAAGC | 254 |
| KDM1A | CAUGUAUACGCUGCCUCGCC | 255 |
| KDM1A | GGUUCCGCCAGGCCCCCGGG | 256 |
| KDM1A | UGUGAUUGUUGGCCGAUGCC | 257 |
| KDM1A | AGCCAUUCCUUACUGUCAGG | 258 |
| KDM1A | AAUAAUAAGCCUGUGUCCCU | 259 |
| KDM1A | UCCAAUACUGUUGGCACUAG | 260 |
| KDM1A | UCCAUGGGGGUCGCAGACCC | 261 |
| KDM1A | GGUGGCACAAACUGAACGGC | 262 |
| KDM1A | AAAUCCCUUUGAGAAUGGCC | 263 |
| KDM1A | UGUAGCUGAUCUUGGAGCCA | 264 |
| KDM1A | UCUUAUUCCUAUGUUGCUGC | 265 |
| KDM1A | AACUGGAAUAGCAGAGACUC | 266 |
| KDM1A | UGCAGCGGCAGCAACCGGGA | 267 |
| KDM1A | UAUACAUGGCCCCCAAAAAC | 268 |
| KDM1A | AAUGCCUGGCCAAGGGACAC | 269 |
| KDM1A | AAGAAACUGUGGUGUCUCGU | 270 |
| KDM1A | UGAGGCCCUGCGGACCCCGG | 271 |
| KDM1A | UUUAAAUGCAGCUGCAGUUG | 272 |
| KDM1A | GUGUUUUGAUCGGGUGUUCU | 273 |
| KDM1A | GAUGAUGACUUUGAGUUCAC | 274 |
| KDM1A | AGGUAAUUAUUAUAGGCUCU | 275 |
| KDM1A | GGACU UCAAGACGACAG U UC | 276 |
| KDM1A | GGGGCCUGGCGGAACCGCCG | 277 |
| KDM1A | GAUGUUUUCCACUCAGGGAG | 278 |
| KDM1A | AUCCAAGUGUCAAUUUGUUC | 279 |
| KDM1A | AAUGCAGCUGCAGUUGUGGU | 280 |
| KDM1A | CAGCAGAGCACCAUGCACUG | 281 |
| KDM1A | CCU UCCACAGGGCAAGCAGG | 282 |
| KDM1A | CUGAGUGGGCUGCGAGAAGC | 283 |
| KDM1A | CUUUAUAGAGGUUCCAGAAG | 284 |
| KDM1A | AACUCGGCAGUAAUAUCUCU | 285 |
| KDM1A | ACAGUUACAAAGUUUUGGAA | 286 |
| KDM1A | CAACUUCCAAUGCCUGGCCA | 287 |
| KDM1A | ACUGCAGAUGUUUUCCACUC | 288 |
| KDM1A | AACUACCCAGCCACAGUGCA | 289 |
| KDM1A | CUCCGAGAACGGGUCUGAGG | 290 |
| KDM1A | AAAACCUUCUGGGUCUGUUG | 291 |
| KDM1A | UAUAAGGUGCUUCUAAUUGU | 292 |
| KDM1A | UGGGCAUUUUUGCUUGAUCU | 293 |
| KDM1A | UUCCCCAGCUCCAAUACUGU | 294 |
| KDM1A | CAGCAGGCGGCUCCGAGAAC | 295 |
| KDM1A | GGUUGCCAAAUCCCAUCCUU | 296 |
| KDM1A | GACGACAGUUCUGGAGGGUA | 297 |
| KDM1A | AUACUGUUGGCACUAGUGGC | 298 |
| KDM1A | CUUCAGCACACCCAGGGGAA | 299 |
| KDM1A | UGGUAGAGCAAGAGUUUAAC | 300 |
| KDM1A | CCCGCAAGAAAGAGCCUCCG | 301 |
| KDM1A | UGUGGGGCACCUGGAAUCGA | 302 |
| KDM1A | CGGCUCCGAGAACGGGUCUG | 303 |
| KDM1A | GUGCCUGUGGCUUUAGCAGA | 304 |
| KDM1A | UUGCCAGCUUCUCACCUGUG | 305 |
| KDM1A | GUUUGUUUUCCUUACCUUGG | 306 |
| KDM1A | CAGGUUCGCUACACGGCUUC | 307 |
| KDM1A | AACUCACCCGAUGGUUCUUC | 308 |
| KDM1A | GCUUUCUUCGUUAGGUGUGG | 309 |
| KDM1A | CAAACAAGUAAAUAUGGAAC | 310 |
| KDM1A | ACCCCCUCAAGCCCCACCUG | 311 |
| KDM1A | GCAGCAACCGGGACGGAGGC | 312 |
| KDM1A | AGGAGAAGCUGCUGGUAUCA | 313 |
| KDM1A | UGUUUGCUGACCACAGCCAU | 314 |
| KDM1A | AAAGAUUCAGCUGACAUUUG | 315 |
| KDM1A | UGUCCGUUGGCUUCAUAAAG | 316 |
| KDM1A | CUUGGAGCCAUGGUGGUAAC | 317 |
| KDM1A | AGUUCUGGAGGGUAUGGAGA | 318 |
| KDM1A | CCGCGGGCCUCGCCCCCCGG | 319 |
| KDM1A | CGGCGGCCCGAGAUGUUAUC | 320 |
| KDM1A | UUGCUUUUAGCUAAAAAGAC | 321 |
| KDM1A | GAAUAGCAGAGACUCCGGAG | 322 |
| KDM1A | UUACCUGAUCCCAGUGCUUA | 323 |
| KDM1A | CUGUGGGGCACCUGGAAUCG | 324 |
| KDM1A | CUGUCGGGCCCAGCCGAGGU | 325 |
| KDM1A | AGGGCCGGCCUGAGGCCCUG | 326 |
| KDM1A | CUGCAGAUGUUUUCCACUCA | 327 |
| KDM1A | GAUGUUAUCUGGGAAGAAGG | 328 |
| KDM1A | CUCUGCUAUUCCAGUUUCCA | 329 |
| KDM1A | GAAGGCAUUUACCUUUAUAG | 330 |
| KDM1A | GAAGAAGAUAGUGAAAACUC | 331 |
| KDM1A | GCCGGCCCUACUGUCGUGCC | 332 |
| KDM1A | GGUGGUGUUGUGUUUUGAUC | 333 |
| KDM1A | GCCUGGGUCUGCGACCCCCA | 334 |
| KDM1A | CUGUAUUCAGUUUAAUGUCU | 335 |
| KDM1A | GAACUCGGCAGUAAUAUCUC | 336 |
| KDM1A | CUUUCUUCGUUAGGUGUGGA | 337 |
| KDM1A | CUAGUGGCAGGAGAAGCUGC | 338 |
| KDM1A | CAGGAACACCUGGUGUGGCC | 339 |
| KDM1A | UGCGUCGCAUUUAUAAAUAA | 340 |
| KDM1A | CGAGAUGUUAUCUGGGAAGA | 341 |
| KDM1A | GCUCGACAGUUACAAAGUUU | 342 |
| KDM1A | CGAUGCCUGGCCAUUCUCAA | 343 |
| KDM1A | AAGUCCUCCUGCUUGCCCUG | 344 |
| KDM1A | GGCCACCUCAGACCCGUUCU | 345 |
| KDM1A | AUUGAAAGAACUUCUUAAUA | 346 |
| KDM1A | GCCAUGGUGGUAACAGGUCU | 347 |
| KDM1A | UAGGAGGUCCUUACUUGGUA | 348 |
| KDM1A | GGAAACUAUGUAGCUGAUCU | 349 |
| KDM1A | UUCCUCAGGUGGGGCUUGAG | 350 |
| KDM1A | CUCGAUUCCAGGUGCCCCAC | 351 |
| KDM1A | ACCAAAAAUCCCUUUGAGAA | 352 |
| KDM1A | GGGCCUGUCGGGCCCAGCCG | 353 |
| KDM1A | CGGUUCCGCCAGGCCCCCGG | 354 |
| KDM1A | AAGGUAAUUAUUAUAGGCUC | 355 |
| KDM1A | GUUCUCUGUACCCUUCCCCU | 356 |
| KDM1A | AUACUCAUCUUCUGAGAGGU | 357 |
| KDM1A | CGAGACACCACAGUUUCUUU | 358 |
| KDM1A | GGUCAGCAAACAAGUAAAUA | 359 |
| KDM1A | AGAAUUGCAGACCAGUUUUU | 360 |
| KDM1A | UGUGUUUUGAUCGGGUGUUC | 361 |
| KDM1A | GCUUCAGCACACCCAGGGGA | 362 |
| KDM1A | GUAGGAGGUCCUUACUUGGU | 363 |
| KDM1A | UUGGCAGUACGACUGCCAGC | 364 |
| KDM1A | GGAGAGAGUUGAGAGAGGUG | 365 |
| KDM1A | GUCGGACCAGCCGGCGCAAG | 366 |
| KDM1A | AGAAGAAAAACUUCAGGAGU | 367 |
| KDM1A | GCUGGCCCUGGGACAGCAGG | 368 |
| KDM1A | CAGAGAGAUGGAUGAAAGCU | 369 |
| KDM1A | UUAAGGGAGAGAGUUGAGAG | 370 |
| KDM1A | GGGGGCCUGGCGGAACCGCC | 371 |
| KDM1A | UACCUGAUCCCAGUGCUUAA | 372 |
| KDM1A | UGGGAAGAAGGCGGCAGCCG | 373 |
| KDM1A | GCUGGGCCCGACAGGCCCGC | 374 |
| KDM1A | CUCUUCUGGAACCUCUAUAA | 375 |
| KDM1A | ACUGAUAAUAUCUGGAAAAC | 376 |
| KDM1A | CGGAGGGGCGUCGGACCAGC | 377 |
| KDM1A | UGUCACACUUUUGGAAGCCA | 378 |
| KDM1A | GCGCAGCCCGCGGGCCUGUC | 379 |
| KDM1A | AUUCCACGACUCUUCUUUGC | 380 |
| KDM1A | AUGCUUCUUUGUAUUGCUGA | 381 |
| KDM1A | CUUUGAGAAUGGCCAGGCAU | 382 |
| KDM1A | AAGAAUUGCAGACCAGUUUU | 383 |
| KDM1A | CUGCCUCGCCAGGCCACACC | 384 |
| KDM1A | CUGUGCAGGAACACCUGGUG | 385 |
| KDM1A | GGAAUAAGAGCCCCGAGCCC | 386 |
| KDM1A | CCGGGACGGAGGCUGGCCCU | 387 |
| KDM1A | CGGAGCCGCCUGCUGUCCCA | 388 |
| KDM1A | CAGAGACUCCGGAGGGGCGU | 389 |
| KDM1A | CUGCUGCUUCAGCACACCCA | 390 |
| KDM1A | GUGCAUGGUGCUCUGCUGAG | 391 |
| KDM1A | ACAGGAAAGGUAAUUAUUAU | 392 |
| KDM1A | UUUCCUUCCACAGGGCAAGC | 393 |
| KDM1A | CAGCCGGCGCAAGCGGGCGA | 394 |
| KDM1A | UGCAUGGUGCUCUGCUGAGU | 395 |
| KDM1A | GCUGCUGCUUCAGCACACCC | 396 |
| KDM1A | UGUCGGGCCCAGCCGAGGUC | 397 |
| KDM1A | CGUGCUGAUCCCUGGGCUCG | 398 |
| KDM1A | GCGGUUCCGCCAGGCCCCCG | 399 |
| KDM1A | ACACACUACUUACUGUUAUA | 400 |
| KDM1A | UAAGCCUGUGUCCCUUGGCC | 401 |
| KDM1A | GCAGUCCAAAGGAUGGGAUU | 402 |
| KDM1A | GGCCCAGCCGAGGUCGGGCC | 403 |
| KDM1A | ACACCUGGUGUGGCCUGGCG | 404 |
| KDM1A | GAUCCAAGUGUCAAUUUGUU | 405 |
| KDM1A | UCAAAUGUCAGCUGAAUCUU | 406 |
| KDM1A | GGGCCCAGCCGAGGUCGGGC | 407 |
| KDM1A | CGACAGGCCCGCGGGCUGCG | 408 |
| KDM1A | GGUCAUGAGGAAGUCGGCUC | 409 |
| KDM1A | UCCGCGGGCCUCGCCCCCCG | 410 |
| KDM1A | CUCGUUGGCGUGCUGAUCCC | 411 |
| KDM1A | ACGAGACACCACAGUUUCUU | 412 |
| KDM1A | UGUGUCCCUUGGCCAGGCAU | 413 |
| KDM1A | GAGGAAGAGCUCACCCCUGC | 414 |
| KDM1A | CGGCUGCAGCGGCAGCAACC | 415 |
| KDM1A | AGUUCUCUGUACCCUUCCCC | 416 |
| KDM1A | CCGCGGAGGCUCUUUCUUGC | 417 |
| KDM1A | GAGAAAUGCCAAAGCAGAGA | 418 |
| KDM1A | GAAGAAGGCGGCAGCCGCGG | 419 |
| KDM1A | CCCCCGGCCCGACCUCGGCU | 420 |
| KDM1A | GGCUGGGCCCGACAGGCCCG | 421 |
| KDM1A | UCUGCGACCCCCAUGGAAAC | 422 |
| KDM1A | GCCUGAGGCCCUGCGGACCC | 423 |
| KDM1A | AGGUGGUGUUGUGUUUUGAU | 424 |
| KDM1A | UUCAUUUUCUUCCUCAGGUG | 425 |
| KDM1A | UUACCUUCGCCCGCUUGCGC | 426 |
| KDM1A | GCCCAGCCGAGGUCGGGCCG | 427 |
| KDM1A | CCCAGGGCCAGCCUCCGUCC | 428 |
| KDM1A | CCGGGGGGCGAGGCCCGCGG | 429 |
| KDM1A | UCUUCCUCAGGUGGGGCUUG | 430 |
| KDM1A | CGACCUCGGCUGGGCCCGAC | 431 |
| KDM1A | UAUGGAGACGGCCAAGCAUC | 432 |
| KDM1A | UAUGUUGCUGCAGGAUCAUC | 433 |
| KDM1A | CUUCUUCCCAGAUAACAUCU | 434 |
| KDM1A | GCCAUUCUCAAAGGGAUUUU | 435 |
| KDM1A | GGCGUGCUGAUCCCUGGGCU | 436 |
| KDM1A | CUCGCCCCCCGGGGGCCUGG | 437 |
| KDM1A | GAGACAGACAAAUACUUGAU | 438 |
| KDM1A | GGGUCCGCAGGGCCUCAGGC | 439 |
| KDM1A | ACUCCUGGCCCCUCGAUUCC | 440 |
| KDM1A | CGCGCAGCCCGCGGGCCUGU | 441 |
| KDM1A | GUGCGCUCCCCCACCGCCCC | 442 |
| KDM1A | CUUUCAUUUUCUUCCUCAGG | 443 |
| KDM1A | GAGGCUGGCCCUGGGACAGC | 444 |
| KDM1A | UGCCACCCUCACCUGAUGCU | 445 |
| KDM1A | GAGGUGGCCGCGCAGCCCGC | 446 |
| KDM1A | UUUCAUUUUCUUCCUCAGGU | 447 |
| KDM1A | AUCGAGGGGCCAGGAGUGAU | 448 |
| KDM1A | CGGCUGGUCCGACGCCCCUC | 449 |
| KDM1A | CGGGGGCCUGGCGGAACCGC | 450 |
| KDM1A | CUUCUCACCUGUGGGGCACC | 451 |
| KDM1A | AGGUCGGGCCGGGGGCGGUG | 452 |
| KDM1A | CGCUGCAGCCGCCGCCGCCG | 453 |
| KDM1A | AGAGCACCAUGCACUGUGGC | 454 |
| KDM1A | CCCAGCCGAGGUCGGGCCGG | 455 |
| KDM1A | GACUGCUGUGCAGGAACACC | 456 |
| KDM1A | AGGCUCUGGGGUCUCAGGCU | 457 |
| KDM1A | UGAGGUGGCCGCGCAGCCCG | 458 |
| KDM1A | GCGUGCUGAUCCCUGGGCUC | 459 |
| KDM1A | CUUCGCCCGCUUGCGCCGGC | 460 |
| KDM1A | GCACCUGGAAUCGAGGGGCC | 461 |
| KDM1A | UCCUAAAGAGAAAGAUGAAA | 462 |
| KDM1A | AUGCUUGGGGACUGCUGUGC | 463 |
| KDM1A | AAUGAAAAGAAAAACCUUCU | 464 |
| KDM1A | GCGGAACCGCCGGGGUCCGC | 465 |
| KDM1A | GAGGUCGGGCCGGGGGCGGU | 466 |
| KDM1A | AUUAUAGGCUCUGGGGUCUC | 467 |
| KDM1A | UCACUUUCAUUUUCUUCCUC | 468 |
| KDM1A | UCGGAGCCGCCUGCUGUCCC | 469 |
| KDM1A | AGCCGAGGUCGGGCCGGGGG | 470 |
| KDM1A | AAAGCUAGAAGAAAAACUUC | 471 |
| KDM1A | UCCUCAGGUGGGGCUUGAGG | 472 |
| KDM1A | CGGCGGUUCCGCCAGGCCCC | 473 |
| KDM1A | UGCAUCUGUCUCACAUGCUU | 474 |
| KDM1A | GGCGGUUCCGCCAGGCCCCC | 475 |
| KDM1A | ACCGGGACGGAGGCUGGCCC | 476 |
| KDM1A | CACCGCCCCCGGCCCGACCU | 477 |
| KDM1A | CGCCAGGCCCCCGGGGGGCG | 478 |
| KDM1A | GGGGUGAGCUCUUCCUCUUC | 479 |
| KDM1A | ACCAUUUCAUCUUUCUCUUU | 480 |
| KDM1A | GGCCUCGCCCCCCGGGGGCC | 481 |
| KDM1A | GCCCCCGGCCCGACCUCGGC | 482 |
| KDM1A | UGGGGCUUGAGGGGGUGGUG | 483 |
| KDM1A | GCCGGGGUCCGCAGGGCCUC | 484 |
| KDM1A | CUUCUUUUCCUUCUCUGCUU | 485 |
| KDM1A | CCUCCGCGGGCCUCGCCCCC | 486 |
| KDM1A | CUCCGCGGGCCUCGCCCCCC | 487 |
| KDM1A | GGUCGGGCCGGGGGCGGUGG | 488 |
| KDM1A | UGGAAUGGAUGUCACACUUU | 489 |
| KDM1A | CUUCCUCAGGUGGGGCUUGA | 490 |
| KDM1A | GUGGGGCUUGAGGGGGUGGU | 491 |
| KDM1A | GCGGCUGCAGCGGCAGCAAC | 492 |
| KDM1A | CCCCCGGGGGGCGAGGCCCG | 493 |
| KDM1A | UAAUGAAAAGAAAAACCUUC | 494 |
| KDM1A | CGCGGCGGCGGCGGCUGCAG | 495 |
| KDM1A | UCAGGUGGGGCUUGAGGGGG | 496 |
| KDM1A | GGUGGGGCUUGAGGGGGUGG | 497 |
| KDM1A | UGGAAAUGACUAUGAUUUAA | 498 |
| KDM1A | GGGGCUUGAGGGGGUGGUGG | 499 |
| KDM1A | CGAGGUCGGGCCGGGGGCGG | 500 |
| KDM1A | GGCGGCAGCCGCGGCGGCGG | 501 |
| KDM1A | GAAGGCGGCAGCCGCGGCGG | 502 |
| KDM1A | AGUGCGACAGGUUCGCUACA | 503 |
| KDM1A | UGGAAUAGCAGAGACUCCGG | 504 |
| KDM1A | CUAAAUAACUGUGAACUCGG | 505 |
| KDM1A | UGCUAUUCCAGUUUCCAUGG | 506 |
| KDM1A | CAGACCCAGGCACGACAGUA | 507 |
| KDM1A | UUUCUGAAACAGGAUCGUGU | 508 |
| KDM1A | UGAGAAGUCAUCCGGUCAUG | 509 |
| KDM1A | GAAUUGCAGACCAGUUUUUG | 510 |
| KDM1A | CGAGUUGCCACAUUUCGCAA | 511 |
| KDM1A | CUGAAACAGGAUCGUGUGGG | 512 |
| KDM1A | GGCGGCCCGAGAUGUUAUCU | 513 |
| KDM1A | UGUAUACCACACCUUGCAUA | 514 |
| KDM1A | UCUUAGUGAAAAGCAAACAC | 515 |
| KDM1A | AUGUAUACCACACCUUGCAU | 516 |
| KDM1A | GGGAUUUGGCAACCUUAACA | 517 |
| KDM1A | UUGUGCCACCUCUCCCUGAG | 518 |
| KDM1A | GGAUCUGACCGCCCUAUGCA | 519 |
| KDM1A | ACCAAGACCUGUUACCACCA | 520 |
| KDM1A | CGUCAUGGUCUUAUCAACUU | 521 |
| KDM1A | UGAAGUCUAGUUGAUGACUA | 522 |
| KDM1A | CACAGUUAUUUAGAGCGUCA | 523 |
| KDM1A | UCGGCAGUAAUAUCUCUGGG | 524 |
| KDM1A | UGCCAACAGUAUUGGAGCUG | 525 |
| KDM1A | GCAUCUGUCUCACAUGCUUG | 526 |
| KDM1A | AGUGCCAACAGUAUUGGAGC | 527 |
| KDM1A | ACAUCUGCAGUCCAAAGGAU | 528 |
| KDM1A | GGCACAAACUGAACGGCUGG | 529 |
| KDM1A | UGGCAGUACGACUGCCAGCA | 530 |
| KDM1A | AAGAUGAGCAGAUUGAACAU | 531 |
| KDM1A | GGUAAUUAUUAUAGGCUCUG | 532 |
| KDM1A | CGCGGAGGCUCUUUCUUGCG | 533 |
| KDM1A | AUAAGUGACGAUGUGAUUGU | 534 |
| KDM1A | GGCAGUACGACUGCCAGCAG | 535 |
| KDM1A | UGCCCGAACAAAUUGACACU | 536 |
| KDM1A | GGAAAACAUCUGCAGUCCAA | 537 |
| KDM1A | ACUGAAUACAGCAGUGCGAC | 538 |
| KDM1A | UUUGCUUUUCACUAAGAACU | 539 |
| KDM1A | CUGGCAGCCACCUGACAGUA | 540 |
| KDM1A | GCAGACCCAGGCACGACAGU | 541 |
| KDM1A | GGGACACAGGCUUAUUAUUG | 542 |
| KDM1A | UGGCUACUCGUGUGUGCCUG | 543 |
| KDM1A | AACAUCUGCAGUCCAAAGGA | 544 |
| KDM1A | CCCAGGCACGACAGUAGGGC | 545 |
| KDM1A | CUUAGUGAAAAGCAAACACA | 546 |
| KDM1A | UCUCCCGCAAAGAAGAGUCG | 547 |
| KDM1A | GUCAAUUUGUUCGGGCAUGU | 548 |
| KDM1A | CUGCUAUUCCAGUUUCCAUG | 549 |
| KDM1A | GAAUAAGAGCCCCGAGCCCA | 550 |
| KDM1A | AAGUGAGCCUGAAGAACCAU | 551 |
| KDM1A | UUUGCUUUCUUCGUUAGGUG | 552 |
| KDM1A | AUUUCUGAAACAGGAUCGUG | 553 |
| KDM1A | GAGAGGUGGCACAAACUGAA | 554 |
| KDM1A | GUCCGUUGGCUUCAUAAAGU | 555 |
| KDM1A | ACGACAGUAGGGCCGGCCUG | 556 |
| KDM1A | AUACCACACCUUGCAUAGGG | 557 |
| KDM1A | GCUCUAAAUAACUGUGAACU | 558 |
| KDM1A | AGGAGGGAAUCCUAUGGCUG | 559 |
| KDM1A | AUAAAUAAAGGUUUGACUCG | 560 |
| KDM1A | CUGGCGAGGCAGCGUAUACA | 561 |
| KDM1A | AGCUGAUCUUGGAGCCAUGG | 562 |
| KDM1A | UAAAGGUUUGACUCGUGGAG | 563 |
| KDM1A | AGUAGCCAUUCCUUACUGUC | 564 |
| KDM1A | GUUUUCCACUCAGGGAGAGG | 565 |
| KDM1A | UGGACCACAACAGACCCAGA | 566 |
| KDM1A | GAUGAAUUAGCUGAAACACA | 567 |
| KDM1A | AGUGAGCCUGAAGAACCAUC | 568 |
| KDM1A | CGGAACCGCCGGGGUCCGCA | 569 |
| KDM1A | UCAACUUCGGCAUCUAUAAG | 570 |
| KDM1A | GUGGGGCACCUGGAAUCGAG | 571 |
| KDM1A | AUAGUUUCCUUUGCGAAAUG | 572 |
| KDM1A | GGAAUAGCAGAGACUCCGGA | 573 |
| KDM1A | AGCCACCUGACAGUAAGGAA | 574 |
| KDM1A | AUGGCUCAGCCAAUCACUCC | 575 |
| KDM1A | AGCGGCAGCAACCGGGACGG | 576 |
| KDM1A | AGUUGGAAGCGAAUCCCCCA | 577 |
| KDM1A | CUUUUAGCCCAAAGAAACUG | 578 |
| KDM1A | AAGAUGUAGCUUCUAGCAAC | 579 |
| KDM1A | CCGGCCCUACUGUCGUGCCU | 580 |
| KDM1A | AGAGCCGACUUCCUCAUGAC | 581 |
| KDM1A | AAUAAUACUCAUCUUCUGAG | 582 |
| KDM1A | CUGCUUCUUGAGAAGUCAUC | 583 |
| KDM1A | AGGCCUUCUGCUAAAGCCAC | 584 |
| KDM1A | GAAUGACAACUUCCAAUGCC | 585 |
| KDM1A | CCGCAAGAAAGAGCCUCCGC | 586 |
| KDM1A | UGCUGCUUCAGCACACCCAG | 587 |
| KDM1A | AACUUCCAAUGCCUGGCCAA | 588 |
| KDM1A | AAAGGUUUGACUCGUGGAGC | 589 |
| KDM1A | GUGCCAACAGUAUUGGAGCU | 590 |
| KDM1A | GCCACUAGUGCCAACAGUAU | 591 |
| KDM1A | UAGGGCAAGCUACCUUGUUA | 592 |
| KDM1A | CAACUCUCUCCCUUAAGCAC | 593 |
| KDM1A | UCAUCCGGUCAUGAGGAAGU | 594 |
| KDM1A | GAUGCCUGGCCAUUCUCAAA | 595 |
| KDM1A | UGUUUUCCAGAUAUUAUCAG | 596 |
| KDM1A | AUGCAUCUGUCUCACAUGCU | 597 |
| KDM1A | GAUUCCACGACUCUUCUUUG | 598 |
| KDM1A | UGGUAGCAGUGCAGUACCUC | 599 |
| KDM1A | AACUCUCUCCCUUAAGCACU | 600 |
| KDM1A | UCGACUUACAGCUUGUCCGU | 601 |
| KDM1A | AGGAGGUCCUUACUUGGUAG | 602 |
| KDM1A | GCACUGUGGCUGGGUAGUUA | 603 |
| KDM1A | AUAGG UAGAG UACAGAGAGA | 604 |
| KDM1A | CGGACCCCGGCGGUUCCGCC | 605 |
| KDM1A | UUUUCUAGGAGGGAAUCCUA | 606 |
| KDM1A | GAGCACCAUGCACUGUGGCU | 607 |
| KDM1A | UGCCCACUUUAUGAAGCCAA | 608 |
| KDM1A | CUCCCUUAAGCACUGGGAUC | 609 |
| KDM1A | GCAAAGAAGAGUCGUGGAAU | 610 |
| KDM1A | CCCGCGGAGGCUCUUUCUUG | 611 |
| KDM1A | AGAUGAUCCUGCAGCAACAU | 612 |
| KDM1A | AAUUGCAGACCAGUUUUUGG | 613 |
| KDM1A | UGUGGUCCACUGAUAAUAUC | 614 |
| KDM1A | UUUAGCUAAAAAGACAGGAA | 615 |
| KDM1A | UCUGCUAUUCCAGUUUCCAU | 616 |
| KDM1A | ACAGCAGGCGGCUCCGAGAA | 617 |
| KDM1A | AUGUCACACUUUUGGAAGCC | 618 |
| KDM1A | UUCUUCCCAGAUAACAUCUC | 619 |
| KDM1A | CAUGUAUACGCUGCCUCGCC | 620 |
| KDM1A | GCUGAGUGGGCUGCGAGAAG | 621 |
| KDM1A | UCGUUGGCGUGCUGAUCCCU | 622 |
| KDM1A | UCGGACCAGCCGGCGCAAGC | 623 |
| KDM1A | AGCCAUUCCUUACUGUCAGG | 624 |
| KDM1A | UGUGAUUGUUGGCCGAUGCC | 625 |
| KDM1A | AAUAAUAAGCCUGUGUCCCU | 626 |
| KDM1A | GGUUCCGCCAGGCCCCCGGG | 627 |
| KDM1A | UCCAAUACUGUUGGCACUAG | 628 |
| KDM1A | UGUAGCUGAUCUUGGAGCCA | 629 |
| KDM1A | UCCAUGGGGGUCGCAGACCC | 630 |
| KDM1A | AAAUCCCUUUGAGAAUGGCC | 631 |
| KDM1A | GGUGGCACAAACUGAACGGC | 632 |
| KDM1A | AACUGGAAUAGCAGAGACUC | 633 |
| KDM1A | UGCAGCGGCAGCAACCGGGA | 634 |
| KDM1A | UCUUAUUCCUAUGUUGCUGC | 635 |
| KDM1A | UAUACAUGGCCCCCAAAAAC | 636 |
| KDM1A | AAUGCCUGGCCAAGGGACAC | 637 |
| KDM1A | AAGAAACUGUGGUGUCUCGU | 638 |
| KDM1A | UUUAAAUGCAGCUGCAGUUG | 639 |
| KDM1A | UGAGGCCCUGCGGACCCCGG | 640 |
| KD M 1A | AGGUAAUUAUUAUAGGCUCU | 641 |
| KDM1A | GUGUUUUGAUCGGGUGUUCU | 642 |
| KDM1A | GAUGAUGACUUUGAGUUCAC | 643 |
| KDM1A | AUCCAAGUGUCAAUUUGUUC | 644 |
| KDM1A | GGGGCCUGGCGGAACCGCCG | 645 |
| KDM1A | GAUGUUUUCCACUCAGGGAG | 646 |
| KDM1A | AAUGCAGCUGCAGUUGUGGU | 647 |
| KDM1A | CAGCAGAGCACCAUGCACUG | 648 |
| KDM1A | CUGAGUGGGCUGCGAGAAGC | 649 |
| KDM1A | CUUUAUAGAGGUUCCAGAAG | 650 |
| KDM1A | ACAGUUACAAAGUUUUGGAA | 651 |
| KDM1A | CAACUUCCAAUGCCUGGCCA | 652 |
| KDM1A | AACUCGGCAGUAAUAUCUCU | 653 |
| KDM1A | AACUACCCAGCCACAGUGCA | 654 |
| KDM1A | ACUGCAGAUGUUUUCCACUC | 655 |
| KDM1A | UAUAAGGUGCUUCUAAUUGU | 656 |
| KDM1A | CUCCGAGAACGGGUCUGAGG | 657 |
| KDM1A | AAAACCUUCUGGGUCUGUUG | 658 |
| KDM1A | UGGGCAUUUUUGCUUGAUCU | 659 |
| KDM1A | GGUUGCCAAAUCCCAUCCUU | 660 |
| KDM1A | CAGCAGGCGGCUCCGAGAAC | 661 |
| KDM1A | UUCCCCAGCUCCAAUACUGU | 662 |
| KDM1A | AUACUGUUGGCACUAGUGGC | 663 |
| KDM1A | CUUCAGCACACCCAGGGGAA | 664 |
| KDM1A | UGGUAGAGCAAGAGUUUAAC | 665 |
| KDM1A | CCCGCAAGAAAGAGCCUCCG | 666 |
| KDM1A | UGUGGGGCACCUGGAAUCGA | 667 |
| KDM1A | CGGCUCCGAGAACGGGUCUG | 668 |
| KDM1A | CAGGUUCGCUACACGGCUUC | 669 |
| KDM1A | UUGCCAGCUUCUCACCUGUG | 670 |
| KDM1A | GUUUGUUUUCCUUACCUUGG | 671 |
| KDM1A | GUGCCUGUGGCUUUAGCAGA | 672 |
| KDM1A | ACCCCCUCAAGCCCCACCUG | 673 |
| KDM1A | AACUCACCCGAUGGUUCUUC | 674 |
| KDM1A | GCUUUCUUCGUUAGGUGUGG | 675 |
| KDM1A | CAAACAAGUAAAUAUGGAAC | 676 |
| KDM1A | GCAGCAACCGGGACGGAGGC | 677 |
| KDM1A | AAAGAUUCAGCUGACAUUUG | 678 |
| KDM1A | UGUUUGCUGACCACAGCCAU | 679 |
| KDM1A | AGGAGAAGCUGCUGGUAUCA | 680 |
| KDM1A | UGUCCGUUGGCUUCAUAAAG | 681 |
| KDM1A | CUUGGAGCCAUGGUGGUAAC | 682 |
| KDM1A | CCGCGGGCCUCGCCCCCCGG | 683 |
| KDM1A | UUGCUUUUAGCUAAAAAGAC | 684 |
| KDM1A | CGGCGGCCCGAGAUGUUAUC | 685 |
| KDM1A | GAAUAGCAGAGACUCCGGAG | 686 |
| KDM1A | UUACCUGAUCCCAGUGCUUA | 687 |
| KDM1A | CUGUGGGGCACCUGGAAUCG | 688 |
| KDM1A | CUGUCGGGCCCAGCCGAGGU | 689 |
| KDM1A | AGGGCCGGCCUGAGGCCCUG | 690 |
| KDM1A | CUGCAGAUGUUUUCCACUCA | 691 |
| KDM1A | GAUGUUAUCUGGGAAGAAGG | 692 |
| KDM1A | GAAGGCAUUUACCUUUAUAG | 693 |
| KDM1A | CUCUGCUAUUCCAGUUUCCA | 694 |
| KDM1A | GAAGAAGAUAGUGAAAACUC | 695 |
| KDM1A | GCCGGCCCUACUGUCGUGCC | 696 |
| KDM1A | GGUGGUGUUGUGUUUUGAUC | 697 |
| KDM1A | CUGUAUUCAGUUUAAUGUCU | 698 |
| KDM1A | GCCUGGGUCUGCGACCCCCA | 699 |
| KDM1A | CUAGUGGCAGGAGAAGCUGC | 700 |
| KDM1A | GAACUCGGCAGUAAUAUCUC | 701 |
| KDM1A | CUUUCUUCGUUAGGUGUGGA | 702 |
| KDM1A | CAGGAACACCUGGUGUGGCC | 703 |
| KDM1A | UGCGUCGCAUUUAUAAAUAA | 704 |
| KDM1A | GCUCGACAGUUACAAAGUUU | 705 |
| KDM1A | CGAGAUGUUAUCUGGGAAGA | 706 |
| KDM1A | GGCCACCUCAGACCCGUUCU | 707 |
| KDM1A | CGAUGCCUGGCCAUUCUCAA | 708 |
| KDM1A | AUUGAAAGAACUUCUUAAUA | 709 |
| KDM1A | UAGGAGGUCCUUACUUGGUA | 710 |
| KDM1A | GCCAUGGUGGUAACAGGUCU | 711 |
| KDM1A | GGAAACUAUGUAGCUGAUCU | 712 |
| KDM1A | UUCCUCAGGUGGGGCUUGAG | 713 |
| KDM1A | CUCGAUUCCAGGUGCCCCAC | 714 |
| KDM1A | CGGUUCCGCCAGGCCCCCGG | 715 |
| KDM1A | ACCAAAAAUCCCUUUGAGAA | 716 |
| KDM1A | GUUCUCUGUACCCUUCCCCU | 717 |
| KDM1A | GGGCCUGUCGGGCCCAGCCG | 718 |
| KDM1A | AAGGUAAUUAUUAUAGGCUC | 719 |
| KDM1A | GGUCAGCAAACAAGUAAAUA | 720 |
| KDM1A | CGAGACACCACAGUUUCUUU | 721 |
| KDM1A | AGAAUUGCAGACCAGUUUUU | 722 |
| KDM1A | AUACUCAUCUUCUGAGAGGU | 723 |
| KDM1A | UGUGUUUUGAUCGGGUGUUC | 724 |
| KDM1A | GGAGAGAGUUGAGAGAGGUG | 725 |
| KDM1A | GCUUCAGCACACCCAGGGGA | 726 |
| KDM1A | GUAGGAGGUCCUUACUUGGU | 727 |
| KDM1A | GUCGGACCAGCCGGCGCAAG | 728 |
| KDM1A | UUGGCAGUACGACUGCCAGC | 729 |
| KDM1A | AGAAGAAAAACUUCAGGAGU | 730 |
| KDM1A | GCUGGCCCUGGGACAGCAGG | 731 |
| KDM1A | CAGAGAGAUGGAUGAAAGCU | 732 |
| KDM1A | UUAAGGGAGAGAGUUGAGAG | 733 |
| KDM1A | GGGGGCCUGGCGGAACCGCC | 734 |
| KDM1A | UACCUGAUCCCAGUGCUUAA | 735 |
| KDM1A | UGGGAAGAAGGCGGCAGCCG | 736 |
| KDM1A | UGUCACACUUUUGGAAGCCA | 737 |
| KDM1A | GCUGGGCCCGACAGGCCCGC | 738 |
| KDM1A | CUCUUCUGGAACCUCUAUAA | 739 |
| KDM1A | ACUGAUAAUAUCUGGAAAAC | 740 |
| KDM1A | CGGAGGGGCGUCGGACCAGC | 741 |
| KDM1A | CUUUGAGAAUGGCCAGGCAU | 742 |
| KDM1A | GCGCAGCCCGCGGGCCUGUC | 743 |
| KDM1A | AUUCCACGACUCUUCUUUGC | 744 |
| KDM1A | AUGCUUCUUUGUAUUGCUGA | 745 |
| KDM1A | AAGAAUUGCAGACCAGUUUU | 746 |
| KDM1A | CUGUGCAGGAACACCUGGUG | 747 |
| KDM1A | GGAAUAAGAGCCCCGAGCCC | 748 |
| KDM1A | CUGCCUCGCCAGGCCACACC | 749 |
| KDM1A | CCGGGACGGAGGCUGGCCCU | 750 |
| KDM1A | CAGAGACUCCGGAGGGGCGU | 751 |
| KDM1A | CGGAGCCGCCUGCUGUCCCA | 752 |
| KDM1A | GUGCAUGGUGCUCUGCUGAG | 753 |
| KDM1A | CUGCUGCUUCAGCACACCCA | 754 |
| KDM1A | ACAGGAAAGGUAAUUAUUAU | 755 |
| KDM1A | CAGCCGGCGCAAGCGGGCGA | 756 |
| KDM1A | UGCAUGGUGCUCUGCUGAGU | 757 |
| KDM1A | GCUGCUGCUUCAGCACACCC | 758 |
| KDM1A | UGUCGGGCCCAGCCGAGGUC | 759 |
| KDM1A | CGUGCUGAUCCCUGGGCUCG | 760 |
| KDM1A | GCGGUUCCGCCAGGCCCCCG | 761 |
| KDM1A | GCAGUCCAAAGGAUGGGAUU | 762 |
| KDM1A | ACACACUACUUACUGUUAUA | 763 |
| KDM1A | UAAGCCUGUGUCCCUUGGCC | 764 |
| KDM1A | GGCCCAGCCGAGGUCGGGCC | 765 |
| KDM1A | ACACCUGGUGUGGCCUGGCG | 766 |
| KDM1A | GAUCCAAGUGUCAAUUUGUU | 767 |
| KDM1A | UCAAAUGUCAGCUGAAUCUU | 768 |
| KDM1A | GGGCCCAGCCGAGGUCGGGC | 769 |
| KDM1A | CGACAGGCCCGCGGGCUGCG | 770 |
| KDM1A | GGUCAUGAGGAAGUCGGCUC | 771 |
| KDM1A | UCCGCGGGCCUCGCCCCCCG | 772 |
| KDM1A | CUCGUUGGCGUGCUGAUCCC | 773 |
| KDM1A | ACGAGACACCACAGUUUCUU | 774 |
| KDM1A | UGUGUCCCUUGGCCAGGCAU | 775 |
| KDM1A | CGGCUGCAGCGGCAGCAACC | 776 |
| KDM1A | GAGGAAGAGCUCACCCCUGC | 777 |
| KDM1A | AGUUCUCUGUACCCUUCCCC | 778 |
| KDM1A | CCGCGGAGGCUCUUUCUUGC | 779 |
| KDM1A | GAGAAAUGCCAAAGCAGAGA | 780 |
| KDM1A | CCCCCGGCCCGACCUCGGCU | 781 |
| KDM1A | GAAGAAGGCGGCAGCCGCGG | 782 |
| KDM1A | GGCUGGGCCCGACAGGCCCG | 783 |
| KDM1A | UCUGCGACCCCCAUGGAAAC | 784 |
| KDM1A | GCCUGAGGCCCUGCGGACCC | 785 |
| KDM1A | AGGUGGUGUUGUGUUUUGAU | 786 |
| KDM1A | UUCAUUUUCUUCCUCAGGUG | 787 |
| KDM1A | UUACCUUCGCCCGCUUGCGC | 788 |
| KDM1A | GCCCAGCCGAGGUCGGGCCG | 789 |
| KDM1A | CCGGGGGGCGAGGCCCGCGG | 790 |
| KDM1A | CCCAGGGCCAGCCUCCGUCC | 791 |
| KDM1A | UCUUCCUCAGGUGGGGCUUG | 792 |
| KDM1A | CGACCUCGGCUGGGCCCGAC | 793 |
| KDM1A | CUUCUUCCCAGAUAACAUCU | 794 |
| KDM1A | UAUGUUGCUGCAGGAUCAUC | 795 |
| KDM1A | CUCGCCCCCCGGGGGCCUGG | 796 |
| KDM1A | GCCAUUCUCAAAGGGAUUUU | 797 |
| KDM1A | GGCGUGCUGAUCCCUGGGCU | 798 |
| KDM1A | GAGACAGACAAAUACUUGAU | 799 |
| KDM1A | GGGUCCGCAGGGCCUCAGGC | 800 |
| KDM1A | ACUCCUGGCCCCUCGAUUCC | 801 |
| KDM1A | CGCGCAGCCCGCGGGCCUGU | 802 |
| KDM1A | CUUUCAUUUUCUUCCUCAGG | 803 |
| KDM1A | GUGCGCUCCCCCACCGCCCC | 804 |
| KDM1A | GAGGCUGGCCCUGGGACAGC | 805 |
| KDM1A | UUUCAUUUUCUUCCUCAGGU | 806 |
| KDM1A | CGGCUGGUCCGACGCCCCUC | 807 |
| KDM1A | AUCGAGGGGCCAGGAGUGAU | 808 |
| KDM1A | GAGGUGGCCGCGCAGCCCGC | 809 |
| KDM1A | CGGGGGCCUGGCGGAACCGC | 810 |
| KDM1A | CGCUGCAGCCGCCGCCGCCG | 811 |
| KDM1A | CUUCUCACCUGUGGGGCACC | 812 |
| KDM1A | AGGUCGGGCCGGGGGCGGUG | 813 |
| KDM1A | AGAGCACCAUGCACUGUGGC | 814 |
| KDM1A | GACUGCUGUGCAGGAACACC | 815 |
| KDM1A | AGGCUCUGGGGUCUCAGGCU | 816 |
| KDM1A | CCCAGCCGAGGUCGGGCCGG | 817 |
| KDM1A | UGAGGUGGCCGCGCAGCCCG | 818 |
| KDM1A | GCGUGCUGAUCCCUGGGCUC | 819 |
| KDM1A | GCACCUGGAAUCGAGGGGCC | 820 |
| KDM1A | CUUCGCCCGCUUGCGCCGGC | 821 |
| KDM1A | UCCUAAAGAGAAAGAUGAAA | 822 |
| KDM1A | AUGCUUGGGGACUGCUGUGC | 823 |
| KDM1A | AAUGAAAAGAAAAACCUUCU | 824 |
| KDM1A | GCGGAACCGCCGGGGUCCGC | 825 |
| KDM1A | GAGGUCGGGCCGGGGGCGGU | 826 |
| KDM1A | AUUAUAGGCUCUGGGGUCUC | 827 |
| KDM1A | UCACUUUCAUUUUCUUCCUC | 828 |
| KDM1A | UCGGAGCCGCCUGCUGUCCC | 829 |
| KDM1A | AGCCGAGGUCGGGCCGGGGG | 830 |
| KDM1A | AAAGCUAGAAGAAAAACUUC | 831 |
| KDM1A | UCCUCAGGUGGGGCUUGAGG | 832 |
| KDM1A | UGCAUCUGUCUCACAUGCUU | 833 |
| KDM1A | GGCGGUUCCGCCAGGCCCCC | 834 |
| KDM1A | CGGCGGUUCCGCCAGGCCCC | 835 |
| KDM1A | ACCGGGACGGAGGCUGGCCC | 836 |
| KDM1A | CACCGCCCCCGGCCCGACCU | 837 |
| KDM1A | GGGGUGAGCUCUUCCUCUUC | 838 |
| KDM1A | CGCCAGGCCCCCGGGGGGCG | 839 |
| KDM1A | ACCAUUUCAUCUUUCUCUUU | 840 |
| KDM1A | GGCCUCGCCCCCCGGGGGCC | 841 |
| KDM1A | UGGGGCUUGAGGGGGUGGUG | 842 |
| KDM1A | GCCGGGGUCCGCAGGGCCUC | 843 |
| KDM1A | GCCCCCGGCCCGACCUCGGC | 844 |
| KDM1A | CUUCUUUUCCUUCUCUGCUU | 845 |
| KDM1A | CCUCCGCGGGCCUCGCCCCC | 846 |
| KDM1A | GGUCGGGCCGGGGGCGGUGG | 847 |
| KDM1A | CUCCGCGGGCCUCGCCCCCC | 848 |
| KDM1A | UGGAAUGGAUGUCACACUUU | 849 |
| KDM1A | CUUCCUCAGGUGGGGCUUGA | 850 |
| KDM1A | GUGGGGCUUGAGGGGGUGGU | 851 |
| KDM1A | GCGGCUGCAGCGGCAGCAAC | 852 |
| KDM1A | CCCCCGGGGGGCGAGGCCCG | 853 |
| KDM1A | UAAUGAAAAGAAAAACCUUC | 854 |
| KDM1A | CGCGGCGGCGGCGGCUGCAG | 855 |
| KDM1A | UCAGGUGGGGCUUGAGGGGG | 856 |
| KDM1A | GGUGGGGCUUGAGGGGGUGG | 857 |
| KDM1A | UGGAAAUGACUAUGAUUUAA | 858 |
| KDM1A | GGGGCUUGAGGGGGUGGUGG | 859 |
| KDM1A | CGAGGUCGGGCCGGGGGCGG | 860 |
| KDM1A | GGCGGCAGCCGCGGCGGCGG | 861 |
| KDM1A | GAAGGCGGCAGCCGCGGCGG | 862 |

### TALEN gene editing systems

TALENs are produced artificially by fusing a TAL effector DNA binding domain to a DNA cleavage domain. Transcription activator-like effects (TALEs) can be engineered to bind any desired DNA sequence, e.g., a target gene. By combining an engineered TALE with a DNA cleavage domain, a restriction enzyme can be produced which is specific to any desired DNA sequence. These can then be used, for example, as components of gene editing systems, e.g., TALEN gene editing systems, by for example, being introduced into a cell, wherein they can be used for genome editing. Boch (2011) Nature Biotech. 29: 135-6; and Boch et al. (2009) Science 326: 1509-12; Moscou et al. (2009) Science 326: 3501.

TALEs are proteins secreted by Xanthomonas bacteria. The DNA binding domain contains a repeated, highly conserved 33-34 amino acid sequence, with the exception of the 12th and 13th amino acids. These two positions are highly variable, showing a strong correlation with specific nucleotide recognition. They can thus be engineered to bind to a desired DNA sequence.

To produce a TALEN, a TALE protein is fused to a nuclease (N), which is, for example, a wild-type or mutated Fokl endonuclease. Several mutations to Fokl have been made for its use in TALENs; these, for example, improve cleavage specificity or activity. Cermak et al. (2011) Nucl. Acids Res. 39: e82; Miller et al. (2011) Nature Biotech. 29: 143-8; Hockemeyer et al. (2011) Nature Biotech. 29: 731-734; Wood et al. (2011) Science 333: 307; Doyon et al. (2010) Nature Methods 8: 74-79; Szczepek et al. (2007) Nature Biotech. 25: 786-793; and Guo et al. (2010) J. Mol. Biol. 200: 96.

The Fokl domain functions as a dimer, requiring two constructs with unique DNA binding domains for sites in the target genome with proper orientation and spacing. Both the number of amino acid residues between the TALE DNA binding domain and the Fokl cleavage domain and the number of bases between the two individual TALEN binding sites appear to be important parameters for achieving high levels of activity. Miller et al. (2011) Nature Biotech. 29: 143-8.

A TALEN (or pair of TALENs) can be used inside a cell to produce a double-stranded break (DSB). A mutation can be introduced at the break site if the repair mechanisms improperly repair the break via non-homologous end joining. For example, improper repair may introduce a frame shift mutation. Alternatively, foreign DNA can be introduced into the cell along with the TALEN, e.g., DNA encoding a transgene, and depending on the sequences of the foreign DNA and chromosomal sequence, this process can be used to integrate the transgene at or near the site targeted by the TALEN. TALENs specific to a target gene, e.g., LSD1, can be constructed using any method known in the art, including various schemes using modular components. Zhang et al. (2011) Nature Biotech. 29: 149-53; Geibler et al. (2011) PLoS ONE 6: e19509; US 8,420,782 ; US 8,470,973.

Thus, in exemplary embodiments, the genome editing system LSD1 inhibitor is a TALEN gene editing system directed to a sequence of an LSD1 gene, e.g., KDM1A. Such systems are known generally in the art and TALEN genome editing systems specific for LSD1 can be generated using known methods. See, e.g., Boch (2011) Nature Biotech. 29: 135-6; and Boch et al. (2009) Science 326: 1509-12; Moscou et al. (2009) Science 326: 3501; Zhang et al. (2011) Nature Biotech. 29: 149-53; Geibler et al. (2011) PLoS ONE 6: e19509; US patent number 8,420,782; US Patent Number 8,470,973.

### Zinc finger nuclease (ZFN) gene editing systems

"ZFN" or "Zinc Finger Nuclease" refer to a zinc finger nuclease, an artificial nuclease or pair of nucleases which can be used, e.g., as part of a ZFN gene editing system to modify, e.g., insert or delete, one or more nucleic acids at or near a desired nucleic acid sequence, e.g., desired sequence of an LSD1 gene.

Like a TALEN, a ZFN comprises a Fokl nuclease domain (or derivative thereof) fused to a DNA-binding domain. In the case of a ZFN, the DNA-binding domain comprises one or more zinc fingers. Carroll et al. (2011) Genetics Society of America 188: 773-782; and Kim et al. (1996) Proc. Natl. Acad. Sci. USA 93: 1156-1160.

A zinc finger is a small protein structural motif stabilized by one or more zinc ions. A zinc finger can comprise, for example, Cys2His2, and can recognize an approximately 3-bp sequence. Various zinc fingers of known specificity can be combined to produce multi-finger polypeptides which recognize about 6, 9, 12, 15 or 18-bp sequences. Various selection and modular assembly techniques are available to generate zinc fingers (and combinations thereof) recognizing specific sequences, including phage display, yeast one-hybrid systems, bacterial one-hybrid and two-hybrid systems, and mammalian cells.

Like a TALEN, a ZFN must dimerize to cleave DNA. Thus, a pair of ZFNs are required to target non-palindromic DNA sites. The two individual ZFNs must bind opposite strands of the DNA with their nucleases properly spaced apart. Bitinaite et al. (1998) Proc. Natl. Acad. Sci. USA 95: 10570-5.

Also like a TALEN, a ZFN can create a double-stranded break in the DNA, which can create a frame-shift mutation if improperly repaired, leading to a decrease in the expression and amount of the target gene in a cell. ZFNs can also be used with homologous recombination to mutate the target gene or locus, or to introduce nucleic acid encoding a desired transgene at a site at or near the targeted sequence.

ZFNs specific to sequences in a target gene can be constructed using any method known in the art. See, e.g., Provasi (2011) Nature Med. 18: 807-815; Torikai (2013) Blood 122: 1341-1349; Cathomen et al. (2008) Mol. Ther. 16: 1200-7; and Guo et al. (2010) J. Mol. Biol. 400: 96; U.S. Patent Publication 2011/0158957; and U.S. Patent Publication 2012/0060230. In embodiments, The ZFN gene editing system may also comprise nucleic acid encoding one or more components of the ZFN gene editing system.

Thus, in exemplary embodiments, the genome editing system LSD1 inhibitor is a zinc finger nuclease gene editing system specific for a LSD1 gene, e.g., KDM1A. Such systems are known generally in the art and zinc finger nuclease genome editing systems specific for LSD1 can be generated using known methods. See, e.g., Provasi (2011) Nature Med. 18: 807-815; Torikai (2013) Blood 122: 1341-1349; Cathomen et al. (2008) Mol. Ther. 16: 1200-7; Guo et al. (2010) J. Mol. Biol. 400: 96; U.S. Patent Publication 2011/0158957; and U.S. Patent Publication 2012/0060230.
In an exemplary embodiment, the genome editing system LSD1 inhibitor is a meganuclease system. Such systems are known generally in the art and meganuclease genome editing systems specific for LSD1 can be generated using known methods.

### Small Molecule LSD1 Inhibitors

In one aspect the LSD1 inhibitor is a small molecule. Exemplary small molecule LSD1 inhibitors are provided below, and additional candidate molecules may be identified by known assays, such as LSD1 binding assays and the assays described herein.

Useful lysine specific demethylase 1 (LSD1) inhibitors include both irreversible and reversible inhibitors. Reviews describing a variety of reversible and irreversible LSD1 inhibitors were published by Mould, Daniel P.,et al., "Reversible Inhibitors of LSD1 as Therapeutic Agents in Acute Myeloid Leukemia: Clinical Significance and Progress to Date," Med. Res. Rev., 35, No. 3, 586-618, (2015); and Xheng, Yi-Choa, et. al., "A Systematic Review of Histone Lysine-Specific Demethylase 1 and Its Inhibitors" Med. Res. Rev., 35, No. 5, 1032-1071, (2015). Suitable LSD1 inhibitors are also disclosed in PCT Patent Publication Nos. WO07/021839; WO2010/043721; WO2010/084160; WO2011/035941; WO2011/042217; WO2012/013727; WO2012/034116; WO2012/071469; WO2012/135113; WO2013/057320; WO2013/057322; WO2014/205213; WO2015/031564; WO2015/123408; WO2015/123437; WO2015/123465; and WO2015/156417. Representative examples of irreversible and reversible LSD1 inhibitors are described herein below.

Exemplary irreversible LSD1 inhibitors include: GSK-LSD1 (trans-racemic) dihydrochloride, rel-N-[(1R,2S)-2-Phenylcyclopropyl]-4-piperidinamine hydrochloride (1:2) (available from Sigma-Aldrich); Tranylcypromine; N-[(1S,2R)-2-phenylcyclopropyl]-4-piperidinemethanamine (GSK2699537, described in PCT publication Nos. WO 2013057320 and WO 2012135113); 4-[[4-[[[(1R,2S)-2-phenylcyclopropyl]amino]methyl]-1-piperidinyl]methyl]-benzoic acid or a pharmaceutically acceptable salt thereof (GSK2879552, described in PCT publication No. WO 2012135113); trans-N1-[(1R,2S)-2-phenylcyclopropyl]-1,4-cyclohexanediamine or a pharmaceutically acceptable salt thereof (ORY-1001, described in PCT publication No. WO 2013057322); rel-1-(4-methyl-1-piperazinyl)-2-[[(1R*,2S*)-2-[4-phenylmethoxy)phenyl]cyclopropyl]amino]ethanone or a pharmaceutically acceptable salt thereof (RN-1, described in PCT Publication No. WO 2010043721); *rel-* 2-[[(1R,2S)-2-[4-[(4-chlorophenyl)methoxy] phenyl]cyclopropyl]amino]-1-(4-methyl-1-piperazinyl)-ethanone or a pharmaceutically acceptable salt thereof (described in PCT Publication No. WO 2010043721); 4'-((1R,2S)-2-Aminocyclopropyl)biphenyl-3-ol or a pharmaceutically acceptable salt thereof (OG-L002, described in PCT Publication No. WO 2012013727); (1S,2R)-N-((2-methoxypyridin-3-yl)methyl)-2-phenylcyclopropan-1-amine (described in PCT Publication No. WO2010/084160) or a pharmaceutically acceptable salt thereof.
Examplary reversible LSD1 inhibitors include: Namoline (available from ChemBridge, San Diego, CA); 3-(4-morpholinylsulfonyl)-benzoic acid, (2E)-2-[1-(5-chloro-2-hydroxyphenyl)ethylidene]hydrazide (SP-2509, described in PCT Publication No. WO 2014205213); 3-[[4-[4-(Aminoiminomethyl)benzoyl]-1-piperazinyl]carbonyl]-5-[[4-(aminoiminomethyl)-1-piperazinyl]methyl]-benzoic acid, methyl ester (CBB-1007, available from DSK Biopharma, Inc., and described in PCT Publication No. WO2012/071469); (R)-4-(5-(pyrrolidin-3-ylmethoxy)-2-(p-tolyl)pyridin-3-yl)benzonitrile (GSK354); N,N-dimethyl-1-((4-(4-(4-(piperidin-4-yl)phenyl)-1H-indazol-1-yl)phenyl)sulfonyl)piperidin-4-amine; 5-(6-chloro-4'-(methylsulfonyl)-[1,1'-biphenyl]-3-yl)-2-(piperazin-1-yl)-1H-pyrrole-3-carbonitrile; and trans-3-(3-amino-2-methylphenyl)-1-(4-hydroxycyclohexyl)-6-methyl-1H-indole-5-carbonitrile; or a pharmaceutically acceptable salt of any of the foregoing.

Additional exemplary LSD1 inhibitors are provided in Table 3, below.

**Table 3.**

| Chemical Name | Structure | LCMS IC50 (uM)* | Also Referred to in this Application As |
|---|---|---|---|
| rel- 2-[[(1R,2S)-2-[4-[(4-chlorophenyl)methoxy]phenyl]cycloprop yl]amino]-1-(4-methyl-1-piperazinyl)-ethanone | 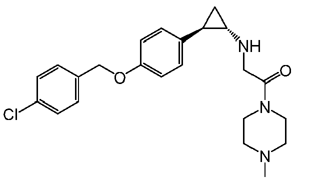 | 0.01 | |
| (1S,2R)-N-((2-methoxypyridin-3-yl)methyl)-2-phenylcyclopropan-1-amine | 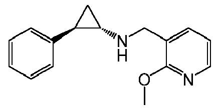 | 0.02 | Compound A |
| rel- N-[(1R,2S)-2-Phenylcyclopropyl]-4-Piperidinamine hydrochloride (1:2) | 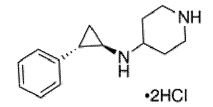 | -- | GSK-LSD1; LSD1i-GSK |
| 2-(1R,2S)-2-(4-(Benzyloxy)phenyl)cyclopropylamino)-1-(4-methylpiperazin-1-yl)ethanone, HCl | 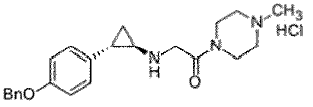 | -- | LSD1i-IV; LSD1i-EMD |
| GSK2699537 | 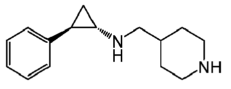 | 0.0007 | Compound B |
| GSK2879552 | 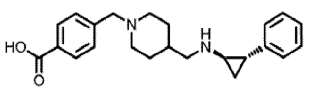 | -- | |
| (R)-4-(5-(pyrrolidin-3-ylmethoxy)-2-(p-tolyl)pyridin-3-yl)benzonitrile | 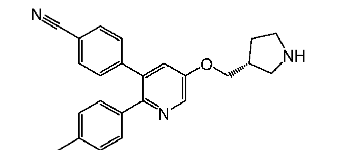 | 0.03 | GSK354; Compound C |
| N,N-dimethyl-1-((4-(4-(4-(piperidin-4-yl)phenyl)-1H-indazol-1-yl)phenyl)sulfonyl)piperidin-4-amine | 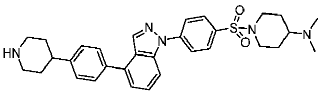 | 0.009 | described in Example 3 |
| 5-(6-chloro-4'-(methylsulfonyl)-[1,1'-biphenyl]-3-yl)-2-(piperazin-1-yl)-1H-pyrrole-3-carbonitrile | 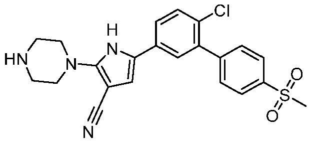 | 0.012 | described in Example 2 |
| Trans-3-(3-amino-2-methylphenyl)-1-(4-hydroxycyclohexyl)-6-methyl-1H-indole-5-carbonitrile (Described in Example 6) | 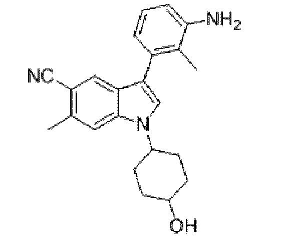 | 0.003 | NVS Compound 1; Compound 93 |

| | | | |
|---|---|---|---|
| * LSD1 IC50 as measured by LCMS. | | | |

Small molecule LSD1 inhibitors useful according to the present invention also include prodrugs, derivatives, pharmaceutically acceptable salts, or analogs thereof of any of the foregoing.

Small molecule LSD1 inhibitors may be formulated for delivery based on well-established methods in the art based on the particular dosages described herein.

In embodiments, the LSD1 small molecule inhibitor may be conjugated to an antibody or antigen binding fragment thereof. In an embodiment, the antibody or antigen-binding fragment thereof has specificity for an antigen expressed on the surface of a T cell.

### Protein LSD1 Inhibitors

In embodiments, the LSD1 inhibitor may be a protein LSD1 inhibitor. In embodiments, the protein LSD1 inhibitor is a dominant negative binding partner of LSD1 (e.g., a histone deacetylase (HDAC) that interacts with LSD1 or other member of the Co-REST or AR co-activator complex), or nucleic acid encoding said dominant negative binding partner of LSD1. In embodiments, the protein LSD1 inhibitor is a dominant negative (e.g., catalytically inactive) LSD1, or nucleic acid encoding said molecule.

### Methods of Preparing Populations of Immune Effector Cells Using LSD1 inhibitors

The disclosure features the use of LSD1 inhibitors in the manufacture of a population of immune effector cells, e.g., engineered to express a CAR molecule, e.g., as described herein. Without being bound by theory, the invention in part rests upon the surprising and unexpected discovery that inhibition of LSD1 in immune effector cells, e.g., T cells, results in a population of immune effector cells, e.g., T cells, with a higher number and/or higher proportion of naive immune effector cells, e.g., T cells, and with improved therapeutic properties. The inhibition of LSD1 in said immune effector cells may occur before and/or concurrently with therapy that includes said cells. Thus, one aspect of the disclosure relates to compositions for and use of LSD1 inhibitors in the manufacture of immune effector cells, e.g., T cells.

In one aspect, the invention provides a method of making a population of immune effector cells, which is optionally a population of T cells, including the steps of:
a) contacting a population of immune effector cells with an LSD1 inhibitor;

thereby making a population of immune effector cells, which is optionally a population of T cells, wherein the contacting with the LSD1 inhibitor causes one or more of the following to occur:
   1) an increase in the proportion of naive T cells, e.g., T_{SCM} cells;
   2) an increase in the number of naive T cells, e.g., T_{SCM} cells;
   3) a decrease in the number of T_{EM} cells;
   4) a decrease in the proportion of T_{EM} cells;
   5) an increase in the proportion of CD45RA+CD62L+ T cells;
   6) an increase in the number of CD45RA+CD62L+ T cells;
   7) an increase in the proportion of CD45RA+CCR7+ T cells;
   8) an increase in the number of CD45RA+CCR7+ T cells;
   9) a decrease in the proportion of PD-1 positive immune effector cells;
   10) an increase in the ratio of PD-1 negative immune effector cells / PD-1 positive immune effector cells;
   11) a decrease in the proportion of PD-1+/Lag3+/Tim3+ immune effector cells;
   12) an increase in the ratio of PD-1-/Lag3-/Tim3- immune effector cells to PD-1+/Lag3+/Tim3+ immune effector cells;
   13) an increase in the proliferation of the immune effector cells;
   14) an increase in the production of cytokines (e.g., IFNg and/or IL-2) from said population of immune effector cells; or
   15) a combination of two, three, four, five, six, seven, eight, nine, ten, eleven, twelve, thirteen or more (e.g., all) of the above;
as compared to a non-contacted population of immune effector cells. In the method of making a population of immune effector cells of the invention, the population of immune effector cells comprises, e.g., consists of, T cells.

In embodiments, the method further includes the step of b) inserting nucleic acid that encodes the CAR into cells of the population of immune effector cells. In embodiments, the contacting of step a) occurs 1) prior to; 2) concurrently with; 3) after; or 4) both before and after; said inserting of step b). In embodiments, the contacting of step a), and optionally the inserting of step b), is *ex vivo.*

In another aspect, the invention provides a method of making a population of immune effector cells, which is optionally a population of T cells, including the steps, optionally in the order listed, of:
a) providing a population of immune effector cells *ex vivo;*
b) contacting a population of immune effector cells *ex vivo* with an LSD1 inhibitor;

thereby making a population of immune effector cells, which is optionally a population of T cells, wherein the contacting with the LSD1 inhibitor causes one or more of the following to occur:
   1) an increase in the proportion of naive T cells, e.g., T_{SCM} cells;
   2) an increase in the number of naive T cells, e.g., T_{SCM} cells;
   3) a decrease in the number of T_{EM} cells;
   4) a decrease in the proportion of T_{EM} cells;
   5) an increase in the proportion of CD45RA+CD62L+ T cells;
   6) an increase in the number of CD45RA+CD62L+ T cells;
   7) an increase in the proportion of CD45RA+CCR7+ T cells;
   8) an increase in the number of CD45RA+CCR7+ T cells;
   9) a decrease in the proportion of PD-1 positive immune effector cells;
   10) an increase in the ratio of PD-1 negative immune effector cells / PD-1 positive immune effector cells;
   11) a decrease in the proportion of PD-1+/Lag3+/Tim3+ immune effector cells;
   12) an increase in the ratio of PD-1-/Lag3-/Tim3- immune effector cells to PD-1+/Lag3+/Tim3+ immune effector cells;
   13) an increase in the proliferation of the immune effector cells;
   14 an increase in the production of cytokines (e.g., IFNg and/or IL-2) from said population of immune effector cells; or
   15) a combination of two, three, four, five, six, seven, eight, nine, ten, eleven, twelve, thirteen or more (e.g., all) of the above;
optionally, as compared to a non-contacted population of immune effector cells.

In embodiments, the method further includes the step of c) inserting nucleic acid that encodes the CAR into cells of the population of immune effector cells. In embodiments, the contacting of step b) occurs 1) prior to; 2) concurrently with; 3) after; or 4) both before and after; said inserting of step c). In embodiments, the contacting of step b), and optionally the inserting of step c), is *ex vivo.*

In another aspect, the disclosure provides a method of making a population of immune effector cells, which is optionally a population of T cells, including the steps, optionally in the order listed, of:
a) administering to a subject an LSD1 inhibitor;
   wherein the administering the LSD1 inhibitor causes one or more of the following to occur in said subject:
      1) an increase in the proportion of naive T cells, e.g., T_{SCM} cells;
      2) an increase in the number of naive T cells, e.g., T_{SCM} cells;
      3) a decrease in the number of T_{EM} cells;
      4) a decrease in the proportion of T_{EM} cells;
      5) an increase in the proportion of CD45RA+CD62L+ T cells;
      6) an increase in the number of CD45RA+CD62L+ T cells;
      7) an increase in the proportion of CD45RA+CCR7+ T cells;
      8) an increase in the number of CD45RA+CCR7+ T cells;
      9) a decrease in the proportion of PD-1 positive immune effector cells;
      10) an increase in the ratio of PD-1 negative immune effector cells / PD-1 positive immune effector cells;
      11) a decrease in the proportion of PD-1+/Lag3+/Tim3+ immune effector cells;
      12) an increase in the ratio of PD-1-/Lag3-/Tim3- immune effector cells to PD-1+/Lag3+/Tim3+ immune effector cells;
      13) an increase in the proliferation of the immune effector cells;
      14) an increase in the production of cytokines (e.g., IFNg and/or IL-2) from said population of immune effector cells; or
      15) a combination of two, three, four, five, six, seven, eight, nine, ten, eleven, twelve, thirteen or more (e.g., all) of the above;
   optionally, as compared to a population of immune effector cells from a non-administered subject;
b) providing a population of immune effector cells from said subject *ex vivo;*
thereby making a population of immune effector cells, which is optionally a population of T cells.
In instances, the method further includes the step of c) inserting nucleic acid that encodes the CAR into cells of the population of immune effector cells.

In another aspect, the disclosure provides a method of making a population of immune effector cells, which is optionally a population of T cells, including the steps, optionally in the order listed, of:
a) administering to a subject an LSD1 inhibitor;
b) providing a population of immune effector cells from said subject *ex vivo;*
c) contacting a population of immune effector cells *ex vivo* with an LSD1 inhibitor;

thereby making a population of immune effector cells, which is optionally a population of T cells, wherein one or more of the following occurs:
   1) an increase in the proportion of naive T cells, e.g., T_{SCM} cells;
   2) an increase in the number of naive T cells, e.g., T_{SCM} cells;
   3) a decrease in the number of T_{EM} cells;
   4) a decrease in the proportion of T_{EM} cells;
   5) an increase in the proportion of CD45RA+CD62L+ T cells;
   6) an increase in the number of CD45RA+CD62L+ T cells;
   7) an increase in the proportion of CD45RA+CCR7+ T cells;
   8) an increase in the number of CD45RA+CCR7+ T cells;
   9) a decrease in the proportion of PD-1 positive immune effector cells;
   10) an increase in the ratio of PD-1 negative immune effector cells / PD-1 positive immune effector cells;
   11) a decrease in the proportion of PD-1+/Lag3+/Tim3+ immune effector cells;
   12) an increase in the ratio of PD-1-/Lag3-/Tim3- immune effector cells to PD-1+/Lag3+/Tim3+ immune effector cells;
   13) an increase in the proliferation of the immune effector cells;
   14 an increase in the production of cytokines (e.g., IFNg and/or IL-2) from said population of immune effector cells; or
   15) a combination of two, three, four, five, six, seven, eight, nine, ten, eleven, twelve, thirteen or more (e.g., all) of the above;
optionally, as compared to a non-contacted and non-administered population of immune effector cells.
In instances, the method further includes the step of d) inserting nucleic acid that encodes the CAR into cells of the population of immune effector cells. In instances, the contacting of step c) occurs 1) prior to; 2) concurrently with; 3) after; or 4) both before and after; said inserting of step d).
In aspects the administration of the LSD1 inhibitor to the subject prior to collection of the population of immune effector cells from said subject may be of sufficient time and/or at a sufficient dose so that one or more of the following occurs:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14) an increase in the production of cytokines (e.g., IFNg and/or IL-2) from said population of immune effector cells; or
15) a combination of two, three, four, five, six, seven, eight, nine, ten, eleven, twelve, thirteen or more (e.g., all) of the above;
optionally, as compared to non-administered population of immune effector cells. The assays described herein may be utilized in order to determine the proper dose and or time of administration. In instances, the LSD1 inhibitor is administered for a period of at least 1 day prior to collection of the population of immune effector cells from said subject. In instances, the LSD1 inhibitor is administered for a period of at least 2 days prior to collection of the population of immune effector cells from said subject. In instances, the LSD1 inhibitor is administered for a period of at least 3 days prior to collection of the population of immune effector cells from said subject. In instances, the LSD1 inhibitor is administered for a period of at least 4 days prior to collection of the population of immune effector cells from said subject. In instances, the LSD1 inhibitor is administered for a period of at least 5 days prior to collection of the population of immune effector cells from said subject. In instances, the LSD1 inhibitor is administered for a period of at least 6 days prior to collection of the population of immune effector cells from said subject. In instances, the LSD1 inhibitor is administered for a period of at least 7 days prior to collection of the population of immune effector cells from said subject. In instances, the LSD1 inhibitor is administered for a period of at least a week or weeks prior to collection of the population of immune effector cells from said subject.
In instances, the administration of the LSD1 inhibitor to the subject continues after collection of the immune effector cells from said subject, e.g., continues for a period of 1, 2, 3, 4, 5, 6, 7, 8, 9, 10, 11, 12, 13, 14, 15, 16, 17, 18, 19, 20 or more days after collection of the immune effector cells, e.g., continues at least until the immune effector cells (modified *ex vivo*) are administered back to the subject, e.g., continues past the time when the immune effector cells (modified *ex vivo*) are administered back to the subject.

In aspects the contacting (e.g., ex vivo) of the LSD1 inhibitor to the population of immune effector cells may be of sufficient time and/or at a sufficient dose so that one or more of the following occurs:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14 an increase in the production of cytokines (e.g., IFNg and/or IL-2) from said population of immune effector cells; or
15) a combination of two, three, four, five, six, seven, eight, nine, ten, eleven, twelve, thirteen or more (e.g., all) of the above;
optionally, as compared to non-contacted population of immune effector cells. The assays described herein may be utilized in order to determine the proper dose and or time of administration. In embodiments, the population of immune effector cells is contacted with an LSD1 inhibitor for a period of at least 1 day. In embodiments, the population of immune effector cells is contacted with an LSD1 inhibitor for a period of at least 2 days. In embodiments, the population of immune effector cells is contacted with an LSD1 inhibitor for a period of at least 3 days. In embodiments, the population of immune effector cells is contacted with an LSD1 inhibitor for a period of at least 4 days. In embodiments, the population of immune effector cells is contacted with an LSD1 inhibitor for a period of at least 5 days. In embodiments, the population of immune effector cells is contacted with an LSD1 inhibitor for a period of at least 6 days. In embodiments, the population of immune effector cells is contacted with an LSD1 inhibitor for a period of at least 7 days. In embodiments, the population of immune effector cells is contacted with an LSD1 inhibitor for a period of at least a week or weeks. In embodiments, media containing the LSD1 inhibitor is replaced with fresh media containing the LSD1 inhibitor, e.g., once, twice, three times, 4 times, 5 times, 6 times, 7 times, or more than 7 times (e.g., every day or every other day) during the time the immune effector cells are ex vivo. The concentration of LSD1 inhibitor can be adjusted in order that the desired effect occurs, and may be, for example, about 0.001 nM to about 10 mM, e.g., about 0.01 nM to about 1 mM, e.g., about 0.1 nM to about 100 uM, e.g., from about 1 nM to about 100 uM, e.g., from about 10 nM to about 100 uM, e.g., from about 100 nM to about 10 uM, e.g., from about 0.001 nM to about 100 nM, or e.g., from about 0.1 uM to about 10 uM. In embodiments, the concentration of LSD1 inhibitor is 100 nM. In embodiments, the concentration of LSD1 inhibitor is about 100 uM. In embodiments, the concentration of LSD1 inhibitor is 200 nM. In embodiments, the concentration of LSD1 inhibitor is about 200 uM.

In another aspect the disclosure provides a composition for use in *ex vivo* manufacturing a population of immune effector cells, that includes an LSD1 inhibitor, e.g., a small molecule LSD1 inhibitor. In instances, the composition includes the small molecule LSD1 inhibitor at a concentration of from about 0.001 nM to about 10 mM, e.g., from about 0.001 nM to about 100 nM, or, e.g., from about 0.1 uM to about 10 uM.

In embodiments involving immune effector cells engineered to express a CAR molecule, e.g., as described herein, it is understood that the method may further include any of the aspects, steps or features described below in the section relating to Chimeric Antigen Receptors.

### Treatment with Immune Effector Cells and LSD1 inhibitors

The disclosure features the use of LSD1 inhibitors in the treatment of a disease, e.g., cancer, in a patient wherein such treatment is in combination with administration of a population of immune effector cells, e.g., immune effector cells engineered to express a CAR molecule, e.g., as described herein. Without being bound by theory, the invention in part rests upon the surprising and unexpected discovery that inhibition of LSD1 in immune effector cells, e.g., T cells, results in a population of immune effector cells, e.g., T cells, with a higher number and/or higher proportion of naive immune effector cells, e.g., T cells, and with improved therapeutic properties. Thus, one aspect of the invention provides treatment of a disease, e.g., a cancer, with a combination of a population of immune effector cells, e.g., engineered to express a CAR molecule, e.g., as described herein, and an LSD1 inhibitor.

In one aspect, the invention relates to treating a subject that includes administering an LSD1 inhibitor to the subject, wherein said subject has received, is receiving or is about to receive a population of immune effector cells engineered to express a chimeric antigen receptor (CAR). In embodiments, the treating includes administering to said subject an LSD1 inhibitor and a population of immune effector cells engineered to express a CAR molecule, e.g., as described herein. In embodiments, the LSD1 inhibitor is administered before the population of immune effector cells engineered to express a CAR molecule, e.g., as described herein, and wherein said administration of the LSD1 inhibitor is continued for a period of time after the administration of the population of immune effector cells engineered to express a CAR molecule, e.g., as described herein. In other embodiments, the administration of the LSD1 inhibitor after the administration of the population of immune effector cells engineered to express a CAR molecule, e.g., as described herein is in an amount sufficient to increase an anti-tumor effect of the population of immune effector cells engineered to express a CAR molecule, e.g., as described herein relative to an equivalent population of immune effector cells engineered to express a CAR molecule, e.g., as described herein administered in the absence of said LSD1 inhibitor.

In another aspect, the invention relates to increasing the therapeutic efficacy in a subject of a population of immune effector cells engineered to express a CAR molecule, e.g., as described herein, e.g., a CAR19 (e.g., CTL019), including a step of decreasing the activity or expression of LSD1 in said cell, at least transiently. In embodiments, tThe step of decreasing the activity or expression of LSD1 in said cell includes contacting the cell with an LSD1 inhibitor. In embodiments, the contacting is done ex vivo. In embodiments, the contacting is done in vivo (e.g., the population of immune effector cells and the LSD1 inhibitor are coadministered to the subject).

In embodiments of any of the forgoing aspect, the administration or the contacting of the LSD1 inhibitor results in:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14) an increase in the production of cytokines (e.g., IFNg and/or IL-2) from said population of immune effector cells; or
15) a combination of two, three, four, five, six, seven, eight, nine, ten, eleven, twelve, thirteen or more (e.g., all) of the above.

In embodiments, the effect is as compared to cells not contacted with the LSD1 inhibitor. In embodiments, the effect is as compared to cells of the same subject not contacted with the LSD1 inhibitor.

In another aspect, disclosed herein is a method of treating a subject, that includes:
a) administering an LSD1 inhibitor to said subject;
b) collecting a population of immune effector cells from said subject after said administration of the LSD1 inhibitor;
c) providing said population of immune effector cells *ex vivo;*
d) contacting said *ex vivo* population of immune effector cells with the LSD1 inhibitor, wherein the contacting with the LSD1 inhibitor causes one or more of the following to occur:
   1) an increase in the proportion of naive T cells, e.g., T_{SCM} cells;
   2) an increase in the number of naive T cells, e.g., T_{SCM} cells;
   3) a decrease in the number of T_{EM} cells;
   4) a decrease in the proportion of T_{EM} cells;
   5) an increase in the proportion of CD45RA+CD62L+ T cells;
   6) an increase in the number of CD45RA+CD62L+ T cells;
   7) an increase in the proportion of CD45RA+CCR7+ T cells;
   8) an increase in the number of CD45RA+CCR7+ T cells;
   9) a decrease in the proportion of PD-1 positive immune effector cells;
   10) an increase in the ratio of PD-1 negative immune effector cells / PD-1 positive immune effector cells;
   11) a decrease in the proportion of PD-1+/Lag3+/Tim3+ immune effector cells;
   12) an increase in the ratio of PD-1-/Lag3-/Tim3- immune effector cells to PD-1+/Lag3+/Tim3+ immune effector cells;
   13) an increase in the proliferation of the immune effector cells;
   14) an increase in the production of cytokines (e.g., IFNg and/or IL-2) from said population of immune effector cells; or
   15) a combination of two, three, four, five, six, seven, eight, nine, ten, eleven, twelve, thirteen or more (e.g., all) of the above;
   optionally, as compared to a non-contacted *ex vivo* population of immune effector cells; and
e) administering the population of immune effector cells to the subject.

In instances, step of e) further includes administering the LSD1 inhibitor to the subject. In instances, the method further includes the step of inserting nucleic acid that encodes a CAR into cells of the *ex vivo* population of immune effector cells.

In another aspect the present disclosure provides for treating a subject in need thereof, including administering to said subject an effective amount of the population of immune effector cells of any of the previous aspects and embodiments. In embodiments, the treating further includes administering to said subject an LSD1 inhibitor. In embodiments, the subject receives a pre-treatment of the LSD1 inhibitor, prior to the administration of the population of immune effector cells. In embodiments, the subject receives concurrent treatment with an LSD1 inhibitor and the population of immune effector cells. In embodiments, the subject receives treatment with an LSD1 inhibitor after administration of the population of immune effector cells; In embodiments, the subject receives a combination of any of the foregoing.

In an aspect, including in the previous aspects relating to treatment, the disclosure relates to treating a subject, wherein the subject has a disease associated with expression of a tumor antigen, e.g., a proliferative disease, a precancerous condition, a cancer, and a non-cancer related indication associated with expression of the tumor antigen. In the invention, the disease associated with expression of a tumor antigen is chosen from a proliferative disease, a precancerous condition, or a cancer. In embodiments, the cancer is a hematologic cancer chosen from one or more of chronic lymphocytic leukemia (CLL), acute leukemias, acute lymphoid leukemia (ALL), acute myeloid leukemia (AML), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, or pre-leukemia. In embodiments, the cancer is selected from the group consisting of colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.

In embodiments involving immune effector cells engineered to express a CAR molecule, e.g., as described herein, it is understood that the treatment method may further include any of the steps, aspects or features described below in the section relating to Chimeric Antigen Receptors.

### Cells

As will be readily apparent to the skilled artisan from this disclosure, the invention relates to cells comprising LSD1 inhibitors. The disclosure further includes cells that have been contacted with an LSD1 inhibitor, e.g., for a period of time and/or at a dose sufficient for one or more of the following to occur:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14 an increase in the production of cytokines (e.g., IFNg and/or IL-2) from said population of immune effector cells; or
15) a combination of two, three, four, five, six, seven, eight, nine, ten, eleven, twelve, thirteen or more (e.g., all) of the above;
optionally, relative to un-contacted cells.

The disclosure further relates to cells made by any of the methods described herein.

The cells disclosed herein are preferably immune effector cells. In an embodiment, the cells are T cells. In an embodiment, the cells are NK cells. In embodiments, the invention relates to a population of cells of the invention, e.g., a population of immune effector cells of the invention. In particular, the invention provides a population of immune effector cells, which is engineered to express a chimeric antigen receptor (CAR), wherein the CAR comprises an antigen-binding domain, a transmembrane domain, and an intracellular signaling domain, and wherein expression and/or function of LSD1 in said cell has been reduced or eliminated, wherein the population of immune effector cells further comprises an LSD1 inhibitor, wherein the population of immune effector cells comprises, e.g., consists of, T cells.

In embodiments, the population of cells of the invention comprises cells of the type indicated, and may comprise other types (e.g., a population of immune effector cells, e.g., T cells, engineered to express a CAR molecule, e.g., as described herein, may include T cells engineered to express a CAR molecule as well as T cells (or other cell types) that have not been engineered to express a CAR molecule). In embodiments, the population of cells of the invention consists essentially of cells of the type indicated. In embodiments, the population of cells of the invention is substantially free of other cell types. In embodiments, the population of cells of the invention consists of the indicated cell type.

In any of the foregoing aspects and embodiments, the cells and/or population of cells are or include immune effector cells, e.g., the population of immune effector cells includes, e.g., consists of, T cells or NK cells. In embodiments the cells are T cells, e.g., CD8+ T cells, CD4+ T cells, or a combination thereof. In embodiments the cells are NK cells. In the invention, the population of immune effector cells comprises, e.g., consists of, T cells.

In embodiments the cells are human cells. In embodiments, the cells are autologous, e.g., to the subject to be administered the cells. In embodiments, the cells are allogeneic, e.g., to the subject to be administered the cells.

In embodiments, the cells are, or include, cells engineered to express a CAR molecule, e.g., as described herein. Additional features and/or aspects of the cells useful in the invention are described below in the section entitled Chimeric Antigen Receptors.

In one embodiment, the immune effector cells expressing a CAR molecule, e.g., a CAR molecule described herein, are obtained from a subject that has received an LSD1 inhibitor. In an embodiment, the population of immune effector cells, e.g., T cells, to be engineered to express a CAR molecule, are harvested after a sufficient time, or after sufficient dosing of the LSD1 inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In other embodiments, a population of immune effector cells, e.g., T cells, which have, or will be engineered to express a CAR molecule, e.g., as described herein, can be treated ex vivo by contact with an amount of an LSD1 inhibitor that increases the number of PD1 negative immune effector cells, e.g., T cells or increases the ratio of PD1 negative immune effector cells, e.g., T cells/ PD1 positive immune effector cells, e.g., T cells.

In an instance, the NK cells are obtained from the subject. In another instance, the NK cells are an NK cell line, e.g., NK-92 cell line (Conkwest).

In an embodiment, immune effector cells, e.g., T cells, are obtained or harvested from a subject after administration to the subject of an LSD1 inhibitor.

In an embodiment, the immune effector cells, e.g., T cells, are collected after an increase in the number of PD1 negative immune effector, e.g., T cells, or after an increase in the ratio of PD1 negative immune effector, e.g., T cells/ PD1 positive immune effector, e.g., T cells, has occurred.

In an embodiment, the immune effector cells, e.g., T cells, are collected after an increase in the number of naive T cells has occurred.

In an embodiment, the immune effector cells, e.g., T cells, are collected after one or more of the following:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14) an increase in the production of cytokines (e.g., IFNg and/or IL-2) from said population of immune effector cells; or
15) a combination of two, three, four, five, six, seven, eight, nine, ten, eleven, twelve, thirteen or more (e.g., all) of the above;

The increase or decrease can be transient. The increase or decrease can be permanent. The increase or decrease can be as compared with a standard, e.g., cells from an untreated subject.

In an embodiment, immune effector cells, e.g., T cells, are contacted, ex vivo (after removal from the subject or a donor and before introduction into the subject), with an LSD1 inhibitor.

In an embodiment, the contact is at a level which results in an increase in the number of PD1 negative immune effector, e.g., T cells, or an increase in the ratio of PD1 negative immune effector cells, e.g., T cells/ PD1 positive immune effector, e.g., T cells.

In an embodiment, immune effector cells, e.g., T cells, are contacted, ex vivo (after removal from the subject or a donor and before introduction into the subject), with an LSD1 inhibitor, at a level which results in an increase in the number of naive T cells.

In an embodiment, immune effector cells, e.g., T cells, are contacted, ex vivo (after removal from the subject or a donor and before introduction into the subject), with an LSD1 inhibitor, at a level which results in one or more of the following:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14) an increase in the production of cytokines (e.g., IFNg and/or IL-2) from said population of immune effector cells; or
15) a combination of two, three, four, five, six, seven, eight, nine, ten, eleven, twelve, thirteen or more (e.g., all) of the above;

The increase or decrease can be transient. The increase or decrease can be permanent. The increase or decrease can be as compared with a standard, e.g., cells from an untreated subject.

In an embodiment a preparation of T cells is evaluated for the level of increase in the number of PD1 negative immune effector, e.g., T cells, or an increase in the ratio of PD1 negative immune effector cells, e.g., T cells/ PD1 positive immune effector, e.g., T cells.

In an embodiment, a preparation of T cells is evaluated for the level of increase in the number of naive T cells. In an embodiment, a preparation of T cells is evaluated for one or more of the following:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14) an increase in the production of cytokines (e.g., IFNg and/or IL-2) from said population of immune effector cells; or
15) a combination of two, three, four, five, six, seven, eight, nine, ten, eleven, twelve, thirteen or more (e.g., all) of the above;

The increase or decrease can be transient. The increase or decrease can be permanent. The increase or decrease can be as compared with a standard, e.g., cells of an untreated subject.

### Pharmaceutical Compositions: LSD1 Inhibitors

In one aspect, the present disclosure includes pharmaceutical compositions comprising an LSD1 inhibitor, e.g., an LSD1 inhibitor as described herein, formulated for use as a medicament.

In one aspect, the present disclosure includes pharmaceutical compositions comprising an LSD1 inhibitor, e.g., an LSD1 inhibitor as described herein, formulated for use in the manufacture of a population of immune effector cells.

In one aspect, the present disclosure includes pharmaceutical compositions comprising an LSD1 inhibitor, e.g., an LSD1 inhibitor as described herein, formulated for use in combination with CAR cells described herein.

In some embodiments, the LSD1 inhibitor is formulated for administration in combination with another agent, in addition to a CAR cell, e.g., as described herein.

In general, compounds of the disclosure will be administered in therapeutically effective amounts as described above via any of the usual and acceptable modes known in the art, either singly or in combination with one or more therapeutic agents.

The pharmaceutical formulations may be prepared using conventional dissolution and mixing procedures. For example, the bulk drug substance (e.g., an LSD1 inhibitor or stabilized form of the compound (e.g., complex with a cyclodextrin derivative or other known complexation agent) is dissolved in a suitable solvent in the presence of one or more of the excipients described herein. The LSD1 inhibitor is typically formulated into pharmaceutical dosage forms to provide an easily controllable dosage of the drug and to give the patient an elegant and easily handleable product.

Compounds of the disclosure can be administered as pharmaceutical compositions by any conventional route, in particular enterally, e.g., orally, e.g., in the form of tablets or capsules, or parenterally, e.g., in the form of injectable solutions or suspensions, topically, e.g., in the form of lotions, gels, ointments or creams, or in a nasal or suppository form. Where an LSD1 inhibitor is administered in combination with (either simultaneously with or separately from) another agent as described herein, in one aspect, both components can be administered by the same route (e.g., parenterally). Alternatively, another agent may be administered by a different route relative to the LSD1 inhibitor. For example, an LSD1 inhibitor may be administered orally and the other agent may be administered parenterally. Pharmaceutical compositions comprising an LSD1 inhibitor in free form or in a pharmaceutically acceptable salt form in association with at least one pharmaceutically acceptable carrier or diluent can be manufactured in a conventional manner by mixing, granulating or coating methods. For example, oral compositions can be tablets or gelatin capsules comprising the active ingredient together with a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrrolidone; if desired d) disintegrants, e.g., starches, agar, alginic acid or its sodium salt, or effervescent mixtures; and/or e) absorbents, colorants, flavors and sweeteners. Oral formulations can also comprise the active ingredient along with 20-60% Eudragit EPO, Hydroxypropyl cellulose EF, Hydroxypropyl methylcellulose, or Kollidon VA64, and up to 5% of pluronic F68, Cremophor EL, or Gelucire 44/14. Injectable compositions can be aqueous isotonic solutions or suspensions, and suppositories can be prepared from fatty emulsions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Suitable formulations for transdermal applications include an effective amount of a compound of the present disclosure with a carrier. A carrier can include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin. Matrix transdermal formulations may also be used. In a further aspect, the LSD1 inhibitors described herein may be administered via a microneedle patch. Microneedle based drug delivery is well known in the art (See, e.g., U.S. Pat. 8,162,901) and these technologies and methods may be adapted by one of skill in the art for administration of an LSD1 inhibitor as described herein. Suitable formulations for topical application, e.g., to the skin and eyes, are preferably aqueous solutions, ointments, creams or gels well-known in the art. Such formulations may contain solubilizers, stabilizers, tonicity enhancing agents, buffers and preservatives.

The pharmaceutical composition (or formulation) for application may be packaged in a variety of ways depending upon the method used for administering the drug. Generally, an article for distribution includes a container having deposited therein the pharmaceutical formulation in an appropriate form. Suitable containers are well-known to those skilled in the art and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings. The disclosure also provides for a pharmaceutical combinations, e.g. a kit, comprising a) a first agent which is an LSD1 inhibitor as disclosed herein, in free form or in pharmaceutically acceptable salt form, and b) at least one additional agent. The kit can comprise instructions for its administration.

The term "pharmaceutical combination" as used herein means a product that results from the mixing or combining of more than one active ingredient and includes both fixed and non-fixed combinations of the active ingredients. The term "fixed combination" means that the active ingredients, e.g. an LSD1 inhibitor and other agent, are both administered to a patient simultaneously in the form of a single entity or dosage. The term "non-fixed combination" means that the active ingredients, e.g. an LSD1 inhibitor and other agent, are both administered to a patient as separate entities either simultaneously, concurrently or sequentially with no specific time limits, wherein such administration provides therapeutically effective levels of the 2 compounds in the body of the patient. The latter also applies to cocktail therapy, e.g. the administration of 3 or more active ingredients.

### Chimeric Antigen Receptors

### General Description of Chimeric Antigen Receptor Technology Relevant to the Invention

Described herein are methods for combining the administration of LSD1 inhibitors with administration of a population of immune effector cells, e.g., T cells or NK cells, engineered to express a CAR molecule, e.g., as described herein (the cell is engineered to express a CAR, and in embodiments, expresses the CAR by the time at which it is administered to the subject. In other embodiments, expression initiates after administration.) In some embodiments, the cell is a T cell engineered to express a CAR molecule, e.g., as described herein, wherein the CAR T cell ("CART") exhibits an anticancer property. Also described herein are methods for using LSD1 inhibitors for the manufacture, e.g., the activation and/or expansion, a population of immune effector cells, e.g., T cells or NK cells, engineered to express a CAR molecule, e.g., as described herein, wherein the cells have enhanced activity (e.g., proliferation, cytokine release, and/or tumor targeting efficacy) and/or a more naive phenotype, relative to cells manufactured without the use of LSD1 inhibitors. In general, the molecules, cells, methods or other aspects discussed in this section may be useful in the methods, compositions, cells and other aspects of the invention, e.g., in combination with LSD1 inhibitors.

In general, the disclosure provides an isolated nucleic acid molecule encoding a chimeric antigen receptor (CAR), wherein the CAR comprises an antigen binding domain (e.g., antibody or antibody fragment, TCR or TCR fragment) that binds to a tumor antigen as described herein, a transmembrane domain (e.g., a transmembrane domain described herein), and an intracellular signaling domain (e.g., an intracellular signaling domain described herein) (e.g., an intracellular signaling domain comprising a costimulatory domain (e.g., a costimulatory domain described herein) and/or a primary signaling domain (e.g., a primary signaling domain described herein). In other aspects, the disclosure includes: host cells containing the above nucleic acids and isolated proteins encoded by such nucleic acid molecules. CAR nucleic acid constructs, encoded proteins, containing vectors, host cells, pharmaceutical compositions, and methods of administration and treatment related to the present invention are disclosed in detail in International Patent Application Publication No. WO2015/142675.

In one aspect, the disclosure provides an isolated nucleic acid molecule encoding a chimeric antigen receptor (CAR), wherein the CAR comprises an antigen binding domain (e.g., antibody or antibody fragment, TCR or TCR fragment) that binds to a tumor-supporting antigen (e.g., a tumor-supporting antigen as described herein), a transmembrane domain (e.g., a transmembrane domain described herein), and an intracellular signaling domain (e.g., an intracellular signaling domain described herein) (e.g., an intracellular signaling domain comprising a costimulatory domain (e.g., a costimulatory domain described herein) and/or a primary signaling domain (e.g., a primary signaling domain described herein). In some embodiments, the tumor-supporting antigen is an antigen present on a stromal cell or a myeloid-derived suppressor cell (MDSC). In other aspects, the disclosure features polypeptides encoded by such nucleic acids and host cells containing such nucleic acids and/or polypeptides. In other aspects, the disclosure features cells (e.g., a population of cells), e.g., immune effector cells, e.g., T cells or NK cells, engineered to express a CAR molecule, e.g., as described herein.

### Targets

The present disclosure provides immune effector cells (e.g., T cells, NK cells) that are engineered to contain one or more CARs that direct the immune effector cells to undesired cells (e.g., cancer cells). This is achieved through an antigen binding domain on the CAR that is specific for a cancer associated antigen. There are two classes of cancer associated antigens (tumor antigens) that can be targeted by the CARs of the instant disclosure: (1) cancer associated antigens that are expressed on the surface of cancer cells; and (2) cancer associated antigens that itself is intracellar, however, a fragment of such antigen (peptide) is presented on the surface of the cancer cells by MHC (major histocompatibility complex).

In some embodiments, the tumor antigen is chosen from one or more of: CD19; CD123; CD22; CD30; CD171; CS-1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRvIII); ganglioside G2 (GD2); ganglioside GD3 (aNeu5Ac(2-8)aNeu5Ac(2-3)bDGalp(1-4)bDGlcp(1-1)Cer); TNF receptor family member B cell maturation (BCMA); Tn antigen ((Tn Ag) or (GaINAcα-Ser/Thr)); prostate-specific membrane antigen (PSMA); Receptor tyrosine kinase-like orphan receptor 1 (ROR1); Fms-Like Tyrosine Kinase 3 (FLT3); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-11Ra); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAIX); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3 (aNeu5Ac(2-3)bDGalp(1-4)bDGlcp(1-1)Cer); transglutaminase 5 (TGS5); high molecular weight-melanoma-associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1 (PCTA-1 or Galectin 8), melanoma antigen recognized by T cells 1 (MelanA or MART1); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); and immunoglobulin lambda-like polypeptide 1 (IGLL1).

A CAR described herein can comprise an antigen binding domain (e.g., antibody or antibody fragment, TCR or TCR fragment) that binds to a tumor-supporting antigen (e.g., a tumor-supporting antigen as described herein). In some embodiments, the tumor-supporting antigen is an antigen present on a stromal cell or a myeloid-derived suppressor cell (MDSC). Stromal cells can secrete growth factors to promote cell division in the microenvironment. MDSC cells can inhibit T cell proliferation and activation. Without wishing to be bound by theory, in some embodiments, the CAR-expressing cells destroy the tumor-supporting cells, thereby indirectly inhibiting tumor growth or survival.

In embodiments, the stromal cell antigen is chosen from one or more of: bone marrow stromal cell antigen 2 (BST2), fibroblast activation protein (FAP) and tenascin. In an embodiment, the FAP-specific antibody is, competes for binding with, or has the same CDRs as, sibrotuzumab. In embodiments, the MDSC antigen is chosen from one or more of: CD33, CD11b, C14, CD15, and CD66b. Accordingly, in some embodiments, the tumor-supporting antigen is chosen from one or more of: bone marrow stromal cell antigen 2 (BST2), fibroblast activation protein (FAP) or tenascin, CD33, CD11b, C14, CD15, and CD66b.

### Antigen Binding Domain Structures

In some embodiments, the antigen binding domain of the encoded CAR molecule comprises an antibody, an antibody fragment, an scFv, a Fv, a Fab, a (Fab')2, a single domain antibody (SDAB), a VH or VL domain, a camelid VHH domain or a bi-functional (e.g. bi-specific) hybrid antibody (e.g., Lanzavecchia et al., Eur. J. Immunol. 17, 105 (1987)).

In some instances, scFvs can be prepared according to method known in the art (see, for example, Bird et al., (1988) Science 242:423-426 and Huston et al., (1988) Proc. Natl. Acad. Sci. USA 85:5879-5883). ScFv molecules can be produced by linking VH and VL regions together using flexible polypeptide linkers. The scFv molecules comprise a linker (e.g., a Ser-Gly linker) with an optimized length and/or amino acid composition. The linker length can greatly affect how the variable regions of a scFv fold and interact. In fact, if a short polypeptide linker is employed (e.g., between 5-10 amino acids) intrachain folding is prevented. Interchain folding is also required to bring the two variable regions together to form a functional epitope binding site. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715.

An scFv can comprise a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its VL and VH regions. The linker sequence may comprise any naturally occurring amino acid. In some embodiments, the linker sequence comprises amino acids glycine and serine. In another embodiment, the linker sequence comprises sets of glycine and serine repeats such as (Gly₄Ser)n, where n is a positive integer equal to or greater than 1 (SEQ ID NO:22). In one embodiment, the linker can be (Gly₄Ser)₄ (SEQ ID NO:29) or (Gly₄Ser)₃(SEQ ID NO:30). Variation in the linker length may retain or enhance activity, giving rise to superior efficacy in activity studies.

In another aspect, the antigen binding domain is a T cell receptor ("TCR"), or a fragment thereof, for example, a single chain TCR (scTCR). Methods to make such TCRs are known in the art. See, e.g., Willemsen RA et al, Gene Therapy 7: 1369-1377 (2000); Zhang T et al, Cancer Gene Ther 11: 487-496 (2004); Aggen et al, Gene Ther. 19(4):365-74 (2012). For example, scTCR can be engineered that contains the Vα and Vβ genes from a T cell clone linked by a linker (e.g., a flexible peptide). This approach is very useful to cancer associated target that itself is intracellar, however, a fragment of such antigen (peptide) is presented on the surface of the cancer cells by MHC.

In certain embodiments, the encoded antigen binding domain has a binding affinity KD of 10⁻⁴ M to 10⁻⁸ M.

In one embodiment, the encoded CAR molecule comprises an antigen binding domain that has a binding affinity KD of 10-4 M to 10-8 M, e.g., 10-5 M to 10-7 M, e.g., 10-6 M or 10-7 M, for the target antigen. In one embodiment, the antigen binding domain has a binding affinity that is at least five-fold, 10-fold, 20-fold, 30-fold, 50-fold, 100-fold or 1,000-fold less than a reference antibody, e.g., an antibody described herein. In one embodiment, the encoded antigen binding domain has a binding affinity at least 5-fold less than a reference antibody (e.g., an antibody from which the antigen binding domain is derived). In one aspect such antibody fragments are functional in that they provide a biological response that can include, but is not limited to, activation of an immune response, inhibition of signal-transduction origination from its target antigen, inhibition of kinase activity, and the like, as will be understood by a skilled artisan.

In one aspect, the antigen binding domain of the CAR is a scFv antibody fragment that is humanized compared to the murine sequence of the scFv from which it is derived.

In one aspect, the antigen binding domain of a CAR in the invention (e.g., a scFv) is encoded by a nucleic acid molecule whose sequence has been codon optimized for expression in a mammalian cell. In one aspect, an entire CAR construct in the invention is encoded by a nucleic acid molecule whose entire sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least US Patent Numbers 5,786,464 and 6,114,148.

### Specific Antigen Binding Domains

In some embodiments, the portion of the CAR comprising the antigen binding domain comprises an antigen binding domain that targets a tumor antigen, e.g., a tumor antigen described herein (e.g., in the section entitled "Targets." In some embodiments, the tumor antigen is a tumor antigen described in International Application WO2015/142675, filed March 13, 2015. Exemplary target antigens that can be targeted using the CAR-expressing cells, include, but are not limited to, CD19, CD123, EGFRvIII, CD33, mesothelin, BCMA, and GFR ALPHA-4, among others, as described in, for example, WO2014/153270, WO 2014/130635, WO2016/028896, WO 2014/130657, WO2016/014576, WO 2015/090230, WO2016/014565, WO2016/014535, and WO2016/025880.

In embodiments, the antigen binding domain comprises one, two, three (e.g., all three) heavy chain CDRs, HC CDR1, HC CDR2 and HC CDR3, from an antibody described herein or in any of the publications referenced herein, and/or one, two, three (e.g., all three) light chain CDRs, LC CDR1, LC CDR2 and LC CDR3, from an antibody described herein or in any of the publications referenced herein. In one embodiment, the antigen binding domain comprises a heavy chain variable region and/or a variable light chain region of an antibody described herein or in any of the publications referenced herein. In one embodiment, the antigen binding domain of any of the CAR molecules described herein (e.g., any of CD19, CD123, EGFRvIII, CD33, mesothelin, BCMA, and GFR ALPHA-4) comprises one, two, three (e.g., all three) heavy chain CDRs, HC CDR1, HC CDR2 and HC CDR3, from an antibody described herein or in any of the publications referenced herein, and/or one, two, three (e.g., all three) light chain CDRs, LC CDR1, LC CDR2 and LC CDR3, from an antigen binding domain described herein or in any of the publications referenced herein. In one embodiment, the antigen binding domain comprises a heavy chain variable region and/or a variable light chain region of an antibody described herein or in any of the publications referenced herein.

In one embodiment, the antigen binding domain comprises one, two three (e.g., all three) heavy chain CDRs, HC CDR1, HC CDR2 and HC CDR3, from an antibody described herein (e.g., an antibody described in WO2015/142675, US-2015-0283178-A1, US-2016-0046724-A1, US2014/0322212A1, US2016/0068601A1, US2016/0051651A1, US2016/0096892A1, US2014/0322275A1, or WO2015/090230), and/or one, two, three (e.g., all three) light chain CDRs, LC CDR1, LC CDR2 and LC CDR3, from an antibody described herein (e.g., an antibody described in WO2015/142675, US-2015-0283178-A1, US-2016-0046724-A1, US2014/0322212A1, US2016/0068601A1, US2016/0051651A1, US2016/0096892A1, US2014/0322275A1, or WO2015/090230). In one embodiment, the antigen binding domain comprises a heavy chain variable region and/or a variable light chain region of an antibody described herein. In embodiments, the antigen binding domain is an antigen binding domain described in WO2015/142675, US-2015-0283178-A1, US-2016-0046724-A1, US2014/0322212A1, US2016/0068601A1, US2016/0051651A1, US2016/0096892A1, US2014/0322275A1, or WO2015/090230.

In one embodiment, an antigen binding domain against CD19 is an antigen binding portion, e.g., CDRs, of a CAR (e.g., CD19 CAR), antibody or antigen-binding fragment thereof described in, e.g., PCT publication WO2012/079000; PCT publication WO2014/153270; Kochenderfer, J.N. et al., J. Immunother. 32 (7), 689-702 (2009); Kochenderfer, J.N., et al., Blood, 116 (20), 4099-4102 (2010); PCT publication WO2014/031687; Bejcek, Cancer Research, 55, 2346-2351, 1995; or U.S. Patent No. 7,446,190.

In one embodiment, the CD19 CAR includes a CAR molecule, or an antigen binding domain (e.g., a humanized antigen binding domain) according to Table 3 of WO2014/153270. The amino acid and nucleotide sequences encoding the CD19 CAR molecules and antigen binding domains (e.g., including one, two, three VH CDRs; and one, two, three VL CDRs according to Kabat or Chothia), are specified in WO2014/153270. In embodiments, the CD19 CAR, or antigen binding domain, comprises an amino acid, or has a nucleotide sequence shown in WO2014/153270, or a sequence substantially identical to any of the aforesaid sequences (e.g., at least 85%, 90%, 95% or more identical to any of the aforesaid sequences).

In another embodiment, the antigen binding domain comprises an anti-CD19 antibody, or fragment thereof, e.g., an scFv. For example, the antigen binding domain comprises a variable heavy chain and a variable light chain listed in Tables 6-9, or a sequence substantially identical to any of the aforesaid sequences (e.g., at least 85%, 90%, 95% or more identical to any of the aforesaid sequences). The linker sequence joining the variable heavy and variable light chains can be, e.g., any of the linker sequences described herein, or alternatively, can be GSTSGSGKPGSGEGSTKG (SEQ ID NO: 871).

**Table 6: Anti-CD19 antibody binding domains**

| | | |
|---|---|---|
| CD19 | huscFv1 (SEQ ID NO: 872) | EIVMTQSPATLSLSPGERATLSCRASQDISKYLNWYQQKPGQAPRLLIYHTSRL HSGIPARFSGSGSGTDYTLTISSLQPEDFAVYFCQQGNTLPYTFGQGTKLEIKG GGGSGGGGSGGGGSQVQLQESGPGLVKPSETLSLTCTVSGVSLPDYGVSWIRQP PGKGLEWIGVIWGSETTYYSSSLKSRVTISKDNSKNQVSLKLSSVTAADTAVYY CAKHYYYGGSYAMDYWGQGTLVTVSS |
| CD19 | huscFv2 (SEQ ID NO: 873) | Eivmtqspatlslspgeratlscrasqdiskylnwyqqkpgqaprlliyhtsrlhsgip arfsgsgsgtdytltisslqpedfavyfcqqgntlpytfgqgtkleikggggsggggsg gggsqvqlqesgpglvkpsetlsltctvsgvslpdygvswirqppgkglewigviwgse |
| | | ttyyqsslksrvtiskdnsknqvslklssvtaadtavyycakhyyyggsyamdywgqgt lvtvss |
| CD19 | huscFv3 (SEQ ID NO: 874) | Qvqlqesgpglvkpsetlsltctvsgvslpdygvswirqppgkglewigviwgsettyy sssslksrvtiskdnsknqvslklssvtaadtavyycakhyyyggsyamdywgqgtlvtv ssggggsggggsggggseivmtqspatlslspgeratlscrasqdiskylnwyqqkpgq aprllliyhtsrlhsgiparfsgsgsgtdytltisslqpedfavyfcqqgntlpytfgqg tkleik |
| CD19 | huscFv4 (SEQ ID NO: 875) | Qvqlqesgpglvkpsetlsltctvsgvslpdygvswirqppgkglewigviwgsettyy qsslksrvtiskdnsknqvslklssvtaadtavyycakhyyyggsyamdywgqgtlvtv ssggggsggggsggggseivmtqspatlslspgeratlscrasqdiskylnwyqqkpgq aprlliyhtsrlhsgiparfsgsgsgtdytltisslqpedfavyfcqqgntlpytfgqg tkleik |
| CD19 | huscFv5 (SEQ ID NO: 876) | Eivmtqspatlslspgeratlscrasqdiskylnwyqqkpgqaprlliyhtsrlhsgip arfsgsgsgtdytltisslqpedfavyfcqqgntlpytfgqgtkleikggggsggggsg gggsggggsqvqlqesgpglvkpsetlsltctvsgvslpdygvswirqppgkglewigv iwgsettyyssslksrvtiskdnsknqvslklssvtaadtavyycakhyyyggsyamdy wgqgtlvtvss |
| CD19 | huscFv6 (SEQ ID NO: 877) | Eivmtqspatlslspgeratlscrasqdiskylnwyqqkpgqaprlliyhtsrlhsgip arfsgsgsgtdytltisslqpedfavyfcqqgntlpytfgqgtkleikggggsggggsg gggsggggsqvqlqesgpglvkpsetlsltctvsgvslpdygvswirqppgkglewigv iwgsettyyqsslksrvtiskdnsknqvslklssvtaadtavyycakhyyyggsyamdy wgqgtlvtvss |
| CD19 | huscFv7 (SEQ ID NO: 878) | Qvqlqesgpglvkpsetlsltctvsgvslpdygvswirqppgkglewigviwgsettyy ssslksrvtiskdnsknqvslklssvtaadtavyycakhyyyggsyamdywgqgtlvtv ssggggsggggsggggsggggseivmtqspatlslspgeratlscrasqdiskylnwyq qkpgqaprllliyhtsrlhsgiparfsgsgsgtdytltisslqpedfavyfcqqgntlpy tfgqgtkleik |
| CD19 | huscFv8 (SEQ ID NO: 879) | Qvqlqesgpglvkpsetlsltctvsgvslpdygvswirqppgkglewigviwgsettyy qsslksrvtiskdnsknqvslklssvtaadtavyycakhyyyggsyamdywgqgtlvtv ssggggsggggsggggsggggseivmtqspatlslspgeratlscrasqdiskylnwyq qkpgqaprlliyhtsrlhsgiparfsgsgsgtdytltisslqpedfavyfcqqgntlpy tfgqgtkleik |
| CD19 | huscFv9 (SEQ ID NO: 880) | Eivmtqspatlslspgeratlscrasqdiskylnwyqqkpgqaprlliyhtsrlhsgip arfsgsgsgtdytltisslqpedfavyfcqqgntlpytfgqgtkleikggggsggggsg gggsggggsqvqlqesgpglvkpsetlsltctvsgvslpdygvswirqppgkglewigv iwgsettyynsslksrvtiskdnsknqvslklssvtaadtavyycakhyyyggsyamdy wgqgtlvtvss |
| CD19 | Hu scfv 10 (SEQ ID NO: 881) | Qvqlqesgpglvkpsetlsltctvsgvslpdygvswirqppgkglewigviwgsettyy nsslksrvtiskdnsknqvslklssvtaadtavyycakhyyyggsyamdywgqgtlvtv ssggggsggggsggggsggggseivmtqspatlslspgeratlscrasqdiskylnwyq qkpgqaprlliyhtsrlhsgiparfsgsgsgtdytltisslqpedfavyfcqqgntlpy tfgqgtkleik |
| CD19 | Hu scFv11 (SEQ ID NO: 882) | Eivmtqspatlslspgeratlscrasqdiskylnwyqqkpgqaprlliyhtsrlhsgip arfsgsgsgtdytltisslqpedfavyfcqqgntlpytfgqgtkleikggggsggggsg gggsqvqlqesgpglvkpsetlsltctvsgvslpdygvswirqppgkglewigviwgse ttyynsslksrvtiskdnsknqvslklssvtaadtavyycakhyyyggsyamdywgqgt lvtvss |
| CD19 | Hu scfv 12 (SEQ ID NO: 883) | Qvqlqesgpglvkpsetlsltctvsgvslpdygvswirqppgkglewigviwgsettyy nsslksrvtiskdnsknqvslklssvtaadtavyycakhyyyggsyamdywgqgtlvtv ssggggsggggsggggseivmtqspatlslspgeratlscrasqdiskylnwyqqkpgq aprlliyhtsrlhsgiparfsgsgsgtdytltisslqpedfavyfcqqgntlpytfgqg tkleik |
| CD19 | muCTL0 19 (SEQ ID NO: 884) | Diqmtqttsslsaslgdrvtiscrasqdiskylnwyqqkpdgtvklliyhtsrlhsgvp srfsgsgsgtdysltisnleqediatyfcqqgntlpytfgggtkleitggggsggggsg gggsevklqesgpglvapsqslsvtctvsgvslpdygvswirqpprkglewlgviwgse |
| | | ttyynsalksrltiikdnsksqvflkmnslqtddtaiyycakhyyyggsyamdywgqgt svtvss |

**Table 7: Additional anti-CD19 antibody binding domains**

| Antibody | VH Sequence | VL Sequence |
|---|---|---|
| SSJ25-C1 | QVQLLESGAELVRPGSSVKISCKASGYAFSS YWMNWVKQRPGQGLEWIGQIYPGDGDTNYNG KFKGQATLTADKSSSTAYMQLSGLTSEDSAV YSCARKTISSVVDFYFDYWGQGTTVT (SEQ ID NO: 885) | ELVLTQSPKFMSTSVGDRVSVTCKASQNVGTNVA WYQQKPGQSPKPLIYSATYRNSGVPDRFTGSGSG TDFTLTITNVQSKDLADYFYFCQYNRYPYTSGGG TKLEIKRRS (SEQ ID NO: 886) |

**Table 8: Additional murine anti-CD19 antibody binding domains**

| | | |
|---|---|---|
| **mCAR1 scFv** | SEQ ID NO: 887 | QVQLLESGAELVRPGSSVKISCKASGYAFSSYWMNWVKQRPGQGLEWIGQIYPGDG DTNYNGKFKGQATLTADKSSSTAYMQLSGLTSEDSAVYSCARKTISSVVDFYFDYW GQGTTVTGGGSGGGSGGGSGGGSELVLTQSPKFMSTSVGDRVSVTCKASQNVGTNV AWYQQKPGQSPKPLIYSATYRNSGVPDRFTGSGSGTDFTLTITNVQSKDLADYFCQ YNRYPYTSFFFTKLEIKRRS |
| **mCAR2 scFv** | SEQ ID NO: 888 | DIQMTQTTSSLSASLGDRVTISCRASQDISKYLNWYQQKPDGTVKLLIYHTSRLHS GVPSRFSGSGSGTDYSLTISNLEQEDIATYFCQQGNTLPYTFGGGTKLEITGSTSG SGKPGSGEGSTKGEVKLQESGPGLVAPSQSLSVTCTVSGVSLPDYGVSWIRQPPRK GLEWLGVIWGSETTYYNSALKSRLTIIKDNSKSQVFLKMNSLQTDDTAIYYCAKHY YYGGSYAMDYWGQGTSVTVSSE |
| **mCAR3 scFv** | SEQ ID NO: 889 | DIQMTQTTSSLSASLGDRVTISCRASQDISKYLNWYQQKPDGTVKLLIYHTSRLHS GVPSRFSGSGSGTDYSLTISNLEQEDIATYFCQQGNTLPYTFGGGTKLEITGSTSG SGKPGSGEGSTKGEVKLQESGPGLVAPSQSLSVTCTVSGVSLPDYGVSWIRQPPRK GLEWLGVIWGSETTYYNSALKSRLTIIKDNSKSQVFLKMNSLQTDDTAIYYCAKHY YYGGSYAMDYWGQGTSVTVSS |

Any CD19 CAR, e.g., the CD19 antigen binding domain of any known CD19 CAR, can be used in accordance with the present disclosure. For example, LG-740; CD19 CAR described in the US Pat. No. 8,399,645; US Pat. No. 7,446,190; Xu et al., Leuk Lymphoma. 2013 54(2):255-260(2012); Cruz et al., Blood 122(17):2965-2973 (2013); Brentjens et al., Blood, 118(18):4817-4828 (2011); Kochenderfer et al., Blood 116(20):4099-102 (2010); Kochenderfer et al., Blood 122 (25):4129-39(2013); and 16th Annu Meet Am Soc Gen Cell Ther (ASGCT) (May 15-18, Salt Lake City) 2013, Abst 10.

In one embodiment, an antigen binding domain against EGFRvIII is an antigen binding portion, e.g., CDRs, of a CAR, antibody or antigen-binding fragment thereof described in, e.g., PCT publication WO2014/130657 or US2014/0322275A1. In one embodiment, the CAR molecule comprises an EGFRvIII CAR, or an antigen binding domain according to Table 2 or SEQ ID NO:11 of WO 2014/130657, or a sequence substantially identical thereto (e.g., at least 85%, 90%, 95% or more identical thereto). The amino acid and nucleotide sequences encoding the EGFRvIII CAR molecules and antigen binding domains (e.g., including one, two, three VH CDRs; and one, two, three VL CDRs according to Kabat or Chothia), are specified in WO 2014/130657.

In one embodiment, an antigen binding domain against mesothelin is an antigen binding portion, e.g., CDRs, of an antibody, antigen-binding fragment or CAR described in, e.g., PCT publication WO2015/090230. In one embodiment, an antigen binding domain against mesothelin is an antigen binding portion, e.g., CDRs, of an antibody, antigen-binding fragment, or CAR described in, e.g., PCT publication WO1997/025068, WO1999/028471, WO2005/014652, WO2006/099141, WO2009/045957, WO2009/068204, WO2013/142034, WO2013/040557, or WO2013/063419.

In an embodiment, the CAR molecule comprises a mesothelin CAR described herein, e.g., a mesothelin CAR described in WO 2015/090230. In embodiments, the mesothelin CAR comprises an amino acid, or has a nucleotide sequence shown in WO 2015/090230, or a sequence substantially identical to any of the aforesaid sequences (e.g., at least 85%, 90%, 95% or more identical to any of the aforesaid mesothelin CAR sequences). In one embodiment, the CAR molecule comprises a mesothelin CAR, or an antigen binding domain according to Tables 2-3 of WO 2015/090230, or a sequence substantially identical thereto (e.g., at least 85%, 90%, 95% or more identical thereto). The amino acid and nucleotide sequences encoding the mesothelin CAR molecules and antigen binding domains (e.g., including one, two, three VH CDRs; and one, two, three VL CDRs according to Kabat or Chothia), are specified in WO 2015/090230.

In one embodiment, an antigen binding domain against CD123 is an antigen binding portion, e.g., CDRs, of an antibody, antigen-binding fragment or CAR described in, e.g., PCT publication WO2016/028896. In one embodiment, an antigen binding domain against CD123 is an antigen binding portion, e.g., CDRs, of an antibody, antigen-binding fragment or CAR described in, e.g., PCT publication WO2014/130635. In one embodiment, an antigen binding domain against CD123 is an antigen binding portion, e.g., CDRs, of an antibody, antigen-binding fragment, or CAR described in, e.g., PCT publication WO2014/138805, WO2014/138819, WO2013/173820, WO2014/144622, WO2001/66139, WO2010/126066, WO2014/144622, or US2009/0252742.

In one embodiment, an antigen binding domain against CD123 is an antigen binding portion, e.g., CDRs, of an antibody, antigen-binding fragment or CAR described in, e.g., US2014/0322212A1 or US2016/0068601A1. In embodiments, the CD123 CAR comprises an amino acid, or has a nucleotide sequence shown in US2014/0322212A1 or US2016/0068601A1, or a sequence substantially identical to any of the aforesaid sequences (e.g., at least 85%, 90%, 95% or more identical to any of the aforesaid CD123 CAR sequences). In one embodiment, the CAR molecule comprises a CD123 CAR (e.g., any of the CAR1-CAR8), or an antigen binding domain according to Tables 1-2 of WO 2014/130635, or a sequence substantially identical thereto (e.g., at least 85%, 90%, 95% or more identical to any of the aforesaid CD123 CAR sequences). The amino acid and nucleotide sequences encoding the CD123 CAR molecules and antigen binding domains (e.g., including one, two, three VH CDRs; and one, two, three VL CDRs according to Kabat or Chothia), are specified in WO 2014/130635.

In other embodiments, the CAR molecule comprises a CD123 CAR comprises a CAR molecule (e.g., any of the CAR123-1 to CAR123-4 and hzCAR123-1 to hzCAR123-32), or an antigen binding domain according to Tables 2, 6, and 9 of WO2016/028896, or a sequence substantially identical thereto (e.g., at least 85%, 90%, 95% or more identical to any of the aforesaid CD123 CAR sequences). The amino acid and nucleotide sequences encoding the CD123 CAR molecules and antigen binding domains (e.g., including one, two, three VH CDRs; and one, two, three VL CDRs according to Kabat or Chothia), are specified in WO2016/028896.

In one embodiment, an antigen binding domain against CD22 is an antigen binding portion, e.g., CDRs, of an antibody described in, e.g., Haso et al., Blood, 121(7): 1165-1174 (2013); Wayne et al., Clin Cancer Res 16(6): 1894-1903 (2010); Kato et al., Leuk Res 37(1):83-88 (2013); Creative BioMart (creativebiomart.net): MOM-18047-S(P).

In one embodiment, an antigen binding domain against CS-1 is an antigen binding portion, e.g., CDRs, of Elotuzumab (BMS), see e.g., Tai et al., 2008, Blood 112(4):1329-37; Tai et al., 2007, Blood. 110(5):1656-63.

In one embodiment, an antigen binding domain against CLL-1 is an antigen binding portion, e.g., CDRs, of an antibody available from R&D, ebiosciences, Abcam, for example, PE-CLL1-hu Cat# 353604 (BioLegend); and PE-CLL1 (CLEC12A) Cat# 562566 (BD).

In other embodiments, the CLL1 CAR includes a CAR molecule, or an antigen binding domain according to Table 2 of WO2016/014535. The amino acid and nucleotide sequences encoding the CLL-1 CAR molecules and antigen binding domains (e.g., including one, two, three VH CDRs; and one, two, three VL CDRs according to Kabat or Chothia), are specified in WO2016/014535.

In one embodiment, an antigen binding domain against CD33 is an antigen binding portion, e.g., CDRs, of an antibody described in, e.g., Bross et al., Clin Cancer Res 7(6):1490-1496 (2001) (Gemtuzumab Ozogamicin, hP67.6),Caron et al., Cancer Res 52(24):6761-6767 (1992) (Lintuzumab, HuM195), Lapusan et al., Invest New Drugs 30(3):1121-1131 (2012) (AVE9633), Aigner et al., Leukemia 27(5): 1107-1115 (2013) (AMG330, CD33 BiTE), Dutour et al., Adv hematol 2012:683065 (2012), and Pizzitola et al., Leukemia doi:10.1038/Lue.2014.62 (2014).

In one embodiment, an antigen binding domain against CD33 is an antigen binding portion, e.g., CDRs, of an antibody described in, US2016/0096892A1. In embodiments, the CD33 CAR comprises an amino acid, or has a nucleotide sequence shown in US2016/0096892A1, or a sequence substantially identical to any of the aforesaid sequences (e.g., at least 85%, 90%, 95% or more identical to any of the aforesaid CD33 CAR sequences). In other embodiments, the CD33 CAR CAR or antigen binding domain thereof can include a CAR molecule (e.g., any of CAR33-1 to CAR-33-9), or an antigen binding domain according to Table 2 or 9 of WO2016/014576, or a sequence substantially identical to any of the aforesaid sequences (e.g., at least 85%, 90%, 95% or more identical to any of the aforesaid CD33 CAR sequences). The amino acid and nucleotide sequences encoding the CD33 CAR molecules and antigen binding domains (e.g., including one, two, three VH CDRs; and one, two, three VL CDRs according to Kabat or Chothia), are specified in WO2016/014576.

In one embodiment, an antigen binding domain against GD2 is an antigen binding portion, e.g., CDRs, of an antibody described in, e.g., Mujoo et al., Cancer Res. 47(4):1098-1104 (1987); Cheung et al., Cancer Res 45(6):2642-2649 (1985), Cheung et al., J Clin Oncol 5(9):1430-1440 (1987), Cheung et al., J Clin Oncol 16(9):3053-3060 (1998), Handgretinger et al., Cancer Immunol Immunother 35(3):199-204 (1992). In some embodiments, an antigen binding domain against GD2 is an antigen binding portion of an antibody selected from mAb 14.18, 14G2a, ch14.18, hu14.18, 3F8, hu3F8, 3G6, 8B6, 60C3, 10B8, ME36.1, and 8H9, see e.g., WO2012033885, WO2013040371, WO2013192294, WO2013061273, WO2013123061, WO2013074916, and WO201385552. In some embodiments, an antigen binding domain against GD2 is an antigen binding portion of an antibody described in US Publication No.: 20100150910 or PCT Publication No.: WO 2011160119.

In one embodiment, an antigen binding domain against BCMA is an antigen binding portion, e.g., CDRs, of an antibody, antigen-binding fragment or CAR described in, e.g., PCT publication WO2016/014565, e.g., the antigen binding portion of CAR BCMA-10 as described in WO2016/014565. In one embodiment, an antigen binding domain against BCMA is an antigen binding portion, e.g., CDRs, of an antibody, antigen-binding fragment or CAR described in, e.g., PCT publication WO2016/014789. In one embodiment, an antigen binding domain against BCMA is an antigen binding portion, e.g., CDRs, of an antibody described in, e.g., WO2012/163805, WO2001/12812, and WO2003/062401.

In other embodiment, the CAR molecule comprises a BCMA CAR molecule, or an antigen binding domain against BCMA described herein, e.g., a BCMA CAR described in US-2016-0046724-A1 or WO2016/014565. In embodiments, the BCMA CAR comprises an amino acid, or has a nucleotide sequence of a CAR molecule, or an antigen binding domain according to US-2016-0046724-A1, or Table 1 or 16, SEQ ID NO: 271 or SEQ ID NO: 273 of WO2016/014565, or a sequence substantially identical to any of the aforesaid sequences (e.g., at least 85%, 90%, 95% or more identical to any of the aforesaid BCMA CAR sequences). The amino acid and nucleotide sequences encoding the BCMA CAR molecules and antigen binding domains (e.g., including one, two, three VH CDRs; and one, two, three VL CDRs according to Kabat or Chothia), are specified in WO2016/014565.

In one embodiment, an antigen binding domain against GFR ALPHA-4 CAR antigen is an antigen binding portion, e.g., CDRs, of an antibody described in, e.g., WO2016/025880. In one embodiment, the CAR molecule comprises an a GFR ALPHA-4 CAR, e.g., a CAR molecule, or an antigen binding domain according to Table 2 of WO2016/025880, or a sequence substantially identical to any of the aforesaid sequences (e.g., at least 85%, 90%, 95% or more identical to any of the aforesaid GFR ALPHA-4 sequences). The amino acid and nucleotide sequences encoding the GFR ALPHA-4 CAR molecules and antigen binding domains (e.g., including one, two, three VH CDRs; and one, two, three VL CDRs according to Kabat or Chothia), are specified in WO2016/025880.

In one embodiment, an antigen binding domain against Tn antigen is an antigen binding portion, e.g., CDRs, of an antibody described in, e.g., US8,440,798; Brooks et al., PNAS 107(22):10056-10061 (2010), and Stone et al., Oncolmmunology 1(6):863-873(2012).

In one embodiment, an antigen binding domain against PSMA is an antigen binding portion, e.g., CDRs, of an antibody described in, e.g., Parker et al., Protein Expr Purif 89(2):136-145 (2013), US 20110268656 (J591 ScFv); Frigerio et al, European J Cancer 49(9):2223-2232 (2013) (scFvD2B); WO 2006125481 (mAbs 3/A12, 3/E7 and 3/F11) and single chain antibody fragments (scFv A5 and D7).

In one embodiment, an antigen binding domain against ROR1 is an antigen binding portion, e.g., CDRs, of an antibody described in, e.g., Hudecek et al., Clin Cancer Res 19(12):3153-3164 (2013); WO 2011159847; and US20130101607.

In one embodiment, an antigen binding domain against FLT3 is an antigen binding portion, e.g., CDRs, of an antibody described in, e.g., WO2011076922, US5777084, EP0754230, US20090297529, and several commercial catalog antibodies (R&D, ebiosciences, Abcam).

In one embodiment, an antigen binding domain against TAG72 is an antigen binding portion, e.g., CDRs, of an antibody described in, e.g., Hombach et al., Gastroenterology 113(4):1163-1170 (1997); and Abcam ab691.

In one embodiment, an antigen binding domain against FAP is an antigen binding portion, e.g., CDRs, of an antibody described in, e.g., Ostermann et al., Clinical Cancer Research 14:4584-4592 (2008) (FAP5), US Pat. Publication No. 2009/0304718; sibrotuzumab (see e.g., Hofheinz et al., Oncology Research and Treatment 26(1), 2003); and Tran et al., J Exp Med 210(6):1125-1135 (2013).

In one embodiment, an antigen binding domain against CD38 is an antigen binding portion, e.g., CDRs, of daratumumab (see, e.g., Groen et al., Blood 116(21):1261-1262 (2010); MOR202 (see, e.g., US8,263,746); or antibodies described in US8,362,211.

In one embodiment, an antigen binding domain against CD44v6 is an antigen binding portion, e.g., CDRs, of an antibody described in, e.g., Casucci et al., Blood 122(20):3461-3472 (2013).

In one embodiment, an antigen binding domain against CEA is an antigen binding portion, e.g., CDRs, of an antibody described in, e.g., Chmielewski et al., Gastoenterology 143(4):1095-1107 (2012).

In one embodiment, an antigen binding domain against EPCAM is an antigen binding portion, e.g., CDRS, of an antibody selected from MT110, EpCAM-CD3 bispecific Ab (see, e.g., clinicaltrials.gov/ct2/show/NCT00635596); Edrecolomab; 3622W94; ING-1; and adecatumumab (MT201).

In one embodiment, an antigen binding domain against PRSS21 is an antigen binding portion, e.g., CDRs, of an antibody described in US Patent No.: 8,080,650.

In one embodiment, an antigen binding domain against B7H3 is an antigen binding portion, e.g., CDRs, of an antibody MGA271 (Macrogenics).

In one embodiment, an antigen binding domain against KIT is an antigen binding portion, e.g., CDRs, of an antibody described in, e.g., US7915391, US20120288506 , and several commercial catalog antibodies.

In one embodiment, an antigen binding domain against IL-13Ra2 is an antigen binding portion, e.g., CDRs, of an antibody described in, e.g., WO2008/146911, WO2004087758, several commercial catalog antibodies, and WO2004087758.

In one embodiment, an antigen binding domain against CD30 is an antigen binding portion, e.g., CDRs, of an antibody described in, e.g., US7090843 B1, and EP0805871.

In one embodiment, an antigen binding domain against GD3 is an antigen binding portion, e.g., CDRs, of an antibody described in, e.g., US7253263; US 8,207,308; US 20120276046; EP1013761; WO2005035577; and US6437098.

In one embodiment, an antigen binding domain against CD171 is an antigen binding portion, e.g., CDRs, of an antibody described in, e.g., Hong et al., J Immunother 37(2):93-104 (2014).

In one embodiment, an antigen binding domain against IL-11Ra is an antigen binding portion, e.g., CDRs, of an antibody available from Abcam (cat# ab55262) or Novus Biologicals (cat# EPR5446). In another embodiment, an antigen binding domain again IL-11Ra is a peptide, see, e.g., Huang et al., Cancer Res 72(1):271-281 (2012).

In one embodiment, an antigen binding domain against PSCA is an antigen binding portion, e.g., CDRs, of an antibody described in, e.g., Morgenroth et al., Prostate 67(10):1121-1131 (2007) (scFv 7F5); Nejatollahi et al., J of Oncology 2013(2013), article ID 839831 (scFv C5-II); and US Pat Publication No. 20090311181.

In one embodiment, an antigen binding domain against VEGFR2 is an antigen binding portion, e.g., CDRs, of an antibody described in, e.g., Chinnasamy et al., J Clin Invest 120(11):3953-3968 (2010).

In one embodiment, an antigen binding domain against LewisY is an antigen binding portion, e.g., CDRs, of an antibody described in, e.g., Kelly et al., Cancer Biother Radiopharm 23(4):411-423 (2008) (hu3S193 Ab (scFvs)); Dolezal et al., Protein Engineering 16(1):47-56 (2003) (NC10 scFv).

In one embodiment, an antigen binding domain against CD24 is an antigen binding portion, e.g., CDRs, of an antibody described in, e.g., Maliar et al., Gastroenterology 143(5):1375-1384 (2012).

In one embodiment, an antigen binding domain against PDGFR-beta is an antigen binding portion, e.g., CDRs, of an antibody Abcam ab32570.

In one embodiment, an antigen binding domain against SSEA-4 is an antigen binding portion, e.g., CDRs, of antibody MC813 (Cell Signaling), or other commercially available antibodies.

In one embodiment, an antigen binding domain against CD20 is an antigen binding portion, e.g., CDRs, of the antibody Rituximab, Ofatumumab, Ocrelizumab, Veltuzumab, or GA101.

In one embodiment, an antigen binding domain against Folate receptor alpha is an antigen binding portion, e.g., CDRs, of the antibody IMGN853, or an antibody described in US20120009181; US4851332, LK26: US5952484.

In one embodiment, an antigen binding domain against ERBB2 (Her2/neu) is an antigen binding portion, e.g., CDRs, of the antibody trastuzumab, or pertuzumab.

In one embodiment, an antigen binding domain against MUC1 is an antigen binding portion, e.g., CDRs, of the antibody SAR566658.

In one embodiment, the antigen binding domain against EGFR is antigen binding portion, e.g., CDRs, of the antibody cetuximab, panitumumab, zalutumumab, nimotuzumab, or matuzumab.

In one embodiment, an antigen binding domain against NCAM is an antigen binding portion, e.g., CDRs, of the antibody clone 2-2B: MAB5324 (EMD Millipore).

In one embodiment, an antigen binding domain against Ephrin B2 is an antigen binding portion, e.g., CDRs, of an antibody described in, e.g., Abengozar et al., Blood 119(19):4565-4576 (2012).

In one embodiment, an antigen binding domain against IGF-I receptor is an antigen binding portion, e.g., CDRs, of an antibody described in, e.g., US8344112 B2; EP2322550 A1; WO 2006/138315, or PCT/US2006/022995.

In one embodiment, an antigen binding domain against CAIX is an antigen binding portion, e.g., CDRs, of the antibody clone 303123 (R&D Systems).

In one embodiment, an antigen binding domain against LMP2 is an antigen binding portion, e.g., CDRs, of an antibody described in, e.g., US7,410,640, or US20050129701.

In one embodiment, an antigen binding domain against gp100 is an antigen binding portion, e.g., CDRs, of the antibody HMB45, NKIbetaB, or an antibody described in WO2013165940, or US20130295007

In one embodiment, an antigen binding domain against tyrosinase is an antigen binding portion, e.g., CDRs, of an antibody described in, e.g., US5843674; or US19950504048.

In one embodiment, an antigen binding domain against EphA2 is an antigen binding portion, e.g., CDRs, of an antibody described in, e.g., Yu et al., Mol Ther 22(1):102-111 (2014).

In one embodiment, an antigen binding domain against GD3 is an antigen binding portion, e.g., CDRs, of an antibody described in, e.g., US7253263; US 8,207,308; US 20120276046; EP1013761 A3; 20120276046; WO2005035577; or US6437098.

In one embodiment, an antigen binding domain against fucosyl GM1 is an antigen binding portion, e.g., CDRs, of an antibody described in, e.g., US20100297138; or WO2007/067992.

In one embodiment, an antigen binding domain against sLe is an antigen binding portion, e.g., CDRs, of the antibody G193 (for lewis Y), see Scott AM et al, Cancer Res 60: 3254-61 (2000), also as described in Neeson et al, J Immunol May 2013 190 (Meeting Abstract Supplement) 177.10.

In one embodiment, an antigen binding domain against GM3 is an antigen binding portion, e.g., CDRs, of the antibody CA 2523449 (mAb 14F7).

In one embodiment, an antigen binding domain against HMWMAA is an antigen binding portion, e.g., CDRs, of an antibody described in, e.g., Kmiecik et al., Oncoimmunology 3(1):e27185 (2014) (PMID: 24575382) (mAb9.2.27); US6528481; WO2010033866; or US 20140004124.

In one embodiment, an antigen binding domain against o-acetyl-GD2 is an antigen binding portion, e.g., CDRs, of the antibody 8B6.

In one embodiment, an antigen binding domain against TEM1/CD248 is an antigen binding portion, e.g., CDRs, of an antibody described in, e.g., Marty et al., Cancer Lett 235(2):298-308 (2006); Zhao et al., J Immunol Methods 363(2):221-232 (2011).

In one embodiment, an antigen binding domain against CLDN6 is an antigen binding portion, e.g., CDRs, of the antibody IMAB027 (Ganymed Pharmaceuticals), see e.g., clinicaltrial.gov/show/NCT02054351.

In one embodiment, an antigen binding domain against TSHR is an antigen binding portion, e.g., CDRs, of an antibody described in, e.g., US8,603,466; US8,501,415; or US8,309,693.

In one embodiment, an antigen binding domain against GPRC5D is an antigen binding portion, e.g., CDRs, of the antibody FAB6300A (R&D Systems); or LS-A4180 (Lifespan Biosciences).

In one embodiment, an antigen binding domain against CD97 is an antigen binding portion, e.g., CDRs, of an antibody described in, e.g., US6,846,911;de Groot et al., J Immunol 183(6):4127-4134 (2009); or an antibody from R&D:MAB3734.

In one embodiment, an antigen binding domain against ALK is an antigen binding portion, e.g., CDRs, of an antibody described in, e.g., Mino-Kenudson et al., Clin Cancer Res 16(5):1561-1571 (2010).

In one embodiment, an antigen binding domain against polysialic acid is an antigen binding portion, e.g., CDRs, of an antibody described in, e.g., Nagae et al., J Biol Chem 288(47):33784-33796 (2013).

In one embodiment, an antigen binding domain against PLAC1 is an antigen binding portion, e.g., CDRs, of an antibody described in, e.g., Ghods et al., Biotechnol Appl Biochem 2013 doi:10.1002/bab.1177.

In one embodiment, an antigen binding domain against GloboH is an antigen binding portion of the antibody VK9; or an antibody described in, e.g., Kudryashov V et al, Glycoconj J.15(3):243-9 ( 1998), Lou et al., Proc Natl Acad Sci USA 111(7):2482-2487 (2014) ; MBr1: Bremer E-G et al. J Biol Chem 259:14773-14777 (1984).

In one embodiment, an antigen binding domain against NY-BR-1 is an antigen binding portion, e.g., CDRs of an antibody described in, e.g., Jager et al., Appl Immunohistochem Mol Morphol 15(1):77-83 (2007).

In one embodiment, an antigen binding domain against WT-1 is an antigen binding portion, e.g., CDRs, of an antibody described in, e.g., Dao et al., Sci Transl Med 5(176):176ra33 (2013); or WO2012/135854.

In one embodiment, an antigen binding domain against MAGE-A1 is an antigen binding portion, e.g., CDRs, of an antibody described in, e.g., Willemsen et al., J Immunol 174(12):7853-7858 (2005) (TCR-like scFv).

In one embodiment, an antigen binding domain against sperm protein 17 is an antigen binding portion, e.g., CDRs, of an antibody described in, e.g., Song et al., Target Oncol 2013 Aug 14 (PMID: 23943313); Song et al., Med Oncol 29(4):2923-2931 (2012).

In one embodiment, an antigen binding domain against Tie 2 is an antigen binding portion, e.g., CDRs, of the antibody AB33 (Cell Signaling Technology).

In one embodiment, an antigen binding domain against MAD-CT-2 is an antigen binding portion, e.g., CDRs, of an antibody described in, e.g., PMID: 2450952; US7635753.

In one embodiment, an antigen binding domain against Fos-related antigen 1 is an antigen binding portion, e.g., CDRs, of the antibody 12F9 (Novus Biologicals).

In one embodiment, an antigen binding domain against MelanA/MART1 is an antigen binding portion, e.g., CDRs, of an antibody described in, EP2514766 A2; or US 7,749,719.

In one embodiment, an antigen binding domain against sarcoma translocation breakpoints is an antigen binding portion, e.g., CDRs, of an antibody described in, e.g., Luo et al, EMBO Mol. Med. 4(6):453-461 (2012).

In one embodiment, an antigen binding domain against TRP-2 is an antigen binding portion, e.g., CDRs, of an antibody described in, e.g., Wang et al, J Exp Med. 184(6):2207-16 (1996).

In one embodiment, an antigen binding domain against CYP1B1 is an antigen binding portion, e.g., CDRs, of an antibody described in, e.g., Maecker et al, Blood 102 (9): 3287-3294 (2003).

In one embodiment, an antigen binding domain against RAGE-1 is an antigen binding portion, e.g., CDRs, of the antibody MAB5328 (EMD Millipore).

In one embodiment, an antigen binding domain against human telomerase reverse transcriptase is an antigen binding portion, e.g., CDRs, of the antibody cat no: LS-B95-100 (Lifespan Biosciences)

In one embodiment, an antigen binding domain against intestinal carboxyl esterase is an antigen binding portion, e.g., CDRs, of the antibody 4F12: cat no: LS-B6190-50 (Lifespan Biosciences).

In one embodiment, an antigen binding domain against mut hsp70-2 is an antigen binding portion, e.g., CDRs, of the antibody Lifespan Biosciences: monoclonal: cat no: LS-C133261-100 (Lifespan Biosciences).

In one embodiment, an antigen binding domain against CD79a is an antigen binding portion, e.g., CDRs, of the antibody Anti-CD79a antibody [HM47/A9] (ab3121), available from Abcam; antibody CD79A Antibody #3351 available from Cell Signalling Technology; or antibody HPA017748 - Anti-CD79A antibody produced in rabbit, available from Sigma Aldrich.

In one embodiment, an antigen binding domain against CD79b is an antigen binding portion, e.g., CDRs, of the antibody polatuzumab vedotin, anti-CD79b described in Dornan et al., "Therapeutic potential of an anti-CD79b antibody-drug conjugate, anti-CD79b-vc-MMAE, for the treatment of non-Hodgkin lymphoma" Blood. 2009 Sep 24;114(13):2721-9. doi: 10.1182/blood-2009-02-205500. Epub 2009 Jul 24, or the bispecific antibody Anti-CD79b/CD3 described in "4507 Pre-Clinical Characterization of T Cell-Dependent Bispecific Antibody Anti-CD79b/CD3 As a Potential Therapy for B Cell Malignancies" Abstracts of 56th ASH Annual Meeting and Exposition, San Francisco, CA December 6-9 2014.

In one embodiment, an antigen binding domain against CD72 is an antigen binding portion, e.g., CDRs, of the antibody J3-109 described in Myers, and Uckun, "An anti-CD72 immunotoxin against therapy-refractory B-lineage acute lymphoblastic leukemia." Leuk Lymphoma. 1995 Jun;18(1-2):119-22, or anti-CD72 (10D6.8.1, mlgG1) described in Polson et al., "Antibody-Drug Conjugates for the Treatment of Non-Hodgkin's Lymphoma: Target and Linker-Drug Selection" Cancer Res March 15, 2009 69; 2358.

In one embodiment, an antigen binding domain against LAIR1 is an antigen binding portion, e.g., CDRs, of the antibody ANT-301 LAIR1 antibody, available from ProSpec; or anti-human CD305 (LAIR1) Antibody, available from BioLegend.

In one embodiment, an antigen binding domain against FCAR is an antigen binding portion, e.g., CDRs, of the antibody CD89/FCARAntibody (Catalog#10414-H08H), available from Sino Biological Inc.

In one embodiment, an antigen binding domain against LILRA2 is an antigen binding portion, e.g., CDRs, of the antibody LILRA2 monoclonal antibody (M17), clone 3C7, available from Abnova, or Mouse Anti-LILRA2 antibody, Monoclonal (2D7), available from Lifespan Biosciences..

In one embodiment, an antigen binding domain against CD300LF is an antigen binding portion, e.g., CDRs, of the antibody Mouse Anti-CMRF35-like molecule 1 antibody, Monoclonal[UP-D2], available from BioLegend, or Rat Anti-CMRF35-like molecule 1 antibody, Monoclonal[234903], available from R&D Systems..

In one embodiment, an antigen binding domain against CLEC12A is an antigen binding portion, e.g., CDRs, of the antibody Bispecific T cell Engager (BiTE) scFv-antibody and ADC described in Noordhuis et al., "Targeting of CLEC12A In Acute Myeloid Leukemia by Antibody-Drug-Conjugates and Bispecific CLL-1xCD3 BiTE Antibody" 53rd ASH Annual Meeting and Exposition, December 10-13, 2011, and MCLA-117 (Merus).

In one embodiment, an antigen binding domain against BST2 (also called CD317) is an antigen binding portion, e.g., CDRs, of the antibody Mouse Anti-CD317 antibody, Monoclonal[3H4], available from Antibodies-Online or Mouse Anti-CD317 antibody, Monoclonal[696739], available from R&D Systems.

In one embodiment, an antigen binding domain against EMR2 (also called CD312) is an antigen binding portion, e.g., CDRs, of the antibody Mouse Anti-CD312 antibody, Monoclonal[LS-B8033] available from Lifespan Biosciences, or Mouse Anti-CD312 antibody, Monoclonal[494025] available from R&D Systems.

In one embodiment, an antigen binding domain against LY75 is an antigen binding portion, e.g., CDRs, of the antibody Mouse Anti-Lymphocyte antigen 75 antibody, Monoclonal[HD30] available from EMD Millipore or Mouse Anti-Lymphocyte antigen 75 antibody, Monoclonal[A15797] available from Life Technologies.

In one embodiment, an antigen binding domain against GPC3 is an antigen binding portion, e.g., CDRs, of the antibody hGC33 described in Nakano K, Ishiguro T, Konishi H, et al. Generation of a humanized anti-glypican 3 antibody by CDR grafting and stability optimization. Anticancer Drugs. 2010 Nov;21(10):907-916, or MDX-1414, HN3, or YP7, all three of which are described in Feng et al., "Glypican-3 antibodies: a new therapeutic target for liver cancer." FEBS Lett. 2014 Jan 21;588(2):377-82.

In one embodiment, an antigen binding domain against FCRL5 is an antigen binding portion, e.g., CDRs, of the anti-FcRL5 antibody described in Elkins et al., "FcRL5 as a target of antibody-drug conjugates for the treatment of multiple myeloma" Mol Cancer Ther. 2012 Oct;11(10):2222-32..

In one embodiment, an antigen binding domain against IGLL1 is an antigen binding portion, e.g., CDRs, of the antibody Mouse Anti-Immunoglobulin lambda-like polypeptide 1 antibody, Monoclonal[AT1G4] available from Lifespan Biosciences, Mouse Anti-Immunoglobulin lambda-like polypeptide 1antibody, Monoclonal[HSL11] available from BioLegend.

In one embodiment, the antigen binding domain comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antibody listed above. In one embodiment, the antigen binding domain comprises a heavy chain variable region and/or a variable light chain region of an antibody listed above.

In another aspect, the antigen binding domain comprises a humanized antibody or an antibody fragment. In some aspects, a non-human antibody is humanized, where specific sequences or regions of the antibody are modified to increase similarity to an antibody naturally produced in a human or fragment thereof. In one aspect, the antigen binding domain is humanized.

### Bispecific CARs

In certain embodiments, the antigen binding domain is a bi- or multi- specific molecule (e.g., a multispecific antibody molecule). In an embodiment a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope. In an embodiment the first and second epitopes are on the same antigen, e.g., the same protein (or subunit of a multimeric protein). In an embodiment the first and second epitopes overlap. In an embodiment the first and second epitopes do not overlap. In an embodiment the first and second epitopes are on different antigens, e.g., different proteins (or different subunits of a multimeric protein). In an embodiment a bispecific antibody molecule comprises a heavy chain variable domain sequence and a light chain variable domain sequence which have binding specificity for a first epitope and a heavy chain variable domain sequence and a light chain variable domain sequence which have binding specificity for a second epitope. In an embodiment a bispecific antibody molecule comprises a half antibody having binding specificity for a first epitope and a half antibody having binding specificity for a second epitope. In an embodiment a bispecific antibody molecule comprises a half antibody, or fragment thereof, having binding specificity for a first epitope and a half antibody, or fragment thereof, having binding specificity for a second epitope. In an embodiment a bispecific antibody molecule comprises a scFv, or fragment thereof, have binding specificity for a first epitope and a scFv, or fragment thereof, have binding specificity for a second epitope.

In certain embodiments, the antibody molecule is a multi-specific (e.g., a bispecific or a trispecific) antibody molecule. Such molecules include bispecific fusion proteins, e.g., an expression construct containing two scFvs with a hydrophilic helical peptide linker between them and a full constant region, as described in, e.g., US5637481; minibody constructs with linked VL and VH chains further connected with peptide spacers to an antibody hinge region and CH3 region, which can be dimerized to form bispecific/multivalent molecules, as described in, e.g., US5837821; String of VH domains (or VL domains in family members) connected by peptide linkages with crosslinkable groups at the C-terminus futher associated with VL domains to form a series of FVs (or scFvs), as described in, *e.g.,* US5864019; and single chain binding polypeptides with both a VH and a VL domain linked through a peptide linker are combined into multivalent structures through non-covalent or chemical crosslinking to form, e.g., homobivalent, heterobivalent, trivalent, and tetravalent structures using both scFV or diabody type format, as described in, e.g., US5869620.

Within each antibody or antibody fragment (e.g., scFv) of a bispecific antibody molecule, the VH can be upstream or downstream of the VL. In some embodiments, the upstream antibody or antibody fragment (e.g., scFv) is arranged with its VH (VH₁) upstream of its VL (VL₁) and the downstream antibody or antibody fragment (e.g., scFv) is arranged with its VL (VL₂) upstream of its VH (VH₂), such that the overall bispecific antibody molecule has the arrangement VH₁-VL₁-VL₂-VH₂. In other embodiments, the upstream antibody or antibody fragment (e.g., scFv) is arranged with its VL (VL₁) upstream of its VH (VH₁) and the downstream antibody or antibody fragment (e.g., scFv) is arranged with its VH (VH₂) upstream of its VL (VL₂), such that the overall bispecific antibody molecule has the arrangement VL₁-VH₁-VH₂-VL₂. Optionally, a linker is disposed between the two antibodies or antibody fragments (e.g., scFvs), e.g., between VL₁ and VL₂ if the construct is arranged as VH₁-VL₁-VL₂VH₂, or between VH₁ and VH₂ if the construct is arranged as VL₁-VH₁-VH₂-VL₂. The linker may be a linker as described herein, e.g., a (Gly₄-Ser)n linker, wherein n is 1, 2, 3, 4, 5, or 6, preferably 4 (SEQ ID NO: 890). In general, the linker between the two scFvs should be long enough to avoid mispairing between the domains of the two scFvs. Optionally, a linker is disposed between the VL and VH of the first scFv. Optionally, a linker is disposed between the VL and VH of the second scFv. In constructs that have multiple linkers, any two or more of the linkers can be the same or different. Accordingly, in some embodiments, a bispecific CAR comprises VLs, VHs, and optionally one or more linkers in an arrangement as described herein.

### Transmembrane domains

With respect to the transmembrane domain, in various embodiments, a chimeric molecule of the invention (e.g., a CAR) can be designed to comprise a transmembrane domain that is attached to the extracellular domain of the chimeric molecule. A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the chimeric protein (e.g., CAR) e.g., in one embodiment, the transmembrane domain may be from the same protein that the signaling domain, costimulatory domain or the hinge domain is derived from. In another aspect, the transmembrane domain is not derived from the same protein that any other domain of the chimeric protein (e.g., CAR) is derived from. In some instances, the transmembrane domain can be selected or modified by amino acid substitution to avoid binding of such domains to the transmembrane domains of the same or different surface membrane proteins, e.g., to minimize interactions with other members of the receptor complex. In one aspect, the transmembrane domain is capable of homodimerization with another CAR on the cell surface of a CAR-expressing cell. In a different aspect, the amino acid sequence of the transmembrane domain may be modified or substituted so as to minimize interactions with the binding domains of the native binding partner present in the same CAR-expressing cell.

The transmembrane domain may be derived either from a natural or from a recombinant source. Where the source is natural, the domain may be derived from any membrane-bound or transmembrane protein. In one aspect the transmembrane domain is capable of signaling to the intracellular domain(s) whenever the CAR has bound to a target. A transmembrane domain of particular use in this invention may include at least the transmembrane region(s) of e.g., the alpha, beta or zeta chain of the T-cell receptor, CD28, CD27, CD3 epsilon, CD45, CD4, CDS, CD8, CD9, CD16, CD22, CD33, CD37, CD64, CD80, CD86, CD134, CD137, CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, or NKG2C.

In some instances, the transmembrane domain can be attached to the extracellular region of the CAR, e.g., the antigen binding domain of the CAR, via a hinge, e.g., a hinge from a human protein. For example, in one embodiment, the hinge can be a human Ig (immunoglobulin) hinge (e.g., an IgG4 hinge, an IgD hinge), a GS linker (e.g., a GS linker described herein), a KIR2DS2 hinge or a CD8a hinge. In one embodiment, the hinge or spacer comprises (e.g., consists of) the amino acid sequence of SEQ ID NO: 4. In one aspect, the transmembrane domain comprises (e.g., consists of) a transmembrane domain of SEQ ID NO: 12.

In certain embodiments, the encoded transmembrane domain comprises an amino acid sequence of a CD8 transmembrane domain having at least one, two or three modifications but not more than 20, 10 or 5 modifications of an amino acid sequence of SEQ ID NO: 12, or a sequence with 95-99% identity to an amino acid sequence of SEQ ID NO: 12. In one embodiment, the encoded transmembrane domain comprises the sequence of SEQ ID NO: 12.

In other embodiments, the nucleic acid molecule encoding the CAR comprises a nucleotide sequence of a CD8 transmembrane domain, e.g., comprising the sequence of SEQ ID NO: 13, or a sequence with 95-99% identity thereof.

In certain embodiments, the encoded antigen binding domain is connected to the transmembrane domain by a hinge region. In one embodiment, the encoded hinge region comprises the amino acid sequence of a CD8 hinge, e.g., SEQ ID NO: 4; or the amino acid sequence of an IgG4 hinge, e.g., SEQ ID NO: 6, or a sequence with 95-99% identity to SEQ ID NO:4 or 6. In other embodiments, the nucleic acid sequence encoding the hinge region comprises a sequence of SEQ ID NO: 5 or SEQ ID NO: 7, corresponding to a CD8 hinge or an IgG4 hinge, respectively, or a sequence with 95-99% identity to SEQ ID NO:5 or 7.

In one aspect, the hinge or spacer comprises an IgG4 hinge. For example, in one embodiment, the hinge or spacer comprises a hinge of the amino acid sequence:

ESKYGPPCPPCPAPEFLGGPSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQEEMTKNQVSLTCLVKGFYPSD
IAVEWESNGQPENNYKTTPPVLDSDGSFFLYSRLTVDKSRWQEGNVFSCSVMHEALHNHYTQKSLSLSLGKM (SEQ ID
NO:6).

In some embodiments, the hinge or spacer comprises a hinge encoded by a nucleotide sequence of:

GAGAGCAAGTACGGCCCTCCCTGCCCCCCTTGCCCTGCCCCCGAGTTCCTGGGCGGACCCAGCGTGTTCCTGTTCC

CCCCCAAGCCCAAGGACACCCTGATGATCAGCCGGACCCCCGAGGTGACCTGTGTGGTGGTGGACGTGTCCCAGG
AGGACCCCGAGGTCCAGTTCAACTGGTACGTGGACGGCGTGGAGGTGCACAACGCCAAGACCAAGCCCCGGGAG
GAGCAGTTCAATAGCACCTACCGGGTGGTGTCCGTGCTGACCGTGCTGCACCAGGACTGGCTGAACGGCAAGGA
ATACAAGTGTAAGGTGTCCAACAAGGGCCTGCCCAGCAGCATCGAGAAAACCATCAGCAAGGCCAAGGGCCAGC
CTCGGGAGCCCCAGGTGTACACCCTGCCCCCTAGCCAAGAGGAGATGACCAAGAACCAGGTGTCCCTGACCTGCC
TGGTGAAGGGCTTCTACCCCAGCGACATCGCCGTGGAGTGGGAGAGCAACGGCCAGCCCGAGAACAACTACAAG
ACCACCCCCCCTGTGCTGGACAGCGACGGCAGCTTCTTCCTGTACAGCCGGCTGACCGTGGACAAGAGCCGGTGG
CAGGAGGGCAACGTCTTTAGCTGCTCCGTGATGCACGAGGCCCTGCACAACCACTACACCCAGAAGAGCCTGAGC
CTGTCCCTGGGCAAGATG (SEQ ID NO:7).

In one aspect, the hinge or spacer comprises an IgD hinge. For example, in one embodiment, the hinge or spacer comprises a hinge of the amino acid sequence

RWPESPKAQASSVPTAQPQAEGSLAKATTAPATTRNTGRGGEEKKKEKEKEEQEERETKTPECPSHTQPLGVYLLTPAV
QDLWLRDKATFTCFVVGSDLKDAHLTWEVAGKVPTGGVEEGLLERHSNGSQSQHSRLTLPRSLWNAGTSVTCTLNHP
SLPPQRLMALREPAAQAPVKLSLNLLASSDPPEAASWLLCEVSGFSPPNILLMWLEDQREVNTSGFAPARPPPQPGSTT
FWAWSVLRVPAPPSPQPATYTCVVSHEDSRTLLNASRSLEVSYVTDH (SEQ ID NO:8).

In some embodiments, the hinge or spacer comprises a hinge encoded by a nucleotide sequence of:

AGGTGGCCCGAAAGTCCCAAGGCCCAGGCATCTAGTGTTCCTACTGCACAGCCCCAGGCAGAAGGCAGCCTAGCC
AAAGCTACTACTGCACCTGCCACTACGCGCAATACTGGCCGTGGCGGGGAGGAGAAGAAAAAGGAGAAAGAGA
AAGAAGAACAGGAAGAGAGGGAGACCAAGACCCCTGAATGTCCATCCCATACCCAGCCGCTGGGCGTCTATCTCT
TGACTCCCGCAGTACAGGACTTGTGGCTTAGAGATAAGGCCACCTTTACATGTTTCGTCGTGGGCTCTGACCTGAA
GGATGCCCATTTGACTTGGGAGGTTGCCGGAAAGGTACCCACAGGGGGGGTTGAGGAAGGGTTGCTGGAGCGC
CATTCCAATGGCTCTCAGAGCCAGCACTCAAGACTCACCCTTCCGAGATCCCTGTGGAACGCCGGGACCTCTGTCA
CATGTACTCTAAATCATCCTAGCCTGCCCCCACAGCGTCTGATGGCCCTTAGAGAGCCAGCCGCCCAGGCACCAGT
TAAGCTTAGCCTGAATCTGCTCGCCAGTAGTGATCCCCCAGAGGCCGCCAGCTGGCTCTTATGCGAAGTGTCCGGC
TTTAGCCCGCCCAACATCTTGCTCATGTGGCTGGAGGACCAGCGAGAAGTGAACACCAGCGGCTTCGCTCCAGCC
CGGCCCCCACCCCAGCCGGGTTCTACCACATTCTGGGCCTGGAGTGTCTTAAGGGTCCCAGCACCACCTAGCCCCC
AGCCAGCCACATACACCTGTGTTGTGTCCCATGAAGATAGCAGGACCCTGCTAAATGCTTCTAGGAGTCTGGAGGT
TTCCTACGTGACTGACCATT (SEQ ID NO:9).

In one aspect, the transmembrane domain may be recombinant, in which case it will comprise predominantly hydrophobic residues such as leucine and valine. In one aspect a triplet of phenylalanine, tryptophan and valine can be found at each end of a recombinant transmembrane domain.

Optionally, a short oligo- or polypeptide linker, between 2 and 10 amino acids in length may form the linkage between the transmembrane domain and the cytoplasmic region of the CAR. A glycine-serine doublet provides a particularly suitable linker. For example, in one aspect, the linker comprises the amino acid sequence of GGGGSGGGGS (SEQ ID NO:10). In some embodiments, the linker is encoded by a nucleotide sequence of GGTGGCGGAGGTTCTGGAGGTGGAGGTTCC (SEQ ID NO:11).

In one aspect, the hinge or spacer comprises a KIR2DS2 hinge.

### Signaling domains

In embodiments of the invention having an intracellular signaling domain, such a domain can contain, e.g., one or more of a primary signaling domain and/or a costimulatory signaling domain. In some embodiments, the intracellular signaling domain comprises a sequence encoding a primary signaling domain. In some embodiments, the intracellular signaling domain comprises a costimulatory signaling domain. In some embodiments, the intracellular signaling domain comprises a a primary signaling domain and a costimulatory signaling domain.

The intracellular signaling sequences within the cytoplasmic portion of the CAR in the invention may be linked to each other in a random or specified order. Optionally, a short oligo- or polypeptide linker, for example, between 2 and 10 amino acids (e.g., 2, 3, 4, 5, 6, 7, 8, 9, or 10 amino acids) in length may form the linkage between intracellular signaling sequences. In one embodiment, a glycine-serine doublet can be used as a suitable linker. In one embodiment, a single amino acid, e.g., an alanine, a glycine, can be used as a suitable linker.

In one aspect, the intracellular signaling domain is designed to comprise two or more, e.g., 2, 3, 4, 5, or more, costimulatory signaling domains. In an embodiment, the two or more, e.g., 2, 3, 4, 5, or more, costimulatory signaling domains, are separated by a linker molecule, e.g., a linker molecule described herein. In one embodiment, the intracellular signaling domain comprises two costimulatory signaling domains. In some embodiments, the linker molecule is a glycine residue. In some embodiments, the linker is an alanine residue.

### Primary Signaling domains

A primary signaling domain regulates primary activation of the TCR complex either in a stimulatory way, or in an inhibitory way. Primary intracellular signaling domains that act in a stimulatory manner may contain signaling motifs which are known as immunoreceptor tyrosine-based activation motifs or ITAMs.

Examples of ITAM containing primary intracellular signaling domains that are of particular use in the invention include those of CD3 zeta, common FcR gamma (FCER1G), Fc gamma RIIa, FcR beta (Fc Epsilon R1b), CD3 gamma, CD3 delta, CD3 epsilon, CD79a, CD79b, DAP10, and DAP12. In one embodiment, a CAR in the invention comprises an intracellular signaling domain, e.g., a primary signaling domain of CD3-zeta.

In one embodiment, the encoded primary signaling domain comprises a functional signaling domain of CD3 zeta. The encoded CD3 zeta primary signaling domain can comprise an amino acid sequence having at least one, two or three modifications but not more than 20, 10 or 5 modifications of an amino acid sequence of SEQ ID NO: 18 or SEQ ID NO: 20, or a sequence with 95-99% identity to an amino acid sequence of SEQ ID NO:18 or SEQ ID NO: 20. In some embodiments, the encoded primary signaling domain comprises a sequence of SEQ ID NO:18 or SEQ ID NO: 20. In other embodiments, the nucleic acid sequence encoding the primary signaling domain comprises a sequence of SEQ ID NO:19 or SEQ ID NO: 21, or a sequence with 95-99% identity thereof.

### Costimulatory Signaling Domains

In some embodiments, the encoded intracellular signaling domain comprises a a costimulatory signaling domain. For example, the intracellular signaling domain can comprise a primary signaling domain and a costimulatory signaling domain. In some embodiments, the encoded costimulatory signaling domain comprises a functional signaling domain of a protein chosen from one or more of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, a ligand that specifically binds with CD83, CDS, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, and NKG2D.

In certain embodiments, the encoded costimulatory signaling domain comprises an amino acid sequence having at least one, two or three modifications but not more than 20, 10 or 5 modifications of an amino acid sequence of SEQ ID NO:14 or SEQ ID NO: 16, or a sequence with 95-99% identity to an amino acid sequence of SEQ ID NO:14 or SEQ ID NO: 16. In one embodiment, the encoded costimulatory signaling domain comprises a sequence of SEQ ID NO: 14 or SEQ ID NO: 16. In other embodiments, the nucleic acid sequence encoding the costimulatory signaling domain comprises a sequence of SEQ ID NO:15 or SEQ ID NO: 17, or a sequence with 95-99% identity thereof.

In other embodiments, the encoded intracellular domain comprises the sequence of SEQ ID NO: 14 or SEQ ID NO: 16, and the sequence of SEQ ID NO: 18 or SEQ ID NO: 20, wherein the sequences comprising the intracellular signaling domain are expressed in the same frame and as a single polypeptide chain.

In certain embodiments, the nucleic acid sequence encoding the intracellular signaling domain comprises a sequence of SEQ ID NO:15 or SEQ ID NO: 17, or a sequence with 95-99% identity thereof, and a sequence of SEQ ID NO:19 or SEQ ID NO:21, or a sequence with 95-99% identity thereof.

In some embodiments, the nucleic acid molecule further encodes a leader sequence. In one embodiment, the leader sequence comprises the sequence of SEQ ID NO: 2.

In one aspect, the intracellular signaling domain is designed to comprise the signaling domain of CD3-zeta and the signaling domain of CD28. In one aspect, the intracellular signaling domain is designed to comprise the signaling domain of CD3-zeta and the signaling domain of 4-1BB. In one aspect, the signaling domain of 4-1BB is a signaling domain of SEQ ID NO: 14. In one aspect, the signaling domain of CD3-zeta is a signaling domain of SEQ ID NO: 18.

In one aspect, the intracellular signaling domain is designed to comprise the signaling domain of CD3-zeta and the signaling domain of CD27. In one aspect, the signaling domain of CD27 comprises an amino acid sequence of QRRKYRSNKGESPVEPAEPCRYSCPREEEGSTIPIQEDYRKPEPACSP (SEQ ID NO:16). In one aspect, the signaling domain of CD27 is encoded by a nucleic acid sequence of:

AGGAGTAAGAGGAGCAGGCTCCTGCACAGTGACTACATGAACATGACTCCCCGCCGCCCCGGGCCCACCCGCAA
GCATTACCAGCCCTATGCCCCACCACGCGACTTCGCAGCCTATCGCTCC (SEQ ID NO:17).

### Exemplary CAR Molecules

The CAR molecules disclosed herein can comprise a binding domain that binds to a target, e.g., a target as described herein; a transmembrane domain, e.g., a transmembrane domain as described herein; and an intracellular signaling domain, e.g., an intracellular domain as described herein. In embodiments, the binding domain comprises a heavy chain complementary determining region 1 (HC CDR1), a heavy chain complementary determining region 2 (HC CDR2), and a heavy chain complementary determining region 3 (HC CDR3) of a heavy chain binding domain described herein, and/or a light chain complementary determining region 1 (LC CDR1), a light chain complementary determining region 2 (LC CDR2), and a light chain complementary determining region 3 (LC CDR3) of a light chain binding domain described herein.

In other embodiments, the CAR molecule comprises a CD19 CAR molecule described herein, e.g., a CD19 CAR molecule described in US-2015-0283178-A1, e.g., CTLO19. In embodiments, the CD19 CAR comprises an amino acid, or has a nucleotide sequence shown in US-2015-0283178-A1, or a sequence substantially identical thereto (e.g., at least 85%, 90%, 95% or more identical thereto).

In one embodiment, the CAR T cell that specifically binds to CD19 has the USAN designation TISAGENLECLEUCEL-T. CTL019 is made by a gene modification of T cells is mediated by stable insertion via transduction with a self-inactivating, replication deficient Lentiviral (LV) vector containing the CTL019 transgene under the control of the EF-1 alpha promoter. CTL019 can be a mixture of transgene positive and negative T cells that are delivered to the subject on the basis of percent transgene positive T cells.

In other embodiments, the CD19 CAR includes a CAR molecule, or an antigen binding domain (e.g., a humanized antigen binding domain) according to Table 3 of WO2014/153270. The amino acid and nucleotide sequences encoding the CD19 CAR molecules and antigen binding domains (e.g., including one, two, three VH CDRs; and one, two, three VL CDRs according to Kabat or Chothia), are specified in WO2014/153270. In embodiments, the CD19 CAR comprises an amino acid, or has a nucleotide sequence shown in WO2014/153270, or a sequence substantially identical to any of the aforesaid sequences (e.g., at least 85%, 90%, 95% or more identical to any of the aforesaid CD19 CAR sequences).

In one embodiment, the parental murine scFv sequence is the CAR19 construct provided in PCT publication WO2012/079000 and provided herein in Table 9. In one embodiment, the anti-CD19 binding domain is a scFv described in WO2012/079000 and provided herein in Table 9.

In one embodiment, the CD19 CAR comprises an amino acid sequence provided as SEQ ID NO: 12 in PCT publication WO2012/079000. In embodiment, the amino acid sequence is:
MALPVTALLLPLALLLHAARPdiqmtqttsslsaslgdrvtiscrasqdiskylnwyqqkpdgtvklliyhtsrlhsgvpsrfsgsgsg tdysltisnleqediatyfcqqgntlpytfgggtkleitggggsggggsggggsevklqesgpglvapsqslsvtctvsgvslpdygvswirqpprkgle wlgviwgsettyynsalksrltiikdnsksqvflkmnslqtddtaiyycakhyyyggsyamdywgqgtsvtvsstttpaprpptpaptiasqplslrp eacrpaaggavhtrgldfacdiyiwaplagtcgvlllslvitlyckrgrkkllyifkqpfmrpvqttqeedgcscrfpeeeeggcelrvkfsrsadapayk qgqnqlynelnlgrreeydvldkrrgrdpemggkprrknpqeglynelqkdkmaeayseigmkgerrrgkghdglyqglstatkdtydalhmq alppr (SEQ ID NO: 891), or a sequence substantially identical thereto (e.g., at least 85%, 90% or 95% or higher identical thereto), with or without the signal peptide sequence indicated in capital letters.

In embodiment, the amino acid sequence is: 
diqmtqttsslsaslgdrvtiscrasqdiskylnwyqqkpdgtvklliyhtsrlhsgvpsrfsgsgsgtdysltisnleqediatyfcqqgntl
pytfgggtkleitggggsggggsggggsevklqesgpglvapsqslsvtctvsgvslpdygvswirqpprkglewlgviwgsettyynsalksrltiikd
nsksqvflkmnslqtddtaiyycakhyyyggsyamdywgqgtsvtvssttpaprpptpaptiasqplslrpeacrpaaggavhtrgldfacdiyi
waplagtcgvlllslvitlyckrgrkkllyifkqpfmrpvqttqeedgcscrfpeeeeggcelrvkfsrsadapaykqgqnqlynelnlgrreeydvldk
rrgrdpemggkprrknpqeglynelqkdkmaeayseigmkgerrrgkghdglyqglstatkdtydalhmqalppr (SEQ ID NO: 892),
 or a sequence substantially homologous thereto (e.g., at least 85%, 90% or 95% or higher identical thereto).

In embodiments, the CAR molecule is a CD19 CAR molecule described herein, e.g., a humanized CAR molecule described herein, e.g., a humanized CD19 CAR molecule of Table 6-9 or having CDRs as set out in Tables 10A and 10B.

In embodiments, the CAR molecule is a CD19 CAR molecule described herein, e.g., a murine CAR molecule described herein, e.g., a murine CD19 CAR molecule of Table 9 or having CDRs as set out in Tables 10A and 10B.

In some embodiments, the CAR molecule comprises one, two, and/or three CDRs from the heavy chain variable region and/or one, two, and/or three CDRs from the light chain variable region of the murine or humanized CD19 CAR of Table 10A and 10B.

In one embodiment, the antigen binding domain comprises one, two three (e.g., all three) heavy chain CDRs, HC CDR1, HC CDR2 and HC CDR3, from an antibody listed herein, and/or one, two, three (e.g., all three) light chain CDRs, LC CDR1, LC CDR2 and LC CDR3, from an antibody listed herein. In one embodiment, the antigen binding domain comprises a heavy chain variable region and/or a variable light chain region of an antibody listed or described herein.

Exemplary CD19 CARs include any of the CD19 CARs or anti-CD19 binding domains described herein, e.g., in one or more tables (e.g., Tables 6-9) described herein (e.g., , or an anti-CD19 CAR described in Xu et al. Blood 123.24(2014):3750-9; Kochenderfer et al. Blood 122.25(2013):4129-39, Cruz et al. Blood 122.17(2013):2965-73, NCT00586391, NCT01087294, NCT02456350, NCT00840853, NCT02659943, NCT02650999, NCT02640209, NCT01747486, NCT02546739, NCT02656147, NCT02772198, NCT00709033, NCT02081937, NCT00924326, NCT02735083, NCT02794246, NCT02746952, NCT01593696, NCT02134262, NCT01853631, NCT02443831, NCT02277522, NCT02348216, NCT02614066, NCT02030834, NCT02624258, NCT02625480, NCT02030847, NCT02644655, NCT02349698, NCT02813837, NCT02050347, NCT01683279, NCT02529813, NCT02537977, NCT02799550, NCT02672501, NCT02819583, NCT02028455, NCT01840566, NCT01318317, NCT01864889, NCT02706405, NCT01475058, NCT01430390, NCT02146924, NCT02051257, NCT02431988, NCT01815749, NCT02153580, NCT01865617, NCT02208362, NCT02685670, NCT02535364, NCT02631044, NCT02728882, NCT02735291, NCT01860937, NCT02822326, NCT02737085, NCT02465983, NCT02132624, NCT02782351, NCT01493453, NCT02652910, NCT02247609, NCT01029366, NCT01626495, NCT02721407, NCT01044069, NCT00422383, NCT01680991, NCT02794961, or NCT02456207.

Exemplary CD19 CAR and antigen binding domain constructs that can be used in the methods described herein are shown in Tables 6-9. The light and heavy chain CDR sequences according to Kabat are shown by the bold and underlined text, and are also summarized in Tables 9 and 10A-10B below. The location of the signal sequence and histidine tag are also underlined. In embodiments, the CD19 CAR sequences and antigen binding fragments thereof do not include the signal sequence and/or histidine tag sequences.

In embodiments, the CD19 CAR comprises an anti- CD19 binding domain (e.g., murine or humanized anti- CD19 binding domain), a transmembrane domain, and an intracellular signaling domain, and wherein said anti- CD19 binding domain comprises a heavy chain complementary determining region 1 (HC CDR1), a heavy chain complementary determining region 2 (HC CDR2), and a heavy chain complementary determining region 3 (HC CDR3) of any anti- CD19 heavy chain binding domain amino acid sequences listed in Tables 6-9 and 10A-10B, or a sequence at least 85%, 90%, 95% or more identical thereto (e.g., having less than 5, 4, 3, 2 or 1 amino acid substitutions, e.g., conservative substitutions).

In one embodiment, the anti- CD19 binding domain comprises a light chain variable region described herein (e.g., in Tables 6-9) and/or a heavy chain variable region described herein (e.g., in Table 9), or a sequence at least 85%, 90%, 95% or more identical thereto.

In one embodiment, the encoded anti- CD19 binding domain is a scFv comprising a light chain and a heavy chain of an amino acid sequence of Tables 6-9, or a sequence at least 85%, 90%, 95% or more identical thereto.

In an embodiment, the human or humanized anti- CD19 binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Tables 6-9, or a sequence at least 85%, 90%, 95% or more identical thereto; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Tables 6-9, or a sequence at least 85%, 90%, 95% or more identical thereto.

**Table 9: CD19 CAR Constructs**

| **Name** | **SEQ ID NO:** | **Sequence** |
|---|---|---|
| **CAR 1** | | |
| **CAR1 scFv domain** | 893 | EIVMTQSPATLSLSPGERATLSCRASQDISKYLNWYQQKPGQAPRLLIYHT SRLHSGIPARFSGSGSGTDYTLTISSLQPEDFAVYFCQQGNTLPYTFGQGT KLEIKGGGGSGGGGSGGGGSQVQLQESGPGLVKPSETLSLTCTVSGVSLPD YGVSWIRQPPGKGLEWIGVIWGSETTYYSSSLKSRVTISKDNSKNQVSLKL SSVTAADTAVYYCAKHYYYGGSYAMDYWGQGTLVTVSS |
| **103101** | 894 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg |
| **CAR1** | | |
| **Soluble scFv** - **nt** | | |
| | | tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactactcttcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt ccagccaccaccatcatcaccatcaccat |
| **103101** | 895 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsqvqlqesgpglvkpsetlsltctvs gvslpdygvswirqppgkglewigviwgsettyysssslksrvtiskdnsknqvslk lssvtaadtavyycakhyyyggsyamdywgqgtlvtvsshhhhhhhh |
| **CAR1** | | |
| **Soluble scFv** - **aa** | | |
| **104875** | 896 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactactcttcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt ccagcaccactaccccagcaccgaggccacccaccccggctcctaccatcgcctcc cagcctctgtccctgcgtccggaggcatgtagacccgcagctggtggggccgtgca tacccggggtcttgacttcgcctgcgatatctacatttgggcccctctggctggta cttgcggggtcctgctgctttcactcgtgatcactctttactgtaagcgcggtcgg aagaagctgctgtacatctttaagcaacccttcatgaggcctgtgcagactactca agaggaggacggctgttcatgccggttcccagaggaggaggaaggcggctgcgaac tgcgcgtgaaattcagccgcagcgcagatgctccagcctacaagcaggggcagaac cagctctacaacgaactcaatcttggtcggagagaggagtacgacgtgctggacaa gcggagaggacgggacccagaaatgggcgggaagccgcgcagaaagaatccccaag agggcctgtacaacgagctccaaaaggataagatggcagaagcctatagcgagatt ggtatgaaaggggaacgcagaagaggcaaaggccacgacggactgtaccagggact cagcaccgccaccaaggacacctatgacgctcttcacatgcaggccctgccgcctc gg |
| **CAR 1** - **Full - nt** | | |
| **104875** | 897 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsqvqlqesgpglvkpsetlsltctvs gvslpdygvswirqppgkglewigviwgsettyysssslksrvtiskdnsknqvslk lssvtaadtavyycakhyyyggsyamdywgqgtlvtvssttt paprpptpaptias |
| **CAR 1 - Full - aa** | | |
| | | qplslrpeacrpaaggavhtrgldfacdiyiwaplagtcgvlllslvitlyckrgr kkllyifkqpfmrpvqttqeedgcscrfpeeeeggcelrvkfsrsadapaykqgqn qlynelnlgrreeydvldkrrgrdpemggkprrknpqeglynelqkdkmaeaysei gmkgerrrgkghdglyqglstatkdtydalhmqalppr |

| **CAR 2** | | |
|---|---|---|
| **CAR2 scFv domain** | 898 | eivmtqspatlslspgeratlscrasqdiskylnwyqqkpgqaprlliyhtsrlhs giparfsgsgsgtdytltisslqpedfavyfcqqgntlpytfgqgtkleikggggs ggggsggggsqvqlqesgpglvkpsetlsltctvsgvslpdygvswirqppgkgle wigviwgsettyyqsslksrvtiskdnsknqvslklssvtaadtavyycakhyyyg gsyamdywgqgtlvtvss |
| **103102** | 899 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactaccaatcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt ccagccaccaccatcatcaccatcaccat |
| **CAR2** - **Soluble scFv - nt** | | |
| **103102** | 900 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsqvqlqesgpglvkpsetlsltctvs gvslpdygvswirqppgkglewigviwgsettyyqsslksrvtiskdnsknqvslk lssvtaadtavyycakhyyyggsyamdywgqgtlvtvsshhhhhhhh |
| **CAR2 - Soluble scFv - aa** | | |
| **104876** | 901 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactaccaatcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt |
| **CAR 2 - Full - nt** | | |
| | | ccagcaccactaccccagcaccgaggccacccaccccggctcctaccatcgcctcc cagcctctgtccctgcgtccggaggcatgtagacccgcagctggtggggccgtgca tacccggggtcttgacttcgcctgcgatatctacatttgggcccctctggctggta cttgcggggtcctgctgctttcactcgtgatcactctttactgtaagcgcggtcgg aagaagctgctgtacatctttaagcaacccttcatgaggcctgtgcagactactca agaggaggacggctgttcatgccggttcccagaggaggaggaaggcggctgcgaac tgcgcgtgaaattcagccgcagcgcagatgctccagcctacaagcaggggcagaac cagctctacaacgaactcaatcttggtcggagagaggagtacgacgtgctggacaa gcggagaggacgggacccagaaatgggcgggaagccgcgcagaaagaatccccaag agggcctgtacaacgagctccaaaaggataagatggcagaagcctatagcgagatt ggtatgaaaggggaacgcagaagaggcaaaggccacgacggactgtaccagggact cagcaccgccaccaaggacacctatgacgctcttcacatgcaggccctgccgcctc gg |
| **104876** | 902 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsqvqlqesgpglvkpsetlsltctvs gvslpdygvswirqppgkglewigviwgsettyyqsslksrvtiskdnsknqvslk lssvtaadtavyycakhyyyggsyamdywgqgtlvtvsstttpaprpptpaptias qplslrpeacrpaaggavhtrgldfacdiyiwaplagtcgvlllslvitlyckrgr kkllyifkqpfmrpvqttqeedgcscrfpeeeeggcelrvkfsrsadapaykqgqn qlynelnlgrreeydvldkrrgrdpemggkprrknpqeglynelqkdkmaeaysei gmkgerrrgkghdglyqglstatkdtydalhmqalppr |
| **CAR 2 - Full - aa** | | |

| **CAR 3** | | |
|---|---|---|
| **CAR3 scFv domain** | 903 | qvqlqesgpglvkpsetlsltctvsgvslpdygvswirqppgkglewigviwgset tyysssslksrvtiskdnsknqvslklssvtaadtavyycakhyyyggsyamdywgq gtlvtvssggggsggggsggggseivmtqspatlslspgeratlscrasqdiskyl nwyqqkpgqaprlliyhtsrlhsgiparfsgsgsgtdytltisslqpedfavyfcq qgntlpytfgqgtkleik |
| **103104** | 904 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttggggtag cgaaaccacttactattcatcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactgg ggccagggaactctggtcactgtgtcatctggtggaggaggtagcggaggaggcgg gagcggtggaggtggctccgaaatcgtgatgacccagagccctgcaaccctgtcc ctttctcccggggaacgggctaccctttcttgtcgggcatcacaagatatctcaaa atacctcaattggtatcaacagaagccgggacaggcccctaggcttcttatctacc acacctctcgcctgcatagcgggattcccgcacgctttagcgggtctggaagcggg accgactacactctgaccatctcatctctccagcccgaggacttcgccgtctactt ctgccagcagggtaacaccctgccgtacaccttcggccagggcaccaagcttgaga tcaaacatcaccaccatcatcaccatcac |
| **CAR 3 - Soluble scFv - nt** | | |
| **103104** **CAR 3 - Soluble scFv - aa** | 905 | MALPVTALLLPLALLLHAARPqvqlqesgpglvkpsetlsltctvsgvslpdygvs wirqppgkglewigviwgsettyyssslksrvtiskdnsknqvslklssvtaadta |
| | | vyycakhyyyggsyamdywgqgtlvtvssggggsggggsggggseivmtqspatls lspgeratlscrasqdiskylnwyqqkpgqaprlliyhtsrlhsgiparfsgsgsg tdytltisslqpedfavyfcqqgntlpytfgqgtkleikhhhhhhhh |
| **104877** | 906 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttggggtag cgaaaccacttactattcatcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactgg ggccagggaactctggtcactgtgtcatctggtggaggaggtagcggaggaggcg ggagcggtggaggtggctccgaaatcgtgatgacccagagccctgcaaccctgtcc ctttctcccggggaacgggctaccctttcttgtcgggcatcacaagatatctcaaa atacctcaattggtatcaacagaagccgggacaggcccctaggcttcttatctacc acacctctcgcctgcatagcgggattcccgcacgctttagcgggtctggaagcggg accgactacactctgaccatctcatctctccagcccgaggacttcgccgtctactt ctgccagcagggtaacaccctgccgtacaccttcggccagggcaccaagcttgaga tcaaaaccactactcccgctccaaggccacccacccctgccccgaccatcgcctct cagccgctttccctgcgtccggaggcatgtagacccgcagctggtggggccgtgca tacccggggtcttgacttcgcctgcgatatctacatttgggcccctctggctggta cttgcggggtcctgctgctttcactcgtgatcactctttactgtaagcgcggtcgg aagaagctgctgtacatctttaagcaacccttcatgaggcctgtgcagactactca agaggaggacggctgttcatgccggttcccagaggaggaggaaggcggctgcgaac tgcgcgtgaaattcagccgcagcgcagatgctccagcctacaagcaggggcagaac cagctctacaacgaactcaatcttggtcggagagaggagtacgacgtgctggacaa gcggagaggacgggacccagaaatgggcgggaagccgcgcagaaagaatccccaag agggcctgtacaacgagctccaaaaggataagatggcagaagcctatagcgagatt ggtatgaaaggggaacgcagaagaggcaaaggccacgacggactgtaccagggact cagcaccgccaccaaggacacctatgacgctcttcacatgcaggccctgccgcctc gg |
| **CAR 3** - **Full - nt** | | |
| **104877** | 907 | MALPVTALLLPLALLLHAARPqvqlqesgpglvkpsetlsltctvsgvslpdygvs wirqppgkglewigviwgsettyyssslksrvtiskdnsknqvslklssvtaadta vyycakhyyyggsyamdywgqgtlvtvssggggsggggsggggseivmtqspatls lspgeratlscrasqdiskylnwyqqkpgqaprlliyhtsrlhsgiparfsgsgsg tdytltisslqpedfavyfcqqgntlpytfgqgtkleikttpaprpptpaptias qplslrpeacrpaaggavhtrgldfacdiyiwaplagtcgvlllslvitlyckrgr kkllyifkqpfmrpvqttqeedgcscrfpeeeeggcelrvkfsrsadapaykqgqn qlynelnlgrreeydvldkrrgrdpemggkprrknpqeglynelqkdkmaeaysei gmkgerrrgkghdglyqglstatkdtydalhmqalppr |
| **CAR 3 - Full - aa** | | |

| **CAR 4** | | |
|---|---|---|
| **CAR4 scFv domain** | 908 | qvqlqesgpglvkpsetlsltctvsgvslpdygvswirqppgkglewigviwgset tyyqsslksrvtiskdnsknqvslklssvtaadtavyycakhyyyggsyamdywgq gtlvtvssggggsggggsggggseivmtqspatlslspgeratlscrasqdiskyl nwyqqkpgqaprlliyhtsrlhsgiparfsgsgsgtdytltisslqpedfavyfcq |
| | | qgntlpytfgqgtkleik |
| **103106** | 909 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttggggtag cgaaaccacttactatcaatcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactg gggccagggaactctggtcactgtgtcatctggtggaggaggtagcggaggaggcg ggagcggtggaggtggctccgaaatcgtgatgacccagagccctgcaaccctgtcc ctttctcccggggaacgggctaccctttcttgtcgggcatcacaagatatctcaaa atacctcaattggtatcaacagaagccgggacaggcccctaggcttcttatctacc acacctctcgcctgcatagcgggattcccgcacgctttagcgggtctggaagcggg accgactacactctgaccatctcatctctccagcccgaggacttcgccgtctactt ctgccagcagggtaacaccctgccgtacaccttcggccagggcaccaagcttgaga tcaaacatcaccaccatcatcaccatcac |
| **CAR4 - Soluble scFv - nt** | | |
| **103106** | 910 | MALPVTALLLPLALLLHAARPqvqlqesgpglvkpsetlsltctvsgvslpdygvs wirqppgkglewigviwgsettyyqsslksrvtiskdnsknqvslklssvtaadta vyycakhyyyggsyamdywgqgtlvtvssggggsggggsggggseivmtqspatls lspgeratlscrasqdiskylnwyqqkpgqaprlliyhtsrlhsgiparfsgsgsg tdytltisslqpedfavyfcqqgntlpytfgqgtkleikhhhhhhhh |
| **CAR4 - Soluble scFv -aa** | | |
| 104878 | 911 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttggggtag cgaaaccacttactatcaatcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactg gggccagggaactctggtcactgtgtcatctggtggaggaggtagcggaggaggcgg gagcggtggaggtggctccgaaatcgtgatgacccagagccctgcaaccctgtcc ctttctcccggggaacgggctaccctttcttgtcgggcatcacaagatatctcaaa atacctcaattggtatcaacagaagccgggacaggcccctaggcttcttatctacc acacctctcgcctgcatagcgggattcccgcacgctttagcgggtctggaagcggg accgactacactctgaccatctcatctctccagcccgaggacttcgccgtctactt ctgccagcagggtaacaccctgccgtacaccttcggccagggcaccaagcttgaga tcaaaaccactactcccgctccaaggccacccacccctgccccgaccatcgcctct cagccgctttccctgcgtccggaggcatgtagacccgcagctggtggggccgtgca tacccggggtcttgacttcgcctgcgatatctacatttgggcccctctggctggta cttgcggggtcctgctgctttcactcgtgatcactctttactgtaagcgcggtcgg aagaagctgctgtacatctttaagcaacccttcatgaggcctgtgcagactactca agaggaggacggctgttcatgccggttcccagaggaggaggaaggcggctgcgaac tgcgcgtgaaattcagccgcagcgcagatgctccagcctacaagcaggggcagaac cagctctacaacgaactcaatcttggtcggagagaggagtacgacgtgctggacaa gcggagaggacgggacccagaaatgggcgggaagccgcgcagaaagaatccccaag agggcctgtacaacgagctccaaaaggataagatggcagaagcctatagcgagatt ggtatgaaaggggaacgcagaagaggcaaaggccacgacggactgtaccagggact cagcaccgccaccaaggacacctatgacgctcttcacatgcaggccctgccgcctc |
| CAR 4 - Full - nt | | |
| | | gg |
| 104878 | 912 | MALPVTALLLPLALLLHAARPqvqlqesgpglvkpsetlsltctvsgvslpdygvs wirqppgkglewigviwgsettyyqsslksrvtiskdnsknqvslklssvtaadta vyycakhyyyggsyamdywgqgtlvtvssggggsggggsggggseivmtqspatls lspgeratlscrasqdiskylnwyqqkpgqaprlliyhtsrlhsgiparfsgsgsg tdytltisslqpedfavyfcqqgntlpytfgqgtkleikttpaprpptpaptias qplslrpeacrpaaggavhtrgldfacdiyiwaplagtcgvllllslvitlyckrgr kkllyifkqpfmrpvqttqeedgcscrfpeeeeggcelrvkfsrsadapaykqgqn qlynelnlgrreeydvldkrrgrdpemggkprrknpqeglynelqkdkmaeaysei gmkgerrrgkghdglyqglstatkdtydalhmqalppr |
| CAR 4 - Full - aa | | |

| **CAR 5** | | |
|---|---|---|
| **CAR5 scFv domain** | 913 | eivmtqspatlslspgeratlscrasqdiskylnwyqqkpgqaprlliyhtsrlhs giparfsgsgsgtdytltisslqpedfavyfcqqgntlpytfgqgtkleikggggs ggggsggggsggggsqvqlqesgpglvkpsetlsltctvsgvslpdygvswirqpp gkglewigviwgsettyysssslksrvtiskdnsknqvslklssvtaadtavyycak hyyyggsyamdywgqgtlvtvss |
| **99789** | 914 | atggccctcccagtgaccgctctgctgctgcctctcgcacttcttctccatgccgc tcggcctgagatcgtcatgacccaaagccccgctaccctgtccctgtcacccggcg agagggcaaccctttcatgcagggccagccaggacatttctaagtacctcaactgg tatcagcagaagccagggcaggctcctcgcctgctgatctaccacaccagccgcct ccacagcggtatccccgccagattttccgggagcgggtctggaaccgactacaccc tcaccatctcttctctgcagcccgaggatttcgccgtctatttctgccagcagggg aatactctgccgtacaccttcggtcaaggtaccaagctggaaatcaagggaggcgg aggatcaggcggtggcggaagcggaggaggtggctccggaggaggaggttcccaag tgcagcttcaagaatcaggacccggacttgtgaagccatcagaaaccctctccctg acttgtaccgtgtccggtgtgagcctccccgactacggagtctcttggattcgcca gcctccggggaagggtcttgaatggattggggtgatttggggatcagagactactt actactcttcatcacttaagtcacgggtcaccatcagcaaagataatagcaagaac caagtgtcacttaagctgtcatctgtgaccgccgctgacaccgccgtgtactattg tgccaaacattactattacggagggtcttatgctatggactactggggacagggga ccctggtgactgtctctagccatcaccatcaccaccatcatcac |
| **CAR5 - Soluble scFv - nt** | | |
| **99789** | 915 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyysslksrvtiskdnskn qvslklssvtaadtavyycakhyyyggsyamdywgqgtlvtvsshhhhhhhh |
| **CAR5** - **Soluble scFv -aa** | | |
| **104879** | 916 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagcggcggaggcgggagccagg |
| **CAR 5** - **Full - nt** | | |
| | | tccaactccaagaaagcggaccgggtcttgtgaagccatcagaaactctttcactg acttgtactgtgagcggagtgtctctccccgattacggggtgtcttggatcagaca gccaccggggaagggtctggaatggattggagtgatttggggctctgagactactt actactcttcatccctcaagtcacgcgtcaccatctcaaaggacaactctaagaat caggtgtcactgaaactgtcatctgtgaccgcagccgacaccgccgtgtactattg cgctaagcattactattatggcgggagctacgcaatggattactggggacagggta ctctggtcaccgtgtccagcaccactaccccagcaccgaggccacccaccccggct cctaccatcgcctcccagcctctgtccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **104879** | 917 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyyssslksrvtiskdnskn qvslklssvtaadtavyycakhyyyggsyamdywgqgtlvtvsstttpaprpptpa ptiasqplslrpeacrpaaggavhtrgldfacdiyiwaplagtcgvlllslvitly ckrgrkkllyifkqpfmrpvqttqeedgcscrfpeeeeggcelrvkfsrsadapay kqgqnqlynelnlgrreeydvldkrrgrdpemggkprrknpqeglynelqkdkmae ayseigmkgerrrgkghdglyqglstatkdtydalhmqalppr |
| **CAR 5 - Full - aa** | | |

| **CAR 6** | | |
|---|---|---|
| **CAR6** | 918 | eivmtqspatlslspgeratlscrasqdiskylnwyqqkpgqaprlliyhtsrlhs giparfsgsgsgtdytltisslqpedfavyfcqqgntlpytfgqgtkleikggggs ggggsggggsggggsqvqlqesgpglvkpsetlsltctvsgvslpdygvswirqpp gkglewigviwgsettyyqsslksrvtiskdnsknqvslklssvtaadtavyycak hyyyggsyamdywgqgtlvtvss |
| **scFv domain** | | |
| **99790** | 919 | atggccctcccagtgaccgctctgctgctgcctctcgcacttcttctccatgccgc tcggcctgagatcgtcatgacccaaagccccgctaccctgtccctgtcacccggcg agagggcaaccctttcatgcagggccagccaggacatttctaagtacctcaactgg tatcagcagaagccagggcaggctcctcgcctgctgatctaccacaccagccgcct ccacagcggtatccccgccagattttccgggagcgggtctggaaccgactacaccc tcaccatctcttctctgcagcccgaggatttcgccgtctatttctgccagcagggg aatactctgccgtacaccttcggtcaaggtaccaagctggaaatcaagggaggcgg aggatcaggcggtggcggaagcggaggaggtggctccggaggaggaggttcccaag tgcagcttcaagaatcaggacccggacttgtgaagccatcagaaaccctctccctg acttgtaccgtgtccggtgtgagcctccccgactacggagtctcttggattcgcca gcctccggggaagggtcttgaatggattggggtgatttggggatcagagactactt actaccagtcatcacttaagtcacgggtcaccatcagcaaagataatagcaagaac |
| **CAR6-Soluble scFv - nt** | | |
| | | caagtgtcacttaagctgtcatctgtgaccgccgctgacaccgccgtgtactattg tgccaaacattactattacggagggtcttatgctatggactactggggacagggga ccctggtgactgtctctagccatcaccatcaccaccatcatcac |
| **99790** | 92 0 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyyqsslksrvtiskdnskn qvslklssvtaadtavyycakhyyyggsyamdywgqgtlvtvsshhhhhhhh |
| **CAR6-Soluble scFv - aa** | | |
| **104880** | 921 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagcggaggcggagggagccagg tccaactccaagaaagcggaccgggtcttgtgaagccatcagaaactctttcactg acttgtactgtgagcggagtgtctctccccgattacggggtgtcttggatcagaca gccaccggggaagggtctggaatggattggagtgatttggggctctgagactactt actaccaatcatccctcaagtcacgcgtcaccatctcaaaggacaactctaagaat caggtgtcactgaaactgtcatctgtgaccgcagccgacaccgccgtgtactattg cgctaagcattactattatggcgggagctacgcaatggattactggggacagggta ctctggtcaccgtgtccagcaccactaccccagcaccgaggccacccaccccggct cctaccatcgcctcccagcctctgtccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **CAR6 - Full - nt** | | |
| **104880** | 922 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyyqsslksrvtiskdnskn qvslklssvtaadtavyycakhyyyggsyamdywgqgtlvtvsstttpaprpptpa ptiasqplslrpeacrpaaggavhtrgldfacdiyiwaplagtcgvlllslvitly ckrgrkkllyifkqpfmrpvqttqeedgcscrfpeeeeggcelrvkfsrsadapay kqgqnqlynelnlgrreeydvldkrrgrdpemggkprrknpqeglynelqkdkmae ayseigmkgerrrgkghdglyqglstatkdtydalhmqalppr |
| **CAR6 - Full - aa** | | |

| **CAR 7** | | |
|---|---|---|
| **CAR7 scFv domain** | 923 | qvqlqesgpglvkpsetlsltctvsgvslpdygvswirqppgkglewigviwgset tyysssslksrvtiskdnsknqvslklssvtaadtavyycakhyyyggsyamdywgq gtlvtvssggggsggggsggggsggggseivmtqspatlslspgeratlscrasqd iskylnwyqqkpgqaprllliyhtsrlhsgiparfsgsgsgtdytltisslqpedfa vyfcqqgntlpytfgqgtkleik |
| **100796** | 924 | atggcactgcctgtcactgccctcctgctgcctctggccctccttctgcatgccgc caggccccaagtccagctgcaagagtcaggacccggactggtgaagccgtctgaga ctctctcactgacttgtaccgtcagcggcgtgtccctccccgactacggagtgtca tggatccgccaacctcccgggaaagggcttgaatggattggtgtcatctggggttc tgaaaccacctactactcatcttccctgaagtccagggtgaccatcagcaaggata attccaagaaccaggtcagccttaagctgtcatctgtgaccgctgctgacaccgcc gtgtattactgcgccaagcactactattacggaggaagctacgctatggactattg gggacagggcactctcgtgactgtgagcagcggcggtggagggtctggaggtggag gatccggtggtggtgggtcaggcggaggagggagcgagattgtgatgactcagtca ccagccaccctttctctttcacccggcgagagagcaaccctgagctgtagagccag ccaggacatttctaagtacctcaactggtatcagcaaaaaccggggcaggcccctc gcctcctgatctaccatacctcacgccttcactctggtatccccgctcggtttagc ggatcaggatctggtaccgactacactctgaccatttccagcctgcagccagaaga tttcgcagtgtatttctgccagcagggcaataccctcccttacaccttcggtcagg gaaccaagctcgaaatcaagcaccatcaccatcatcaccaccat |
| **CAR7 - Soluble scFv - nt** | | |
| **100796** | 925 | MALPVTALLLPLALLLHAARPqvqlqesgpglvkpsetlsltctvsgvslpdygvs wirqppgkglewigviwgsettyyssslksrvtiskdnsknqvslklssvtaadta vyycakhyyyggsyamdywgqgtlvtvssggggsggggsggggsggggseivmtqs patlslspgeratlscrasqdiskylnwyqqkpgqaprlliyhtsrlhsgiparfs gsgsgtdytltisslqpedfavyfcqqgntlpytfgqgtkleikhhhhhhhh |
| **CAR7 - Soluble scFv - aa** | | |
| **104881** | 926 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttggggtag cgaaaccacttactattcatcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactg gggccagggaactctggtcactgtgtcatctggtggaggaggtagcggaggaggcg ggagcggtggaggtggctccggaggtggcggaagcgaaatcgtgatgacccagagc cctgcaaccctgtccctttctcccggggaacgggctaccctttcttgtcgggcatc acaagatatctcaaaatacctcaattggtatcaacagaagccgggacaggccccta ggcttcttatctaccacacctctcgcctgcatagcgggattcccgcacgctttagc gggtctggaagcgggaccgactacactctgaccatctcatctctccagcccgagga cttcgccgtctacttctgccagcagggtaacaccctgccgtacaccttcggccagg gcaccaagcttgagatcaaaaccactactcccgctccaaggccacccacccctgcc ccgaccatcgcctctcagccgctttccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc |
| **CAR 7** | | |
| **Full - nt** | | |
| | | tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **104881** | 927 | MALPVTALLLPLALLLHAARPqvqlqesgpglvkpsetlsltctvsgvslpdygvs wirqppgkglewigviwgsettyyssslksrvtiskdnsknqvslklssvtaadta vyycakhyyyggsyamdywgqgtlvtvssggggsggggsggggsggggseivmtqs patlslspgeratlscrasqdiskylnwyqqkpgqaprlliyhtsrlhsgiparfs gsgsgtdytltisslqpedfavyfcqqgntlpytfgqgtkleikttpaprpptpa ptiasqplslrpeacrpaaggavhtrgldfacdiyiwaplagtcgvlllslvitly ckrgrkkllyifkqpfmrpvqttqeedgcscrfpeeeeggcelrvkfsrsadapay kqgqnqlynelnlgrreeydvldkrrgrdpemggkprrknpqeglynelqkdkmae ayseigmkgerrrgkghdglyqglstatkdtydalhmqalppr |
| **CAR 7** | | |
| **Full** - **aa** | | |

| **CAR 8** | | |
|---|---|---|
| **CAR8 scFv domain** | 928 | qvqlqesgpglvkpsetlsltctvsgvslpdygvswirqppgkglewigviwgset tyyqsslksrvtiskdnsknqvslklssvtaadtavyycakhyyyggsyamdywgq gtlvtvssggggsggggsggggsggggseivmtqspatlslspgeratlscrasqd iskylnwyqqkpgqaprlliyhtsrlhsgiparfsgsgsgtdytltisslqpedfa vyfcqqgntlpytfgqgtkleik |
| **100798** | 929 | atggcactgcctgtcactgccctcctgctgcctctggccctccttctgcatgccgc caggccccaagtccagctgcaagagtcaggacccggactggtgaagccgtctgaga ctctctcactgacttgtaccgtcagcggcgtgtccctccccgactacggagtgtca tggatccgccaacctcccgggaaagggcttgaatggattggtgtcatctggggttc tgaaaccacctactaccagtcttccctgaagtccagggtgaccatcagcaaggata attccaagaaccaggtcagccttaagctgtcatctgtgaccgctgctgacaccgcc gtgtattactgcgccaagcactactattacggaggaagctacgctatggactattg gggacagggcactctcgtgactgtgagcagcggcggtggagggtctggaggtggag gatccggtggtggtgggtcaggcggaggagggagcgagattgtgatgactcagtca ccagccaccctttctctttcacccggcgagagagcaaccctgagctgtagagccag ccaggacatttctaagtacctcaactggtatcagcaaaaaccggggcaggcccctc gcctcctgatctaccatacctcacgccttcactctggtatccccgctcggtttagc ggatcaggatctggtaccgactacactctgaccatttccagcctgcagccagaaga tttcgcagtgtatttctgccagcagggcaatacccttccttacaccttcggtcagg gaaccaagctcgaaatcaagcaccatcaccatcatcatcaccac |
| **CAR8** - **Soluble scFv - nt** | | |
| **100798** | 930 | MALPVTALLLPLALLLHAARPqvqlqesgpglvkpsetlsltctvsgvslpdygvs wirqppgkglewigviwgsettyyqsslksrvtiskdnsknqvslklssvtaadta vyycakhyyyggsyamdywgqgtlvtvssggggsggggsggggsggggseivmtqs patlslspgeratlscrasqdiskylnwyqqkpgqaprlliyhtsrlhsgiparfs gsgsgtdytltisslqpedfavyfcqqgntlpytfgqgtkleikhhhhhhhh |
| **CAR8 - Soluble scFv - aa** | | |
| **104882 CAR** | 931 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttggggtag cgaaaccacttactatcaatcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactg gggccagggaactctggtcactgtgtcatctggtggaggaggtagcggaggaggcgg gagcggtggaggtggctccggaggcggtgggtcagaaatcgtgatgacccagagc cctgcaaccctgtccctttctcccggggaacgggctaccctttcttgtcgggcatc acaagatatctcaaaatacctcaattggtatcaacagaagccgggacaggccccta ggcttcttatctaccacacctctcgcctgcatagcgggattcccgcacgctttagc gggtctggaagcgggaccgactacactctgaccatctcatctctccagcccgagga cttcgccgtctacttctgccagcagggtaacaccctgccgtacaccttcggccagg gcaccaagcttgagatcaaaaccactactcccgctccaaggccacccacccctgcc ccgaccatcgcctctcagccgctttccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **8 - Full** - **nt** | | |
| **104882** | 932 | MALPVTALLLPLALLLHAARPqvqlqesgpglvkpsetlsltctvsgvslpdygvs wirqppgkglewigviwgsettyyqsslksrvtiskdnsknqvslklssvtaadta vyycakhyyyggsyamdywgqgtlvtvssggggsggggsggggsggggseivmtqs patlslspgeratlscrasqdiskylnwyqqkpgqaprlliyhtsrlhsgiparfs gsgsgtdytltisslqpedfavyfcqqgntlpytfgqgtkleikttpaprpptpa ptiasqplslrpeacrpaaggavhtrgldfacdiyiwaplagtcgvlllslvitly ckrgrkkllyifkqpfmrpvqttqeedgcscrfpeeeeggcelrvkfsrsadapay kqgqnqlynelnlgrreeydvldkrrgrdpemggkprrknpqeglynelqkdkmae ayseigmkgerrrgkghdglyqglstatkdtydalhmqalppr |
| **CAR 8** - **Full - aa** | | |

| **CAR 9** | | |
|---|---|---|
| **CAR9 scFv domain** | 933 | eivmtqspatlslspgeratlscrasqdiskylnwyqqkpgqaprlliyhtsrlhs giparfsgsgsgtdytltisslqpedfavyfcqqgntlpytfgqgtkleikggggs ggggsggggsggggsqvqlqesgpglvkpsetlsltctvsgvslpdygvswirqpp gkglewigviwgsettyynsslksrvtiskdnsknqvslklssvtaadtavyycak hyyyggsyamdywgqgtlvtvss |
| **99789** | 934 | atggccctcccagtgaccgctctgctgctgcctctcgcacttcttctccatgccgc tcggcctgagatcgtcatgacccaaagccccgctaccctgtccctgtcacccggcg agagggcaaccctttcatgcagggccagccaggacatttctaagtacctcaactgg tatcagcagaagccagggcaggctcctcgcctgctgatctaccacaccagccgcct |
| **CAR9** -**Soluble scFv - nt** | | |
| | | ccacagcggtatccccgccagattttccgggagcgggtctggaaccgactacaccc tcaccatctcttctctgcagcccgaggatttcgccgtctatttctgccagcagggg aatactctgccgtacaccttcggtcaaggtaccaagctggaaatcaagggaggcgg aggatcaggcggtggcggaagcggaggaggtggctccggaggaggaggttcccaag tgcagcttcaagaatcaggacccggacttgtgaagccatcagaaaccctctccctg acttgtaccgtgtccggtgtgagcctccccgactacggagtctcttggattcgcca gcctccggggaagggtcttgaatggattggggtgatttggggatcagagactactt actacaattcatcacttaagtcacgggtcaccatcagcaaagataatagcaagaac caagtgtcacttaagctgtcatctgtgaccgccgctgacaccgccgtgtactattg tgccaaacattactattacggagggtcttatgctatggactactggggacagggga ccctggtgactgtctctagccatcaccatcaccaccatcatcac |
| **99789** | 935 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyynsslksrvtiskdnskn qvslklssvtaadtavyycakhyyyggsyamdywgqgtlvtvsshhhhhhhh |
| **CAR9 - Soluble scFv - aa** | | |
| **105974** | 936 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagcggaggcggtgggagccagg tccaactccaagaaagcggaccgggtcttgtgaagccatcagaaactctttcactg acttgtactgtgagcggagtgtctctccccgattacggggtgtcttggatcagaca gccaccggggaagggtctggaatggattggagtgatttggggctctgagactactt actacaactcatccctcaagtcacgcgtcaccatctcaaaggacaactctaagaat caggtgtcactgaaactgtcatctgtgaccgcagccgacaccgccgtgtactattg cgctaagcattactattatggcgggagctacgcaatggattactggggacagggta ctctggtcaccgtgtccagcaccactaccccagcaccgaggccacccaccccggct cctaccatcgcctcccagcctctgtccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **CAR 9** - **Full - nt** | | |
| **105974** | 937 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl |
| **CAR 9** - **Full- aa** | | |
| | | tctvsgvslpdygvswirqppgkglewigviwgsettyynsslksrvtiskdnskn qvslklssvtaadtavyycakhyyyggsyamdywgqgtlvtvsstttpaprpptpa ptiasqplslrpeacrpaaggavhtrgldfacdiyiwaplagtcgvlllslvitly ckrgrkkllyifkqpfmrpvqttqeedgcscrfpeeeeggcelrvkfsrsadapay kqgqnqlynelnlgrreeydvldkrrgrdpemggkprrknpqeglynelqkdkmae ayseigmkgerrrgkghdglyqglstatkdtydalhmqalppr |

| **CAR10** | | |
|---|---|---|
| **CAR10 scFv domain** | 938 | qvqlqesgpglvkpsetlsltctvsgvslpdygvswirqppgkglewigviwgset tyynsslksrvtiskdnsknqvslklssvtaadtavyycakhyyyggsyamdywgq gtlvtvssggggsggggsggggsggggseivmtqspatlslspgeratlscrasqd iskylnwyqqkpgqaprlliyhtsrlhsgiparfsgsgsgtdytltisslqpedfa vyfcqqgntlpytfgqgtkleik |
| **100796CAR10** - **Soluble scFv - nt** | 939 | atggcactgcctgtcactgccctcctgctgcctctggccctccttctgcatgccgc caggccccaagtccagctgcaagagtcaggacccggactggtgaagccgtctgaga ctctctcactgacttgtaccgtcagcggcgtgtccctccccgactacggagtgtca tggatccgccaacctcccgggaaagggcttgaatggattggtgtcatctggggttc tgaaaccacctactacaactcttccctgaagtccagggtgaccatcagcaaggata attccaagaaccaggtcagccttaagctgtcatctgtgaccgctgctgacaccgcc gtgtattactgcgccaagcactactattacggaggaagctacgctatggactattg gggacagggcactctcgtgactgtgagcagcggcggtggagggtctggaggtggag gatccggtggtggtgggtcaggcggaggagggagcgagattgtgatgactcagtca ccagccaccctttctctttcacccggcgagagagcaaccctgagctgtagagccag ccaggacatttctaagtacctcaactggtatcagcaaaaaccggggcaggcccctc gcctcctgatctaccatacctcacgccttcactctggtatccccgctcggtttagc ggatcaggatctggtaccgactacactctgaccatttccagcctgcagccagaaga tttcgcagtgtatttctgccagcagggcaatacccttccttacaccttcggtcagg gaaccaagctcgaaatcaagcaccatcaccatcatcaccaccat |
| **100796 CAR10** - **Soluble scFv - aa** | 940 | MALPVTALLLPLALLLHAARPqvqlqesgpglvkpsetlsltctvsgvslpdygvs wirqppgkglewigviwgsettyynsslksrvtiskdnsknqvslklssvtaadta vyycakhyyyggsyamdywgqgtlvtvssggggsggggsggggsggggseivmtqs patlslspgeratlscrasqdiskylnwyqqkpgqaprlliyhtsrlhsgiparfs gsgsgtdytltisslqpedfavyfcqqgntlpytfgqgtkleikhhhhhhhh |
| **105975 CAR** | 941 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagcggaggcggtgggagccagg tccaactccaagaaagcggaccgggtcttgtgaagccatcagaaactctttcactg acttgtactgtgagcggagtgtctctccccgattacggggtgtcttggatcagaca gccaccggggaagggtctggaatggattggagtgatttggggctctgagactactt actacaactcatccctcaagtcacgcgtcaccatctcaaaggacaactctaagaat |
| **10 Full** - **nt** | | |
| | | caggtgtcactgaaactgtcatctgtgaccgcagccgacaccgccgtgtactattg cgctaagcattactattatggcgggagctacgcaatggattactggggacagggta ctctggtcaccgtgtccagcaccactaccccagcaccgaggccacccaccccggct cctaccatcgcctcccagcctctgtccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **105975** | 942 | MALPVTALLLPLALLLHAARPEIVMTQSPATLSLSPGERATLSCRASQDISKYLNW YQQKPGQAPRLLIYHTSRLHSGIPARFSGSGSGTDYTLTISSLQPEDFAVYFCQQG NTLPYTFGQGTKLEIKGGGGSGGGGSGGGGSGGGGSQVQLQESGPGLVKPSETLSL TCTVSGVSLPDYGVSWIRQPPGKGLEWIGVIWGSETTYYNSSLKSRVTISKDNSKN QVSLKLSSVTAADTAVYYCAKHYYYGGSYAMDYWGQGTLVTVSSTTTPAPRPPTPA PTIASQPLSLRPEACRPAAGGAVHTRGLDFACDIYIWAPLAGTCGVLLLSLVITLY CKRGRKKLLYIFKQPFMRPVQTTQEEDGCSCRFPEEEEGGCELRVKFSRSADAPAY KQGQNQLYNELNLGRREEYDVLDKRRGRDPEMGGKPRRKNPQEGLYNELQKDKMAE AYSEIGMKGERRRGKGHDGLYQGLSTATKDTYDALHMQALPPR |
| **CAR 10 Full** - **aa** | | |

| **CAR11** | | |
|---|---|---|
| **CAR11 scFv domain** | 943 | eivmtqspatlslspgeratlscrasqdiskylnwyqqkpgqaprlliyhtsrlhs giparfsgsgsgtdytltisslqpedfavyfcqqgntlpytfgqgtkleikggggs ggggsggggsqvqlqesgpglvkpsetlsltctvsgvslpdygvswirqppgkgle wigviwgsettyynsslksrvtiskdnsknqvslklssvtaadtavyycakhyyyg gsyamdywgqgtlvtvss |
| **103101** | 944 | Atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactacaattcatccc tcaagtcacgcgtcaccatctcaaaggacaactctaagaatcaggtgtcactgaaa |
| **CAR11** - **Soluble scFv - nt** | | |
| | | ctgtcatctgtgaccgcagccgacaccgccgtgtactattgcgctaagcattacta ttatggcgggagctacgcaatggattactggggacagggtactctggtcaccgtgt ccagccaccaccatcatcaccatcaccat |
| **103101** | 945 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsqvqlqesgpglvkpsetlsltctvs gvslpdygvswirqppgkglewigviwgsettyynsslksrvtiskdnsknqvslk lssvtaadtavyycakhyyyggsyamdywgqgtlvtvsshhhhhhhh |
| **CAR11** - **Soluble scFv - aa** | | |
| **105976 CAR** | 946 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttggggtag cgaaaccacttactataactcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactg gggccagggaactctggtcactgtgtcatctggtggaggaggtagcggaggaggcg ggagcggtggaggtggctccggaggtggcggaagcgaaatcgtgatgacccagagc cctgcaaccctgtccctttctcccggggaacgggctaccctttcttgtcgggcatc acaagatatctcaaaatacctcaattggtatcaacagaagccgggacaggccccta ggcttcttatctaccacacctctcgcctgcatagcgggattcccgcacgctttagc gggtctggaagcgggaccgactacactctgaccatctcatctctccagcccgagga cttcgccgtctacttctgccagcagggtaacaccctgccgtacaccttcggccagg gcaccaagcttgagatcaaaaccactactcccgctccaaggccacccacccctgcc ccgaccatcgcctctcagccgctttccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **11 Full** - **nt** | | |
| **105976** | 947 | MALPVTALLLPLALLLHAARPQVQLQESGPGLVKPSETLSLTCTVSGVSLPDYGVS WIRQPPGKGLEWIGVIWGSETTYYNSSLKSRVTISKDNSKNQVSLKLSSVTAADTA VYYCAKHYYYGGSYAMDYWGQGTLVTVSSGGGGSGGGGSGGGGSGGGGSEIVMTQS PATLSLSPGERATLSCRASQDISKYLNWYQQKPGQAPRLLIYHTSRLHSGIPARFS GSGSGTDYTLTISSLQPEDFAVYFCQQGNTLPYTFGQGTKLEIKTTTPAPRPPTPA PTIASQPLSLRPEACRPAAGGAVHTRGLDFACDIYIWAPLAGTCGVLLLSLVITLY CKRGRKKLLYIFKQPFMRPVQTTQEEDGCSCRFPEEEEGGCELRVKFSRSADAPAY KQGQNQLYNELNLGRREEYDVLDKRRGRDPEMGGKPRRKNPQEGLYNELQKDKMAE AYSEIGMKGERRRGKGHDGLYQGLSTATKDTYDALHMQALPPR |
| **CAR 11 Full** - **aa** | | |
| | | |

| **CAR12** | | |
|---|---|---|
| **CAR12** | 948 | qvqlqesgpglvkpsetlsltctvsgvslpdygvswirqppgkglewigviwgset tyynsslksrvtiskdnsknqvslklssvtaadtavyycakhyyyggsyamdywgq gtlvtvssggggsggggsggggseivmtqspatlslspgeratlscrasqdiskyl nwyqqkpgqaprlliyhtsrlhsgiparfsgsgsgtdytltisslqpedfavyfcq qgntlpytfgqgtkleik |
| **scFv domain** | | |
| **103104** | 949 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttggggtag cgaaaccacttactataactcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactgg ggccagggaactctggtcactgtgtcatctggtggaggaggtagcggaggaggcg ggagcggtggaggtggctccgaaatcgtgatgacccagagccctgcaaccctgtcc ctttctcccggggaacgggctaccctttcttgtcgggcatcacaagatatctcaaa atacctcaattggtatcaacagaagccgggacaggcccctaggcttcttatctacc acacctctcgcctgcatagcgggattcccgcacgctttagcgggtctggaagcggg accgactacactctgaccatctcatctctccagcccgaggacttcgccgtctactt ctgccagcagggtaacaccctgccgtacaccttcggccagggcaccaagcttgaga tcaaacatcaccaccatcatcaccatcac |
| **CAR12 - Soluble scFv - nt** | | |
| **103104** | 950 | MALPVTALLLPLALLLHAARPqvqlqesgpglvkpsetlsltctvsgvslpdygvs wirqppgkglewigviwgsettyynsslksrvtiskdnsknqvslklssvtaadta vyycakhyyyggsyamdywgqgtlvtvssggggsggggsggggseivmtqspatls lspgeratlscrasqdiskylnwyqqkpgqaprlliyhtsrlhsgiparfsgsgsg tdytltisslqpedfavyfcqqgntlpytfgqgtkleikhhhhhhhh |
| **CAR12** - **Soluble scFv -aa** | | |
| **105977** | 951 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactacaactcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt ccagcaccactaccccagcaccgaggccacccaccccggctcctaccatcgcctcc cagcctctgtccctgcgtccggaggcatgtagacccgcagctggtggggccgtgca tacccggggtcttgacttcgcctgcgatatctacatttgggcccctctggctggta |
| **CAR 12** - **Full** - **nt** | | |
| | | cttgcggggtcctgctgctttcactcgtgatcactctttactgtaagcgcggtcgg aagaagctgctgtacatctttaagcaacccttcatgaggcctgtgcagactactca agaggaggacggctgttcatgccggttcccagaggaggaggaaggcggctgcgaac tgcgcgtgaaattcagccgcagcgcagatgctccagcctacaagcaggggcagaac cagctctacaacgaactcaatcttggtcggagagaggagtacgacgtgctggacaa gcggagaggacgggacccagaaatgggcgggaagccgcgcagaaagaatccccaag agggcctgtacaacgagctccaaaaggataagatggcagaagcctatagcgagatt ggtatgaaaggggaacgcagaagaggcaaaggccacgacggactgtaccagggact cagcaccgccaccaaggacacctatgacgctcttcacatgcaggccctgccgcctc gg |
| **105977** | 952 | MALPVTALLLPLALLLHAARPEIVMTQSPATLSLSPGERATLSCRASQDISKYLNW YQQKPGQAPRLLIYHTSRLHSGIPARFSGSGSGTDYTLTISSLQPEDFAVYFCQQG NTLPYTFGQGTKLEIKGGGGSGGGGSGGGGSQVQLQESGPGLVKPSETLSLTCTVS GVSLPDYGVSWIRQPPGKGLEWIGVIWGSETTYYNSSLKSRVTISKDNSKNQVSLK LSSVTAADTAVYYCAKHYYYGGSYAMDYWGQGTLVTVSSTTTPAPRPPTPAPTIAS QPLSLRPEACRPAAGGAVHTRGLDFACDIYIWAPLAGTCGVLLLSLVITLYCKRGR KKLLYIFKQPFMRPVQTTQEEDGCSCRFPEEEEGGCELRVKFSRSADAPAYKQGQN QLYNELNLGRREEYDVLDKRRGRDPEMGGKPRRKNPQEGLYNELQKDKMAEAYSEI GMKGERRRGKGHDGLYQGLSTATKDTYDALHMQALPPR |
| **CAR 12** - **Full - aa** | | |

| **CTL019** | | |
|---|---|---|
| **CTL019** - **Soluble** | 953 | atggccctgcccgtcaccgctctgctgctgccccttgctctgcttcttcatgcagc aaggccggacatccagatgacccaaaccacctcatccctctctgcctctcttggag acagggtgaccatttcttgtcgcgccagccaggacatcagcaagtatctgaactgg tatcagcagaagccggacggaaccgtgaagctcctgatctaccatacctctcgcct gcatagcggcgtgccctcacgcttctctggaagcggatcaggaaccgattattctc tcactatttcaaatcttgagcaggaagatattgccacctatttctgccagcagggt aataccctgccctacaccttcggaggagggaccaagctcgaaatcaccggtggagg aggcagcggcggtggagggtctggtggaggtggttctgaggtgaagctgcaagaat caggccctggacttgtggccccttcacagtccctgagcgtgacttgcaccgtgtcc ggagtctccctgcccgactacggagtgtcatggatcagacaacctccacggaaagg actggaatggctcggtgtcatctggggtagcgaaactacttactacaattcagccc tcaaaagcaggctgactattatcaaggacaacagcaagtcccaagtctttcttaag atgaactcactccagactgacgacaccgcaatctactattgtgctaagcactacta ctacggaggatcctacgctatggattactggggacaaggtacttccgtcactgtct cttcacaccatcatcaccatcaccatcac |
| **scFv-Histag - nt** | | |
| **CTL019** - **Soluble** | 954 | MALPVTALLLPLALLLHAARPdiqmtqttsslsaslgdrvtiscrasqdiskylnw yqqkpdgtvklliyhtsrlhsgvpsrfsgsgsgtdysltisnleqediatyfcqqg ntlpytfgggtkleitggggsggggsggggsevklqesgpglvapsqslsvtctvs gvslpdygvswirqpprkglewlgviwgsettyynsalksrltiikdnsksqvflk mnslqtddtaiyycakhyyyggsyamdywgqgtsvtvsshhhhhhhh |
| **scFv-Histag** - **aa** | | |
| **CTL019 Full** - nt | 955 | atggccttaccagtgaccgccttgctcctgccgctggccttgctgctccacgccgc caggccggacatccagatgacacagactacatcctccctgtctgcctctctgggag acagagtcaccatcagttgcagggcaagtcaggacattagtaaatatttaaattgg tatcagcagaaaccagatggaactgttaaactcctgatctaccatacatcaagatt acactcaggagtcccatcaaggttcagtggcagtgggtctggaacagattattctc tcaccattagcaacctggagcaagaagatattgccacttacttttgccaacagggt aatacgcttccgtacacgttcggaggggggaccaagctggagatcacaggtggcgg tggctcgggcggtggtgggtcgggtggcggcggatctgaggtgaaactgcaggagt caggacctggcctggtggcgccctcacagagcctgtccgtcacatgcactgtctca ggggtctcattacccgactatggtgtaagctggattcgccagcctccacgaaaggg tctggagtggctgggagtaatatggggtagtgaaaccacatactataattcagctc tcaaatccagactgaccatcatcaaggacaactccaagagccaagttttcttaaaa atgaacagtctgcaaactgatgacacagccatttactactgtgccaaacattatta ctacggtggtagctatgctatggactactggggccaaggaacctcagtcaccgtct cctcaaccacgacgccagcgccgcgaccaccaacaccggcgcccaccatcgcgtcg cagcccctgtccctgcgcccagaggcgtgccggccagcggcggggggcgcagtgca cacgagggggctggacttcgcctgtgatatctacatctgggcgcccttggccggga cttgtggggtccttctcctgtcactggttatcaccctttactgcaaacggggcaga aagaaactcctgtatatattcaaacaaccatttatgagaccagtacaaactactca agaggaagatggctgtagctgccgatttccagaagaagaagaaggaggatgtgaac tgagagtgaagttcagcaggagcgcagacgcccccgcgtacaagcagggccagaac cagctctataacgagctcaatctaggacgaagagaggagtacgatgttttggacaa gagacgtggccgggaccctgagatggggggaaagccgagaaggaagaaccctcagg aaggcctgtacaatgaactgcagaaagataagatggcggaggcctacagtgagatt gggatgaaaggcgagcgccggaggggcaaggggcacgatggcctttaccagggtct cagtacagccaccaaggacacctacgacgcccttcacatgcaggccctgccccctc gc |
| **CTL019 Full** - **aa** | 956 | MALPVTALLLPLALLLHAARPdiqmtqttsslsaslgdrvtiscrasqdiskylnw yqqkpdgtvklliyhtsrlhsgvpsrfsgsgsgtdysltisnleqediatyfcqqg ntlpytfgggtkleitggggsggggsggggsevklqesgpglvapsqslsvtctvs gvslpdygvswirqpprkglewlgviwgsettyynsalksrltiikdnsksqvflk mnslqtddtaiyycakhyyyggsyamdywgqgtsvtvsstttpaprpptpaptias qplslrpeacrpaaggavhtrgldfacdiyiwaplagtcgvlllslvitlyckrgr kkllyifkqpfmrpvqttqeedgcscrfpeeeeggcelrvkfsrsadapaykqgqn qlynelnlgrreeydvldkrrgrdpemggkprrknpqeglynelqkdkmaeaysei gmkgerrrgkghdglyqglstatkdtydalhmqalppr |
| **CTL019 scFv domain** | 957 | diqmtqttsslsaslgdrvtiscrasqdiskylnwyqqkpdgtvklliyhtsrlhs gvpsrfsgsgsgtdysltisnleqediatyfcqqgntlpytfgggtkleitggggs ggggsggggsevklqesgpglvapsqslsvtctvsgvslpdygvswirqpprkgle wlgviwgsettyynsalksrltiikdnsksqvflkmnslqtddtaiyycakhyyyg gsyamdywgqgtsvtvss |

In some embodiments, the CD19 CAR or binding domain includes the amino acid sequence of CTL019, or is encoded by the nucleotide sequence of CTL019 according to Table 9 with or without the leader sequence or the his tag, or a sequence substantially identical thereto (e.g., at least 85%, 90%, 95% or higher identity).

In some embodiments, the CDRs are defined according to the Kabat numbering scheme, the Chothia numbering scheme, or a combination thereof.

The sequences of humanized CDR sequences of the scFv domains are shown in Table 10A for the heavy chain variable domains and in Table 10B for the light chain variable domains. "ID" stands for the respective SEQ ID NO for each CDR.

**Table 10A. Heavy Chain Variable Domain CDRs (according to Kabat)**

| Candidate | FW | HCDR1 | SEQ ID | HCDR2 | SEQ ID | HCDR3 | SEQ ID |
|---|---|---|---|---|---|---|---|
| murine_CART19 | | DYGVS | 958 | VIWGSETTYYNSALKS | 959 | HYYYGGSYAMDY | 960 |
| humanized_CART19 a | VH4 | DYGVS | 958 | VIWGSETTYYSSSLKS | 961 | HYYYGGSYAMDY | 960 |
| humanized_CART19 b | VH4 | DYGVS | 958 | VIWGSETTYYQSSLKS | 962 | HYYYGGSYAMDY | 960 |
| humanized_CART19 c | VH4 | DYGVS | 958 | VIWGSETTYYNSSLKS | 963 | HYYYGGSYAMDY | 960 |

**Table 10B Light Chain Variable Domain CDRs (according to Kabat)**

| Candidate | FW | LCDR1 | SEQ ID | LCDR2 | SEQ ID | LCDR3 | SEQ ID |
|---|---|---|---|---|---|---|---|
| murine_CART19 | | RASQDISKYLN | 964 | HTSRLHS | 965 | QQGNTLPYT | 966 |
| humanized_CART19 a | VK3 | RASQDISKYLN | 964 | HTSRLHS | 965 | QQGNTLPYT | 966 |
| humanized_CART19 b | VK3 | RASQDISKYLN | 964 | HTSRLHS | 965 | QQGNTLPYT | 966 |
| humanized_CART19 c | VK3 | RASQDISKYLN | 964 | HTSRLHS | 965 | QQGNTLPYT | 966 |

In one embodiment, the CAR molecule comprises a BCMA CAR molecule described herein, e.g., a BCMA CAR described in US-2016-0046724-A1 or WO2016/014565. In embodiments, the BCMA CAR comprises an amino acid, or has a nucleotide sequence of a CAR molecule, or an antigen binding domain according to US-2016-0046724-A1, or Table 1 or 16, SEQ ID NO: 271 or SEQ ID NO: 273 of WO2016/014565, or a sequence substantially identical to any of the aforesaid sequences (e.g., at least 85%, 90%, 95% or more identical to any of the aforesaid BCMA CAR sequences). The amino acid and nucleotide sequences encoding the BCMA CAR molecules and antigen binding domains (e.g., including one, two, three VH CDRs; and one, two, three VL CDRs according to Kabat or Chothia), are specified in WO2016/014565.

In embodiments, the BCMA CAR comprises an anti-BCMA binding domain (e.g., human or humanized anti-BCMA binding domain), a transmembrane domain, and an intracellular signaling domain, and wherein said anti-BCMA binding domain comprises a heavy chain complementary determining region 1 (HC CDR1), a heavy chain complementary determining region 2 (HC CDR2), and a heavy chain complementary determining region 3 (HC CDR3) of any anti-BMCA heavy chain binding domain amino acid sequences listed in Table 11A or 11B, or a sequence at least 85%, 90%, 95% or more identical thereto (e.g., having less than 5, 4, 3, 2 or 1 amino acid substitutions, e.g., conservative substitutions).

In one embodiment, the anti- BCMA binding domain comprises a light chain variable region described herein (e.g., in Table 11A or 11B) and/or a heavy chain variable region described herein (e.g., in Table 11A or 11B), or a sequence at least 85%, 90%, 95% or more identical thereto.

In one embodiment, the encoded anti- BCMA binding domain is a scFv comprising a light chain and a heavy chain of an amino acid sequence of Table 11A or 11B.

In an embodiment, the human or humanized anti-BCMA binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 11A or 11B, or a sequence at least 85%, 90%, 95% or more identical thereto; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 11A or 11B, or a sequence at least 85%, 90%, 95% or more identical thereto.

**Table 11A. Amino Acid and Nucleic Acid Sequences of exemplary anti-BCMA scFv domains and BCMA CAR molecules**

| **Name/ Description** | **SEQ ID NO:** | **Sequence** |
|---|---|---|
| **139109** | | |
| **139109- aa** | 967 | EVQLVESGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSSASGGGGSGGRASGGGGSDIQLTQSPSSLS ASVGDRVTITCRASQSISSYLNWYQQKPGKAPKLLIYAASSLQSGVPS RFSGSGSGTDFTLTISSLQPEDFATYYCQQSYSTPYTFGQGTKVEIK |
| **ScFv domain** | | |
| **139109- nt** | 968 | GAAGTGCAATTGGTGGAATCAGGGGGAGGACTTGTGCAGCCTGGAGGA TCGCTGAGACTGTCATGTGCCGTGTCCGGCTTTGCCCTGTCCAACCAC GGGATGTCCTGGGTCCGCCGCGCGCCTGGAAAGGGCCTCGAATGGGTG TCGGGTATTGTGTACAGCGGTAGCACCTACTATGCCGCATCCGTGAAG GGGAGATTCACCATCAGCCGGGACAACTCCAGGAACACTCTGTACCTC CAAATGAATTCGCTGAGGCCAGAGGACACTGCCATCTACTACTGCTCC GCGCATGGCGGAGAGTCCGACGTCTGGGGACAGGGGACCACCGTGACC GTGTCTAGCGCGTCCGGCGGAGGCGGCAGCGGGGGTCGGGCATCAGGG GGCGGCGGATCGGACATCCAGCTCACCCAGTCCCCGAGCTCGCTGTCC GCCTCCGTGGGAGATCGGGTCACCATCACGTGCCGCGCCAGCCAGTCG ATTTCCTCCTACCTGAACTGGTACCAACAGAAGCCCGGAAAAGCCCCG AAGCTTCTCATCTACGCCGCCTCGAGCCTGCAGTCAGGAGTGCCCTCA CGGTTCTCCGGCTCCGGTTCCGGTACTGATTTCACCCTGACCATTTCC TCCCTGCAACCGGAGGACTTCGCTACTTACTACTGCCAGCAGTCGTAC TCCACCCCCTACACTTTCGGACAAGGCACCAAGGTCGAAATCAAG |
| **ScFv domain** | | |
| **139109- aa** | 969 | EVQLVESGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSS |
| **VH** | | |
| **139109- aa** | 970 | DIQLTQSPSSLSASVGDRVTITCRASQSISSYLNWYQQKPGKAPKLLI YAASSLQSGVPSRFSGSGSGTDFTLTISSLQPEDFATYYCQQSYSTPY TFGQGTKVEIK |
| **VL** | | |
| **139109- aa** | 971 | MALPVTALLLPLALLLHAARPEVQLVESGGGLVQPGGSLRLSCAVSGF ALSNHGMSWVRRAPGKGLEWVSGIVYSGSTYYAASVKGRFTISRDNSR NTLYLQMNSLRPEDTAIYYCSAHGGESDVWGQGTTVTVSSASGGGGSG GRASGGGGSDIQLTQSPSSLSASVGDRVTITCRASQSISSYLNWYQQK PGKAPKLLIYAASSLQSGVPSRFSGSGSGTDFTLTISSLQPEDFATYY CQQSYSTPYTFGQGTKVEIKTTTPAPRPPTPAPTIASQPLSLRPEACR PAAGGAVHTRGLDFACDIYIWAPLAGTCGVLLLSLVITLYCKRGRKKL LYIFKQPFMRPVQTTQEEDGCSCRFPEEEEGGCELRVKFSRSADAPAY KQGQNQLYNELNLGRREEYDVLDKRRGRDPEMGGKPRRKNPQEGLYNE LQKDKMAEAYSEIGMKGERRRGKGHDGLYQGLSTATKDTYDALHMQAL PPR |
| **Full CAR** | | |
| **139109- nt Full CAR** | 972 | ATGGCCCTCCCTGTCACCGCCCTGCTGCTTCCGCTGGCTCTTCTGCTC CACGCCGCTCGGCCCGAAGTGCAATTGGTGGAATCAGGGGGAGGACTT GTGCAGCCTGGAGGATCGCTGAGACTGTCATGTGCCGTGTCCGGCTTT GCCCTGTCCAACCACGGGATGTCCTGGGTCCGCCGCGCGCCTGGAAAG GGCCTCGAATGGGTGTCGGGTATTGTGTACAGCGGTAGCACCTACTAT GCCGCATCCGTGAAGGGGAGATTCACCATCAGCCGGGACAACTCCAGG AACACTCTGTACCTCCAAATGAATTCGCTGAGGCCAGAGGACACTGCC ATCTACTACTGCTCCGCGCATGGCGGAGAGTCCGACGTCTGGGGACAG GGGACCACCGTGACCGTGTCTAGCGCGTCCGGCGGAGGCGGCAGCGGG GGTCGGGCATCAGGGGGCGGCGGATCGGACATCCAGCTCACCCAGTCC CCGAGCTCGCTGTCCGCCTCCGTGGGAGATCGGGTCACCATCACGTGC CGCGCCAGCCAGTCGATTTCCTCCTACCTGAACTGGTACCAACAGAAG CCCGGAAAAGCCCCGAAGCTTCTCATCTACGCCGCCTCGAGCCTGCAG TCAGGAGTGCCCTCACGGTTCTCCGGCTCCGGTTCCGGTACTGATTTC ACCCTGACCATTTCCTCCCTGCAACCGGAGGACTTCGCTACTTACTAC TGCCAGCAGTCGTACTCCACCCCCTACACTTTCGGACAAGGCACCAAG GTCGAAATCAAGACCACTACCCCAGCACCGAGGCCACCCACCCCGGCT CCTACCATCGCCTCCCAGCCTCTGTCCCTGCGTCCGGAGGCATGTAGA CCCGCAGCTGGTGGGGCCGTGCATACCCGGGGTCTTGACTTCGCCTGC GATATCTACATTTGGGCCCCTCTGGCTGGTACTTGCGGGGTCCTGCTG CTTTCACTCGTGATCACTCTTTACTGTAAGCGCGGTCGGAAGAAGCTG CTGTACATCTTTAAGCAACCCTTCATGAGGCCTGTGCAGACTACTCAA GAGGAGGACGGCTGTTCATGCCGGTTCCCAGAGGAGGAGGAAGGCGGC TGCGAACTGCGCGTGAAATTCAGCCGCAGCGCAGATGCTCCAGCCTAC AAGCAGGGGCAGAACCAGCTCTACAACGAACTCAATCTTGGTCGGAGA GAGGAGTACGACGTGCTGGACAAGCGGAGAGGACGGGACCCAGAAATG GGCGGGAAGCCGCGCAGAAAGAATCCCCAAGAGGGCCTGTACAACGAG CTCCAAAAGGATAAGATGGCAGAAGCCTATAGCGAGATTGGTATGAAA GGGGAACGCAGAAGAGGCAAAGGCCACGACGGACTGTACCAGGGACTC AGCACCGCCACCAAGGACACCTATGACGCTCTTCACATGCAGGCCCTG CCGCCTCGG |

| **139103** | | |
|---|---|---|
| **139103- aa** | 973 | QVQLVESGGGLVQPGRSLRLSCAASGFTFSNYAMSWVRQAPGKGLGWV SGISRSGENTYYADSVKGRFTISRDNSKNTLYLQMNSLRDEDTAVYYC ARSPAHYYGGMDVWGQGTTVTVSSASGGGGSGGRASGGGGSDIVLTQS PGTLSLSPGERATLSCRASQSISSSFLAWYQQKPGQAPRLLIYGASRR ATGIPDRFSGSGSGTDFTLTISRLEPEDSAVYYCQQYHSSPSWTFGQG TKLEIK |
| **ScFv domain** | | |
| **139103- nt** | 974 | CAAGTGCAACTCGTGGAATCTGGTGGAGGACTCGTGCAACCCGGAAGA TCGCTTAGACTGTCGTGTGCCGCCAGCGGGTTCACTTTCTCGAACTAC GCGATGTCCTGGGTCCGCCAGGCACCCGGAAAGGGACTCGGTTGGGTG TCCGGCATTTCCCGGTCCGGCGAAAATACCTACTACGCCGACTCCGTG AAGGGCCGCTTCACCATCTCAAGGGACAACAGCAAAAACACCCTGTAC TTGCAAATGAACTCCCTGCGGGATGAAGATACAGCCGTGTACTATTGC GCCCGGTCGCCTGCCCATTACTACGGCGGAATGGACGTCTGGGGACAG |
| **ScFv domain** | | |
| | | GGAACCACTGTGACTGTCAGCAGCGCGTCGGGTGGCGGCGGCTCAGGG GGTCGGGCCTCCGGGGGGGGAGGGTCCGACATCGTGCTGACCCAGTCC CCGGGAACCCTGAGCCTGAGCCCGGGAGAGCGCGCGACCCTGTCATGC CGGGCATCCCAGAGCATTAGCTCCTCCTTTCTCGCCTGGTATCAGCAG AAGCCCGGACAGGCCCCGAGGCTGCTGATCTACGGCGCTAGCAGAAGG GCTACCGGAATCCCAGACCGGTTCTCCGGCTCCGGTTCCGGGACCGAT TTCACCCTTACTATCTCGCGCCTGGAACCTGAGGACTCCGCCGTCTAC TACTGCCAGCAGTACCACTCATCCCCGTCGTGGACGTTCGGACAGGGC ACCAAGCTGGAGATTAAG |
| **139103- aa** | 975 | QVQLVESGGGLVQPGRSLRLSCAASGFTFSNYAMSWVRQAPGKGLGWV SGISRSGENTYYADSVKGRFTISRDNSKNTLYLQMNSLRDEDTAVYYC ARSPAHYYGGMDVWGQGTTVTVSS |
| **VH** | | |
| **139103- aa** | 976 | DIVLTQSPGTLSLSPGERATLSCRASQSISSSFLAWYQQKPGQAPRLL IYGASRRATGIPDRFSGSGSGTDFTLTISRLEPEDSAVYYCQQYHSSP SWTFGQGTKLEIK |
| **VL** | | |
| **139103- aa** | 977 | MALPVTALLLPLALLLHAARPQVQLVESGGGLVQPGRSLRLSCAASGF TFSNYAMSWVRQAPGKGLGWVSGISRSGENTYYADSVKGRFTISRDNS KNTLYLQMNSLRDEDTAVYYCARSPAHYYGGMDVWGQGTTVTVSSASG GGGSGGRASGGGGSDIVLTQSPGTLSLSPGERATLSCRASQSISSSFL AWYQQKPGQAPRLLIYGASRRATGIPDRFSGSGSGTDFTLTISRLEPE DSAVYYCQQYHSSPSWTFGQGTKLEIKTTTPAPRPPTPAPTIASQPLS LRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNP QEGLYNELQKDKMAEAYSEIGMKGERRRGKGHDGLYQGLSTATKDTYD ALHMQALPPR |
| **Full CAR** | | |
| **139103- nt** | 978 | ATGGCCCTCCCTGTCACCGCCCTGCTGCTTCCGCTGGCTCTTCTGCTC CACGCCGCTCGGCCCCAAGTGCAACTCGTGGAATCTGGTGGAGGACTC GTGCAACCCGGAAGATCGCTTAGACTGTCGTGTGCCGCCAGCGGGTTC ACTTTCTCGAACTACGCGATGTCCTGGGTCCGCCAGGCACCCGGAAAG GGACTCGGTTGGGTGTCCGGCATTTCCCGGTCCGGCGAAAATACCTAC TACGCCGACTCCGTGAAGGGCCGCTTCACCATCTCAAGGGACAACAGC AAAAACACCCTGTACTTGCAAATGAACTCCCTGCGGGATGAAGATACA GCCGTGTACTATTGCGCCCGGTCGCCTGCCCATTACTACGGCGGAATG GACGTCTGGGGACAGGGAACCACTGTGACTGTCAGCAGCGCGTCGGGT GGCGGCGGCTCAGGGGGTCGGGCCTCCGGGGGGGGAGGGTCCGACATC GTGCTGACCCAGTCCCCGGGAACCCTGAGCCTGAGCCCGGGAGAGCGC GCGACCCTGTCATGCCGGGCATCCCAGAGCATTAGCTCCTCCTTTCTC GCCTGGTATCAGCAGAAGCCCGGACAGGCCCCGAGGCTGCTGATCTAC GGCGCTAGCAGAAGGGCTACCGGAATCCCAGACCGGTTCTCCGGCTCC GGTTCCGGGACCGATTTCACCCTTACTATCTCGCGCCTGGAACCTGAG GACTCCGCCGTCTACTACTGCCAGCAGTACCACTCATCCCCGTCGTGG ACGTTCGGACAGGGCACCAAGCTGGAGATTAAGACCACTACCCCAGCA CCGAGGCCACCCACCCCGGCTCCTACCATCGCCTCCCAGCCTCTGTCC |
| **Full CAR** | | |
| | | CTGCGTCCGGAGGCATGTAGACCCGCAGCTGGTGGGGCCGTGCATACC CGGGGTCTTGACTTCGCCTGCGATATCTACATTTGGGCCCCTCTGGCT GGTACTTGCGGGGTCCTGCTGCTTTCACTCGTGATCACTCTTTACTGT AAGCGCGGTCGGAAGAAGCTGCTGTACATCTTTAAGCAACCCTTCATG AGGCCTGTGCAGACTACTCAAGAGGAGGACGGCTGTTCATGCCGGTTC CCAGAGGAGGAGGAAGGCGGCTGCGAACTGCGCGTGAAATTCAGCCGC AGCGCAGATGCTCCAGCCTACAAGCAGGGGCAGAACCAGCTCTACAAC GAACTCAATCTTGGTCGGAGAGAGGAGTACGACGTGCTGGACAAGCGG AGAGGACGGGACCCAGAAATGGGCGGGAAGCCGCGCAGAAAGAATCCC CAAGAGGGCCTGTACAACGAGCTCCAAAAGGATAAGATGGCAGAAGCC TATAGCGAGATTGGTATGAAAGGGGAACGCAGAAGAGGCAAAGGCCAC GACGGACTGTACCAGGGACTCAGCACCGCCACCAAGGACACCTATGAC GCTCTTCACATGCAGGCCCTGCCGCCTCGG |

| **139105** | | |
|---|---|---|
| **139105- aa** | 979 | QVQLVESGGGLVQPGRSLRLSCAASGFTFDDYAMHWVRQAPGKGLEWV SGISWNSGSIGYADSVKGRFTISRDNAKNSLYLQMNSLRAEDTALYYC SVHSFLAYWGQGTLVTVSSASGGGGSGGRASGGGGSDIVMTQTPLSLP VTPGEPASISCRSSQSLLHSNGYNYLDWYLQKPGQSPQLLIYLGSNRA SGVPDRFSGSGSGTDFTLKISRVEAEDVGVYYCMQALQTPYTFGQGTK VEIK |
| **ScFv domain** | | |
| **139105- nt** | 980 | CAAGTGCAACTCGTCGAATCCGGTGGAGGTCTGGTCCAACCTGGTAGA AGCCTGAGACTGTCGTGTGCGGCCAGCGGATTCACCTTTGATGACTAT GCTATGCACTGGGTGCGGCAGGCCCCAGGAAAGGGCCTGGAATGGGTG TCGGGAATTAGCTGGAACTCCGGGTCCATTGGCTACGCCGACTCCGTG AAGGGCCGCTTCACCATCTCCCGCGACAACGCAAAGAACTCCCTGTAC TTGCAAATGAACTCGCTCAGGGCTGAGGATACCGCGCTGTACTACTGC TCCGTGCATTCCTTCCTGGCCTACTGGGGACAGGGAACTCTGGTCACC GTGTCGAGCGCCTCCGGCGGCGGGGGCTCGGGTGGACGGGCCTCGGGC GGAGGGGGGTCCGACATCGTGATGACCCAGACCCCGCTGAGCTTGCCC GTGACTCCCGGAGAGCCTGCATCCATCTCCTGCCGGTCATCCCAGTCC CTTCTCCACTCCAACGGATACAACTACCTCGACTGGTACCTCCAGAAG CCGGGACAGAGCCCTCAGCTTCTGATCTACCTGGGGTCAAATAGAGCC TCAGGAGTGCCGGATCGGTTCAGCGGATCTGGTTCGGGAACTGATTTC ACTCTGAAGATTTCCCGCGTGGAAGCCGAGGACGTGGGCGTCTACTAC TGTATGCAGGCGCTGCAGACCCCCTATACCTTCGGCCAAGGGACGAAA GTGGAGATCAAG |
| **ScFv domain** | | |
| **139105- aa** | 981 | QVQLVESGGGLVQPGRSLRLSCAASGFTFDDYAMHWVRQAPGKGLEWV SGISWNSGSIGYADSVKGRFTISRDNAKNSLYLQMNSLRAEDTALYYC SVHSFLAYWGQGTLVTVSS |
| **VH** | | |
| **139105- aa** | 982 | DIVMTQTPLSLPVTPGEPASISCRSSQSLLHSNGYNYLDWYLQKPGQS PQLLIYLGSNRASGVPDRFSGSGSGTDFTLKISRVEAEDVGVYYCMQA LQTPYTFGQGTKVEIK |
| **VL** | | |
| **139105- aa** | 983 | MALPVTALLLPLALLLHAARPQVQLVESGGGLVQPGRSLRLSCAASGF TFDDYAMHWVRQAPGKGLEWVSGISWNSGSIGYADSVKGRFTISRDNA KNSLYLQMNSLRAEDTALYYCSVHSFLAYWGQGTLVTVSSASGGGGSG GRASGGGGSDIVMTQTPLSLPVTPGEPASISCRSSQSLLHSNGYNYLD WYLQKPGQSPQLLIYLGSNRASGVPDRFSGSGSGTDFTLKISRVEAED VGVYYCMQALQTPYTFGQGTKVEIKTTTPAPRPPTPAPTIASQPLSLR PEACRPAAGGAVHTRGLDFACDIYIWAPLAGTCGVLLLSLVITLYCKR GRKKLLYIFKQPFMRPVQTTQEEDGCSCRFPEEEEGGCELRVKFSRSA DAPAYKQGQNQLYNELNLGRREEYDVLDKRRGRDPEMGGKPRRKNPQE GLYNELQKDKMAEAYSEIGMKGERRRGKGHDGLYQGLSTATKDTYDAL HMQALPPR |
| **Full CAR** | | |
| **139105- nt** | 984 | ATGGCCCTCCCTGTCACCGCCCTGCTGCTTCCGCTGGCTCTTCTGCTC CACGCCGCTCGGCCCCAAGTGCAACTCGTCGAATCCGGTGGAGGTCTG GTCCAACCTGGTAGAAGCCTGAGACTGTCGTGTGCGGCCAGCGGATTC ACCTTTGATGACTATGCTATGCACTGGGTGCGGCAGGCCCCAGGAAAG GGCCTGGAATGGGTGTCGGGAATTAGCTGGAACTCCGGGTCCATTGGC TACGCCGACTCCGTGAAGGGCCGCTTCACCATCTCCCGCGACAACGCA AAGAACTCCCTGTACTTGCAAATGAACTCGCTCAGGGCTGAGGATACC GCGCTGTACTACTGCTCCGTGCATTCCTTCCTGGCCTACTGGGGACAG GGAACTCTGGTCACCGTGTCGAGCGCCTCCGGCGGCGGGGGCTCGGGT GGACGGGCCTCGGGCGGAGGGGGGTCCGACATCGTGATGACCCAGACC CCGCTGAGCTTGCCCGTGACTCCCGGAGAGCCTGCATCCATCTCCTGC CGGTCATCCCAGTCCCTTCTCCACTCCAACGGATACAACTACCTCGAC TGGTACCTCCAGAAGCCGGGACAGAGCCCTCAGCTTCTGATCTACCTG GGGTCAAATAGAGCCTCAGGAGTGCCGGATCGGTTCAGCGGATCTGGT TCGGGAACTGATTTCACTCTGAAGATTTCCCGCGTGGAAGCCGAGGAC GTGGGCGTCTACTACTGTATGCAGGCGCTGCAGACCCCCTATACCTTC GGCCAAGGGACGAAAGTGGAGATCAAGACCACTACCCCAGCACCGAGG CCACCCACCCCGGCTCCTACCATCGCCTCCCAGCCTCTGTCCCTGCGT CCGGAGGCATGTAGACCCGCAGCTGGTGGGGCCGTGCATACCCGGGGT CTTGACTTCGCCTGCGATATCTACATTTGGGCCCCTCTGGCTGGTACT TGCGGGGTCCTGCTGCTTTCACTCGTGATCACTCTTTACTGTAAGCGC GGTCGGAAGAAGCTGCTGTACATCTTTAAGCAACCCTTCATGAGGCCT GTGCAGACTACTCAAGAGGAGGACGGCTGTTCATGCCGGTTCCCAGAG GAGGAGGAAGGCGGCTGCGAACTGCGCGTGAAATTCAGCCGCAGCGCA GATGCTCCAGCCTACAAGCAGGGGCAGAACCAGCTCTACAACGAACTC AATCTTGGTCGGAGAGAGGAGTACGACGTGCTGGACAAGCGGAGAGGA CGGGACCCAGAAATGGGCGGGAAGCCGCGCAGAAAGAATCCCCAAGAG GGCCTGTACAACGAGCTCCAAAAGGATAAGATGGCAGAAGCCTATAGC GAGATTGGTATGAAAGGGGAACGCAGAAGAGGCAAAGGCCACGACGGA CTGTACCAGGGACTCAGCACCGCCACCAAGGACACCTATGACGCTCTT CACATGCAGGCCCTGCCGCCTCGG |
| **Full CAR** | | |

| **139111** | | |
|---|---|---|
| **139111- aa** **ScFv domain** | 985 | EVQLLESGGGLVQPGGSLRLSCAVSGFALSNHGMSWVRRAPGKGLEWV SGIVYSGSTYYAASVKGRFTISRDNSRNTLYLQMNSLRPEDTAIYYCS |
| | | AHGGESDVWGQGTTVTVSSASGGGGSGGRASGGGGSDIVMTQTPLSLS VTPGQPASISCKSSQSLLRNDGKTPLYWYLQKAGQPPQLLIYEVSNRF SGVPDRFSGSGSGTDFTLKISRVEAEDVGAYYCMQNIQFPSFGGGTKL EIK |
| **139111- nt** | 986 | GAAGTGCAATTGTTGGAATCTGGAGGAGGACTTGTGCAGCCTGGAGGA TCACTGAGACTTTCGTGTGCGGTGTCAGGCTTCGCCCTGAGCAACCAC GGCATGAGCTGGGTGCGGAGAGCCCCGGGGAAGGGTCTGGAATGGGTG TCCGGGATCGTCTACTCCGGTTCAACTTACTACGCCGCAAGCGTGAAG GGTCGCTTCACCATTTCCCGCGATAACTCCCGGAACACCCTGTACCTC CAAATGAACTCCCTGCGGCCCGAGGACACCGCCATCTACTACTGTTCC GCGCATGGAGGAGAGTCCGATGTCTGGGGACAGGGCACTACCGTGACC GTGTCGAGCGCCTCGGGGGGAGGAGGCTCCGGCGGTCGCGCCTCCGGG GGGGGTGGCAGCGACATTGTGATGACGCAGACTCCACTCTCGCTGTCC GTGACCCCGGGACAGCCCGCGTCCATCTCGTGCAAGAGCTCCCAGAGC CTGCTGAGGAACGACGGAAAGACTCCTCTGTATTGGTACCTCCAGAAG GCTGGACAGCCCCCGCAACTGCTCATCTACGAAGTGTCAAATCGCTTC TCCGGGGTGCCGGATCGGTTTTCCGGCTCGGGATCGGGCACCGACTTC ACCCTGAAAATCTCCAGGGTCGAGGCCGAGGACGTGGGAGCCTACTAC TGCATGCAAAACATCCAGTTCCCTTCCTTCGGCGGCGGCACAAAGCTG GAGATTAAG |
| **ScFv domain** | | |
| **139111- aa** | 987 | EVQLLESGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSS |
| **VH** | | |
| **139111- aa** | 988 | DIVMTQTPLSLSVTPGQPASISCKSSQSLLRNDGKTPLYWYLQKAGQP PQLLIYEVSNRFSGVPDRFSGSGSGTDFTLKISRVEAEDVGAYYCMQN IQFPSFGGGTKLEIK |
| **VL** | | |
| **139111- aa** | 989 | MALPVTALLLPLALLLHAARPEVQLLESGGGLVQPGGSLRLSCAVSGF ALSNHGMSWVRRAPGKGLEWVSGIVYSGSTYYAASVKGRFTISRDNSR NTLYLQMNSLRPEDTAIYYCSAHGGESDVWGQGTTVTVSSASGGGGSG GRASGGGGSDIVMTQTPLSLSVTPGQPASISCKSSQSLLRNDGKTPLY WYLQKAGQPPQLLIYEVSNRFSGVPDRFSGSGSGTDFTLKISRVEAED VGAYYCMQNIQFPSFGGGTKLEIKTTTPAPRPPTPAPTIASQPLSLRP EACRPAAGGAVHTRGLDFACDIYIWAPLAGTCGVLLLSLVITLYCKRG RKKLLYIFKQPFMRPVQTTQEEDGCSCRFPEEEEGGCELRVKFSRSAD APAYKQGQNQLYNELNLGRREEYDVLDKRRGRDPEMGGKPRRKNPQEG LYNELQKDKMAEAYSEIGMKGERRRGKGHDGLYQGLSTATKDTYDALH MQALPPR |
| **Full CAR** | | |
| **139111- nt** | 990 | ATGGCCCTCCCTGTCACCGCCCTGCTGCTTCCGCTGGCTCTTCTGCTC CACGCCGCTCGGCCCGAAGTGCAATTGTTGGAATCTGGAGGAGGACTT GTGCAGCCTGGAGGATCACTGAGACTTTCGTGTGCGGTGTCAGGCTTC GCCCTGAGCAACCACGGCATGAGCTGGGTGCGGAGAGCCCCGGGGAAG GGTCTGGAATGGGTGTCCGGGATCGTCTACTCCGGTTCAACTTACTAC GCCGCAAGCGTGAAGGGTCGCTTCACCATTTCCCGCGATAACTCCCGG |
| **Full CAR** | | |
| | | AACACCCTGTACCTCCAAATGAACTCCCTGCGGCCCGAGGACACCGCC ATCTACTACTGTTCCGCGCATGGAGGAGAGTCCGATGTCTGGGGACAG GGCACTACCGTGACCGTGTCGAGCGCCTCGGGGGGAGGAGGCTCCGGC GGTCGCGCCTCCGGGGGGGGTGGCAGCGACATTGTGATGACGCAGACT CCACTCTCGCTGTCCGTGACCCCGGGACAGCCCGCGTCCATCTCGTGC AAGAGCTCCCAGAGCCTGCTGAGGAACGACGGAAAGACTCCTCTGTAT TGGTACCTCCAGAAGGCTGGACAGCCCCCGCAACTGCTCATCTACGAA GTGTCAAATCGCTTCTCCGGGGTGCCGGATCGGTTTTCCGGCTCGGGA TCGGGCACCGACTTCACCCTGAAAATCTCCAGGGTCGAGGCCGAGGAC GTGGGAGCCTACTACTGCATGCAAAACATCCAGTTCCCTTCCTTCGGC GGCGGCACAAAGCTGGAGATTAAGACCACTACCCCAGCACCGAGGCCA CCCACCCCGGCTCCTACCATCGCCTCCCAGCCTCTGTCCCTGCGTCCG GAGGCATGTAGACCCGCAGCTGGTGGGGCCGTGCATACCCGGGGTCTT GACTTCGCCTGCGATATCTACATTTGGGCCCCTCTGGCTGGTACTTGC GGGGTCCTGCTGCTTTCACTCGTGATCACTCTTTACTGTAAGCGCGGT CGGAAGAAGCTGCTGTACATCTTTAAGCAACCCTTCATGAGGCCTGTG CAGACTACTCAAGAGGAGGACGGCTGTTCATGCCGGTTCCCAGAGGAG GAGGAAGGCGGCTGCGAACTGCGCGTGAAATTCAGCCGCAGCGCAGAT GCTCCAGCCTACAAGCAGGGGCAGAACCAGCTCTACAACGAACTCAAT CTTGGTCGGAGAGAGGAGTACGACGTGCTGGACAAGCGGAGAGGACGG GACCCAGAAATGGGCGGGAAGCCGCGCAGAAAGAATCCCCAAGAGGGC CTGTACAACGAGCTCCAAAAGGATAAGATGGCAGAAGCCTATAGCGAG ATTGGTATGAAAGGGGAACGCAGAAGAGGCAAAGGCCACGACGGACTG TACCAGGGACTCAGCACCGCCACCAAGGACACCTATGACGCTCTTCAC ATGCAGGCCCTGCCGCCTCGG |

| **139100** | | |
|---|---|---|
| **139100- aa** | 991 | QVQLVQSGAEVRKTGASVKVSCKASGYIFDNFGINWVRQAPGQGLEWM GWINPKNNNTNYAQKFQGRVTITADESTNTAYMEVSSLRSEDTAVYYC ARGPYYYQSYMDVWGQGTMVTVSSASGGGGSGGRASGGGGSDIVMTQT PLSLPVTPGEPASISCRSSQSLLHSNGYNYLNWYLQKPGQSPQLLIYL GSKRASGVPDRFSGSGSGTDFTLHITRVGAEDVGVYYCMQALQTPYTF GQGTKLEIK |
| **ScFv domain** | | |
| **139100- nt** | 992 | CAAGTCCAACTCGTCCAGTCCGGCGCAGAAGTCAGAAAAACCGGTGCT AGCGTGAAAGTGTCCTGCAAGGCCTCCGGCTACATTTTCGATAACTTC GGAATCAACTGGGTCAGACAGGCCCCGGGCCAGGGGCTGGAATGGATG GGATGGATCAACCCCAAGAACAACAACACCAACTACGCACAGAAGTTC CAGGGCCGCGTGACTATCACCGCCGATGAATCGACCAATACCGCCTAC ATGGAGGTGTCCTCCCTGCGGTCGGAGGACACTGCCGTGTATTACTGC GCGAGGGGCCCATACTACTACCAAAGCTACATGGACGTCTGGGGACAG GGAACCATGGTGACCGTGTCATCCGCCTCCGGTGGTGGAGGCTCCGGG GGGCGGGCTTCAGGAGGCGGAGGAAGCGATATTGTGATGACCCAGACT CCGCTTAGCCTGCCCGTGACTCCTGGAGAACCGGCCTCCATTTCCTGC CGGTCCTCGCAATCACTCCTGCATTCCAACGGTTACAACTACCTGAAT TGGTACCTCCAGAAGCCTGGCCAGTCGCCCCAGTTGCTGATCTATCTG GGCTCGAAGCGCGCCTCCGGGGTGCCTGACCGGTTTAGCGGATCTGGG |
| **ScFv domain** | | |
| | | AGCGGCACGGACTTCACTCTCCACATCACCCGCGTGGGAGCGGAGGAC GTGGGAGTGTACTACTGTATGCAGGCGCTGCAGACTCCGTACACATTC GGACAGGGCACCAAGCTGGAGATCAAG |
| **139100- aa** | 993 | QVQLVQSGAEVRKTGASVKVSCKASGYIFDNFGINWVRQAPGQGLEWM GWINPKNNNTNYAQKFQGRVTITADESTNTAYMEVSSLRSEDTAVYYC ARGPYYYQSYMDVWGQGTMVTVSS |
| **VH** | | |
| **139100- aa** | 994 | DIVMTQTPLSLPVTPGEPASISCRSSQSLLHSNGYNYLNWYLQKPGQS PQLLIYLGSKRASGVPDRFSGSGSGTDFTLHITRVGAEDVGVYYCMQA LQTPYTFGQGTKLEIK |
| **VL** | | |
| **139100- aa** | 995 | MALPVTALLLPLALLLHAARPQVQLVQSGAEVRKTGASVKVSCKASGY IFDNFGINWVRQAPGQGLEWMGWINPKNNNTNYAQKFQGRVTITADES TNTAYMEVSSLRSEDTAVYYCARGPYYYQSYMDVWGQGTMVTVSSASG GGGSGGRASGGGGSDIVMTQTPLSLPVTPGEPASISCRSSQSLLHSNG YNYLNWYLQKPGQSPQLLIYLGSKRASGVPDRFSGSGSGTDFTLHITR VGAEDVGVYYCMQALQTPYTFGQGTKLEIKTTTPAPRPPTPAPTIASQ PLSLRPEACRPAAGGAVHTRGLDFACDIYIWAPLAGTCGVLLLSLVIT LYCKRGRKKLLYIFKQPFMRPVQTTQEEDGCSCRFPEEEEGGCELRVK FSRSADAPAYKQGQNQLYNELNLGRREEYDVLDKRRGRDPEMGGKPRR KNPQEGLYNELQKDKMAEAYSEIGMKGERRRGKGHDGLYQGLSTATKD TYDALHMQALPPR |
| **Full CAR** | | |
| **139100- nt** | 996 | ATGGCCCTCCCTGTCACCGCCCTGCTGCTTCCGCTGGCTCTTCTGCTC CACGCCGCTCGGCCCCAAGTCCAACTCGTCCAGTCCGGCGCAGAAGTC AGAAAAACCGGTGCTAGCGTGAAAGTGTCCTGCAAGGCCTCCGGCTAC ATTTTCGATAACTTCGGAATCAACTGGGTCAGACAGGCCCCGGGCCAG GGGCTGGAATGGATGGGATGGATCAACCCCAAGAACAACAACACCAAC TACGCACAGAAGTTCCAGGGCCGCGTGACTATCACCGCCGATGAATCG ACCAATACCGCCTACATGGAGGTGTCCTCCCTGCGGTCGGAGGACACT GCCGTGTATTACTGCGCGAGGGGCCCATACTACTACCAAAGCTACATG GACGTCTGGGGACAGGGAACCATGGTGACCGTGTCATCCGCCTCCGGT GGTGGAGGCTCCGGGGGGCGGGCTTCAGGAGGCGGAGGAAGCGATATT GTGATGACCCAGACTCCGCTTAGCCTGCCCGTGACTCCTGGAGAACCG GCCTCCATTTCCTGCCGGTCCTCGCAATCACTCCTGCATTCCAACGGT TACAACTACCTGAATTGGTACCTCCAGAAGCCTGGCCAGTCGCCCCAG TTGCTGATCTATCTGGGCTCGAAGCGCGCCTCCGGGGTGCCTGACCGG TTTAGCGGATCTGGGAGCGGCACGGACTTCACTCTCCACATCACCCGC GTGGGAGCGGAGGACGTGGGAGTGTACTACTGTATGCAGGCGCTGCAG ACTCCGTACACATTCGGACAGGGCACCAAGCTGGAGATCAAGACCACT ACCCCAGCACCGAGGCCACCCACCCCGGCTCCTACCATCGCCTCCCAG CCTCTGTCCCTGCGTCCGGAGGCATGTAGACCCGCAGCTGGTGGGGCC GTGCATACCCGGGGTCTTGACTTCGCCTGCGATATCTACATTTGGGCC CCTCTGGCTGGTACTTGCGGGGTCCTGCTGCTTTCACTCGTGATCACT CTTTACTGTAAGCGCGGTCGGAAGAAGCTGCTGTACATCTTTAAGCAA CCCTTCATGAGGCCTGTGCAGACTACTCAAGAGGAGGACGGCTGTTCA TGCCGGTTCCCAGAGGAGGAGGAAGGCGGCTGCGAACTGCGCGTGAAA |
| **Full CAR** | | |
| | | TTCAGCCGCAGCGCAGATGCTCCAGCCTACAAGCAGGGGCAGAACCAG CTCTACAACGAACTCAATCTTGGTCGGAGAGAGGAGTACGACGTGCTG GACAAGCGGAGAGGACGGGACCCAGAAATGGGCGGGAAGCCGCGCAGA AAGAATCCCCAAGAGGGCCTGTACAACGAGCTCCAAAAGGATAAGATG GCAGAAGCCTATAGCGAGATTGGTATGAAAGGGGAACGCAGAAGAGGC AAAGGCCACGACGGACTGTACCAGGGACTCAGCACCGCCACCAAGGAC ACCTATGACGCTCTTCACATGCAGGCCCTGCCGCCTCGG |

| **139101** | | |
|---|---|---|
| **139101- aa** | 997 | QVQLQESGGGLVQPGGSLRLSCAASGFTFSSDAMTWVRQAPGKGLEWV SVISGSGGTTYYADSVKGRFTISRDNSKNTLYLQMNSLRAEDTAVYYC AKLDSSGYYYARGPRYWGQGTLVTVSSASGGGGSGGRASGGGGSDIQL TQSPSSLSASVGDRVTITCRASQSISSYLNWYQQKPGKAPKLLIYGAS TLASGVPARFSGSGSGTHFTLTINSLQSEDSATYYCQQSYKRASFGQG TKVEIK |
| **ScFv domain** | | |
| **139101- nt** | 998 | CAAGTGCAACTTCAAGAATCAGGCGGAGGACTCGTGCAGCCCGGAGGA TCATTGCGGCTCTCGTGCGCCGCCTCGGGCTTCACCTTCTCGAGCGAC GCCATGACCTGGGTCCGCCAGGCCCCGGGGAAGGGGCTGGAATGGGTG TCTGTGATTTCCGGCTCCGGGGGAACTACGTACTACGCCGATTCCGTG AAAGGTCGCTTCACTATCTCCCGGGACAACAGCAAGAACACCCTTTAT CTGCAAATGAATTCCCTCCGCGCCGAGGACACCGCCGTGTACTACTGC GCCAAGCTGGACTCCTCGGGCTACTACTATGCCCGGGGTCCGAGATAC TGGGGACAGGGAACCCTCGTGACCGTGTCCTCCGCGTCCGGCGGAGGA GGGTCGGGAGGGCGGGCCTCCGGCGGCGGCGGTTCGGACATCCAGCTG ACCCAGTCCCCATCCTCACTGAGCGCAAGCGTGGGCGACAGAGTCACC ATTACATGCAGGGCGTCCCAGAGCATCAGCTCCTACCTGAACTGGTAC CAACAGAAGCCTGGAAAGGCTCCTAAGCTGTTGATCTACGGGGCTTCG ACCCTGGCATCCGGGGTGCCCGCGAGGTTTAGCGGAAGCGGTAGCGGC ACTCACTTCACTCTGACCATTAACAGCCTCCAGTCCGAGGATTCAGCC ACTTACTACTGTCAGCAGTCCTACAAGCGGGCCAGCTTCGGACAGGGC ACTAAGGTCGAGATCAAG |
| **ScFv domain** | | |
| **139101- aa** | 999 | QVQLQESGGGLVQPGGSLRLSCAASGFTFSSDAMTWVRQAPGKGLEWV SVISGSGGTTYYADSVKGRFTISRDNSKNTLYLQMNSLRAEDTAVYYC AKLDSSGYYYARGPRYWGQGTLVTVSS |
| **VH** | | |
| **139101- aa** | 1000 | DIQLTQSPSSLSASVGDRVTITCRASQSISSYLNWYQQKPGKAPKLLI YGASTLASGVPARFSGSGSGTHFTLTINSLQSEDSATYYCQQSYKRAS FGQGTKVEIK |
| **VL** | | |
| **139101- aa** | 1001 | MALPVTALLLPLALLLHAARPQVQLQESGGGLVQPGGSLRLSCAASGF TFSSDAMTWVRQAPGKGLEWVSVISGSGGTTYYADSVKGRFTISRDNS KNTLYLQMNSLRAEDTAVYYCAKLDSSGYYYARGPRYWGQGTLVTVSS ASGGGGSGGRASGGGGSDIQLTQSPSSLSASVGDRVTITCRASQSISS YLNWYQQKPGKAPKLLIYGASTLASGVPARFSGSGSGTHFTLTINSLQ SEDSATYYCQQSYKRASFGQGTKVEIKTTTPAPRPPTPAPTIASQPLS |
| **Full CAR** | | |
| | | LRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNP QEGLYNELQKDKMAEAYSEIGMKGERRRGKGHDGLYQGLSTATKDTYD ALHMQALPPR |
| **139101- nt** | 1002 | ATGGCCCTCCCTGTCACCGCCCTGCTGCTTCCGCTGGCTCTTCTGCTC CACGCCGCTCGGCCCCAAGTGCAACTTCAAGAATCAGGCGGAGGACTC GTGCAGCCCGGAGGATCATTGCGGCTCTCGTGCGCCGCCTCGGGCTTC ACCTTCTCGAGCGACGCCATGACCTGGGTCCGCCAGGCCCCGGGGAAG GGGCTGGAATGGGTGTCTGTGATTTCCGGCTCCGGGGGAACTACGTAC TACGCCGATTCCGTGAAAGGTCGCTTCACTATCTCCCGGGACAACAGC AAGAACACCCTTTATCTGCAAATGAATTCCCTCCGCGCCGAGGACACC GCCGTGTACTACTGCGCCAAGCTGGACTCCTCGGGCTACTACTATGCC CGGGGTCCGAGATACTGGGGACAGGGAACCCTCGTGACCGTGTCCTCC GCGTCCGGCGGAGGAGGGTCGGGAGGGCGGGCCTCCGGCGGCGGCGGT TCGGACATCCAGCTGACCCAGTCCCCATCCTCACTGAGCGCAAGCGTG GGCGACAGAGTCACCATTACATGCAGGGCGTCCCAGAGCATCAGCTCC TACCTGAACTGGTACCAACAGAAGCCTGGAAAGGCTCCTAAGCTGTTG ATCTACGGGGCTTCGACCCTGGCATCCGGGGTGCCCGCGAGGTTTAGC GGAAGCGGTAGCGGCACTCACTTCACTCTGACCATTAACAGCCTCCAG TCCGAGGATTCAGCCACTTACTACTGTCAGCAGTCCTACAAGCGGGCC AGCTTCGGACAGGGCACTAAGGTCGAGATCAAGACCACTACCCCAGCA CCGAGGCCACCCACCCCGGCTCCTACCATCGCCTCCCAGCCTCTGTCC CTGCGTCCGGAGGCATGTAGACCCGCAGCTGGTGGGGCCGTGCATACC CGGGGTCTTGACTTCGCCTGCGATATCTACATTTGGGCCCCTCTGGCT GGTACTTGCGGGGTCCTGCTGCTTTCACTCGTGATCACTCTTTACTGT AAGCGCGGTCGGAAGAAGCTGCTGTACATCTTTAAGCAACCCTTCATG AGGCCTGTGCAGACTACTCAAGAGGAGGACGGCTGTTCATGCCGGTTC CCAGAGGAGGAGGAAGGCGGCTGCGAACTGCGCGTGAAATTCAGCCGC AGCGCAGATGCTCCAGCCTACAAGCAGGGGCAGAACCAGCTCTACAAC GAACTCAATCTTGGTCGGAGAGAGGAGTACGACGTGCTGGACAAGCGG AGAGGACGGGACCCAGAAATGGGCGGGAAGCCGCGCAGAAAGAATCCC CAAGAGGGCCTGTACAACGAGCTCCAAAAGGATAAGATGGCAGAAGCC TATAGCGAGATTGGTATGAAAGGGGAACGCAGAAGAGGCAAAGGCCAC GACGGACTGTACCAGGGACTCAGCACCGCCACCAAGGACACCTATGAC GCTCTTCACATGCAGGCCCTGCCGCCTCGG |
| **Full CAR** | | |

| **139102** | | |
|---|---|---|
| **139102- aa** | 1003 | QVQLVQSGAEVKKPGASVKVSCKASGYTFSNYGITWVRQAPGQGLEWM GWISAYNGNTNYAQKFQGRVTMTRNTSISTAYMELSSLRSEDTAVYYC ARGPYYYYMDVWGKGTMVTVSSASGGGGSGGRASGGGGSEIVMTQSPL SLPVTPGEPASISCRSSQSLLYSNGYNYVDWYLQKPGQSPQLLIYLGS NRASGVPDRFSGSGSGTDFKLQISRVEAEDVGIYYCMQGRQFPYSFGQ GTKVEIK |
| **ScFv domain** | | |
| **139102- nt** | 1004 | CAAGTCCAACTGGTCCAGAGCGGTGCAGAAGTGAAGAAGCCCGGAGCG AGCGTGAAAGTGTCCTGCAAGGCTTCCGGGTACACCTTCTCCAACTAC GGCATCACTTGGGTGCGCCAGGCCCCGGGACAGGGCCTGGAATGGATG GGGTGGATTTCCGCGTACAACGGCAATACGAACTACGCTCAGAAGTTC CAGGGTAGAGTGACCATGACTAGGAACACCTCCATTTCCACCGCCTAC ATGGAACTGTCCTCCCTGCGGAGCGAGGACACCGCCGTGTACTATTGC GCCCGGGGACCATACTACTACTACATGGATGTCTGGGGGAAGGGGACT ATGGTCACCGTGTCATCCGCCTCGGGAGGCGGCGGATCAGGAGGACGC GCCTCTGGTGGTGGAGGATCGGAGATCGTGATGACCCAGAGCCCTCTC TCCTTGCCCGTGACTCCTGGGGAGCCCGCATCCATTTCATGCCGGAGC TCCCAGTCACTTCTCTACTCCAACGGCTATAACTACGTGGATTGGTAC CTCCAAAAGCCGGGCCAGAGCCCGCAGCTGCTGATCTACCTGGGCTCG AACAGGGCCAGCGGAGTGCCTGACCGGTTCTCCGGGTCGGGAAGCGGG ACCGACTTCAAGCTGCAAATCTCGAGAGTGGAGGCCGAGGACGTGGGA ATCTACTACTGTATGCAGGGCCGCCAGTTTCCGTACTCGTTCGGACAG GGCACCAAAGTGGAAATCAAG |
| **ScFv domain** | | |
| **139102- aa** | 1005 | QVQLVQSGAEVKKPGASVKVSCKASGYTFSNYGITWVRQAPGQGLEWM GWISAYNGNTNYAQKFQGRVTMTRNTSISTAYMELSSLRSEDTAVYYC ARGPYYYYMDVWGKGTMVTVSS |
| **VH** | | |
| **139102- aa** | 1006 | EIVMTQSPLSLPVTPGEPASISCRSSQSLLYSNGYNYVDWYLQKPGQS PQLLIYLGSNRASGVPDRFSGSGSGTDFKLQISRVEAEDVGIYYCMQG RQFPYSFGQGTKVEIK |
| **VL** | | |
| **139102- aa** | 1007 | MALPVTALLLPLALLLHAARPQVQLVQSGAEVKKPGASVKVSCKASGY TFSNYGITWVRQAPGQGLEWMGWISAYNGNTNYAQKFQGRVTMTRNTS ISTAYMELSSLRSEDTAVYYCARGPYYYYMDVWGKGTMVTVSSASGGG GSGGRASGGGGSEIVMTQSPLSLPVTPGEPASISCRSSQSLLYSNGYN YVDWYLQKPGQSPQLLIYLGSNRASGVPDRFSGSGSGTDFKLQISRVE AEDVGIYYCMQGRQFPYSFGQGTKVEIKTTTPAPRPPTPAPTIASQPL SLRPEACRPAAGGAVHTRGLDFACDIYIWAPLAGTCGVLLLSLVITLY CKRGRKKLLYIFKQPFMRPVQTTQEEDGCSCRFPEEEEGGCELRVKFS RSADAPAYKQGQNQLYNELNLGRREEYDVLDKRRGRDPEMGGKPRRKN PQEGLYNELQKDKMAEAYSEIGMKGERRRGKGHDGLYQGLSTATKDTY DALHMQALPPR |
| **Full CAR** | | |
| **139102- nt** | 1008 | ATGGCCCTCCCTGTCACCGCCCTGCTGCTTCCGCTGGCTCTTCTGCTC CACGCCGCTCGGCCCCAAGTCCAACTGGTCCAGAGCGGTGCAGAAGTG AAGAAGCCCGGAGCGAGCGTGAAAGTGTCCTGCAAGGCTTCCGGGTAC ACCTTCTCCAACTACGGCATCACTTGGGTGCGCCAGGCCCCGGGACAG GGCCTGGAATGGATGGGGTGGATTTCCGCGTACAACGGCAATACGAAC TACGCTCAGAAGTTCCAGGGTAGAGTGACCATGACTAGGAACACCTCC ATTTCCACCGCCTACATGGAACTGTCCTCCCTGCGGAGCGAGGACACC GCCGTGTACTATTGCGCCCGGGGACCATACTACTACTACATGGATGTC TGGGGGAAGGGGACTATGGTCACCGTGTCATCCGCCTCGGGAGGCGGC GGATCAGGAGGACGCGCCTCTGGTGGTGGAGGATCGGAGATCGTGATG ACCCAGAGCCCTCTCTCCTTGCCCGTGACTCCTGGGGAGCCCGCATCC |
| **Full CAR** | | |
| | | ATTTCATGCCGGAGCTCCCAGTCACTTCTCTACTCCAACGGCTATAAC TACGTGGATTGGTACCTCCAAAAGCCGGGCCAGAGCCCGCAGCTGCTG ATCTACCTGGGCTCGAACAGGGCCAGCGGAGTGCCTGACCGGTTCTCC GGGTCGGGAAGCGGGACCGACTTCAAGCTGCAAATCTCGAGAGTGGAG GCCGAGGACGTGGGAATCTACTACTGTATGCAGGGCCGCCAGTTTCCG TACTCGTTCGGACAGGGCACCAAAGTGGAAATCAAGACCACTACCCCA GCACCGAGGCCACCCACCCCGGCTCCTACCATCGCCTCCCAGCCTCTG TCCCTGCGTCCGGAGGCATGTAGACCCGCAGCTGGTGGGGCCGTGCAT ACCCGGGGTCTTGACTTCGCCTGCGATATCTACATTTGGGCCCCTCTG GCTGGTACTTGCGGGGTCCTGCTGCTTTCACTCGTGATCACTCTTTAC TGTAAGCGCGGTCGGAAGAAGCTGCTGTACATCTTTAAGCAACCCTTC ATGAGGCCTGTGCAGACTACTCAAGAGGAGGACGGCTGTTCATGCCGG TTCCCAGAGGAGGAGGAAGGCGGCTGCGAACTGCGCGTGAAATTCAGC CGCAGCGCAGATGCTCCAGCCTACAAGCAGGGGCAGAACCAGCTCTAC AACGAACTCAATCTTGGTCGGAGAGAGGAGTACGACGTGCTGGACAAG CGGAGAGGACGGGACCCAGAAATGGGCGGGAAGCCGCGCAGAAAGAAT CCCCAAGAGGGCCTGTACAACGAGCTCCAAAAGGATAAGATGGCAGAA GCCTATAGCGAGATTGGTATGAAAGGGGAACGCAGAAGAGGCAAAGGC CACGACGGACTGTACCAGGGACTCAGCACCGCCACCAAGGACACCTAT GACGCTCTTCACATGCAGGCCCTGCCGCCTCGG |

| **139104** | | |
|---|---|---|
| **139104- aa** | 1009 | EVQLLETGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSSASGGGGSGGRASGGGGSEIVLTQSPATLS VSPGESATLSCRASQSVSSNLAWYQQKPGQAPRLLIYGASTRASGIPD RFSGSGSGTDFTLTISSLQAEDVAVYYCQQYGSSLTFGGGTKVEIK |
| **ScFv domain** | | |
| **139104- nt** | 1010 | GAAGTGCAATTGCTCGAAACTGGAGGAGGTCTGGTGCAACCTGGAGGA TCACTTCGCCTGTCCTGCGCCGTGTCGGGCTTTGCCCTGTCCAACCAT GGAATGAGCTGGGTCCGCCGCGCGCCGGGGAAGGGCCTCGAATGGGTG TCCGGCATCGTCTACTCCGGCTCCACCTACTACGCCGCGTCCGTGAAG GGCCGGTTCACGATTTCACGGGACAACTCGCGGAACACCCTGTACCTC CAAATGAATTCCCTTCGGCCGGAGGATACTGCCATCTACTACTGCTCC GCCCACGGTGGCGAATCCGACGTCTGGGGCCAGGGAACCACCGTGACC GTGTCCAGCGCGTCCGGGGGAGGAGGAAGCGGGGGTAGAGCATCGGGT GGAGGCGGATCAGAGATCGTGCTGACCCAGTCCCCCGCCACCTTGAGC GTGTCACCAGGAGAGTCCGCCACCCTGTCATGCCGCGCCAGCCAGTCC GTGTCCTCCAACCTGGCTTGGTACCAGCAGAAGCCGGGGCAGGCCCCT AGACTCCTGATCTATGGGGCGTCGACCCGGGCATCTGGAATTCCCGAT AGGTTCAGCGGATCGGGCTCGGGCACTGACTTCACTCTGACCATCTCC TCGCTGCAAGCCGAGGACGTGGCTGTGTACTACTGTCAGCAGTACGGA AGCTCCCTGACTTTCGGTGGCGGGACCAAAGTCGAGATTAAG |
| **ScFv domain** | | |
| **139104- aa VH** | 1011 | EVQLLETGGGLVQPGGSLRLSCAVSGFALSNHGMSWVRRAPGKGLEWV SGIVYSGSTYYAASVKGRFTISRDNSRNTLYLQMNSLRPEDTAIYYCS |
| | | AHGGESDVWGQGTTVTVSS |
| **139104- aa** | 1012 | EIVLTQSPATLSVSPGESATLSCRASQSVSSNLAWYQQKPGQAPRLLI YGASTRASGIPDRFSGSGSGTDFTLTISSLQAEDVAVYYCQQYGSSLT FGGGTKVEIK |
| **VL** | | |
| **139104- aa** | 1013 | MALPVTALLLPLALLLHAARPEVQLLETGGGLVQPGGSLRLSCAVSGF ALSNHGMSWVRRAPGKGLEWVSGIVYSGSTYYAASVKGRFTISRDNSR NTLYLQMNSLRPEDTAIYYCSAHGGESDVWGQGTTVTVSSASGGGGSG GRASGGGGSEIVLTQSPATLSVSPGESATLSCRASQSVSSNLAWYQQK PGQAPRLLIYGASTRASGIPDRFSGSGSGTDFTLTISSLQAEDVAVYY CQQYGSSLTFGGGTKVEIKTTTPAPRPPTPAPTIASQPLSLRPEACRP AAGGAVHTRGLDFACDIYIWAPLAGTCGVLLLSLVITLYCKRGRKKLL YIFKQPFMRPVQTTQEEDGCSCRFPEEEEGGCELRVKFSRSADAPAYK QGQNQLYNELNLGRREEYDVLDKRRGRDPEMGGKPRRKNPQEGLYNEL QKDKMAEAYSEIGMKGERRRGKGHDGLYQGLSTATKDTYDALHMQALP PR |
| **Full CAR** | | |
| **139104- nt** | 1014 | ATGGCCCTCCCTGTCACCGCCCTGCTGCTTCCGCTGGCTCTTCTGCTC CACGCCGCTCGGCCCGAAGTGCAATTGCTCGAAACTGGAGGAGGTCTG GTGCAACCTGGAGGATCACTTCGCCTGTCCTGCGCCGTGTCGGGCTTT GCCCTGTCCAACCATGGAATGAGCTGGGTCCGCCGCGCGCCGGGGAAG GGCCTCGAATGGGTGTCCGGCATCGTCTACTCCGGCTCCACCTACTAC GCCGCGTCCGTGAAGGGCCGGTTCACGATTTCACGGGACAACTCGCGG AACACCCTGTACCTCCAAATGAATTCCCTTCGGCCGGAGGATACTGCC ATCTACTACTGCTCCGCCCACGGTGGCGAATCCGACGTCTGGGGCCAG GGAACCACCGTGACCGTGTCCAGCGCGTCCGGGGGAGGAGGAAGCGGG GGTAGAGCATCGGGTGGAGGCGGATCAGAGATCGTGCTGACCCAGTCC CCCGCCACCTTGAGCGTGTCACCAGGAGAGTCCGCCACCCTGTCATGC CGCGCCAGCCAGTCCGTGTCCTCCAACCTGGCTTGGTACCAGCAGAAG CCGGGGCAGGCCCCTAGACTCCTGATCTATGGGGCGTCGACCCGGGCA TCTGGAATTCCCGATAGGTTCAGCGGATCGGGCTCGGGCACTGACTTC ACTCTGACCATCTCCTCGCTGCAAGCCGAGGACGTGGCTGTGTACTAC TGTCAGCAGTACGGAAGCTCCCTGACTTTCGGTGGCGGGACCAAAGTC GAGATTAAGACCACTACCCCAGCACCGAGGCCACCCACCCCGGCTCCT ACCATCGCCTCCCAGCCTCTGTCCCTGCGTCCGGAGGCATGTAGACCC GCAGCTGGTGGGGCCGTGCATACCCGGGGTCTTGACTTCGCCTGCGAT ATCTACATTTGGGCCCCTCTGGCTGGTACTTGCGGGGTCCTGCTGCTT TCACTCGTGATCACTCTTTACTGTAAGCGCGGTCGGAAGAAGCTGCTG TACATCTTTAAGCAACCCTTCATGAGGCCTGTGCAGACTACTCAAGAG GAGGACGGCTGTTCATGCCGGTTCCCAGAGGAGGAGGAAGGCGGCTGC GAACTGCGCGTGAAATTCAGCCGCAGCGCAGATGCTCCAGCCTACAAG CAGGGGCAGAACCAGCTCTACAACGAACTCAATCTTGGTCGGAGAGAG GAGTACGACGTGCTGGACAAGCGGAGAGGACGGGACCCAGAAATGGGC GGGAAGCCGCGCAGAAAGAATCCCCAAGAGGGCCTGTACAACGAGCTC CAAAAGGATAAGATGGCAGAAGCCTATAGCGAGATTGGTATGAAAGGG GAACGCAGAAGAGGCAAAGGCCACGACGGACTGTACCAGGGACTCAGC ACCGCCACCAAGGACACCTATGACGCTCTTCACATGCAGGCCCTGCCG |
| **Full CAR** | | |
| | | CCTCGG |

| **139106** | | |
|---|---|---|
| **139106- aa** | 1015 | EVQLVETGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSSASGGGGSGGRASGGGGSEIVMTQSPATLS VSPGERATLSCRASQSVSSKLAWYQQKPGQAPRLLMYGASIRATGIPD RFSGSGSGTEFTLTISSLEPEDFAVYYCQQYGSSSWTFGQGTKVEIK |
| **ScFv domain** | | |
| **139106- nt** | 1016 | GAAGTGCAATTGGTGGAAACTGGAGGAGGACTTGTGCAACCTGGAGGA TCATTGAGACTGAGCTGCGCAGTGTCGGGATTCGCCCTGAGCAACCAT GGAATGTCCTGGGTCAGAAGGGCCCCTGGAAAAGGCCTCGAATGGGTG TCAGGGATCGTGTACTCCGGTTCCACTTACTACGCCGCCTCCGTGAAG GGGCGCTTCACTATCTCACGGGATAACTCCCGCAATACCCTGTACCTC CAAATGAACAGCCTGCGGCCGGAGGATACCGCCATCTACTACTGTTCC GCCCACGGTGGAGAGTCTGACGTCTGGGGCCAGGGAACTACCGTGACC GTGTCCTCCGCGTCCGGCGGTGGAGGGAGCGGCGGCCGCGCCAGCGGC GGCGGAGGCTCCGAGATCGTGATGACCCAGAGCCCCGCTACTCTGTCG GTGTCGCCCGGAGAAAGGGCGACCCTGTCCTGCCGGGCGTCGCAGTCC GTGAGCAGCAAGCTGGCTTGGTACCAGCAGAAGCCGGGCCAGGCACCA CGCCTGCTTATGTACGGTGCCTCCATTCGGGCCACCGGAATCCCGGAC CGGTTCTCGGGGTCGGGGTCCGGTACCGAGTTCACACTGACCATTTCC TCGCTCGAGCCCGAGGACTTTGCCGTCTATTACTGCCAGCAGTACGGC TCCTCCTCATGGACGTTCGGCCAGGGGACCAAGGTCGAAATCAAG |
| **ScFv domain** | | |
| **139106-** aa | 1017 | EVQLVETGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSS |
| **VH** | | |
| **139106- aa** | 1018 | EIVMTQSPATLSVSPGERATLSCRASQSVSSKLAWYQQKPGQAPRLLM YGASIRATGIPDRFSGSGSGTEFTLTISSLEPEDFAVYYCQQYGSSSW TFGQGTKVEIK |
| **VL** | | |
| **139106- aa** | 1019 | MALPVTALLLPLALLLHAARPEVQLVETGGGLVQPGGSLRLSCAVSGF ALSNHGMSWVRRAPGKGLEWVSGIVYSGSTYYAASVKGRFTISRDNSR NTLYLQMNSLRPEDTAIYYCSAHGGESDVWGQGTTVTVSSASGGGGSG GRASGGGGSEIVMTQSPATLSVSPGERATLSCRASQSVSSKLAWYQQK PGQAPRLLMYGASIRATGIPDRFSGSGSGTEFTLTISSLEPEDFAVYY CQQYGSSSWTFGQGTKVEIKTTTPAPRPPTPAPTIASQPLSLRPEACR PAAGGAVHTRGLDFACDIYIWAPLAGTCGVLLLSLVITLYCKRGRKKL LYIFKQPFMRPVQTTQEEDGCSCRFPEEEEGGCELRVKFSRSADAPAY KQGQNQLYNELNLGRREEYDVLDKRRGRDPEMGGKPRRKNPQEGLYNE LQKDKMAEAYSEIGMKGERRRGKGHDGLYQGLSTATKDTYDALHMQAL PPR |
| **Full CAR** | | |
| **139106- nt Full CAR** | 1020 | ATGGCCCTCCCTGTCACCGCCCTGCTGCTTCCGCTGGCTCTTCTGCTC CACGCCGCTCGGCCCGAAGTGCAATTGGTGGAAACTGGAGGAGGACTT GTGCAACCTGGAGGATCATTGAGACTGAGCTGCGCAGTGTCGGGATTC |
| | | GCCCTGAGCAACCATGGAATGTCCTGGGTCAGAAGGGCCCCTGGAAAA GGCCTCGAATGGGTGTCAGGGATCGTGTACTCCGGTTCCACTTACTAC GCCGCCTCCGTGAAGGGGCGCTTCACTATCTCACGGGATAACTCCCGC AATACCCTGTACCTCCAAATGAACAGCCTGCGGCCGGAGGATACCGCC ATCTACTACTGTTCCGCCCACGGTGGAGAGTCTGACGTCTGGGGCCAG GGAACTACCGTGACCGTGTCCTCCGCGTCCGGCGGTGGAGGGAGCGGC GGCCGCGCCAGCGGCGGCGGAGGCTCCGAGATCGTGATGACCCAGAGC CCCGCTACTCTGTCGGTGTCGCCCGGAGAAAGGGCGACCCTGTCCTGC CGGGCGTCGCAGTCCGTGAGCAGCAAGCTGGCTTGGTACCAGCAGAAG CCGGGCCAGGCACCACGCCTGCTTATGTACGGTGCCTCCATTCGGGCC ACCGGAATCCCGGACCGGTTCTCGGGGTCGGGGTCCGGTACCGAGTTC ACACTGACCATTTCCTCGCTCGAGCCCGAGGACTTTGCCGTCTATTAC TGCCAGCAGTACGGCTCCTCCTCATGGACGTTCGGCCAGGGGACCAAG GTCGAAATCAAGACCACTACCCCAGCACCGAGGCCACCCACCCCGGCT CCTACCATCGCCTCCCAGCCTCTGTCCCTGCGTCCGGAGGCATGTAGA CCCGCAGCTGGTGGGGCCGTGCATACCCGGGGTCTTGACTTCGCCTGC GATATCTACATTTGGGCCCCTCTGGCTGGTACTTGCGGGGTCCTGCTG CTTTCACTCGTGATCACTCTTTACTGTAAGCGCGGTCGGAAGAAGCTG CTGTACATCTTTAAGCAACCCTTCATGAGGCCTGTGCAGACTACTCAA GAGGAGGACGGCTGTTCATGCCGGTTCCCAGAGGAGGAGGAAGGCGGC TGCGAACTGCGCGTGAAATTCAGCCGCAGCGCAGATGCTCCAGCCTAC AAGCAGGGGCAGAACCAGCTCTACAACGAACTCAATCTTGGTCGGAGA GAGGAGTACGACGTGCTGGACAAGCGGAGAGGACGGGACCCAGAAATG GGCGGGAAGCCGCGCAGAAAGAATCCCCAAGAGGGCCTGTACAACGAG CTCCAAAAGGATAAGATGGCAGAAGCCTATAGCGAGATTGGTATGAAA GGGGAACGCAGAAGAGGCAAAGGCCACGACGGACTGTACCAGGGACTC AGCACCGCCACCAAGGACACCTATGACGCTCTTCACATGCAGGCCCTG CCGCCTCGG |

| **139107** | | |
|---|---|---|
| **139107- aa** | 1021 | EVQLVETGGGVVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSSASGGGGSGGRASGGGGSEIVLTQSPGTLS LSPGERATLSCRASQSVGSTNLAWYQQKPGQAPRLLIYDASNRATGIP DRFSGGGSGTDFTLTISRLEPEDFAVYYCQQYGSSPPWTFGQGTKVEI K |
| **ScFv domain** | | |
| **139107- nt** | 1022 | GAAGTGCAATTGGTGGAGACTGGAGGAGGAGTGGTGCAACCTGGAGGA AGCCTGAGACTGTCATGCGCGGTGTCGGGCTTCGCCCTCTCCAACCAC GGAATGTCCTGGGTCCGCCGGGCCCCTGGGAAAGGACTTGAATGGGTG TCCGGCATCGTGTACTCGGGTTCCACCTACTACGCGGCCTCAGTGAAG GGCCGGTTTACTATTAGCCGCGACAACTCCAGAAACACACTGTACCTC CAAATGAACTCGCTGCGGCCGGAAGATACCGCTATCTACTACTGCTCC GCCCATGGGGGAGAGTCGGACGTCTGGGGACAGGGCACCACTGTCACT GTGTCCAGCGCTTCCGGCGGTGGTGGAAGCGGGGGACGGGCCTCAGGA GGCGGTGGCAGCGAGATTGTGCTGACCCAGTCCCCCGGGACCCTGAGC CTGTCCCCGGGAGAAAGGGCCACCCTCTCCTGTCGGGCATCCCAGTCC |
| **ScFv domain** | | |
| | | GTGGGGTCTACTAACCTTGCATGGTACCAGCAGAAGCCCGGCCAGGCC CCTCGCCTGCTGATCTACGACGCGTCCAATAGAGCCACCGGCATCCCG GATCGCTTCAGCGGAGGCGGATCGGGCACCGACTTCACCCTCACCATT TCAAGGCTGGAACCGGAGGACTTCGCCGTGTACTACTGCCAGCAGTAT GGTTCGTCCCCACCCTGGACGTTCGGCCAGGGGACTAAGGTCGAGATC AAG |
| **139107- aa** | 1023 | EVQLVETGGGVVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSS |
| **VH** | | |
| **139107- aa** | 1024 | EIVLTQSPGTLSLSPGERATLSCRASQSVGSTNLAWYQQKPGQAPRLL IYDASNRATGIPDRFSGGGSGTDFTLTISRLEPEDFAVYYCQQYGSSP PWTFGQGTKVEIK |
| **VL** | | |
| **139107- aa** | 1025 | MALPVTALLLPLALLLHAARPEVQLVETGGGVVQPGGSLRLSCAVSGF ALSNHGMSWVRRAPGKGLEWVSGIVYSGSTYYAASVKGRFTISRDNSR NTLYLQMNSLRPEDTAIYYCSAHGGESDVWGQGTTVTVSSASGGGGSG GRASGGGGSEIVLTQSPGTLSLSPGERATLSCRASQSVGSTNLAWYQQ KPGQAPRLLIYDASNRATGIPDRFSGGGSGTDFTLTISRLEPEDFAVY YCQQYGSSPPWTFGQGTKVEIKTTTPAPRPPTPAPTIASQPLSLRPEA CRPAAGGAVHTRGLDFACDIYIWAPLAGTCGVLLLSLVITLYCKRGRK KLLYIFKQPFMRPVQTTQEEDGCSCRFPEEEEGGCELRVKFSRSADAP AYKQGQNQLYNELNLGRREEYDVLDKRRGRDPEMGGKPRRKNPQEGLY NELQKDKMAEAYSEIGMKGERRRGKGHDGLYQGLSTATKDTYDALHMQ ALPPR |
| **Full CAR** | | |
| **139107- nt** | 1026 | ATGGCCCTCCCTGTCACCGCCCTGCTGCTTCCGCTGGCTCTTCTGCTC CACGCCGCTCGGCCCGAAGTGCAATTGGTGGAGACTGGAGGAGGAGTG GTGCAACCTGGAGGAAGCCTGAGACTGTCATGCGCGGTGTCGGGCTTC GCCCTCTCCAACCACGGAATGTCCTGGGTCCGCCGGGCCCCTGGGAAA GGACTTGAATGGGTGTCCGGCATCGTGTACTCGGGTTCCACCTACTAC GCGGCCTCAGTGAAGGGCCGGTTTACTATTAGCCGCGACAACTCCAGA AACACACTGTACCTCCAAATGAACTCGCTGCGGCCGGAAGATACCGCT ATCTACTACTGCTCCGCCCATGGGGGAGAGTCGGACGTCTGGGGACAG GGCACCACTGTCACTGTGTCCAGCGCTTCCGGCGGTGGTGGAAGCGGG GGACGGGCCTCAGGAGGCGGTGGCAGCGAGATTGTGCTGACCCAGTCC CCCGGGACCCTGAGCCTGTCCCCGGGAGAAAGGGCCACCCTCTCCTGT CGGGCATCCCAGTCCGTGGGGTCTACTAACCTTGCATGGTACCAGCAG AAGCCCGGCCAGGCCCCTCGCCTGCTGATCTACGACGCGTCCAATAGA GCCACCGGCATCCCGGATCGCTTCAGCGGAGGCGGATCGGGCACCGAC TTCACCCTCACCATTTCAAGGCTGGAACCGGAGGACTTCGCCGTGTAC TACTGCCAGCAGTATGGTTCGTCCCCACCCTGGACGTTCGGCCAGGGG ACTAAGGTCGAGATCAAGACCACTACCCCAGCACCGAGGCCACCCACC CCGGCTCCTACCATCGCCTCCCAGCCTCTGTCCCTGCGTCCGGAGGCA TGTAGACCCGCAGCTGGTGGGGCCGTGCATACCCGGGGTCTTGACTTC GCCTGCGATATCTACATTTGGGCCCCTCTGGCTGGTACTTGCGGGGTC CTGCTGCTTTCACTCGTGATCACTCTTTACTGTAAGCGCGGTCGGAAG |
| **Full CAR** | | |
| | | AAGCTGCTGTACATCTTTAAGCAACCCTTCATGAGGCCTGTGCAGACT ACTCAAGAGGAGGACGGCTGTTCATGCCGGTTCCCAGAGGAGGAGGAA GGCGGCTGCGAACTGCGCGTGAAATTCAGCCGCAGCGCAGATGCTCCA GCCTACAAGCAGGGGCAGAACCAGCTCTACAACGAACTCAATCTTGGT CGGAGAGAGGAGTACGACGTGCTGGACAAGCGGAGAGGACGGGACCCA GAAATGGGCGGGAAGCCGCGCAGAAAGAATCCCCAAGAGGGCCTGTAC AACGAGCTCCAAAAGGATAAGATGGCAGAAGCCTATAGCGAGATTGGT ATGAAAGGGGAACGCAGAAGAGGCAAAGGCCACGACGGACTGTACCAG GGACTCAGCACCGCCACCAAGGACACCTATGACGCTCTTCACATGCAG GCCCTGCCGCCTCGG |

| **139108** | | |
|---|---|---|
| **139108- aa** | 1027 | QVQLVESGGGLVKPGGSLRLSCAASGFTFSDYYMSWIRQAPGKGLEWV SYISSSGSTIYYADSVKGRFTISRDNAKNSLYLQMNSLRAEDTAVYYC ARESGDGMDVWGQGTTVTVSSASGGGGSGGRASGGGGSDIQMTQSPSS LSASVGDRVTITCRASQSISSYLNWYQQKPGKAPKLLIYAASSLQSGV PSRFSGSGSGTDFTLTISSLQPEDFATYYCQQSYTLAFGQGTKVDIK |
| **ScFv domain** | | |
| **139108- nt** | 1028 | CAAGTGCAACTCGTGGAATCTGGTGGAGGACTCGTGAAACCTGGAGGA TCATTGAGACTGTCATGCGCGGCCTCGGGATTCACGTTCTCCGATTAC TACATGAGCTGGATTCGCCAGGCTCCGGGGAAGGGACTGGAATGGGTG TCCTACATTTCCTCATCCGGCTCCACCATCTACTACGCGGACTCCGTG AAGGGGAGATTCACCATTAGCCGCGATAACGCCAAGAACAGCCTGTAC CTTCAGATGAACTCCCTGCGGGCTGAAGATACTGCCGTCTACTACTGC GCAAGGGAGAGCGGAGATGGGATGGACGTCTGGGGACAGGGTACCACT GTGACCGTGTCGTCGGCCTCCGGCGGAGGGGGTTCGGGTGGAAGGGCC AGCGGCGGCGGAGGCAGCGACATCCAGATGACCCAGTCCCCCTCATCG CTGTCCGCCTCCGTGGGCGACCGCGTCACCATCACATGCCGGGCCTCA CAGTCGATCTCCTCCTACCTCAATTGGTATCAGCAGAAGCCCGGAAAG GCCCCTAAGCTTCTGATCTACGCAGCGTCCTCCCTGCAATCCGGGGTC CCATCTCGGTTCTCCGGCTCGGGCAGCGGTACCGACTTCACTCTGACC ATCTCGAGCCTGCAGCCGGAGGACTTCGCCACTTACTACTGTCAGCAA AGCTACACCCTCGCGTTTGGCCAGGGCACCAAAGTGGACATCAAG |
| **ScFv domain** | | |
| **139108- aa** | 1029 | QVQLVESGGGLVKPGGSLRLSCAASGFTFSDYYMSWIRQAPGKGLEWV SYISSSGSTIYYADSVKGRFTISRDNAKNSLYLQMNSLRAEDTAVYYC ARESGDGMDVWGQGTTVTVSS |
| **VH** | | |
| **139108- aa** | 1030 | DIQMTQSPSSLSASVGDRVTITCRASQSISSYLNWYQQKPGKAPKLLI YAASSLQSGVPSRFSGSGSGTDFTLTISSLQPEDFATYYCQQSYTLAF GQGTKVDIK |
| **VL** | | |
| **139108- aa** | 1031 | MALPVTALLLPLALLLHAARPQVQLVESGGGLVKPGGSLRLSCAASGF TFSDYYMSWIRQAPGKGLEWVSYISSSGSTIYYADSVKGRFTISRDNA KNSLYLQMNSLRAEDTAVYYCARESGDGMDVWGQGTTVTVSSASGGGG SGGRASGGGGSDIQMTQSPSSLSASVGDRVTITCRASQSISSYLNWYQ QKPGKAPKLLIYAASSLQSGVPSRFSGSGSGTDFTLTISSLQPEDFAT |
| **Full CAR** | | |
| | | YYCQQSYTLAFGQGTKVDIKTTTPAPRPPTPAPTIASQPLSLRPEACR PAAGGAVHTRGLDFACDIYIWAPLAGTCGVLLLSLVITLYCKRGRKKL LYIFKQPFMRPVQTTQEEDGCSCRFPEEEEGGCELRVKFSRSADAPAY KQGQNQLYNELNLGRREEYDVLDKRRGRDPEMGGKPRRKNPQEGLYNE LQKDKMAEAYSEIGMKGERRRGKGHDGLYQGLSTATKDTYDALHMQAL PPR |
| **139108- nt** | 1032 | ATGGCCCTCCCTGTCACCGCCCTGCTGCTTCCGCTGGCTCTTCTGCTC CACGCCGCTCGGCCCCAAGTGCAACTCGTGGAATCTGGTGGAGGACTC GTGAAACCTGGAGGATCATTGAGACTGTCATGCGCGGCCTCGGGATTC ACGTTCTCCGATTACTACATGAGCTGGATTCGCCAGGCTCCGGGGAAG GGACTGGAATGGGTGTCCTACATTTCCTCATCCGGCTCCACCATCTAC TACGCGGACTCCGTGAAGGGGAGATTCACCATTAGCCGCGATAACGCC AAGAACAGCCTGTACCTTCAGATGAACTCCCTGCGGGCTGAAGATACT GCCGTCTACTACTGCGCAAGGGAGAGCGGAGATGGGATGGACGTCTGG GGACAGGGTACCACTGTGACCGTGTCGTCGGCCTCCGGCGGAGGGGGT TCGGGTGGAAGGGCCAGCGGCGGCGGAGGCAGCGACATCCAGATGACC CAGTCCCCCTCATCGCTGTCCGCCTCCGTGGGCGACCGCGTCACCATC ACATGCCGGGCCTCACAGTCGATCTCCTCCTACCTCAATTGGTATCAG CAGAAGCCCGGAAAGGCCCCTAAGCTTCTGATCTACGCAGCGTCCTCC CTGCAATCCGGGGTCCCATCTCGGTTCTCCGGCTCGGGCAGCGGTACC GACTTCACTCTGACCATCTCGAGCCTGCAGCCGGAGGACTTCGCCACT TACTACTGTCAGCAAAGCTACACCCTCGCGTTTGGCCAGGGCACCAAA GTGGACATCAAGACCACTACCCCAGCACCGAGGCCACCCACCCCGGCT CCTACCATCGCCTCCCAGCCTCTGTCCCTGCGTCCGGAGGCATGTAGA CCCGCAGCTGGTGGGGCCGTGCATACCCGGGGTCTTGACTTCGCCTGC GATATCTACATTTGGGCCCCTCTGGCTGGTACTTGCGGGGTCCTGCTG CTTTCACTCGTGATCACTCTTTACTGTAAGCGCGGTCGGAAGAAGCTG CTGTACATCTTTAAGCAACCCTTCATGAGGCCTGTGCAGACTACTCAA GAGGAGGACGGCTGTTCATGCCGGTTCCCAGAGGAGGAGGAAGGCGGC TGCGAACTGCGCGTGAAATTCAGCCGCAGCGCAGATGCTCCAGCCTAC AAGCAGGGGCAGAACCAGCTCTACAACGAACTCAATCTTGGTCGGAGA GAGGAGTACGACGTGCTGGACAAGCGGAGAGGACGGGACCCAGAAATG GGCGGGAAGCCGCGCAGAAAGAATCCCCAAGAGGGCCTGTACAACGAG CTCCAAAAGGATAAGATGGCAGAAGCCTATAGCGAGATTGGTATGAAA GGGGAACGCAGAAGAGGCAAAGGCCACGACGGACTGTACCAGGGACTC AGCACCGCCACCAAGGACACCTATGACGCTCTTCACATGCAGGCCCTG CCGCCTCGG |
| **Full CAR** | | |

| **139110** | | |
|---|---|---|
| **139110- aa** | 1033 | QVQLVQSGGGLVKPGGSLRLSCAASGFTFSDYYMSWIRQAPGKGLEWV SYISSSGNTIYYADSVKGRFTISRDNAKNSLYLQMNSLRAEDTAVYYC ARSTMVREDYWGQGTLVTVSSASGGGGSGGRASGGGGSDIVLTQSPLS LPVTLGQPASISCKSSESLVHNSGKTYLNWFHQRPGQSPRRLIYEVSN RDSGVPDRFTGSGSGTDFTLKISRVEAEDVGVYYCMQGTHWPGTFGQG TKLEIK |
| **ScFv domain** | | |
| **139110- nt** | 1034 | CAAGTGCAACTGGTGCAAAGCGGAGGAGGATTGGTCAAACCCGGAGGA AGCCTGAGACTGTCATGCGCGGCCTCTGGATTCACCTTCTCCGATTAC TACATGTCATGGATCAGACAGGCCCCGGGGAAGGGCCTCGAATGGGTG TCCTACATCTCGTCCTCCGGGAACACCATCTACTACGCCGACAGCGTG AAGGGCCGCTTTACCATTTCCCGCGACAACGCAAAGAACTCGCTGTAC CTTCAGATGAATTCCCTGCGGGCTGAAGATACCGCGGTGTACTATTGC GCCCGGTCCACTATGGTCCGGGAGGACTACTGGGGACAGGGCACACTC GTGACCGTGTCCAGCGCGAGCGGGGGTGGAGGCAGCGGTGGACGCGCC TCCGGCGGCGGCGGTTCAGACATCGTGCTGACTCAGTCGCCCCTGTCG CTGCCGGTCACCCTGGGCCAACCGGCCTCAATTAGCTGCAAGTCCTCG GAGAGCCTGGTGCACAACTCAGGAAAGACTTACCTGAACTGGTTCCAT CAGCGGCCTGGACAGTCCCCACGGAGGCTCATCTATGAAGTGTCCAAC AGGGATTCGGGGGTGCCCGACCGCTTCACTGGCTCCGGGTCCGGCACC GACTTCACCTTGAAAATCTCCAGAGTGGAAGCCGAGGACGTGGGCGTG TACTACTGTATGCAGGGTACCCACTGGCCTGGAACCTTTGGACAAGGA ACTAAGCTCGAGATTAAG |
| **ScFv domain** | | |
| **139110- aa** | 1035 | QVQLVQSGGGLVKPGGSLRLSCAASGFTFSDYYMSWIRQAPGKGLEWV SYISSSGNTIYYADSVKGRFTISRDNAKNSLYLQMNSLRAEDTAVYYC ARSTMVREDYWGQGTLVTVSS |
| **VH** | | |
| **139110- aa** | 1036 | DIVLTQSPLSLPVTLGQPASISCKSSESLVHNSGKTYLNWFHQRPGQS PRRLIYEVSNRDSGVPDRFTGSGSGTDFTLKISRVEAEDVGVYYCMQG THWPGTFGQGTKLEIK |
| **VL** | | |
| **139110- aa** | 1037 | MALPVTALLLPLALLLHAARPQVQLVQSGGGLVKPGGSLRLSCAASGF TFSDYYMSWIRQAPGKGLEWVSYISSSGNTIYYADSVKGRFTISRDNA KNSLYLQMNSLRAEDTAVYYCARSTMVREDYWGQGTLVTVSSASGGGG SGGRASGGGGSDIVLTQSPLSLPVTLGQPASISCKSSESLVHNSGKTY LNWFHQRPGQSPRRLIYEVSNRDSGVPDRFTGSGSGTDFTLKISRVEA EDVGVYYCMQGTHWPGTFGQGTKLEIKTTTPAPRPPTPAPTIASQPLS LRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNP QEGLYNELQKDKMAEAYSEIGMKGERRRGKGHDGLYQGLSTATKDTYD ALHMQALPPR |
| **Full CAR** | | |
| **139110- nt** | 1038 | ATGGCCCTCCCTGTCACCGCCCTGCTGCTTCCGCTGGCTCTTCTGCTC CACGCCGCTCGGCCCCAAGTGCAACTGGTGCAAAGCGGAGGAGGATTG GTCAAACCCGGAGGAAGCCTGAGACTGTCATGCGCGGCCTCTGGATTC ACCTTCTCCGATTACTACATGTCATGGATCAGACAGGCCCCGGGGAAG GGCCTCGAATGGGTGTCCTACATCTCGTCCTCCGGGAACACCATCTAC TACGCCGACAGCGTGAAGGGCCGCTTTACCATTTCCCGCGACAACGCA AAGAACTCGCTGTACCTTCAGATGAATTCCCTGCGGGCTGAAGATACC GCGGTGTACTATTGCGCCCGGTCCACTATGGTCCGGGAGGACTACTGG GGACAGGGCACACTCGTGACCGTGTCCAGCGCGAGCGGGGGTGGAGGC AGCGGTGGACGCGCCTCCGGCGGCGGCGGTTCAGACATCGTGCTGACT CAGTCGCCCCTGTCGCTGCCGGTCACCCTGGGCCAACCGGCCTCAATT |
| **Full CAR** | | |
| | | AGCTGCAAGTCCTCGGAGAGCCTGGTGCACAACTCAGGAAAGACTTAC CTGAACTGGTTCCATCAGCGGCCTGGACAGTCCCCACGGAGGCTCATC TATGAAGTGTCCAACAGGGATTCGGGGGTGCCCGACCGCTTCACTGGC TCCGGGTCCGGCACCGACTTCACCTTGAAAATCTCCAGAGTGGAAGCC GAGGACGTGGGCGTGTACTACTGTATGCAGGGTACCCACTGGCCTGGA ACCTTTGGACAAGGAACTAAGCTCGAGATTAAGACCACTACCCCAGCA CCGAGGCCACCCACCCCGGCTCCTACCATCGCCTCCCAGCCTCTGTCC CTGCGTCCGGAGGCATGTAGACCCGCAGCTGGTGGGGCCGTGCATACC CGGGGTCTTGACTTCGCCTGCGATATCTACATTTGGGCCCCTCTGGCT GGTACTTGCGGGGTCCTGCTGCTTTCACTCGTGATCACTCTTTACTGT AAGCGCGGTCGGAAGAAGCTGCTGTACATCTTTAAGCAACCCTTCATG AGGCCTGTGCAGACTACTCAAGAGGAGGACGGCTGTTCATGCCGGTTC CCAGAGGAGGAGGAAGGCGGCTGCGAACTGCGCGTGAAATTCAGCCGC AGCGCAGATGCTCCAGCCTACAAGCAGGGGCAGAACCAGCTCTACAAC GAACTCAATCTTGGTCGGAGAGAGGAGTACGACGTGCTGGACAAGCGG AGAGGACGGGACCCAGAAATGGGCGGGAAGCCGCGCAGAAAGAATCCC CAAGAGGGCCTGTACAACGAGCTCCAAAAGGATAAGATGGCAGAAGCC TATAGCGAGATTGGTATGAAAGGGGAACGCAGAAGAGGCAAAGGCCAC GACGGACTGTACCAGGGACTCAGCACCGCCACCAAGGACACCTATGAC GCTCTTCACATGCAGGCCCTGCCGCCTCGG |

| **139112** | | |
|---|---|---|
| **139112- aa** | 1039 | QVQLVESGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSSASGGGGSGGRASGGGGSDIRLTQSPSPLS ASVGDRVTITCQASEDINKFLNWYHQTPGKAPKLLIYDASTLQTGVPS RFSGSGSGTDFTLTINSLQPEDIGTYYCQQYESLPLTFGGGTKVEIK |
| **ScFv domain** | | |
| **139112- nt** | 1040 | CAAGTGCAACTCGTGGAATCTGGTGGAGGACTCGTGCAACCCGGTGGA AGCCTTAGGCTGTCGTGCGCCGTCAGCGGGTTTGCTCTGAGCAACCAT GGAATGTCCTGGGTCCGCCGGGCACCGGGAAAAGGGCTGGAATGGGTG TCCGGCATCGTGTACAGCGGGTCAACCTATTACGCCGCGTCCGTGAAG GGCAGATTCACTATCTCAAGAGACAACAGCCGGAACACCCTGTACTTG CAAATGAATTCCCTGCGCCCCGAGGACACCGCCATCTACTACTGCTCC GCCCACGGAGGAGAGTCGGACGTGTGGGGCCAGGGAACGACTGTGACT GTGTCCAGCGCATCAGGAGGGGGTGGTTCGGGCGGCCGGGCCTCGGGG GGAGGAGGTTCCGACATTCGGCTGACCCAGTCCCCGTCCCCACTGTCG GCCTCCGTCGGCGACCGCGTGACCATCACTTGTCAGGCGTCCGAGGAC ATTAACAAGTTCCTGAACTGGTACCACCAGACCCCTGGAAAGGCCCCC AAGCTGCTGATCTACGATGCCTCGACCCTTCAAACTGGAGTGCCTAGC CGGTTCTCCGGGTCCGGCTCCGGCACTGATTTCACTCTGACCATCAAC TCATTGCAGCCGGAAGATATCGGGACCTACTATTGCCAGCAGTACGAA TCCCTCCCGCTCACATTCGGCGGGGGAACCAAGGTCGAGATTAAG |
| **ScFv domain** | | |
| **139112- aa VH** | 1041 | QVQLVESGGGLVQPGGSLRLSCAVSGFALSNHGMSWVRRAPGKGLEWV SGIVYSGSTYYAASVKGRFTISRDNSRNTLYLQMNSLRPEDTAIYYCS |
| | | AHGGESDVWGQGTTVTVSS |
| **139112- aa** | 1042 | DIRLTQSPSPLSASVGDRVTITCQASEDINKFLNWYHQTPGKAPKLLI YDASTLQTGVPSRFSGSGSGTDFTLTINSLQPEDIGTYYCQQYESLPL TFGGGTKVEIK |
| **VL** | | |
| **139112- aa** | 1043 | MALPVTALLLPLALLLHAARPQVQLVESGGGLVQPGGSLRLSCAVSGF ALSNHGMSWVRRAPGKGLEWVSGIVYSGSTYYAASVKGRFTISRDNSR NTLYLQMNSLRPEDTAIYYCSAHGGESDVWGQGTTVTVSSASGGGGSG GRASGGGGSDIRLTQSPSPLSASVGDRVTITCQASEDINKFLNWYHQT PGKAPKLLIYDASTLQTGVPSRFSGSGSGTDFTLTINSLQPEDIGTYY CQQYESLPLTFGGGTKVEIKTTTPAPRPPTPAPTIASQPLSLRPEACR PAAGGAVHTRGLDFACDIYIWAPLAGTCGVLLLSLVITLYCKRGRKKL LYIFKQPFMRPVQTTQEEDGCSCRFPEEEEGGCELRVKFSRSADAPAY KQGQNQLYNELNLGRREEYDVLDKRRGRDPEMGGKPRRKNPQEGLYNE LQKDKMAEAYSEIGMKGERRRGKGHDGLYQGLSTATKDTYDALHMQAL PPR |
| **Full CAR** | | |
| **139112- nt** | 1044 | ATGGCCCTCCCTGTCACCGCCCTGCTGCTTCCGCTGGCTCTTCTGCTC CACGCCGCTCGGCCCCAAGTGCAACTCGTGGAATCTGGTGGAGGACTC GTGCAACCCGGTGGAAGCCTTAGGCTGTCGTGCGCCGTCAGCGGGTTT GCTCTGAGCAACCATGGAATGTCCTGGGTCCGCCGGGCACCGGGAAAA GGGCTGGAATGGGTGTCCGGCATCGTGTACAGCGGGTCAACCTATTAC GCCGCGTCCGTGAAGGGCAGATTCACTATCTCAAGAGACAACAGCCGG AACACCCTGTACTTGCAAATGAATTCCCTGCGCCCCGAGGACACCGCC ATCTACTACTGCTCCGCCCACGGAGGAGAGTCGGACGTGTGGGGCCAG GGAACGACTGTGACTGTGTCCAGCGCATCAGGAGGGGGTGGTTCGGGC GGCCGGGCCTCGGGGGGAGGAGGTTCCGACATTCGGCTGACCCAGTCC CCGTCCCCACTGTCGGCCTCCGTCGGCGACCGCGTGACCATCACTTGT CAGGCGTCCGAGGACATTAACAAGTTCCTGAACTGGTACCACCAGACC CCTGGAAAGGCCCCCAAGCTGCTGATCTACGATGCCTCGACCCTTCAA ACTGGAGTGCCTAGCCGGTTCTCCGGGTCCGGCTCCGGCACTGATTTC ACTCTGACCATCAACTCATTGCAGCCGGAAGATATCGGGACCTACTAT TGCCAGCAGTACGAATCCCTCCCGCTCACATTCGGCGGGGGAACCAAG GTCGAGATTAAGACCACTACCCCAGCACCGAGGCCACCCACCCCGGCT CCTACCATCGCCTCCCAGCCTCTGTCCCTGCGTCCGGAGGCATGTAGA CCCGCAGCTGGTGGGGCCGTGCATACCCGGGGTCTTGACTTCGCCTGC GATATCTACATTTGGGCCCCTCTGGCTGGTACTTGCGGGGTCCTGCTG CTTTCACTCGTGATCACTCTTTACTGTAAGCGCGGTCGGAAGAAGCTG CTGTACATCTTTAAGCAACCCTTCATGAGGCCTGTGCAGACTACTCAA GAGGAGGACGGCTGTTCATGCCGGTTCCCAGAGGAGGAGGAAGGCGGC TGCGAACTGCGCGTGAAATTCAGCCGCAGCGCAGATGCTCCAGCCTAC AAGCAGGGGCAGAACCAGCTCTACAACGAACTCAATCTTGGTCGGAGA GAGGAGTACGACGTGCTGGACAAGCGGAGAGGACGGGACCCAGAAATG GGCGGGAAGCCGCGCAGAAAGAATCCCCAAGAGGGCCTGTACAACGAG CTCCAAAAGGATAAGATGGCAGAAGCCTATAGCGAGATTGGTATGAAA GGGGAACGCAGAAGAGGCAAAGGCCACGACGGACTGTACCAGGGACTC AGCACCGCCACCAAGGACACCTATGACGCTCTTCACATGCAGGCCCTG |
| **Full CAR** | | |
| | | CCGCCTCGG |

| **139113** | | |
|---|---|---|
| **139113- aa** | 1045 | EVQLVETGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSSASGGGGSGGRASGGGGSETTLTQSPATLS VSPGERATLSCRASQSVGSNLAWYQQKPGQGPRLLIYGASTRATGIPA RFSGSGSGTEFTLTISSLQPEDFAVYYCQQYNDWLPVTFGQGTKVEIK |
| **ScFv domain** | | |
| **139113- nt** | 1046 | GAAGTGCAATTGGTGGAAACTGGAGGAGGACTTGTGCAACCTGGAGGA TCATTGCGGCTCTCATGCGCTGTCTCCGGCTTCGCCCTGTCAAATCAC GGGATGTCGTGGGTCAGACGGGCCCCGGGAAAGGGTCTGGAATGGGTG TCGGGGATTGTGTACAGCGGCTCCACCTACTACGCCGCTTCGGTCAAG GGCCGCTTCACTATTTCACGGGACAACAGCCGCAACACCCTCTATCTG CAAATGAACTCTCTCCGCCCGGAGGATACCGCCATCTACTACTGCTCC GCACACGGCGGCGAATCCGACGTGTGGGGACAGGGAACCACTGTCACC GTGTCGTCCGCATCCGGTGGCGGAGGATCGGGTGGCCGGGCCTCCGGG GGCGGCGGCAGCGAGACTACCCTGACCCAGTCCCCTGCCACTCTGTCC GTGAGCCCGGGAGAGAGAGCCACCCTTAGCTGCCGGGCCAGCCAGAGC GTGGGCTCCAACCTGGCCTGGTACCAGCAGAAGCCAGGACAGGGTCCC AGGCTGCTGATCTACGGAGCCTCCACTCGCGCGACCGGCATCCCCGCG AGGTTCTCCGGGTCGGGTTCCGGGACCGAGTTCACCCTGACCATCTCC TCCCTCCAACCGGAGGACTTCGCGGTGTACTACTGTCAGCAGTACAAC GATTGGCTGCCCGTGACATTTGGACAGGGGACGAAGGTGGAAATCAAA |
| **ScFv domain** | | |
| **139113- aa** | 1047 | EVQLVETGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSS |
| **VH** | | |
| **139113- aa** | 1048 | ETTLTQSPATLSVSPGERATLSCRASQSVGSNLAWYQQKPGQGPRLLI YGASTRATGIPARFSGSGSGTEFTLTISSLQPEDFAVYYCQQYNDWLP VTFGQGTKVEIK |
| **VL** | | |
| **139113- aa** | 1049 | MALPVTALLLPLALLLHAARPEVQLVETGGGLVQPGGSLRLSCAVSGF ALSNHGMSWVRRAPGKGLEWVSGIVYSGSTYYAASVKGRFTISRDNSR NTLYLQMNSLRPEDTAIYYCSAHGGESDVWGQGTTVTVSSASGGGGSG GRASGGGGSETTLTQSPATLSVSPGERATLSCRASQSVGSNLAWYQQK PGQGPRLLIYGASTRATGIPARFSGSGSGTEFTLTISSLQPEDFAVYY CQQYNDWLPVTFGQGTKVEIKTTTPAPRPPTPAPTIASQPLSLRPEAC RPAAGGAVHTRGLDFACDIYIWAPLAGTCGVLLLSLVITLYCKRGRKK LLYIFKQPFMRPVQTTQEEDGCSCRFPEEEEGGCELRVKFSRSADAPA YKQGQNQLYNELNLGRREEYDVLDKRRGRDPEMGGKPRRKNPQEGLYN ELQKDKMAEAYSEIGMKGERRRGKGHDGLYQGLSTATKDTYDALHMQA LPPR |
| **Full CAR** | | |
| **139113- nt Full CAR** | 1050 | ATGGCCCTCCCTGTCACCGCCCTGCTGCTTCCGCTGGCTCTTCTGCTC CACGCCGCTCGGCCCGAAGTGCAATTGGTGGAAACTGGAGGAGGACTT GTGCAACCTGGAGGATCATTGCGGCTCTCATGCGCTGTCTCCGGCTTC |
| | | GCCCTGTCAAATCACGGGATGTCGTGGGTCAGACGGGCCCCGGGAAAG GGTCTGGAATGGGTGTCGGGGATTGTGTACAGCGGCTCCACCTACTAC GCCGCTTCGGTCAAGGGCCGCTTCACTATTTCACGGGACAACAGCCGC AACACCCTCTATCTGCAAATGAACTCTCTCCGCCCGGAGGATACCGCC ATCTACTACTGCTCCGCACACGGCGGCGAATCCGACGTGTGGGGACAG GGAACCACTGTCACCGTGTCGTCCGCATCCGGTGGCGGAGGATCGGGT GGCCGGGCCTCCGGGGGCGGCGGCAGCGAGACTACCCTGACCCAGTCC CCTGCCACTCTGTCCGTGAGCCCGGGAGAGAGAGCCACCCTTAGCTGC CGGGCCAGCCAGAGCGTGGGCTCCAACCTGGCCTGGTACCAGCAGAAG CCAGGACAGGGTCCCAGGCTGCTGATCTACGGAGCCTCCACTCGCGCG ACCGGCATCCCCGCGAGGTTCTCCGGGTCGGGTTCCGGGACCGAGTTC ACCCTGACCATCTCCTCCCTCCAACCGGAGGACTTCGCGGTGTACTAC TGTCAGCAGTACAACGATTGGCTGCCCGTGACATTTGGACAGGGGACG AAGGTGGAAATCAAAACCACTACCCCAGCACCGAGGCCACCCACCCCG GCTCCTACCATCGCCTCCCAGCCTCTGTCCCTGCGTCCGGAGGCATGT AGACCCGCAGCTGGTGGGGCCGTGCATACCCGGGGTCTTGACTTCGCC TGCGATATCTACATTTGGGCCCCTCTGGCTGGTACTTGCGGGGTCCTG CTGCTTTCACTCGTGATCACTCTTTACTGTAAGCGCGGTCGGAAGAAG CTGCTGTACATCTTTAAGCAACCCTTCATGAGGCCTGTGCAGACTACT CAAGAGGAGGACGGCTGTTCATGCCGGTTCCCAGAGGAGGAGGAAGGC GGCTGCGAACTGCGCGTGAAATTCAGCCGCAGCGCAGATGCTCCAGCC TACAAGCAGGGGCAGAACCAGCTCTACAACGAACTCAATCTTGGTCGG AGAGAGGAGTACGACGTGCTGGACAAGCGGAGAGGACGGGACCCAGAA ATGGGCGGGAAGCCGCGCAGAAAGAATCCCCAAGAGGGCCTGTACAAC GAGCTCCAAAAGGATAAGATGGCAGAAGCCTATAGCGAGATTGGTATG AAAGGGGAACGCAGAAGAGGCAAAGGCCACGACGGACTGTACCAGGGA CTCAGCACCGCCACCAAGGACACCTATGACGCTCTTCACATGCAGGCC CTGCCGCCTCGG |

| **139114** | | |
|---|---|---|
| **139114- aa** | 1051 | EVQLVESGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSSASGGGGSGGRASGGGGSEIVLTQSPGTLS LSPGERATLSCRASQSIGSSSLAWYQQKPGQAPRLLMYGASSRASGIP DRFSGSGSGTDFTLTISRLEPEDFAVYYCQQYAGSPPFTFGQGTKVEI K |
| **ScFv domain** | | |
| **139114- nt** | 1052 | GAAGTGCAATTGGTGGAATCTGGTGGAGGACTTGTGCAACCTGGAGGA TCACTGAGACTGTCATGCGCGGTGTCCGGTTTTGCCCTGAGCAATCAT GGGATGTCGTGGGTCCGGCGCGCCCCCGGAAAGGGTCTGGAATGGGTG TCGGGTATCGTCTACTCCGGGAGCACTTACTACGCCGCGAGCGTGAAG GGCCGCTTCACCATTTCCCGCGATAACTCCCGCAACACCCTGTACTTG CAAATGAACTCGCTCCGGCCTGAGGACACTGCCATCTACTACTGCTCC GCACACGGAGGAGAATCCGACGTGTGGGGCCAGGGAACTACCGTGACC GTCAGCAGCGCCTCCGGCGGCGGGGGCTCAGGCGGACGGGCTAGCGGC GGCGGTGGCTCCGAGATCGTGCTGACCCAGTCGCCTGGCACTCTCTCG CTGAGCCCCGGGGAAAGGGCAACCCTGTCCTGTCGGGCCAGCCAGTCC |
| **ScFv domain** | | |
| | | ATTGGATCATCCTCCCTCGCCTGGTATCAGCAGAAACCGGGACAGGCT CCGCGGCTGCTTATGTATGGGGCCAGCTCAAGAGCCTCCGGCATTCCC GACCGGTTCTCCGGGTCCGGTTCCGGCACCGATTTCACCCTGACTATC TCGAGGCTGGAGCCAGAGGACTTCGCCGTGTACTACTGCCAGCAGTAC GCGGGGTCCCCGCCGTTCACGTTCGGACAGGGAACCAAGGTCGAGATC AAG |
| **139114- aa** | 1053 | EVQLVESGGGLVQPGGSLRLSCAVSGFALSNHGMSWVRRAPGKGLEWV SGIVYSGSTYYAASVKGRFTISRDNSRNTLYLQMNSLRPEDTAIYYCS AHGGESDVWGQGTTVTVSS |
| **VH** | | |
| **139114- aa** | 1054 | EIVLTQSPGTLSLSPGERATLSCRASQSIGSSSLAWYQQKPGQAPRLL MYGASSRASGIPDRFSGSGSGTDFTLTISRLEPEDFAVYYCQQYAGSP PFTFGQGTKVEIK |
| **VL** | | |
| **139114- aa** | 1055 | MALPVTALLLPLALLLHAARPEVQLVESGGGLVQPGGSLRLSCAVSGF ALSNHGMSWVRRAPGKGLEWVSGIVYSGSTYYAASVKGRFTISRDNSR NTLYLQMNSLRPEDTAIYYCSAHGGESDVWGQGTTVTVSSASGGGGSG GRASGGGGSEIVLTQSPGTLSLSPGERATLSCRASQSIGSSSLAWYQQ KPGQAPRLLMYGASSRASGIPDRFSGSGSGTDFTLTISRLEPEDFAVY YCQQYAGSPPFTFGQGTKVEIKTTTPAPRPPTPAPTIASQPLSLRPEA CRPAAGGAVHTRGLDFACDIYIWAPLAGTCGVLLLSLVITLYCKRGRK KLLYIFKQPFMRPVQTTQEEDGCSCRFPEEEEGGCELRVKFSRSADAP AYKQGQNQLYNELNLGRREEYDVLDKRRGRDPEMGGKPRRKNPQEGLY NELQKDKMAEAYSEIGMKGERRRGKGHDGLYQGLSTATKDTYDALHMQ ALPPR |
| **Full CAR** | | |
| **139114- nt** | 1056 | ATGGCCCTCCCTGTCACCGCCCTGCTGCTTCCGCTGGCTCTTCTGCTC CACGCCGCTCGGCCCGAAGTGCAATTGGTGGAATCTGGTGGAGGACTT GTGCAACCTGGAGGATCACTGAGACTGTCATGCGCGGTGTCCGGTTTT GCCCTGAGCAATCATGGGATGTCGTGGGTCCGGCGCGCCCCCGGAAAG GGTCTGGAATGGGTGTCGGGTATCGTCTACTCCGGGAGCACTTACTAC GCCGCGAGCGTGAAGGGCCGCTTCACCATTTCCCGCGATAACTCCCGC AACACCCTGTACTTGCAAATGAACTCGCTCCGGCCTGAGGACACTGCC ATCTACTACTGCTCCGCACACGGAGGAGAATCCGACGTGTGGGGCCAG GGAACTACCGTGACCGTCAGCAGCGCCTCCGGCGGCGGGGGCTCAGGC GGACGGGCTAGCGGCGGCGGTGGCTCCGAGATCGTGCTGACCCAGTCG CCTGGCACTCTCTCGCTGAGCCCCGGGGAAAGGGCAACCCTGTCCTGT CGGGCCAGCCAGTCCATTGGATCATCCTCCCTCGCCTGGTATCAGCAG AAACCGGGACAGGCTCCGCGGCTGCTTATGTATGGGGCCAGCTCAAGA GCCTCCGGCATTCCCGACCGGTTCTCCGGGTCCGGTTCCGGCACCGAT TTCACCCTGACTATCTCGAGGCTGGAGCCAGAGGACTTCGCCGTGTAC TACTGCCAGCAGTACGCGGGGTCCCCGCCGTTCACGTTCGGACAGGGA ACCAAGGTCGAGATCAAGACCACTACCCCAGCACCGAGGCCACCCACC CCGGCTCCTACCATCGCCTCCCAGCCTCTGTCCCTGCGTCCGGAGGCA TGTAGACCCGCAGCTGGTGGGGCCGTGCATACCCGGGGTCTTGACTTC GCCTGCGATATCTACATTTGGGCCCCTCTGGCTGGTACTTGCGGGGTC CTGCTGCTTTCACTCGTGATCACTCTTTACTGTAAGCGCGGTCGGAAG |
| **Full CAR** | | |
| | | AAGCTGCTGTACATCTTTAAGCAACCCTTCATGAGGCCTGTGCAGACT ACTCAAGAGGAGGACGGCTGTTCATGCCGGTTCCCAGAGGAGGAGGAA GGCGGCTGCGAACTGCGCGTGAAATTCAGCCGCAGCGCAGATGCTCCA GCCTACAAGCAGGGGCAGAACCAGCTCTACAACGAACTCAATCTTGGT CGGAGAGAGGAGTACGACGTGCTGGACAAGCGGAGAGGACGGGACCCA GAAATGGGCGGGAAGCCGCGCAGAAAGAATCCCCAAGAGGGCCTGTAC AACGAGCTCCAAAAGGATAAGATGGCAGAAGCCTATAGCGAGATTGGT ATGAAAGGGGAACGCAGAAGAGGCAAAGGCCACGACGGACTGTACCAG GGACTCAGCACCGCCACCAAGGACACCTATGACGCTCTTCACATGCAG GCCCTGCCGCCTCGG |

| **149362** | | |
|---|---|---|
| **149362-aa** | 1057 | QVQLQESGPGLVKPSETLSLTCTVSGGSISSSYYYWGWIRQPPGKGLE WIGSIYYSGSAYYNPSLKSRVTISVDTSKNQFSLRLSSVTAADTAVYY CARHWQEWPDAFDIWGQGTMVTVSSGGGGSGGGGSGGGGSETTLTQSP AFMSATPGDKVIISCKASQDIDDAMNWYQQKPGEAPLFIIQSATSPVP GIPPRFSGSGFGTDFSLTINNIESEDAAYYFCLQHDNFPLTFGQGTKL EIK |
| **ScFv domain** | | |
| **149362-nt** | 1058 | CAAGTGCAGCTTCAGGAAAGCGGACCGGGCCTGGTCAAGCCATCCGAA ACTCTCTCCCTGACTTGCACTGTGTCTGGCGGTTCCATCTCATCGTCG TACTACTACTGGGGCTGGATTAGGCAGCCGCCCGGAAAGGGACTGGAG TGGATCGGAAGCATCTACTATTCCGGCTCGGCGTACTACAACCCTAGC CTCAAGTCGAGAGTGACCATCTCCGTGGATACCTCCAAGAACCAGTTT TCCCTGCGCCTGAGCTCCGTGACCGCCGCTGACACCGCCGTGTACTAC TGTGCTCGGCATTGGCAGGAATGGCCCGATGCCTTCGACATTTGGGGC CAGGGCACTATGGTCACTGTGTCATCCGGGGGTGGAGGCAGCGGGGGA GGAGGGTCCGGGGGGGGAGGTTCAGAGACAACCTTGACCCAGTCACCC GCATTCATGTCCGCCACTCCGGGAGACAAGGTCATCATCTCGTGCAAA GCGTCCCAGGATATCGACGATGCCATGAATTGGTACCAGCAGAAGCCT GGCGAAGCGCCGCTGTTCATTATCCAATCCGCAACCTCGCCCGTGCCT GGAATCCCACCGCGGTTCAGCGGCAGCGGTTTCGGAACCGACTTTTCC CTGACCATTAACAACATTGAGTCCGAGGACGCCGCCTACTACTTCTGC CTGCAACACGACAACTTCCCTCTCACGTTCGGCCAGGGAACCAAGCTG GAAATCAAG |
| **ScFv domain** | | |
| **149362-aa** | 1059 | QVQLQESGPGLVKPSETLSLTCTVSGGSISSSYYYWGWIRQPPGKGLE WIGSIYYSGSAYYNPSLKSRVTISVDTSKNQFSLRLSSVTAADTAVYY CARHWQEWPDAFDIWGQGTMVTVSS |
| **VH** | | |
| **149362-aa** | 1060 | ETTLTQSPAFMSATPGDKVIISCKASQDIDDAMNWYQQKPGEAPLFII QSATSPVPGIPPRFSGSGFGTDFSLTINNIESEDAAYYFCLQHDNFPL TFGQGTKLEIK |
| **VL** | | |
| **149362-aa** | 1061 | MALPVTALLLPLALLLHAARPQVQLQESGPGLVKPSETLSLTCTVSGG SISSSYYYWGWIRQPPGKGLEWIGSIYYSGSAYYNPSLKSRVTISVDT SKNQFSLRLSSVTAADTAVYYCARHWQEWPDAFDIWGQGTMVTVSSGG |
| **Full CAR** | | |
| | | GGSGGGGSGGGGSETTLTQSPAFMSATPGDKVIISCKASQDIDDAMNW YQQKPGEAPLFIIQSATSPVPGIPPRFSGSGFGTDFSLTINNIESEDA AYYFCLQHDNFPLTFGQGTKLEIKTTTPAPRPPTPAPTIASQPLSLRP EACRPAAGGAVHTRGLDFACDIYIWAPLAGTCGVLLLSLVITLYCKRG RKKLLYIFKQPFMRPVQTTQEEDGCSCRFPEEEEGGCELRVKFSRSAD APAYKQGQNQLYNELNLGRREEYDVLDKRRGRDPEMGGKPRRKNPQEG LYNELQKDKMAEAYSEIGMKGERRRGKGHDGLYQGLSTATKDTYDALH MQALPPR |
| **149362-nt** | 1062 | ATGGCCCTCCCTGTCACCGCCCTGCTGCTTCCGCTGGCTCTTCTGCTC CACGCCGCTCGGCCCCAAGTGCAGCTTCAGGAAAGCGGACCGGGCCTG GTCAAGCCATCCGAAACTCTCTCCCTGACTTGCACTGTGTCTGGCGGT TCCATCTCATCGTCGTACTACTACTGGGGCTGGATTAGGCAGCCGCCC GGAAAGGGACTGGAGTGGATCGGAAGCATCTACTATTCCGGCTCGGCG TACTACAACCCTAGCCTCAAGTCGAGAGTGACCATCTCCGTGGATACC TCCAAGAACCAGTTTTCCCTGCGCCTGAGCTCCGTGACCGCCGCTGAC ACCGCCGTGTACTACTGTGCTCGGCATTGGCAGGAATGGCCCGATGCC TTCGACATTTGGGGCCAGGGCACTATGGTCACTGTGTCATCCGGGGGT GGAGGCAGCGGGGGAGGAGGGTCCGGGGGGGGAGGTTCAGAGACAACC TTGACCCAGTCACCCGCATTCATGTCCGCCACTCCGGGAGACAAGGTC ATCATCTCGTGCAAAGCGTCCCAGGATATCGACGATGCCATGAATTGG TACCAGCAGAAGCCTGGCGAAGCGCCGCTGTTCATTATCCAATCCGCA ACCTCGCCCGTGCCTGGAATCCCACCGCGGTTCAGCGGCAGCGGTTTC GGAACCGACTTTTCCCTGACCATTAACAACATTGAGTCCGAGGACGCC GCCTACTACTTCTGCCTGCAACACGACAACTTCCCTCTCACGTTCGGC CAGGGAACCAAGCTGGAAATCAAGACCACTACCCCAGCACCGAGGCCA CCCACCCCGGCTCCTACCATCGCCTCCCAGCCTCTGTCCCTGCGTCCG GAGGCATGTAGACCCGCAGCTGGTGGGGCCGTGCATACCCGGGGTCTT GACTTCGCCTGCGATATCTACATTTGGGCCCCTCTGGCTGGTACTTGC GGGGTCCTGCTGCTTTCACTCGTGATCACTCTTTACTGTAAGCGCGGT CGGAAGAAGCTGCTGTACATCTTTAAGCAACCCTTCATGAGGCCTGTG CAGACTACTCAAGAGGAGGACGGCTGTTCATGCCGGTTCCCAGAGGAG GAGGAAGGCGGCTGCGAACTGCGCGTGAAATTCAGCCGCAGCGCAGAT GCTCCAGCCTACAAGCAGGGGCAGAACCAGCTCTACAACGAACTCAAT CTTGGTCGGAGAGAGGAGTACGACGTGCTGGACAAGCGGAGAGGACGG GACCCAGAAATGGGCGGGAAGCCGCGCAGAAAGAATCCCCAAGAGGGC CTGTACAACGAGCTCCAAAAGGATAAGATGGCAGAAGCCTATAGCGAG ATTGGTATGAAAGGGGAACGCAGAAGAGGCAAAGGCCACGACGGACTG TACCAGGGACTCAGCACCGCCACCAAGGACACCTATGACGCTCTTCAC ATGCAGGCCCTGCCGCCTCGG |
| **Full CAR** | | |

| 149363 | | |
|---|---|---|
| **149363-aa** | 1063 | VNLRESGPALVKPTQTLTLTCTFSGFSLRTSGMCVSWIRQPPGKALEW LARIDWDEDKFYSTSLKTRLTISKDTSDNQVVLRMTNMDPADTATYYC ARSGAGGTSATAFDIWGPGTMVTVSSGGGGSGGGGSGGGGSDIQMTQS PSSLSASVGDRVTITCRASQDIYNNLAWFQLKPGSAPRSLMYAANKSQ SGVPSRFSGSASGTDFTLTISSLQPEDFATYYCQHYYRFPYSFGQGTK |
| **ScFv domain** | | |
| | | LEIK |
| **149363-nt** | 1064 | CAAGTCAATCTGCGCGAATCCGGCCCCGCCTTGGTCAAGCCTACCCAG ACCCTCACTCTGACCTGTACTTTCTCCGGCTTCTCCCTGCGGACTTCC GGGATGTGCGTGTCCTGGATCAGACAGCCTCCGGGAAAGGCCCTGGAG TGGCTCGCTCGCATTGACTGGGATGAGGACAAGTTCTACTCCACCTCA CTCAAGACCAGGCTGACCATCAGCAAAGATACCTCTGACAACCAAGTG GTGCTCCGCATGACCAACATGGACCCAGCCGACACTGCCACTTACTAC TGCGCGAGGAGCGGAGCGGGCGGAACCTCCGCCACCGCCTTCGATATT TGGGGCCCGGGTACCATGGTCACCGTGTCAAGCGGAGGAGGGGGGTCC GGGGGCGGCGGTTCCGGGGGAGGCGGATCGGACATTCAGATGACTCAG TCACCATCGTCCCTGAGCGCTAGCGTGGGCGACAGAGTGACAATCACT TGCCGGGCATCCCAGGACATCTATAACAACCTTGCGTGGTTCCAGCTG AAGCCTGGTTCCGCACCGCGGTCACTTATGTACGCCGCCAACAAGAGC CAGTCGGGAGTGCCGTCCCGGTTTTCCGGTTCGGCCTCGGGAACTGAC TTCACCCTGACGATCTCCAGCCTGCAACCCGAGGATTTCGCCACCTAC TACTGCCAGCACTACTACCGCTTTCCCTACTCGTTCGGACAGGGAACC AAGCTGGAAATCAAG |
| **ScFv domain** | | |
| **149363-aa** | 1065 | QVNLRESGPALVKPTQTLTLTCTFSGFSLRTSGMCVSWIRQPPGKALE WLARIDWDEDKFYSTSLKTRLTISKDTSDNQVVLRMTNMDPADTATYY CARSGAGGTSATAFDIWGPGTMVTVSS |
| **VH** | | |
| **149363-aa** | 1066 | DIQMTQSPSSLSASVGDRVTITCRASQDIYNNLAWFQLKPGSAPRSLM YAANKSQSGVPSRFSGSASGTDFTLTISSLQPEDFATYYCQHYYRFPY SFGQGTKLEIK |
| **VL** | | |
| **149363-aa** | 1067 | MALPVTALLLPLALLLHAARPQVNLRESGPALVKPTQTLTLTCTFSGF SLRTSGMCVSWIRQPPGKALEWLARIDWDEDKFYSTSLKTRLTISKDT SDNQVVLRMTNMDPADTATYYCARSGAGGTSATAFDIWGPGTMVTVSS GGGGSGGGGSGGGGSDIQMTQSPSSLSASVGDRVTITCRASQDIYNNL AWFQLKPGSAPRSLMYAANKSQSGVPSRFSGSASGTDFTLTISSLQPE DFATYYCQHYYRFPYSFGQGTKLEIKTTTPAPRPPTPAPTIASQPLSL RPEACRPAAGGAVHTRGLDFACDIYIWAPLAGTCGVLLLSLVITLYCK RGRKKLLYIFKQPFMRPVQTTQEEDGCSCRFPEEEEGGCELRVKFSRS ADAPAYKQGQNQLYNELNLGRREEYDVLDKRRGRDPEMGGKPRRKNPQ EGLYNELQKDKMAEAYSEIGMKGERRRGKGHDGLYQGLSTATKDTYDA LHMQALPPR |
| **Full CAR** | | |
| **149363-nt** | 1068 | ATGGCCCTCCCTGTCACCGCCCTGCTGCTTCCGCTGGCTCTTCTGCTC CACGCCGCTCGGCCCCAAGTCAATCTGCGCGAATCCGGCCCCGCCTTG GTCAAGCCTACCCAGACCCTCACTCTGACCTGTACTTTCTCCGGCTTC TCCCTGCGGACTTCCGGGATGTGCGTGTCCTGGATCAGACAGCCTCCG GGAAAGGCCCTGGAGTGGCTCGCTCGCATTGACTGGGATGAGGACAAG TTCTACTCCACCTCACTCAAGACCAGGCTGACCATCAGCAAAGATACC TCTGACAACCAAGTGGTGCTCCGCATGACCAACATGGACCCAGCCGAC ACTGCCACTTACTACTGCGCGAGGAGCGGAGCGGGCGGAACCTCCGCC ACCGCCTTCGATATTTGGGGCCCGGGTACCATGGTCACCGTGTCAAGC |
| **Full CAR** | | |
| | | GGAGGAGGGGGGTCCGGGGGCGGCGGTTCCGGGGGAGGCGGATCGGAC ATTCAGATGACTCAGTCACCATCGTCCCTGAGCGCTAGCGTGGGCGAC AGAGTGACAATCACTTGCCGGGCATCCCAGGACATCTATAACAACCTT GCGTGGTTCCAGCTGAAGCCTGGTTCCGCACCGCGGTCACTTATGTAC GCCGCCAACAAGAGCCAGTCGGGAGTGCCGTCCCGGTTTTCCGGTTCG GCCTCGGGAACTGACTTCACCCTGACGATCTCCAGCCTGCAACCCGAG GATTTCGCCACCTACTACTGCCAGCACTACTACCGCTTTCCCTACTCG TTCGGACAGGGAACCAAGCTGGAAATCAAGACCACTACCCCAGCACCG AGGCCACCCACCCCGGCTCCTACCATCGCCTCCCAGCCTCTGTCCCTG CGTCCGGAGGCATGTAGACCCGCAGCTGGTGGGGCCGTGCATACCCGG GGTCTTGACTTCGCCTGCGATATCTACATTTGGGCCCCTCTGGCTGGT ACTTGCGGGGTCCTGCTGCTTTCACTCGTGATCACTCTTTACTGTAAG CGCGGTCGGAAGAAGCTGCTGTACATCTTTAAGCAACCCTTCATGAGG CCTGTGCAGACTACTCAAGAGGAGGACGGCTGTTCATGCCGGTTCCCA GAGGAGGAGGAAGGCGGCTGCGAACTGCGCGTGAAATTCAGCCGCAGC GCAGATGCTCCAGCCTACAAGCAGGGGCAGAACCAGCTCTACAACGAA CTCAATCTTGGTCGGAGAGAGGAGTACGACGTGCTGGACAAGCGGAGA GGACGGGACCCAGAAATGGGCGGGAAGCCGCGCAGAAAGAATCCCCAA GAGGGCCTGTACAACGAGCTCCAAAAGGATAAGATGGCAGAAGCCTAT AGCGAGATTGGTATGAAAGGGGAACGCAGAAGAGGCAAAGGCCACGAC GGACTGTACCAGGGACTCAGCACCGCCACCAAGGACACCTATGACGCT CTTCACATGCAGGCCCTGCCGCCTCGG |

| **149364** | | |
|---|---|---|
| **149364-aa** | 1069 | EVQLVESGGGLVKPGGSLRLSCAASGFTFSSYSMNWVRQAPGKGLEWV SSISSSSSYIYYADSVKGRFTISRDNAKNSLYLQMNSLRAEDTAVYYC AKTIAAVYAFDIWGQGTTVTVSSGGGGSGGGGSGGGGSEIVLTQSPLS LPVTPEEPASISCRSSQSLLHSNGYNYLDWYLQKPGQSPQLLIYLGSN RASGVPDRFSGSGSGTDFTLKISRVEAEDVGVYYCMQALQTPYTFGQG TKLEIK |
| **ScFv domain** | | |
| **149364-nt** | 1070 | GAAGTGCAGCTTGTCGAATCCGGGGGGGGACTGGTCAAGCCGGGCGGA TCACTGAGACTGTCCTGCGCCGCGAGCGGCTTCACGTTCTCCTCCTAC TCCATGAACTGGGTCCGCCAAGCCCCCGGGAAGGGACTGGAATGGGTG TCCTCTATCTCCTCGTCGTCGTCCTACATCTACTACGCCGACTCCGTG AAGGGAAGATTCACCATTTCCCGCGACAACGCAAAGAACTCACTGTAC TTGCAAATGAACTCACTCCGGGCCGAAGATACTGCTGTGTACTATTGC GCCAAGACTATTGCCGCCGTCTACGCTTTCGACATCTGGGGCCAGGGA ACCACCGTGACTGTGTCGTCCGGTGGTGGTGGCTCGGGCGGAGGAGGA AGCGGCGGCGGGGGGTCCGAGATTGTGCTGACCCAGTCGCCACTGAGC CTCCCTGTGACCCCCGAGGAACCCGCCAGCATCAGCTGCCGGTCCAGC CAGTCCCTGCTCCACTCCAACGGATACAATTACCTCGATTGGTACCTT CAGAAGCCTGGACAAAGCCCGCAGCTGCTCATCTACTTGGGATCAAAC CGCGCGTCAGGAGTGCCTGACCGGTTCTCCGGCTCGGGCAGCGGTACC GATTTCACCCTGAAAATCTCCAGGGTGGAGGCAGAGGACGTGGGAGTG TATTACTGTATGCAGGCGCTGCAGACTCCGTACACATTTGGGCAGGGC |
| **ScFv domain** | | |
| | | ACCAAGCTGGAGATCAAG |
| **149364-aa** | 1071 | EVQLVESGGGLVKPGGSLRLSCAASGFTFSSYSMNWVRQAPGKGLEWV SSISSSSSYIYYADSVKGRFTISRDNAKNSLYLQMNSLRAEDTAVYYC AKTIAAVYAFDIWGQGTTVTVSS |
| **VH** | | |
| **149364-aa** | 1072 | EIVLTQSPLSLPVTPEEPASISCRSSQSLLHSNGYNYLDWYLQKPGQS PQLLIYLGSNRASGVPDRFSGSGSGTDFTLKISRVEAEDVGVYYCMQA LQTPYTFGQGTKLEIK |
| **VL** | | |
| **149364-aa** | 1073 | MALPVTALLLPLALLLHAARPEVQLVESGGGLVKPGGSLRLSCAASGF TFSSYSMNWVRQAPGKGLEWVSSISSSSSYIYYADSVKGRFTISRDNA KNSLYLQMNSLRAEDTAVYYCAKTIAAVYAFDIWGQGTTVTVSSGGGG SGGGGSGGGGSEIVLTQSPLSLPVTPEEPASISCRSSQSLLHSNGYNY LDWYLQKPGQSPQLLIYLGSNRASGVPDRFSGSGSGTDFTLKISRVEA EDVGVYYCMQALQTPYTFGQGTKLEIKTTTPAPRPPTPAPTIASQPLS LRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNP QEGLYNELQKDKMAEAYSEIGMKGERRRGKGHDGLYQGLSTATKDTYD ALHMQALPPR |
| **Full CAR** | | |
| **149364-nt** | 1074 | ATGGCCCTCCCTGTCACCGCCCTGCTGCTTCCGCTGGCTCTTCTGCTC CACGCCGCTCGGCCCGAAGTGCAGCTTGTCGAATCCGGGGGGGGACTG GTCAAGCCGGGCGGATCACTGAGACTGTCCTGCGCCGCGAGCGGCTTC ACGTTCTCCTCCTACTCCATGAACTGGGTCCGCCAAGCCCCCGGGAAG GGACTGGAATGGGTGTCCTCTATCTCCTCGTCGTCGTCCTACATCTAC TACGCCGACTCCGTGAAGGGAAGATTCACCATTTCCCGCGACAACGCA AAGAACTCACTGTACTTGCAAATGAACTCACTCCGGGCCGAAGATACT GCTGTGTACTATTGCGCCAAGACTATTGCCGCCGTCTACGCTTTCGAC ATCTGGGGCCAGGGAACCACCGTGACTGTGTCGTCCGGTGGTGGTGGC TCGGGCGGAGGAGGAAGCGGCGGCGGGGGGTCCGAGATTGTGCTGACC CAGTCGCCACTGAGCCTCCCTGTGACCCCCGAGGAACCCGCCAGCATC AGCTGCCGGTCCAGCCAGTCCCTGCTCCACTCCAACGGATACAATTAC CTCGATTGGTACCTTCAGAAGCCTGGACAAAGCCCGCAGCTGCTCATC TACTTGGGATCAAACCGCGCGTCAGGAGTGCCTGACCGGTTCTCCGGC TCGGGCAGCGGTACCGATTTCACCCTGAAAATCTCCAGGGTGGAGGCA GAGGACGTGGGAGTGTATTACTGTATGCAGGCGCTGCAGACTCCGTAC ACATTTGGGCAGGGCACCAAGCTGGAGATCAAGACCACTACCCCAGCA CCGAGGCCACCCACCCCGGCTCCTACCATCGCCTCCCAGCCTCTGTCC CTGCGTCCGGAGGCATGTAGACCCGCAGCTGGTGGGGCCGTGCATACC CGGGGTCTTGACTTCGCCTGCGATATCTACATTTGGGCCCCTCTGGCT GGTACTTGCGGGGTCCTGCTGCTTTCACTCGTGATCACTCTTTACTGT AAGCGCGGTCGGAAGAAGCTGCTGTACATCTTTAAGCAACCCTTCATG AGGCCTGTGCAGACTACTCAAGAGGAGGACGGCTGTTCATGCCGGTTC CCAGAGGAGGAGGAAGGCGGCTGCGAACTGCGCGTGAAATTCAGCCGC AGCGCAGATGCTCCAGCCTACAAGCAGGGGCAGAACCAGCTCTACAAC GAACTCAATCTTGGTCGGAGAGAGGAGTACGACGTGCTGGACAAGCGG |
| **Full CAR** | | |
| | | AGAGGACGGGACCCAGAAATGGGCGGGAAGCCGCGCAGAAAGAATCCC CAAGAGGGCCTGTACAACGAGCTCCAAAAGGATAAGATGGCAGAAGCC TATAGCGAGATTGGTATGAAAGGGGAACGCAGAAGAGGCAAAGGCCAC GACGGACTGTACCAGGGACTCAGCACCGCCACCAAGGACACCTATGAC GCTCTTCACATGCAGGCCCTGCCGCCTCGG |

| **149365** | | |
|---|---|---|
| **149365-aa** | 1075 | EVQLVESGGGLVKPGGSLRLSCAASGFTFSDYYMSWIRQAPGKGLEWV SYISSSGSTIYYADSVKGRFTISRDNAKNSLYLQMNSLRAEDTAVYYC ARDLRGAFDIWGQGTMVTVSSGGGGSGGGGSGGGGSSYVLTQSPSVSA APGYTATISCGGNNIGTKSVHWYQQKPGQAPLLVIRDDSVRPSKIPGR FSGSNSGNMATLTISGVQAGDEADFYCQVWDSDSEHVVFGGGTKLTVL |
| **ScFv domain** | | |
| **149365-nt** | 1076 | GAAGTCCAGCTCGTGGAGTCCGGCGGAGGCCTTGTGAAGCCTGGAGGT TCGCTGAGACTGTCCTGCGCCGCCTCCGGCTTCACCTTCTCCGACTAC TACATGTCCTGGATCAGACAGGCCCCGGGAAAGGGCCTGGAATGGGTG TCCTACATCTCGTCATCGGGCAGCACTATCTACTACGCGGACTCAGTG AAGGGGCGGTTCACCATTTCCCGGGATAACGCGAAGAACTCGCTGTAT CTGCAAATGAACTCACTGAGGGCCGAGGACACCGCCGTGTACTACTGC GCCCGCGATCTCCGCGGGGCATTTGACATCTGGGGACAGGGAACCATG GTCACAGTGTCCAGCGGAGGGGGAGGATCGGGTGGCGGAGGTTCCGGG GGTGGAGGCTCCTCCTACGTGCTGACTCAGAGCCCAAGCGTCAGCGCT GCGCCCGGTTACACGGCAACCATCTCCTGTGGCGGAAACAACATTGGG ACCAAGTCTGTGCACTGGTATCAGCAGAAGCCGGGCCAAGCTCCCCTG TTGGTGATCCGCGATGACTCCGTGCGGCCTAGCAAAATTCCGGGACGG TTCTCCGGCTCCAACAGCGGCAATATGGCCACTCTCACCATCTCGGGA GTGCAGGCCGGAGATGAAGCCGACTTCTACTGCCAAGTCTGGGACTCA GACTCCGAGCATGTGGTGTTCGGGGGCGGAACCAAGCTGACTGTGCTC |
| **ScFv domain** | | |
| **149365-aa** | 1077 | EVQLVESGGGLVKPGGSLRLSCAASGFTFSDYYMSWIRQAPGKGLEWV SYISSSGSTIYYADSVKGRFTISRDNAKNSLYLQMNSLRAEDTAVYYC ARDLRGAFDIWGQGTMVTVSS |
| **VH** | | |
| **149365-aa** | 1078 | SYVLTQSPSVSAAPGYTATISCGGNNIGTKSVHWYQQKPGQAPLLVIR DDSVRPSKIPGRFSGSNSGNMATLTISGVQAGDEADFYCQVWDSDSEH VVFGGGTKLTVL |
| **VL** | | |
| **149365-aa** | 1079 | MALPVTALLLPLALLLHAARPEVQLVESGGGLVKPGGSLRLSCAASGF TFSDYYMSWIRQAPGKGLEWVSYISSSGSTIYYADSVKGRFTISRDNA KNSLYLQMNSLRAEDTAVYYCARDLRGAFDIWGQGTMVTVSSGGGGSG GGGSGGGGSSYVLTQSPSVSAAPGYTATISCGGNNIGTKSVHWYQQKP GQAPLLVIRDDSVRPSKIPGRFSGSNSGNMATLTISGVQAGDEADFYC QVWDSDSEHVVFGGGTKLTVLTTTPAPRPPTPAPTIASQPLSLRPEAC RPAAGGAVHTRGLDFACDIYIWAPLAGTCGVLLLSLVITLYCKRGRKK LLYIFKQPFMRPVQTTQEEDGCSCRFPEEEEGGCELRVKFSRSADAPA YKQGQNQLYNELNLGRREEYDVLDKRRGRDPEMGGKPRRKNPQEGLYN ELQKDKMAEAYSEIGMKGERRRGKGHDGLYQGLSTATKDTYDALHMQA |
| **Full CAR** | | |
| | | LPPR |
| **149365-nt** | 1080 | ATGGCCCTCCCTGTCACCGCCCTGCTGCTTCCGCTGGCTCTTCTGCTC CACGCCGCTCGGCCCGAAGTCCAGCTCGTGGAGTCCGGCGGAGGCCTT GTGAAGCCTGGAGGTTCGCTGAGACTGTCCTGCGCCGCCTCCGGCTTC ACCTTCTCCGACTACTACATGTCCTGGATCAGACAGGCCCCGGGAAAG GGCCTGGAATGGGTGTCCTACATCTCGTCATCGGGCAGCACTATCTAC TACGCGGACTCAGTGAAGGGGCGGTTCACCATTTCCCGGGATAACGCG AAGAACTCGCTGTATCTGCAAATGAACTCACTGAGGGCCGAGGACACC GCCGTGTACTACTGCGCCCGCGATCTCCGCGGGGCATTTGACATCTGG GGACAGGGAACCATGGTCACAGTGTCCAGCGGAGGGGGAGGATCGGGT GGCGGAGGTTCCGGGGGTGGAGGCTCCTCCTACGTGCTGACTCAGAGC CCAAGCGTCAGCGCTGCGCCCGGTTACACGGCAACCATCTCCTGTGGC GGAAACAACATTGGGACCAAGTCTGTGCACTGGTATCAGCAGAAGCCG GGCCAAGCTCCCCTGTTGGTGATCCGCGATGACTCCGTGCGGCCTAGC AAAATTCCGGGACGGTTCTCCGGCTCCAACAGCGGCAATATGGCCACT CTCACCATCTCGGGAGTGCAGGCCGGAGATGAAGCCGACTTCTACTGC CAAGTCTGGGACTCAGACTCCGAGCATGTGGTGTTCGGGGGCGGAACC AAGCTGACTGTGCTCACCACTACCCCAGCACCGAGGCCACCCACCCCG GCTCCTACCATCGCCTCCCAGCCTCTGTCCCTGCGTCCGGAGGCATGT AGACCCGCAGCTGGTGGGGCCGTGCATACCCGGGGTCTTGACTTCGCC TGCGATATCTACATTTGGGCCCCTCTGGCTGGTACTTGCGGGGTCCTG CTGCTTTCACTCGTGATCACTCTTTACTGTAAGCGCGGTCGGAAGAAG CTGCTGTACATCTTTAAGCAACCCTTCATGAGGCCTGTGCAGACTACT CAAGAGGAGGACGGCTGTTCATGCCGGTTCCCAGAGGAGGAGGAAGGC GGCTGCGAACTGCGCGTGAAATTCAGCCGCAGCGCAGATGCTCCAGCC TACAAGCAGGGGCAGAACCAGCTCTACAACGAACTCAATCTTGGTCGG AGAGAGGAGTACGACGTGCTGGACAAGCGGAGAGGACGGGACCCAGAA ATGGGCGGGAAGCCGCGCAGAAAGAATCCCCAAGAGGGCCTGTACAAC GAGCTCCAAAAGGATAAGATGGCAGAAGCCTATAGCGAGATTGGTATG AAAGGGGAACGCAGAAGAGGCAAAGGCCACGACGGACTGTACCAGGGA CTCAGCACCGCCACCAAGGACACCTATGACGCTCTTCACATGCAGGCC CTGCCGCCTCGG |
| **Full CAR** | | |

| 149366 | | |
|---|---|---|
| **149366-aa** | 1081 | QVQLVQSGAEVKKPGASVKVSCKPSGYTVTSHYIHWVRRAPGQGLEWM GMINPSGGVTAYSQTLQGRVTMTSDTSSSTVYMELSSLRSEDTAMYYC AREGSGSGWYFDFWGRGTLVTVSSGGGGSGGGGSGGGGSSYVLTQPPS VSVSPGQTASITCSGDGLSKKYVSWYQQKAGQSPVVLISRDKERPSGI PDRFSGSNSADTATLTISGTQAMDEADYYCQAWDDTTVVFGGGTKLTV L |
| **ScFv domain** | | |
| **149366-nt** | 1082 | CAAGTGCAGCTGGTGCAGAGCGGGGCCGAAGTCAAGAAGCCGGGAGCC TCCGTGAAAGTGTCCTGCAAGCCTTCGGGATACACCGTGACCTCCCAC TACATTCATTGGGTCCGCCGCGCCCCCGGCCAAGGACTCGAGTGGATG GGCATGATCAACCCTAGCGGCGGAGTGACCGCGTACAGCCAGACGCTG CAGGGACGCGTGACTATGACCTCGGATACCTCCTCCTCCACCGTCTAT |
| **ScFv domain** | | |
| | | ATGGAACTGTCCAGCCTGCGGTCCGAGGATACCGCCATGTACTACTGC GCCCGGGAAGGATCAGGCTCCGGGTGGTATTTCGACTTCTGGGGAAGA GGCACCCTCGTGACTGTGTCATCTGGGGGAGGGGGTTCCGGTGGTGGC GGATCGGGAGGAGGCGGTTCATCCTACGTGCTGACCCAGCCACCCTCC GTGTCCGTGAGCCCCGGCCAGACTGCATCGATTACATGTAGCGGCGAC GGCCTCTCCAAGAAATACGTGTCGTGGTACCAGCAGAAGGCCGGACAG AGCCCGGTGGTGCTGATCTCAAGAGATAAGGAGCGGCCTAGCGGAATC CCGGACAGGTTCTCGGGTTCCAACTCCGCGGACACTGCTACTCTGACC ATCTCGGGGACCCAGGCTATGGACGAAGCCGATTACTACTGCCAAGCC TGGGACGACACTACTGTCGTGTTTGGAGGGGGCACCAAGTTGACCGTC CTT |
| **149366-aa** | 1083 | QVQLVQSGAEVKKPGASVKVSCKPSGYTVTSHYIHWVRRAPGQGLEWM GMINPSGGVTAYSQTLQGRVTMTSDTSSSTVYMELSSLRSEDTAMYYC AREGSGSGWYFDFWGRGTLVTVSS |
| **VH** | | |
| **149366-aa** | 1084 | SYVLTQPPSVSVSPGQTASITCSGDGLSKKYVSWYQQKAGQSPVVLIS RDKERPSGIPDRFSGSNSADTATLTISGTQAMDEADYYCQAWDDTTVV FGGGTKLTVL |
| **VL** | | |
| **149366-aa** | 1085 | MALPVTALLLPLALLLHAARPQVQLVQSGAEVKKPGASVKVSCKPSGY TVTSHYIHWVRRAPGQGLEWMGMINPSGGVTAYSQTLQGRVTMTSDTS SSTVYMELSSLRSEDTAMYYCAREGSGSGWYFDFWGRGTLVTVSSGGG GSGGGGSGGGGSSYVLTQPPSVSVSPGQTASITCSGDGLSKKYVSWYQ QKAGQSPVVLISRDKERPSGIPDRFSGSNSADTATLTISGTQAMDEAD YYCQAWDDTTVVFGGGTKLTVLTTTPAPRPPTPAPTIASQPLSLRPEA CRPAAGGAVHTRGLDFACDIYIWAPLAGTCGVLLLSLVITLYCKRGRK KLLYIFKQPFMRPVQTTQEEDGCSCRFPEEEEGGCELRVKFSRSADAP AYKQGQNQLYNELNLGRREEYDVLDKRRGRDPEMGGKPRRKNPQEGLY NELQKDKMAEAYSEIGMKGERRRGKGHDGLYQGLSTATKDTYDALHMQ ALPPR |
| **Full CAR** | | |
| **149366-nt** | 1086 | ATGGCCCTCCCTGTCACCGCCCTGCTGCTTCCGCTGGCTCTTCTGCTC CACGCCGCTCGGCCCCAAGTGCAGCTGGTGCAGAGCGGGGCCGAAGTC AAGAAGCCGGGAGCCTCCGTGAAAGTGTCCTGCAAGCCTTCGGGATAC ACCGTGACCTCCCACTACATTCATTGGGTCCGCCGCGCCCCCGGCCAA GGACTCGAGTGGATGGGCATGATCAACCCTAGCGGCGGAGTGACCGCG TACAGCCAGACGCTGCAGGGACGCGTGACTATGACCTCGGATACCTCC TCCTCCACCGTCTATATGGAACTGTCCAGCCTGCGGTCCGAGGATACC GCCATGTACTACTGCGCCCGGGAAGGATCAGGCTCCGGGTGGTATTTC GACTTCTGGGGAAGAGGCACCCTCGTGACTGTGTCATCTGGGGGAGGG GGTTCCGGTGGTGGCGGATCGGGAGGAGGCGGTTCATCCTACGTGCTG ACCCAGCCACCCTCCGTGTCCGTGAGCCCCGGCCAGACTGCATCGATT ACATGTAGCGGCGACGGCCTCTCCAAGAAATACGTGTCGTGGTACCAG CAGAAGGCCGGACAGAGCCCGGTGGTGCTGATCTCAAGAGATAAGGAG CGGCCTAGCGGAATCCCGGACAGGTTCTCGGGTTCCAACTCCGCGGAC ACTGCTACTCTGACCATCTCGGGGACCCAGGCTATGGACGAAGCCGAT TACTACTGCCAAGCCTGGGACGACACTACTGTCGTGTTTGGAGGGGGC |
| **Full CAR** | | |
| | | ACCAAGTTGACCGTCCTTACCACTACCCCAGCACCGAGGCCACCCACC CCGGCTCCTACCATCGCCTCCCAGCCTCTGTCCCTGCGTCCGGAGGCA TGTAGACCCGCAGCTGGTGGGGCCGTGCATACCCGGGGTCTTGACTTC GCCTGCGATATCTACATTTGGGCCCCTCTGGCTGGTACTTGCGGGGTC CTGCTGCTTTCACTCGTGATCACTCTTTACTGTAAGCGCGGTCGGAAG AAGCTGCTGTACATCTTTAAGCAACCCTTCATGAGGCCTGTGCAGACT ACTCAAGAGGAGGACGGCTGTTCATGCCGGTTCCCAGAGGAGGAGGAA GGCGGCTGCGAACTGCGCGTGAAATTCAGCCGCAGCGCAGATGCTCCA GCCTACAAGCAGGGGCAGAACCAGCTCTACAACGAACTCAATCTTGGT CGGAGAGAGGAGTACGACGTGCTGGACAAGCGGAGAGGACGGGACCCA GAAATGGGCGGGAAGCCGCGCAGAAAGAATCCCCAAGAGGGCCTGTAC AACGAGCTCCAAAAGGATAAGATGGCAGAAGCCTATAGCGAGATTGGT ATGAAAGGGGAACGCAGAAGAGGCAAAGGCCACGACGGACTGTACCAG GGACTCAGCACCGCCACCAAGGACACCTATGACGCTCTTCACATGCAG GCCCTGCCGCCTCGG |

| **149367** | | |
|---|---|---|
| **149367-aa** | 1087 | QVQLQESGPGLVKPSQTLSLTCTVSGGSISSGGYYWSWIRQHPGKGLE WIGYIYYSGSTYYNPSLKSRVTISVDTSKNQFSLKLSSVTAADTAVYY CARAGIAARLRGAFDIWGQGTMVTVSSGGGGSGGGGSGGGGSDIVMTQ SPSSVSASVGDRVIITCRASQGIRNWLAWYQQKPGKAPNLLIYAASNL QSGVPSRFSGSGSGADFTLTISSLQPEDVATYYCQKYNSAPFTFGPGT KVDIK |
| **ScFv domain** | | |
| **149367-nt** | 1088 | CAAGTGCAGCTTCAGGAGAGCGGCCCGGGACTCGTGAAGCCGTCCCAG ACCCTGTCCCTGACTTGCACCGTGTCGGGAGGAAGCATCTCGAGCGGA GGCTACTATTGGTCGTGGATTCGGCAGCACCCTGGAAAGGGCCTGGAA TGGATCGGCTACATCTACTACTCCGGCTCGACCTACTACAACCCATCG CTGAAGTCCAGAGTGACAATCTCAGTGGACACGTCCAAGAATCAGTTC AGCCTGAAGCTCTCTTCCGTGACTGCGGCCGACACCGCCGTGTACTAC TGCGCACGCGCTGGAATTGCCGCCCGGCTGAGGGGTGCCTTCGACATT TGGGGACAGGGCACCATGGTCACCGTGTCCTCCGGCGGCGGAGGTTCC GGGGGTGGAGGCTCAGGAGGAGGGGGGTCCGACATCGTCATGACTCAG TCGCCCTCAAGCGTCAGCGCGTCCGTCGGGGACAGAGTGATCATCACC TGTCGGGCGTCCCAGGGAATTCGCAACTGGCTGGCCTGGTATCAGCAG AAGCCCGGAAAGGCCCCCAACCTGTTGATCTACGCCGCCTCAAACCTC CAATCCGGGGTGCCGAGCCGCTTCAGCGGCTCCGGTTCGGGTGCCGAT TTCACTCTGACCATCTCCTCCCTGCAACCTGAAGATGTGGCTACCTAC TACTGCCAAAAGTACAACTCCGCACCTTTTACTTTCGGACCGGGGACC AAAGTGGACATTAAG |
| **ScFv domain** | | |
| **149367-aa** | 1089 | QVQLQESGPGLVKPSQTLSLTCTVSGGSISSGGYYWSWIRQHPGKGLE WIGYIYYSGSTYYNPSLKSRVTISVDTSKNQFSLKLSSVTAADTAVYY CARAGIAARLRGAFDIWGQGTMVTVSS |
| **VH** | | |
| **149367-aa VL** | 1090 | DIVMTQSPSSVSASVGDRVIITCRASQGIRNWLAWYQQKPGKAPNLLI YAASNLQSGVPSRFSGSGSGADFTLTISSLQPEDVATYYCQKYNSAPF |
| | | TFGPGTKVDIK |
| **149367-aa** | 1091 | MALPVTALLLPLALLLHAARPQVQLQESGPGLVKPSQTLSLTCTVSGG SISSGGYYWSWIRQHPGKGLEWIGYIYYSGSTYYNPSLKSRVTISVDT SKNQFSLKLSSVTAADTAVYYCARAGIAARLRGAFDIWGQGTMVTVSS GGGGSGGGGSGGGGSDIVMTQSPSSVSASVGDRVIITCRASQGIRNWL AWYQQKPGKAPNLLIYAASNLQSGVPSRFSGSGSGADFTLTISSLQPE DVATYYCQKYNSAPFTFGPGTKVDIKTTTPAPRPPTPAPTIASQPLSL RPEACRPAAGGAVHTRGLDFACDIYIWAPLAGTCGVLLLSLVITLYCK RGRKKLLYIFKQPFMRPVQTTQEEDGCSCRFPEEEEGGCELRVKFSRS ADAPAYKQGQNQLYNELNLGRREEYDVLDKRRGRDPEMGGKPRRKNPQ EGLYNELQKDKMAEAYSEIGMKGERRRGKGHDGLYQGLSTATKDTYDA LHMQALPPR |
| **Full CAR** | | |
| **149367-nt** | 1092 | ATGGCCCTCCCTGTCACCGCCCTGCTGCTTCCGCTGGCTCTTCTGCTC CACGCCGCTCGGCCCCAAGTGCAGCTTCAGGAGAGCGGCCCGGGACTC GTGAAGCCGTCCCAGACCCTGTCCCTGACTTGCACCGTGTCGGGAGGA AGCATCTCGAGCGGAGGCTACTATTGGTCGTGGATTCGGCAGCACCCT GGAAAGGGCCTGGAATGGATCGGCTACATCTACTACTCCGGCTCGACC TACTACAACCCATCGCTGAAGTCCAGAGTGACAATCTCAGTGGACACG TCCAAGAATCAGTTCAGCCTGAAGCTCTCTTCCGTGACTGCGGCCGAC ACCGCCGTGTACTACTGCGCACGCGCTGGAATTGCCGCCCGGCTGAGG GGTGCCTTCGACATTTGGGGACAGGGCACCATGGTCACCGTGTCCTCC GGCGGCGGAGGTTCCGGGGGTGGAGGCTCAGGAGGAGGGGGGTCCGAC ATCGTCATGACTCAGTCGCCCTCAAGCGTCAGCGCGTCCGTCGGGGAC AGAGTGATCATCACCTGTCGGGCGTCCCAGGGAATTCGCAACTGGCTG GCCTGGTATCAGCAGAAGCCCGGAAAGGCCCCCAACCTGTTGATCTAC GCCGCCTCAAACCTCCAATCCGGGGTGCCGAGCCGCTTCAGCGGCTCC GGTTCGGGTGCCGATTTCACTCTGACCATCTCCTCCCTGCAACCTGAA GATGTGGCTACCTACTACTGCCAAAAGTACAACTCCGCACCTTTTACT TTCGGACCGGGGACCAAAGTGGACATTAAGACCACTACCCCAGCACCG AGGCCACCCACCCCGGCTCCTACCATCGCCTCCCAGCCTCTGTCCCTG CGTCCGGAGGCATGTAGACCCGCAGCTGGTGGGGCCGTGCATACCCGG GGTCTTGACTTCGCCTGCGATATCTACATTTGGGCCCCTCTGGCTGGT ACTTGCGGGGTCCTGCTGCTTTCACTCGTGATCACTCTTTACTGTAAG CGCGGTCGGAAGAAGCTGCTGTACATCTTTAAGCAACCCTTCATGAGG CCTGTGCAGACTACTCAAGAGGAGGACGGCTGTTCATGCCGGTTCCCA GAGGAGGAGGAAGGCGGCTGCGAACTGCGCGTGAAATTCAGCCGCAGC GCAGATGCTCCAGCCTACAAGCAGGGGCAGAACCAGCTCTACAACGAA CTCAATCTTGGTCGGAGAGAGGAGTACGACGTGCTGGACAAGCGGAGA GGACGGGACCCAGAAATGGGCGGGAAGCCGCGCAGAAAGAATCCCCAA GAGGGCCTGTACAACGAGCTCCAAAAGGATAAGATGGCAGAAGCCTAT AGCGAGATTGGTATGAAAGGGGAACGCAGAAGAGGCAAAGGCCACGAC GGACTGTACCAGGGACTCAGCACCGCCACCAAGGACACCTATGACGCT CTTCACATGCAGGCCCTGCCGCCTCGG |
| **Full CAR** | | |

| **149368** | | |
|---|---|---|
| **149368-aa** | 1093 | QVQLVQSGAEVKKPGSSVKVSCKASGGTFSSYAISWVRQAPGQGLEWM GGIIPIFGTANYAQKFQGRVTITADESTSTAYMELSSLRSEDTAVYYC ARRGGYQLLRWDVGLLRSAFDIWGQGTMVTVSSGGGGSGGGGSGGGGS SYVLTQPPSVSVAPGQTARITCGGNNIGSKSVHWYQQKPGQAPVLVLY GKNNRPSGVPDRFSGSRSGTTASLTITGAQAEDEADYYCSSRDSSGDH LRVFGTGTKVTVL |
| **ScFv domain** | | |
| **149368-nt** | 1094 | CAAGTGCAGCTGGTCCAGTCGGGCGCCGAGGTCAAGAAGCCCGGGAGC TCTGTGAAAGTGTCCTGCAAGGCCTCCGGGGGCACCTTTAGCTCCTAC GCCATCTCCTGGGTCCGCCAAGCACCGGGTCAAGGCCTGGAGTGGATG GGGGGAATTATCCCTATCTTCGGCACTGCCAACTACGCCCAGAAGTTC CAGGGACGCGTGACCATTACCGCGGACGAATCCACCTCCACCGCTTAT ATGGAGCTGTCCAGCTTGCGCTCGGAAGATACCGCCGTGTACTACTGC GCCCGGAGGGGTGGATACCAGCTGCTGAGATGGGACGTGGGCCTCCTG CGGTCGGCGTTCGACATCTGGGGCCAGGGCACTATGGTCACTGTGTCC AGCGGAGGAGGCGGATCGGGAGGCGGCGGATCAGGGGGAGGCGGTTCC AGCTACGTGCTTACTCAACCCCCTTCGGTGTCCGTGGCCCCGGGACAG ACCGCCAGAATCACTTGCGGAGGAAACAACATTGGGTCCAAGAGCGTG CATTGGTACCAGCAGAAGCCAGGACAGGCCCCTGTGCTGGTGCTCTAC GGGAAGAACAATCGGCCCAGCGGAGTGCCGGACAGGTTCTCGGGTTCA CGCTCCGGTACAACCGCTTCACTGACTATCACCGGGGCCCAGGCAGAG GATGAAGCGGACTACTACTGTTCCTCCCGGGATTCATCCGGCGACCAC CTCCGGGTGTTCGGAACCGGAACGAAGGTCACCGTGCTG |
| **ScFv domain** | | |
| **149368-aa** | 1095 | QVQLVQSGAEVKKPGSSVKVSCKASGGTFSSYAISWVRQAPGQGLEWM GGIIPIFGTANYAQKFQGRVTITADESTSTAYMELSSLRSEDTAVYYC ARRGGYQLLRWDVGLLRSAFDIWGQGTMVTVSS |
| **VH** | | |
| **149368-aa** | 1096 | SYVLTQPPSVSVAPGQTARITCGGNNIGSKSVHWYQQKPGQAPVLVLY GKNNRPSGVPDRFSGSRSGTTASLTITGAQAEDEADYYCSSRDSSGDH LRVFGTGTKVTVL |
| **VL** | | |
| **149368-aa** | 1097 | MALPVTALLLPLALLLHAARPQVQLVQSGAEVKKPGSSVKVSCKASGG TFSSYAISWVRQAPGQGLEWMGGIIPIFGTANYAQKFQGRVTITADES TSTAYMELSSLRSEDTAVYYCARRGGYQLLRWDVGLLRSAFDIWGQGT MVTVSSGGGGSGGGGSGGGGSSYVLTQPPSVSVAPGQTARITCGGNNI GSKSVHWYQQKPGQAPVLVLYGKNNRPSGVPDRFSGSRSGTTASLTIT GAQAEDEADYYCSSRDSSGDHLRVFGTGTKVTVLTTTPAPRPPTPAPT IASQPLSLRPEACRPAAGGAVHTRGLDFACDIYIWAPLAGTCGVLLLS LVITLYCKRGRKKLLYIFKQPFMRPVQTTQEEDGCSCRFPEEEEGGCE LRVKFSRSADAPAYKQGQNQLYNELNLGRREEYDVLDKRRGRDPEMGG KPRRKNPQEGLYNELQKDKMAEAYSEIGMKGERRRGKGHDGLYQGLST ATKDTYDALHMQALPPR |
| **Full CAR** | | |
| **149368-nt** | 1098 | ATGGCCCTCCCTGTCACCGCCCTGCTGCTTCCGCTGGCTCTTCTGCTC CACGCCGCTCGGCCCCAAGTGCAGCTGGTCCAGTCGGGCGCCGAGGTC AAGAAGCCCGGGAGCTCTGTGAAAGTGTCCTGCAAGGCCTCCGGGGGC ACCTTTAGCTCCTACGCCATCTCCTGGGTCCGCCAAGCACCGGGTCAA |
| **Full CAR** | | |
| | | GGCCTGGAGTGGATGGGGGGAATTATCCCTATCTTCGGCACTGCCAAC TACGCCCAGAAGTTCCAGGGACGCGTGACCATTACCGCGGACGAATCC ACCTCCACCGCTTATATGGAGCTGTCCAGCTTGCGCTCGGAAGATACC GCCGTGTACTACTGCGCCCGGAGGGGTGGATACCAGCTGCTGAGATGG GACGTGGGCCTCCTGCGGTCGGCGTTCGACATCTGGGGCCAGGGCACT ATGGTCACTGTGTCCAGCGGAGGAGGCGGATCGGGAGGCGGCGGATCA GGGGGAGGCGGTTCCAGCTACGTGCTTACTCAACCCCCTTCGGTGTCC GTGGCCCCGGGACAGACCGCCAGAATCACTTGCGGAGGAAACAACATT GGGTCCAAGAGCGTGCATTGGTACCAGCAGAAGCCAGGACAGGCCCCT GTGCTGGTGCTCTACGGGAAGAACAATCGGCCCAGCGGAGTGCCGGAC AGGTTCTCGGGTTCACGCTCCGGTACAACCGCTTCACTGACTATCACC GGGGCCCAGGCAGAGGATGAAGCGGACTACTACTGTTCCTCCCGGGAT TCATCCGGCGACCACCTCCGGGTGTTCGGAACCGGAACGAAGGTCACC GTGCTGACCACTACCCCAGCACCGAGGCCACCCACCCCGGCTCCTACC ATCGCCTCCCAGCCTCTGTCCCTGCGTCCGGAGGCATGTAGACCCGCA GCTGGTGGGGCCGTGCATACCCGGGGTCTTGACTTCGCCTGCGATATC TACATTTGGGCCCCTCTGGCTGGTACTTGCGGGGTCCTGCTGCTTTCA CTCGTGATCACTCTTTACTGTAAGCGCGGTCGGAAGAAGCTGCTGTAC ATCTTTAAGCAACCCTTCATGAGGCCTGTGCAGACTACTCAAGAGGAG GACGGCTGTTCATGCCGGTTCCCAGAGGAGGAGGAAGGCGGCTGCGAA CTGCGCGTGAAATTCAGCCGCAGCGCAGATGCTCCAGCCTACAAGCAG GGGCAGAACCAGCTCTACAACGAACTCAATCTTGGTCGGAGAGAGGAG TACGACGTGCTGGACAAGCGGAGAGGACGGGACCCAGAAATGGGCGGG AAGCCGCGCAGAAAGAATCCCCAAGAGGGCCTGTACAACGAGCTCCAA AAGGATAAGATGGCAGAAGCCTATAGCGAGATTGGTATGAAAGGGGAA CGCAGAAGAGGCAAAGGCCACGACGGACTGTACCAGGGACTCAGCACC GCCACCAAGGACACCTATGACGCTCTTCACATGCAGGCCCTGCCGCCT CGG |

| **149369** | | |
|---|---|---|
| **149369-aa** | 1099 | EVQLQQSGPGLVKPSQTLSLTCAISGDSVSSNSAAWNWIRQSPSRGLE WLGRTYYRSKWYSFYAISLKSRIIINPDTSKNQFSLQLKSVTPEDTAV YYCARSSPEGLFLYWFDPWGQGTLVTVSSGGDGSGGGGSGGGGSSSEL TQDPAVSVALGQTIRITCQGDSLGNYYATWYQQKPGQAPVLVIYGTNN RPSGIPDRFSASSSGNTASLTITGAQAEDEADYYCNSRDSSGHHLLFG TGTKVTVL |
| **ScFv domain** | | |
| **149369-nt** | 1100 | GAAGTGCAGCTCCAACAGTCAGGACCGGGGCTCGTGAAGCCATCCCAG ACCCTGTCCCTGACTTGTGCCATCTCGGGAGATAGCGTGTCATCGAAC TCCGCCGCCTGGAACTGGATTCGGCAGAGCCCGTCCCGCGGACTGGAG TGGCTTGGAAGGACCTACTACCGGTCCAAGTGGTACTCTTTCTACGCG ATCTCGCTGAAGTCCCGCATTATCATTAACCCTGATACCTCCAAGAAT CAGTTCTCCCTCCAACTGAAATCCGTCACCCCCGAGGACACAGCAGTG TATTACTGCGCACGGAGCAGCCCCGAAGGACTGTTCCTGTATTGGTTT GACCCCTGGGGCCAGGGGACTCTTGTGACCGTGTCGAGCGGCGGAGAT GGGTCCGGTGGCGGTGGTTCGGGGGGCGGCGGATCATCATCCGAACTG ACCCAGGACCCGGCTGTGTCCGTGGCGCTGGGACAAACCATCCGCATT |
| **ScFv domain** | | |
| | | ACGTGCCAGGGAGACTCCCTGGGCAACTACTACGCCACTTGGTACCAG CAGAAGCCGGGCCAAGCCCCTGTGTTGGTCATCTACGGGACCAACAAC AGACCTTCCGGCATCCCCGACCGGTTCAGCGCTTCGTCCTCCGGCAAC ACTGCCAGCCTGACCATCACTGGAGCGCAGGCCGAAGATGAGGCCGAC TACTACTGCAACAGCAGAGACTCCTCGGGTCATCACCTCTTGTTCGGA ACTGGAACCAAGGTCACCGTGCTG |
| **149369-aa** | 1101 | EVQLQQSGPGLVKPSQTLSLTCAISGDSVSSNSAAWNWIRQSPSRGLE WLGRTYYRSKWYSFYAISLKSRIIINPDTSKNQFSLQLKSVTPEDTAV YYCARSSPEGLFLYWFDPWGQGTLVTVSS |
| **VH** | | |
| **149369-aa** | 1102 | SSELTQDPAVSVALGQTIRITCQGDSLGNYYATWYQQKPGQAPVLVIY GTNNRPSGIPDRFSASSSGNTASLTITGAQAEDEADYYCNSRDSSGHH LLFGTGTKVTVL |
| **VL** | | |
| **149369-aa** | 1103 | MALPVTALLLPLALLLHAARPEVQLQQSGPGLVKPSQTLSLTCAISGD SVSSNSAAWNWIRQSPSRGLEWLGRTYYRSKWYSFYAISLKSRIIINP DTSKNQFSLQLKSVTPEDTAVYYCARSSPEGLFLYWFDPWGQGTLVTV SSGGDGSGGGGSGGGGSSSELTQDPAVSVALGQTIRITCQGDSLGNYY ATWYQQKPGQAPVLVIYGTNNRPSGIPDRFSASSSGNTASLTITGAQA EDEADYYCNSRDSSGHHLLFGTGTKVTVLTTTPAPRPPTPAPTIASQP LSLRPEACRPAAGGAVHTRGLDFACDIYIWAPLAGTCGVLLLSLVITL YCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRK NPQEGLYNELQKDKMAEAYSEIGMKGERRRGKGHDGLYQGLSTATKDT YDALHMQALPPR |
| **Full CAR** | | |
| **149369-nt** | 1104 | ATGGCCCTCCCTGTCACCGCCCTGCTGCTTCCGCTGGCTCTTCTGCTC CACGCCGCTCGGCCCGAAGTGCAGCTCCAACAGTCAGGACCGGGGCTC GTGAAGCCATCCCAGACCCTGTCCCTGACTTGTGCCATCTCGGGAGAT AGCGTGTCATCGAACTCCGCCGCCTGGAACTGGATTCGGCAGAGCCCG TCCCGCGGACTGGAGTGGCTTGGAAGGACCTACTACCGGTCCAAGTGG TACTCTTTCTACGCGATCTCGCTGAAGTCCCGCATTATCATTAACCCT GATACCTCCAAGAATCAGTTCTCCCTCCAACTGAAATCCGTCACCCCC GAGGACACAGCAGTGTATTACTGCGCACGGAGCAGCCCCGAAGGACTG TTCCTGTATTGGTTTGACCCCTGGGGCCAGGGGACTCTTGTGACCGTG TCGAGCGGCGGAGATGGGTCCGGTGGCGGTGGTTCGGGGGGCGGCGGA TCATCATCCGAACTGACCCAGGACCCGGCTGTGTCCGTGGCGCTGGGA CAAACCATCCGCATTACGTGCCAGGGAGACTCCCTGGGCAACTACTAC GCCACTTGGTACCAGCAGAAGCCGGGCCAAGCCCCTGTGTTGGTCATC TACGGGACCAACAACAGACCTTCCGGCATCCCCGACCGGTTCAGCGCT TCGTCCTCCGGCAACACTGCCAGCCTGACCATCACTGGAGCGCAGGCC GAAGATGAGGCCGACTACTACTGCAACAGCAGAGACTCCTCGGGTCAT CACCTCTTGTTCGGAACTGGAACCAAGGTCACCGTGCTGACCACTACC CCAGCACCGAGGCCACCCACCCCGGCTCCTACCATCGCCTCCCAGCCT CTGTCCCTGCGTCCGGAGGCATGTAGACCCGCAGCTGGTGGGGCCGTG CATACCCGGGGTCTTGACTTCGCCTGCGATATCTACATTTGGGCCCCT CTGGCTGGTACTTGCGGGGTCCTGCTGCTTTCACTCGTGATCACTCTT |
| **Full CAR** | | |
| | | TACTGTAAGCGCGGTCGGAAGAAGCTGCTGTACATCTTTAAGCAACCC TTCATGAGGCCTGTGCAGACTACTCAAGAGGAGGACGGCTGTTCATGC CGGTTCCCAGAGGAGGAGGAAGGCGGCTGCGAACTGCGCGTGAAATTC AGCCGCAGCGCAGATGCTCCAGCCTACAAGCAGGGGCAGAACCAGCTC TACAACGAACTCAATCTTGGTCGGAGAGAGGAGTACGACGTGCTGGAC AAGCGGAGAGGACGGGACCCAGAAATGGGCGGGAAGCCGCGCAGAAAG AATCCCCAAGAGGGCCTGTACAACGAGCTCCAAAAGGATAAGATGGCA GAAGCCTATAGCGAGATTGGTATGAAAGGGGAACGCAGAAGAGGCAAA GGCCACGACGGACTGTACCAGGGACTCAGCACCGCCACCAAGGACACC TATGACGCTCTTCACATGCAGGCCCTGCCGCCTCGG |

| **BCMA_EBB-C1978-A4** | | |
|---|---|---|
| **BCMA_EBB-C1978-A4 - aa** | 1105 | EVQLVESGGGLVQPGGSLRLSCAASGFTFSSYAMSWVRQAPGKGLEWV SAISGSGGSTYYADSVKGRFTISRDNSKNTLYLQMNSLRAEDTAVYYC AKVEGSGSLDYWGQGTLVTVSSGGGGSGGGGSGGGGSEIVMTQSPGTL SLSPGERATLSCRASQSVSSAYLAWYQQKPGQPPRLLISGASTRATGI PDRFGGSGSGTDFTLTISRLEPEDFAVYYCQHYGSSFNGSSLFTFGQG TRLEIK |
| **ScFv domain** | | |
| **BCMA_EBB-C1978-A4 - nt** | 1106 | GAAGTGCAGCTCGTGGAGTCAGGAGGCGGCCTGGTCCAGCCGGGAGGG TCCCTTAGACTGTCATGCGCCGCAAGCGGATTCACTTTCTCCTCCTAT GCCATGAGCTGGGTCCGCCAAGCCCCCGGAAAGGGACTGGAATGGGTG TCCGCCATCTCGGGGTCTGGAGGCTCAACTTACTACGCTGACTCCGTG AAGGGACGGTTCACCATTAGCCGCGACAACTCCAAGAACACCCTCTAC CTCCAAATGAACTCCCTGCGGGCCGAGGATACCGCCGTCTACTACTGC GCCAAAGTGGAAGGTTCAGGATCGCTGGACTACTGGGGACAGGGTACT CTCGTGACCGTGTCATCGGGCGGAGGAGGTTCCGGCGGTGGCGGCTCC GGCGGCGGAGGGTCGGAGATCGTGATGACCCAGAGCCCTGGTACTCTG AGCCTTTCGCCGGGAGAAAGGGCCACCCTGTCCTGCCGCGCTTCCCAA TCCGTGTCCTCCGCGTACTTGGCGTGGTACCAGCAGAAGCCGGGACAG CCCCCTCGGCTGCTGATCAGCGGGGCCAGCACCCGGGCAACCGGAATC CCAGACAGATTCGGGGGTTCCGGCAGCGGCACAGATTTCACCCTGACT ATTTCGAGGTTGGAGCCCGAGGACTTTGCGGTGTATTACTGTCAGCAC TACGGGTCGTCCTTTAATGGCTCCAGCCTGTTCACGTTCGGACAGGGG ACCCGCCTGGAAATCAAG |
| **ScFv domain** | | |
| **BCMA_EBB-C1978-A4 - aa** | 1107 | EVQLVESGGGLVQPGGSLRLSCAASGFTFSSYAMSWVRQAPGKGLEWV SAISGSGGSTYYADSVKGRFTISRDNSKNTLYLQMNSLRAEDTAVYYC AKVEGSGSLDYWGQGTLVTVSS |
| **VH** | | |
| **BCMA_EBB-C1978-A4 - aa VL** | 1108 | EIVMTQSPGTLSLSPGERATLSCRASQSVSSAYLAWYQQKPGQPPRLL ISGASTRATGIPDRFGGSGSGTDFTLTISRLEPEDFAVYYCQHYGSSF NGSSLFTFGQGTRLEIK |
| **BCMA_EBB-C1978-A4 - aa** | 1109 | MALPVTALLLPLALLLHAARPEVQLVESGGGLVQPGGSLRLSCAASGF TFSSYAMSWVRQAPGKGLEWVSAISGSGGSTYYADSVKGRFTISRDNS KNTLYLQMNSLRAEDTAVYYCAKVEGSGSLDYWGQGTLVTVSSGGGGS GGGGSGGGGSEIVMTQSPGTLSLSPGERATLSCRASQSVSSAYLAWYQ QKPGQPPRLLISGASTRATGIPDRFGGSGSGTDFTLTISRLEPEDFAV YYCQHYGSSFNGSSLFTFGQGTRLEIKTTTPAPRPPTPAPTIASQPLS LRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNP QEGLYNELQKDKMAEAYSEIGMKGERRRGKGHDGLYQGLSTATKDTYD ALHMQALPPR |
| **Full CART** | | |
| **BCMA_EBB-C1978-A4 - nt** | 1110 | ATGGCCCTCCCTGTCACCGCCCTGCTGCTTCCGCTGGCTCTTCTGCTC CACGCCGCTCGGCCCGAAGTGCAGCTCGTGGAGTCAGGAGGCGGCCTG GTCCAGCCGGGAGGGTCCCTTAGACTGTCATGCGCCGCAAGCGGATTC ACTTTCTCCTCCTATGCCATGAGCTGGGTCCGCCAAGCCCCCGGAAAG GGACTGGAATGGGTGTCCGCCATCTCGGGGTCTGGAGGCTCAACTTAC TACGCTGACTCCGTGAAGGGACGGTTCACCATTAGCCGCGACAACTCC AAGAACACCCTCTACCTCCAAATGAACTCCCTGCGGGCCGAGGATACC GCCGTCTACTACTGCGCCAAAGTGGAAGGTTCAGGATCGCTGGACTAC TGGGGACAGGGTACTCTCGTGACCGTGTCATCGGGCGGAGGAGGTTCC GGCGGTGGCGGCTCCGGCGGCGGAGGGTCGGAGATCGTGATGACCCAG AGCCCTGGTACTCTGAGCCTTTCGCCGGGAGAAAGGGCCACCCTGTCC TGCCGCGCTTCCCAATCCGTGTCCTCCGCGTACTTGGCGTGGTACCAG CAGAAGCCGGGACAGCCCCCTCGGCTGCTGATCAGCGGGGCCAGCACC CGGGCAACCGGAATCCCAGACAGATTCGGGGGTTCCGGCAGCGGCACA GATTTCACCCTGACTATTTCGAGGTTGGAGCCCGAGGACTTTGCGGTG TATTACTGTCAGCACTACGGGTCGTCCTTTAATGGCTCCAGCCTGTTC ACGTTCGGACAGGGGACCCGCCTGGAAATCAAGACCACTACCCCAGCA CCGAGGCCACCCACCCCGGCTCCTACCATCGCCTCCCAGCCTCTGTCC CTGCGTCCGGAGGCATGTAGACCCGCAGCTGGTGGGGCCGTGCATACC CGGGGTCTTGACTTCGCCTGCGATATCTACATTTGGGCCCCTCTGGCT GGTACTTGCGGGGTCCTGCTGCTTTCACTCGTGATCACTCTTTACTGT AAGCGCGGTCGGAAGAAGCTGCTGTACATCTTTAAGCAACCCTTCATG AGGCCTGTGCAGACTACTCAAGAGGAGGACGGCTGTTCATGCCGGTTC CCAGAGGAGGAGGAAGGCGGCTGCGAACTGCGCGTGAAATTCAGCCGC AGCGCAGATGCTCCAGCCTACAAGCAGGGGCAGAACCAGCTCTACAAC GAACTCAATCTTGGTCGGAGAGAGGAGTACGACGTGCTGGACAAGCGG AGAGGACGGGACCCAGAAATGGGCGGGAAGCCGCGCAGAAAGAATCCC CAAGAGGGCCTGTACAACGAGCTCCAAAAGGATAAGATGGCAGAAGCC TATAGCGAGATTGGTATGAAAGGGGAACGCAGAAGAGGCAAAGGCCAC GACGGACTGTACCAGGGACTCAGCACCGCCACCAAGGACACCTATGAC GCTCTTCACATGCAGGCCCTGCCGCCTCGG |
| **Full CART** | | |

| **BCMA_EBB-C1978-G1** | | |
|---|---|---|
| **BCMA_EBB-C1978-G1-aa** | 1111 | EVQLVETGGGLVQPGGSLRLSCAASGITFSRYPMSWVRQAPGKGLEWVSGISD SGVSTYYADSAKGRFTISRDNSKNTLFLQMSSLRDEDTAVYYCVTRAGSEASDI WGQGTMVTVSSGGGGSGGGGSGGGGSEIVLTQSPATLSLSPGERATLSCRAS QSVSNSLAWYQQKPGQAPRLLIYDASSRATGIPDRFSGSGSGTDFTLTISRLEPE DFAIYYCQQFGTSSGLTFGGGTKLEIK |
| **ScFv domain** | | |
| **BCMA_EBB-C1978-G1-nt** | 1112 | GAAGTGCAACTGGTGGAAACCGGTGGCGGCCTGGTGCAGCCTGGAGGA TCATTGAGGCTGTCATGCGCGGCCAGCGGTATTACCTTCTCCCGGTAC CCCATGTCCTGGGTCAGACAGGCCCCGGGGAAAGGGCTTGAATGGGTG TCCGGGATCTCGGACTCCGGTGTCAGCACTTACTACGCCGACTCCGCC AAGGGACGCTTCACCATTTCCCGGGACAACTCGAAGAACACCCTGTTC CTCCAAATGAGCTCCCTCCGGGACGAGGATACTGCAGTGTACTACTGC GTGACCCGCGCCGGGTCCGAGGCGTCTGACATTTGGGGACAGGGCACT ATGGTCACCGTGTCGTCCGGCGGAGGGGGCTCGGGAGGCGGTGGCAGC GGAGGAGGAGGGTCCGAGATCGTGCTGACCCAATCCCCGGCCACCCTC TCGCTGAGCCCTGGAGAAAGGGCAACCTTGTCCTGTCGCGCGAGCCAG TCCGTGAGCAACTCCCTGGCCTGGTACCAGCAGAAGCCCGGACAGGCT CCGAGACTTCTGATCTACGACGCTTCGAGCCGGGCCACTGGAATCCCC GACCGCTTTTCGGGGTCCGGCTCAGGAACCGATTTCACCCTGACAATC TCACGGCTGGAGCCAGAGGATTTCGCCATCTATTACTGCCAGCAGTTC GGTACTTCCTCCGGCCTGACTTTCGGAGGCGGCACGAAGCTCGAAATC AAG |
| **ScFv domain** | | |
| **BCMA_EBB-C1978-G1-aa** | 1113 | EVQLVETGGGLVQPGGSLRLSCAASGITFSRYPMSWVRQAPGKGLEWV SGISDSGVSTYYADSAKGRFTISRDNSKNTLFLQMSSLRDEDTAVYYC VTRAGSEASDIWGQGTMVTVSS |
| **VH** | | |
| **BCMA_EBB-C1978-G1-aa** | 1114 | EIVLTQSPATLSLSPGERATLSCRASQSVSNSLAWYQQKPGQAPRLLI YDASSRATGIPDRFSGSGSGTDFTLTISRLEPEDFAIYYCQQFGTSSG LTFGGGTKLEIK |
| **VL** | | |
| **BCMA_EBB-C1978-G1-aa** | 1115 | MALPVTALLLPLALLLHAARPEVQLVETGGGLVQPGGSLRLSCAASGITFSRYPM SWVRQAPGKGLEWVSGISDSGVSTYYADSAKGRFTISRDNSKNTLFLQMSSLR DEDTAVYYCVTRAGSEASDIWGQGTMVTVSSGGGGSGGGGSGGGGSEIVLTQ SPATLSLSPGERATLSCRASQSVSNSLAWYQQKPGQAPRLLIYDASSRATGIPDR FSGSGSGTDFTLTISRLEPEDFAIYYCQQFGTSSGLTFGGGTKLEIKTTTPAPRPPT PAPTIASQPLSLRPEACRPAAGGAVHTRGLDFACDIYIWAPLAGTCGVLLLSLVIT LYCKRGRKKLLYIFKQPFMRPVQTTQEEDGCSCRFPEEEEGGCELRVKFSRSADA PAYKQGQNQLYNELNLGRREEYDVLDKRRGRDPEMGGKPRRKNPQEGLYNEL QKDKMAEAYSEIGMKGERRRGKGHDGLYQGLSTATKDTYDALHMQALPPR |
| **Full CART** | | |
| **BCMA_EBB-C1978-G1-nt Full CART** | 1116 | ATGGCCCTCCCTGTCACCGCCCTGCTGCTTCCGCTGGCTCTTCTGCTC CACGCCGCTCGGCCCGAAGTGCAACTGGTGGAAACCGGTGGCGGCCTG |
| | | GTGCAGCCTGGAGGATCATTGAGGCTGTCATGCGCGGCCAGCGGTATT ACCTTCTCCCGGTACCCCATGTCCTGGGTCAGACAGGCCCCGGGGAAA GGGCTTGAATGGGTGTCCGGGATCTCGGACTCCGGTGTCAGCACTTAC TACGCCGACTCCGCCAAGGGACGCTTCACCATTTCCCGGGACAACTCG AAGAACACCCTGTTCCTCCAAATGAGCTCCCTCCGGGACGAGGATACT GCAGTGTACTACTGCGTGACCCGCGCCGGGTCCGAGGCGTCTGACATT TGGGGACAGGGCACTATGGTCACCGTGTCGTCCGGCGGAGGGGGCTCG GGAGGCGGTGGCAGCGGAGGAGGAGGGTCCGAGATCGTGCTGACCCAA TCCCCGGCCACCCTCTCGCTGAGCCCTGGAGAAAGGGCAACCTTGTCC TGTCGCGCGAGCCAGTCCGTGAGCAACTCCCTGGCCTGGTACCAGCAG AAGCCCGGACAGGCTCCGAGACTTCTGATCTACGACGCTTCGAGCCGG GCCACTGGAATCCCCGACCGCTTTTCGGGGTCCGGCTCAGGAACCGAT TTCACCCTGACAATCTCACGGCTGGAGCCAGAGGATTTCGCCATCTAT TACTGCCAGCAGTTCGGTACTTCCTCCGGCCTGACTTTCGGAGGCGGC ACGAAGCTCGAAATCAAGACCACTACCCCAGCACCGAGGCCACCCACC CCGGCTCCTACCATCGCCTCCCAGCCTCTGTCCCTGCGTCCGGAGGCA TGTAGACCCGCAGCTGGTGGGGCCGTGCATACCCGGGGTCTTGACTTC GCCTGCGATATCTACATTTGGGCCCCTCTGGCTGGTACTTGCGGGGTC CTGCTGCTTTCACTCGTGATCACTCTTTACTGTAAGCGCGGTCGGAAG AAGCTGCTGTACATCTTTAAGCAACCCTTCATGAGGCCTGTGCAGACT ACTCAAGAGGAGGACGGCTGTTCATGCCGGTTCCCAGAGGAGGAGGAA GGCGGCTGCGAACTGCGCGTGAAATTCAGCCGCAGCGCAGATGCTCCA GCCTACAAGCAGGGGCAGAACCAGCTCTACAACGAACTCAATCTTGGT CGGAGAGAGGAGTACGACGTGCTGGACAAGCGGAGAGGACGGGACCCA GAAATGGGCGGGAAGCCGCGCAGAAAGAATCCCCAAGAGGGCCTGTAC AACGAGCTCCAAAAGGATAAGATGGCAGAAGCCTATAGCGAGATTGGT ATGAAAGGGGAACGCAGAAGAGGCAAAGGCCACGACGGACTGTACCAG GGACTCAGCACCGCCACCAAGGACACCTATGACGCTCTTCACATGCAG GCCCTGCCGCCTCGG |

| **BCMA_EBB-C1979-C1** | | |
|---|---|---|
| **BCMA_EBB-C1979-C1-aa** | 1117 | QVQLVESGGGLVQPGGSLRLSCAASGFTFSSYAMSWVRQAPGKGLEWV SAISGSGGSTYYADSVKGRFTISRDNAKNSLYLQMNSLRAEDTAIYYC ARATYKRELRYYYGMDVWGQGTMVTVSSGGGGSGGGGSGGGGSEIVMT QSPGTVSLSPGERATLSCRASQSVSSSFLAWYQQKPGQAPRLLIYGAS SRATGIPDRFSGSGSGTDFTLTISRLEPEDSAVYYCQQYHSSPSWTFG QGTRLEIK |
| **ScFv domain** | | |
| **BCMA_EBB-C1979-C1 - nt** | 1118 | CAAGTGCAGCTCGTGGAATCGGGTGGCGGACTGGTGCAGCCGGGGGGC TCACTTAGACTGTCCTGCGCGGCCAGCGGATTCACTTTCTCCTCCTAC GCCATGTCCTGGGTCAGACAGGCCCCTGGAAAGGGCCTGGAATGGGTG TCCGCAATCAGCGGCAGCGGCGGCTCGACCTATTACGCGGATTCAGTG AAGGGCAGATTCACCATTTCCCGGGACAACGCCAAGAACTCCTTGTAC CTTCAAATGAACTCCCTCCGCGCGGAAGATACCGCAATCTACTACTGC GCTCGGGCCACTTACAAGAGGGAACTGCGCTACTACTACGGGATGGAC GTCTGGGGCCAGGGAACCATGGTCACCGTGTCCAGCGGAGGAGGAGGA TCGGGAGGAGGCGGTAGCGGGGGTGGAGGGTCGGAGATCGTGATGACC |
| **ScFv domain** | | |
| | | CAGTCCCCCGGCACTGTGTCGCTGTCCCCCGGCGAACGGGCCACCCTG TCATGTCGGGCCAGCCAGTCAGTGTCGTCAAGCTTCCTCGCCTGGTAC CAGCAGAAACCGGGACAAGCTCCCCGCCTGCTGATCTACGGAGCCAGC AGCCGGGCCACCGGTATTCCTGACCGGTTCTCCGGTTCGGGGTCCGGG ACCGACTTTACTCTGACTATCTCTCGCCTCGAGCCAGAGGACTCCGCC GTGTATTACTGCCAGCAGTACCACTCCTCCCCGTCCTGGACGTTCGGA CAGGGCACAAGGCTGGAGATTAAG |
| **BCMA_EBB-C1979-C1-aa** | 1119 | QVQLVESGGGLVQPGGSLRLSCAASGFTFSSYAMSWVRQAPGKGLEWV SAISGSGGSTYYADSVKGRFTISRDNAKNSLYLQMNSLRAEDTAIYYC ARATYKRELRYYYGMDVWGQGTMVTVSS |
| **VH** | | |
| **BCMA_EBB-C1979-C1-aa** | 1120 | EIVMTQSPGTVSLSPGERATLSCRASQSVSSSFLAWYQQKPGQAPRLL IYGASSRATGIPDRFSGSGSGTDFTLTISRLEPEDSAVYYCQQYHSSP SWTFGQGTRLEIK |
| **VL** | | |
| **BCMA_EBB-C1979-C1-aa** | 1121 | MALPVTALLLPLALLLHAARPQVQLVESGGGLVQPGGSLRLSCAASGF TFSSYAMSWVRQAPGKGLEWVSAISGSGGSTYYADSVKGRFTISRDNA KNSLYLQMNSLRAEDTAIYYCARATYKRELRYYYGMDVWGQGTMVTVS SGGGGSGGGGSGGGGSEIVMTQSPGTVSLSPGERATLSCRASQSVSSS FLAWYQQKPGQAPRLLIYGASSRATGIPDRFSGSGSGTDFTLTISRLE PEDSAVYYCQQYHSSPSWTFGQGTRLEIKTTTPAPRPPTPAPTIASQP LSLRPEACRPAAGGAVHTRGLDFACDIYIWAPLAGTCGVLLLSLVITL YCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRK NPQEGLYNELQKDKMAEAYSEIGMKGERRRGKGHDGLYQGLSTATKDT YDALHMQALPPR |
| **Full CART** | | |
| **BCMA_EBB-C1979-C1 - nt** | 1122 | ATGGCCCTCCCTGTCACCGCCCTGCTGCTTCCGCTGGCTCTTCTGCTC CACGCCGCTCGGCCCCAAGTGCAGCTCGTGGAATCGGGTGGCGGACTG GTGCAGCCGGGGGGCTCACTTAGACTGTCCTGCGCGGCCAGCGGATTC ACTTTCTCCTCCTACGCCATGTCCTGGGTCAGACAGGCCCCTGGAAAG GGCCTGGAATGGGTGTCCGCAATCAGCGGCAGCGGCGGCTCGACCTAT TACGCGGATTCAGTGAAGGGCAGATTCACCATTTCCCGGGACAACGCC AAGAACTCCTTGTACCTTCAAATGAACTCCCTCCGCGCGGAAGATACC GCAATCTACTACTGCGCTCGGGCCACTTACAAGAGGGAACTGCGCTAC TACTACGGGATGGACGTCTGGGGCCAGGGAACCATGGTCACCGTGTCC AGCGGAGGAGGAGGATCGGGAGGAGGCGGTAGCGGGGGTGGAGGGTCG GAGATCGTGATGACCCAGTCCCCCGGCACTGTGTCGCTGTCCCCCGGC GAACGGGCCACCCTGTCATGTCGGGCCAGCCAGTCAGTGTCGTCAAGC TTCCTCGCCTGGTACCAGCAGAAACCGGGACAAGCTCCCCGCCTGCTG ATCTACGGAGCCAGCAGCCGGGCCACCGGTATTCCTGACCGGTTCTCC GGTTCGGGGTCCGGGACCGACTTTACTCTGACTATCTCTCGCCTCGAG CCAGAGGACTCCGCCGTGTATTACTGCCAGCAGTACCACTCCTCCCCG |
| **Full CART** | | |
| | | TCCTGGACGTTCGGACAGGGCACAAGGCTGGAGATTAAGACCACTACC CCAGCACCGAGGCCACCCACCCCGGCTCCTACCATCGCCTCCCAGCCT CTGTCCCTGCGTCCGGAGGCATGTAGACCCGCAGCTGGTGGGGCCGTG CATACCCGGGGTCTTGACTTCGCCTGCGATATCTACATTTGGCCCCCT CTGGCTGGTACTTGCGGGGTCCTGCTGCTTTCACTCGTGATCACTCTT TACTGTAAGCGCGGTCGGAAGAAGCTGCTGTACATCTTTAAGCAACCC TTCATGAGGCCTGTGCAGACTACTCAAGAGGAGGACGGCTGTTCATGC CGGTTCCCAGAGGAGGAGGAAGGCGGCTGCGAACTGCGCGTGAAATTC AGCCGCAGCGCAGATGCTCCAGCCTACAAGCAGGGGCAGAACCAGCTC TACAACGAACTCAATCTTGGTCGGAGAGAGGAGTACGACGTGCTGGAC AAGCGGAGAGGACGGGACCCAGAAATGGGCGGGAAGCCGCGCAGAAAG AATCCCCAAGAGGGCCTGTACAACGAGCTCCAAAAGGATAAGATGGCA GAAGCCTATAGCGAGATTGGTATGAAAGGGGAACGCAGAAGAGGCAAA GGCCACGACGGACTGTACCAGGGACTCAGCACCGCCACCAAGGACACC TATGACGCTCTTCACATGCAGGCCCTGCCGCCTCGG |

| **BCMA_EBB-C1978-C7** | | |
|---|---|---|
| **BCMA_EBB-C1978-C7 - aa** | 1123 | EVQLVETGGGLVQPGGSLRLSCAASGFTFSSYAMSWVRQAPGKGLEWV SAISGSGGSTYYADSVKGRFTISRDNSKNTLYLQMNTLKAEDTAVYYC ARATYKRELRYYYGMDVWGQGTTVTVSSGGGGSGGGGSGGGGSEIVLT QSPSTLSLSPGESATLSCRASQSVSTTFLAWYQQKPGQAPRLLIYGSS NRATGIPDRFSGSGSGTDFTLTIRRLEPEDFAVYYCQQYHSSPSWTFG QGTKVEIK |
| **ScFv domain** | | |
| **BCMA_EBB-C1978-C7 - nt** | 1124 | GAGGTGCAGCTTGTGGAAACCGGTGGCGGACTGGTGCAGCCCGGAGGA AGCCTCAGGCTGTCCTGCGCCGCGTCCGGCTTCACCTTCTCCTCGTAC GCCATGTCCTGGGTCCGCCAGGCCCCCGGAAAGGGCCTGGAATGGGTG TCCGCCATCTCTGGAAGCGGAGGTTCCACGTACTACGCGGACAGCGTC AAGGGAAGGTTCACAATCTCCCGCGATAATTCGAAGAACACTCTGTAC CTTCAAATGAACACCCTGAAGGCCGAGGACACTGCTGTGTACTACTGC GCACGGGCCACCTACAAGAGAGAGCTCCGGTACTACTACGGAATGGAC GTCTGGGGCCAGGGAACTACTGTGACCGTGTCCTCGGGAGGGGGTGGC TCCGGGGGGGGCGGCTCCGGCGGAGGCGGTTCCGAGATTGTGCTGACC CAGTCACCTTCAACTCTGTCGCTGTCCCCGGGAGAGAGCGCTACTCTG AGCTGCCGGGCCAGCCAGTCCGTGTCCACCACCTTCCTCGCCTGGTAT CAGCAGAAGCCGGGGCAGGCACCACGGCTCTTGATCTACGGGTCAAGC AACAGAGCGACCGGAATTCCTGACCGCTTCTCGGGGAGCGGTTCAGGC ACCGACTTCACCCTGACTATCCGGCGCCTGGAACCCGAAGATTTCGCC GTGTATTACTGTCAACAGTACCACTCCTCGCCGTCCTGGACCTTTGGC CAAGGAACCAAAGTGGAAATCAAG |
| **ScFv domain** | | |
| **BCMA_EBB-C1978-C7 - aa** | 1125 | EVQLVETGGGLVQPGGSLRLSCAASGFTFSSYAMSWVRQAPGKGLEWV SAISGSGGSTYYADSVKGRFTISRDNSKNTLYLQMNTLKAEDTAVYYC ARATYKRELRYYYGMDVWGQGTTVTVSS |
| **VH** | | |
| **BCMA_EBB-C1978-C7 - aa** | 1126 | EIVLTQSPSTLSLSPGESATLSCRASQSVSTTFLAWYQQKPGQAPRLL IYGSSNRATGIPDRFSGSGSGTDFTLTIRRLEPEDFAVYYCQQYHSSP SWTFGQGTKVEIK |
| **VL** | | |
| **BCMA_EBB-C1978-C7 - aa** | 1127 | MALPVTALLLPLALLLHAARPEVQLVETGGGLVQPGGSLRLSCAASGF TFSSYAMSWVRQAPGKGLEWVSAISGSGGSTYYADSVKGRFTISRDNS KNTLYLQMNTLKAEDTAVYYCARATYKRELRYYYGMDVWGQGTTVTVS SGGGGSGGGGSGGGGSEIVLTQSPSTLSLSPGESATLSCRASQSVSTT FLAWYQQKPGQAPRLLIYGSSNRATGIPDRFSGSGSGTDFTLTIRRLE PEDFAVYYCQQYHSSPSWTFGQGTKVEIKTTTPAPRPPTPAPTIASQP LSLRPEACRPAAGGAVHTRGLDFACDIYIWAPLAGTCGVLLLSLVITL YCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRK NPQEGLYNELQKDKMAEAYSEIGMKGERRRGKGHDGLYQGLSTATKDT YDALHMQALPPR |
| **Full CART** | | |
| **BCMA_EBB-C1978-C7 - nt** | 1128 | ATGGCCCTCCCTGTCACCGCCCTGCTGCTTCCGCTGGCTCTTCTGCTC CACGCCGCTCGGCCCGAGGTGCAGCTTGTGGAAACCGGTGGCGGACTG GTGCAGCCCGGAGGAAGCCTCAGGCTGTCCTGCGCCGCGTCCGGCTTC ACCTTCTCCTCGTACGCCATGTCCTGGGTCCGCCAGGCCCCCGGAAAG GGCCTGGAATGGGTGTCCGCCATCTCTGGAAGCGGAGGTTCCACGTAC TACGCGGACAGCGTCAAGGGAAGGTTCACAATCTCCCGCGATAATTCG AAGAACACTCTGTACCTTCAAATGAACACCCTGAAGGCCGAGGACACT GCTGTGTACTACTGCGCACGGGCCACCTACAAGAGAGAGCTCCGGTAC TACTACGGAATGGACGTCTGGGGCCAGGGAACTACTGTGACCGTGTCC TCGGGAGGGGGTGGCTCCGGGGGGGGCGGCTCCGGCGGAGGCGGTTCC GAGATTGTGCTGACCCAGTCACCTTCAACTCTGTCGCTGTCCCCGGGA GAGAGCGCTACTCTGAGCTGCCGGGCCAGCCAGTCCGTGTCCACCACC TTCCTCGCCTGGTATCAGCAGAAGCCGGGGCAGGCACCACGGCTCTTG ATCTACGGGTCAAGCAACAGAGCGACCGGAATTCCTGACCGCTTCTCG GGGAGCGGTTCAGGCACCGACTTCACCCTGACTATCCGGCGCCTGGAA CCCGAAGATTTCGCCGTGTATTACTGTCAACAGTACCACTCCTCGCCG TCCTGGACCTTTGGCCAAGGAACCAAAGTGGAAATCAAGACCACTACC CCAGCACCGAGGCCACCCACCCCGGCTCCTACCATCGCCTCCCAGCCT CTGTCCCTGCGTCCGGAGGCATGTAGACCCGCAGCTGGTGGGGCCGTG CATACCCGGGGTCTTGACTTCGCCTGCGATATCTACATTTGGGCCCCT CTGGCTGGTACTTGCGGGGTCCTGCTGCTTTCACTCGTGATCACTCTT TACTGTAAGCGCGGTCGGAAGAAGCTGCTGTACATCTTTAAGCAACCC TTCATGAGGCCTGTGCAGACTACTCAAGAGGAGGACGGCTGTTCATGC CGGTTCCCAGAGGAGGAGGAAGGCGGCTGCGAACTGCGCGTGAAATTC AGCCGCAGCGCAGATGCTCCAGCCTACAAGCAGGGGCAGAACCAGCTC TACAACGAACTCAATCTTGGTCGGAGAGAGGAGTACGACGTGCTGGAC AAGCGGAGAGGACGGGACCCAGAAATGGGCGGGAAGCCGCGCAGAAAG AATCCCCAAGAGGGCCTGTACAACGAGCTCCAAAAGGATAAGATGGCA GAAGCCTATAGCGAGATTGGTATGAAAGGGGAACGCAGAAGAGGCAAA GGCCACGACGGACTGTACCAGGGACTCAGCACCGCCACCAAGGACACC |
| **Full CART** | | |
| | | TATGACGCTCTTCACATGCAGGCCCTGCCGCCTCGG |

| **BCMA_EBB-C1978-D10** | | |
|---|---|---|
| **BCMA_EBB-C1978-D10 - aa** | 1129 | EVQLVETGGGLVQPGRSLRLSCAASGFTFDDYAMHWVRQAPGKGLEWV SGISWNSGSIGYADSVKGRFTISRDNAKNSLYLQMNSLRDEDTAVYYC ARVGKAVPDVWGQGTTVTVSSGGGGSGGGGSGGGGSDIVMTQTPSSLS ASVGDRVTITCRASQSISSYLNWYQQKPGKAPKLLIYAASSLQSGVPS RFSGSGSGTDFTLTISSLQPEDFATYYCQQSYSTPYSFGQGTRLEIK |
| **ScFv domain** | | |
| **BCMA_EBB-C1978-D10-nt** | 1130 | GAAGTGCAGCTCGTGGAAACTGGAGGTGGACTCGTGCAGCCTGGACGG TCGCTGCGGCTGAGCTGCGCTGCATCCGGCTTCACCTTCGACGATTAT GCCATGCACTGGGTCAGACAGGCGCCAGGGAAGGGACTTGAGTGGGTG TCCGGTATCAGCTGGAATAGCGGCTCAATCGGATACGCGGACTCCGTG AAGGGAAGGTTCACCATTTCCCGCGACAACGCCAAGAACTCCCTGTAC TTGCAAATGAACAGCCTCCGGGATGAGGACACTGCCGTGTACTACTGC GCCCGCGTCGGAAAAGCTGTGCCCGACGTCTGGGGCCAGGGAACCACT GTGACCGTGTCCAGCGGCGGGGGTGGATCGGGCGGTGGAGGGTCCGGT GGAGGGGGCTCAGATATTGTGATGACCCAGACCCCCTCGTCCCTGTCC GCCTCGGTCGGCGACCGCGTGACTATCACATGTAGAGCCTCGCAGAGC ATCTCCAGCTACCTGAACTGGTATCAGCAGAAGCCGGGGAAGGCCCCG AAGCTCCTGATCTACGCGGCATCATCACTGCAATCGGGAGTGCCGAGC CGGTTTTCCGGGTCCGGCTCCGGCACCGACTTCACGCTGACCATTTCT TCCCTGCAACCCGAGGACTTCGCCACTTACTACTGCCAGCAGTCCTAC TCCACCCCTTACTCCTTCGGCCAAGGAACCAGGCTGGAAATCAAG |
| **ScFv domain** | | |
| **BCMA_EBB-C1978-D10 - aa** | 1131 | EVQLVETGGGLVQPGRSLRLSCAASGFTFDDYAMHWVRQAPGKGLEWV SGISWNSGSIGYADSVKGRFTISRDNAKNSLYLQMNSLRDEDTAVYYC ARVGKAVPDVWGQGTTVTVSS |
| **VH** | | |
| **BCMA_EBB-C1978-D10-aa** | 1132 | DIVMTQTPSSLSASVGDRVTITCRASQSISSYLNWYQQKPGKAPKLLI YAASSLQSGVPSRFSGSGSGTDFTLTISSLQPEDFATYYCQQSYSTPY SFGQGTRLEIK |
| **VL** | | |
| **BCMA_EBB-C1978-D10 - aa** | 1133 | MALPVTALLLPLALLLHAARPEVQLVETGGGLVQPGRSLRLSCAASGF TFDDYAMHWVRQAPGKGLEWVSGISWNSGSIGYADSVKGRFTISRDNA KNSLYLQMNSLRDEDTAVYYCARVGKAVPDVWGQGTTVTVSSGGGGSG GGGSGGGGSDIVMTQTPSSLSASVGDRVTITCRASQSISSYLNWYQQK PGKAPKLLIYAASSLQSGVPSRFSGSGSGTDFTLTISSLQPEDFATYY CQQSYSTPYSFGQGTRLEIKTTTPAPRPPTPAPTIASQPLSLRPEACR PAAGGAVHTRGLDFACDIYIWAPLAGTCGVLLLSLVITLYCKRGRKKL LYIFKQPFMRPVQTTQEEDGCSCRFPEEEEGGCELRVKFSRSADAPAY KQGQNQLYNELNLGRREEYDVLDKRRGRDPEMGGKPRRKNPQEGLYNE LQKDKMAEAYSEIGMKGERRRGKGHDGLYQGLSTATKDTYDALHMQAL |
| **Full CART** | | |
| | | PPR |
| **BCMA_EBB-C1978-D10 - nt** | 1134 | ATGGCCCTCCCTGTCACCGCCCTGCTGCTTCCGCTGGCTCTTCTGCTC CACGCCGCTCGGCCCGAAGTGCAGCTCGTGGAAACTGGAGGTGGACTC GTGCAGCCTGGACGGTCGCTGCGGCTGAGCTGCGCTGCATCCGGCTTC ACCTTCGACGATTATGCCATGCACTGGGTCAGACAGGCGCCAGGGAAG GGACTTGAGTGGGTGTCCGGTATCAGCTGGAATAGCGGCTCAATCGGA TACGCGGACTCCGTGAAGGGAAGGTTCACCATTTCCCGCGACAACGCC AAGAACTCCCTGTACTTGCAAATGAACAGCCTCCGGGATGAGGACACT GCCGTGTACTACTGCGCCCGCGTCGGAAAAGCTGTGCCCGACGTCTGG GGCCAGGGAACCACTGTGACCGTGTCCAGCGGCGGGGGTGGATCGGGC GGTGGAGGGTCCGGTGGAGGGGGCTCAGATATTGTGATGACCCAGACC CCCTCGTCCCTGTCCGCCTCGGTCGGCGACCGCGTGACTATCACATGT AGAGCCTCGCAGAGCATCTCCAGCTACCTGAACTGGTATCAGCAGAAG CCGGGGAAGGCCCCGAAGCTCCTGATCTACGCGGCATCATCACTGCAA TCGGGAGTGCCGAGCCGGTTTTCCGGGTCCGGCTCCGGCACCGACTTC ACGCTGACCATTTCTTCCCTGCAACCCGAGGACTTCGCCACTTACTAC TGCCAGCAGTCCTACTCCACCCCTTACTCCTTCGGCCAAGGAACCAGG CTGGAAATCAAGACCACTACCCCAGCACCGAGGCCACCCACCCCGGCT CCTACCATCGCCTCCCAGCCTCTGTCCCTGCGTCCGGAGGCATGTAGA CCCGCAGCTGGTGGGGCCGTGCATACCCGGGGTCTTGACTTCGCCTGC GATATCTACATTTGGGCCCCTCTGGCTGGTACTTGCGGGGTCCTGCTG CTTTCACTCGTGATCACTCTTTACTGTAAGCGCGGTCGGAAGAAGCTG CTGTACATCTTTAAGCAACCCTTCATGAGGCCTGTGCAGACTACTCAA GAGGAGGACGGCTGTTCATGCCGGTTCCCAGAGGAGGAGGAAGGCGGC TGCGAACTGCGCGTGAAATTCAGCCGCAGCGCAGATGCTCCAGCCTAC AAGCAGGGGCAGAACCAGCTCTACAACGAACTCAATCTTGGTCGGAGA GAGGAGTACGACGTGCTGGACAAGCGGAGAGGACGGGACCCAGAAATG GGCGGGAAGCCGCGCAGAAAGAATCCCCAAGAGGGCCTGTACAACGAG CTCCAAAAGGATAAGATGGCAGAAGCCTATAGCGAGATTGGTATGAAA GGGGAACGCAGAAGAGGCAAAGGCCACGACGGACTGTACCAGGGACTC AGCACCGCCACCAAGGACACCTATGACGCTCTTCACATGCAGGCCCTG CCGCCTCGG |
| **Full CART** | | |

| **BCMA_EBB-C1979-C12** | | |
|---|---|---|
| **BCMA_EBB-C1979-C12-aa** | 1135 | EVQLVESGGGLVQPGRSLRLSCTASGFTFDDYAMHWVRQRPGKGLEWV ASINWKGNSLAYGDSVKGRFAISRDNAKNTVFLQMNSLRTEDTAVYYC ASHQGVAYYNYAMDVWGRGTLVTVSSGGGGSGGGGSGGGGSEIVLTQS PGTLSLSPGERATLSCRATQSIGSSFLAWYQQRPGQAPRLLIYGASQR ATGIPDRFSGRGSGTDFTLTISRVEPEDSAVYYCQHYESSPSWTFGQG TKVEIK |
| **ScFv domain** | | |
| **BCMA_EBB-C1979-C12 - nt ScFv domain** | 1136 | GAAGTGCAGCTCGTGGAGAGCGGGGGAGGATTGGTGCAGCCCGGAAGG TCCCTGCGGCTCTCCTGCACTGCGTCTGGCTTCACCTTCGACGACTAC GCGATGCACTGGGTCAGACAGCGCCCGGGAAAGGGCCTGGAATGGGTC GCCTCAATCAACTGGAAGGGAAACTCCCTGGCCTATGGCGACAGCGTG AAGGGCCGCTTCGCCATTTCGCGCGACAACGCCAAGAACACCGTGTTT |
| | | CTGCAAATGAATTCCCTGCGGACCGAGGATACCGCTGTGTACTACTGC GCCAGCCACCAGGGCGTGGCATACTATAACTACGCCATGGACGTGTGG GGAAGAGGGACGCTCGTCACCGTGTCCTCCGGGGGCGGTGGATCGGGT GGAGGAGGAAGCGGTGGCGGGGGCAGCGAAATCGTGCTGACTCAGAGC CCGGGAACTCTTTCACTGTCCCCGGGAGAACGGGCCACTCTCTCGTGC CGGGCCACCCAGTCCATCGGCTCCTCCTTCCTTGCCTGGTACCAGCAG AGGCCAGGACAGGCGCCCCGCCTGCTGATCTACGGTGCTTCCCAACGC GCCACTGGCATTCCTGACCGGTTCAGCGGCAGAGGGTCGGGAACCGAT TTCACACTGACCATTTCCCGGGTGGAGCCCGAAGATTCGGCAGTCTAC TACTGTCAGCATTACGAGTCCTCCCCTTCATGGACCTTCGGTCAAGGG ACCAAAGTGGAGATCAAG |
| **BCMA_EBB-C1979-C12 - aa** | 1137 | EVQLVESGGGLVQPGRSLRLSCTASGFTFDDYAMHWVRQRPGKGLEWV ASINWKGNSLAYGDSVKGRFAISRDNAKNTVFLQMNSLRTEDTAVYYC ASHQGVAYYNYAMDVWGRGTLVTVSS |
| **VH** | | |
| **BCMA_EBB-C1979-C12** - **aa** | 1138 | EIVLTQSPGTLSLSPGERATLSCRATQSIGSSFLAWYQQRPGQAPRLL IYGASQRATGIPDRFSGRGSGTDFTLTISRVEPEDSAVYYCQHYESSP SWTFGQGTKVEIK |
| **VL** | | |
| **BCMA_EBB-C1979-C12** - **aa** | 1139 | MALPVTALLLPLALLLHAARPEVQLVESGGGLVQPGRSLRLSCTASGF TFDDYAMHWVRQRPGKGLEWVASINWKGNSLAYGDSVKGRFAISRDNA KNTVFLQMNSLRTEDTAVYYCASHQGVAYYNYAMDVWGRGTLVTVSSG GGGSGGGGSGGGGSEIVLTQSPGTLSLSPGERATLSCRATQSIGSSFL AWYQQRPGQAPRLLIYGASQRATGIPDRFSGRGSGTDFTLTISRVEPE DSAVYYCQHYESSPSWTFGQGTKVEIKTTTPAPRPPTPAPTIASQPLS LRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNP QEGLYNELQKDKMAEAYSEIGMKGERRRGKGHDGLYQGLSTATKDTYD ALHMQALPPR |
| **Full CART** | | |
| **BCMA_EBB-C1979-C12 - nt** | 1140 | ATGGCCCTCCCTGTCACCGCCCTGCTGCTTCCGCTGGCTCTTCTGCTC CACGCCGCTCGGCCCGAAGTGCAGCTCGTGGAGAGCGGGGGAGGATTG GTGCAGCCCGGAAGGTCCCTGCGGCTCTCCTGCACTGCGTCTGGCTTC ACCTTCGACGACTACGCGATGCACTGGGTCAGACAGCGCCCGGGAAAG GGCCTGGAATGGGTCGCCTCAATCAACTGGAAGGGAAACTCCCTGGCC TATGGCGACAGCGTGAAGGGCCGCTTCGCCATTTCGCGCGACAACGCC AAGAACACCGTGTTTCTGCAAATGAATTCCCTGCGGACCGAGGATACC GCTGTGTACTACTGCGCCAGCCACCAGGGCGTGGCATACTATAACTAC GCCATGGACGTGTGGGGAAGAGGGACGCTCGTCACCGTGTCCTCCGGG GGCGGTGGATCGGGTGGAGGAGGAAGCGGTGGCGGGGGCAGCGAAATC GTGCTGACTCAGAGCCCGGGAACTCTTTCACTGTCCCCGGGAGAACGG GCCACTCTCTCGTGCCGGGCCACCCAGTCCATCGGCTCCTCCTTCCTT |
| **Full CART** | | |
| | | GCCTGGTACCAGCAGAGGCCAGGACAGGCGCCCCGCCTGCTGATCTAC GGTGCTTCCCAACGCGCCACTGGCATTCCTGACCGGTTCAGCGGCAGA GGGTCGGGAACCGATTTCACACTGACCATTTCCCGGGTGGAGCCCGAA GATTCGGCAGTCTACTACTGTCAGCATTACGAGTCCTCCCCTTCATGG ACCTTCGGTCAAGGGACCAAAGTGGAGATCAAGACCACTACCCCAGCA CCGAGGCCACCCACCCCGGCTCCTACCATCGCCTCCCAGCCTCTGTCC CTGCGTCCGGAGGCATGTAGACCCGCAGCTGGTGGGGCCGTGCATACC CGGGGTCTTGACTTCGCCTGCGATATCTACATTTGGGCCCCTCTGGCT GGTACTTGCGGGGTCCTGCTGCTTTCACTCGTGATCACTCTTTACTGT AAGCGCGGTCGGAAGAAGCTGCTGTACATCTTTAAGCAACCCTTCATG AGGCCTGTGCAGACTACTCAAGAGGAGGACGGCTGTTCATGCCGGTTC CCAGAGGAGGAGGAAGGCGGCTGCGAACTGCGCGTGAAATTCAGCCGC AGCGCAGATGCTCCAGCCTACAAGCAGGGGCAGAACCAGCTCTACAAC GAACTCAATCTTGGTCGGAGAGAGGAGTACGACGTGCTGGACAAGCGG AGAGGACGGGACCCAGAAATGGGCGGGAAGCCGCGCAGAAAGAATCCC CAAGAGGGCCTGTACAACGAGCTCCAAAAGGATAAGATGGCAGAAGCC TATAGCGAGATTGGTATGAAAGGGGAACGCAGAAGAGGCAAAGGCCAC GACGGACTGTACCAGGGACTCAGCACCGCCACCAAGGACACCTATGAC GCTCTTCACATGCAGGCCCTGCCGCCTCGG |

| **BCMA_EBB-C1980-G4** | | |
|---|---|---|
| **BCMA_EBB-C1980-G4- aa** | 1141 | EVQLVESGGGLVQPGGSLRLSCAASGFTFSSYAMSWVRQAPGKGLEWV SAISGSGGSTYYADSVKGRFTISRDNSKNTLYLQMNSLRAEDTAVYYC AKVVRDGMDVWGQGTTVTVSSGGGGSGGGGSGGGGSEIVLTQSPATLS LSPGERATLSCRASQSVSSSYLAWYQQKPGQAPRLLIYGASSRATGIP DRFSGNGSGTDFTLTISRLEPEDFAVYYCQQYGSPPRFTFGPGTKVDI K |
| **ScFv domain** | | |
| **BCMA_EBB-C1980-G4- nt** | 1142 | GAGGTGCAGTTGGTCGAAAGCGGGGGCGGGCTTGTGCAGCCTGGCGGA TCACTGCGGCTGTCCTGCGCGGCATCAGGCTTCACGTTTTCTTCCTAC GCCATGTCCTGGGTGCGCCAGGCCCCTGGAAAGGGACTGGAATGGGTG TCCGCGATTTCGGGGTCCGGCGGGAGCACCTACTACGCCGATTCCGTG AAGGGCCGCTTCACTATCTCGCGGGACAACTCCAAGAACACCCTCTAC CTCCAAATGAATAGCCTGCGGGCCGAGGATACCGCCGTCTACTATTGC GCTAAGGTCGTGCGCGACGGAATGGACGTGTGGGGACAGGGTACCACC GTGACAGTGTCCTCGGGGGGAGGCGGTAGCGGCGGAGGAGGAAGCGGT GGTGGAGGTTCCGAGATTGTGCTGACTCAATCACCCGCGACCCTGAGC CTGTCCCCCGGCGAAAGGGCCACTCTGTCCTGTCGGGCCAGCCAATCA GTCTCCTCCTCGTACCTGGCCTGGTACCAGCAGAAGCCAGGACAGGCT CCGAGACTCCTTATCTATGGCGCATCCTCCCGCGCCACCGGAATCCCG GATAGGTTCTCGGGAAACGGATCGGGGACCGACTTCACTCTCACCATC TCCCGGCTGGAACCGGAGGACTTCGCCGTGTACTACTGCCAGCAGTAC GGCAGCCCGCCTAGATTCACTTTCGGCCCCGGCACCAAAGTGGACATC AAG |
| **ScFv domain** | | |
| **BCMA_EBB-C1980-G4- aa VH** | 1143 | EVQLVESGGGLVQPGGSLRLSCAASGFTFSSYAMSWVRQAPGKGLEWV SAISGSGGSTYYADSVKGRFTISRDNSKNTLYLQMNSLRAEDTAVYYC |
| | | AKVVRDGMDVWGQGTTVTVSS |
| **BCMA_EBB-C1980-G4- aa** | 1144 | EIVLTQSPATLSLSPGERATLSCRASQSVSSSYLAWYQQKPGQAPRLL IYGASSRATGIPDRFSGNGSGTDFTLTISRLEPEDFAVYYCQQYGSPP RFTFGPGTKVDIK |
| **VL** | | |
| **BCMA_EBB-C1980-G4- aa** | 1145 | MALPVTALLLPLALLLHAARPEVQLVESGGGLVQPGGSLRLSCAASGF TFSSYAMSWVRQAPGKGLEWVSAISGSGGSTYYADSVKGRFTISRDNS KNTLYLQMNSLRAEDTAVYYCAKVVRDGMDVWGQGTTVTVSSGGGGSG GGGSGGGGSEIVLTQSPATLSLSPGERATLSCRASQSVSSSYLAWYQQ KPGQAPRLLIYGASSRATGIPDRFSGNGSGTDFTLTISRLEPEDFAVY YCQQYGSPPRFTFGPGTKVDIKTTTPAPRPPTPAPTIASQPLSLRPEA CRPAAGGAVHTRGLDFACDIYIWAPLAGTCGVLLLSLVITLYCKRGRK KLLYIFKQPFMRPVQTTQEEDGCSCRFPEEEEGGCELRVKFSRSADAP AYKQGQNQLYNELNLGRREEYDVLDKRRGRDPEMGGKPRRKNPQEGLY NELQKDKMAEAYSEIGMKGERRRGKGHDGLYQGLSTATKDTYDALHMQ ALPPR |
| **Full CART** | | |
| **BCMA_EBB-C1980-G4- nt** | 1146 | ATGGCCCTCCCTGTCACCGCCCTGCTGCTTCCGCTGGCTCTTCTGCTC CACGCCGCTCGGCCCGAGGTGCAGTTGGTCGAAAGCGGGGGCGGGCTT GTGCAGCCTGGCGGATCACTGCGGCTGTCCTGCGCGGCATCAGGCTTC ACGTTTTCTTCCTACGCCATGTCCTGGGTGCGCCAGGCCCCTGGAAAG GGACTGGAATGGGTGTCCGCGATTTCGGGGTCCGGCGGGAGCACCTAC TACGCCGATTCCGTGAAGGGCCGCTTCACTATCTCGCGGGACAACTCC AAGAACACCCTCTACCTCCAAATGAATAGCCTGCGGGCCGAGGATACC GCCGTCTACTATTGCGCTAAGGTCGTGCGCGACGGAATGGACGTGTGG GGACAGGGTACCACCGTGACAGTGTCCTCGGGGGGAGGCGGTAGCGGC GGAGGAGGAAGCGGTGGTGGAGGTTCCGAGATTGTGCTGACTCAATCA CCCGCGACCCTGAGCCTGTCCCCCGGCGAAAGGGCCACTCTGTCCTGT CGGGCCAGCCAATCAGTCTCCTCCTCGTACCTGGCCTGGTACCAGCAG AAGCCAGGACAGGCTCCGAGACTCCTTATCTATGGCGCATCCTCCCGC GCCACCGGAATCCCGGATAGGTTCTCGGGAAACGGATCGGGGACCGAC TTCACTCTCACCATCTCCCGGCTGGAACCGGAGGACTTCGCCGTGTAC TACTGCCAGCAGTACGGCAGCCCGCCTAGATTCACTTTCGGCCCCGGC ACCAAAGTGGACATCAAGACCACTACCCCAGCACCGAGGCCACCCACC CCGGCTCCTACCATCGCCTCCCAGCCTCTGTCCCTGCGTCCGGAGGCA TGTAGACCCGCAGCTGGTGGGGCCGTGCATACCCGGGGTCTTGACTTC GCCTGCGATATCTACATTTGGGCCCCTCTGGCTGGTACTTGCGGGGTC CTGCTGCTTTCACTCGTGATCACTCTTTACTGTAAGCGCGGTCGGAAG AAGCTGCTGTACATCTTTAAGCAACCCTTCATGAGGCCTGTGCAGACT ACTCAAGAGGAGGACGGCTGTTCATGCCGGTTCCCAGAGGAGGAGGAA GGCGGCTGCGAACTGCGCGTGAAATTCAGCCGCAGCGCAGATGCTCCA GCCTACAAGCAGGGGCAGAACCAGCTCTACAACGAACTCAATCTTGGT CGGAGAGAGGAGTACGACGTGCTGGACAAGCGGAGAGGACGGGACCCA GAAATGGGCGGGAAGCCGCGCAGAAAGAATCCCCAAGAGGGCCTGTAC |
| **Full CART** | | |
| | | AACGAGCTCCAAAAGGATAAGATGGCAGAAGCCTATAGCGAGATTGGT ATGAAAGGGGAACGCAGAAGAGGCAAAGGCCACGACGGACTGTACCAG GGACTCAGCACCGCCACCAAGGACACCTATGACGCTCTTCACATGCAG GCCCTGCCGCCTCGG |

| **BCMA_EBB-C1980-D2** | | |
|---|---|---|
| **BCMA_EBB-C1980-D2- aa** | 1147 | EVQLLESGGGLVQPGGSLRLSCAASGFTFSSYAMSWVRQAPGKGLEWV SAISGSGGSTYYADSVKGRFTISRDNSKNTLYLQMNSLRAEDTAVYYC AKIPQTGTFDYWGQGTLVTVSSGGGGSGGGGSGGGGSEIVLTQSPGTL SLSPGERATLSCRASQSVSSSYLAWYQQRPGQAPRLLIYGASSRATGI PDRFSGSGSGTDFTLTISRLEPEDFAVYYCQHYGSSPSWTFGQGTRLE IK |
| **ScFv domain** | | |
| **BCMA_EBB-C1980-D2- nt** | 1148 | GAAGTGCAGCTGCTGGAGTCCGGCGGTGGATTGGTGCAACCGGGGGGA TCGCTCAGACTGTCCTGTGCGGCGTCAGGCTTCACCTTCTCGAGCTAC GCCATGTCATGGGTCAGACAGGCCCCTGGAAAGGGTCTGGAATGGGTG TCCGCCATTTCCGGGAGCGGGGGATCTACATACTACGCCGATAGCGTG AAGGGCCGCTTCACCATTTCCCGGGACAACTCCAAGAACACTCTCTAT CTGCAAATGAACTCCCTCCGCGCTGAGGACACTGCCGTGTACTACTGC GCCAAAATCCCTCAGACCGGCACCTTCGACTACTGGGGACAGGGGACT CTGGTCACCGTCAGCAGCGGTGGCGGAGGTTCGGGGGGAGGAGGAAGC GGCGGCGGAGGGTCCGAGATTGTGCTGACCCAGTCACCCGGCACTTTG TCCCTGTCGCCTGGAGAAAGGGCCACCCTTTCCTGCCGGGCATCCCAA TCCGTGTCCTCCTCGTACCTGGCCTGGTACCAGCAGAGGCCCGGACAG GCCCCACGGCTTCTGATCTACGGAGCAAGCAGCCGCGCGACCGGTATC CCGGACCGGTTTTCGGGCTCGGGCTCAGGAACTGACTTCACCCTCACC ATCTCCCGCCTGGAACCCGAAGATTTCGCTGTGTATTACTGCCAGCAC TACGGCAGCTCCCCGTCCTGGACGTTCGGCCAGGGAACTCGGCTGGAG ATCAAG |
| **ScFv domain** | | |
| **BCMA_EBB-C1980-D2- aa** | 1149 | EVQLLESGGGLVQPGGSLRLSCAASGFTFSSYAMSWVRQAPGKGLEWV SAISGSGGSTYYADSVKGRFTISRDNSKNTLYLQMNSLRAEDTAVYYC AKIPQTGTFDYWGQGTLVTVSS |
| **VH** | | |
| **BCMA_EBB-C1980-D2- aa** | 1150 | EIVLTQSPGTLSLSPGERATLSCRASQSVSSSYLAWYQQRPGQAPRLL IYGASSRATGIPDRFSGSGSGTDFTLTISRLEPEDFAVYYCQHYGSSP SWTFGQGTRLEIK |
| **VL** | | |
| **BCMA_EBB-C1980-D2- aa** | 1151 | MALPVTALLLPLALLLHAARPEVQLLESGGGLVQPGGSLRLSCAASGF TFSSYAMSWVRQAPGKGLEWVSAISGSGGSTYYADSVKGRFTISRDNS KNTLYLQMNSLRAEDTAVYYCAKIPQTGTFDYWGQGTLVTVSSGGGGS GGGGSGGGGSEIVLTQSPGTLSLSPGERATLSCRASQSVSSSYLAWYQ QRPGQAPRLLIYGASSRATGIPDRFSGSGSGTDFTLTISRLEPEDFAV YYCQHYGSSPSWTFGQGTRLEIKTTTPAPRPPTPAPTIASQPLSLRPE ACRPAAGGAVHTRGLDFACDIYIWAPLAGTCGVLLLSLVITLYCKRGR |
| **Full CART** | | |
| | | KKLLYIFKQPFMRPVQTTQEEDGCSCRFPEEEEGGCELRVKFSRSADA PAYKQGQNQLYNELNLGRREEYDVLDKRRGRDPEMGGKPRRKNPQEGL YNELQKDKMAEAYSEIGMKGERRRGKGHDGLYQGLSTATKDTYDALHM QALPPR |
| **BCMA_EBB-C1980-D2- nt** | 1152 | ATGGCCCTCCCTGTCACCGCCCTGCTGCTTCCGCTGGCTCTTCTGCTC CACGCCGCTCGGCCCGAAGTGCAGCTGCTGGAGTCCGGCGGTGGATTG GTGCAACCGGGGGGATCGCTCAGACTGTCCTGTGCGGCGTCAGGCTTC ACCTTCTCGAGCTACGCCATGTCATGGGTCAGACAGGCCCCTGGAAAG GGTCTGGAATGGGTGTCCGCCATTTCCGGGAGCGGGGGATCTACATAC TACGCCGATAGCGTGAAGGGCCGCTTCACCATTTCCCGGGACAACTCC AAGAACACTCTCTATCTGCAAATGAACTCCCTCCGCGCTGAGGACACT GCCGTGTACTACTGCGCCAAAATCCCTCAGACCGGCACCTTCGACTAC TGGGGACAGGGGACTCTGGTCACCGTCAGCAGCGGTGGCGGAGGTTCG GGGGGAGGAGGAAGCGGCGGCGGAGGGTCCGAGATTGTGCTGACCCAG TCACCCGGCACTTTGTCCCTGTCGCCTGGAGAAAGGGCCACCCTTTCC TGCCGGGCATCCCAATCCGTGTCCTCCTCGTACCTGGCCTGGTACCAG CAGAGGCCCGGACAGGCCCCACGGCTTCTGATCTACGGAGCAAGCAGC CGCGCGACCGGTATCCCGGACCGGTTTTCGGGCTCGGGCTCAGGAACT GACTTCACCCTCACCATCTCCCGCCTGGAACCCGAAGATTTCGCTGTG TATTACTGCCAGCACTACGGCAGCTCCCCGTCCTGGACGTTCGGCCAG GGAACTCGGCTGGAGATCAAGACCACTACCCCAGCACCGAGGCCACCC ACCCCGGCTCCTACCATCGCCTCCCAGCCTCTGTCCCTGCGTCCGGAG GCATGTAGACCCGCAGCTGGTGGGGCCGTGCATACCCGGGGTCTTGAC TTCGCCTGCGATATCTACATTTGGGCCCCTCTGGCTGGTACTTGCGGG GTCCTGCTGCTTTCACTCGTGATCACTCTTTACTGTAAGCGCGGTCGG AAGAAGCTGCTGTACATCTTTAAGCAACCCTTCATGAGGCCTGTGCAG ACTACTCAAGAGGAGGACGGCTGTTCATGCCGGTTCCCAGAGGAGGAG GAAGGCGGCTGCGAACTGCGCGTGAAATTCAGCCGCAGCGCAGATGCT CCAGCCTACAAGCAGGGGCAGAACCAGCTCTACAACGAACTCAATCTT GGTCGGAGAGAGGAGTACGACGTGCTGGACAAGCGGAGAGGACGGGAC CCAGAAATGGGCGGGAAGCCGCGCAGAAAGAATCCCCAAGAGGGCCTG TACAACGAGCTCCAAAAGGATAAGATGGCAGAAGCCTATAGCGAGATT GGTATGAAAGGGGAACGCAGAAGAGGCAAAGGCCACGACGGACTGTAC CAGGGACTCAGCACCGCCACCAAGGACACCTATGACGCTCTTCACATG CAGGCCCTGCCGCCTCGG |
| **Full CART** | | |

| **BCMA_EBB-C1978-A10** | | |
|---|---|---|
| **BCMA_EBB-C1978-A10-aa** | 1153 | EVQLVETGGGLVQPGGSLRLSCAASGFTFSSYAMSWVRQAPGKGLEWV SAISGSGGSTYYADSVKGRFTMSRENDKNSVFLQMNSLRVEDTGVYYC ARANYKRELRYYYGMDVWGQGTMVTVSSGGGGSGGGGSGGGGSEIVMT QSPGTLSLSPGESATLSCRASQRVASNYLAWYQHKPGQAPSLLISGAS SRATGVPDRFSGSGSGTDFTLAISRLEPEDSAVYYCQHYDSSPSWTFG QGTKVEIK |
| **ScFv domain** | | |
| **BCMA_EBB-C1978-A10-nt ScFv domain** | 1154 | GAAGTGCAACTGGTGGAAACCGGTGGAGGACTCGTGCAGCCTGGCGGC AGCCTCCGGCTGAGCTGCGCCGCTTCGGGATTCACCTTTTCCTCCTAC |
| | | GCGATGTCTTGGGTCAGACAGGCCCCCGGAAAGGGGCTGGAATGGGTG TCAGCCATCTCCGGCTCCGGCGGATCAACGTACTACGCCGACTCCGTG AAAGGCCGGTTCACCATGTCGCGCGAGAATGACAAGAACTCCGTGTTC CTGCAAATGAACTCCCTGAGGGTGGAGGACACCGGAGTGTACTATTGT GCGCGCGCCAACTACAAGAGAGAGCTGCGGTACTACTACGGAATGGAC GTCTGGGGACAGGGAACTATGGTGACCGTGTCATCCGGTGGAGGGGGA AGCGGCGGTGGAGGCAGCGGGGGCGGGGGTTCAGAAATTGTCATGACC CAGTCCCCGGGAACTCTTTCCCTCTCCCCCGGGGAATCCGCGACTTTG TCCTGCCGGGCCAGCCAGCGCGTGGCCTCGAACTACCTCGCATGGTAC CAGCATAAGCCAGGCCAAGCCCCTTCCCTGCTGATTTCCGGGGCTAGC AGCCGCGCCACTGGCGTGCCGGATAGGTTCTCGGGAAGCGGCTCGGGT ACCGATTTCACCCTGGCAATCTCGCGGCTGGAACCGGAGGATTCGGCC GTGTACTACTGCCAGCACTATGACTCATCCCCCTCCTGGACATTCGGA CAGGGCACCAAGGTCGAGATCAAG |
| **BCMA_EBB-C1978-A10-aa** | 1155 | EVQLVETGGGLVQPGGSLRLSCAASGFTFSSYAMSWVRQAPGKGLEWV SAISGSGGSTYYADSVKGRFTMSRENDKNSVFLQMNSLRVEDTGVYYC ARANYKRELRYYYGMDVWGQGTMVTVSS |
| **VH** | | |
| **BCMA_EBB-C1978-A10-aa** | 1156 | EIVMTQSPGTLSLSPGESATLSCRASQRVASNYLAWYQHKPGQAPSLL ISGASSRATGVPDRFSGSGSGTDFTLAISRLEPEDSAVYYCQHYDSSP SWTFGQGTKVEIK |
| **VL** | | |
| **BCMA_EBB-C1978-A10-aa** | 1157 | MALPVTALLLPLALLLHAARPEVQLVETGGGLVQPGGSLRLSCAASGF TFSSYAMSWVRQAPGKGLEWVSAISGSGGSTYYADSVKGRFTMSREND KNSVFLQMNSLRVEDTGVYYCARANYKRELRYYYGMDVWGQGTMVTVS SGGGGSGGGGSGGGGSEIVMTQSPGTLSLSPGESATLSCRASQRVASN YLAWYQHKPGQAPSLLISGASSRATGVPDRFSGSGSGTDFTLAISRLE PEDSAVYYCQHYDSSPSWTFGQGTKVEIKTTTPAPRPPTPAPTIASQP LSLRPEACRPAAGGAVHTRGLDFACDIYIWAPLAGTCGVLLLSLVITL YCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRK NPQEGLYNELQKDKMAEAYSEIGMKGERRRGKGHDGLYQGLSTATKDT YDALHMQALPPR |
| **Full CART** | | |
| **BCMA_EBB-C1978-A10-nt** | 1158 | ATGGCCCTCCCTGTCACCGCCCTGCTGCTTCCGCTGGCTCTTCTGCTC CACGCCGCTCGGCCCGAAGTGCAACTGGTGGAAACCGGTGGAGGACTC GTGCAGCCTGGCGGCAGCCTCCGGCTGAGCTGCGCCGCTTCGGGATTC ACCTTTTCCTCCTACGCGATGTCTTGGGTCAGACAGGCCCCCGGAAAG GGGCTGGAATGGGTGTCAGCCATCTCCGGCTCCGGCGGATCAACGTAC TACGCCGACTCCGTGAAAGGCCGGTTCACCATGTCGCGCGAGAATGAC AAGAACTCCGTGTTCCTGCAAATGAACTCCCTGAGGGTGGAGGACACC GGAGTGTACTATTGTGCGCGCGCCAACTACAAGAGAGAGCTGCGGTAC TACTACGGAATGGACGTCTGGGGACAGGGAACTATGGTGACCGTGTCA |
| **Full CART** | | |
| | | TCCGGTGGAGGGGGAAGCGGCGGTGGAGGCAGCGGGGGCGGGGGTTCA GAAATTGTCATGACCCAGTCCCCGGGAACTCTTTCCCTCTCCCCCGGG GAATCCGCGACTTTGTCCTGCCGGGCCAGCCAGCGCGTGGCCTCGAAC TACCTCGCATGGTACCAGCATAAGCCAGGCCAAGCCCCTTCCCTGCTG ATTTCCGGGGCTAGCAGCCGCGCCACTGGCGTGCCGGATAGGTTCTCG GGAAGCGGCTCGGGTACCGATTTCACCCTGGCAATCTCGCGGCTGGAA CCGGAGGATTCGGCCGTGTACTACTGCCAGCACTATGACTCATCCCCC TCCTGGACATTCGGACAGGGCACCAAGGTCGAGATCAAGACCACTACC CCAGCACCGAGGCCACCCACCCCGGCTCCTACCATCGCCTCCCAGCCT CTGTCCCTGCGTCCGGAGGCATGTAGACCCGCAGCTGGTGGGGCCGTG CATACCCGGGGTCTTGACTTCGCCTGCGATATCTACATTTGGGCCCCT CTGGCTGGTACTTGCGGGGTCCTGCTGCTTTCACTCGTGATCACTCTT TACTGTAAGCGCGGTCGGAAGAAGCTGCTGTACATCTTTAAGCAACCC TTCATGAGGCCTGTGCAGACTACTCAAGAGGAGGACGGCTGTTCATGC CGGTTCCCAGAGGAGGAGGAAGGCGGCTGCGAACTGCGCGTGAAATTC AGCCGCAGCGCAGATGCTCCAGCCTACAAGCAGGGGCAGAACCAGCTC TACAACGAACTCAATCTTGGTCGGAGAGAGGAGTACGACGTGCTGGAC AAGCGGAGAGGACGGGACCCAGAAATGGGCGGGAAGCCGCGCAGAAAG AATCCCCAAGAGGGCCTGTACAACGAGCTCCAAAAGGATAAGATGGCA GAAGCCTATAGCGAGATTGGTATGAAAGGGGAACGCAGAAGAGGCAAA GGCCACGACGGACTGTACCAGGGACTCAGCACCGCCACCAAGGACACC TATGACGCTCTTCACATGCAGGCCCTGCCGCCTCGG |

| **BCMA_EBB-C1978-D4** | | |
|---|---|---|
| **BCMA_EBB-C1978-D4- aa** | 1159 | EVQLLETGGGLVQPGGSLRLSCAASGFSFSSYAMSWVRQAPGKGLEWV SAISGSGGSTYYADSVKGRFTISRDNSKNTLYLQMNSLRAEDTAVYYC AKALVGATGAFDIWGQGTLVTVSSGGGGSGGGGSGGGGSEIVLTQSPG TLSLSPGERATLSCRASQSLSSNFLAWYQQKPGQAPGLLIYGASNWAT GTPDRFSGSGSGTDFTLTITRLEPEDFAVYYCQYYGTSPMYTFGQGTK VEIK |
| **ScFv domain** | | |
| **BCMA_EBB-C1978-D4- nt** | 1160 | GAAGTGCAGCTGCTCGAAACCGGTGGAGGGCTGGTGCAGCCAGGGGGC TCCCTGAGGCTTTCATGCGCCGCTAGCGGATTCTCCTTCTCCTCTTAC GCCATGTCGTGGGTCCGCCAAGCCCCTGGAAAAGGCCTGGAATGGGTG TCCGCGATTTCCGGGAGCGGAGGTTCGACCTATTACGCCGACTCCGTG AAGGGCCGCTTTACCATCTCCCGGGATAACTCCAAGAACACTCTGTAC CTCCAAATGAACTCGCTGAGAGCCGAGGACACCGCCGTGTATTACTGC GCGAAGGCGCTGGTCGGCGCGACTGGGGCATTCGACATCTGGGGACAG GGAACTCTTGTGACCGTGTCGAGCGGAGGCGGCGGCTCCGGCGGAGGA GGGAGCGGGGGCGGTGGTTCCGAAATCGTGTTGACTCAGTCCCCGGGA ACCCTGAGCTTGTCACCCGGGGAGCGGGCCACTCTCTCCTGTCGCGCC TCCCAATCGCTCTCATCCAATTTCCTGGCCTGGTACCAGCAGAAGCCC GGACAGGCCCCGGGCCTGCTCATCTACGGCGCTTCAAACTGGGCAACG GGAACCCCTGATCGGTTCAGCGGAAGCGGATCGGGTACTGACTTTACC CTGACCATCACCAGACTGGAACCGGAGGACTTCGCCGTGTACTACTGC CAGTACTACGGCACCTCCCCCATGTACACATTCGGACAGGGTACCAAG |
| **ScFv domain** | | |
| | | GTCGAGATTAAG |
| **BCMA_EBB-C1978-D4- aa** | 1161 | EVQLLETGGGLVQPGGSLRLSCAASGFSFSSYAMSWVRQAPGKGLEWV SAISGSGGSTYYADSVKGRFTISRDNSKNTLYLQMNSLRAEDTAVYYC AKALVGATGAFDIWGQGTLVTVSS |
| **VH** | | |
| **BCMA_EBB-C1978-D4- aa** | 1162 | EIVLTQSPGTLSLSPGERATLSCRASQSLSSNFLAWYQQKPGQAPGLL IYGASNWATGTPDRFSGSGSGTDFTLTITRLEPEDFAVYYCQYYGTSP MYTFGQGTKVEIK |
| **VL** | | |
| **BCMA_EBB-C1978-D4- aa** | 1163 | MALPVTALLLPLALLLHAARPEVQLLETGGGLVQPGGSLRLSCAASGF SFSSYAMSWVRQAPGKGLEWVSAISGSGGSTYYADSVKGRFTISRDNS KNTLYLQMNSLRAEDTAVYYCAKALVGATGAFDIWGQGTLVTVSSGGG GSGGGGSGGGGSEIVLTQSPGTLSLSPGERATLSCRASQSLSSNFLAW YQQKPGQAPGLLIYGASNWATGTPDRFSGSGSGTDFTLTITRLEPEDF AVYYCQYYGTSPMYTFGQGTKVEIKTTTPAPRPPTPAPTIASQPLSLR PEACRPAAGGAVHTRGLDFACDIYIWAPLAGTCGVLLLSLVITLYCKR GRKKLLYIFKQPFMRPVQTTQEEDGCSCRFPEEEEGGCELRVKFSRSA DAPAYKQGQNQLYNELNLGRREEYDVLDKRRGRDPEMGGKPRRKNPQE GLYNELQKDKMAEAYSEIGMKGERRRGKGHDGLYQGLSTATKDTYDAL HMQALPPR |
| **Full CART** | | |
| **BCMA_EBB-C1978-D4- nt** | 1164 | ATGGCCCTCCCTGTCACCGCCCTGCTGCTTCCGCTGGCTCTTCTGCTC CACGCCGCTCGGCCCGAAGTGCAGCTGCTCGAAACCGGTGGAGGGCTG GTGCAGCCAGGGGGCTCCCTGAGGCTTTCATGCGCCGCTAGCGGATTC TCCTTCTCCTCTTACGCCATGTCGTGGGTCCGCCAAGCCCCTGGAAAA GGCCTGGAATGGGTGTCCGCGATTTCCGGGAGCGGAGGTTCGACCTAT TACGCCGACTCCGTGAAGGGCCGCTTTACCATCTCCCGGGATAACTCC AAGAACACTCTGTACCTCCAAATGAACTCGCTGAGAGCCGAGGACACC GCCGTGTATTACTGCGCGAAGGCGCTGGTCGGCGCGACTGGGGCATTC GACATCTGGGGACAGGGAACTCTTGTGACCGTGTCGAGCGGAGGCGGC GGCTCCGGCGGAGGAGGGAGCGGGGGCGGTGGTTCCGAAATCGTGTTG ACTCAGTCCCCGGGAACCCTGAGCTTGTCACCCGGGGAGCGGGCCACT CTCTCCTGTCGCGCCTCCCAATCGCTCTCATCCAATTTCCTGGCCTGG TACCAGCAGAAGCCCGGACAGGCCCCGGGCCTGCTCATCTACGGCGCT TCAAACTGGGCAACGGGAACCCCTGATCGGTTCAGCGGAAGCGGATCG GGTACTGACTTTACCCTGACCATCACCAGACTGGAACCGGAGGACTTC GCCGTGTACTACTGCCAGTACTACGGCACCTCCCCCATGTACACATTC GGACAGGGTACCAAGGTCGAGATTAAGACCACTACCCCAGCACCGAGG CCACCCACCCCGGCTCCTACCATCGCCTCCCAGCCTCTGTCCCTGCGT CCGGAGGCATGTAGACCCGCAGCTGGTGGGGCCGTGCATACCCGGGGT CTTGACTTCGCCTGCGATATCTACATTTGGGCCCCTCTGGCTGGTACT TGCGGGGTCCTGCTGCTTTCACTCGTGATCACTCTTTACTGTAAGCGC GGTCGGAAGAAGCTGCTGTACATCTTTAAGCAACCCTTCATGAGGCCT GTGCAGACTACTCAAGAGGAGGACGGCTGTTCATGCCGGTTCCCAGAG GAGGAGGAAGGCGGCTGCGAACTGCGCGTGAAATTCAGCCGCAGCGCA |
| **Full CART** | | |
| | | GATGCTCCAGCCTACAAGCAGGGGCAGAACCAGCTCTACAACGAACTC AATCTTGGTCGGAGAGAGGAGTACGACGTGCTGGACAAGCGGAGAGGA CGGGACCCAGAAATGGGCGGGAAGCCGCGCAGAAAGAATCCCCAAGAG GGCCTGTACAACGAGCTCCAAAAGGATAAGATGGCAGAAGCCTATAGC GAGATTGGTATGAAAGGGGAACGCAGAAGAGGCAAAGGCCACGACGGA CTGTACCAGGGACTCAGCACCGCCACCAAGGACACCTATGACGCTCTT CACATGCAGGCCCTGCCGCCTCGG |

| **BCMA_EBB-C1980-A2** | | |
|---|---|---|
| **BCMA_EBB-C1980-A2- aa** | 1165 | EVQLLESGGGLVQPGGSLRLSCAASGFTFSSYAMSWVRQAPGKGLEWV SAISGSGGSTYYADSVKGRFTISRDNSKNTLYLQMNSLRAEDTAVYYC VLWFGEGFDPWGQGTLVTVSSGGGGSGGGGSGGGGSDIVLTQSPLSLP VTPGEPASISCRSSQSLLHSNGYNYLDWYLQKPGQSPQLLIYLGSNRA SGVPDRFSGSGSGTDFTLKISRVEAEDVGVYYCMQALQTPLTFGGGTK VDIK |
| **ScFv domain** | | |
| **BCMA_EBB-C1980-A2- nt** | 1166 | GAAGTGCAGCTGCTTGAGAGCGGTGGAGGTCTGGTGCAGCCCGGGGGA TCACTGCGCCTGTCCTGTGCCGCGTCCGGTTTCACTTTCTCCTCGTAC GCCATGTCGTGGGTCAGACAGGCACCGGGAAAGGGACTGGAATGGGTG TCAGCCATTTCGGGTTCGGGGGGCAGCACCTACTACGCTGACTCCGTG AAGGGCCGGTTCACCATTTCCCGCGACAACTCCAAGAACACCTTGTAC CTCCAAATGAACTCCCTGCGGGCCGAAGATACCGCCGTGTATTACTGC GTGCTGTGGTTCGGAGAGGGATTCGACCCGTGGGGACAAGGAACACTC GTGACTGTGTCATCCGGCGGAGGCGGCAGCGGTGGCGGCGGTTCCGGC GGCGGCGGATCTGACATCGTGTTGACCCAGTCCCCTCTGAGCCTGCCG GTCACTCCTGGCGAACCAGCCAGCATCTCCTGCCGGTCGAGCCAGTCC CTCCTGCACTCCAATGGGTACAACTACCTCGATTGGTATCTGCAAAAG CCGGGCCAGAGCCCCCAGCTGCTGATCTACCTTGGGTCAAACCGCGCT TCCGGGGTGCCTGATAGATTCTCCGGGTCCGGGAGCGGAACCGACTTT ACCCTGAAAATCTCGAGGGTGGAGGCCGAGGACGTCGGAGTGTACTAC TGCATGCAGGCGCTCCAGACTCCCCTGACCTTCGGAGGAGGAACGAAG GTCGACATCAAGA |
| **ScFv domain** | | |
| **BCMA_EBB-C1980-A2- aa** | 1167 | EVQLLESGGGLVQPGGSLRLSCAASGFTFSSYAMSWVRQAPGKGLEWV SAISGSGGSTYYADSVKGRFTISRDNSKNTLYLQMNSLRAEDTAVYYC VLWFGEGFDPWGQGTLVTVSS |
| **VH** | | |
| **BCMA_EBB-C1980-A2- aa** | 1168 | DIVLTQSPLSLPVTPGEPASISCRSSQSLLHSNGYNYLDWYLQKPGQS PQLLIYLGSNRASGVPDRFSGSGSGTDFTLKISRVEAEDVGVYYCMQA LQTPLTFGGGTKVDIK |
| **VL** | | |
| **BCMA_EBB-C1980-A2- aa** | 1169 | MALPVTALLLPLALLLHAARPEVQLLESGGGLVQPGGSLRLSCAASGF TFSSYAMSWVRQAPGKGLEWVSAISGSGGSTYYADSVKGRFTISRDNS KNTLYLQMNSLRAEDTAVYYCVLWFGEGFDPWGQGTLVTVSSGGGGSG GGGSGGGGSDIVLTQSPLSLPVTPGEPASISCRSSQSLLHSNGYNYLD |
| **Full CART** | | |
| | | WYLQKPGQSPQLLIYLGSNRASGVPDRFSGSGSGTDFTLKISRVEAED VGVYYCMQALQTPLTFGGGTKVDIKTTTPAPRPPTPAPTIASQPLSLR PEACRPAAGGAVHTRGLDFACDIYIWAPLAGTCGVLLLSLVITLYCKR GRKKLLYIFKQPFMRPVQTTQEEDGCSCRFPEEEEGGCELRVKFSRSA DAPAYKQGQNQLYNELNLGRREEYDVLDKRRGRDPEMGGKPRRKNPQE GLYNELQKDKMAEAYSEIGMKGERRRGKGHDGLYQGLSTATKDTYDAL HMQALPPR |
| **BCMA_EBB-C1980-A2- nt** | 1170 | ATGGCCCTCCCTGTCACCGCCCTGCTGCTTCCGCTGGCTCTTCTGCTC CACGCCGCTCGGCCCGAAGTGCAGCTGCTTGAGAGCGGTGGAGGTCTG GTGCAGCCCGGGGGATCACTGCGCCTGTCCTGTGCCGCGTCCGGTTTC ACTTTCTCCTCGTACGCCATGTCGTGGGTCAGACAGGCACCGGGAAAG GGACTGGAATGGGTGTCAGCCATTTCGGGTTCGGGGGGCAGCACCTAC TACGCTGACTCCGTGAAGGGCCGGTTCACCATTTCCCGCGACAACTCC AAGAACACCTTGTACCTCCAAATGAACTCCCTGCGGGCCGAAGATACC GCCGTGTATTACTGCGTGCTGTGGTTCGGAGAGGGATTCGACCCGTGG GGACAAGGAACACTCGTGACTGTGTCATCCGGCGGAGGCGGCAGCGGT GGCGGCGGTTCCGGCGGCGGCGGATCTGACATCGTGTTGACCCAGTCC CCTCTGAGCCTGCCGGTCACTCCTGGCGAACCAGCCAGCATCTCCTGC CGGTCGAGCCAGTCCCTCCTGCACTCCAATGGGTACAACTACCTCGAT TGGTATCTGCAAAAGCCGGGCCAGAGCCCCCAGCTGCTGATCTACCTT GGGTCAAACCGCGCTTCCGGGGTGCCTGATAGATTCTCCGGGTCCGGG AGCGGAACCGACTTTACCCTGAAAATCTCGAGGGTGGAGGCCGAGGAC GTCGGAGTGTACTACTGCATGCAGGCGCTCCAGACTCCCCTGACCTTC GGAGGAGGAACGAAGGTCGACATCAAGACCACTACCCCAGCACCGAGG CCACCCACCCCGGCTCCTACCATCGCCTCCCAGCCTCTGTCCCTGCGT CCGGAGGCATGTAGACCCGCAGCTGGTGGGGCCGTGCATACCCGGGGT CTTGACTTCGCCTGCGATATCTACATTTGGGCCCCTCTGGCTGGTACT TGCGGGGTCCTGCTGCTTTCACTCGTGATCACTCTTTACTGTAAGCGC GGTCGGAAGAAGCTGCTGTACATCTTTAAGCAACCCTTCATGAGGCCT GTGCAGACTACTCAAGAGGAGGACGGCTGTTCATGCCGGTTCCCAGAG GAGGAGGAAGGCGGCTGCGAACTGCGCGTGAAATTCAGCCGCAGCGCA GATGCTCCAGCCTACAAGCAGGGGCAGAACCAGCTCTACAACGAACTC AATCTTGGTCGGAGAGAGGAGTACGACGTGCTGGACAAGCGGAGAGGA CGGGACCCAGAAATGGGCGGGAAGCCGCGCAGAAAGAATCCCCAAGAG GGCCTGTACAACGAGCTCCAAAAGGATAAGATGGCAGAAGCCTATAGC GAGATTGGTATGAAAGGGGAACGCAGAAGAGGCAAAGGCCACGACGGA CTGTACCAGGGACTCAGCACCGCCACCAAGGACACCTATGACGCTCTT CACATGCAGGCCCTGCCGCCTCGG |
| **Full CART** | | |

| **BCMA_EBB-C1981-C3** | | |
|---|---|---|
| **BCMA_EBB-C1981-C3- aa** | 1171 | QVQLVESGGGLVQPGGSLRLSCAASGFTFSSYAMSWVRQAPGKGLEWV SAISGSGGSTYYADSVKGRFTISRDNSKNTLYLQMNSLRAEDTAVYYC AKVGYDSSGYYRDYYGMDVWGQGTTVTVSSGGGGSGGGGSGGGGSEIV LTQSPGTLSLSPGERATLSCRASQSVSSSYLAWYQQKPGQAPRLLIYG TSSRATGISDRFSGSGSGTDFTLTISRLEPEDFAVYYCQHYGNSPPKF |
| **ScFv domain** | | |
| | | TFGPGTKLEIK |
| **BCMA_EBB-C1981-C3- nt** | 1172 | CAAGTGCAGCTCGTGGAGTCAGGCGGAGGACTGGTGCAGCCCGGGGGC TCCCTGAGACTTTCCTGCGCGGCATCGGGTTTTACCTTCTCCTCCTAT GCTATGTCCTGGGTGCGCCAGGCCCCGGGAAAGGGACTGGAATGGGTG TCCGCAATCAGCGGTAGCGGGGGCTCAACATACTACGCCGACTCCGTC AAGGGTCGCTTCACTATTTCCCGGGACAACTCCAAGAATACCCTGTAC CTCCAAATGAACAGCCTCAGGGCCGAGGATACTGCCGTGTACTACTGC GCCAAAGTCGGATACGATAGCTCCGGTTACTACCGGGACTACTACGGA ATGGACGTGTGGGGACAGGGCACCACCGTGACCGTGTCAAGCGGCGGA GGCGGTTCAGGAGGGGGAGGCTCCGGCGGTGGAGGGTCCGAAATCGTC CTGACTCAGTCGCCTGGCACTCTGTCGTTGTCCCCGGGGGAGCGCGCT ACCCTGTCGTGTCGGGCGTCGCAGTCCGTGTCGAGCTCCTACCTCGCG TGGTACCAGCAGAAGCCCGGACAGGCCCCTAGACTTCTGATCTACGGC ACTTCTTCACGCGCCACCGGGATCAGCGACAGGTTCAGCGGCTCCGGC TCCGGGACCGACTTCACCCTGACCATTAGCCGGCTGGAGCCTGAAGAT TTCGCCGTGTATTACTGCCAACACTACGGAAACTCGCCGCCAAAGTTC ACGTTCGGACCCGGAACCAAGCTGGAAATCAAG |
| **ScFv domain** | | |
| **BCMA_EBB-C1981-C3- aa** | 1173 | QVQLVESGGGLVQPGGSLRLSCAASGFTFSSYAMSWVRQAPGKGLEWV SAISGSGGSTYYADSVKGRFTISRDNSKNTLYLQMNSLRAEDTAVYYC AKVGYDSSGYYRDYYGMDVWGQGTTVTVSS |
| **VH** | | |
| **BCMA_EBB-C1981-C3- aa** | 1174 | EIVLTQSPGTLSLSPGERATLSCRASQSVSSSYLAWYQQKPGQAPRLL IYGTSSRATGISDRFSGSGSGTDFTLTISRLEPEDFAVYYCQHYGNSP PKFTFGPGTKLEIK |
| **VL** | | |
| **BCMA_EBB-C1981-C3- aa** | 1175 | MALPVTALLLPLALLLHAARPQVQLVESGGGLVQPGGSLRLSCAASGF TFSSYAMSWVRQAPGKGLEWVSAISGSGGSTYYADSVKGRFTISRDNS KNTLYLQMNSLRAEDTAVYYCAKVGYDSSGYYRDYYGMDVWGQGTTVT VSSGGGGSGGGGSGGGGSEIVLTQSPGTLSLSPGERATLSCRASQSVS SSYLAWYQQKPGQAPRLLIYGTSSRATGISDRFSGSGSGTDFTLTISR LEPEDFAVYYCQHYGNSPPKFTFGPGTKLEIKTTTPAPRPPTPAPTIA SQPLSLRPEACRPAAGGAVHTRGLDFACDIYIWAPLAGTCGVLLLSLV ITLYCKRGRKKLLYIFKQPFMRPVQTTQEEDGCSCRFPEEEEGGCELR VKFSRSADAPAYKQGQNQLYNELNLGRREEYDVLDKRRGRDPEMGGKP RRKNPQEGLYNELQKDKMAEAYSEIGMKGERRRGKGHDGLYQGLSTAT KDTYDALHMQALPPR |
| **Full CART** | | |
| **BCMA_EBB-C1981-C3- nt** | 1176 | ATGGCCCTCCCTGTCACCGCCCTGCTGCTTCCGCTGGCTCTTCTGCTC CACGCCGCTCGGCCCCAAGTGCAGCTCGTGGAGTCAGGCGGAGGACTG GTGCAGCCCGGGGGCTCCCTGAGACTTTCCTGCGCGGCATCGGGTTTT ACCTTCTCCTCCTATGCTATGTCCTGGGTGCGCCAGGCCCCGGGAAAG GGACTGGAATGGGTGTCCGCAATCAGCGGTAGCGGGGGCTCAACATAC |
| **Full CART** | | |
| | | TACGCCGACTCCGTCAAGGGTCGCTTCACTATTTCCCGGGACAACTCC AAGAATACCCTGTACCTCCAAATGAACAGCCTCAGGGCCGAGGATACT GCCGTGTACTACTGCGCCAAAGTCGGATACGATAGCTCCGGTTACTAC CGGGACTACTACGGAATGGACGTGTGGGGACAGGGCACCACCGTGACC GTGTCAAGCGGCGGAGGCGGTTCAGGAGGGGGAGGCTCCGGCGGTGGA GGGTCCGAAATCGTCCTGACTCAGTCGCCTGGCACTCTGTCGTTGTCC CCGGGGGAGCGCGCTACCCTGTCGTGTCGGGCGTCGCAGTCCGTGTCG AGCTCCTACCTCGCGTGGTACCAGCAGAAGCCCGGACAGGCCCCTAGA CTTCTGATCTACGGCACTTCTTCACGCGCCACCGGGATCAGCGACAGG TTCAGCGGCTCCGGCTCCGGGACCGACTTCACCCTGACCATTAGCCGG CTGGAGCCTGAAGATTTCGCCGTGTATTACTGCCAACACTACGGAAAC TCGCCGCCAAAGTTCACGTTCGGACCCGGAACCAAGCTGGAAATCAAG ACCACTACCCCAGCACCGAGGCCACCCACCCCGGCTCCTACCATCGCC TCCCAGCCTCTGTCCCTGCGTCCGGAGGCATGTAGACCCGCAGCTGGT GGGGCCGTGCATACCCGGGGTCTTGACTTCGCCTGCGATATCTACATT TGGGCCCCTCTGGCTGGTACTTGCGGGGTCCTGCTGCTTTCACTCGTG ATCACTCTTTACTGTAAGCGCGGTCGGAAGAAGCTGCTGTACATCTTT AAGCAACCCTTCATGAGGCCTGTGCAGACTACTCAAGAGGAGGACGGC TGTTCATGCCGGTTCCCAGAGGAGGAGGAAGGCGGCTGCGAACTGCGC GTGAAATTCAGCCGCAGCGCAGATGCTCCAGCCTACAAGCAGGGGCAG AACCAGCTCTACAACGAACTCAATCTTGGTCGGAGAGAGGAGTACGAC GTGCTGGACAAGCGGAGAGGACGGGACCCAGAAATGGGCGGGAAGCCG CGCAGAAAGAATCCCCAAGAGGGCCTGTACAACGAGCTCCAAAAGGAT AAGATGGCAGAAGCCTATAGCGAGATTGGTATGAAAGGGGAACGCAGA AGAGGCAAAGGCCACGACGGACTGTACCAGGGACTCAGCACCGCCACC AAGGACACCTATGACGCTCTTCACATGCAGGCCCTGCCGCCTCGG |

| **BCMA_EBB-C1978-G4** | | |
|---|---|---|
| **BCMA_EBB-C1978-G4- aa** | 1177 | EVQLVESGGGLVQPGGSLRLSCAASGFTFSSYAMSWVRQAPGKGLEWV SAISGSGGSTYYADSVKGRFTISRDNSKNTLYLQMNSLRAEDTAVYYC AKMGWSSGYLGAFDIWGQGTTVTVSSGGGGSGGGGSGGGGSEIVLTQS PGTLSLSPGERATLSCRASQSVASSFLAWYQQKPGQAPRLLIYGASGR ATGIPDRFSGSGSGTDFTLTISRLEPEDFAVYYCQHYGGSPRLTFGGG TKVDIK |
| **ScFv domain** | | |
| **BCMA_EBB-C1978-G4- nt** | 1178 | GAAGTCCAACTGGTGGAGTCCGGGGGAGGGCTCGTGCAGCCCGGAGGC AGCCTTCGGCTGTCGTGCGCCGCCTCCGGGTTCACGTTCTCATCCTAC GCGATGTCGTGGGTCAGACAGGCACCAGGAAAGGGACTGGAATGGGTG TCCGCCATTAGCGGCTCCGGCGGTAGCACCTACTATGCCGACTCAGTG AAGGGAAGGTTCACTATCTCCCGCGACAACAGCAAGAACACCCTGTAC CTCCAAATGAACTCTCTGCGGGCCGAGGATACCGCGGTGTACTATTGC GCCAAGATGGGTTGGTCCAGCGGATACTTGGGAGCCTTCGACATTTGG GGACAGGGCACTACTGTGACCGTGTCCTCCGGGGGTGGCGGATCGGGA GGCGGCGGCTCGGGTGGAGGGGGTTCCGAAATCGTGTTGACCCAGTCA CCGGGAACCCTCTCGCTGTCCCCGGGAGAACGGGCTACACTGTCATGT AGAGCGTCCCAGTCCGTGGCTTCCTCGTTCCTGGCCTGGTACCAGCAG AAGCCGGGACAGGCACCCCGCCTGCTCATCTACGGAGCCAGCGGCCGG |
| **ScFv domain** | | |
| | | GCGACCGGCATCCCTGACCGCTTCTCCGGTTCCGGCTCGGGCACCGAC TTTACTCTGACCATTAGCAGGCTTGAGCCCGAGGATTTTGCCGTGTAC TACTGCCAACACTACGGGGGGAGCCCTCGCCTGACCTTCGGAGGCGGA ACTAAGGTCGATATCAAAA |
| **BCMA_EBB-C1978-G4- aa** | 1179 | EVQLVESGGGLVQPGGSLRLSCAASGFTFSSYAMSWVRQAPGKGLEWV SAISGSGGSTYYADSVKGRFTISRDNSKNTLYLQMNSLRAEDTAVYYC AKMGWSSGYLGAFDIWGQGTTVTVSS |
| **VH** | | |
| **BCMA_EBB-C1978-G4- aa** | 1180 | EIVLTQSPGTLSLSPGERATLSCRASQSVASSFLAWYQQKPGQAPRLL IYGASGRATGIPDRFSGSGSGTDFTLTISRLEPEDFAVYYCQHYGGSP RLTFGGGTKVDIK |
| **VL** | | |
| **BCMA_EBB-C1978-G4- aa** | 1181 | MALPVTALLLPLALLLHAARPEVQLVESGGGLVQPGGSLRLSCAASGF TFSSYAMSWVRQAPGKGLEWVSAISGSGGSTYYADSVKGRFTISRDNS KNTLYLQMNSLRAEDTAVYYCAKMGWSSGYLGAFDIWGQGTTVTVSSG GGGSGGGGSGGGGSEIVLTQSPGTLSLSPGERATLSCRASQSVASSFL AWYQQKPGQAPRLLIYGASGRATGIPDRFSGSGSGTDFTLTISRLEPE DFAVYYCQHYGGSPRLTFGGGTKVDIKTTTPAPRPPTPAPTIASQPLS LRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNP QEGLYNELQKDKMAEAYSEIGMKGERRRGKGHDGLYQGLSTATKDTYD ALHMQALPPR |
| **Full CART** | | |
| **BCMA_EBB-C1978-G4- nt** | 1182 | ATGGCCCTCCCTGTCACCGCCCTGCTGCTTCCGCTGGCTCTTCTGCTC CACGCCGCTCGGCCCGAAGTCCAACTGGTGGAGTCCGGGGGAGGGCTC GTGCAGCCCGGAGGCAGCCTTCGGCTGTCGTGCGCCGCCTCCGGGTTC ACGTTCTCATCCTACGCGATGTCGTGGGTCAGACAGGCACCAGGAAAG GGACTGGAATGGGTGTCCGCCATTAGCGGCTCCGGCGGTAGCACCTAC TATGCCGACTCAGTGAAGGGAAGGTTCACTATCTCCCGCGACAACAGC AAGAACACCCTGTACCTCCAAATGAACTCTCTGCGGGCCGAGGATACC GCGGTGTACTATTGCGCCAAGATGGGTTGGTCCAGCGGATACTTGGGA GCCTTCGACATTTGGGGACAGGGCACTACTGTGACCGTGTCCTCCGGG GGTGGCGGATCGGGAGGCGGCGGCTCGGGTGGAGGGGGTTCCGAAATC GTGTTGACCCAGTCACCGGGAACCCTCTCGCTGTCCCCGGGAGAACGG GCTACACTGTCATGTAGAGCGTCCCAGTCCGTGGCTTCCTCGTTCCTG GCCTGGTACCAGCAGAAGCCGGGACAGGCACCCCGCCTGCTCATCTAC GGAGCCAGCGGCCGGGCGACCGGCATCCCTGACCGCTTCTCCGGTTCC GGCTCGGGCACCGACTTTACTCTGACCATTAGCAGGCTTGAGCCCGAG GATTTTGCCGTGTACTACTGCCAACACTACGGGGGGAGCCCTCGCCTG ACCTTCGGAGGCGGAACTAAGGTCGATATCAAAACCACTACCCCAGCA CCGAGGCCACCCACCCCGGCTCCTACCATCGCCTCCCAGCCTCTGTCC CTGCGTCCGGAGGCATGTAGACCCGCAGCTGGTGGGGCCGTGCATACC CGGGGTCTTGACTTCGCCTGCGATATCTACATTTGGGCCCCTCTGGCT GGTACTTGCGGGGTCCTGCTGCTTTCACTCGTGATCACTCTTTACTGT |
| **Full CART** | | |
| | | AAGCGCGGTCGGAAGAAGCTGCTGTACATCTTTAAGCAACCCTTCATG AGGCCTGTGCAGACTACTCAAGAGGAGGACGGCTGTTCATGCCGGTTC CCAGAGGAGGAGGAAGGCGGCTGCGAACTGCGCGTGAAATTCAGCCGC AGCGCAGATGCTCCAGCCTACAAGCAGGGGCAGAACCAGCTCTACAAC GAACTCAATCTTGGTCGGAGAGAGGAGTACGACGTGCTGGACAAGCGG AGAGGACGGGACCCAGAAATGGGCGGGAAGCCGCGCAGAAAGAATCCC CAAGAGGGCCTGTACAACGAGCTCCAAAAGGATAAGATGGCAGAAGCC TATAGCGAGATTGGTATGAAAGGGGAACGCAGAAGAGGCAAAGGCCAC GACGGACTGTACCAGGGACTCAGCACCGCCACCAAGGACACCTATGAC GCTCTTCACATGCAGGCCCTGCCGCCTCGG |

**Table 11B. Additional exemplary BCMA CAR sequences**

| **Name** | **Sequence** | **SEQ ID NO:** |
|---|---|---|
| **A7D12.2 VH** | QIQLVQSGPDLKKPGETVKLSCKASGYTFTNFGMNWVKQAPGKGFKWMAWINTYTGESYFA DDFKGRFAFSVETSATTAYLQINNLKTEDTATYFCARGEIYYGYDGGFAYWGQGTLVTVSA | 1183 |
| **A7D12.2 VL** | DVVMTQSHRFMSTSVGDRVSITCRASQDVNTAVSWYQQKPGQSPKLLIFSASYRYTGVPDR FTGSGSGADFTLTISSVQAEDLAVYYCQQHYSTPWTFGGGTKLDIK | 1184 |
| **A7D12.2 scFv domain** | QIQLVQSGPDLKKPGETVKLSCKASGYTFTNFGMNWVKQAPGKGFKWMAWINTYTGESYFA DDFKGRFAFSVETSATTAYLQINNLKTEDTATYFCARGEIYYGYDGGFAYWGQGTLVTVSA GGGGSGGGGSGGGGSDVVMTQSHRFMSTSVGDRVSITCRASQDVNTAVSWYQQKPGQSPKL LIFSASYRYTGVPDRFTGSGSGADFTLTISSVQAEDLAVYYCQQHYSTPWTFGGGTKLDIK | 1185 |
| **A7D12.2 Full CART** | QIQLVQSGPDLKKPGETVKLSCKASGYTFTNFGMNWVKQAPGKGFKWMAWINTYTGESYFA DDFKGRFAFSVETSATTAYLQINNLKTEDTATYFCARGEIYYGYDGGFAYWGQGTLVTVSA GGGGSGGGGSGGGGSDVVMTQSHRFMSTSVGDRVSITCRASQDVNTAVSWYQQKPGQSPKL LIFSASYRYTGVPDRFTGSGSGADFTLTISSVQAEDLAVYYCQQHYSTPWTFGGGTKLDIK TTTPAPRPPTPAPTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCELRVKFSRSADAP AYKQGQNQLYNELNLGRREEYDVLDKRRGRDPEMGGKPRRKNPQEGLYNELQKDKMAEAYS EIGMKGERRRGKGHDGLYQGLSTATKDTYDALHMQALPPR | 1186 |
| **C11D5.3 VH** | QIQLVQSGPELKKPGETVKISCKASGYTFTDYSINWVKRAPGKGLKWMGWINTETREPAYA YDFRGRFAFSLETSASTAYLQINNLKYEDTATYFCALDYSYAMDYWGQGTSVTVSS | 1187 |
| **C11D5.3 VL** | DIVLTQSPASLAMSLGKRATISCRASESVSVIGAHLIHWYQQKPGQPPKLLIYLASNLETG VPARFSGSGSGTDFTLTIDPVEEDDVAIYSCLQSRIFPRTFGGGTKLEIK | 1188 |
| **C11D5.3 scFv domain** | QIQLVQSGPELKKPGETVKISCKASGYTFTDYSINWVKRAPGKGLKWMGWINTETREPAYA YDFRGRFAFSLETSASTAYLQINNLKYEDTATYFCALDYSYAMDYWGQGTSVTVSSGGGGS GGGGSGGGGSQIQLVQSGPELKKPGETVKISCKASGYTFTDYSINWVKRAPGKGLKWMGWI NTETREPAYAYDFRGRFAFSLETSASTAYLQINNLKYEDTATYFCALDYSYAMDYWGQGTS VTVSS | 1189 |
| **C11D5.3 Full CART** | QIQLVQSGPELKKPGETVKISCKASGYTFTDYSINWVKRAPGKGLKWMGWINTETREPAYA YDFRGRFAFSLETSASTAYLQINNLKYEDTATYFCALDYSYAMDYWGQGTSVTVSSGGGGS GGGGSGGGGSQIQLVQSGPELKKPGETVKISCKASGYTFTDYSINWVKRAPGKGLKWMGWI NTETREPAYAYDFRGRFAFSLETSASTAYLQINNLKYEDTATYFCALDYSYAMDYWGQGTS VTVSSTTTPAPRPPTPAPTIASQPLSLRPEACRPAAGGAVHTRGLDFACDIYIWAPLAGTC | 1190 |
| | GVLLLSLVITLYCKRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNPQEGLYNELQKDKM AEAYSEIGMKGERRRGKGHDGLYQGLSTATKDTYDALHMQALPPR | |
| **C12A3.2 VH** | QIQLVQSGPELKKPGETVKISCKASGYTFRHYSMNWVKQAPGKGLKWMGRINTESGVPIYA DDFKGRFAFSVETSASTAYLVINNLKDEDTASYFCSNDYLYSLDFWGQGTALTVSS | 1191 |
| **C12A3.2 VL** | DIVLTQSPPSLAMSLGKRATISCRASESVTILGSHLIYWYQQKPGQPPTLLIQLASNVQTG VPARFSGSGSRTDFTLTIDPVEEDDVAVYYCLQSRTIPRTFGGGTKLEIK | 1192 |
| **C12A3.2 scFv domain** | QIQLVQSGPELKKPGETVKISCKASGYTFRHYSMNWVKQAPGKGLKWMGRINTESGVPIYA DDFKGRFAFSVETSASTAYLVINNLKDEDTASYFCSNDYLYSLDFWGQGTALTVSSGGGGS GGGGSGGGGSDIVLTQSPPSLAMSLGKRATISCRASESVTILGSHLIYWYQQKPGQPPTLL IQLASNVQTGVPARFSGSGSRTDFTLTIDPVEEDDVAVYYCLQSRTIPRTFGGGTKLEIK | 1193 |
| **C12A3.2 Full CART** | QIQLVQSGPELKKPGETVKISCKASGYTFRHYSMNWVKQAPGKGLKWMGRINTESGVPIYA DDFKGRFAFSVETSASTAYLVINNLKDEDTASYFCSNDYLYSLDFWGQGTALTVSSGGGGS GGGGSGGGGSDIVLTQSPPSLAMSLGKRATISCRASESVTILGSHLIYWYQQKPGQPPTLL IQLASNVQTGVPARFSGSGSRTDFTLTIDPVEEDDVAVYYCLQSRTIPRTFGGGTKLEIKT TTPAPRPPTPAPTIASQPLSLRPEACRPAAGGAVHTRGLDFACDIYIWAPLAGTCGVLLLS LVITLYCKRGRKKLLYIFKQPFMRPVQTTQEEDGCSCRFPEEEEGGCELRVKFSRSADAPA YKQGQNQLYNELNLGRREEYDVLDKRRGRDPEMGGKPRRKNPQEGLYNELQKDKMAEAYSE IGMKGERRRGKGHDGLYQGLSTATKDTYDALHMQALPPR | 1194 |
| **C13F12.1 VH** | QIQLVQSGPELKKPGETVKISCKASGYTFTHYSMNWVKQAPGKGLKWMGRINTETGEPLYA DDFKGRFAFSLETSASTAYLVINNLKNEDTATFFCSNDYLYSCDYWGQGTTLTVSS | 1195 |
| **C13F12.1 VL** | DIVLTQSPPSLAMSLGKRATISCRASESVTILGSHLIYWYQQKPGQPPTLLIQLASNVQTG VPARFSGSGSRTDFTLTIDPVEEDDVAVYYCLQSRTIPRTFGGGTKLEIK | 1196 |
| **C13F12.1 scFv domain** | QIQLVQSGPELKKPGETVKISCKASGYTFTHYSMNWVKQAPGKGLKWMGRINTETGEPLYA DDFKGRFAFSLETSASTAYLVINNLKNEDTATFFCSNDYLYSCDYWGQGTTLTVSSGGGGS GGGGSGGGGSDIVLTQSPPSLAMSLGKRATISCRASESVTILGSHLIYWYQQKPGQPPTLL IQLASNVQTGVPARFSGSGSRTDFTLTIDPVEEDDVAVYYCLQSRTIPRTFGGGTKLEIK | 1197 |
| **C13F12.1 Full CART** | QIQLVQSGPELKKPGETVKISCKASGYTFTHYSMNWVKQAPGKGLKWMGRINTETGEPLYA DDFKGRFAFSLETSASTAYLVINNLKNEDTATFFCSNDYLYSCDYWGQGTTLTVSSGGGGS GGGGSGGGGSDIVLTQSPPSLAMSLGKRATISCRASESVTILGSHLIYWYQQKPGQPPTLL IQLASNVQTGVPARFSGSGSRTDFTLTIDPVEEDDVAVYYCLQSRTIPRTFGGGTKLEIKT TTPAPRPPTPAPTIASQPLSLRPEACRPAAGGAVHTRGLDFACDIYIWAPLAGTCGVLLLS LVITLYCKRGRKKLLYIFKQPFMRPVQTTQEEDGCSCRFPEEEEGGCELRVKFSRSADAPA YKQGQNQLYNELNLGRREEYDVLDKRRGRDPEMGGKPRRKNPQEGLYNELQKDKMAEAYSE IGMKGERRRGKGHDGLYQGLSTATKDTYDALHMQALPPR | 1198 |

Exemplary BCMA CAR constructs disclose herein comprise an scFv (e.g., a scFv as disclosed in Tables 11A or 11B, optionally preceded with an optional leader sequence (e.g., SEQ ID NO: 2 and SEQ ID NO: 3 or 1938 for exemplary leader amino acid and nucleotide sequences, respectively). The sequences of the scFv fragments (e.g., an ScFv from any of SEQ ID NOs: 967-1182, e.g., SEQ ID NOs: 967, 973, 979, 985, 991, 997, 1003, 1009, 1015, 1021, 1027, 1033, 1039, 1045, 1051, 1057, 1063, 1069, 1075, 1081, 1087, 1093, 1099, 1105, 1111, 1117, 1123, 1129, 1135, 1141, 1147, 1153, 1159, 1165, 1171, 1177, not including the optional leader sequence) are provided herein in Tables 11A or 11B. The BCMA CAR construct can further include an optional hinge domain, e.g., a CD8 hinge domain (e.g., including the amino acid sequence of SEQ ID NO: 4 or encoded by a nucleic acid sequence of SEQ ID NO: 5); a transmembrane domain, e.g., a CD8 transmembrane domain (e.g., including the amino acid sequence of SEQ ID NO: 12 or encoded by the nucleotide sequence of SEQ ID NO: 13 or 1939); an intracellular domain, e.g., a 4-1BB intracellular domain (e.g., including the amino acid sequence of SEQ ID NO: 14 or encoded by the nucleotide sequence of SEQ ID NO: 15 or 1940; and a functional signaling domain, e.g., a CD3 zeta domain (e.g., including amino acid sequence of SEQ ID NO: 18 or 20, or encoded by the nucleotide sequence of SEQ ID NO: 19, 1941, or 21). In certain embodiments, the domains are contiguous with and in the same reading frame to form a single fusion protein. In other embodiments, the domain are in separate polypeptides, e.g., as in an RCAR molecule as described herein.

In certain embodiments, the full length BCMA CAR molecule includes the amino acid sequence of, or is encoded by the nucleotide sequence of, BCMA-1, BCMA-2, BCMA-3, BCMA-4, BCMA-5, BCMA-6, BCMA-7, BCMA-8, BCMA-9, BCMA-10, BCMA-11, BCMA-12, BCMA-13, BCMA-14, BCMA-15, 149362, 149363, 149364, 149365, 149366, 149367, 149368, 149369, BCMA_EBB-C1978-A4, BCMA_EBB-C1978-G1, BCMA_EBB-C1979-C1, BCMA_EBB-C1978-C7, BCMA_EBB-C1978-D10, BCMA_EBB-C1979-C12, BCMA_EBB-C1980-G4, BCMA_EBB-C1980-D2, BCMA_EBB-C1978-A10, BCMA_EBB-C1978-D4, BCMA_EBB-C1980-A2, BCMA_EBB-C1981-C3, BCMA_EBB-C1978-G4, A7D12.2, C11D5.3, C12A3.2, or C13F12.1 provided in Table 11A or 11B, or a sequence substantially (e.g., 85%, 95-99% or higher) identical thereto.

In certain embodiments, the BCMA CAR molecule, or the anti-BCMA antigen binding domain, includes the scFv amino acid sequence of BCMA-1, BCMA-2, BCMA-3, BCMA-4, BCMA-5, BCMA-6, BCMA-7, BCMA-8, BCMA-9, BCMA-10, BCMA-11, BCMA-12, BCMA-13, BCMA-14, BCMA-15, 149362, 149363, 149364, 149365, 149366, 149367, 149368, 149369, BCMA_EBB-C1978-A4, BCMA_EBB-C1978-G1, BCMA_EBB-C1979-C1, BCMA_EBB-C1978-C7, BCMA_EBB-C1978-D10, BCMA_EBB-C1979-C12, BCMA_EBB-C1980-G4, BCMA_EBB-C1980-D2, BCMA_EBB-C1978-A10, BCMA_EBB-C1978-D4, BCMA_EBB-C1980-A2, BCMA_EBB-C1981-C3, BCMA_EBB-C1978-G4, A7D12.2, C11D5.3, C12A3.2, or C13F12.1 provided in Table 11A or 11B (with or without the leader sequence), or a sequence substantially identical (e.g., 85%, 95-99% or higher identical, or up to 20, 15, 10, 8, 6, 5, 4, 3, 2, or 1 amino acid changes, e.g., substitutions (e.g., conservative substitutions)) to any of the aforesaid sequences.

In certain embodiments, the BCMA CAR molecule, or the anti-BCMA antigen binding domain, includes the heavy chain variable region and/or the light chain variable region of BCMA-1, BCMA-2, BCMA-3, BCMA-4, BCMA-5, BCMA-6, BCMA-7, BCMA-8, BCMA-9, BCMA-10, BCMA-11, BCMA-12, BCMA-13, BCMA-14, BCMA-15, 149362, 149363, 149364, 149365, 149366, 149367, 149368, 149369, BCMA_EBB-C1978-A4, BCMA_EBB-C1978-G1, BCMA_EBB-C1979-C1, BCMA_EBB-C1978-C7, BCMA_EBB-C1978-D10, BCMA_EBB-C1979-C12, BCMA_EBB-C1980-G4, BCMA_EBB-C1980-D2, BCMA_EBB-C1978-A10, BCMA_EBB-C1978-D4, BCMA_EBB-C1980-A2, BCMA_EBB-C1981-C3, BCMA_EBB-C1978-G4, A7D12.2, C11D5.3, C12A3.2, or C13F12.1 provided in Table 11A or 11B, or a sequence substantially identical (e.g., 85%, 95-99% or higher identical, or up to 20, 15, 10, 8, 6, 5, 4, 3, 2, or 1 amino acid changes, e.g., substitutions (e.g., conservative substitutions)) to any of the aforesaid sequences.

In certain embodiments, the BCMA CAR molecule, or the anti-BCMA antigen binding domain, includes one, two or three CDRs from the heavy chain variable region (e.g., HCDR1, HCDR2 and/or HCDR3), provided in Table 12; and/or one, two or three CDRs from the light chain variable region (e.g., LCDR1, LCDR2 and/or LCDR3) of BCMA-1, BCMA-2, BCMA-3, BCMA-4, BCMA-5, BCMA-6, BCMA-7, BCMA-8, BCMA-9, BCMA-10, BCMA-11, BCMA-12, BCMA-13, BCMA-14, BCMA-15, 149362, 149363, 149364, 149365, 149366, 149367, 149368, 149369, BCMA_EBB-C1978-A4, BCMA_EBB-C1978-G1, BCMA_EBB-C1979-C1, BCMA_EBB-C1978-C7, BCMA_EBB-C1978-D10, BCMA_EBB-C1979-C12, BCMA_EBB-C1980-G4, BCMA_EBB-C1980-D2, BCMA_EBB-C1978-A10, BCMA_EBB-C1978-D4, BCMA_EBB-C1980-A2, BCMA_EBB-C1981-C3, BCMA_EBB-C1978-G4, A7D12.2, C11D5.3, C12A3.2, or C13F12.1, provided in Table 13; or a sequence substantially identical (e.g., 85%, 95-99% or higher identical, or up to 20, 15, 10, 8, 6, 5, 4, 3, 2, or 1 amino acid changes, e.g., substitutions (e.g., conservative substitutions)) to any of the aforesaid sequences.

In certain embodiments, the BCMA CAR molecule, or the anti-BCMA antigen binding domain, includes one, two or three CDRs from the heavy chain variable region (e.g., HCDR1, HCDR2 and/or HCDR3), provided in Table 14; and/or one, two or three CDRs from the light chain variable region (e.g., LCDR1, LCDR2 and/or LCDR3) of BCMA-1, BCMA-2, BCMA-3, BCMA-4, BCMA-5, BCMA-6, BCMA-7, BCMA-8, BCMA-9, BCMA-10, BCMA-11, BCMA-12, BCMA-13, BCMA-14, BCMA-15, 149362, 149363, 149364, 149365, 149366, 149367, 149368, 149369, BCMA_EBB-C1978-A4, BCMA_EBB-C1978-G1, BCMA_EBB-C1979-C1, BCMA_EBB-C1978-C7, BCMA_EBB-C1978-D10, BCMA_EBB-C1979-C12, BCMA_EBB-C1980-G4, BCMA_EBB-C1980-D2, BCMA_EBB-C1978-A10, BCMA_EBB-C1978-D4, BCMA_EBB-C1980-A2, BCMA_EBB-C1981-C3, BCMA_EBB-C1978-G4, A7D12.2, C11D5.3, C12A3.2, or C13F12.1, provided in Table 15; or a sequence substantially identical (e.g., 85%, 95-99% or higher identical, or up to 20, 15, 10, 8, 6, 5, 4, 3, 2, or 1 amino acid changes, e.g., substitutions (e.g., conservative substitutions)) to any of the aforesaid sequences.

In certain embodiments, the BCMA CAR molecule, or the anti-BCMA antigen binding domain, includes one, two or three CDRs from the heavy chain variable region (e.g., HCDR1, HCDR2 and/or HCDR3), provided in Table 16; and/or one, two or three CDRs from the light chain variable region (e.g., LCDR1, LCDR2 and/or LCDR3) of BCMA-1, BCMA-2, BCMA-3, BCMA-4, BCMA-5, BCMA-6, BCMA-7, BCMA-8, BCMA-9, BCMA-10, BCMA-11, BCMA-12, BCMA-13, BCMA-14, BCMA-15, 149362, 149363, 149364, 149365, 149366, 149367, 149368, 149369, BCMA_EBB-C1978-A4, BCMA_EBB-C1978-G1, BCMA_EBB-C1979-C1, BCMA_EBB-C1978-C7, BCMA_EBB-C1978-D10, BCMA_EBB-C1979-C12, BCMA_EBB-C1980-G4, BCMA_EBB-C1980-D2, BCMA_EBB-C1978-A10, BCMA_EBB-C1978-D4, BCMA_EBB-C1980-A2, BCMA_EBB-C1981-C3, BCMA_EBB-C1978-G4, A7D12.2, C11D5.3, C12A3.2, or C13F12.1, provided in Table 17; or a sequence substantially identical (e.g., 85%, 95-99% or higher identical, or up to 20, 15, 10, 8, 6, 5, 4, 3, 2, or 1 amino acid changes, e.g., substitutions (e.g., conservative substitutions)) to any of the aforesaid sequences.

The sequences of human CDR sequences of the scFv domains are shown in Tables 12, 14, and 16 for the heavy chain variable domains and in Tables 13, 15, and 17 for the light chain variable domains. "ID" stands for the respective SEQ ID NO for each CDR.

**Table 12: Heavy Chain Variable Domain CDRs according to the Kabat numbering scheme (Kabat et al. (1991), "Sequences of Proteins of Immunological Interest," 5th Ed. Public Health Service, National Institutes of Health, Bethesda, MD)**

| **Candidate** | **HCDR1** | **ID** | **HCDR2** | **ID** | **HCDR3** | **ID** |
|---|---|---|---|---|---|---|
| 139109 | NHGMS | 1199 | GIVYSGSTYYAASVKG | 1239 | HGGESDV | 1279 |
| 139103 | NYAMS | 1200 | GISRSGENTYYADSVKG | 1240 | SPAHYYGGMDV | 1280 |
| 139105 | DYAMH | 1201 | GISWNSGSIGYADSVKG | 1241 | HSFLAY | 1281 |
| 139111 | NHGMS | 1202 | GIVYSGSTYYAASVKG | 1242 | HGGESDV | 1282 |
| 139100 | NFGIN | 1203 | WINPKNNNTNYAQKFQG | 1243 | GPYYYQSYMDV | 1283 |
| 139101 | SDAMT | 1204 | VISGSGGTTYYADSVKG | 1244 | LDSSGYYYARGPRY | 1284 |
| 139102 | NYGIT | 1205 | WISAYNGNTNYAQKFQG | 1245 | GPYYYYMDV | 1285 |
| 139104 | NHGMS | 1206 | GIVYSGSTYYAASVKG | 1246 | HGGESDV | 1286 |
| 139106 | NHGMS | 1207 | GIVYSGSTYYAASVKG | 1247 | HGGESDV | 1287 |
| 139107 | NHGMS | 1208 | GIVYSGSTYYAASVKG | 1248 | HGGESDV | 1288 |
| 139108 | DYYMS | 1209 | YISSSGSTIYYADSVKG | 1249 | ESGDGMDV | 1289 |
| 139110 | DYYMS | 1210 | YISSSG NTIYYADSVKG | 1250 | STMVREDY | 1290 |
| 139112 | NHGMS | 1211 | GIVYSGSTYYAASVKG | 1251 | HGGESDV | 1291 |
| 139113 | NHGMS | 1212 | GIVYSGSTYYAASVKG | 1252 | HGGESDV | 1292 |
| 139114 | NHGMS | 1213 | GIVYSGSTYYAASVKG | 1253 | HGGESDV | 1293 |
| 149362 | SSYYYWG | 1214 | SIYYSGSAYYN PSLKS | 1254 | HWQEWPDAFDI | 1294 |
| 149363 | TSGMCVS | 1215 | RIDWDEDKFYSTSLKT | 1255 | SGAGGTSATAFDI | 1295 |
| 149364 | SYSMN | 1216 | SISSSSSYIYYADSVKG | 1256 | TIAAVYAFDI | 1296 |
| 149365 | DYYMS | 1217 | YISSSGSTIYYADSVKG | 1257 | DLRGAFDI | 1297 |
| 149366 | SHYIH | 1218 | MINPSGGVTAYSQTLQG | 1258 | EGSGSGWYFDF | 1298 |
| 149367 | SGGYYWS | 1219 | YIYYSGSTYYN PSLKS | 1259 | AGIAARLRGAFDI | 1299 |
| 149368 | SYAIS | 1220 | GIIPIFGTANYAQKFQG | 1260 | RGGYQLLRWDVGLLR SAFDI | 1300 |
| 149369 | SNSAAWN | 1221 | RTYYRSKWYSFYAISLKS | 1261 | SSPEGLFLYWFDP | 1301 |
| BCMA_EBB-C1978-A4 | SYAMS | 1222 | AISGSGGSTYYADSVKG | 1262 | VEGSGSLDY | 1302 |
| BCMA_EBB-C1978-G1 | RYPMS | 1223 | GISDSGVSTYYADSAKG | 1263 | RAGSEASDI | 1303 |
| BCMA_EBB-C1979-C1 | SYAMS | 1224 | AISGSGGSTYYADSVKG | 1264 | ATYKRELRYYYGMDV | 1304 |
| BCMA_EBB-C1978-C7 | SYAMS | 1225 | AISGSGGSTYYADSVKG | 1265 | ATYKRELRYYYGMDV | 1305 |
| BCMA_EBB-C1978-D10 | DYAMH | 1226 | GISWNSGSIGYADSVKG | 1266 | VGKAVPDV | 1306 |
| BCMA_EBB-C1979-C12 | DYAMH | 1227 | SI N W K6 NS LAYG DSV KG | 1267 | HQGVAYYNYAM DV | 1307 |
| BCMA_EBB-C1980-G4 | SYAMS | 1228 | AISGSGGSTYYADSVKG | 1268 | VVRDGMDV | 1308 |
| BCMA_EBB-C1980-D2 | SYAMS | 1229 | AISGSGGSTYYADSVKG | 1269 | IPQTGTFDY | 1309 |
| BCMA_EBB-C1978-A10 | SYAMS | 1230 | AISGSGGSTYYADSVKG | 1270 | ANYKRELRYYYGMDV | 1310 |
| BCMA_EBB-C1978-D4 | SYAMS | 1231 | AISGSGGSTYYADSVKG | 1271 | ALVGATGAFDI | 1311 |
| BCMA_EBB-C1980-A2 | SYAMS | 1232 | AISGSGGSTYYADSVKG | 1272 | WFGEGFDP | 1312 |
| BCMA_EBB-C1981-C3 | SYAMS | 1233 | AISGSGGSTYYADSVKG | 1273 | VGYDSSGYYRDYYGM DV | 1313 |
| BCMA_EBB-C1978-G4 | SYAMS | 1234 | AISGSGGSTYYADSVKG | 1274 | MGWSSGYLGAFDI | 1314 |
| A7 D 12.2 | NFGMN | 1235 | WINTYTGESYFADDFKG | 1275 | GEIYYGYDGGFAY | 1315 |
| C11D5.3 | DYSIN | 1236 | WINTETREPAYAYDFRG | 1276 | DYSYAMDY | 1316 |
| C12A3.2 | HYSMN | 1237 | RINTESGVPIYADDFKG | 1277 | DYLYSLDF | 1317 |
| C13F12.1 | HYSMN | 1238 | RINTETGEPLYADDFKG | 1278 | DYLYSCDY | 1318 |

**Table 13:**

| Light Chain Variable Domain CDRs according to the Kabat numbering scheme (Kabat et al. (1991), "Sequences of Proteins of Immunological Interest," 5th Ed. Public Health Service, National Institutes of Health, Bethesda, MD) | | | | | | |
|---|---|---|---|---|---|---|
| **Candidate** | **LCDR1** | **ID** | **LCDR2** | **ID** | **LCDR3** | **ID** |
| 139109 | RASQSISSYLN | 1319 | AASSLQS | 1359 | QQSYSTPYT | 1399 |
| 139103 | RASQSISSSFLA | 1320 | GASRRAT | 1360 | QQYHSSPSWT | 1400 |
| 139105 | RSSQSLLHSNGYNYLD | 1321 | LGSNRAS | 1361 | MQALQTPYT | 1401 |
| 139111 | KSSQSLLRNDGKTPLY | 1322 | EVSNRFS | 1362 | MQNIQFPS | 1402 |
| 139100 | RSSQSLLHSNGYNYLN | 1323 | LGSKRAS | 1363 | MQALQTPYT | 1403 |
| 139101 | RASQSISSYLN | 1324 | GASTLAS | 1364 | QQSYKRAS | 1404 |
| 139102 | RSSQSLLYSNGYNYVD | 1325 | LGSNRAS | 1365 | MQGRQFPYS | 1405 |
| 139104 | RASQSVSSNLA | 1326 | GASTRAS | 1366 | QQYGSSLT | 1406 |
| 139106 | RASQSVSSKLA | 1327 | GASIRAT | 1367 | QQYGSSSWT | 1407 |
| 139107 | RASQSVGSTNLA | 1328 | DASNRAT | 1368 | QQYGSSPPWT | 1408 |
| 139108 | RASQSISSYLN | 1329 | AASSLQS | 1369 | QQSYTLA | 1409 |
| 139110 | KSSESLVHNSGKTYLN | 1330 | EVSNRDS | 1370 | MQGTHWPGT | 1410 |
| 139112 | QASEDINKFLN | 1331 | DASTLQT | 1371 | QQYESLPLT | 1411 |
| 139113 | RASQSVGSNLA | 1332 | GASTRAT | 1372 | QQYNDWLPVT | 1412 |
| 139114 | RASQSIGSSSLA | 1333 | GASSRAS | 1373 | QQYAGSPPFT | 1413 |
| 149362 | KASQDIDDAMN | 1334 | SATSPVP | 1374 | LQHDNFPLT | 1414 |
| 149363 | RASQDIYNNLA | 1335 | AANKSQS | 1375 | QHYYRFPYS | 1415 |
| 149364 | RSSQSLLHSNGYNYLD | 1336 | LGSNRAS | 1376 | MQALQTPYT | 1416 |
| 149365 | GGNNIGTKSVH | 1337 | DDSVRPS | 1377 | QVWDSDSEHVV | 1417 |
| 149366 | SGDGLSKKYVS | 1338 | RDKERPS | 1378 | QAWDDTTVV | 1418 |
| 149367 | RASQGIRNWLA | 1339 | AASNLQS | 1379 | QKYNSAPFT | 1419 |
| 149368 | GGNNIGSKSVH | 1340 | GKNNRPS | 1380 | SSRDSSGDHLRV | 1420 |
| 149369 | QGDSLGNYYAT | 1341 | GTNNRPS | 1381 | NSRDSSGHHLL | 1421 |
| BCMA_EBB-C1978-A4 | RASQSVSSAYLA | 1342 | GASTRAT | 1382 | QHYGSSFNGSSLF T | 1422 |
| BCMA_EBB-C1978-G1 | RASQSVSNSLA | 1343 | DASSRAT | 1383 | QQFGTSSG LT | 1423 |
| BCMA_EBB-C1979-C1 | RASQSVSSSFLA | 1344 | GASSRAT | 1384 | QQYHSSPSWT | 1424 |
| BCMA_EBB-C1978-C7 | RASQSVSTTFLA | 1345 | GSSNRAT | 1385 | QQYHSSPSWT | 1425 |
| BCMA_EBB-C1978-D10 | RASQSISSYLN | 1346 | AASSLQS | 1386 | QQSYSTPYS | 1426 |
| BCMA_EBB-C1979-C12 | RATQSIGSSFLA | 1347 | GASQRAT | 1387 | QHYESSPSWT | 1427 |
| BCMA_EBB-C1980-G4 | RASQSVSSSYLA | 1348 | GASSRAT | 1388 | QQYGSPPRFT | 1428 |
| BCMA_EBB-C1980-D2 | RASQSVSSSYLA | 1349 | GASSRAT | 1389 | QHYGSSPSWT | 1429 |
| BCMA_EBB-C1978-A10 | RASQRVASNYLA | 1350 | GASSRAT | 1390 | QHYDSSPSWT | 1430 |
| BCMA_EBB-C1978-D4 | RASQSLSSNFLA | 1351 | GASNWAT | 1391 | QYYGTSPMYT | 1431 |
| BCMA_EBB-C1980-A2 | RSSQSLLHSNGYNYLD | 1352 | LGSNRAS | 1392 | MQALQTPLT | 1432 |
| BCMA_EBB-C1981-C3 | RASQSVSSSYLA | 1353 | GTSSRAT | 1393 | QHYGNSPPKFT | 1433 |
| BCMA_EBB-C1978-G4 | RASQSVASSFLA | 1354 | GASGRAT | 1394 | QHYGGSPRLT | 1434 |
| A7D12.2 | RASQDVNTAVS | 1355 | SASYRYT | 1395 | QQHYSTPWT | 1435 |
| C11D5.3 | RASESVSVIGAHLIH | 1356 | LASNLET | 1396 | LQSRIFPRT | 1436 |
| C12A3.2 | RASESVTILGSHLIY | 1357 | LASNVQT | 1397 | LQSRTIPRT | 1437 |
| C13F12.1 | RASESVTILGSHLIY | 1358 | LAS NVQT | 1398 | LQSRTIPRT | 1438 |

**Table 14:**

| Heavy Chain Variable Domain CDRs according to the Chothia numbering scheme (Al-Lazikani et al., (1997) JMB 273,927-948) | | | | | | |
|---|---|---|---|---|---|---|
| Candidate | HCDR1 | ID | HCDR2 | ID | HCDR3 | ID |
| 139109 | GFALSNH | 1439 | VYSGS | 1479 | HGGESDV | 1519 |
| 139103 | GFTFSNY | 1440 | SRSGEN | 1480 | SPAHYYGGMDV | 1520 |
| 139105 | GFTFDDY | 1441 | SWNSGS | 1481 | HSFLAY | 1521 |
| 139111 | GFALSNH | 1442 | VYSGS | 1482 | HGGESDV | 1522 |
| 139100 | GYIFDNF | 1443 | NPKNNN | 1483 | GPYYYQSYMDV | 1523 |
| 139101 | GFTFSSD | 1444 | SGSGGT | 1484 | LDSSGYYYARGPRY | 1524 |
| 139102 | GYTFSNY | 1445 | SAYNGN | 1485 | GPYYYYMDV | 1525 |
| 139104 | GFALSNH | 1446 | VYSGS | 1486 | HGGESDV | 1526 |
| 139106 | GFALSNH | 1447 | VYSGS | 1487 | HGGESDV | 1527 |
| 139107 | GFALSNH | 1448 | VYSGS | 1488 | HGGESDV | 1528 |
| 139108 | GFTFSDY | 1449 | SSSGST | 1489 | ESGDGMDV | 1529 |
| 139110 | GFTFSDY | 1450 | SSSG NT | 1490 | STMVREDY | 1530 |
| 139112 | GFALSNH | 1451 | VYSGS | 1491 | HGGESDV | 1531 |
| 139113 | GFALSNH | 1452 | VYSGS | 1492 | HGGESDV | 1532 |
| 139114 | GFALSNH | 1453 | VYSGS | 1493 | HGGESDV | 1533 |
| 149362 | GGSISSSYY | 1454 | YYSGS | 1494 | HWQEWPDAFDI | 1534 |
| 149363 | GFSLRTSGM | 1455 | DWDED | 1495 | SGAGGTSATAFDI | 1535 |
| 149364 | GFTFSSY | 1456 | SSSSSY | 1496 | TIAAVYAFDI | 1536 |
| 149365 | GFTFSDY | 1457 | SSSGST | 1497 | DLRGAFDI | 1537 |
| 149366 | GYTVTSH | 1458 | NPSGGV | 1498 | EGSGSGWYFDF | 1538 |
| 149367 | GGSISSGGY | 1459 | YYSGS | 1499 | AGIAARLRGAFDI | 1539 |
| 149368 | GGTFSSY | 1460 | IPIFGT | 1500 | RGGYQLLRWDVGLLRSAF DI | 1540 |
| 149369 | G DSVSSNSA | 1461 | YYRSKWY | 1501 | SSPEGLFLYWFDP | 1541 |
| BCMA_EBB-C1978-A4 | GFTFSSY | 1462 | SGSGGS | 1502 | VEGSGSLDY | 1542 |
| BCMA_EBB-C1978-G1 | GITFSRY | 1463 | SDSGVS | 1503 | RAGSEASDI | 1543 |
| BCMA_EBB-C1979-C1 | G FTFSSY | 1464 | SGSGGS | 1504 | ATYKRELRYYYGMDV | 1544 |
| BCMA_EBB-C1978-C7 | GFTFSSY | 1465 | SGSGGS | 1505 | ATYKRELRYYYGMDV | 1545 |
| BCMA_EBB-C1978-D10 | GFTFDDY | 1466 | SWNSGS | 1506 | VGKAVPDV | 1546 |
| BCMA_EBB-C1979-C12 | GFTFDDY | 1467 | NWKGNS | 1507 | HQGVAYYNYAM DV | 1547 |
| BCMA_EBB-C1980-G4 | GFTFSSY | 1468 | SGSGGS | 1508 | VVRDGMDV | 1548 |
| BCMA_EBB-C1980-D2 | G FTFSSY | 1469 | SGSGGS | 1509 | IPQTGTFDY | 1549 |
| BCMA_EBB-C1978-A10 | GFTFSSY | 1470 | SGSGGS | 1510 | ANYKRELRYYYGMDV | 1550 |
| BCMA_EBB-C1978-D4 | GFSFSSY | 1471 | SGSGGS | 1511 | ALVGATGAFDI | 1551 |
| BCMA_EBB-C1980-A2 | GFTFSSY | 1472 | SGSGGS | 1512 | WFGEGFDP | 1552 |
| BCMA_EBB-C1981-C3 | GFTFSSY | 1473 | SGSGGS | 1513 | VGYDSSGYYRDYYGMDV | 1553 |
| BCMA_EBB-C1978-G4 | GFTFSSY | 1474 | SGSGGS | 1514 | MGWSSGYLGAFDI | 1554 |
| A7D12.2 | GYTFTNF | 1475 | NTYTG E | 1515 | GEIYYGYDGGFAY | 1555 |
| C11D5.3 | GYTFTDY | 1476 | NTETRE | 1516 | DYSYAMDY | 1556 |
| C12A3.2 | GYTFRHY | 1477 | NTESGV | 1517 | DYLYSLDF | 1557 |
| C13F12.1 | GYTFTHY | 1478 | NTETGE | 1518 | DYLYSCDY | 1558 |

**Table 15: Light Chain Variable Domain CDRs according to the Chothia numbering scheme (Al-Lazikani et al., (1997) JMB 273,927-948)**

| Candidate | LCDR1 | ID | LCDR2 | ID | LCDR3 | ID |
|---|---|---|---|---|---|---|
| 139109 | SQSISSY | 1559 | AAS | 1599 | SYSTPY | 1639 |
| 139103 | SQSISSSF | 1560 | GAS | 1600 | YHSSPSW | 1640 |
| 139105 | SQSLLHSNGYNY | 1561 | LGS | 1601 | ALQTPY | 1641 |
| 139111 | SQSLLRNDGKTP | 1562 | EVS | 1602 | NIQFP | 1642 |
| 139100 | SQSLLHSNGYNY | 1563 | LGS | 1603 | ALQTPY | 1643 |
| 139101 | SQSISSY | 1564 | GAS | 1604 | SYKRA | 1644 |
| 139102 | SQSLLYSNGYNY | 1565 | LGS | 1605 | GRQFPY | 1645 |
| 139104 | SQSVSSN | 1566 | GAS | 1606 | YGSSL | 1646 |
| 139106 | SQSVSSK | 1567 | GAS | 1607 | YGSSSW | 1647 |
| 139107 | SQSVGSTN | 1568 | DAS | 1608 | YGSSPPW | 1648 |
| 139108 | SQSISSY | 1569 | AAS | 1609 | SYTL | 1649 |
| 139110 | SESLVHNSGKTY | 1570 | EVS | 1610 | GTHWPG | 1650 |
| 139112 | SEDINKF | 1571 | DAS | 1611 | YESLPL | 1651 |
| 139113 | SQSVGSN | 1572 | GAS | 1612 | YNDWLPV | 1652 |
| 139114 | SQSIGSSS | 1573 | GAS | 1613 | YAGSPPF | 1653 |
| 149362 | SQDIDDA | 1574 | SAT | 1614 | HDNFPL | 1654 |
| 149363 | SQDIYNN | 1575 | AAN | 1615 | YYRFPY | 1655 |
| 149364 | SQSLLHSNGYNY | 1576 | LGS | 1616 | ALQTPY | 1656 |
| 149365 | NNIGTKS | 1577 | DDS | 1617 | WDSDSEHV | 1657 |
| 149366 | DGLSKKY | 1578 | RDK | 1618 | WDDTTV | 1658 |
| 149367 | SQGIRNW | 1579 | AAS | 1619 | YNSAPF | 1659 |
| 149368 | NNIGSKS | 1580 | GKN | 1620 | RDSSGDHLR | 1660 |
| 149369 | DSLGNYY | 1581 | GTN | 1621 | RDSSGHHL | 1661 |
| BCMA_EBB-C1978-A4 | SQSVSSAY | 1582 | GAS | 1622 | YGSSFNGSSLF | 1662 |
| BCMA_EBB-C1978-G1 | SQSVSNS | 1583 | DAS | 1623 | FGTSSGL | 1663 |
| BCMA_EBB-C1979-C1 | SQSVSSSF | 1584 | GAS | 1624 | YHSSPSW | 1664 |
| BCMA_EBB-C1978-C7 | SQSVSTTF | 1585 | GSS | 1625 | YHSSPSW | 1665 |
| BCMA_EBB-C1978-D10 | SQSISSY | 1586 | AAS | 1626 | SYSTPY | 1666 |
| BCMA_EBB-C1979-C12 | TQSIGSSF | 1587 | GAS | 1627 | YESSPSW | 1667 |
| BCMA_EBB-C1980-G4 | SQSVSSSY | 1588 | GAS | 1628 | YGSPPRF | 1668 |
| BCMA_EBB-C1980-D2 | SQSVSSSY | 1589 | GAS | 1629 | YGSSPSW | 1669 |
| BCMA_EBB-C1978-A10 | SQRVASNY | 1590 | GAS | 1630 | YDSSPSW | 1670 |
| BCMA_EBB-C1978-D4 | SQSLSSNF | 1591 | GAS | 1631 | YGTSPMY | 1671 |
| BCMA_EBB-C1980-A2 | SQSLLHSNGYNY | 1592 | LGS | 1632 | ALQTPL | 1672 |
| BCMA_EBB-C1981-C3 | SQSVSSSY | 1593 | GTS | 1633 | YGNSPPKF | 1673 |
| BCMA_EBB-C1978-G4 | SQSVASSF | 1594 | GAS | 1634 | YGGSPRL | 1674 |
| A7 D 12.2 | SQDVNTA | 1595 | SAS | 1635 | HYSTPW | 1675 |
| C11D5.3 | SESVSVIGAHL | 1596 | LAS | 1636 | SRIFPR | 1676 |
| C12A3.2 | SESVTILGSHL | 1597 | LAS | 1637 | SRTIPR | 1677 |
| C13F12.1 | SESVTILGSHL | 1598 | LAS | 1638 | SRTIPR | 1678 |

**Table 16. Heavy Chain Variable Domain CDRs according to a combination of the Kabat numbering scheme (Kabat et al. (1991), "Sequences of Proteins of Immunological Interest," 5th Ed. Public Health Service, National Institutes of Health, Bethesda, MD) and the Chothia numbering scheme (Al-Lazikani et al., (1997) JMB 273,927-948).**

| Candidate | HCDR1 | ID | HCDR2 | ID | HCDR3 | ID |
|---|---|---|---|---|---|---|
| 139109 | GFALSNHGMS | 1679 | GIVYSGSTYYAASVKG | 1719 | HGGESDV | 1759 |
| 139103 | GFTFSNYAMS | 1680 | GISRSGENTYYADSVK G | 1720 | SPAHYYGGMDV | 1760 |
| 139105 | GFTFDDYAMH | 1681 | GISWNSGSIGYADSV KG | 1721 | HSFLAY | 1761 |
| 139111 | GFALSNHGMS | 1682 | GIVYSGSTYYAASVKG | 1722 | HGGESDV | 1762 |
| 139100 | GYIFDNFGIN | 1683 | WINPKNNNTNYAQK FQG | 1723 | G PYYYQSYM DV | 1763 |
| 139101 | GFTFSSDAMT | 1684 | VISGSGGTTYYADSVK G | 1724 | LDSSGYYYARGPRY | 1764 |
| 139102 | GYTFSNYGIT | 1685 | WISAYNGNTNYAQKF QG | 1725 | G PYYYYM DV | 1765 |
| 139104 | GFALSNHGMS | 1686 | GIVYSGSTYYAASVKG | 1726 | HGGESDV | 1766 |
| 139106 | GFALSNHGMS | 1687 | GIVYSGSTYYAASVKG | 1727 | HGGESDV | 1767 |
| 139107 | GFALSNHGMS | 1688 | GIVYSGSTYYAASVKG | 1728 | HGGESDV | 1768 |
| 139108 | GFTFSDYYMS | 1689 | YISSSGSTIYYADSVKG | 1729 | ESGDGMDV | 1769 |
| 139110 | GFTFSDYYMS | 1690 | YISSSGNTIYYADSVK G | 1730 | STMVREDY | 1770 |
| 139112 | GFALSNHGMS | 1691 | GIVYSGSTYYAASVKG | 1731 | HGGESDV | 1771 |
| 139113 | GFALSNHGMS | 1692 | GIVYSGSTYYAASVKG | 1732 | HGGESDV | 1772 |
| 139114 | GFALSNHGMS | 1693 | GIVYSGSTYYAASVKG | 1733 | HGGESDV | 1773 |
| 149362 | GGSISSSYYYWG | 1694 | SIYYSGSAYYNPSLKS | 1734 | HWQEWPDAFDI | 1774 |
| 149363 | GFSLRTSGMCVS | 1695 | RIDWDEDKFYSTSLKT | 1735 | SGAGGTSATAFDI | 1775 |
| 149364 | GFTFSSYSMN | 1696 | SISSSSSYIYYADSVKG | 1736 | TIAAVYAFDI | 1776 |
| 149365 | GFTFSDYYMS | 1697 | YISSSGSTIYYADSVKG | 1737 | DLRGAFDI | 1777 |
| 149366 | GYTVTSHYIH | 1698 | MINPSGGVTAYSQTL QG | 1738 | EGSGSGWYFDF | 1778 |
| 149367 | GGSISSGGYYWS | 1699 | YIYYSGSTYYN PSLKS | 1739 | AGIAARLRGAFDI | 1779 |
| 149368 | GGTFSSYAIS | 1700 | GIIPIFGTANYAQKFQ G | 1740 | RGGYQLLRWDVGLLR SAFDI | 1780 |
| 149369 | GDSVSSNSAAWN | 1701 | RTYYRSKWYSFYAISL KS | 1741 | SSPEGLFLYWFDP | 1781 |
| BCMA_EBB-C1978-A4 | GFTFSSYAMS | 1702 | AISGSGGSTYYADSVK G | 1742 | VEGSGSLDY | 1782 |
| BCMA_EBB-C1978-G1 | GITFSRYPMS | 1703 | GISDSGVSTYYADSAK G | 1743 | RAGSEASDI | 1783 |
| BCMA_EBB-C1979-C1 | GFTFSSYAMS | 1704 | AISGSGGSTYYADSVK G | 1744 | ATYKRELRYYYGMDV | 1784 |
| BCMA_EBB-C1978-C7 | GFTFSSYAMS | 1705 | AISGSGGSTYYADSVK G | 1745 | ATYKRELRYYYGMDV | 1785 |
| BCMA_EBB-C1978-D10 | GFTFDDYAMH | 1706 | GISWNSGSIGYADSV KG | 1746 | VGKAVPDV | 1786 |
| BCMA_EBB-C1979-C12 | GFTFDDYAMH | 1707 | SINWKGNSLAYGDSV KG | 1747 | HQGVAYYNYAMDV | 1787 |
| BCMA_EBB-C1980-G4 | GFTFSSYAMS | 1708 | AISGSGGSTYYADSVK G | 1748 | VVRDGMDV | 1788 |
| BCMA_EBB-C1980-D2 | GFTFSSYAMS | 1709 | AISGSGGSTYYADSVK G | 1749 | IPQTGTFDY | 1789 |
| BCMA_EBB-C1978-A10 | GFTFSSYAMS | 1710 | AISGSGGSTYYADSVK G | 1750 | ANYKRELRYYYGMDV | 1790 |
| BCMA_EBB-C1978-D4 | GFSFSSYAMS | 1711 | AISGSGGSTYYADSVK G | 1751 | ALVGATGAFDI | 1791 |
| BCMA_EBB-C1980-A2 | GFTFSSYAMS | 1712 | AISGSGGSTYYADSVK G | 1752 | WFGEGFDP | 1792 |
| BCMA_EBB-C1981-C3 | GFTFSSYAMS | 1713 | AISGSGGSTYYADSVK G | 1753 | VGYDSSGYYRDYYGM DV | 1793 |
| BCMA_EBB-C1978-G4 | GFTFSSYAMS | 1714 | AISGSGGSTYYADSVK G | 1754 | MGWSSGYLGAFDI | 1794 |
| A7 D 12.2 | GYTFTNFGMN | 1715 | WINTYTGESYFADDF KG | 1755 | GEIYYGYDGGFAY | 1795 |
| C11D5.3 | GYTFTDYSIN | 1716 | WINTETREPAYAYDF | 1756 | DYSYAMDY | 1796 |
| | | | RG | | | |
| C12A3.2 | GYTFRHYSMN | 1717 | RINTESGVPIYADDFK G | 1757 | DYLYSLDF | 1797 |
| C13F12.1 | GYTFTHYSMN | 1718 | RINTETGEPLYADDFK G | 1758 | DYLYSCDY | 1798 |

**Table 17. Light Chain Variable Domain CDRs according to a combination of the Kabat numbering scheme (Kabat et al. (1991), "Sequences of Proteins of Immunological Interest," 5th Ed. Public Health Service, National Institutes of Health, Bethesda, MD) and the Chothia numbering scheme (Al-Lazikani et al., (1997) JMB 273,927-948).**

| Candidate | LCDR1 | ID | LCDR2 | ID | LCDR3 | ID |
|---|---|---|---|---|---|---|
| 139109 | RASQSISSYLN | 1799 | AASSLQS | 1839 | QQSYSTPYT | 1879 |
| 139103 | RASQSISSSFLA | 1800 | GASRRAT | 1840 | QQYHSSPSWT | 1880 |
| 139105 | RSSQSLLHSNGYNYLD | 1801 | LGSNRAS | 1841 | MQALQTPYT | 1881 |
| 139111 | KSSQSLLRNDGKTPLY | 1802 | EVSNRFS | 1842 | MQNIQFPS | 1882 |
| 139100 | RSSQSLLHSNGYNYLN | 1803 | LGSKRAS | 1843 | MQALQTPYT | 1883 |
| 139101 | RASQSISSYLN | 1804 | GASTLAS | 1844 | QQSYKRAS | 1884 |
| 139102 | RSSQSLLYSNGYNYVD | 1805 | LGSNRAS | 1845 | MQGRQFPYS | 1885 |
| 139104 | RASQSVSSNLA | 1806 | GASTRAS | 1846 | QQYGSSLT | 1886 |
| 139106 | RASQSVSSKLA | 1807 | GASIRAT | 1847 | QQYGSSSWT | 1887 |
| 139107 | RASQSVGSTNLA | 1808 | DASNRAT | 1848 | QQYGSSPPWT | 1888 |
| 139108 | RASQSISSYLN | 1809 | AASSLQS | 1849 | QQSYTLA | 1889 |
| 139110 | KSSESLVHNSGKTYLN | 1810 | EVSNRDS | 1850 | MQGTHWPGT | 1890 |
| 139112 | QASEDINKFLN | 1811 | DASTLQT | 1851 | QQYESLPLT | 1891 |
| 139113 | RASQSVGSNLA | 1812 | GASTRAT | 1852 | QQYNDWLPVT | 1892 |
| 139114 | RASQSIGSSSLA | 1813 | GASSRAS | 1853 | QQYAGSPPFT | 1893 |
| 149362 | KASQDIDDAMN | 1814 | SATSPVP | 1854 | LQHDNFPLT | 1894 |
| 149363 | RASQDIYNNLA | 1815 | AANKSQS | 1855 | QHYYRFPYS | 1895 |
| 149364 | RSSQSLLHSNGYNYLD | 1816 | LGSNRAS | 1856 | MQALQTPYT | 1896 |
| 149365 | GGNNIGTKSVH | 1817 | DDSVRPS | 1857 | QVWDSDSEHVV | 1897 |
| 149366 | SGDGLSKKYVS | 1818 | RDKERPS | 1858 | QAWDDTTVV | 1898 |
| 149367 | RASQGIRNWLA | 1819 | AASNLQS | 1859 | QKYNSAPFT | 1899 |
| 149368 | GGNNIGSKSVH | 1820 | GKNNRPS | 1860 | SSRDSSGDHLRV | 1900 |
| 149369 | QGDSLGNYYAT | 1821 | GTNNRPS | 1861 | NSRDSSGHHLL | 1901 |
| BCMA_EBB-C1978-A4 | RASQSVSSAYLA | 1822 | GASTRAT | 1862 | QHYGSSFNGSSLF T | 1902 |
| BCMA_EBB-C1978-G1 | RASQSVSNSLA | 1823 | DASSRAT | 1863 | QQFGTSSGLT | 1903 |
| BCMA_EBB-C1979-C1 | RASQSVSSSFLA | 1824 | GASSRAT | 1864 | QQYHSSPSWT | 1904 |
| BCMA_EBB-C1978-C7 | RASQSVSTTFLA | 1825 | GSSNRAT | 1865 | QQYHSSPSWT | 1905 |
| BCMA_EBB-C1978-D10 | RASQSISSYLN | 1826 | AASSLQS | 1866 | QQSYSTPYS | 1906 |
| BCMA_EBB-C1979-C12 | RATQSIGSSFLA | 1827 | GASQRAT | 1867 | QHYESSPSWT | 1907 |
| BCMA_EBB-C1980-G4 | RASQSVSSSYLA | 1828 | GASSRAT | 1868 | QQYGSPPRFT | 1908 |
| BCMA_EBB-C1980-D2 | RASQSVSSSYLA | 1829 | GASSRAT | 1869 | QHYGSSPSWT | 1909 |
| BCMA_EBB-C1978-A10 | RASQRVASNYLA | 1830 | GASSRAT | 1870 | QHYDSSPSWT | 1910 |
| BCMA_EBB-C1978-D4 | RASQSLSSNFLA | 1831 | GASNWAT | 1871 | QYYGTSPMYT | 1911 |
| BCMA_EBB-C1980-A2 | RSSQSLLHSNGYNYLD | 1832 | LGSNRAS | 1872 | MQALQTPLT | 1912 |
| BCMA_EBB-C1981-C3 | RASQSVSSSYLA | 1833 | GTSSRAT | 1873 | QHYGNSPPKFT | 1913 |
| BCMA_EBB-C1978-G4 | RASQSVASSFLA | 1834 | GASGRAT | 1874 | QHYGGSPRLT | 1914 |
| A7 D 12.2 | RASQDVNTAVS | 1835 | SASYRYT | 1875 | QQHYSTPWT | 1915 |
| C11D5.3 | RASESVSVIGAHLIH | 1836 | LASNLET | 1876 | LQSRIFPRT | 1916 |
| C12A3.2 | RASESVTILGSHLIY | 1837 | LASNVQT | 1877 | LQSRTIPRT | 1917 |
| C13F12.1 | RASESVTILGSHLIY | 1838 | LASNVQT | 1878 | LQSRTIPRT | 1918 |

In certain embodiments, the CAR molecule described herein (*e.g*., the CAR nucleic acid or the CAR polypeptide) or a BCMA binding domain includes:
(1) one, two, or three light chain (LC) CDRs chosen from one of the following:
   (i) a LC CDR1 of SEQ ID NO: 1320, LC CDR2 of SEQ ID NO: 1360 and LC CDR3 of SEQ ID NO: 1400 of BCMA-4 CAR (139103);
   (ii) a LC CDR1 of SEQ ID NO: 1319, LC CDR2 of SEQ ID NO: 1359 and LC CDR3 of SEQ ID NO: 1399 of BCMA-10 CAR (139109);
   (iii) a LC CDR1 of SEQ ID NO: 1331, LC CDR2 of SEQ ID NO: 1371and LC CDR3 of SEQ ID NO: 1411of BCMA-13 CAR (139112); or
   (iv) a LC CDR1 of SEQ ID NO: 1333, LC CDR2 of SEQ ID NO: 1373 and LC CDR3 of SEQ ID NO: 1413 of BCMA-15 CAR (139114), and/or
(2) one, two, or three heavy chain (HC) CDRs from one of the following:
   (i) a HC CDR1 of SEQ ID NO: 1200, HC CDR2 of SEQ ID NO: 1240 and HC CDR3 of SEQ ID NO: 1280 of BCMA-4 CAR (139103);
   (ii) a HC CDR1 of SEQ ID NO: 1199, HC CDR2 of SEQ ID NO: 1239 and HC CDR3 of SEQ ID NO: 1279 of BCMA-10 CAR (139109);
   (iii) a HC CDR1 of SEQ ID NO: 1121, HC CDR2 of SEQ ID NO: 1251 and HC CDR3 of SEQ ID NO: 1291 of BCMA-13 CAR (139112); or
   (iv) a HC CDR1 of SEQ ID NO: 1213, HC CDR2 of SEQ ID NO: 1253 and HC CDR3 of SEQ ID NO: 1293 of BCMA-15 (139114).

In certain embodiments, the CAR molecule described herein (*e.g.*, the CAR nucleic acid or the CAR polypeptide) includes:
(1) one, two, or three light chain (LC) CDRs chosen from one of the following:
   (i) a LC CDR1 of SEQ ID NO: 1560, LC CDR2 of SEQ ID NO: 1600 and LC CDR3 of SEQ ID NO: 1640 of BCMA-4 CAR (139103);
   (ii) a LC CDR1 of SEQ ID NO: 1559, LC CDR2 of SEQ ID NO: 1599 and LC CDR3 of SEQ ID NO: 1639 of BCMA-10 CAR (139109);
   (iii) a LC CDR1 of SEQ ID NO: 1571, LC CDR2 of SEQ ID NO: 1611 and LC CDR3 of SEQ ID NO: 1651 of BCMA-13 CAR (139112); or
   (iv) a LC CDR1 of SEQ ID NO: 1573, LC CDR2 of SEQ ID NO: 1613 and LC CDR3 of SEQ ID NO: 1653 of BCMA-15 CAR (139114); and/or
(2) one, two, or three heavy chain (HC) CDRs chosen from one of the following:
   (i) a HC CDR1 of SEQ ID NO: 1440, HC CDR2 of SEQ ID NO: 1480 and HC CDR3 of SEQ ID NO: 1520 of BCMA-4 CAR (139103);
   (ii) a HC CDR1 of SEQ ID NO: 1439, HC CDR2 of SEQ ID NO: 1479 and HC CDR3 of SEQ ID NO: 1519 of BCMA-10 CAR (139109);
   (iii) a HC CDR1 of SEQ ID NO: 1451, HC CDR2 of SEQ ID NO: 1491 and HC CDR3 of SEQ ID NO: 1531 of BCMA-13 CAR (139112); or
   (iv) a HC CDR1 of SEQ ID NO: 1453, HC CDR2 of SEQ ID NO: 1493 and HC CDR3 of SEQ ID NO: 1533 of BCMA-15 CAR (139114).

In certain embodiments, the CAR molecule described herein (*e.g.*, the CAR nucleic acid or the CAR polypeptide) includes:
(1) one, two, or three light chain (LC) CDRs chosen from one of the following:
   (i) a LC CDR1 of SEQ ID NO: 1800 LC CDR2 of SEQ ID NO: 1840 and LC CDR3 of SEQ ID NO: 1880 of BCMA-4 CAR (139103);
   (ii) a LC CDR1 of SEQ ID NO: 1799, LC CDR2 of SEQ ID NO: 1839 and LC CDR3 of SEQ ID NO: 1879 of BCMA-10 CAR (139109);
   (iii) a LC CDR1 of SEQ ID NO: 1811, LC CDR2 of SEQ ID NO: 1851 and LC CDR3 of SEQ ID NO: 1891 of BCMA-13 CAR (139112); or
   (iv) a LC CDR1 of SEQ ID NO: 1813, LC CDR2 of SEQ ID NO: 1853 and LC CDR3 of SEQ ID NO: 1893 of BCMA-15 CAR (139114); and/or
(2) one, two, or three heavy chain (HC) CDRs chosen from one of the following:
   (i) a HC CDR1 of SEQ ID NO: 1680, HC CDR2 of SEQ ID NO: 1720 and HC CDR3 of SEQ ID NO: 1760 of BCMA-4 CAR (139103);
   (ii) a HC CDR1 of SEQ ID NO: 1679, HC CDR2 of SEQ ID NO: 1719 and HC CDR3 of SEQ ID NO: 1759 of BCMA-10 CAR (139109);
   (iii) a HC CDR1 of SEQ ID NO: 1691, HC CDR2 of SEQ ID NO: 1731 and HC CDR3 of SEQ ID NO: 1771 of BCMA-13 CAR (139112);
   (iv) a HC CDR1 of SEQ ID NO: 1693, HC CDR2 of SEQ ID NO: 1733 and HC CDR3 of SEQ ID NO: 1773 of BCMA-15 CAR (139114).

Exemplary components of the CAR molecules:
**Leader (amino acid sequence) (SEQ ID NO: 1919)**
   MALPVTALLLPLALLLHAARP
**leader (nucleic acid sequence) (SEQ ID NO: 1920)**
   ATGGCCCTGCCTGTGACAGCCCTGCTGCTGCCTCTGGCTCTGCTGCTGCATGCCGCTAGACCC
**CDS hinge (amino acid sequence) (SEQ ID NO: 1921)**
   TTTPAPRPPTPAPTIASQPLSLRPEACRPAAGGAVHTRGLDFACD
**CD8 hinge (nucleic acid sequence) (SEQ ID NO: 1922)** 
ACCACGACGCCAGCGCCGCGACCACCAACACCGGCGCCCACCATCGCGTCGCAGCCCCTGTCCCTGCGCCCAGAG
GCGTGCCGGCCAGCGGCGGGGGGCGCAGTGCACACGAGGGGGCTGGACTTCGCCTGTGAT
**CD8 transmembrane (amino acid sequence) (SEQ ID NO: 1923)**
   IYIWAPLAGTCGVLLLSLVITLYC
**CD8 transmembrane (nucleic acid sequence) (SEQ ID NO: 1924)**
   ATCTACATCTGGGCGCCCTTGGCCGGGACTTGTGGGGTCCTTCTCCTGTCACTGGTTATCACCCTTTACTGC
**4-1BB Intracellular domain (amino acid sequence) (SEQ ID NO: 1925)**
   KRGRKKLLYIFKQPFMRPVQTTQEEDGCSCRFPEEEEGGCEL
**4-1BB Intracellular domain (nucleic acid sequence) (SEQ ID NO: 1926)** 
AAACGGGGCAGAAAGAAACTCCTGTATATATTCAAACAACCATTTATGAGACCAGTACAAACTACTCAAGAGGAA
GATGGCTGTAGCTGCCGATTTCCAGAAGAAGAAGAAGGAGGATGTGAACTG
**CD28 Intracellular domain (amino acid sequence) (SEQ ID NO: 1927)**
   RSKRSRLLHSDYMNMTPRRPGPTRKHYQPYAPPRDFAAYRS (SEQ ID NO: 1927)
**CD28 Intracellular domain (nucleotide sequence) (SEQ ID NO: 1928)** 
AGGAGTAAGAGGAGCAGGCTCCTGCACAGTGACTACATGAACATGACTCCCCGCCGCCCCGGGCCCACCCGC
AAGCATTACCAGCCCTATGCCCCACCACGCGACTTCGCAGCCTATCGCTCC (SEQ ID NO: 1928)
**ICOS Intracellular domain (amino acid sequence) (SEQ ID NO: 1929)**
   T K K K Y S S S V H D P N G E Y M F M R A V N T A K K S R L T D V T L (SEQ ID NO: 1929)
**ICOS Intracellular domain (nucleotide sequence) (SEQ ID NO: 1930)** 
ACAAAAAAGAAGTATTCATCCAGTGTGCACGACCCTAACGGTGAATACATGTTCATGAGAGCAGTGAACACA
GCCAAAAAATCCAGACTCACAGATGTGACCCTA (SEQ ID NO: 1930)
**CD3 zeta domain (amino acid sequence) (SEQ ID NO: 1931)** 
RVKFSRSADAPAYKQGQNQLYNELNLGRREEYDVLDKRRGRDPEMGGKPRRKNPQEGLYNELQKDKMAEAYSEIGM
KGERRRGKGHDGLYQGLSTATKDTYDALHMQALPPR
**CD3 zeta (nucleic acid sequence) (SEQ ID NO: 1932)** 
AGAGTGAAGTTCAGCAGGAGCGCAGACGCCCCCGCGTACAAGCAGGGCCAGAACCAGCTCTATAACGAGCTCAA
TCTAGGACGAAGAGAGGAGTACGATGTTTTGGACAAGAGACGTGGCCGGGACCCTGAGATGGGGGGAAAGCCG
AGAAGGAAGAACCCTCAGGAAGGCCTGTACAATGAACTGCAGAAAGATAAGATGGCGGAGGCCTACAGTGAGAT
TGGGATGAAAGGCGAGCGCCGGAGGGGCAAGGGGCACGATGGCCTTTACCAGGGTCTCAGTACAGCCACCAAG
GACACCTACGACGCCCTTCACATGCAGGCCCTGCCCCCTCGC
**CD3 zeta domain (amino acid sequence; NCBI Reference NM_000734.3) (SEQ ID NO: 1933)** 
RVKFSRSADAPAYQQGQNQLYNELNLGRREEYDVLDKRRGRDPEMGGKPRRKNPQEGLYNELQKDKMAEAYSEIGM
KGERRRGKGHDGLYQGLSTATKDTYDALHMQALPPR
**CD3 zeta (nucleic acid sequence; NCBI Reference Sequence NM_000734.3); (SEQ ID NO: 1934)** 
AGAGTGAAGTTCAGCAGGAGCGCAGACGCCCCCGCGTACCAGCAGGGCCAG
AACCAGCTCTATAACGAGCTCAATCTAGGACGAAGAGAGGAGTACGATGTTT
TGGACAAGAGACGTGGCCGGGACCCTGAGATGGGGGGAAAGCCGAGAAGGA
 
AGAACCCTCAGGAAGGCCTGTACAATGAACTGCAGAAAGATAAGATGGCGG
AGGCCTACAGTGAGATTGGGATGAAAGGCGAGCGCCGGAGGGGCAAGGGGC
ACGATGGCCTTTACCAGGGTCTCAGTACAGCCACCAAGGACACCTACGACGC
CCTTCACATGCAGGCCCTGCCCCCTCGC
**IgG4 Hinge (amino acid sequence) (SEQ ID NO: 1935)** 
ESKYGPPCPPCPAPEFLGGPSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQEEMTKNQVSLTCLVKGFYPSD
IAVEWESNGQPENNYKTTPPVLDSDGSFFLYSRLTVDKSRWQEGNVFSCSVMHEALHNHYTQKSLSLSLGKM
**IgG4 Hinge (nucleotide sequence) (SEQ ID NO: 1936)**

GAGAGCAAGTACGGCCCTCCCTGCCCCCCTTGCCCTGCCCCCGAGTTCCTGGGCGGACCCAGCGTGTTCCTGTTCC
CCCCCAAGCCCAAGGACACCCTGATGATCAGCCGGACCCCCGAGGTGACCTGTGTGGTGGTGGACGTGTCCCAGG
AGGACCCCGAGGTCCAGTTCAACTGGTACGTGGACGGCGTGGAGGTGCACAACGCCAAGACCAAGCCCCGGGAG
GAGCAGTTCAATAGCACCTACCGGGTGGTGTCCGTGCTGACCGTGCTGCACCAGGACTGGCTGAACGGCAAGGA
ATACAAGTGTAAGGTGTCCAACAAGGGCCTGCCCAGCAGCATCGAGAAAACCATCAGCAAGGCCAAGGGCCAGC
CTCGGGAGCCCCAGGTGTACACCCTGCCCCCTAGCCAAGAGGAGATGACCAAGAACCAGGTGTCCCTGACCTGCC
TGGTGAAGGGCTTCTACCCCAGCGACATCGCCGTGGAGTGGGAGAGCAACGGCCAGCCCGAGAACAACTACAAG
ACCACCCCCCCTGTGCTGGACAGCGACGGCAGCTTCTTCCTGTACAGCCGGCTGACCGTGGACAAGAGCCGGTGG
CAGGAGGGCAACGTCTTTAGCTGCTCCGTGATGCACGAGGCCCTGCACAACCACTACACCCAGAAGAGCCTGAGC
CTGTCCCTGGGCAAGATG

In an embodiment, the CAR molecule comprises a mesothelin CAR described herein, e.g., a mesothelin CAR described in WO 2015/090230. In embodiments, the mesothelin CAR comprises an amino acid, or has a nucleotide sequence shown in WO 2015/090230, or a sequence substantially identical to any of the aforesaid sequences (e.g., at least 85%, 90%, 95% or more identical to any of the aforesaid mesothelin CAR sequences). In one embodiment, the CAR molecule comprises a mesothelin CAR, or an antigen binding domain according to Tables 2-3 of WO 2015/090230, or a sequence substantially identical thereto (e.g., at least 85%, 90%, 95% or more identical thereto). The amino acid and nucleotide sequences encoding the mesothelin CAR molecules and antigen binding domains (e.g., including one, two, three VH CDRs; and one, two, three VL CDRs according to Kabat or Chothia), are specified in WO 2015/090230.

In an embodiment, the CAR molecule comprises a CLL1 CAR described herein, e.g., a CLL1 CAR described in US2016/0051651A1. In embodiments, the CLL1 CAR comprises an amino acid, or has a nucleotide sequence shown in US2016/0051651A1, or a sequence substantially identical to any of the aforesaid sequences (e.g., at least 85%, 90%, 95% or more identical to any of the aforesaid CLL1 CAR sequences).

In other embodiments, the CLL1 CAR includes a CAR molecule, or an antigen binding domain according to Table 2 of WO2016/014535, or a sequence substantially identical to any of the aforesaid sequences (e.g., at least 85%, 90%, 95% or more identical to any of the aforesaid CLL1 CAR sequences). The amino acid and nucleotide sequences encoding the CLL-1 CAR molecules and antigen binding domains (e.g., including one, two, three VH CDRs; and one, two, three VL CDRs according to Kabat or Chothia), are specified in WO2016/014535.

In an embodiment, the CAR molecule comprises a CD33 CAR described herein, e.g., a CD33 CAR described in US2016/0096892A1. In embodiments, the CD33 CAR comprises an amino acid, or has a nucleotide sequence shown in US2016/0096892A1, or a sequence substantially identical to any of the aforesaid sequences (e.g., at least 85%, 90%, 95% or more identical to any of the aforesaid CD33 CAR sequences). In other embodiments, the CD33 CAR CAR or antigen binding domain thereof can include a CAR molecule (e.g., any of CAR33-1 to CAR-33-9), or an antigen binding domain according to Table 2 or 9 of WO2016/014576, or a sequence substantially identical to any of the aforesaid sequences (e.g., at least 85%, 90%, 95% or more identical to any of the aforesaid CD33 CAR sequences). The amino acid and nucleotide sequences encoding the CD33 CAR molecules and antigen binding domains (e.g., including one, two, three VH CDRs; and one, two, three VL CDRs according to Kabat or Chothia), are specified in WO2016/014576.

In embodiments, the CAR molecule comprises a CD123 CAR described herein, e.g., a CD123 CAR described in US2014/0322212A1 or US2016/0068601A1. In embodiments, the CD123 CAR comprises an amino acid, or has a nucleotide sequence shown in US2014/0322212A1 or US2016/0068601A1, or a sequence substantially identical to any of the aforesaid sequences (e.g., at least 85%, 90%, 95% or more identical to any of the aforesaid CD123 CAR sequences). In one embodiment, the CAR molecule comprises a CD123 CAR (*e.g*., any of the CAR1-CAR8), or an antigen binding domain according to Tables 1-2 of WO 2014/130635, or a sequence substantially identical thereto (e.g., at least 85%, 90%, 95% or more identical to any of the aforesaid CD123 CAR sequences). The amino acid and nucleotide sequences encoding the CD123 CAR molecules and antigen binding domains (e.g., including one, two, three VH CDRs; and one, two, three VL CDRs according to Kabat or Chothia), are specified in WO 2014/130635.

In other embodiments, the CAR molecule comprises a CD123 CAR comprises a CAR molecule (e.g., any of the CAR123-1 to CAR123-4 and hzCAR123-1 to hzCAR123-32), or an antigen binding domain according to Tables 2, 6, and 9 of WO2016/028896, or a sequence substantially identical thereto (e.g., at least 85%, 90%, 95% or more identical to any of the aforesaid CD123 CAR sequences). The amino acid and nucleotide sequences encoding the CD123 CAR molecules and antigen binding domains (e.g., including one, two, three VH CDRs; and one, two, three VL CDRs according to Kabat or Chothia), are specified in WO2016/028896.

In an embodiment, the CAR molecule comprises an EGFRvIII CAR molecule described herein, e.g., an EGFRvIII CAR described US2014/0322275A1. In embodiments, the EGFRvIII CAR comprises an amino acid, or has a nucleotide sequence shown in US2014/0322275A1, or a sequence substantially identical to any of the aforesaid sequences (e.g., at least 85%, 90%, 95% or more identical to any of the aforesaid EGFRvIII CAR sequences). In one embodiment, the CAR molecule comprises an EGFRvIII CAR, or an antigen binding domain according to Table 2 or SEQ ID NO:11 of WO 2014/130657, or a sequence substantially identical thereto (e.g., at least 85%, 90%, 95% or more identical thereto). The amino acid and nucleotide sequences encoding the EGFRvIII CAR molecules and antigen binding domains (e.g., including one, two, three VH CDRs; and one, two, three VL CDRs according to Kabat or Chothia), are specified in WO 2014/130657.

In other embodiments, the CAR molecule comprises an a GFR ALPHA-4 CAR, e.g., can include a CAR molecule, or an antigen binding domain according to Table 2 of WO2016/025880, or a sequence substantially identical to any of the aforesaid sequences (e.g., at least 85%, 90%, 95% or more identical to any of the aforesaid GFR ALPHA-4 sequences). The amino acid and nucleotide sequences encoding the GFR ALPHA-4 CAR molecules and antigen binding domains (e.g., including one, two, three VH CDRs; and one, two, three VL CDRs according to Kabat or Chothia), are specified in WO2016/025880.

### Inhibitory domains

In an embodiment, the vector comprises a nucleic acid sequence that encodes a CAR, e.g., a CAR described herein, and a nucleic acid sequence that encodes an inhibitory molecule comprising: an inhKIR cytoplasmic domain; a transmembrane domain, e.g., a KIR transmembrane domain; and an inhibitor cytoplasmic domain, e.g., an ITIM domain, e.g., an inhKIR ITIM domain. In an embodiment the inhibitory molecule is a naturally occurring inhKIR, or a sequence sharing at least 50, 60, 70, 80, 85, 90, 95, or 99% homology with, or that differs by no more than 1, 2, 3, 4, 5, 6, 7, 8, 9, 10, 15, or 20 residues from, a naturally occurring inhKIR.

In an embodiment, the nucleic acid sequence that encodes an inhibitory molecule comprises: a SLAM family cytoplasmic domain; a transmembrane domain, e.g., a SLAM family transmembrane domain; and an inhibitor cytoplasmic domain, e.g., a SLAM family domain, e.g., an SLAM family ITIM domain. In an embodiment the inhibitory molecule is a naturally occurring SLAM family member, or a sequence sharing at least 50, 60, 70, 80, 85, 90, 95, or 99% homology with, or that differs by no more than 1, 2, 3, 4, 5, 6, 7, 8, 9, 10, 15, or 20 residues from, a naturally occurring SLAM family member.

In one embodiment, the vector is an *in vitro* transcribed vector, e.g., a vector that transcribes RNA of a nucleic acid molecule described herein. In one embodiment, the nucleic acid sequence in the vector further comprises a poly(A) tail, e.g., a poly A tail. In one embodiment, the nucleic acid sequence in the vector further comprises a 3'UTR, e.g., a 3' UTR described herein, e.g., comprising at least one repeat of a 3'UTR derived from human beta-globulin. In one embodiment, the nucleic acid sequence in the vector further comprises promoter, e.g., a T2A promoter.

### Promoters

In one embodiment, the vector further comprises a promoter. In some embodiments, the promoter is chosen from an EF-1 promoter, a CMV IE gene promoter, an EF-1α promoter, an ubiquitin C promoter, or a phosphoglycerate kinase (PGK) promoter. In one embodiment, the promoter is an EF-1 promoter. In one embodiment, the EF-1 promoter comprises a sequence of SEQ ID NO: 1.

### Host cells

As noted above, in some aspects the disclosure pertains to a cell, e.g., an immune effector cell, (e.g., a population of cells, e.g., a population of immune effector cells) comprising a nucleic acid molecule, a chimeric polypeptide molecule, or a vector as described herein.

In certain aspects of the present disclosure, immune effector cells, e.g., T cells, can be obtained from a unit of blood collected from a subject using any number of techniques known to the skilled artisan, such as FicoII^{™} separation. In one preferred aspect, cells from the circulating blood of an individual are obtained by apheresis. The apheresis product typically contains lymphocytes, including T cells, monocytes, granulocytes, B cells, other nucleated white blood cells, red blood cells, and platelets. In one aspect, the cells collected by apheresis may be washed to remove the plasma fraction and, optionally, to place the cells in an appropriate buffer or media for subsequent processing steps. In one embodiment, the cells are washed with phosphate buffered saline (PBS). In an alternative embodiment, the wash solution lacks calcium and may lack magnesium or may lack many if not all divalent cations.

Initial activation steps in the absence of calcium can lead to magnified activation. As those of ordinary skill in the art would readily appreciate a washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

It is recognized that the methods of the application can utilize culture media conditions comprising 5% or less, for example 2%, human AB serum, and employ known culture media conditions and compositions, for example those described in Smith et al., "Ex vivo expansion of human T cells for adoptive immunotherapy using the novel Xeno-free CTS Immune Cell Serum Replacement" Clinical & Translational Immunology (2015) 4, e31; doi:10.1038/cti.2014.31. The culture media may additionally include one or more, e.g., one, LSD1 inhibitor(s) as described herein.

In one aspect, T cells are isolated from peripheral blood lymphocytes by lysing the red blood cells and depleting the monocytes, for example, by centrifugation through a PERCOLL^{™} gradient or by counterflow centrifugal elutriation. Once isolated, the cells may be contacted, e.g., ex vivo, with an LSD1 inhibitor as described herein.

The methods described herein can include, e.g., selection of a specific subpopulation of immune effector cells, e.g., T cells, that are a T regulatory cell-depleted population, CD25+ depleted cells, using, e.g., a negative selection technique, e.g., described herein. Preferably, the population of T regulatory depleted cells contains less than 30%, 25%, 20%, 15%, 10%, 5%, 4%, 3%, 2%, 1% of CD25+ cells. Either before or after selection, the cells may be contacted, e.g., ex vivo, with an LSD1 inhibitor as described herein.

In one embodiment, T regulatory cells, e.g., CD25+ T cells, are removed from the population using an anti-CD25 antibody, or fragment thereof, or a CD25-binding ligand, IL-2. In one embodiment, the anti-CD25 antibody, or fragment thereof, or CD25-binding ligand is conjugated to a substrate, e.g., a bead, or is otherwise coated on a substrate, e.g., a bead. In one embodiment, the anti-CD25 antibody, or fragment thereof, is conjugated to a substrate as described herein.

In one embodiment, the T regulatory cells, e.g., CD25+ T cells, are removed from the population using CD25 depletion reagent from Miltenyi^{™}. In one embodiment, the ratio of cells to CD25 depletion reagent is 1e7 cells to 20 uL, or 1e7 cells to15 uL, or 1e7 cells to 10 uL, or 1e7 cells to 5 uL, or 1e7 cells to 2.5 uL, or 1e7 cells to 1.25 uL. In one embodiment, e.g., for T regulatory cells, e.g., CD25+ depletion, greater than 500 million cells/ml is used. In a further aspect, a concentration of cells of 600, 700, 800, or 900 million cells/ml is used.

In one embodiment, the population of immune effector cells to be depleted includes about 6 × 10⁹ CD25+ T cells. In other aspects, the population of immune effector cells to be depleted include about 1 × 10⁹ to 1× 10¹⁰ CD25+ T cell, and any integer value in between. In one embodiment, the resulting population T regulatory depleted cells has 2 × 10⁹T regulatory cells, e.g., CD25+ cells, or less (e.g., 1 × 10⁹, 5 × 10⁸, 1 × 10⁸, 5 × 10⁷, 1 × 10⁷, or less CD25+ cells).

In one embodiment, the T regulatory cells, e.g., CD25+ cells, are removed from the population using the CliniMAC system with a depletion tubing set, such as, e.g., tubing 162-01. In one embodiment, the CliniMAC system is run on a depletion setting such as, e.g., DEPLETION2.1.

Without wishing to be bound by a particular theory, decreasing the level of negative regulators of immune cells (e.g., decreasing the number of unwanted immune cells, e.g., T_{REG} cells), in a subject prior to apheresis or during manufacturing of a CAR-expressing cell product can reduce the risk of subject relapse. For example, methods of depleting T_{REG} cells are known in the art. Methods of decreasing T_{REG} cells include, but are not limited to, cyclophosphamide, anti-GITR antibody (an anti-GITR antibody described herein), CD25-depletion, and combinations thereof. These methods may be combined with the methods of manufacture that include contacting the population of immune effector cells with an LSD1 inhibitor as described herein.

In some embodiments, the manufacturing methods comprise reducing the number of (e.g., depleting) T_{REG} cells prior to manufacturing of the CAR-expressing cell. For example, manufacturing methods comprise contacting the sample, e.g., the apheresis sample, with an anti-GITR antibody and/or an anti-CD25 antibody (or fragment thereof, or a CD25-binding ligand), e.g., to deplete T_{REG} cells prior to manufacturing of the CAR-expressing cell (e.g., T cell, NK cell) product. These methods may be combined with the methods of manufacture that include contacting the population of immune effector cells with an LSD1 inhibitor as described herein.

In an embodiment, a subject is pre-treated with one or more therapies that reduce T_{REG} cells prior to collection of cells for CAR-expressing cell product manufacturing, thereby reducing the risk of subject relapse to CAR-expressing cell treatment. In an embodiment, methods of decreasing T_{REG} cells include, but are not limited to, administration to the subject of one or more of cyclophosphamide, anti-GITR antibody, CD25-depletion, or a combination thereof. Administration of one or more of cyclophosphamide, anti-GITR antibody, CD25-depletion, or a combination thereof, can occur before, during or after an infusion of the CAR-expressing cell product. These methods may be combined with the methods of manufacture that include contacting the population of immune effector cells with an LSD1 inhibitor as described herein.

In an embodiment, a subject is pre-treated with cyclophosphamide prior to collection of cells for CAR-expressing cell product manufacturing, thereby reducing the risk of subject relapse to CAR-expressing cell treatment. In an embodiment, a subject is pre-treated with an anti-GITR antibody prior to collection of cells for CAR-expressing cell product manufacturing, thereby reducing the risk of subject relapse to CAR-expressing cell treatment. These methods may be combined with the methods of manufacture that include contacting the population of immune effector cells with an LSD1 inhibitor as described herein.

In one embodiment, the population of cells to be removed are neither the regulatory T cells or tumor cells, but cells that otherwise negatively affect the expansion and/or function of CART cells, e.g. cells expressing CD14, CD11b, CD33, CD15, or other markers expressed by potentially immune suppressive cells. In one embodiment, such cells are envisioned to be removed concurrently with regulatory T cells and/or tumor cells, or following said depletion, or in another order.

The methods described herein can include more than one selection step, e.g., more than one depletion step. Enrichment of a T cell population by negative selection can be accomplished, e.g., with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail can include antibodies to CD14, CD20, CD11b, CD16, HLA-DR, and CD8. These steps may be combined with the methods of manufacture that include contacting the population of immune effector cells with an LSD1 inhibitor as described herein.

The methods described herein can further include removing cells from the population which express a tumor antigen, e.g., a tumor antigen that does not comprise CD25, e.g., CD19, CD30, CD38, CD123, CD20, CD14 or CD11b, to thereby provide a population of T regulatory depleted, e.g., CD25+ depleted, and tumor antigen depleted cells that are suitable for expression of a CAR, e.g., a CAR described herein. In one embodiment, tumor antigen expressing cells are removed simultaneously with the T regulatory, e.g., CD25+ cells. For example, an anti-CD25 antibody, or fragment thereof, and an anti-tumor antigen antibody, or fragment thereof, can be attached to the same substrate, e.g., bead, which can be used to remove the cells or an anti-CD25 antibody, or fragment thereof, or the anti-tumor antigen antibody, or fragment thereof, can be attached to separate beads, a mixture of which can be used to remove the cells. In other embodiments, the removal of T regulatory cells, e.g., CD25+ cells, and the removal of the tumor antigen expressing cells is sequential, and can occur, e.g., in either order. These steps may be combined with the methods of manufacture that include contacting the population of immune effector cells with an LSD1 inhibitor as described herein.

Also provided are methods that include removing cells from the population which express a check point inhibitor, e.g., a check point inhibitor described herein, e.g., one or more of PD1+ cells, LAG3+ cells, and TIM3+ cells, to thereby provide a population of T regulatory depleted, e.g., CD25+ depleted cells, and check point inhibitor depleted cells, e.g., PD1+, LAG3+ and/or TIM3+ depleted cells. Exemplary check point inhibitors include B7-H1, B7-1, CD160, P1H, 2B4, PD1, TIM3, CEACAM (e.g., CEACAM-1, CEACAM-3 and/or CEACAM-5), LAG3, TIGIT, CTLA-4, BTLA and LAIR1. In one embodiment, check point inhibitor expressing cells are removed simultaneously with the T regulatory, e.g., CD25+ cells. For example, an anti-CD25 antibody, or fragment thereof, and an anti-check point inhibitor antibody, or fragment thereof, can be attached to the same bead which can be used to remove the cells, or an anti-CD25 antibody, or fragment thereof, and the anti-check point inhibitor antibody, or fragment there, can be attached to separate beads, a mixture of which can be used to remove the cells. In other embodiments, the removal of T regulatory cells, e.g., CD25+ cells, and the removal of the check point inhibitor expressing cells is sequential, and can occur, e.g., in either order. These steps may be combined with the methods of manufacture that include contacting the population of immune effector cells with an LSD1 inhibitor as described herein.

Methods described herein can include a positive selection step. For example, T cells can isolated by incubation with anti-CD3/anti-CD28 (e.g., 3x28)-conjugated beads, such as DYNABEADS^{®} M-450 CD3/CD28 T, for a time period sufficient for positive selection of the desired T cells. In one embodiment, the time period is about 30 minutes. In a further embodiment, the time period ranges from 30 minutes to 36 hours or longer and all integer values there between. In a further embodiment, the time period is at least 1, 2, 3, 4, 5, or 6 hours. In yet another embodiment, the time period is 10 to 24 hours, e.g., 24 hours. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points. These steps may be combined with the methods of manufacture that include contacting the population of immune effector cells with an LSD1 inhibitor as described herein.

In one embodiment, a T cell population can be selected that expresses one or more of IFN-^{γ}, TNFα, IL-17A, IL-2, IL-3, IL-4, GM-CSF, IL-10, IL-13, granzyme B, and perforin, or other appropriate molecules, e.g., other cytokines. Methods for screening for cell expression can be determined, e.g., by the methods described in PCT Publication No.: WO 2013/126712.

For isolation of a desired population of cells by positive or negative selection, the concentration of cells and surface (e.g., particles such as beads) can be varied. In certain aspects, it may be desirable to significantly decrease the volume in which beads and cells are mixed together (e.g., increase the concentration of cells), to ensure maximum contact of cells and beads. For example, in one aspect, a concentration of 10 billion cells/ml, 9 billion/ml, 8 billion/ml, 7 billion/ml, 6 billion/ml, or 5 billion/ml is used. In one aspect, a concentration of 1 billion cells/ml is used. In yet one aspect, a concentration of cells from 75, 80, 85, 90, 95, or 100 million cells/ml is used. In further aspects, concentrations of 125 or 150 million cells/ml can be used.

Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (e.g., leukemic blood, tumor tissue, etc.). Such populations of cells may have therapeutic value and would be desirable to obtain. For example, using high concentration of cells allows more efficient selection of CD8+ T cells that normally have weaker CD28 expression.

In a related aspect, it may be desirable to use lower concentrations of cells. By significantly diluting the mixture of T cells and surface (e.g., particles such as beads), interactions between the particles and cells are minimized. This selects for cells that express high amounts of desired antigens to be bound to the particles. For example, CD4+ T cells express higher levels of CD28 and are more efficiently captured than CD8+ T cells in dilute concentrations. In one aspect, the concentration of cells used is 5 × 10⁶/ml. In other aspects, the concentration used can be from about 1 × 10⁵/ml to 1 × 10⁶/ml, and any integer value in between.

In other aspects, the cells may be incubated on a rotator for varying lengths of time at varying speeds at either 2-10°C or at room temperature.

T cells for stimulation can also be frozen after a washing step. Wishing not to be bound by theory, the freeze and subsequent thaw step provide a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20° C or in liquid nitrogen.

In certain aspects, cryopreserved cells are thawed and washed as described herein and allowed to rest for one hour at room temperature prior to activation using the methods of the present invention.

Also contemplated in the context of the invention is the collection of blood samples or apheresis product from a subject at a time period prior to when the expanded cells as described herein might be needed. As such, the source of the cells to be expanded can be collected at any time point necessary, and desired cells, such as T cells, can be isolated and frozen for later use in immune effector cell therapy for any number of diseases or conditions that would benefit from immune effector cell therapy, such as those described herein. In one aspect a blood sample or an apheresis is taken from a generally healthy subject. In certain aspects, a blood sample or an apheresis is taken from a generally healthy subject who is at risk of developing a disease, but who has not yet developed a disease, and the cells of interest are isolated and frozen for later use. In certain aspects, the T cells may be expanded, frozen, and used at a later time. In certain aspects, samples are collected from a patient shortly after diagnosis of a particular disease as described herein but prior to any treatments. In a further aspect, the cells are isolated from a blood sample or an apheresis from a subject prior to any number of relevant treatment modalities, including but not limited to treatment with agents such as natalizumab, efalizumab, antiviral agents, chemotherapy, radiation, immunosuppressive agents, such as cyclosporin, azathioprine, methotrexate, mycophenolate, and FK506, antibodies, or other immunoablative agents such as CAMPATH, anti-CD3 antibodies, cytoxan, fludarabine, cyclosporin, FK506, rapamycin, mycophenolic acid, steroids, FR901228, and irradiation.

In a further aspect of the present invention, T cells are obtained from a patient directly following treatment that leaves the subject with functional T cells. In this regard, it has been observed that following certain cancer treatments, in particular treatments with drugs that damage the immune system, shortly after treatment during the period when patients would normally be recovering from the treatment, the quality of T cells obtained may be optimal or improved for their ability to expand ex vivo. Likewise, following ex vivo manipulation using the methods described herein, these cells may be in a preferred state for enhanced engraftment and in vivo expansion. Thus, it is contemplated within the context of the present invention to collect blood cells, including T cells, dendritic cells, or other cells of the hematopoietic lineage, during this recovery phase. Further, in certain aspects, mobilization (for example, mobilization with GM-CSF) and conditioning regimens can be used to create a condition in a subject wherein repopulation, recirculation, regeneration, and/or expansion of particular cell types is favored, especially during a defined window of time following therapy. Illustrative cell types include T cells, B cells, dendritic cells, and other cells of the immune system.

In one embodiment, the immune effector cells expressing a CAR molecule, e.g., a CAR molecule described herein, are obtained from a subject that has received an LSD1 inhibitor. In an embodiment, the population of immune effector cells, e.g., T cells, to be engineered to express a CAR, are harvested after a sufficient time, or after sufficient dosing of the LSD1 inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In other embodiments, population of immune effector cells, e.g., T cells, which have, or will be engineered to express a CAR, can be treated ex vivo by contact with an amount of an LSD1 inhibitor that increases the number of PD1 negative immune effector cells, e.g., T cells or increases the ratio of PD1 negative immune effector cells, e.g., T cells/ PD1 positive immune effector cells, e.g., T cells.

In one embodiment, a T cell population is diaglycerol kinase (DGK)-deficient. DGK-deficient cells include cells that do not express DGK RNA or protein, or have reduced or inhibited DGK activity. DGK-deficient cells can be generated by genetic approaches, e.g., administering RNA-interfering agents, e.g., siRNA, shRNA, miRNA, to reduce or prevent DGK expression. Alternatively, DGK-deficient cells can be generated by treatment with DGK inhibitors described herein.

In one embodiment, a T cell population is Ikaros-deficient. Ikaros-deficient cells include cells that do not express lkaros RNA or protein, or have reduced or inhibited Ikaros activity, Ikaros-deficient cells can be generated by genetic approaches, e.g., administering RNA-interfering agents, e.g., siRNA, shRNA, miRNA, to reduce or prevent Ikaros expression. Alternatively, Ikaros-deficient cells can be generated by treatment with Ikaros inhibitors, e.g., lenalidomide.

In embodiments, a T cell population is DGK-deficient and Ikaros-deficient, e.g., does not express DGK and Ikaros, or has reduced or inhibited DGK and Ikaros activity. Such DGK and Ikaros-deficient cells can be generated by any of the methods described herein.

In an embodiment, the NK cells are obtained from the subject. In another embodiment, the NK cells are an NK cell line, e.g., NK-92 cell line (Conkwest).

### Additional Expressed Agents

In another embodiment, a CAR-expressing immune effector cell described herein can further express another agent, e.g., an agent which enhances the activity of a CAR-expressing cell. For example, in one embodiment, the agent can be an agent which inhibits an inhibitory molecule. Examples of inhibitory molecules include PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGF beta, e.g., as described herein. In one embodiment, the agent that inhibits an inhibitory molecule comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 or TGF beta, or a fragment of any of these, and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD-1 or a fragment thereof, and a second polypeptide of an intracellular signaling domain described herein (e.g., a CD28, CD27, OX40 or 4-IBB signaling domain described herein and/or a CD3 zeta signaling domain described herein).

In one embodiment, the CAR-expressing immune effector cell described herein can further comprise a second CAR, e.g., a second CAR that includes a different antigen binding domain, e.g., to the same target (e.g., a target described above) or a different target. In one embodiment, the second CAR includes an antigen binding domain to a target expressed on the same cancer cell type as the target of the first CAR. In one embodiment, the CAR-expressing immune effector cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain.

While not wishing to be bound by theory, placement of a costimulatory signaling domain, e.g., 4-1BB, CD28, CD27 or OX-40, onto the first CAR, and the primary signaling domain, e.g., CD3 zeta, on the second CAR can limit the CAR activity to cells where both targets are expressed. In one embodiment, the CAR expressing immune effector cell comprises a first CAR that includes an antigen binding domain that targets, e.g., a target described above, a transmembrane domain and a costimulatory domain and a second CAR that targets an antigen other than antigen targeted by the first CAR (e.g., an antigen expressed on the same cancer cell type as the first target) and includes an antigen binding domain, a transmembrane domain and a primary signaling domain. In another embodiment, the CAR expressing immune effector cell comprises a first CAR that includes an antigen binding domain that targets, e.g., a target described above, a transmembrane domain and a primary signaling domain and a second CAR that targets an antigen other than antigen targeted by the first CAR (e.g., an antigen expressed on the same cancer cell type as the first target) and includes an antigen binding domain to the antigen, a transmembrane domain and a costimulatory signaling domain.

In one embodiment, the CAR-expressing immune effector cell comprises a CAR described herein, e.g., a CAR to a target described above, and an inhibitory CAR. In one embodiment, the inhibitory CAR comprises an antigen binding domain that binds an antigen found on normal cells but not cancer cells, e.g., normal cells that also express the target. In one embodiment, the inhibitory CAR comprises the antigen binding domain, a transmembrane domain and an intracellular domain of an inhibitory molecule. For example, the intracellular domain of the inhibitory CAR can be an intracellular domain of PD1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 or TGF beta.

In one embodiment, an immune effector cell (e.g., T cell, NK cell) comprises a first CAR comprising an antigen binding domain that binds to a tumor antigen as described herein, and a second CAR comprising a PD1 extracellular domain or a fragment thereof.

In one embodiment, the cell further comprises an inhibitory molecule as described above.

In one embodiment, the second CAR in the cell is an inhibitory CAR, wherein the inhibitory CAR comprises an antigen binding domain, a transmembrane domain, and an intracellular domain of an inhibitory molecule. The inhibitory molecule can be chosen from one or more of: PD1, PD-L1, CTLA-4, TIM-3, LAG-3, VISTA, BTLA, TIGIT, LAIR1, CD160, 2B4, TGF beta, CEACAM-1, CEACAM-3, and CEACAM-5. In one embodiment, the second CAR molecule comprises the extracellular domain of PD1 or a fragment thereof.

In embodiments, the second CAR molecule in the cell further comprises an intracellular signaling domain comprising a primary signaling domain and/or an intracellular signaling domain.

In other embodiments, the intracellular signaling domain in the cell comprises a primary signaling domain comprising the functional domain of CD3 zeta and a costimulatory signaling domain comprising the functional domain of 4-1BB.

In one embodiment, the second CAR molecule in the cell comprises the amino acid sequence of SEQ ID NO: 26.

In certain embodiments, the antigen binding domain of the first CAR molecule comprises a scFv and the antigen binding domain of the second CAR molecule does not comprise a scFv. For example, the antigen binding domain of the first CAR molecule comprises a scFv and the antigen binding domain of the second CAR molecule comprises a camelid VHH domain.

### Split CAR

In some embodiments, the CAR-expressing cell uses a split CAR. The split CAR approach is described in more detail in publications WO2014/055442 and WO2014/055657. Briefly, a split CAR system comprises a cell expressing a first CAR having a first antigen binding domain and a costimulatory domain (e.g., 41BB), and the cell also expresses a second CAR having a second antigen binding domain and an intracellular signaling domain (e.g., CD3 zeta). When the cell encounters the first antigen, the costimulatory domain is activated, and the cell proliferates. When the cell encounters the second antigen, the intracellular signaling domain is activated and cell-killing activity begins. Thus, the CAR-expressing cell is only fully activated in the presence of both antigens.

### Multiple CAR expression

In one aspect, the CAR-expressing cell described herein can further comprise a second CAR, e.g., a second CAR that includes a different antigen binding domain, e.g., to the same target or a different target (e.g., a target other than a cancer associated antigen described herein or a different cancer associated antigen described herein). In one embodiment, the second CAR includes an antigen binding domain to a target expressed the same cancer cell type as the cancer associated antigen. In one embodiment, the CAR-expressing cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain. While not wishing to be bound by theory, placement of a costimulatory signaling domain, e.g., 4-1BB, CD28, CD27 or OX-40, onto the first CAR, and the primary signaling domain, e.g.,CD3 zeta, on the second CAR can limit the CAR activity to cells where both targets are expressed. In one embodiment, the CAR expressing cell comprises a first cancer associated antigen CAR that includes an antigen binding domain that binds a target antigen described herein, a transmembrane domain and a costimulatory domain and a second CAR that targets a different target antigen (e.g., an antigen expressed on that same cancer cell type as the first target antigen) and includes an antigen binding domain, a transmembrane domain and a primary signaling domain. In another embodiment, the CAR expressing cell comprises a first CAR that includes an antigen binding domain that binds a target antigen described herein, a transmembrane domain and a primary signaling domain and a second CAR that targets an antigen other than the first target antigen (e.g., an antigen expressed on the same cancer cell type as the first target antigen) and includes an antigen binding domain to the antigen, a transmembrane domain and a costimulatory signaling domain.

In some embodiments, the disclosure comprises a first and second CAR, wherein the antigen binding domain of one of said first CAR said second CAR does not comprise a variable light domain and a variable heavy domain. In some embodiments, the antigen binding domain of one of said first CAR said second CAR is an scFv, and the other is not an scFv. In some embodiments, the antigen binding domain of one of said first CAR said second CAR comprises a single VH domain, e.g., a camelid, shark, or lamprey single VH domain, or a single VH domain derived from a human or mouse sequence. In some embodiments, the antigen binding domain of one of said first CAR said second CAR comprises a nanobody. In some embodiments, the antigen binding domain of one of said first CAR said second CAR comprises a camelid VHH domain.

### Allogeneic cells

In instances described herein, the immune effector cell can be an allogeneic immune effector cell, e.g., T cell or NK cell. For example, the cell can be an allogeneic T cell, e.g., an allogeneic T cell lacking expression of a functional T cell receptor (TCR) and/or human leukocyte antigen (HLA), e.g., HLA class I and/or HLA class II.

AT cell lacking a functional TCR can be, e.g., engineered such that it does not express any functional TCR on its surface, engineered such that it does not express one or more subunits that comprise a functional TCR or engineered such that it produces very little functional TCR on its surface. Alternatively, the T cell can express a substantially impaired TCR, e.g., by expression of mutated or truncated forms of one or more of the subunits of the TCR. The term "substantially impaired TCR" means that this TCR will not elicit an adverse immune reaction in a host.

AT cell described herein can be, e.g., engineered such that it does not express a functional HLA on its surface. For example, a T cell described herein, can be engineered such that cell surface expression HLA, e.g., HLA class 1 and/or HLA class II, is downregulated.

In some embodiments, the T cell can lack a functional TCR and a functional HLA, e.g., HLA class I and/or HLA class II.

Modified T cells that lack expression of a functional TCR and/or HLA can be obtained by any suitable means, including a knock out or knock down of one or more subunit of TCR or HLA. For example, the T cell can include a knock down of TCR and/or HLA using siRNA, shRNA, clustered regularly interspaced short palindromic repeats (CRISPR) transcription-activator like effector nuclease (TALEN), or zinc finger endonuclease (ZFN).

In some embodiments, the allogeneic cell can be a cell which does not express or expresses at low levels an inhibitory molecule, e.g. by any method described herein. For example, the cell can be a cell that does not express or expresses at low levels an inhibitory molecule, e.g., that can decrease the ability of a CAR-expressing cell to mount an immune effector response. Examples of inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGF beta. Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used.

### siRNA and shRNA to inhibit TCR or HLA

In some embodiments, TCR expression and/or HLA expression can be inhibited using siRNA or shRNA that targets a nucleic acid encoding a TCR and/or HLA in a T cell.

### CRISPR Systems to inhibit TCR or HLA

"CRISPR system to TCR and/or HLA" or "CRISPR to inhibit TCR and/or HLA" as used herein refers to a CRISPR system (e.g., a CRISPR/Cas9 system) comprising one or more guide RNA molecules comprising a targeting domain complementary to target sequence within a gene for a component of the TCR and/or HLA or beta-2 microglobulin (B2M), and a RNA-guided endonuclease (e.g., a Cas, e.g., a Cas9).

Artificial CRISPR/Cas systems can be generated which inhibit TCR and/or HLA, using technology known in the art, e.g., that are described in U.S. Publication No. 20140068797, and Cong (2013) Science 339: 819-823. Other artificial CRISPR/Cas systems that are known in the art may also be generated which inhibit TCR and/or HLA, e.g., that described in Tsai (2014) Nature Biotechnol., 32:6 569-576, U.S. Patent No.: 8,871,445; 8,865,406; 8,795,965; 8,771,945; and 8,697,359.

### TALEN to inhibit TCR and/or HLA

"TALEN genome editing system to HLA and/or TCR" or "TALEN to inhibit HLA and/or TCR" refers to a transcription activator-like effector nuclease, an artificial nuclease which can be used to edit the HLA and/or TCR gene.

TALENs are produced artificially by fusing a TAL effector DNA binding domain to a DNA cleavage domain. Transcription activator-like effects (TALEs) can be engineered to bind any desired DNA sequence, including a portion of the HLA or TCR gene. By combining an engineered TALE with a DNA cleavage domain, a restriction enzyme can be produced which is specific to any desired DNA sequence, including a HLA or TCR sequence. These can then be introduced into a cell, wherein they can be used for genome editing. Boch (2011) Nature Biotech. 29: 135-6; and Boch et al. (2009) Science 326: 1509-12; Moscou et al. (2009) Science 326: 3501.

TALENs specific to sequences in HLA or TCR can be constructed using any method known in the art, including various schemes using modular components. Zhang et al. (2011) Nature Biotech. 29: 149-53; Geibler et al. (2011) PLoS ONE 6: e19509.

### Zinc finger nuclease to inhibit HLA and/or TCR

"ZFN genome editing system to HLA and/or TCR" or "ZFN to inhibit HLA and/or TCR" refer to a zinc finger nuclease, an artificial nuclease which can be used to edit the HLA and/or TCR gene.

ZFNs specific to sequences in HLA AND/OR TCR can be constructed using any method known in the art. See, e.g., Provasi (2011) Nature Med. 18: 807-815; Torikai (2013) Blood 122: 1341-1349; Cathomen et al. (2008) Mol. Ther. 16: 1200-7; Guo et al. (2010) J. Mol. Biol. 400: 96; U.S. Patent Publication 2011/0158957; and U.S. Patent Publication 2012/0060230.

### Telomerase expression

While not wishing to be bound by any particular theory, in some embodiments, a therapeutic T cell has short term persistence in a patient, due to shortened telomeres in the T cell; accordingly, transfection with a telomerase gene can lengthen the telomeres of the T cell and improve persistence of the T cell in the patient. See Carl June, "Adoptive T cell therapy for cancer in the clinic", Journal of Clinical Investigation, 117:1466-1476 (2007). Thus, in an embodiment, an immune effector cell, e.g., a T cell, ectopically expresses a telomerase subunit, e.g., the catalytic subunit of telomerase, e.g., TERT, e.g., hTERT. In some aspects, this disclosure provides a method of producing a CAR-expressing cell, comprising contacting a cell with a nucleic acid encoding a telomerase subunit, e.g., the catalytic subunit of telomerase, e.g., TERT, e.g., hTERT. The cell may be contacted with the nucleic acid before, simultaneous with, or after being contacted with a construct encoding a CAR.

### Expansion and Activation

Immune effector cells such as T cells may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

Generally, a population of immune effector cells e.g., T cells, may be expanded by contact with a surface having attached thereto an agent that stimulates a CD3/TCR complex associated signal and a ligand that stimulates a costimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody can be used. Examples of an anti-CD28 antibody include 9.3, B-T3, XR-CD28 (Diaclone, Besançon, France) can be used as can other methods commonly known in the art (Berg et al., Transplant Proc. 30(8):3975-3977, 1998; Haanen et al., J. Exp. Med. 190(9):13191328, 1999; Garland et al., J. Immunol Meth. 227(1-2):53-63, 1999).

In certain aspects, the primary stimulatory signal and the costimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one aspect, the agent providing the costimulatory signal is bound to a cell surface and the agent providing the primary activation signal is in solution or coupled to a surface. In certain aspects, both agents can be in solution. In one aspect, the agents may be in soluble form, and then cross-linked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T cells in the present invention.

In one aspect, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the costimulatory signal is an anti-CD28 antibody or antigen-binding fragment thereof; and both agents are co-immobilized to the same bead in equivalent molecular amounts. In one aspect, a 1:1 ratio of each antibody bound to the beads for CD4+ T cell expansion and T cell growth is used. In certain aspects of the present invention, a ratio of anti CD3:CD28 antibodies bound to the beads is used such that an increase in T cell expansion is observed as compared to the expansion observed using a ratio of 1:1. In one particular aspect an increase of from about 1 to about 3 fold is observed as compared to the expansion observed using a ratio of 1:1. In one aspect, the ratio of CD3:CD28 antibody bound to the beads ranges from 100:1 to 1:100 and all integer values there between. In one aspect, more anti-CD28 antibody is bound to the particles than anti-CD3 antibody, i.e., the ratio of CD3:CD28 is less than one. In certain aspects, the ratio of anti CD28 antibody to anti CD3 antibody bound to the beads is greater than 2:1. In one particular aspect, a 1:100 CD3:CD28 ratio of antibody bound to beads is used. In one aspect, a 1:75 CD3:CD28 ratio of antibody bound to beads is used. In a further aspect, a 1:50 CD3:CD28 ratio of antibody bound to beads is used. In one aspect, a 1:30 CD3:CD28 ratio of antibody bound to beads is used. In one preferred aspect, a 1:10 CD3:CD28 ratio of antibody bound to beads is used. In one aspect, a 1:3 CD3:CD28 ratio of antibody bound to the beads is used. In yet one aspect, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain aspects the ratio of cells to particles ranges from 1:100 to 100:1 and any integer values in-between and in further aspects the ratio comprises 1:9 to 9:1 and any integer values in between, can also be used to stimulate T cells. The ratio of anti-CD3- and anti-CD28-coupled particles to T cells that result in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In one aspect, a ratio of particles to cells of 1:1 or less is used. In one particular aspect, a preferred particle: cell ratio is 1:5. In further aspects, the ratio of particles to cells can be varied depending on the day of stimulation. For example, in one aspect, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular aspect, the ratio of particles to cells is 1:1 on the first day of stimulation and adjusted to 1:5 on the third and fifth days of stimulation. In one aspect, particles are added on a daily or every other day basis to a final ratio of 1:1 on the first day, and 1:5 on the third and fifth days of stimulation. In one aspect, the ratio of particles to cells is 2:1 on the first day of stimulation and adjusted to 1:10 on the third and fifth days of stimulation. In one aspect, particles are added on a daily or every other day basis to a final ratio of 1:1 on the first day, and 1:10 on the third and fifth days of stimulation. One of skill in the art will appreciate that a variety of other ratios may be suitable for use in the present invention. In particular, ratios will vary depending on particle size and on cell size and type. In one aspect, the most typical ratios for use are in the neighborhood of 1:1, 2:1 and 3:1 on the first day.

In further aspects, the cells, such as T cells, are combined with agent-coated beads, the beads and the cells are subsequently separated, and then the cells are cultured. In an alternative aspect, prior to culture, the agent-coated beads and cells are not separated but are cultured together. In a further aspect, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the T cells. In one aspect the cells (for example, 10⁴ to 10⁹ T cells) and beads (for example, DYNABEADS^{®} M-450 CD3/CD28 T paramagnetic beads at a ratio of 1:1) are combined in a buffer, for example PBS (without divalent cations such as, calcium and magnesium). Again, those of ordinary skill in the art can readily appreciate any cell concentration may be used. For example, the target cell may be very rare in the sample and comprise only 0.01% of the sample or the entire sample (i.e., 100%) may comprise the target cell of interest. Accordingly, any cell number is within the context of the present invention. In certain aspects, it may be desirable to significantly decrease the volume in which particles and cells are mixed together (i.e., increase the concentration of cells), to ensure maximum contact of cells and particles. For example, in one aspect, a concentration of about 10 billion cells/ml, 9 billion/ml, 8 billion/ml, 7 billion/ml, 6 billion/ml, 5 billion/ml, or 2 billion cells/ml is used. In one aspect, greater than 100 million cells/ml is used. In a further aspect, a concentration of cells of 10, 15, 20, 25, 30, 35, 40, 45, or 50 million cells/ml is used. In yet one aspect, a concentration of cells from 75, 80, 85, 90, 95, or 100 million cells/ml is used. In further aspects, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain aspects. For example, using high concentration of cells allows more efficient selection of CD8+ T cells that normally have weaker CD28 expression.

In one embodiment, cells transduced with a nucleic acid encoding a CAR, e.g., a CAR described herein, are expanded, e.g., by a method described herein. In one embodiment, the cells are expanded in culture for a period of several hours (e.g., about 2, 3, 4, 5, 6, 7, 8, 9, 10, 15, 18, 21 hours) to about 14 days (e.g., 1, 2, 3, 4, 5, 6, 7, 8, 9, 10, 11, 12, 13 or 14 days). In one embodiment, the cells are expanded for a period of 4 to 9 days. In one embodiment, the cells are expanded for a period of 8 days or less, e.g., 7, 6 or 5 days. In one embodiment, the cells are expanded in culture for 5 days, and the resulting cells are more potent than the same cells expanded in culture for 9 days under the same culture conditions. Potency can be defined, e.g., by various T cell functions, e.g. proliferation, target cell killing, cytokine production, activation, migration, or combinations thereof. In one embodiment, the cells are expanded for 5 days show at least a one, two, three or four fold increase in cells doublings upon antigen stimulation as compared to the same cells expanded in culture for 9 days under the same culture conditions. In one embodiment, the cells are expanded in culture for 5 days, and the resulting cells exhibit higher proinflammatory cytokine production, e.g., IFN-y and/or GM-CSF levels, as compared to the same cells expanded in culture for 9 days under the same culture conditions. In one embodiment, the cells expanded for 5 days show at least a one, two, three, four, five, ten fold or more increase in pg/ml of proinflammatory cytokine production, e.g., IFN-y and/or GM-CSF levels, as compared to the same cells expanded in culture for 9 days under the same culture conditions.

Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-y, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% CO₂).

In one embodiment, the cells are expanded in an appropriate media (e.g., media described herein) that includes one or more interleukin that result in at least a 200-fold (e.g., 200-fold, 250-fold, 300-fold, 350-fold) increase in cells over a 14 day expansion period, e.g., as measured by a method described herein such as flow cytometry. In one embodiment, the cells are expanded in the presence of IL-15 and/or IL-7 (e.g., IL-15 and IL-7).

In embodiments, methods described herein, e.g., CAR-expressing cell manufacturing methods, comprise removing T regulatory cells, e.g., CD25+ T cells, from a cell population, e.g., using an anti-CD25 antibody, or fragment thereof, or a CD25-binding ligand, IL-2. Methods of removing T regulatory cells, e.g., CD25+ T cells, from a cell population are described herein. In embodiments, the methods, e.g., manufacturing methods, further comprise contacting a cell population (e.g., a cell population in which T regulatory cells, such as CD25+ T cells, have been depleted; or a cell population that has previously contacted an anti-CD25 antibody, fragment thereof, or CD25-binding ligand) with IL-15 and/or IL-7. For example, the cell population (e.g., that has previously contacted an anti-CD25 antibody, fragment thereof, or CD25-binding ligand) is expanded in the presence of IL-15 and/or IL-7.

In some embodiments a CAR-expressing cell described herein is contacted with a composition comprising a interleukin-15 (IL-15) polypeptide, a interleukin-15 receptor alpha (IL-15Ra) polypeptide, or a combination of both a IL-15 polypeptide and a IL-15Ra polypeptide e.g., hetlL-15, during the manufacturing of the CAR-expressing cell, e.g., ex vivo. In embodiments, a CAR-expressing cell described herein is contacted with a composition comprising a IL-15 polypeptide during the manufacturing of the CAR-expressing cell, e.g., ex vivo. In embodiments, a CAR-expressing cell described herein is contacted with a composition comprising a combination of both a IL-15 polypeptide and a IL-15 Ra polypeptide during the manufacturing of the CAR-expressing cell, e.g., ex vivo. In embodiments, a CAR-expressing cell described herein is contacted with a composition comprising hetlL-15 during the manufacturing of the CAR-expressing cell, e.g., ex vivo.

In one embodiment the CAR-expressing cell described herein is contacted with a composition comprising hetlL-15 during ex vivo expansion. In an embodiment, the CAR-expressing cell described herein is contacted with a composition comprising an IL-15 polypeptide during ex vivo expansion. In an embodiment, the CAR-expressing cell described herein is contacted with a composition comprising both an IL-15 polypeptide and an IL-15Ra polypeptide during ex vivo expansion. In one embodiment the contacting results in the survival and proliferation of a lymphocyte subpopulation, e.g., CD8+ T cells.

T cells that have been exposed to varied stimulation times may exhibit different characteristics. For example, typical blood or apheresed peripheral blood mononuclear cell products have a helper T cell population (TH, CD4+) that is greater than the cytotoxic or suppressor T cell population (TC, CD8+). Ex vivo expansion of T cells by stimulating CD3 and CD28 receptors produces a population of T cells that prior to about days 8-9 consists predominately of TH cells, while after about days 8-9, the population of T cells comprises an increasingly greater population of TC cells. Accordingly, depending on the purpose of treatment, infusing a subject with a T cell population comprising predominately of TH cells may be advantageous. Similarly, if an antigen-specific subset of TC cells has been isolated it may be beneficial to expand this subset to a greater degree.

Further, in addition to CD4 and CD8 markers, other phenotypic markers vary significantly, but in large part, reproducibly during the course of the cell expansion process. Thus, such reproducibility enables the ability to tailor an activated T cell product for specific purposes.

Once a CAR described herein is constructed, various assays can be used to evaluate the activity of the molecule, such as but not limited to, the ability to expand T cells following antigen stimulation, sustain T cell expansion in the absence of re-stimulation, and anti-cancer activities in appropriate in vitro and animal models. Assays to evaluate the effects of a CAR of the present invention are described in further detail below.

Western blot analysis of CAR expression in primary T cells can be used to detect the presence of monomers and dimers. See, *e.g.,* Milone et al., Molecular Therapy 17(8): 1453-1464 (2009). Very briefly, T cells (1:1 mixture of CD4⁺ and CD8⁺ T cells) expressing the CARs are expanded *in vitro* for more than 10 days followed by lysis and SDS-PAGE under reducing conditions. CARs containing the full length TCR-ζ cytoplasmic domain and the endogenous TCR-ζ chain are detected by western blotting using an antibody to the TCR-ζ chain. The same T cell subsets are used for SDS-PAGE analysis under non-reducing conditions to permit evaluation of covalent dimer formation.

*In vitro* expansion of CAR⁺ T cells following antigen stimulation can be measured by flow cytometry. For example, a mixture of CD4⁺ and CD8⁺ T cells are stimulated with αCD3/αCD28 aAPCs followed by transduction with lentiviral vectors expressing GFP under the control of the promoters to be analyzed. Exemplary promoters include the CMV IE gene, EF-1α, ubiquitin C, or phosphoglycerokinase (PGK) promoters. GFP fluorescence is evaluated on day 6 of culture in the CD4⁺ and/or CD8⁺ T cell subsets by flow cytometry. See, *e.g.,* Milone et al., Molecular Therapy 17(8): 1453-1464 (2009). Alternatively, a mixture of CD4⁺ and CD8⁺ T cells are stimulated with αCD3/αCD28 coated magnetic beads on day 0, and transduced with CAR on day 1 using a bicistronic lentiviral vector expressing CAR along with eGFP using a 2A ribosomal skipping sequence. Cultures are re-stimulated with either a cancer associated antigen as described herein⁺ K562 cells (K562 expressing a cancer associated antigen as described herein), wild-type K562 cells (K562 wild type) or K562 cells expressing hCD32 and 4-1BBL in the presence of antiCD3 and anti-CD28 antibody (K562-BBL-3/28) following washing. Exogenous IL-2 is added to the cultures every other day at 100 IU/ml. GFP⁺ T cells are enumerated by flow cytometry using bead-based counting. See, *e.g.,* Milone et al., Molecular Therapy 17(8): 1453-1464 (2009).

Sustained CAR⁺ T cell expansion in the absence of re-stimulation can also be measured. See, *e.g.,* Milone et al., Molecular Therapy 17(8): 1453-1464 (2009). Briefly, mean T cell volume (fl) is measured on day 8 of culture using a Coulter Multisizer III particle counter, a Nexcelom Cellometer Vision or Millipore Scepter, following stimulation with αCD3/αCD28 coated magnetic beads on day 0, and transduction with the indicated CAR on day 1.

Animal models can also be used to measure a CART activity. For example, xenograft model using human a cancer associated antigen described herein-specific CAR⁺ T cells to treat a primary human pre-B ALL in immunodeficient mice can be used. See, *e.g.,* Milone et al., Molecular Therapy 17(8): 1453-1464 (2009). Very briefly, after establishment of ALL, mice are randomized as to treatment groups. Different numbers of a cancer associated antigen -specific CARengineered T cells are coinjected at a 1:1 ratio into NOD-SCID-γ^{-/-} mice bearing B-ALL. The number of copies of a cancer associated antigen - specific CAR vector in spleen DNA from mice is evaluated at various times following T cell injection. Animals are assessed for leukemia at weekly intervals. Peripheral blood a cancer associate antigen as described herein⁺ B-ALL blast cell counts are measured in mice that are injected with a cancer associated antigen described herein-ζ CAR⁺ T cells or mock-transduced T cells. Survival curves for the groups are compared using the log-rank test. In addition, absolute peripheral blood CD4⁺ and CD8⁺ T cell counts 4 weeks following T cell injection in NOD-SCID-γ^{-/-} mice can also be analyzed. Mice are injected with leukemic cells and 3 weeks later are injected with T cells engineered to express CAR by a bicistronic lentiviral vector that encodes the CAR linked to eGFP. T cells are normalized to 45-50% input GFP⁺ T cells by mixing with mock-transduced cells prior to injection, and confirmed by flow cytometry. Animals are assessed for leukemia at 1-week intervals. Survival curves for the CAR⁺ T cell groups are compared using the log-rank test.

Dose dependent CAR treatment response can be evaluated. See, *e.g.,* Milone et al., Molecular Therapy 17(8): 1453-1464 (2009). For example, peripheral blood is obtained 35-70 days after establishing leukemia in mice injected on day 21 with CAR T cells, an equivalent number of mock-transduced T cells, or no T cells. Mice from each group are randomly bled for determination of peripheral blood a cancer associate antigen as described herein⁺ ALL blast counts and then killed on days 35 and 49. The remaining animals are evaluated on days 57 and 70.

Assessment of cell proliferation and cytokine production has been previously described, *e.g.*, at Milone et al., Molecular Therapy 17(8): 1453-1464 (2009). Briefly, assessment of CAR-mediated proliferation is performed in microtiter plates by mixing washed T cells with K562 cells expressing a cancer associated antigen described herein (K19) or CD32 and CD137 (KT32-BBL) for a final T-cell:K562 ratio of 2:1. K562 cells are irradiated with gamma-radiation prior to use. Anti-CD3 (clone OKT3) and anti-CD28 (clone 9.3) monoclonal antibodies are added to cultures with KT32-BBL cells to serve as a positive control for stimulating T-cell proliferation since these signals support long-term CD8⁺ T cell expansion *ex vivo.* T cells are enumerated in cultures using CountBright^{™} fluorescent beads (Invitrogen, Carlsbad, CA) and flow cytometry as described by the manufacturer. CAR⁺ T cells are identified by GFP expression using T cells that are engineered with eGFP-2A linked CAR-expressing lentiviral vectors. For CAR+ T cells not expressing GFP, the CAR+ T cells are detected with biotinylated recombinant a cancer associate antigen as described herein protein and a secondary avidin-PE conjugate. CD4+ and CD8⁺ expression on T cells are also simultaneously detected with specific monoclonal antibodies (BD Biosciences). Cytokine measurements are performed on supernatants collected 24 hours following re-stimulation using the human TH1/TH2 cytokine cytometric bead array kit (BD Biosciences, San Diego, CA) according the manufacturer's instructions. Fluorescence is assessed using a FACScalibur flow cytometer, and data is analyzed according to the manufacturer's instructions.

Cytotoxicity can be assessed by a standard 51Cr-release assay. See, *e.g.,* Milone et al., Molecular Therapy 17(8): 1453-1464 (2009). Briefly, target cells (K562 lines and primary pro-B-ALL cells) are loaded with 51Cr (as NaCrO4, New England Nuclear, Boston, MA) at 37°C for 2 hours with frequent agitation, washed twice in complete RPMI and plated into microtiter plates. Effector T cells are mixed with target cells in the wells in complete RPMI at varying ratios of effector cell:target cell (E:T). Additional wells containing media only (spontaneous release, SR) or a 1% solution of triton-X 100 detergent (total release, TR) are also prepared. After 4 hours of incubation at 37°C, supernatant from each well is harvested. Released 51Cr is then measured using a gamma particle counter (Packard Instrument Co., Waltham, MA). Each condition is performed in at least triplicate, and the percentage of lysis is calculated using the formula: % Lysis = (ER- SR) / (TR - SR), where ER represents the average 51Cr released for each experimental condition.

Imaging technologies can be used to evaluate specific trafficking and proliferation of CARs in tumor-bearing animal models. Such assays have been described, for example, in Barrett et al., Human Gene Therapy 22:1575-1586 (2011). Briefly, NOD/SCID/γc^{-/-} (NSG) mice are injected IV with Nalm-6 cells followed 7 days later with T cells 4 hour after electroporation with the CAR constructs. The T cells are stably transfected with a lentiviral construct to express firefly luciferase, and mice are imaged for bioluminescence. Alternatively, therapeutic efficacy and specificity of a single injection of CAR⁺ T cells in Nalm-6 xenograft model can be measured as the following: NSG mice are injected with Nalm-6 transduced to stably express firefly luciferase, followed by a single tail-vein injection of T cells engineered to express a CAR of the present disclosure (e.g., transduced with nucleic acid encoding a CAR of the present disclosure) 7 days later. Animals are imaged at various time points post injection. For example, photon-density heat maps of firefly luciferasepositive leukemia in representative mice at day 5 (2 days before treatment) and day 8 (24 hr post CAR⁺ PBLs) can be generated.

Other assays, including those described in the Example section herein as well as those that are known in the art can also be used to evaluate the CARs described herein.

### Methods of treatment/Combination therapies

In another aspect, the present disclosure provides a method comprising administering the LSD1 inhibitors of the invention in combination with a population of immune effector cells, e.g., engineered to express a CAR molecule, e.g., as described herein, as a therapy. For example, the invention provides a composition comprising a population of immune effector cells engineered to express a CAR for use, in combination with an LSD1 inhibitor, in a method of treating a disease associated with the expression of a tumor antigen in a subject, said method comprising administering to said subject an LSD1 inhibitor and a population of immune effector cells engineered to express a CAR, wherein the population of immune effector cells comprises, e.g., consists of, T cells, wherein the disease associated with expression of a tumor antigen is chosen from a proliferative disease, a precancerous condition, and a cancer.

Typically, such administration will be in the form of cells (e.g., autologous or allogeneic host cells) expressing a CAR and separate administration of the LSD1 inhibitor. Alternatively, the LSD1 inhibitor may be administered in the same composition as the cell engineered to express a CAR molecule. Alternatively, the cell engineered to express a CAR molecule may also be engineered to express an LSD1 inhibitor. In one embodiment, the subject has a disorder described herein, e.g., the subject has cancer, e.g., the subject has a cancer which expresses a target antigen described herein. In one embodiment, the subject is a human.

Methods described herein that comprise administering an LSD1 inhibitor and a CAR-expressing cell described herein may be used in combination with other known agents and therapies.

Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

A CAR-expressing cell described herein, an LSD1 inhibitor, and the at least one additional therapeutic agent can be administered simultaneously, in the same or in separate compositions, or sequentially. The three agents can be administered in any order. For example, in sequential administration, the LSD1 inhibitor and the CAR-expressing cell described herein can be administered first, and the additional agent can be administered second, or the order of administration can be reversed.

The CAR therapy and/or other therapeutic agents, procedures or modalities can be administered during periods of active disorder, or during a period of remission or less active disease. The CAR therapy can be administered before another treatment, concurrently with the treatment, posttreatment, or during remission of the disorder.

In another aspect, the invention pertains to treating a subject having a disease associated with expression of a cancer associated antigen as described herein comprising administering to the subject an effective amount of a cell comprising a CAR molecule, e.g., a CAR molecule described herein.

In one aspect, the invention provides for treating a disease associated with expression of a cancer associated antigen as described herein. The method comprises the administration of an LSD1 inhibitor and administrartion of a cell, e.g., a T cell, e.g., that expresses, or can express a CAR.

In one aspect, the present invention provides for treating cancer by providing to the subject in need thereof an LSD1 inhibitor and immune effector cells (e.g., T cells, NK cells) that are engineered to express an XCAR, wherein X represents a tumor marker (or cancer associated antigen; as used herein, the terms XCAR and CARX are used interchangeably, e.g., a CAR19 or CARCD19 is the same as a CD19 CAR) as described herein, and wherein said cancer cells express said X tumor marker (or cancer associated antigen).

In one aspect, the present invention provides for treating cancer by providing to the subject in need thereof an LSD1 inhibitor and immune effector cells (e.g., T cells, NK cells) that are engineered to express a CD19CAR, wherein the cancer cells express CD19. In one embodiment, the cancer to be treated is ALL (acute lymphoblastic leukemia), CLL (chronic lymphocytic leukemia), DLBCL (diffuse large B-cell lymphoma), MCL (Mantle cell lymphoma, or MM (multiple myeloma).

In one aspect, the present invention provides for treating cancer by providing to the subject in need thereof an LSD1 inhibitor and immune effector cells (e.g., T cells, NK cells) that are engineered to express an EGFRvIIICAR, wherein the cancer cells express EGFRvIII. In one embodiment, the cancer to be treated is glioblastoma.

In one aspect, the present invention provides for treating cancer by providing to the subject in need thereof an LSD1 inhibitor and immune effector cells (e.g., T cells, NK cells) that are engineered to express a mesothelin CAR, wherein the cancer cells express mesothelin. In one embodiment, the cancer to be treated is mesothelioma, pancreatic cancer, or ovarian cancer.

In one aspect, the present invention provides for treating cancer by providing to the subject in need thereof an LSD1 inhibitor and immune effector cells (e.g., T cells, NK cells) that are engineered to express a CD123CAR, wherein the cancer cells express CD123. In one embodiment, the cancer to be treated is AML.

In one aspect, the present invention provides for treating cancer by providing to the subject in need thereof an LSD1 inhibitor and immune effector cells (e.g., T cells, NK cells) that are engineered to express a CD22CAR, wherein the cancer cells express CD22. In one embodiment, the cancer to be treated is B cell malignancies.

In one aspect, the present invention provides for treating cancer by providing to the subject in need thereof an LSD1 inhibitor and immune effector cells (e.g., T cells, NK cells) that are engineered to express a CS-1CAR, wherein the cancer cells express CS-1. In one embodiment, the cancer to be treated is multiple myeloma.

In one aspect, the present invention provides for treating cancer by providing to the subject in need thereof an LSD1 inhibitor and immune effector cells (e.g., T cells, NK cells) that are engineered to express a CLL-1CAR, wherein the cancer cells express CLL-1. In one embodiment, the cancer to be treated is AML.

In one aspect, the present invention provides for treating cancer by providing to the subject in need thereof an LSD1 inhibitor and immune effector cells (e.g., T cells, NK cells) that are engineered to express a CD33CAR, wherein the cancer cells express CD33. In one embodiment, the cancer to be treated is AML.

In one aspect, the present invention provides for treating cancer by providing to the subject in need thereof an LSD1 inhibitor and immune effector cells (e.g., T cells, NK cells) that are engineered to express a GD2CAR, wherein the cancer cells express GD2. In one embodiment, the cancer to be treated is neuroblastoma.

In one aspect, the present invention provides for treating cancer by providing to the subject in need thereof an LSD1 inhibitor and immune effector cells (e.g., T cells, NK cells) that are engineered to express a BCMACAR, wherein the cancer cells express BCMA. In one embodiment, the cancer to be treated is multiple myeloma.

In one aspect, the present invention provides for treating cancer by providing to the subject in need thereof an LSD1 inhibitor and immune effector cells (e.g., T cells, NK cells) that are engineered to express a TnCAR, wherein the cancer cells express Tn antigen. In one embodiment, the cancer to be treated is ovarian cancer.

In one aspect, the present invention provides for treating cancer by providing to the subject in need thereof an LSD1 inhibitor and immune effector cells (e.g., T cells, NK cells) that are engineered to express a PSMACAR, wherein the cancer cells express PSMA. In one embodiment, the cancer to be treated is prostate cancer.

In one aspect, the present invention provides for treating cancer by providing to the subject in need thereof an LSD1 inhibitor and immune effector cells (e.g., T cells, NK cells) that are engineered to express a ROR1CAR, wherein the cancer cells express ROR1. In one embodiment, the cancer to be treated is B cell malignancies.

In one aspect, the present invention provides for treating cancer by providing to the subject in need thereof an LSD1 inhibitor and immune effector cells (e.g., T cells, NK cells) that are engineered to express a FLT3 CAR, wherein the cancer cells express FLT3. In one embodiment, the cancer to be treated is AML.

In one aspect, the present invention provides for treating cancer by providing to the subject in need thereo an LSD1 inhibitor and f immune effector cells (e.g., T cells, NK cells) that are engineered to express a TAG72CAR, wherein the cancer cells express TAG72. In one embodiment, the cancer to be treated is gastrointestinal cancer.

In one aspect, the present invention provides for treating cancer by providing to the subject in need thereof an LSD1 inhibitor and immune effector cells (e.g., T cells, NK cells) that are engineered to express a CD38CAR, wherein the cancer cells express CD38. In one embodiment, the cancer to be treated is multiple myeloma.

In one aspect, the present invention provides for treating cancer by providing to the subject in need thereof an LSD1 inhibitor and immune effector cells (e.g., T cells, NK cells) that are engineered to express a CD44v6CAR, wherein the cancer cells express CD44v6. In one embodiment, the cancer to be treated is cervical cancer, AML, or MM.

In one aspect, the present invention provides for treating cancer by providing to the subject in need thereof an LSD1 inhibitor and immune effector cells (e.g., T cells, NK cells) that are engineered to express a CEACAR, wherein the cancer cells express CEA. In one embodiment, the cancer to be treated is pastrointestinal cancer, or pancreatic cancer.

In one aspect, the present invention provides for treating cancer by providing to the subject in need thereof an LSD1 inhibitor and immune effector cells (e.g., T cells, NK cells) that are engineered to express a EPCAMCAR, wherein the cancer cells express EPCAM. In one embodiment, the cancer to be treated is gastrointestinal cancer.

In one aspect, the present invention provides for treating cancer by providing to the subject in need thereof an LSD1 inhibitor and immune effector cells (e.g., T cells, NK cells) that are engineered to express a B7H3CAR, wherein the cancer cells express B7H3.

In one aspect, the present invention provides for treating cancer by providing to the subject in need thereof an LSD1 inhibitor and immune effector cells (e.g., T cells, NK cells) that are engineered to express a KITCAR, wherein the cancer cells express KIT. In one embodiment, the cancer to be treated is gastrointestinal cancer.

In one aspect, the present invention provides for treating cancer by providing to the subject in need thereof an LSD1 inhibitor and immune effector cells (e.g., T cells, NK cells) that are engineered to express a IL-13Ra2CAR, wherein the cancer cells express IL-13Ra2. In one embodiment, the cancer to be treated is glioblastoma.

In one aspect, the present invention provides for treating cancer by providing to the subject in need thereof an LSD1 inhibitor and immune effector cells (e.g., T cells, NK cells) that are engineered to express a CD30CAR, wherein the cancer cells express CD30. In one embodiment, the cancer to be treated is lymphomas, or leukemias.

In one aspect, the present invention provides for treating cancer by providing to the subject in need thereof an LSD1 inhibitor and immune effector cells (e.g., T cells, NK cells) that are engineered to express a GD3CAR, wherein the cancer cells express GD3. In one embodiment, the cancer to be treated is melanoma.

In one aspect, the present invention provides for treating cancer by providing to the subject in need thereof an LSD1 inhibitor and immune effector cells (e.g., T cells, NK cells) that are engineered to express a CD171CAR, wherein the cancer cells express CD171. In one embodiment, the cancer to be treated is neuroblastoma, ovarian cancer, melanoma, breast cancer, pancreatic cancer, colon cancers, or NSCLC (non-small cell lung cancer).

In one aspect, the present invention provides for treating cancer by providing to the subject in need thereof an LSD1 inhibitor and immune effector cells (e.g., T cells, NK cells) that are engineered to express a IL-11RaCAR, wherein the cancer cells express IL-11Ra. In one embodiment, the cancer to be treated is osteosarcoma.

In one aspect, the present invention provides for treating cancer by providing to the subject in need thereof an LSD1 inhibitor and immune effector cells (e.g., T cells, NK cells) that are engineered to express a PSCACAR, wherein the cancer cells express PSCA. In one embodiment, the cancer to be treated is prostate cancer.

In one aspect, the present invention provides for treating cancer by providing to the subject in need thereof an LSD1 inhibitor and immune effector cells (e.g., T cells, NK cells) that are engineered to express a VEGFR2CAR, wherein the cancer cells express VEGFR2. In one embodiment, the cancer to be treated is a solid tumor.

In one aspect, the present invention provides for treating cancer by providing to the subject in need thereof an LSD1 inhibitor and immune effector cells (e.g., T cells, NK cells) that are engineered to express a LewisYCAR, wherein the cancer cells express LewisY. In one embodiment, the cancer to be treated is ovarian cancer, or AML.

In one aspect, the present invention provides for treating cancer by providing to the subject in need thereof an LSD1 inhibitor and immune effector cells (e.g., T cells, NK cells) that are engineered to express a CD24CAR, wherein the cancer cells express CD24. In one embodiment, the cancer to be treated is pancreatic cancer.

In one aspect, the present invention provides for treating cancer by providing to the subject in need thereof an LSD1 inhibitor and immune effector cells (e.g., T cells, NK cells) that are engineered to express a PDGFR-betaCAR, wherein the cancer cells express PDGFR-beta. In one embodiment, the cancer to be treated is breast cancer, prostate cancer, GIST (gastrointestinal stromal tumor), CML, DFSP (dermatofibrosarcoma protuberans), or glioma.

In one aspect, the present invention provides for treating cancer by providing to the subject in need thereof an LSD1 inhibitor and immune effector cells (e.g., T cells, NK cells) that are engineered to express a SSEA-4CAR, wherein the cancer cells express SSEA-4. In one embodiment, the cancer to be treated is glioblastoma, breast cancer, lung cancer, or stem cell cancer.

In one aspect, the present invention provides for treating cancer by providing to the subject in need thereof an LSD1 inhibitor and immune effector cells (e.g., T cells, NK cells) that are engineered to express a CD20CAR, wherein the cancer cells express CD20. In one embodiment, the cancer to be treated is B cell malignancies.

In one aspect, the present invention provides for treating cancer by providing to the subject in need thereof an LSD1 inhibitor and immune effector cells (e.g., T cells, NK cells) that are engineered to express a Folate receptor alphaCAR, wherein the cancer cells express folate receptor alpha. In one embodiment, the cancer to be treated is ovarian cancer, NSCLC, endometrial cancer, renal cancer, or other solid tumors.

In one aspect, the present invention provides for treating cancer by providing to the subject in need thereof an LSD1 inhibitor and immune effector cells (e.g., T cells, NK cells) that are engineered to express an ERBB2CAR, wherein the cancer cells express ERBB2 (Her2/neu). In one embodiment, the cancer to be treated is breast cancer, gastric cancer, colorectal cancer, lung cancer, or other solid tumors.

In one aspect, the present invention provides for treating cancer by providing to the subject in need thereof an LSD1 inhibitor and immune effector cells (e.g., T cells, NK cells) that are engineered to express a MUC1CAR, wherein the cancer cells express MUC1. In one embodiment, the cancer to be treated is breast cancer, lung cancer, or other solid tumors.

In one aspect, the present invention provides for treating cancer by providing to the subject in need thereof an LSD1 inhibitor and immune effector cells (e.g., T cells, NK cells) that are engineered to express a EGFRCAR, wherein the cancer cells express EGFR. In one embodiment, the cancer to be treated is glioblastoma, SCLC (small cell lung cancer), SCCHN (squamous cell carcinoma of the head and neck), NSCLC, or other solid tumors.

In one aspect, the present invention provides for treating cancer by providing to the subject in need thereof an LSD1 inhibitor and immune effector cells (e.g., T cells, NK cells) that are engineered to express a NCAMCAR, wherein the cancer cells express NCAM. In one embodiment, the cancer to be treated is neuroblastoma, or other solid tumors.

In one aspect, the present invention provides for treating cancer by providing to the subject in need thereof an LSD1 inhibitor and immune effector cells (e.g., T cells, NK cells) that are engineered to express a CAIXCAR, wherein the cancer cells express CAIX. In one embodiment, the cancer to be treated is renal cancer, CRC, cervical cancer, or other solid tumors.

In one aspect, the present invention provides for treating cancer by providing to the subject in need thereof an LSD1 inhibitor and immune effector cells (e.g., T cells, NK cells) that are engineered to express an EphA2CAR, wherein the cancer cells express EphA2. In one embodiment, the cancer to be treated is GBM.

In one aspect, the present invention provides for treating cancer by providing to the subject in need thereof an LSD1 inhibitor and immune effector cells (e.g., T cells, NK cells) that are engineered to express a GD3CAR, wherein the cancer cells express GD3. In one embodiment, the cancer to be treated is melanoma.

In one aspect, the present invention provides for treating cancer by providing to the subject in need thereof an LSD1 inhibitor and immune effector cells (e.g., T cells, NK cells) that are engineered to express a Fucosyl GM1CAR, wherein the cancer cells express Fucosyl GM

In one aspect, the present invention provides for treating cancer by providing to the subject in need thereof an LSD1 inhibitor and immune effector cells (e.g., T cells, NK cells) that are engineered to express a sLeCAR, wherein the cancer cells express sLe. In one embodiment, the cancer to be treated is NSCLC, or AML.

In one aspect, the present invention provides for treating cancer by providing to the subject in need thereof an LSD1 inhibitor and immune effector cells (e.g., T cells, NK cells) that are engineered to express a GM3CAR, wherein the cancer cells express GM3.

In one aspect, the present invention provides for treating cancer by providing to the subject in need thereof an LSD1 inhibitor and immune effector cells (e.g., T cells, NK cells) that are engineered to express a TGS5CAR, wherein the cancer cells express TGS5.

In one aspect, the present invention provides for treating cancer by providing to the subject in need thereof an LSD1 inhibitor and immune effector cells (e.g., T cells, NK cells) that are engineered to express a HMWMAACAR, wherein the cancer cells express HMWMAA. In one embodiment, the cancer to be treated is melanoma, glioblastoma, or breast cancer.

In one aspect, the present invention provides for treating cancer by providing to the subject in need thereof an LSD1 inhibitor and immune effector cells (e.g., T cells, NK cells) that are engineered to express an o-acetyl-GD2CAR, wherein the cancer cells express o-acetyl-GD2. In one embodiment, the cancer to be treated is neuroblastoma, or melanoma.

In one aspect, the present invention provides for treating cancer by providing to the subject in need thereof an LSD1 inhibitor and immune effector cells (e.g., T cells, NK cells) that are engineered to express a Folate receptor betaCAR, wherein the cancer cells express CD19. In one embodiment, the cancer to be treated is AML, or myeloma.

In one aspect, the present invention provides for treating cancer by providing to the subject in need thereof an LSD1 inhibitor and immune effector cells (e.g., T cells, NK cells) that are engineered to express a TEM1/CD248CAR, wherein the cancer cells express TEM1/CD248. In one embodiment, the cancer to be treated is a solid tumor.

In one aspect, the present invention provides for treating cancer by providing to the subject in need thereof an LSD1 inhibitor and immune effector cells (e.g., T cells, NK cells) that are engineered to express a TEM7RCAR, wherein the cancer cells express TEM7R. In one embodiment, the cancer to be treated is solid tumor.

In one aspect, the present invention provides for treating cancer by providing to the subject in need thereof an LSD1 inhibitor and immune effector cells (e.g., T cells, NK cells) that are engineered to express a CLDN6CAR, wherein the cancer cells express CLDN6. In one embodiment, the cancer to be treated is ovarian cancer, lung cancer, or breast cancer.

In one aspect, the present invention provides for treating cancer by providing to the subject in need thereof an LSD1 inhibitor and immune effector cells (e.g., T cells, NK cells) that are engineered to express a TSHRCAR, wherein the cancer cells express TSHR. In one embodiment, the cancer to be treated is thyroid cancer, or multiple myeloma.

In one aspect, the present invention provides for treating cancer by providing to the subject in need thereof an LSD1 inhibitor and immune effector cells (e.g., T cells, NK cells) that are engineered to express a GPRC5DCAR, wherein the cancer cells express GPRC5D. In one embodiment, the cancer to be treated is multiple myeloma.

In one aspect, the present invention provides for treating cancer by providing to the subject in need thereof an LSD1 inhibitor and immune effector cells (e.g., T cells, NK cells) that are engineered to express a CXORF61CAR, wherein the cancer cells express CXORF61.

In one aspect, the present invention provides for treating cancer by providing to the subject in need thereof an LSD1 inhibitor and immune effector cells (e.g., T cells, NK cells) that are engineered to express a CD97CAR, wherein the cancer cells express CD97. In one embodiment, the cancer to be treated is B cell malignancies, gastric cancer, pancreatic cancer, esophageal cancer, glioblastoma, breast cancer, or colorectal cancer.

In one aspect, the present invention provides for treating cancer by providing to the subject in need thereof an LSD1 inhibitor and immune effector cells (e.g., T cells, NK cells) that are engineered to express a CD179aCAR, wherein the cancer cells express CD179a. In one embodiment, the cancer to be treated is B cell malignancies.

In one aspect, the present invention provides for treating cancer by providing to the subject in need thereof an LSD1 inhibitor and immune effector cells (e.g., T cells, NK cells) that are engineered to express an ALK CAR, wherein the cancer cells express ALK. In one embodiment, the cancer to be treated is NSCLC, ALCL (anaplastic large cell lymphoma), IMT (inflammatory myofibroblastic tumor), or neuroblastoma.

In one aspect, the present invention provides for treating cancer by providing to the subject in need thereof an LSD1 inhibitor and immune effector cells (e.g., T cells, NK cells) that are engineered to express a Plysialic acid CAR, wherein the cancer cells express Plysialic acid. In one embodiment, the cancer to be treated is small cell lung cancer.

In one aspect, the present invention provides for treating cancer by providing to the subject in need thereof an LSD1 inhibitor and immune effector cells (e.g., T cells, NK cells) that are engineered to express a PLAC1CAR, wherein the cancer cells express PLAC1. In one embodiment, the cancer to be treated is HCC (hepatocellular carcinoma).

In one aspect, the present invention provides for treating cancer by providing to the subject in need thereof an LSD1 inhibitor and immune effector cells (e.g., T cells, NK cells) that are engineered to express a GloboHCAR, wherein the cancer cells express GloboH. In one embodiment, the cancer to be treated is ovarian cancer, gastric cancer, prostate cancer, lung cancer, breast cancer, or pancreatic cancer.

In one aspect, the present invention provides for treating cancer by providing to the subject in need thereof an LSD1 inhibitor and immune effector cells (e.g., T cells, NK cells) that are engineered to express a NY-BR-1CAR, wherein the cancer cells express NY-BR-1. In one embodiment, the cancer to be treated is breast cancer.

In one aspect, the present invention provides for treating cancer by providing to the subject in need thereof an LSD1 inhibitor and immune effector cells (e.g., T cells, NK cells) that are engineered to express a UPK2CAR, wherein the cancer cells express UPK2. In one embodiment, the cancer to be treated is bladder cancer.

In one aspect, the present invention provides for treating cancer by providing to the subject in need thereof an LSD1 inhibitor and immune effector cells (e.g., T cells, NK cells) that are engineered to express a HAVCR1CAR, wherein the cancer cells express HAVCR1. In one embodiment, the cancer to be treated is renal cancer.

In one aspect, the present invention provides for treating cancer by providing to the subject in need thereof an LSD1 inhibitor and immune effector cells (e.g., T cells, NK cells) that are engineered to express a ADRB3CAR, wherein the cancer cells express ADRB3. In one embodiment, the cancer to be treated is Ewing sarcoma.

In one aspect, the present invention provides for treating cancer by providing to the subject in need thereof an LSD1 inhibitor and immune effector cells (e.g., T cells, NK cells) that are engineered to express a PANX3CAR, wherein the cancer cells express PANX3. In one embodiment, the cancer to be treated is osteosarcoma.

In one aspect, the present invention provides for treating cancer by providing to the subject in need thereof an LSD1 inhibitor and immune effector cells (e.g., T cells, NK cells) that are engineered to express a GPR20CAR, wherein the cancer cells express GPR20. In one embodiment, the cancer to be treated is GIST.

In one aspect, the present invention provides for treating cancer by providing to the subject in need thereof an LSD1 inhibitor and immune effector cells (e.g., T cells, NK cells) that are engineered to express a LY6KCAR, wherein the cancer cells express LY6K. In one embodiment, the cancer to be treated is breast cancer, lung cancer, ovary caner, or cervix cancer.

In one aspect, the present invention provides for treating cancer by providing to the subject in need thereof an LSD1 inhibitor and immune effector cells (e.g., T cells, NK cells) that are engineered to express a OR51E2CAR, wherein the cancer cells express OR51E2. In one embodiment, the cancer to be treated is prostate cancer.

In one aspect, the present invention provides for treating cancer by providing to the subject in need thereof an LSD1 inhibitor and immune effector cells (e.g., T cells, NK cells) that are engineered to express a TARPCAR, wherein the cancer cells express TARP. In one embodiment, the cancer to be treated is prostate cancer.

In one aspect, the present invention provides for treating cancer by providing to the subject in need thereof an LSD1 inhibitor and immune effector cells (e.g., T cells, NK cells) that are engineered to express a WT1CAR, wherein the cancer cells express WT1.

In one aspect, the present invention provides for treating cancer by providing to the subject in need thereof an LSD1 inhibitor and immune effector cells (e.g., T cells, NK cells) that are engineered to express a NY-ESO-1CAR, wherein the cancer cells express NY-ESO-1.

In one aspect, the present invention provides for treating cancer by providing to the subject in need thereof an LSD1 inhibitor and immune effector cells (e.g., T cells, NK cells) that are engineered to express a LAGE-1a CAR, wherein the cancer cells express LAGE-1a.

In one aspect, the present invention provides for treating cancer by providing to the subject in need thereof an LSD1 inhibitor and immune effector cells (e.g., T cells, NK cells) that are engineered to express a MAGE-A1CAR, wherein the cancer cells express MAGE-A1. In one embodiment, the cancer to be treated is melanoma.

In one aspect, the present invention provides for treating cancer by providing to the subject in need thereof an LSD1 inhibitor and immune effector cells (e.g., T cells, NK cells) that are engineered to express a MAGE A1CAR, wherein the cancer cells express MAGE A1.

In one aspect, the present invention provides for treating cancer by providing to the subject in need thereof an LSD1 inhibitor and immune effector cells (e.g., T cells, NK cells) that are engineered to express a ETV6-AML CAR, wherein the cancer cells express ETV6-AML.

In one aspect, the present invention provides for treating cancer by providing to the subject in need thereof an LSD1 inhibitor and immune effector cells (e.g., T cells, NK cells) that are engineered to express a sperm protein 17 CAR, wherein the cancer cells express sperm protein 17. In one embodiment, the cancer to be treated is ovarian cancer, HCC, or NSCLC.

In one aspect, the present invention provides for treating cancer by providing to the subject in need thereof an LSD1 inhibitor and immune effector cells (e.g., T cells, NK cells) that are engineered to express a XAGE1CAR, wherein the cancer cells express XAGE1. In one embodiment, the cancer to be treated is Ewings, or rhabdo cancer.

In one aspect, the present invention provides for treating cancer by providing to the subject in need thereof an LSD1 inhibitor and immune effector cells (e.g., T cells, NK cells) that are engineered to express a Tie 2 CAR, wherein the cancer cells express Tie 2. In one embodiment, the cancer to be treated is a solid tumor.

In one aspect, the present invention provides for treating cancer by providing to the subject in need thereof an LSD1 inhibitor and immune effector cells (e.g., T cells, NK cells) that are engineered to express a MAD-CT-1CAR, wherein the cancer cells express MAD-CT-1. In one embodiment, the cancer to be treated is prostate cancer, or melanoma.

In one aspect, the present invention provides for treating cancer by providing to the subject in need thereof an LSD1 inhibitor and immune effector cells (e.g., T cells, NK cells) that are engineered to express a MAD-CT-2CAR, wherein the cancer cells express MAD-CT-2. In one embodiment, the cancer to be treated is prostate cancer, melanoma.

In one aspect, the present invention provides for treating cancer by providing to the subject in need thereof an LSD1 inhibitor and immune effector cells (e.g., T cells, NK cells) that are engineered to express a Fos-related antigen 1 CAR, wherein the cancer cells express Fos-related antigen 1. In one embodiment, the cancer to be treated is glioma, squamous cell cancer, or pancreatic cancer.

In one aspect, the present invention provides for treating cancer by providing to the subject in need thereof an LSD1 inhibitor and immune effector cells (e.g., T cells, NK cells) that are engineered to express a p53CAR, wherein the cancer cells express p53.

In one aspect, the present invention provides for treating cancer by providing to the subject in need thereof an LSD1 inhibitor and immune effector cells (e.g., T cells, NK cells) that are engineered to express a prostein CAR, wherein the cancer cells express prostein.

In one aspect, the present invention provides for treating cancer by providing to the subject in need thereof an LSD1 inhibitor and immune effector cells (e.g., T cells, NK cells) that are engineered to express a survivin and telomerase CAR, wherein the cancer cells express survivin and telomerase.

In one aspect, the present invention provides for treating cancer by providing to the subject in need thereof an LSD1 inhibitor and immune effector cells (e.g., T cells, NK cells) that are engineered to express a PCTA-1/Galectin 8 CAR, wherein the cancer cells express PCTA-1/Galectin 8.

In one aspect, the present invention provides for treating cancer by providing to the subject in need thereof an LSD1 inhibitor and immune effector cells (e.g., T cells, NK cells) that are engineered to express a MelanA/MART1CAR, wherein the cancer cells express MelanA/MART1.

In one aspect, the present invention provides for treating cancer by providing to the subject in need thereof an LSD1 inhibitor and immune effector cells (e.g., T cells, NK cells) that are engineered to express a Ras mutant CAR, wherein the cancer cells express Ras mutant.

In one aspect, the present invention provides for treating cancer by providing to the subject in need thereof an LSD1 inhibitor and immune effector cells (e.g., T cells, NK cells) that are engineered to express a p53 mutant CAR, wherein the cancer cells express p53 mutant.

In one aspect, the present invention provides for treating cancer by providing to the subject in need thereof an LSD1 inhibitor and immune effector cells (e.g., T cells, NK cells) that are engineered to express a hTERT CAR, wherein the cancer cells express hTERT.

In one aspect, the present invention provides for treating cancer by providing to the subject in need thereof an LSD1 inhibitor and immune effector cells (e.g., T cells, NK cells) that are engineered to express a sarcoma translocation breakpoints CAR, wherein the cancer cells express sarcoma translocation breakpoints. In one embodiment, the cancer to be treated is sarcoma.

In one aspect, the present invention provides for treating cancer by providing to the subject in need thereof an LSD1 inhibitor and immune effector cells (e.g., T cells, NK cells) that are engineered to express a ML-IAP CAR, wherein the cancer cells express ML-IAP. In one embodiment, the cancer to be treated is melanoma.

In one aspect, the present invention provides for treating cancer by providing to the subject in need thereof an LSD1 inhibitor and immune effector cells (e.g., T cells, NK cells) that are engineered to express a ERGCAR, wherein the cancer cells express ERG (TMPRSS2 ETS fusion gene).

In one aspect, the present invention provides for treating cancer by providing to the subject in need thereof an LSD1 inhibitor and immune effector cells (e.g., T cells, NK cells) that are engineered to express a NA17CAR, wherein the cancer cells express NA17. In one embodiment, the cancer to be treated is melanoma.

In one aspect, the present invention provides for treating cancer by providing to the subject in need thereof an LSD1 inhibitor and immune effector cells (e.g., T cells, NK cells) that are engineered to express a PAX3CAR, wherein the cancer cells express PAX3. In one embodiment, the cancer to be treated is alveolar rhabdomyosarcoma.

In one aspect, the present invention provides for treating cancer by providing to the subject in need thereof an LSD1 inhibitor and immune effector cells (e.g., T cells, NK cells) that are engineered to express an androgen receptor CAR, wherein the cancer cells express androgen receptor. In one embodiment, the cancer to be treated is metastatic prostate cancer.

In one aspect, the present invention provides for treating cancer by providing to the subject in need thereof an LSD1 inhibitor and immune effector cells (e.g., T cells, NK cells) that are engineered to express a Cyclin B1CAR, wherein the cancer cells express Cyclin B1.

In one aspect, the present invention provides for treating cancer by providing to the subject in need thereof an LSD1 inhibitor and immune effector cells (e.g., T cells, NK cells) that are engineered to express a MYCNCAR, wherein the cancer cells express MYCN.

In one aspect, the present invention provides for treating cancer by providing to the subject in need thereof an LSD1 inhibitor and immune effector cells (e.g., T cells, NK cells) that are engineered to express a RhoC CAR, wherein the cancer cells express RhoC.

In one aspect, the present invention provides for treating cancer by providing to the subject in need thereof an LSD1 inhibitor and immune effector cells (e.g., T cells, NK cells) that are engineered to express a TRP-2CAR, wherein the cancer cells express TRP-2. In one embodiment, the cancer to be treated is melanoma.

In one aspect, the present invention provides for treating cancer by providing to the subject in need thereof an LSD1 inhibitor and immune effector cells (e.g., T cells, NK cells) that are engineered to express a CYP1B1CAR, wherein the cancer cells express CYP1B1. In one embodiment, the cancer to be treated is breast cancer, colon cancer, lung cancer, esophagus cancer, skin cancer, lymph node cancer, brain cancer, or testis cancer.

In one aspect, the present invention provides for treating cancer by providing to the subject in need thereof an LSD1 inhibitor and immune effector cells (e.g., T cells, NK cells) that are engineered to express a BORIS CAR, wherein the cancer cells express BORIS. In one embodiment, the cancer to be treated is lung cancer.

In one aspect, the present invention provides for treating cancer by providing to the subject in need thereof an LSD1 inhibitor and immune effector cells (e.g., T cells, NK cells) that are engineered to express a SART3CAR, wherein the cancer cells express SART3

In one aspect, the present invention provides for treating cancer by providing to the subject in need thereof an LSD1 inhibitor and immune effector cells (e.g., T cells, NK cells) that are engineered to express a PAX5CAR, wherein the cancer cells express PAX5.

In one aspect, the present invention provides for treating cancer by providing to the subject in need thereof an LSD1 inhibitor and immune effector cells (e.g., T cells, NK cells) that are engineered to express a OY-TES1CAR, wherein the cancer cells express OY-TES1.

In one aspect, the present invention provides for treating cancer by providing to the subject in need thereof an LSD1 inhibitor and immune effector cells (e.g., T cells, NK cells) that are engineered to express a LCK CAR, wherein the cancer cells express LCK.

In one aspect, the present invention provides for treating cancer by providing to the subject in need thereof an LSD1 inhibitor and immune effector cells (e.g., T cells, NK cells) that are engineered to express a AKAP-4CAR, wherein the cancer cells express AKAP-4.

In one aspect, the present invention provides for treating cancer by providing to the subject in need thereof an LSD1 inhibitor and immune effector cells (e.g., T cells, NK cells) that are engineered to express a SSX2CAR, wherein the cancer cells express SSX2.

In one aspect, the present invention provides for treating cancer by providing to the subject in need thereof an LSD1 inhibitor and immune effector cells (e.g., T cells, NK cells) that are engineered to express a RAGE-1CAR, wherein the cancer cells express RAGE-1. In one embodiment, the cancer to be treated is RCC (renal cell cancer), or other solid tumors

In one aspect, the present invention provides for treating cancer by providing to the subject in need thereof an LSD1 inhibitor and immune effector cells (e.g., T cells, NK cells) that are engineered to express a human telomerase reverse transcriptaseCAR, wherein the cancer cells express human telomerase reverse transcriptase. In one embodiment, the cancer to be treated is solid tumors.

In one aspect, the present invention provides for treating cancer by providing to the subject in need thereof an LSD1 inhibitor and immune effector cells (e.g., T cells, NK cells) that are engineered to express a RU1CAR, wherein the cancer cells express RU1.

In one aspect, the present invention provides for treating cancer by providing to the subject in need thereof an LSD1 inhibitor and immune effector cells (e.g., T cells, NK cells) that are engineered to express a RU2CAR, wherein the cancer cells express RU2.

In one aspect, the present invention provides for treating cancer by providing to the subject in need thereof an LSD1 inhibitor and immune effector cells (e.g., T cells, NK cells) that are engineered to express an intestinal carboxyl esteraseCAR, wherein the cancer cells express intestinal carboxyl esterase. In one embodiment, the cancer to be treated is thyroid cancer, RCC, CRC (colorectal cancer), breast cancer, or other solid tumors.

In one aspect, the present invention provides for treating cancer by providing to the subject in need thereof an LSD1 inhibitor and immune effector cells (e.g., T cells, NK cells) that are engineered to express a Prostase CAR, wherein the cancer cells express Prostase.

In one aspect, the present invention provides for treating cancer by providing to the subject in need thereof an LSD1 inhibitor and immune effector cells (e.g., T cells, NK cells) that are engineered to express a PAPCAR, wherein the cancer cells express PAP.

In one aspect, the present invention provides for treating cancer by providing to the subject in need thereof an LSD1 inhibitor and immune effector cells (e.g., T cells, NK cells) that are engineered to express an IGF-I receptor CAR, wherein the cancer cells express IGF-I receptor.

In one aspect, the present invention provides for treating cancer by providing to the subject in need thereof an LSD1 inhibitor and immune effector cells (e.g., T cells, NK cells) that are engineered to express a gp100 CAR, wherein the cancer cells express gp100.

In one aspect, the present invention provides for treating cancer by providing to the subject in need thereof an LSD1 inhibitor and immune effector cells (e.g., T cells, NK cells) that are engineered to express a bcr-abl CAR, wherein the cancer cells express bcr-abl.

In one aspect, the present invention provides for treating cancer by providing to the subject in need thereof an LSD1 inhibitor and immune effector cells (e.g., T cells, NK cells) that are engineered to express a tyrosinase CAR, wherein the cancer cells express tyrosinase.

In one aspect, the present invention provides for treating cancer by providing to the subject in need thereof immune effector cells (e.g., T cells, NK cells) that are engineered to express a Fucosyl GM1CAR, wherein the cancer cells express Fucosyl GM1.

In one aspect, the present invention provides for treating cancer by providing to the subject in need thereof an LSD1 inhibitor and immune effector cells (e.g., T cells, NK cells) that are engineered to express a mut hsp70-2CAR, wherein the cancer cells express mut hsp70-2. In one embodiment, the cancer to be treated is melanoma.

In one aspect, the present invention provides for treating cancer by providing to the subject in need thereof immune effector cells (e.g., T cells, NK cells) that are engineered to express a CD79a CAR, wherein the cancer cells express CD79a.

In one aspect, the present invention provides for treating cancer by providing to the subject in need thereof immune effector cells (e.g., T cells, NK cells) that are engineered to express a CD79b CAR, wherein the cancer cells express CD79b.

In one aspect, the present invention provides for treating cancer by providing to the subject in need thereof immune effector cells (e.g., T cells, NK cells) that are engineered to express a CD72 CAR, wherein the cancer cells express CD72.

In one aspect, the present invention provides for treating cancer by providing to the subject in need thereof immune effector cells (e.g., T cells, NK cells) that are engineered to express a LAIR1 CAR, wherein the cancer cells express LAIR1.

In one aspect, the present invention provides for treating cancer by providing to the subject in need thereof immune effector cells (e.g., T cells, NK cells) that are engineered to express a FCAR CAR, wherein the cancer cells express FCAR.

In one aspect, the present invention provides for treating cancer by providing to the subject in need thereof immune effector cells (e.g., T cells, NK cells) that are engineered to express a LILRA2 CAR, wherein the cancer cells express LILRA2.

In one aspect, the present invention provides for treating cancer by providing to the subject in need thereof immune effector cells (e.g., T cells, NK cells) that are engineered to express a CD300LF CAR, wherein the cancer cells express CD300LF.

In one aspect, the present invention provides for treating cancer by providing to the subject in need thereof immune effector cells (e.g., T cells, NK cells) that are engineered to express a CLEC12A CAR, wherein the cancer cells express CLEC12A.

In one aspect, the present invention provides for treating cancer by providing to the subject in need thereof immune effector cells (e.g., T cells, NK cells) that are engineered to express a BST2 CAR, wherein the cancer cells express BST2.

In one aspect, the present invention provides for treating cancer by providing to the subject in need thereof immune effector cells (e.g., T cells, NK cells) that are engineered to express an EMR2 CAR, wherein the cancer cells express EMR2.

In one aspect, the present invention provides for treating cancer by providing to the subject in need thereof immune effector cells (e.g., T cells, NK cells) that are engineered to express a LY75 CAR, wherein the cancer cells express LY75.

In one aspect, the present invention provides for treating cancer by providing to the subject in need thereof immune effector cells (e.g., T cells, NK cells) that are engineered to express a GPC3 CAR, wherein the cancer cells express GPC3.

In one aspect, the present invention provides for treating cancer by providing to the subject in need thereof immune effector cells (e.g., T cells, NK cells) that are engineered to express a FCRL5 CAR, wherein the cancer cells express FCRL5.

In one aspect, the present invention provides for treating cancer by providing to the subject in need thereof immune effector cells (e.g., T cells, NK cells) that are engineered to express an IGLL1 CAR, wherein the cancer cells express IGLL1.

In the treatments of the invention, the population of immune effector cells comprises, e.g., consists of, T cells.

In one aspect, the present invention relates to treating a subject in vivo using an LSD1 inhibitor and a PD1 CAR such that growth of cancerous tumors is inhibited. A PD1 CAR may be used alone to inhibit the growth of cancerous tumors. Alternatively, PD1 CAR may be used in conjunction with other CARs, immunogenic agents, standard cancer treatments, or other antibodies. In one embodiment, the subject is treated with a PD1 CAR and an XCAR described herein. In an embodiment, a PD1 CAR is used in conjunction with another CAR, e.g., a CAR described herein, and a kinase inhibitor, e.g., a kinase inhibitor described herein.

In another aspect, treating a subject, e.g., reducing or ameliorating, a hyperproliferative condition or disorder (e.g., a cancer), e.g., a solid tumor, a soft tissue tumor, a hematological cancer, or a metastatic lesion, in a subject, is provided. In embodiments the treating comprises administration of an LSD1 inhibitor and a population of immune effector cells, e.g., T cells, engineered to express a CAR. In the treatments of the invention, the population of immune effector cells comprises, e.g., consists of, T cells.

In yet another aspect, the invention features treating a subject having a disease associated with expression of a tumor antigen (e.g., an antigen described herein), comprising administering to the subject an effective amount of a cell, e.g., an immune effector cell (*e.g*., a population of immune effector cells) comprising a CAR molecule, wherein the CAR molecule comprises an antigen binding domain, a transmembrane domain, and an intracellular domain, said intracellular domain comprises a costimulatory domain and/or a primary signaling domain, wherein said antigen binding domain binds to the tumor antigen associated with the disease, e.g. a tumor antigen as described herein. In the treatments of the invention, the population of immune effector cells comprises, e.g., consists of, T cells.

In a related aspect, the invention features treating a subject having a disease associated with expression of a tumor antigen. The treating comprises administering to the subject an effective amount of a cell, e.g., an immune effector cell (*e.g.*, a population of immune effector cells) comprising a CAR molecule, in combination (in addition to the LSD1 inhibitor) with an agent that increases the efficacy of the immune cell, wherein:
the agent that increases the efficacy of the immune cell is chosen from one or more of:
(i) a protein phosphatase inhibitor;
(ii) a kinase inhibitor;
(iii) a cytokine;
(iv) an inhibitor of an immune inhibitory molecule; or
(v) an agent that decreases the level or activity of a T_{REG} cell. In the treatments of the invention, the population of immune effector cells comprises, e.g., consists of, T cells.

In another aspect, the disclosure features a composition comprising an immune effector cell (*e.g*., a population of immune effector cells) comprising a CAR molecule (e.g., a CAR molecule as described herein) for use in treating a subject having a disease associated with expression of a tumor antigen, e.g., a disorder as described herein.

In certain instances of any of the aforesaid methods, treatments or uses, the disease associated with a tumor antigen, e.g., a tumor antigen described herein, is selected from a proliferative disease such as a cancer or malignancy or a precancerous condition such as a myelodysplasia, a myelodysplastic syndrome or a preleukemia, or is a non-cancer related indication associated with expression of a tumor antigen described herein. In the treatments of the invention, the disease associated with expression of a tumor antigen is chosen from a proliferative disease, a precancerous condition, and a cancer. In one embodiment, the disease is a cancer described herein, e.g., a cancer described herein as being associated with a target described herein. In one embodiment, the disease is a hematologic cancer. In one embodiment, the hematologic cancer is leukemia. In one embodiment, the cancer is selected from the group consisting of one or more acute leukemias including but not limited to B-cell acute lymphoid leukemia ("BALL"), T-cell acute lymphoid leukemia ("TALL"), acute lymphoid leukemia (ALL); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and to disease associated with expression of a tumor antigen described herein include, but not limited to, atypical and/or non-classical cancers, malignancies, precancerous conditions or proliferative diseases expressing a tumor antigen as described herein; and any combination thereof. In another embodiment, the disease associated with a tumor antigen described herein is a solid tumor.

In certain embodiments, the methods, treatments or or uses are carried out in combination with an agent that increases the efficacy of the immune effector cell, e.g., an agent as described herein.

In any of the aforesaid methods, treatments or uses, the disease associated with expression of the tumor antigen is selected from the group consisting of a proliferative disease, a precancerous condition, a cancer, and a non-cancer related indication associated with expression of the tumor antigen. In the treatments of the invention, the disease associated with expression of a tumor antigen is chosen from a proliferative disease, a precancerous condition, and a cancer.

The cancer can be a hematologic cancer, e.g., a cancer chosen from one or more of chronic lymphocytic leukemia (CLL), acute leukemias, acute lymphoid leukemia (ALL), acute myeloid leukemia (AML), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, or pre-leukemia.

The cancer can also be chosen from colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.

In certain embodiments of the methods, treatments or uses described herein, the CAR molecule is administered in combination with an agent that increases the efficacy of the immune effector cell (in addition to the LSD1 inhibitor), e.g., one or more of a protein phosphatase inhibitor, a kinase inhibitor, a cytokine, an inhibitor of an immune inhibitory molecule; or an agent that decreases the level or activity of a T_{REG} cell.

In certain embodiments of the methods, treatments or uses described herein, the protein phosphatase inhibitor is a SHP-1 inhibitor and/or an SHP-2 inhibitor.

In other embodiments of the methods, treatments or uses described herein, kinase inhibitor is chosen from one or more of a CDK4 inhibitor, a CDK4/6 inhibitor (e.g., palbociclib), a BTK inhibitor (e.g., ibrutinib or RN-486), an mTOR inhibitor (e.g., rapamycin or everolimus (RAD001)), an MNK inhibitor, or a dual P13K/mTOR inhibitor. In one embodiment, the BTK inhibitor does not reduce or inhibit the kinase activity of interleukin-2-inducible kinase (ITK).

In other embodiments of the methods, treatments or uses described herein, the agent that inhibits the immune inhibitory molecule comprises an antibody or antibody fragment, an inhibitory nucleic acid, a clustered regularly interspaced short palindromic repeats (CRISPR), a transcription-activator like effector nuclease (TALEN), or a zinc finger endonuclease (ZFN) that inhibits the expression of the inhibitory molecule.

In other embodiments of the methods, treatments or uses described herein, the agent that decreases the level or activity of the TREG cells is chosen from cyclophosphamide, anti-GITR antibody, CD25-depletion, or a combination thereof.

In certain embodiments of the methods, treatments or uses described herein, the immune inhibitory molecule is selected from the group consisting of PD1, PD-L1, CTLA-4, TIM-3, LAG-3, VISTA, BTLA, TIGIT, LAIR1, CD160, 2B4, TGF beta, CEACAM-1, CEACAM-3, and CEACAM-5.

In other embodiments, the agent that inhibits the inhibitory molecule comprises a first polypeptide comprising an inhibitory molecule or a fragment thereof and a second polypeptide that provides a positive signal to the cell, and wherein the first and second polypeptides are expressed on the CAR-containing immune cells, wherein (i) the first polypeptide comprises PD1, PD-L1, CTLA-4, TIM-3, LAG3, VISTA, BTLA, TIGIT, LAIR1, CD160, 2B4, TGF beta, CEACAM-1, CEACAM-3, and CEACAM-5 or a fragment thereof; and/or (ii) the second polypeptide comprises an intracellular signaling domain comprising a primary signaling domain and/or a costimulatory signaling domain. In one embodiment, the primary signaling domain comprises a functional domain of CD3 zeta; and/or the costimulatory signaling domain comprises a functional domain of a protein selected from 41BB, CD27 and CD28.

In other embodiments, cytokine is chosen from IL-7, IL-15 or IL-21, or combinations thereof.

In other embodiments, the immune effector cell comprising the CAR molecule and a second, e.g., any of the combination therapies disclosed herein (e.g., the agent that that increases the efficacy of the immune effector cell) are administered substantially simultaneously or sequentially.

In other embodiments, the immune cell comprising the CAR molecule is administered in combination with a molecule that targets GITR and/or modulates GITR function. In certain embodiments, the molecule targeting GITR and/or modulating GITR function is administered prior to the CAR-expressing cell or population of cells, or prior to apheresis.

In one embodiment, lymphocyte infusion, for example allogeneic lymphocyte infusion, is used in the treatment of the cancer, wherein the lymphocyte infusion comprises at least one CAR-expressing cell of the present disclosure. In one embodiment, autologous lymphocyte infusion is used in the treatment of the cancer, wherein the autologous lymphocyte infusion comprises at least one CAR-expressing cell described herein.

In one embodiment, the cell is a T cell and the T cell is diaglycerol kinase (DGK) deficient. In one embodiment, the cell is a T cell and the T cell is Ikaros deficient. In one embodiment, the cell is a T cell and the T cell is both DGK and Ikaros deficient.

In one embodiment, the method or treatment includes administering a cell expressing the CAR molecule, as described herein, in combination with an agent which enhances the activity of a CAR-expressing cell, wherein the agent is a cytokine, e.g., IL-7, IL-15, IL-18, IL-21, or a combination thereof. The cytokine can be delivered in combination with, e.g., simultaneously or shortly after, administration of the CAR-expressing cell. Alternatively, the cytokine can be delivered after a prolonged period of time after administration of the CAR-expressing cell, e.g., after assessment of the subject's response to the CAR-expressing cell. In one embodiment the cytokine is administered to the subject simultaneously (e.g., administered on the same day) with or shortly after administration (e.g., administered 1 day, 2 days, 3 days, 4 days, 5 days, 6 days, or 7 days after administration) of the cell or population of cells of any of claims 61-80. In other embodiments, the cytokine is administered to the subject after a prolonged period of time (e.g., e.g., at least 2 weeks, 3 weeks, 4 weeks, 6 weeks, 8 weeks, 10 weeks, or more) after administration of the cell or population of cells of any of claims 61-80, or after assessment of the subject's response to the cell.

In other embodiments, the cells expressing a CAR molecule are administered in combination with an agent that ameliorates one or more side effects associated with administration of a cell expressing a CAR molecule. Side effects associated with the CAR-expressing cell can be chosen from cytokine release syndrome (CRS) or hemophagocytic lymphohistiocytosis (HLH).

In embodiments of any of the aforeseaid methods, treatments or uses, the cells expressing the CAR molecule are administered in combination with an agent that treats the disease associated with expression of the tumor antigen, e.g., any of the second or third therapies disclosed herein. Additional exemplary combinations include one or more of the following.

In another embodiment, the cell expressing the CAR molecule, e.g., as described herein, can be administered in combination with another agent, e.g., a kinase inhibitor and/or checkpoint inhibitor described herein. In an embodiment, a cell expressing the CAR molecule can further express another agent, e.g., an agent which enhances the activity of a CAR-expressing cell.

For example, in one embodiment, the agent that enhances the activity of a CAR-expressing cell can be an agent which inhibits an inhibitory molecule (e.g., an immune inhibitor molecule). Examples of inhibitory molecules include PD1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGF beta.

In one embodiment, the agent that inhibits the inhibitory molecule is an inhibitory nucleic acid is a dsRNA, a siRNA, or a shRNA. In embodiments, the inhibitory nucleic acid is linked to the nucleic acid that encodes a component of the CAR molecule. For example, the inhibitory molecule can be expressed on the CAR-expressing cell.

In another embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 or TGF beta, or a fragment of any of these (e.g., at least a portion of the extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD1 or a fragment thereof (e.g., at least a portion of the extracellular domain of PD1), and a second polypeptide of an intracellular signaling domain described herein (e.g., a CD28 signaling domain described herein and/or a CD3 zeta signaling domain described herein).

In one embodiment, the CAR-expressing immune effector cell of the present disclosure, e.g., T cell or NK cell, is administered to a subject that has received a previous stem cell transplantation, e.g., autologous stem cell transplantation.

In one embodiment, the CAR-expressing immune effector cell of the present disclosure, e.g., T cell or NK cells, is administered to a subject that has received a previous dose of melphalan.

In one embodiment, the cell expressing a CAR molecule, e.g., a CAR molecule described herein, is administered in combination with an agent that increases the efficacy of a cell expressing a CAR molecule, e.g., an agent described herein.

In one embodiment, the cells expressing a CAR molecule, e.g., a CAR molecule described herein, are administered in combination with an LSD1 inhibitor. While not wishing to be bound by theory, it is believed that treatment with an LSD1 inhibitor is accompanied by a decrease in PD-1 positive T cells or an increase in PD-1 negative cells. PD-1 positive T cells, but not PD-1 negative T cells, can be exhausted by engagement with cells which express a PD-1 ligand, e.g., PD-L1 or PD-L2.

In an embodiment this approach can be used to optimize the performance of CAR cells described herein in the subject. While not wishing to be bound by theory, it is believed that, in an embodiment, the performance of endogenous, non-modified immune effector cells, e.g., T cells or NK cells, is improved. While not wishing to be bound by theory, it is believed that, in an embodiment, the performance of a target antigen CAR- expressing cell is improved. In other embodiments, cells, e.g., T cells or NK cells, which have, or will be engineered to express a CAR, can be treated ex vivo by contact with an amount of an LSD1 inhibitor that increases the number of PD1 negative immune effector cells, e.g., T cells or increases the ratio of PD1 negative immune effector cells, e.g., T cells/ PD1 positive immune effector cells, e.g., T cells.

In an embodiment, administration of an LSD1 inhibitor is initiated prior to administration of an CAR expressing cell described herein, e.g., T cells or NK cells. In an embodiment, the CAR cells are administered after a sufficient time, or sufficient dosing, of an LSD1 inhibitor, such that the level of PD1 negative immune effector cells, e.g., T cells or NK cells, or the ratio of PD1 negative immune effector cells, e.g., T cells/ PD1 positive immune effector cells, e.g., T cells, has been, at least transiently, increased.

In an embodiment, the cell, e.g., T cell or NK cell, to be engineered to express a CAR, is harvested after a sufficient time, or after sufficient dosing of the LSD1 inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In one embodiment, the cell expressing a CAR molecule, e.g., a CAR molecule described herein, is administered in combination with an agent that ameliorates one or more side effect associated with administration of a cell expressing a CAR molecule, e.g., an agent described herein.

In one embodiment, the cell expressing a CAR molecule, e.g., a CAR molecule described herein, is administered in combination with an agent that treats the disease associated with a cancer associated antigen as described herein, e.g., an agent described herein.

In one embodiment, a cell expressing two or more CAR molecules, e.g., as described herein, is administered to a subject in need thereof to treat cancer. In one embodiment, a population of cells including a CAR expressing cell, e.g., as described herein, is administered to a subject in need thereof to treat cancer.

In one embodiment, the cell expressing a CAR molecule, e.g., a CAR molecule described herein, is administered at a dose and/or dosing schedule described herein.

In one embodiment, the CAR molecule is introduced into immune effector cells (e.g., T cells, NK cells), e.g., using *in vitro* transcription, and the subject (e.g., human) receives an initial administration of cells comprising a CAR molecule, and one or more subsequent administrations of cells comprising a CAR molecule, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of cells comprising a CAR molecule are administered to the subject (e.g., human) per week, e.g., 2, 3, or 4 administrations of cells comprising a CAR molecule are administered per week. In one embodiment, the subject (e.g., human subject) receives more than one administration of cells comprising a CAR molecule per week (e.g., 2, 3 or 4 administrations per week) (also referred to herein as a cycle), followed by a week of no administration of cells comprising a CAR molecule, and then one or more additional administration of cells comprising a CAR molecule (e.g., more than one administration of the cells comprising a CAR molecule per week) is administered to the subject. In another embodiment, the subject (e.g., human subject) receives more than one cycle of cells comprising a CAR molecule, and the time between each cycle is less than 10, 9, 8, 7, 6, 5, 4, or 3 days. In one embodiment, the cells comprising a CAR molecule are administered every other day for 3 administrations per week. In one embodiment, the cells comprising a CAR molecule are administered for at least two, three, four, five, six, seven, eight or more weeks.

In one embodiment, the cells expressing a CAR molecule, e.g., a CAR molecule described herein, are administered as a first line treatment for the disease, e.g., the cancer, e.g., the cancer described herein. In another embodiment, the cells expressing a CAR molecule, e.g., a CAR molecule described herein, are administered as a second, third, fourth line treatment for the disease, e.g., the cancer, e.g., the cancer described herein.

In one embodiment, a population of cells described herein is administered.

In another aspect, the disclosure pertains to the isolated nucleic acid molecule encoding a CAR of the disclosure, the isolated polypeptide molecule of a CAR of the disclosure, the vector comprising a CAR of the disclosure, and the cell comprising a CAR of the disclosure for use as a medicament.

In another aspect, the disclosure pertains to the isolated nucleic acid molecule encoding a CAR of the disclosure, the isolated polypeptide molecule of a CAR of the disclosure, the vector comprising a CAR of the disclosure, and the cell comprising a CAR of the disclosure for use in the treatment of a disease expressing a cancer associated antigen as described herein.

In another aspect, the disclosure pertains to a cell expressing a CAR molecule described herein for use as a medicament in combination with a cytokine, e.g., IL-7, IL-15 and/or IL-21 as described herein. In another aspect, the disclosure pertains to a cytokine described herein for use as a medicament in combination with a cell expressing a CAR molecule described herein.

In another aspect, the disclosure pertains to a cell expressing a CAR molecule described herein for use as a medicament in combination with a kinase inhibitor and/or a checkpoint inhibitor as described herein. In another aspect, the disclosure pertains to a kinase inhibitor and/or a checkpoint inhibitor described herein for use as a medicament in combination with a cell expressing a CAR molecule described herein.

In another aspect, the disclosure pertains to a cell expressing a CAR molecule described herein for use in combination with a cytokine, e.g., IL-7, IL-15 and/or IL-21 as described herein, in the treatment of a disease expressing a tumor antigen targeted by the CAR. In another aspect, the disclosure pertains to a cytokine described herein for use in combination with a cell expressing a CAR molecule described herein, in the treatment of a disease expressing a tumor antigen targeted by the CAR.

In another aspect, the disclosure pertains to a cell expressing a CAR molecule described herein for use in combination with a kinase inhibitor and/or a checkpoint inhibitor as described herein, in the treatment of a disease expressing a tumor antigen targeted by the CAR. In another aspect, the disclosure pertains to a kinase inhibitor and/or a checkpoint inhibitor described herein for use in combination with a cell expressing a CAR molecule described herein, in the treatment of a disease expressing a tumor antigen targeted by the CAR.

In another aspect, the present disclosure provides a method comprising administering a CAR molecule, e.g., a CAR molecule described herein, or a cell comprising a nucleic acid encoding a CAR molecule, e.g., a CAR molecule described herein. In one instance, the subject has a disorder described herein, e.g., the subject has cancer, e.g., the subject has a cancer and has tumor-supporting cells which express a tumor-supporting antigen described herein. In one embodiment, the subject is a human.

In another aspect, the disclosure pertains to treating a subject having a disease associated with expression of a tumor-supporting antigen as described herein comprising administering to the subject an effective amount of a cell comprising a CAR molecule, e.g., a CAR molecule described herein.

In yet another aspect, the disclosure features treating a subject having a disease associated with expression of a tumor-supporting antigen, comprising administering to the subject an effective amount of a cell, e.g., an immune effector cell (*e.g*., a population of immune effector cells) comprising a CAR molecule, wherein the CAR molecule comprises an antigen binding domain, a transmembrane domain, and an intracellular domain, said intracellular domain comprises a costimulatory domain and/or a primary signaling domain, wherein said antigen binding domain binds to the tumor-supporting antigen associated with the disease, e.g. a tumor-supporting antigen as described herein.

In another aspect, the disclosure features a composition comprising an immune effector cell (e.g., a population of immune effector cells) comprising a CAR molecule (e.g., a CAR molecule as described herein) for use in treating a subject having a disease associated with expression of a tumor-supporting antigen, e.g., a disorder as described herein.

In any of the aforesaid methods or uses, the disease associated with expression of the tumor-supporting antigen is selected from the group consisting of a proliferative disease, a precancerous condition, a cancer, and a non-cancer related indication associated with expression of the tumor-supporting antigen. In an embodiment, the disease associated with a tumor-supporting antigen described herein is a solid tumor.

In one embodiment of the methods or uses described herein, the CAR molecule is administered in combination with another agent. In one embodiment, the agent can be a kinase inhibitor, e.g., a CDK4/6 inhibitor, a BTK inhibitor, an mTOR inhibitor, a MNK inhibitor, or a dual PI3K/mTOR inhibitor, and combinations thereof. In one embodiment, the kinase inhibitor is a CDK4 inhibitor, e.g., a CDK4 inhibitor described herein, e.g., a CD4/6 inhibitor, such as, e.g., 6-Acetyl-8-cyclopentyl-5-methyl-2-(5-piperazin-1-yl-pyridin-2-ylamino)-8*H*-pyrido[2,3-*d*]pyrimidin-7-one, hydrochloride (also referred to as palbociclib or PD0332991). In one embodiment, the kinase inhibitor is a BTK inhibitor, e.g., a BTK inhibitor described herein, such as, e.g., ibrutinib. In one embodiment, the kinase inhibitor is an mTOR inhibitor, e.g., an mTOR inhibitor described herein, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor described herein. In one embodiment, the kinase inhibitor is a MNK inhibitor, e.g., a MNK inhibitor described herein, such as, e.g., 4-amino-5-(4-fluoroanilino)-pyrazolo [3,4-*d*] pyrimidine. The MNK inhibitor can be, e.g., a MNK1a, MNK1b, MNK2a and/or MNK2b inhibitor. The dual PI3K/mTOR inhibitor can be, e.g., PF-04695102.

In one embodiment of the methods or uses described herein, the kinase inhibitor is a CDK4 inhibitor selected from aloisine A; flavopiridol or HMR-1275, 2-(2-chlorophenyl)-5,7-dihydroxy-8-[(3S,4R)-3-hydroxy-1-methyl-4-piperidinyl]-4-chromenone; crizotinib (PF-02341066; 2-(2-Chlorophenyl)-5,7-dihydroxy-8-[(2*R*,3*S*)-2-(hydroxymethyl)-1-methyl-3-pyrrolidinyl]- 4*H*-1-benzopyran-4-one, hydrochloride (P276-00); 1-methyl-5-[[2-[5-(trifluoromethyl)-1*H*-imidazol-2-yl]-4-pyridinyl]oxy]-*N*-[4-(trifluoromethyl)phenyl]-1*H*-benzimidazol-2-amine (RAF265); indisulam (E7070); roscovitine (CYC202); palbociclib (PD0332991); dinaciclib (SCH727965); N-[5-[[(5-*tert*-butyloxazol-2-yl)methyl]thio]thiazol-2-yl]piperidine-4-carboxamide (BMS 387032); 4-[[9-chloro-7-(2,6-difluorophenyl)-5*H*-pyrimido[5,4-*d*][2]benzazepin-2-yl]amino]-benzoic acid (MLN8054); 5-[3-(4,6-difluoro-1H-benzimidazol-2-yl)-1H-indazol-5-yl]-N-ethyl-4-methyl-3-pyridinemethanamine (AG-024322); 4-(2,6-dichlorobenzoylamino)-1H-pyrazole-3-carboxylic acid N-(piperidin-4-yl)amide (AT7519); 4-[2-methyl-1-(1-methylethyl)-1*H*-imidazol-5-yl]-*N*-[4-(methylsulfonyl)phenyl]- 2-pyrimidinamine (AZD5438); and XL281 (BMS908662).

In one embodiment of the methods or uses described herein, the kinase inhibitor is a CDK4 inhibitor, e.g., palbociclib (PD0332991), and the palbociclib is administered at a dose of about 50 mg, 60 mg, 70 mg, 75 mg, 80 mg, 90 mg, 100 mg, 105 mg, 110 mg, 115 mg, 120 mg, 125 mg, 130 mg, 135 mg (e.g., 75 mg, 100 mg or 125 mg) daily for a period of time, e.g., daily for 14-21 days of a 28 day cycle, or daily for 7-12 days of a 21 day cycle. In one embodiment, 1, 2, 3, 4, 5, 6, 7, 8, 9, 10, 11, 12 or more cycles of palbociclib are administered.

In one embodiment of the methods or uses described herein, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13. In one embodiment, the BTK inhibitor does not reduce or inhibit the kinase activity of interleukin-2-inducible kinase (ITK), and is selected from GDC-0834; RN-486; CGI-560; CGI-1764; HM-71224; CC-292; ONO-4059; CNX-774; and LFM-A13.

In one embodiment of the methods or uses described herein, the kinase inhibitor is a BTK inhibitor, e.g., ibrutinib (PCI-32765), and the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle, or daily for 28 day cycle. In one embodiment, 1, 2, 3, 4, 5, 6, 7, 8, 9, 10, 11, 12 or more cycles of ibrutinib are administered.

In one embodiment of the methods or uses described herein, the kinase inhibitor is a BTK inhibitor that does not inhibit the kinase activity of ITK, e.g., RN-486, and RN-486 is administered at a dose of about 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg (e.g., 150 mg, 200 mg or 250 mg) daily for a period of time, e.g., daily a 28 day cycle. In one embodiment, 1, 2, 3, 4, 5, 6, 7, or more cycles of RN-486 are administered.

In one embodiment of the methods or uses described herein, the kinase inhibitor is an mTOR inhibitor selected from temsirolimus; ridaforolimus (1*R*,2*R*,4*S*)-4-[(2*R*)-2 [(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669; everolimus (RAD001); rapamycin (AY22989); simapimod; (5-{2,4-bis[(3**S**)-3-methylmorpholin-4-yl]pyrido[2,3-**d**]pyrimidin-7-yl}-2-methoxyphenyl)methanol (AZD8055); 2-amino-8-[trans-4-(2-hydroxyethoxy)cyclohexyl]-6-(6-methoxy-3-pyridinyl)-4-methyl-pyrido[2,3-d]pyrimidin-7(8*H*)-one (PF04691502); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine-, inner salt (SF1126) (SEQ ID NO: 1937); and XL765.

In one embodiment of the methods or uses described herein, the kinase inhibitor is an mTOR inhibitor, e.g., rapamycin, and the rapamycin is administered at a dose of about 3 mg, 4 mg, 5 mg, 6 mg, 7 mg, 8 mg, 9 mg, 10 mg (e.g., 6 mg) daily for a period of time, e.g., daily for 21 day cycle, or daily for 28 day cycle. In one embodiment, 1, 2, 3, 4, 5, 6, 7, 8, 9, 10, 11, 12 or more cycles of rapamycin are administered. In one embodiment, the kinase inhibitor is an mTOR inhibitor, e.g., everolimus and the everolimus is administered at a dose of about 2 mg, 2.5 mg, 3 mg, 4 mg, 5 mg, 6 mg, 7 mg, 8 mg, 9 mg, 10 mg, 11 mg, 12 mg, 13 mg, 14 mg, 15 mg (e.g., 10 mg) daily for a period of time, e.g., daily for 28 day cycle. In one embodiment, 1, 2, 3, 4, 5, 6, 7, 8, 9, 10, 11, 12 or more cycles of everolimus are administered.

In one embodiment of the methods or uses described herein, the kinase inhibitor is an MNK inhibitor selected from CGP052088; 4-amino-3-(p-fluorophenylamino)-pyrazolo [3,4-*d*] pyrimidine (CGP57380); cercosporamide; ETC-1780445-2; and 4-amino-5-(4-fluoroanilino)-pyrazolo [3,4-d] pyrimidine.

In one embodiment of the methods or uses described herein, the kinase inhibitor is a dual phosphatidylinositol 3-kinase (PI3K) and mTOR inhibitor selected from 2-Amino-8-[trans-4-(2-hydroxyethoxy)cyclohexyl]-6-(6-methoxy-3-pyridinyl)-4-methyl-pyrido[2,3-d]pyrimidin-7(8H)-one (PF-04691502); N-[4-[[4-(Dimethylamino)-1-piperidinyl]carbonyl]phenyl]-N'-[4-(4,6-di-4-morpholinyl-1,3,5-triazin-2-yl)phenyl]urea (PF-05212384, PKI-587); 2-Methyl-2-{4-[3-methyl-2-oxo-8-(quinolin-3-yl)-2,3-dihydro-1*H*-imidazo[4,5-c]quinolin-1-yl]phenyl}propanenitrile (BEZ-235); apitolisib (GDC-0980, RG7422); 2,4-Difluoro-N-{2-(methyloxy)-5-[4-(4-pyridazinyl)-6-quinolinyl]-3-pyridinyl}benzenesulfonamide (GSK2126458); 8-(6-methoxypyridin-3-yl)-3-methyl-1-(4-(piperazin-1-yl)-3-(trifluoromethyl)phenyl)-1H-imidazo[4,5-c]quinolin-2(3H)-one Maleic acid (NVP-BGT226); 3-[4-(4-Morpholinylpyrido[3',2':4,5]furo[3,2-d]pyrimidin-2-yl]phenol (PI-103); 5-(9-isopropyl-8-methyl-2-morpholino-9H-purin-6-yl)pyrimidin-2-amine (VS-5584, SB2343); and N-[2-[(3,5-Dimethoxyphenyl)amino]quinoxalin-3-yl]-4-[(4-methyl-3-methoxyphenyl)carbonyl]aminophenylsulfonamide (XL765).

In one embodiment of the methods or uses described herein, a CAR expressing immune effector cell described herein is administered to a subject in combination with a protein tyrosine phosphatase inhibitor, e.g., a protein tyrosine phosphatase inhibitor described herein. In one embodiment, the protein tyrosine phosphatase inhibitor is an SHP-1 inhibitor, e.g., an SHP-1 inhibitor described herein, such as, e.g., sodium stibogluconate. In one embodiment, the protein tyrosine phosphatase inhibitor is an SHP-2 inhibitor.

In one embodiment of the methods or uses described herein, the CAR molecule is administered in combination with another agent, and the agent is a cytokine. The cytokine can be, e.g., IL-7, IL-15, IL-21, or a combination thereof. In another embodiment, the CAR molecule is administered in combination with a checkpoint inhibitor, e.g., a checkpoint inhibitor described herein. For example, in one embodiment, the check point inhibitor inhibits an inhibitory molecule selected from PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGF beta.

In one aspect, the CAR of the disclosure can be used to eradicate a normal cell that express a tumor antigen as described herein, thereby applicable for use as a cellular conditioning therapy prior to cell transplantation. In one aspect, the normal cell that expresses a tumor antigen as described herein is a normal stem cell and the cell transplantation is a stem cell transplantation.

### Therapeutic Applications

In another aspect, treating a subject, e.g., reducing or ameliorating, a hyperproliferative condition or disorder (e.g., a cancer), e.g., solid tumor, a soft tissue tumor, or a metastatic lesion, in a subject is provided. As used herein, the term "cancer" is meant to include all types of cancerous growths or oncogenic processes, metastatic tissues or malignantly transformed cells, tissues, or organs, irrespective of histopathologic type or stage of invasiveness. Examples of solid tumors include malignancies, e.g., sarcomas, adenocarcinomas, and carcinomas, of the various organ systems, such as those affecting liver, lung, breast, lymphoid, gastrointestinal (e.g., colon), genitourinary tract (e.g., renal, urothelial cells), prostate and pharynx. Adenocarcinomas include malignancies such as most colon cancers, rectal cancer, renal-cell carcinoma, liver cancer, non-small cell carcinoma of the lung, cancer of the small intestine and cancer of the esophagus. In one embodiment, the cancer is a melanoma, e.g., an advanced stage melanoma. Metastatic lesions of the aforementioned cancers can also be treated or prevented using the methods, treatments and compositions of the invention. Examples of other cancers that can be treated include bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin Disease, non-Hodgkin lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers. Treatment of metastatic cancers, e.g., metastatic cancers that express PD-L1 (Iwai et al. (2005) Int. Immunol. 17:133-144) can be effected using the antibody molecules described herein.

Exemplary cancers whose growth can be inhibited include cancers typically responsive to immunotherapy. Non-limiting examples of cancers for treatment include melanoma (e.g., metastatic malignant melanoma), renal cancer (e.g. clear cell carcinoma), prostate cancer (e.g. hormone refractory prostate adenocarcinoma), breast cancer, colon cancer and lung cancer (e.g. non-small cell lung cancer). Additionally, refractory or recurrent malignancies can be treated using the molecules described herein.

In one aspect, the disclosure pertains to a vector comprising a CAR operably linked to promoter for expression in mammalian immune effector cells (e.g., T cells, NK cells). In one aspect, the disclosure provides a recombinant immune effector cell expressing a CAR of the present disclosure for use in treating cancer expressing a cancer associate antigen as described herein. In one aspect, CAR-expressing cells of the disclosure is capable of contacting a tumor cell with at least one cancer associated antigen expressed on its surface such that the CAR-expressing cell targets the cancer cell and growth of the cancer is inhibited.

In one aspect, the disclosure pertains to inhibiting growth of a cancer, comprising contacting the cancer cell with a CAR-expressing cell of the present disclosure such that the CART is activated in response to the antigen and targets the cancer cell, wherein the growth of the tumor is inhibited.

In one aspect, the disclosure pertains to a method of treating cancer in a subject. The method comprises administering to the subject CAR-expressing cell of the present disclosure such that the cancer is treated in the subject. In one aspect, the cancer associated with expression of a cancer associate antigen as described herein is a hematological cancer. In one aspect, the hematological cancer is a leukemia or a lymphoma. In one aspect, a cancer associated with expression of a cancer associate antigen as described herein includes cancers and malignancies including, but not limited to, e.g., one or more acute leukemias including but not limited to, e.g., B-cell acute Lymphoid Leukemia ("BALL"), T-cell acute Lymphoid Leukemia ("TALL"), acute lymphoid leukemia (ALL); one or more chronic leukemias including but not limited to, e.g., chronic myelogenous leukemia (CML), Chronic Lymphoid Leukemia (CLL). Additional cancers or hematologic conditions associated with expression of a cancer associate antigen as described herein include, but are not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. Further a disease associated with a cancer associate antigen as described herein expression include, but not limited to, e.g., atypical and/or non-classical cancers, malignancies, precancerous conditions or proliferative diseases associated with expression of a cancer associate antigen as described herein.

In some embodiments, a cancer that can be treated with CAR-expressing cell of the present disclosure is multiple myeloma. Generally, myeloma cells are thought to be negative for a cancer associate antigen as described herein expression by flow cytometry. Thus, in some embodiments, a CD19 CAR, e.g., as described herein, may be used to target myeloma cells. In some embodiments, CARs of the present disclosure therapy can be used in combination with one or more additional therapies, e.g., lenalidomide treatment.

The disclosure includes a type of cellular therapy where immune effector cells (e.g., T cells, NK cells) are genetically modified to express a chimeric antigen receptor (CAR) and the CAR-expressing T cell or NK cell is infused to a recipient in need thereof. The infused cell is able to kill tumor cells in the recipient. Unlike antibody therapies, CAR-modified immune effector cells (e.g., T cells, NK cells) are able to replicate in vivo resulting in long-term persistence that can lead to sustained tumor control. In various aspects, the immune effector cells (e.g., T cells, NK cells) administered to the patient, or their progeny, persist in the patient for at least four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty-three months, two years, three years, four years, or five years after administration of the T cell or NK cell to the patient.

The disclosure also includes a type of cellular therapy where immune effector cells (e.g., T cells, NK cells) are modified, e.g., by in vitro transcribed RNA, to transiently express a chimeric antigen receptor (CAR) and the CAR T cell or NK cell is infused to a recipient in need thereof. The infused cell is able to kill tumor cells in the recipient. Thus, in various aspects, the immune effector cells (e.g., T cells, NK cells) administered to the patient, is present for less than one month, e.g., three weeks, two weeks, one week, after administration of the T cell or NK cell to the patient.

Without wishing to be bound by any particular theory, the anti-tumor immunity response elicited by the CAR-modified immune effector cells (e.g., T cells, NK cells) may be an active or a passive immune response, or alternatively may be due to a direct vs indirect immune response. In one aspect, the CAR transduced immune effector cells (e.g., T cells, NK cells) exhibit specific proinflammatory cytokine secretion and potent cytolytic activity in response to human cancer cells expressing the a cancer associate antigen as described herein, resist soluble a cancer associate antigen as described herein inhibition, mediate bystander killing and mediate regression of an established human tumor. For example, antigen-less tumor cells within a heterogeneous field of a cancer associate antigen as described herein-expressing tumor may be susceptible to indirect destruction by a cancer associate antigen as described herein-redirected immune effector cells (e.g., T cells, NK cells) that has previously reacted against adjacent antigen-positive cancer cells.

In one aspect, the fully-human CAR-modified immune effector cells (e.g., T cells, NK cells) of the disclosure may be a type of vaccine for ex vivo immunization and/or in vivo therapy in a mammal. In one aspect, the mammal is a human.

With respect to ex vivo immunization, at least one of the following occurs in vitro prior to administering the cell into a mammal: i) expansion of the cells, ii) introducing a nucleic acid encoding a CAR to the cells or iii) cryopreservation of the cells.

Ex vivo procedures are well known in the art and are discussed more fully below. Briefly, cells are isolated from a mammal (e.g., a human) and genetically modified (i.e., transduced or transfected in vitro) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

The procedure for ex vivo expansion of hematopoietic stem and progenitor cells is described in U.S. Pat. No. 5,199,942, can be applied to the cells of the present invention. Other suitable methods are known in the art, therefore the present invention is not limited to any particular method of ex vivo expansion of the cells. Briefly, ex vivo culture and expansion of immune effector cells (e.g., T cells, NK cells) comprises: (1) collecting CD34+ hematopoietic stem and progenitor cells from a mammal from peripheral blood harvest or bone marrow explants; and (2) expanding such cells ex vivo. In addition to the cellular growth factors described in U.S. Pat. No. 5,199,942, other factors such as flt3-L, IL-1, IL-3 and c-kit ligand, can be used for culturing and expansion of the cells.

In addition to using a cell-based vaccine in terms of ex vivo immunization, the present disclosure also provides compositions and methods for in vivo immunization to elicit an immune response directed against an antigen in a patient.

Generally, the cells activated and expanded as described herein may be utilized in the treatment and prevention of diseases that arise in individuals who are immunocompromised. In particular, the CAR-modified immune effector cells (e.g., T cells, NK cells) of the disclosure are used in the treatment of diseases, disorders and conditions associated with expression of a cancer associate antigen as described herein. In certain aspects, the cells of the disclosure are used in the treatment of patients at risk for developing diseases, disorders and conditions associated with expression of a cancer associate antigen as described herein. Thus, the present disclosure provides for the treatment or prevention of diseases, disorders and conditions associated with expression of a cancer associate antigen as described herein comprising administering to a subject in need thereof, a therapeutically effective amount of the CAR-modified immune effector cells (e.g., T cells, NK cells) of the disclosure.

In the invention, the population of immune effector cells engineered to express a CAR is used in combination with an LSD1 inhibitor.

In one aspect the CAR-expressing cells of the disclosure may be used to treat a proliferative disease such as a cancer or malignancy or is a precancerous condition such as a myelodysplasia, a myelodysplastic syndrome or a preleukemia. Further, a disease associated with a cancer associate antigen as described herein expression include, but not limited to, e.g., atypical and/or non-classical cancers, malignancies, precancerous conditions or proliferative diseases expressing a cancer associated antigen as described herein. Non-cancer related indications associated with expression of a cancer associate antigen as described herein include, but are not limited to, e.g., autoimmune disease, (e.g., lupus), inflammatory disorders (allergy and asthma) and transplantation.

The CAR-modified immune effector cells (e.g., T cells, NK cells) of the present invention may be administered either alone, or as a pharmaceutical composition in combination with diluents and/or with other components such as IL-2 or other cytokines or cell populations.

### Hematologic Cancer

Hematological cancer conditions are the types of cancer such as leukemia, lymphoma, and malignant lymphoproliferative conditions that affect blood, bone marrow and the lymphatic system.

Leukemia can be classified as acute leukemia and chronic leukemia. Acute leukemia can be further classified as acute myelogenous leukemia (AML) and acute lymphoid leukemia (ALL). Chronic leukemia includes chronic myelogenous leukemia (CML) and chronic lymphoid leukemia (CLL). Other related conditions include myelodysplastic syndromes (MDS, formerly known as "preleukemia") which are a diverse collection of hematological conditions united by ineffective production (or dysplasia) of myeloid blood cells and risk of transformation to AML.

Lymphoma is a group of blood cell tumors that develop from lymphocytes. Exemplary lymphomas include non-Hodgkin lymphoma and Hodgkin lymphoma.

The present disclosure also provides for inhibiting the proliferation or reducing a cancer associated antigen as described herein-expressing cell population, the methods comprising contacting a population of cells comprising a cancer associated antigen as described herein-expressing cell with a CAR-expressing T cell or NK cell of the disclosure that binds to the a cancer associate antigen as described herein-expressing cell. In a specific aspect, the present disclosure provides for inhibiting the proliferation or reducing the population of cancer cells expressing a cancer associated antigen as described herein, the methods comprising contacting a cancer associate antigen as described herein-expressing cancer cell population with a CAR-expressing T cell or NK cell of the disclosure that binds to a cancer associated antigen as described herein-expressing cell. In one aspect, the present disclosure provides for inhibiting the proliferation or reducing the population of cancer cells expressing a cancer associated antigen as described herein, the methods comprising contacting a cancer associated antigen as described herein-expressing cancer cell population with a CAR-expressing T cell or NK cell of the disclosure that binds to a cancer associated antigen as described herein-expressing cell. In certain aspects, a CAR-expressing T cell or NK cell of the disclosure reduces the quantity, number, amount or percentage of cells and/or cancer cells by at least 25%, at least 30%, at least 40%, at least 50%, at least 65%, at least 75%, at least 85%, at least 95%, or at least 99% in a subject with or animal model for myeloid leukemia or another cancer associated with a cancer associated antigen as described herein-expressing cells relative to a negative control. In one aspect, the subject is a human.

The present disclosure also provides for preventing, treating and/or managing a disease associated with a cancer associated antigen as described herein-expressing cells (e.g., a hematologic cancer or atypical cancer expessing a cancer associated antigen as described herein), the methods comprising administering to a subject in need a CAR T cell or NK cell of the disclosure that binds to a cancer associated antigen as described herein-expressing cell. In one aspect, the subject is a human. Non-limiting examples of disorders associated with a cancer associated antigen as described herein-expressing cells include autoimmune disorders (such as lupus), inflammatory disorders (such as allergies and asthma) and cancers (such as hematological cancers or atypical cancers expessing a cancer associated antigen as described herein).

The present disclosure also provides for preventing, treating and/or managing a disease associated with a cancer associated antigen as described herein-expressing cells, the methods comprising administering to a subject in need a CAR T cell or NK cell of the disclosure that binds to a cancer associated antigen as described herein-expressing cell. In one aspect, the subject is a human.

The present disclosure provides for preventing relapse of cancer associated with a cancer associated antigen as described herein-expressing cells, the methods comprising administering to a subject in need thereof aCAR T cell or NK cell of the disclosure that binds to a cancer associated antigen as described herein-expressing cell. In one aspect, the methods comprise administering to the subject in need thereof an effective amount of a CAR-expressingT cell or NK cell described herein that binds to a cancer associated antigen as described herein-expressing cell in combination with an effective amount of another therapy.

### Pharmaceutical compositions and treatments: Combinations of LSD1 inhibitors and CAR-expressing cells

Pharmaceutical compositions of the present disclosure may comprise a CAR-expressing cell, e.g., a plurality of CAR-expressing cells, as described herein, in combination with one or more pharmaceutically or physiologically acceptable carriers, diluents or excipients. The pharmaceutical compositions may additionally comprise one or more, e.g., one LSD1 inhibitor. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are in one aspect formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

In one embodiment, the pharmaceutical composition is substantially free of, e.g., there are no detectable levels of a contaminant, e.g., selected from the group consisting of endotoxin, mycoplasma, replication competent lentivirus (RCL), p24, VSV-G nucleic acid, HIV gag, residual anti-CD3/anti-CD28 coated beads, mouse antibodies, pooled human serum, bovine serum albumin, bovine serum, culture media components, vector packaging cell or plasmid components, a bacterium and a fungus. In one embodiment, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

When "an immunologically effective amount," "an anti-tumor effective amount," "a tumor-inhibiting effective amount," or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the immune effector cells (e.g., T cells, NK cells) described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988).

In certain aspects, it may be desired to administer activated immune effector cells (e.g., T cells, NK cells) to a subject and then subsequently redraw blood (or have an apheresis performed), activate immune effector cells (e.g., T cells, NK cells) therefrom according to the present invention, and reinfuse the patient with these activated and expanded immune effector cells (e.g., T cells, NK cells). This process can be carried out multiple times every few weeks. In certain aspects, immune effector cells (e.g., T cells, NK cells) can be activated from blood draws of from 10cc to 400cc. In certain aspects, immune effector cells (e.g., T cells, NK cells) are activated from blood draws of 20cc, 30cc, 40cc, 50cc, 60cc, 70cc, 80cc, 90cc, or 100cc.

The administration of the subject compositions may be carried out in any convenient manner, including by aerosol inhalation, injection, ingestion, transfusion, implantation or transplantation. The compositions described herein may be administered to a patient trans arterially, subcutaneously, intradermally, intratumorally, intranodally, intramedullary, intramuscularly, by intravenous (i.v.) injection, or intraperitoneally. In one aspect, the T cell compositions of the present invention are administered to a patient by intradermal or subcutaneous injection. In one aspect, the T cell compositions of the present invention are administered by i.v. injection. The compositions of immune effector cells (e.g., T cells, NK cells) may be injected directly into a tumor, lymph node, or site of infection.

In a particular exemplary aspect, subjects may undergo leukapheresis, wherein leukocytes are collected, enriched, or depleted ex vivo to select and/or isolate the cells of interest, e.g., T cells. These T cell isolates may be expanded by methods known in the art and treated such that one or more CAR constructs of the disclosure may be introduced, thereby creating a CAR T cell of the disclosure. Subjects in need thereof may subsequently undergo standard treatment with high dose chemotherapy followed by peripheral blood stem cell transplantation. In certain aspects, following or concurrent with the transplant, subjects receive an infusion of the expanded CAR T cells of the present disclosure. In an additional aspect, expanded cells are administered before or following surgery.

The dosage of the above treatments to be administered to a patient will vary with the precise nature of the condition being treated and the recipient of the treatment. The scaling of dosages for human administration can be performed according to art-accepted practices. The dose for CAM PATH, for example, will generally be in the range 1 to about 100 mg for an adult patient, usually administered daily for a period between 1 and 30 days. The preferred daily dose is 1 to 10 mg per day although in some instances larger doses of up to 40 mg per day may be used (described in U.S. Patent No. 6,120,766).

In one embodiment, the CAR is introduced into immune effector cells (e.g., T cells, NK cells), e.g., using in vitro transcription, and the subject (e.g., human) receives an initial administration of CAR immune effector cells (e.g., T cells, NK cells) of the disclosure, and one or more subsequent administrations of the CAR immune effector cells (e.g., T cells, NK cells) of the disclosure, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of the CAR immune effector cells (e.g., T cells, NK cells) of the disclosure are administered to the subject (e.g., human) per week, e.g., 2, 3, or 4 administrations of the CAR immune effector cells (e.g., T cells, NK cells) of the disclosure are administered per week. In one embodiment, the subject (e.g., human subject) receives more than one administration of the CAR immune effector cells (e.g., T cells, NK cells) per week (e.g., 2, 3 or 4 administrations per week) (also referred to herein as a cycle), followed by a week of no CAR immune effector cells (e.g., T cells, NK cells) administrations, and then one or more additional administration of the CAR immune effector cells (e.g., T cells, NK cells) (e.g., more than one administration of the CAR immune effector cells (e.g., T cells, NK cells) per week) is administered to the subject. In another embodiment, the subject (e.g., human subject) receives more than one cycle of CAR immune effector cells (e.g., T cells, NK cells), and the time between each cycle is less than 10, 9, 8, 7, 6, 5, 4, or 3 days. In one embodiment, the CAR immune effector cells (e.g., T cells, NK cells) are administered every other day for 3 administrations per week. In one embodiment, the CAR immune effector cells (e.g., T cells, NK cells) of the invention are administered for at least two, three, four, five, six, seven, eight or more weeks.

In one aspect, CAR-expressing cells of the present inventions are generated using lentiviral viral vectors, such as lentivirus. Cells, e.g., CARTs, generated that way will have stable CAR expression.

In one aspect, CAR-expressing cells, e.g., CARTs, are generated using a viral vector such as a gammaretroviral vector, e.g., a gammaretroviral vector described herein. CARTs generated using these vectors can have stable CAR expression.

In one aspect, CARTs transiently express CAR vectors for 4, 5, 6, 7, 8, 9, 10, 11, 12, 13, 14, 15 days after transduction. Transient expression of CARs can be effected by RNA CAR vector delivery. In one aspect, the CAR RNA is transduced into the T cell by electroporation.

A potential issue that can arise in patients being treated using transiently expressing CAR immune effector cells (e.g., T cells, NK cells) (particularly with murine scFv bearing CARTs) is anaphylaxis after multiple treatments.

Without being bound by this theory, it is believed that such an anaphylactic response might be caused by a patient developing humoral anti-CAR response, i.e., anti-CAR antibodies having an anti-IgE isotype. It is thought that a patient's antibody producing cells undergo a class switch from IgG isotype (that does not cause anaphylaxis) to IgE isotype when there is a ten to fourteen day break in exposure to antigen.

If a patient is at high risk of generating an anti-CAR antibody response during the course of transient CAR therapy (such as those generated by RNA transductions), CART infusion breaks should not last more than ten to fourteen days.

### Methods of making CAR-expressing cells

In another aspect, the disclosure pertains to a method of making a cell (e.g., an immune effector cell or population thereof) comprising introducing into (e.g., transducing) a cell, e.g., a T cell or a NK cell described herein, with a vector of comprising a nucleic acid encoding a CAR molecule, e.g., a CAR molecule described herein; or a nucleic acid encoding a CAR molecule e.g., a CAR described herein.

The cell in the disclosed methods is an immune effector cell (e.g., a T cell or a NK cell, or a combination thereof). In the methods of the invention, the population of immune effector cells comprises, e.g., consists of, T cells. In some embodiments, the cell in the methods is diaglycerol kinase (DGK) and/or Ikaros deficient.

In some embodiment, the introducing the nucleic acid molecule encoding a CAR molecule comprises transducing a vector comprising the nucleic acid molecule encoding a CAR molecule, or transfecting the nucleic acid molecule encoding a CAR molecule, wherein the nucleic acid molecule is an in vitro transcribed RNA.

In some embodiments, the method further comprises:
providing a population of immune effector cells (e.g., T cells or NK cells); and
removing T regulatory cells from the population, thereby providing a population of T regulatory-depleted cells;
wherein steps a) and b) are performed prior to introducing the nucleic acid encoding the CAR molecule to the population.

In embodiments of the methods, the T regulatory cells comprise CD25+ T cells, and are removed from the cell population using an anti-CD25 antibody, or fragment thereof. The anti-CD25 antibody, or fragment thereof, can be conjugated to a substrate, e.g., a bead.

In other embodiments, the population of T regulatory-depleted cells provided from step (b) contains less than 30%, 25%, 20%, 15%, 10%, 5%, 4%, 3%, 2%, 1% of CD25+ cells.

In yet other embodiments, the method further comprises removing cells from the population which express a tumor antigen that does not comprise CD25 to provide a population of T regulatory-depleted and tumor antigen depleted cells prior to introducing the nucleic acid encoding a CAR molecule to the population. The tumor antigen can be selected from CD19, CD30, CD38, CD123, CD20, CD14 or CD11b, or a combination thereof.

In other embodiments, the method further comprises removing cells from the population which express a checkpoint inhibitor, to provide a population of T regulatory-depleted and inhibitory molecule depleted cells prior to introducing the nucleic acid encoding a CAR molecule to the population. The checkpoint inhibitor can be chosen from PD-1, LAG-3, TIM3, B7-H1, CD160, P1H, 2B4, CEACAM (e.g., CEACAM-1, CEACAM-3, and/or CEACAM-5), TIGIT, CTLA-4, BTLA, and LAIR1.

Further embodiments disclosed herein encompass providing a population of immune effector cells. The population of immune effector cells provided can be selected based upon the expression of one or more of CD3, CD28, CD4, CD8, CD45RA, and/or CD45RO. In certain embodiments, the population of immune effector cells provided are CD3+ and/or CD28+.

In certain embodiments of the method, the method further comprises expanding the population of cells after the nucleic acid molecule encoding a CAR molecule has been introduced.

In embodiments, the population of cells is expanded for a period of 8 days or less.

In certain embodiments, the population of cells is expanded in culture for 5 days, and the resulting cells are more potent than the same cells expanded in culture for 9 days under the same culture conditions.

In other embodiments, the population of cells is expanded in culture for 5 days show at least a one, two, three or four fold increase in cell doublings upon antigen stimulation as compared to the same cells expanded in culture for 9 days under the same culture conditions.

In yet other embodiments, the population of cells is expanded in culture for 5 days, and the resulting cells exhibit higher proinflammatory IFN-y and/or GM-CSF levels, as compared to the same cells expanded in culture for 9 days under the same culture conditions.

In other embodiments, the population of cells is expanded by culturing the cells in the presence of an agent that stimulates a CD3/TCR complex associated signal and/or a ligand that stimulates a costimulatory molecule on the surface of the cells. The agent can be a bead conjugated with anti-CD3 antibody, or a fragment thereof, and/or anti-CD28 antibody, or a fragment thereof.

In other embodiments, the population of cells is expanded in an appropriate media that includes one or more interleukin that result in at least a 200-fold, 250-fold, 300-fold, or 350-fold increase in cells over a 14 day expansion period, as measured by flow cytometry.

In other embodiments, the population of cells is expanded in the presence IL-15 and/or IL-7.

In certain embodiments, the method further includes cryopreserving he population of the cells after the appropriate expansion period.

In yet other embodiments, the method of making disclosed herein further comprises contacting the population of immune effector cells with a nucleic acid encoding a telomerase subunit, e.g., hTERT. The nucleic acid encoding the telomerase subunit can be DNA.

The present disclosure also provides a method of generating a population of RNA-engineered cells, e.g., cells described herein, e.g., immune effector cells (e.g., T cells, NK cells), transiently expressing exogenous RNA. The method comprises introducing an *in vitro* transcribed RNA or synthetic RNA into a cell, where the RNA comprises a nucleic acid encoding a CAR molecule described herein.

In another aspect, the disclosure pertains to providing an anti-tumor immunity in a subject comprising administering to the subject an effective amount of a cell comprising a CAR molecule, e.g., a cell expressing a CAR molecule described herein. In one instance, the cell is an autologous T cell or NK cell. In one instance, the cell is an allogeneic T cell or NK cell. In one instance, the subject is a human.

In one aspect, the disclosure includes a population of autologous cells that are transfected or transduced with a vector comprising a nucleic acid molecule encoding a CAR molecule, e.g., as described herein. In one instance, the vector is a retroviral vector. In one instance, the vector is a self-inactivating lentiviral vector as described elsewhere herein. In one instance, the vector is delivered (e.g., by transfecting or electroporating) to a cell, e.g., a T cell or a NK cell, wherein the vector comprises a nucleic acid molecule encoding a CAR of the present disclosure as described herein, which is transcribed as an mRNA molecule, and the CARs of the present disclosure is translated from the RNA molecule and expressed on the surface of the cell.

In another aspect, the present disclosure provides a population of CAR-expressing cells, e.g., CAR-expressing immune effector cells (e.g., T cells or NK cells). In some instances, the population of CAR-expressing cells comprises a mixture of cells expressing different CARs. For example, in one instance, the population of CAR-expressing immune effector cells (e.g., T cells or NK cells) can include a first cell expressing a CAR having an antigen binding domain that binds to a first tumor antigen as described herein, and a second cell expressing a CAR having a different antigen binding domain that binds to a second tumor antigen as described herein. As another example, the population of CAR-expressing cells can include a first cell expressing a CAR that includes an antigen binding domain that binds to a tumor antigen as described herein, and a second cell expressing a CAR that includes an antigen binding domain to a target other than a tumor antigen as described herein. In one embodiment, the population of CAR-expressing cells includes, e.g., a first cell expressing a CAR that includes a primary intracellular signaling domain, and a second cell expressing a CAR that includes a secondary signaling domain, e.g., a costimulatory signaling domain.

In another aspect, the present disclosure provides a population of cells wherein at least one cell in the population expresses a CAR having an antigen binding domain that binds to a tumor antigen as described herein, and a second cell expressing another agent, e.g., an agent which enhances the activity of a CAR-expressing cell. For example, in one instance, the agent can be an agent which inhibits an inhibitory molecule. Examples of inhibitory molecules include PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGF beta. In one instance, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one instance, the agent comprises a first polypeptide, e.g., of an inhibitory molecule such as PD-1, LAG-3, CTLA-4, CD160, BTLA, LAIR1, TIM-3, CEACAM (e.g., CEACAM-1, CEACAM-3 and/or CEACAM-5), 2B4 and TIGIT, or a fragment of any of these, and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one instance, the agent comprises a first polypeptide of PD-1 or a fragment thereof, and a second polypeptide of an intracellular signaling domain described herein (e.g., a CD28, CD27, OX40 or 4-IBB signaling domain described herein and/or a CD3 zeta signaling domain described herein).

In one instance, the nucleic acid molecule encoding a CAR of the present disclosure, e.g., as described herein, is expressed as an mRNA molecule. In one instance, the genetically modified CAR of the present disclosure-expressing cells, e.g., immune effector cells (e.g., T cells, NK cells), can be generated by transfecting or electroporating an RNA molecule encoding the desired CARs (e.g., without a vector sequence) into the cell. In one instance, a CAR of the present disclosure is translated from the RNA molecule once it is incorporated and expressed on the surface of the recombinant cell.

A method for generating mRNA for use in transfection involves *in vitro* transcription (IVT) of a template with specially designed primers, followed by polyA addition, to produce a construct containing 3' and 5' untranslated sequence ("UTR") (e.g., a 3' and/or 5' UTR described herein), a 5' cap (e.g., a 5' cap described herein) and/or Internal Ribosome Entry Site (IRES) (e.g., an IRES described herein), the nucleic acid to be expressed, and a polyA tail, typically 50-2000 bases in length (SEQ ID NO:32). RNA so produced can efficiently transfect different kinds of cells. In one instance, the template includes sequences for the CAR. In an embodiment, an RNA CAR vector is transduced into a cell, e.g., a T cell or a NK cell, by electroporation.

**Table 18: Sequences of various components of CAR (aa - amino acids, na - nucleic acids that encodes the corresponding protein)**

| SEQ ID NO | description | Sequence |
|---|---|---|
| 1 | EF-1 promoter | CGTGAGGCTCCGGTGCCCGTCAGTGGGCAGAGCGCACATCGCCCAC AGTCCCCGAGAAGTTGGGGGGAGGGGTCGGCAATTGAACCGGTGC CTAGAGAAGGTGGCGCGGGGTAAACTGGGAAAGTGATGTCGTGTA CTGGCTCCGCCTTTTTCCCGAGGGTGGGGGAGAACCGTATATAAGT GCAGTAGTCGCCGTGAACGTTCTTTTTCGCAACGGGTTTGCCGCCAG |
| | | AACACAGGTAAGTGCCGTGTGTGGTTCCCGCGGGCCTGGCCTCTTTA CGGGTTATGGCCCTTGCGTGCCTTGAATTACTTCCACCTGGCTGCAG TACGTGATTCTTGATCCCGAGCTTCGGGTTGGAAGTGGGTGGGAGA GTTCGAGGCCTTGCGCTTAAGGAGCCCCTTCGCCTCGTGCTTGAGTT GAGGCCTGGCCTGGGCGCTGGGGCCGCCGCGTGCGAATCTGGTGG CACCTTCGCGCCTGTCTCGCTGCTTTCGATAAGTCTCTAGCCATTTAA AATTTTTGATGACCTGCTGCGACGCTTTTTTTCTGGCAAGATAGTCTT GTAAATGCGGGCCAAGATCTGCACACTGGTATTTCGGTTTTTGGGGC CGCGGGCGGCGACGGGGCCCGTGCGTCCCAGCGCACATGTTCGGC GAGGCGGGGCCTGCGAGCGCGGCCACCGAGAATCGGACGGGGGTA GTCTCAAGCTGGCCGGCCTGCTCTGGTGCCTGGCCTCGCGCCGCCGT GTATCGCCCCGCCCTGGGCGGCAAGGCTGGCCCGGTCGGCACCAGT TGCGTGAGCGGAAAGATGGCCGCTTCCCGGCCCTGCTGCAGGGAGC TCAAAATGGAGGACGCGGCGCTCGGGAGAGCGGGCGGGTGAGTCA CCCACACAAAGGAAAAGGGCCTTTCCGTCCTCAGCCGTCGCTTCATG TGACTCCACGGAGTACCGGGCGCCGTCCAGGCACCTCGATTAGTTCT CGAGCTTTTGGAGTACGTCGTCTTTAGGTTGGGGGGAGGGGTTTTAT GCGATGGAGTTTCCCCACACTGAGTGGGTGGAGACTGAAGTTAGGC CAGCTTGGCACTTGATGTAATTCTCCTTGGAATTTGCCCTTTTTGAGT TTGGATCTTGGTTCATTCTCAAGCCTCAGACAGTGGTTCAAAGTTTTT TTCTTCCATTTCAGGTGTCGTGA |
| 2 | Leader (aa) | MALPVTALLLPLALLLHAARP |
| 3 | Leader (na) | ATGGCCCTGCCTGTGACAGCCCTGCTGCTGCCTCTGGCTCTGCTGCT GCATGCCGCTAGACCC |
| 1938 | Leader (na) | ATGGCCCTCCCTGTCACCGCCCTGCTGCTTCCGCTGGCTCTTCTGCTC CACGCCGCTCGGCCC |
| 4 | CD 8 hinge (aa) | TTTPAPRPPTPAPTIASQPLSLRPEACRPAAGGAVHTRGLDFACD |
| 5 | CD8 hinge (na) | ACCACGACGCCAGCGCCGCGACCACCAACACCGGCGCCCACCATCG CGTCGCAGCCCCTGTCCCTGCGCCCAGAGGCGTGCCGGCCAGCGGC GGGGGGCGCAGTGCACACGAGGGGGCTGGACTTCGCCTGTGAT |
| 6 | Ig4 hinge (aa) | ESKYGPPCPPCPAPEFLGGPSVFLFPPKPKDTLMISRTPEVTCVVVDVSQ EDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDKS RWQEGNVFSCSVMHEALHNHYTQKSLSLSLGKM |
| 7 | Ig4 hinge (na) | GAGAGCAAGTACGGCCCTCCCTGCCCCCCTTGCCCTGCCCCCGAGTT CCTGGGCGGACCCAGCGTGTTCCTGTTCCCCCCCAAGCCCAAGGACA CCCTGATGATCAGCCGGACCCCCGAGGTGACCTGTGTGGTGGTGGA CGTGTCCCAGGAGGACCCCGAGGTCCAGTTCAACTGGTACGTGGAC GGCGTGGAGGTGCACAACGCCAAGACCAAGCCCCGGGAGGAGCAG TTCAATAGCACCTACCGGGTGGTGTCCGTGCTGACCGTGCTGCACCA GGACTGGCTGAACGGCAAGGAATACAAGTGTAAGGTGTCCAACAAG GGCCTGCCCAGCAGCATCGAGAAAACCATCAGCAAGGCCAAGGGCC AGCCTCGGGAGCCCCAGGTGTACACCCTGCCCCCTAGCCAAGAGGA GATGACCAAGAACCAGGTGTCCCTGACCTGCCTGGTGAAGGGCTTCT ACCCCAGCGACATCGCCGTGGAGTGGGAGAGCAACGGCCAGCCCG |
| | | AGAACAACTACAAGACCACCCCCCCTGTGCTGGACAGCGACGGCAG CTTCTTCCTGTACAGCCGGCTGACCGTGGACAAGAGCCGGTGGCAG GAGGGCAACGTCTTTAGCTGCTCCGTGATGCACGAGGCCCTGCACA ACCACTACACCCAGAAGAGCCTGAGCCTGTCCCTGGGCAAGATG |
| 8 | IgD hinge (aa) | RWPESPKAQASSVPTAQPQAEGSLAKATTAPATTRNTGRGGEEKKKEK EKEEQEERETKTPECPSHTQPLGVYLLTPAVQDLWLRDKATFTCFVVGS DLKDAHLTWEVAGKVPTGGVEEGLLERHSNGSQSQHSRLTLPRSLWN AGTSVTCTLNHPSLPPQRLMALREPAAQAPVKLSLNLLASSDPPEAASW LLCEVSGFSPPNILLMWLEDQREVNTSGFAPARPPPQPGSTTFWAWSV LRVPAPPSPQPATYTCVVSHEDSRTLLNASRSLEVSYVTDH |
| 9 | IgD hinge (na) | AGGTGGCCCGAAAGTCCCAAGGCCCAGGCATCTAGTGTTCCTACTGC ACAGCCCCAGGCAGAAGGCAGCCTAGCCAAAGCTACTACTGCACCT GCCACTACGCGCAATACTGGCCGTGGCGGGGAGGAGAAGAAAAAG GAGAAAGAGAAAGAAGAACAGGAAGAGAGGGAGACCAAGACCCCT GAATGTCCATCCCATACCCAGCCGCTGGGCGTCTATCTCTTGACTCCC GCAGTACAGGACTTGTGGCTTAGAGATAAGGCCACCTTTACATGTTT CGTCGTGGGCTCTGACCTGAAGGATGCCCATTTGACTTGGGAGGTT GCCGGAAAGGTACCCACAGGGGGGGTTGAGGAAGGGTTGCTGGAG CGCCATTCCAATGGCTCTCAGAGCCAGCACTCAAGACTCACCCTTCC GAGATCCCTGTGGAACGCCGGGACCTCTGTCACATGTACTCTAAATC ATCCTAGCCTGCCCCCACAGCGTCTGATGGCCCTTAGAGAGCCAGCC GCCCAGGCACCAGTTAAGCTTAGCCTGAATCTGCTCGCCAGTAGTGA TCCCCCAGAGGCCGCCAGCTGGCTCTTATGCGAAGTGTCCGGCTTTA GCCCGCCCAACATCTTGCTCATGTGGCTGGAGGACCAGCGAGAAGT GAACACCAGCGGCTTCGCTCCAGCCCGGCCCCCACCCCAGCCGGGTT CTACCACATTCTGGGCCTGGAGTGTCTTAAGGGTCCCAGCACCACCT AGCCCCCAGCCAGCCACATACACCTGTGTTGTGTCCCATGAAGATAG CAGGACCCTGCTAAATGCTTCTAGGAGTCTGGAGGTTTCCTACGTGA CTGACCATT |
| 10 | GS hinge/linker (aa) | GGGGSGGGGS |
| 11 | GS hinge/linker (na) | GGTGGCGGAGGTTCTGGAGGTGGAGGTTCC |
| 12 | CD8 TM (aa) | IYIWAPLAGTCGVLLLSLVITLYC |
| 13 | CD8 TM (na) | ATCTACATCTGGGCGCCCTTGGCCGGGACTTGTGGGGTCCTTCTCCT GTCACTGGTTATCACCCTTTACTGC |
| 1939 | CD8 TM (na) | ATCTACATTTGGGCCCCTCTGGCTGGTACTTGCGGGGTCCTGCTGCTT TCACTCGTGATCACTCTTTACTGT |
| 14 | 4-1BB intracellular domain (aa) | KRGRKKLLYIFKQPFM RPVQTTQEEDGCSCRFPEEEEGGCEL |
| 15 | 4-1BB intracellular domain (na) | AAACGGGGCAGAAAGAAACTCCTGTATATATTCAAACAACCATTTAT GAGACCAGTACAAACTACTCAAGAGGAAGATGGCTGTAGCTGCCGA TTTCCAGAAGAAGAAGAAGGAGGATGTGAACTG |
| 1940 | 4-1BB) | AAGCGCGGTCGGAAGAAGCTGCTGTACATCTTTAAGCAACCCTTCAT |
| | intracellular domain (na | GAGGCCTGTGCAGACTACTCAAGAGGAGGACGGCTGTTCATGCCGG TTCCCAGAGGAGGAGGAAGGCGGCTGCGAACTG |
| 16 | CD27 (aa) | QRRKYRSNKGESPVEPAEPCRYSCPREEEGSTIPIQEDYRKPEPACSP |
| 17 | CD27 (na) | AGGAGTAAGAGGAGCAGGCTCCTGCACAGTGACTACATGAACATGA CTCCCCGCCGCCCCGGGCCCACCCGCAAGCATTACCAGCCCTATGCC CCACCACGCGACTTCGCAGCCTATCGCTCC |
| 18 | CD3-zeta (aa) | RVKFSRSADAPAYKQGQNQLYNELNLGRREEYDVLDKRRGRDPEMGG KPRRKNPQEGLYNELQKDKMAEAYSEIGMKGERRRGKGHDGLYQGLS TATKDTYDALHMQALPPR |
| 19 | CD3-zeta (na) | AGAGTGAAGTTCAGCAGGAGCGCAGACGCCCCCGCGTACAAGCAG GGCCAGAACCAGCTCTATAACGAGCTCAATCTAGGACGAAGAGAGG AGTACGATGTTTTGGACAAGAGACGTGGCCGGGACCCTGAGATGGG GGGAAAGCCGAGAAGGAAGAACCCTCAGGAAGGCCTGTACAATGA ACTGCAGAAAGATAAGATGGCGGAGGCCTACAGTGAGATTGGGAT GAAAGGCGAGCGCCGGAGGGGCAAGGGGCACGATGGCCTTTACCA GGGTCTCAGTACAGCCACCAAGGACACCTACGACGCCCTTCACATGC AGGCCCTGCCCCCTCGC |
| 1941 | CD3-zeta (na) | CGCGTGAAATTCAGCCGCAGCGCAGATGCTCCAGCCTACAAGCAGG GGCAGAACCAGCTCTACAACGAACTCAATCTTGGTCGGAGAGAGGA GTACGACGTGCTGGACAAGCGGAGAGGACGGGACCCAGAAATGGG CGGGAAGCCGCGCAGAAAGAATCCCCAAGAGGGCCTGTACAACGA GCTCCAAAAGGATAAGATGGCAGAAGCCTATAGCGAGATTGGTATG AAAGGGGAACGCAGAAGAGGCAAAGGCCACGACGGACTGTACCAG GGACTCAGCACCGCCACCAAGGACACCTATGACGCTCTTCACATGCA GGCCCTGCCGCCTCGG |
| 20 | CD3-zeta (aa) | RVKFSRSADAPAYQQGQNQLYNELNLGRREEYDVLDKRRGRDPEMGG KPRRKNPQEGLYNELQKDKMAEAYSEIGMKGERRRGKGHDGLYQGLS TATKDTYDALHMQALPPR |
| 21 | CD3-zeta (na) | AGAGTGAAGTTCAGCAGGAGCGCAGACGCCCCCGCGTACCAGCAG GGCCAG AACCAGCTCTATAACGAGCTCAATCTAGGACGAAGAGAGGAGTACG ATGTTT TGGACAAGAGACGTGGCCGGGACCCTGAGATGGGGGGAAAGCCGA GAAGGA AGAACCCTCAGGAAGGCCTGTACAATGAACTGCAGAAAGATAAGAT GGCGG AGGCCTACAGTGAGATTGGGATGAAAGGCGAGCGCCGGAGGGGCA AGGGGC ACGATGGCCTTTACCAGGGTCTCAGTACAGCCACCAAGGACACCTAC GACGC CCTTCACATGCAGGCCCTGCCCCCTCGC |
| 22 | linker | GGGGS |
| 23 | linker | GGTGGCGGAGGTTCTGGAGGTGGAGGTTCC |
| 24 | PD-1 extracellular domain (aa) | |
| 25 | PD-1 | Cccggatggtttctggactctccggatcgcccgtggaatcccccaaccttctcaccggcact |
| | extracellular domain (na) | cttggttgtgactgagggcgataatgcgaccttcacgtgctcgttctccaacacctccgaat cattcgtgctgaactggtaccgcatgagcccgtcaaaccagaccgacaagctcgccgcgtt tccggaagatcggtcgcaaccgggacaggattgtcggttccgcgtgactcaactgccgaat ggcagagacttccacatgagcgtggtccgcgctaggcgaaacgactccgggacctacctg tgcggagccatctcgctggcgcctaaggcccaaatcaaagagagcttgagggccgaactg agagtgaccgagcgcagagctgaggtgccaactgcacatccatccccatcgcctcggcct gcggggcagtttcagaccctggtc |
| 26 | PD-1 CAR (aa) with signal | Malpvtalllplalllhaarppgwfldspdrpwnpptfspallvvtegdnatftcsfsntse sfvlnwyrmspsnqtdklaafpedrsqpgqdcrfrvtqlpngrdfhmsvvrarrndsgt ylcgaislapkaqikeslraelrvterraevptahpspsprpagqfqtlvtttpaprpptpa ptiasqplslrpeacrpaaggavhtrgldfacdiyiwaplagtcgvlllslvitlyckrgrkklly ifkqpfmrpvqttqeedgcscrfpeeeeggcelrvkfsrsadapaykqgqnqlynelnl grreeydvldkrrgrdpemggkprrknpqeglynelqkdkmaeayseigmkgerrrg kghdglyqglstatkdtydalhmqalppr |
| 27 | PD-1 CAR (na) | Atggccctccctgtcactgccctgcttctccccctcgcactcctgctccacgccgctagacca cccggatggtttctggactctccggatcgcccgtggaatcccccaaccttctcaccggcact cttggttgtgactgagggcgataatgcgaccttcacgtgctcgttctccaacacctccgaat cattcgtgctgaactggtaccgcatgagcccgtcaaaccagaccgacaagctcgccgcgtt tccggaagatcggtcgcaaccgggacaggattgtcggttccgcgtgactcaactgccgaat ggcagagacttccacatgagcgtggtccgcgctaggcgaaacgactccgggacctacctg tgcggagccatctcgctggcgcctaaggcccaaatcaaagagagcttgagggccgaactg agagtgaccgagcgcagagctgaggtgccaactgcacatccatccccatcgcctcggcct gcggggcagtttcagaccctggtcacgaccactccggcgccgcgcccaccgactccggcc ccaactatcgcgagccagcccctgtcgctgaggccggaagcatgccgccctgccgccgga ggtgctgtgcatacccggggattggacttcgcatgcgacatctacatttgggctcctctcgc cggaacttgtggcgtgctccttctgtccctggtcatcaccctgtactgcaagcggggtcgga aaaagcttctgtacattttcaagcagcccttcatgaggcccgtgcaaaccacccaggagga ggacggttgctcctgccggttccccgaagaggaagaaggaggttgcgagctgcgcgtgaa gttctcccggagcgccgacgcccccgcctataagcagggccagaaccagctgtacaacga actgaacctgggacggcgggaagagtacgatgtgctggacaagcggcgcggccgggacc ccgaaatgggcgggaagcctagaagaaagaaccctcaggaaggcctgtataacgagctg cagaaggacaagatggccgaggcctactccgaaattgggatgaagggagagcggcgga ggggaaaggggcacgacggcctgtaccaaggactgtccaccgccaccaaggacacatac gatgccctgcacatgcaggcccttccccctcgc |
| 28 | linker | (Gly-Gly-Gly-Ser)n, where n = 1-10 |
| 29 | linker | (Gly4 Ser)4 |
| 30 | linker | (Gly4 Ser)3 |
| 31 | linker | (Gly3Ser) |
| 39 | PD1 CAR (aa) | Pgwfldspdrpwnpptfspallvvtegdnatftcsfsntsesfvlnwyrmspsnqtdkl aafpedrsqpgqdcrfrvtqlpngrdfhmsvvrarrndsgtylcgaislapkaqikeslra elrvterraevptahpspsprpagqfqtlvtttpaprpptpaptiasqplslrpeacrpaa ggavhtrgldfacdiyiwaplagtcgvlllslvitlyckrgrkkllyifkqpfmrpvqttqeed gcscrfpeeeeggcelrvkfsrsadapaykqgqnqlynelnlgrreeydvldkrrgrdpe mggkprrknpqeglynelqkdkmaeayseigmkgerrrgkghdglyqglstatkdty dalhmqalppr |

### EXAMPLES

### Example 1

Identification of genes important in naive T cell, e.g., T_{SCM} cell, proliferation and expansion. Next generation sequencing on T cells transduced with a barcoded shRNA library was conducted to find genes that, when inhibited through shRNA, promoted the expansion of naive T cells, e.g.,m T_{SCM} cells. On target, LSD1, emerged as a gene which, when inhibited, resulted in a more naive T cell phenotype. The effects of LSD1 inhibition on T cell, e.g., CAR T cell, phenotype and/or function were investigated further, as described below.

### FL-LSD1 LC-MS Assay

Representative compounds of the present disclosure were serially and separately diluted 3-fold in DMSO to obtain a total of twelve concentrations. Then the test compounds at each concentration (100 nL of each) were transferred into a 384-well Perkin Elmer ProxiPlate 384 plus plates by Mosquito^{™} Solutions (5 µL) of 0.8 nM, the full-length LSD1 and 0.5 µM FAD in reaction buffer (40 mM Tris-HCl, 0.01% Triton-X100,10 mM KCI,1 mM DTT) were added into the wells and then incubated with the test compound for 30 min. A 5 µL solution of 1 µM of the peptide substrate H3K4me1 (histone H3[1-21]-biotin) in reaction buffer was added to each initiate reaction. The final components in the reaction solution include 0.4 nM FL-LSD1, 0.25 µM FAD, and 0.5 µM H3K4me1 peptide with varying concentration of the compounds. A positive control consisted of the enzyme, 0.25 µM FAD and 0.5 µM substrate in the absence of the test compound, and a negative control consisted of 0.5 µM substrate only. Each reaction was incubated at room temperature for 60 min, and then stopped by the addition of 3 µL quench solution (2.5% TFA with 320 nM d4-SAH). The reaction mixture was centrifuged (Eppendorf centrifuge 5810, Rotor A-4-62) for 1 min at 2000 rpm and read on an API 4000 triple quadrupole mass spec with Turbulon Spray (Applied Biosystem) coupled with Prominence UFLC (Shimadzu). The conversion ratio of H3K4me1 substrate to the H3K4me0 product was calculated by dividing the peak area of the H3K4me0 peptide by the total peak area of all those two peptides on the assumption that the ionization efficiency of those peptides is the same. The data were then fit to a dose response equation using the program Helios to get the IC50 values of the test compound. IC50 values determined according to this method are presented in Table 3.

### Example 2

### Preparation of 5-(6-chloro-4'-(methylsulfonyl)biphenyl-3-yl)-2-(piperazin-1-yl)-1H-pyrrole-3-carbonitrile (1):

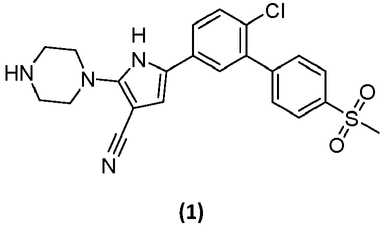

### Preparation of Intermediate 2-bromo-1-(3-bromo-4-chlorophenyl)ethanone (1b):

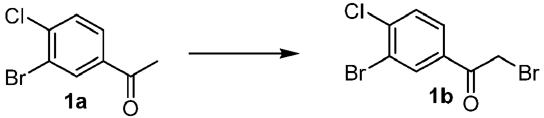

To a solution of 1-(3-bromo-4-chlorophenyl)ethanone (0.4 g, 1.713 mmol) **(1a)** in mixture of EtOAc (10 mL) and CHCl₃ (10.00 ml) was added copper(II) bromide (0.689 g, 3.08 mmol). The reaction mixture was heated at reflux and stirred for 3 h. After cooling to rt, the mixture was added with lOmL of NH₄Cl(aq), and extracted with EtOAc (20mL × 2). The combined organic phase was washed with brine (20mL), dried over Na₂SO₄ (anhydrous), filtered and concentrated under reduced pressure. The residue was purified by flash chromatography( EA/PE=0:100-3:100) to give the title compound (0.23 g, 50%). ¹H NMR (400 MHz, CDCl₃) δ ppm 4.39 (s, 2H), 7.52 - 7.65 (m, 1H), 7.79 - 7.96 (m, 1H), 8.14 - 8.37 (m, 1H).

### Preparation of Intermediate tert-butyl4-(5-(3-bromo-4-chlorophenyl)-3-cyano-1H-pyrrol-2-yl)piperazine-1-carboxylate (1c):

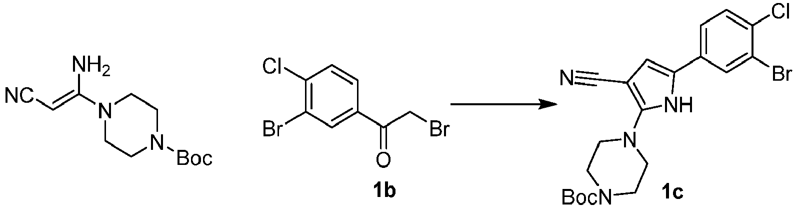

To a solution of (E)-tert-butyl 4-(1-amino-2-cyanovinyl)piperazine-1-carboxylate (242 mg, 0.960 mmol) in anhydrous EtOH (6 ml) was added 2-bromo-1-(3-bromo-4-chlorophenyl)ethanone (300 mg, 0.960 mmol) **(1b)** and sodium hydrogencarbonate (323 mg, 3.84 mmol). The reaction mixture was heated at reflux and stirred for 3 h. After cooling to rt, the mixture was diluted with lOmL of NH₄Cl(aq), and extracted with EtOAc (20mL × 2). The combined organic phase was washed with brine (30mL), dried over Na₂SO₄ (anhydrous), filtered and concentrated under reduced pressure. The residue was purified by flash chromatography(EA/PE =0:100-20:80) to give the title compound (180mg, 36%). LC-MS: [M+H]⁺ =467.0.

### Preparation of Intermediate tert-butyl 4-(5-(6-chloro-4'-(methylsulfonyl)biphenyl-3-yl)-3-cyano-1H-pyrrol-2-yl)piperazine-1-carboxylate (1d):

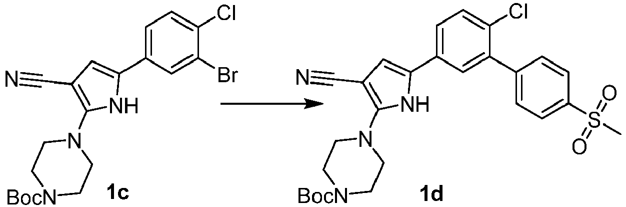

To a solution of tert-butyl 4-(5-(3-bromo-4-chlorophenyl)-3-cyano-1H-pyrrol-2-yl)piperazine-1-carboxylate (50 mg, 0.107 mmol) **(1c)** in mixture of H₂O (0.050 ml) and n-propanol (0.5 ml) was added 4-(methylsulfonyl)phenylboronic acid (42.9 mg, 0.215 mmol), PdCl₂(PPh₃) (7.56 mg, 10.73 µmol), Na₂CO₃ (28.4 mg, 0.268 mmol). The reaction mixture was heated at 100 °C for 1 h under N₂ with microwave. After cooling to rt, the mixture was diluted with 5mL of NH₄Cl(aq), and extracted with EtOAc (10mL × 2). The combined organic phase was washed with brine (10mL), dried over Na₂SO₄ (anhydrous), filtered and concentrated under reduced pressure. The residue was purified by flash chromatography (EA/PE=0:100-35:65) to give the title compound (40mg, 62%). ¹H NMR (400 MHz, CDCl₃) δ ppm 1.47 (s, 9 H), 3.08 (s, 3H), 3.33 (br s., 4 H), 3.53 (t, *J*=4.89 Hz, 4H), 6.26 - 6.67 (m, 1H), 7.38 - 7.53(m, 3H), 7.66 (d, *J*=8.28 Hz, 2H), 7.92 (d, *J*=8.28 Hz, 2H), 8.87 - 9.05 (m, 1H).

### Preparation of 5-(6-chloro-4'-(methylsulfonyl)biphenyl-3-yl)-2-(piperazin-1-yl)-1H-pyrrole-3-carbonitrile (1):

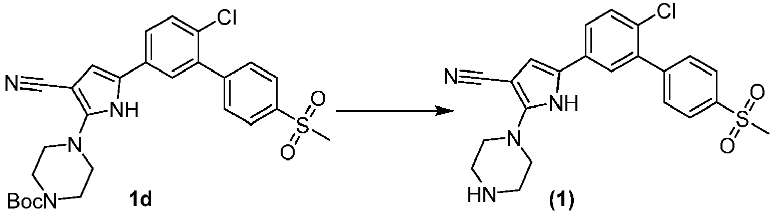

A mixture of tert-butyl 4-(5-(6-chloro-4'-(methylsulfonyl)biphenyl-3-yl)-3-cyano-1H-pyrrol-2-yl)piperazine-1-carboxylate **(Id)** (30 mg, 0.055 mmol) and HCl in EtOAc (2 ml, 2.000 mmol) was stirred at 25 °C for 1 h. The precipitate was filtered and washed by EtOAc to afford desired product (25mg, 97%). ¹H NMR (400 MHz, MeOD-*d*₄) δ ppm 3.20 (s, 3H), 3.39-3.42 (m, 4H), 3.64 - 3.66 (m, 4H), 6.67 (s, 1H), 7.53 (d, *J*=8.68 Hz, 1H), 7.60-7.63 (m, 2H), 7.74-7.76 (m, 2H), 8.06 (d, *J*=8.20 Hz, 2H). LC-MS: [M+H]⁺ =441.1.

### Example 3

### Preparation of N,N-dimethyl-1-((4-(4-(4-(piperidin-4-yl)phenyl)-1H-indazol-1-yl)phenyl)sulfonyl)piperidin-4-amine (2):

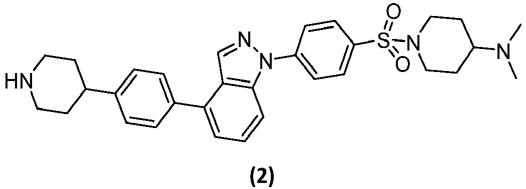

### Preparation of Intermediate tert-butyl4-(4-(4,4,5,5-tetramethyl-1,3,2-dioxaborolan-2-yl)phenyl)piperidine-1-carboxylate (2a):

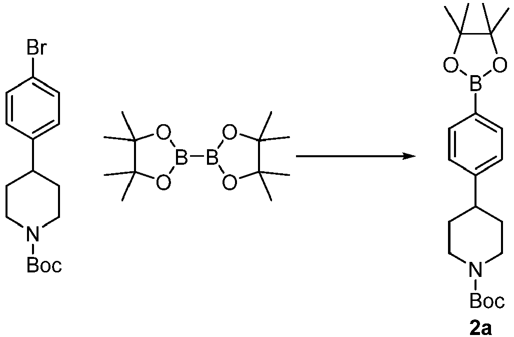

A mixture of tert-butyl 4-(4-bromophenyl) piperidine-1-carboxylate (510 mg, 1.499 mmol), 4,4,4',4',5,5,5',5'-octamethyl-2,2'-bi(1,3,2-dioxaborolane) (761 mg, 3 mmol) and KOAc (441 mg, 4.5 mmol) in DME (20 ml) was degassed with N₂ and PdCl₂(dppf)-CH₂Cl₂ (122 mg, 0.15 mmol) was added. The resulting mixture was degassed with N₂ again and was heated to 80°C overnight. After cooling to rt, the mixture was added with lOmL of NH₄Cl(aq), and extracted with EtOAc (20mL × 2). The combined organic phase was washed with brine (20mL), dried over Na₂SO₄ (anhydrous), filtered and concentrated under reduced pressure. The residue was purified by flash chromatography (EA/Hexane =0:100-1:5) to give the title compound (500 mg , 86%). ¹H NMR (400 MHz, CDCl₃) δ ppm 1.27 (s, 9H), 1.34 (s, 12H), 1.57 - 1.72 (m, 2H), 1.82 (br d, *J*=13.05 Hz, 2H), 2.66 (br s, 1 H), 2.80 (br t, *J*=12.05 Hz, 2H), 4.25 (br d, *J*=12.80 Hz, 2H), 7.11 - 7.42 (m, 2H), 7.77 (d, *J*=8.03 Hz, 2H). LC-MS: [M+H-100]⁺ = 288.2.

### Preparation of Intermediate 1-((4-iodophenyl)sulfonyl)-N,N-dimethylpiperidin-4-amine (2b):

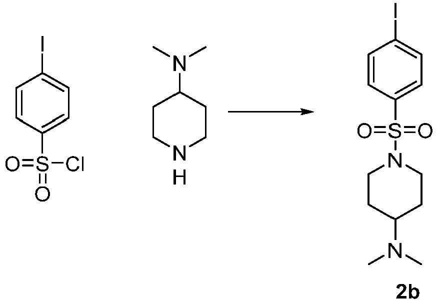

To a solution of 4-iodobenzene-1-sulfonyl chloride (2 g, 6.61 mol) and N,N-dimethylpiperidin-4-amine (0.848 g, 6.61 mmol) in DCM (20 ml) was added TEA (1.115 ml, 7.93 mmol). The mixture was stirred at rt for 3 h. LC-MS indicated the reaction was complete. The mixture was concentrated. The residue was purified by flash chromatography (MeOH: DCM=0:100 to 10:100) to give the title compound (2.85 g , 98%) as a white solid. ¹H-NMR (400 MHz, MeOD-d*4*) δ ppm 1.38 - 1.61 (m, 2H), 1.86 (br d, *J*=11.80 Hz, 2H), 2.19 - 2.56 (m, 6H), 3.01 (br d, *J*=6.53 Hz, 3H), 3.61 (br d, *J*=11.80 Hz, 2H), 7.49 (d, *J*=8.53 Hz, 2H), 8.02 (s, 2H). LC-MS: [M+H]⁺ = 394.9.

### Preparation of Intermediate 1-((4-(4-bromo-1H-indazol-1-yl)phenyl)sulfonyl)-N,N-dimethylpiperidin-4-amine (2c):

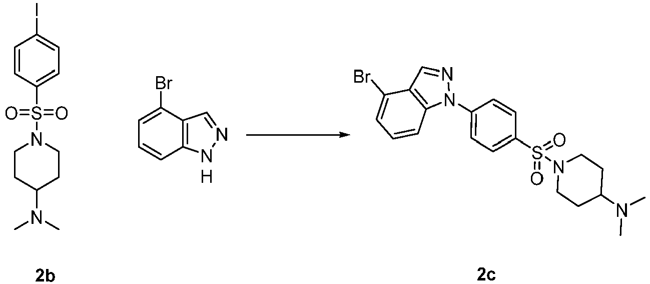

A mixture of 1-((4-iodophenyl)sulfonyl)-N,N-dimethylpiperidin-4-amine **(2b)** (550 mg, 1.255 mmol), 4-bromo-1H-indazole (297 mg, 1.507 mmol) and K₂CO₃ (521 mg, 3.77 mmol) in DMSO (10 ml) was degassed with N₂ and L-Proline (116 mg, 1.004 mmol) and Cul (96 mg, 0.502 mmol) was added. The resulting mixture was degassed with N₂ again and was heated to 105°C for 18h. After cooling to rt, the mixture was added with 10mL of NH₄Cl(aq), and extracted with EtOAc (20mL × 2). The combined organic phase was washed with brine (20mL), dried over Na₂SO₄ (anhydrous), filtered and concentrated under reduced pressure. The residue was purified via prep-HPLC to give the title compound (170 mg, 29.2%) as a white solid. LC-MS: [M+H]⁺ = 463.0; [M+H+2]⁺ = 465.0.

### Preparation of Intermediate tert-butyl4-(4-(1-(4-((4-(dimethylamino)piperidin-1-yl)sulfonyl)phenyl)-1H-indazol-4-yl)phenyl)piperidine-1-carboxylate (2d):

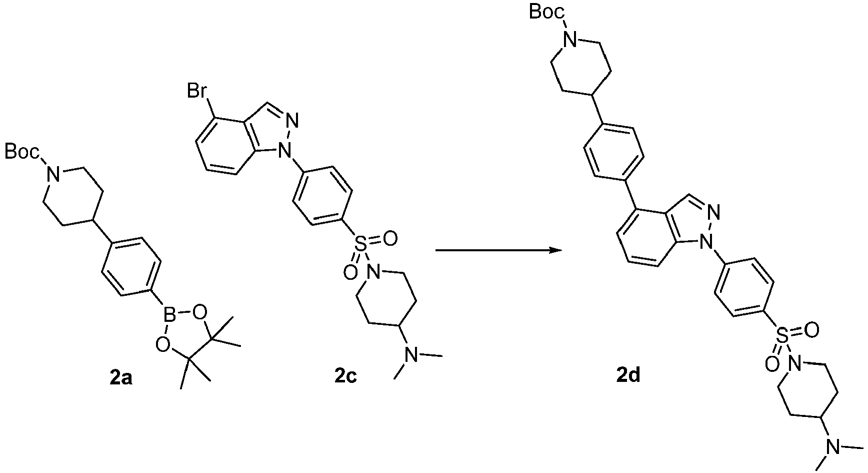

To a mixture of tert-butyl 4-(4-(4,4,5,5-tetramethyl-1,3,2-dioxaborolan-2-yl)phenyl)piperidine-1-carboxylate **(2a)** (251 mg, 0.647 mmol), 1-((4-(4-bromo-1H-indazol-1-yl)phenyl)sulfonyl)-N,N-dimethylpiperidin-4-amine **(2c)** (200 mg, 0.432 mmol) and K₂CO₃ (179 mg, 1.295 mmol) in a mixture of dioxane (8 mL) and H₂O (1.6 mL) was added PdCl₂(dppf).CH₂Cl₂ adduct (35.2 mg, 0.043 mmol) with N₂ atmosphere. The reaction mixture was heated at 100 °C under microwave for 30 min. After cooling to rt, the mixture was added with lOmL of NH₄Cl(aq), and extracted with EtOAc (20mL × 2). The combined organic phase was washed with brine (20mL), dried over Na₂SO₄ (anhydrous), filtered and concentrated under reduced pressure. The residue was purified via prep-HPLC to give the title compound (120 mg, 43.2%) as a white solid. LC-MS: [M+H]⁺ = 644.3.

### Preparation of N,N-dimethyl-1-((4-(4-(4-(piperidin-4-yl)phenyl)-1H-indazol-1-yl)phenyl)sulfonyl)piperidin-4-amine (2):

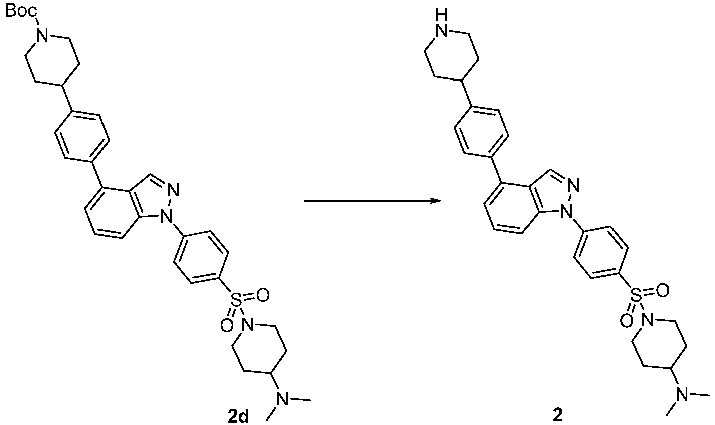

To a solution of *tert*-butyl 4-(4-(1-(4-((4-(dimethylamino)piperidin-1-yl)sulfonyl)phenyl)-1H-indazol-4-yl)phenyl)piperidine-1-carboxylate **(2d)** (490 mg, 0.761mmol) in DCM (20 mL) was added 1 M of HCl in EtOAc (10 mL). The mixture was stirred at 40°C for 3 h. The mixture was concentrated and dried on vacuum to give the title compound (486 mg, 96%). ¹H NMR (400 MHz, DMSO) δ ppm 1.67 - 1.78 (m, 2H), 1.87 - 2.15 (m, 6H), 2.37 (t, *J*=11.42 Hz, 2H), 2.66 (d, *J*=4.52 Hz, 6H), 2.89 - 3.11 (m, 3H), 3.19 (br s., 1H), 3.40 (d, *J*=12.55 Hz, 2H), 3.85 (d, *J*=11.54 Hz, 2H), 7.44 (d, *J*=7.78 Hz, 2H), 7.66 (t, *J*=7.91 Hz, 1H), 7.75 (d, *J*=8.03 Hz, 2 H), 7.92 - 8.04 (m, 3H), 8.13 (d, *J*=8.53 Hz, 2H), 8.56 (s, 1H) 8.73 - 8.89 (m, 1H) 10.52 (br s, 1H). LC-MS: [M+H]⁺ = 544.2.

### Example 4 - Investigations of LSD1 inhibitors

### Generation of CAR19 lentiviral constructs

The scFvs used in the CAR19 constructs that were assessed in the Examples were independently synthesized based on an anti-CD19 scFV sequence (as described in WO2012/079000). The scFV was cloned in a light-to-heavy chain direction, with a flexible linker connecting the VL and VH domains, into a vector backbone containing the CD8 hinge region along with the 4-1BB molecule and the CD3zeta molecule.

### Cell lines and cell transduction

Human T cells were transduced by spinoculation with lentiviral supernatant from 293T cells transfected with CAR19 plasmid DNA.

### Cancer Cells

NALM6 and K562 cell lines were obtained from the ATCC and maintained in media according to supplier's recommendations. To produce a bioluminescent model for T cell killing assays, all cells were transduced with a luciferase lentiviral construct and kept under antibiotic selection.

### Flow cytometry

Cells were isolated from *in vitro* culture, washed once in MACS+0.5% BSA buffer, and stained on ice using either biotinylated Protein L followed by incubated with a fluorochrome-conjugated streptavidin reagent or an antibody recognizing the given target antigen. In all analyses, the population of interest was gated based on forward vs side scatter characteristics, followed by singlet gating, and live cells were gated. Flow cytometry was performed on a four laser Fortessa (Becton-Dickinson).

### Compound treatments

In all instances, compounds were either resuspended in either water (H₂O) or DMSO, depending on compound solubility. In all instances compounds were added throughout the T cell culture, and T cells were washed extensively prior to assay. Compounds and concentrations used were 5ng/mL IL-7 and 5ng/mL IL-15 (recombinant protein; Peprotech), 5 µM TWS119 (also referred to as GSK3bi or GSK3β-i) (GSK3-beta inhibitor; CAS Number 601514-19-6; Cayman), 1 µM Akt l/Ili (also referred to as AKTi or Aktl/II inh) (Akt Inhibitor VIII, Isozyme-Selective, Akti-1/2; EMD Millipore), 100 nM LSD1i-GSK (GSK-LSD1 inhibitor, CAS Number 1431368-48-7, Sigma), 200nM LSD1i-EMD (LSD1 Inhibitor IV, RN-1, HCl; EMD Millipore) 100nM Compound A (LSD1 inhibitor; Novartis), 100nM Compound B (LSD1 inhibitor; Novartis), or 100nM Compound C (LSD1 inhibitor; Novartis) were used. In the figures, "control" refers to vehicle with no additional compound added. IL7/IL15, TWS119, and Akt l/Iii are published compounds known in the art to expand T_{SCM} T cells (IL7/IL15: Xu et al, Blood. 2014; 123(24):3750-9; AKTi: Crompton et al., Cancer Res. 2015; 75(2):296-305, and van der Waart et al, Blood, 2014;124(23):3490-500; TWS119: Gattoni et al., Nature Medicine, 2011;17(10):1290-129).

### Killing assay

T cell killing was directed towards CAR19-expressing NALM6 cell lines stably expressing luciferase. Untransduced cells (UTD) and a non-targeting isotype control scFV CART (IsoCAR) were used to determine non-specific background killing levels. The cytolytic activities of CAR19 were measured as a titration of effector:target cell ratios of 10:1 and 2-fold downward dilutions of T cells where effectors were defined as total T cells, and percentages of CAR bearing T cells were normalized by addition of UTD T cells. Assays were initiated by mixing an appropriate number of T cells with a constant number of targets cells. After 20 hours luciferase signal was measured using the Bright-Glo^{™} Luciferase Assay on the EnVision instrument. Loss of luciferase activity indicates specific killing.

### Cytokine secretion

T cell killing was directed towards CAR19-expressing NALM6 cell lines stably expressing luciferase. Untransduced cells (UTD) and a non-targeting isotype control scFV CART (IsoCAR) were used to determine non-specific background cytokine levels. Effector and target cells were incubated at a 3:1 ratio in RPMI containing 10% FBS for 18 hours. Supernatant was analysed by 3-plex array according the manufacturer's instructions (Invitrogen).

### Proliferation Assay

CAR19 T cells were tested for their ability to proliferate in response to exposure to antigen on target cells. Target cells included NALM6, Raji, and K5624 cells. Untransduced T cells (UTD) and a non-targeting isotype control scFV CART (IsoCAR) were used as non-specific controls for background T cell effects. On the day of assay (Day 0), target cells were counted and transferred to a 50ml tube in 6 mL of T cell media at 3e6 cells/ml. Target cells were irradiated on ice at 10,000 rad. After irradiation, target cells were washed twice in T cell media, counted, and resuspended to 5e5 cells/ml in T cell media on ice.

Frozen transduced T cells were thawed, washed in 10mL complete T cell media, spun at 300g for 10min, and resuspended gently in 3mL of complete T cell media at RT. T-cells were then counted in a cellometer and resuspended to 2.5e6/mL in 10 mL of media. In a 96 well U-bottom plate, 25,000 irradiated target cells and 25,000 transduced CAR T cells (1:1 ratio) were combined in duplicate wells. In a separate well, 75,000 Anti-CD3/CD28 beads were added in 100µl of medium to 25,000 transduced T cells to create a 1:3 cells-to-beads ratio as positive control; in another well, 100µl of medium was added to 25,000 transduced T cells alone as media-only control. Cells were incubated for 4 days at 37°C, 5% CO2.

On day 4, cells were harvested and duplicates were combined by pipetting and transferring into the same well on the U-bottom plate for staining for FACS of CD3 and CAR using protein L. After staining, cells were resuspended in 120µl MACS+0.5% BSA buffer and 20µl/well countbright beads were added to each well. Proliferation was measured as the number of FACS positive cells detected in the period of time used to count 2500 beads.

### Results

### Inhibition of LSD1 by shRNA promotes the expansion of T_{SCM} cells

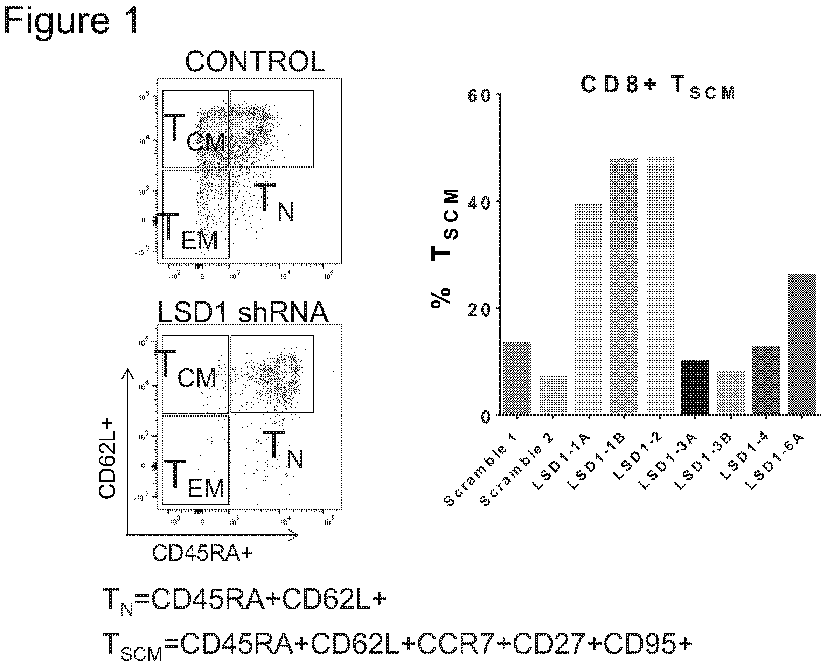

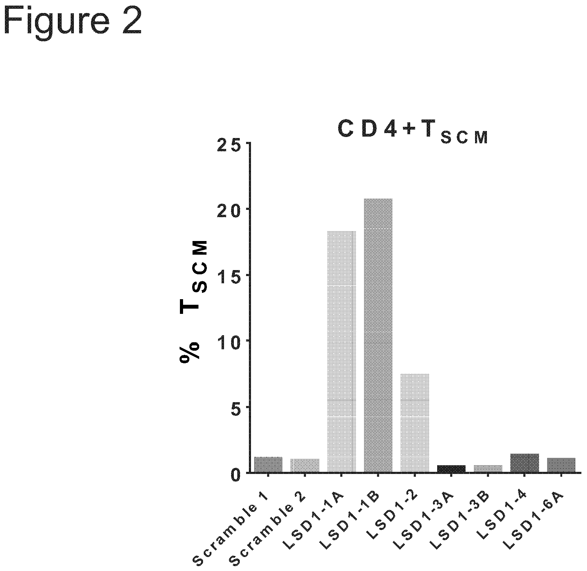

T cells were isolated by negative selection and expanded with anti-CD3/CD28 beads at a 3:1 ratio for 10 days. On day 1, T cells were transduced with lentiviral expression vectors containing LSD1 shRNA targeting sequences that co-expressed the fluorescent protein mCherry. Following expansion, T cell phenotypes of mCherry+ T cells were assessed by flow cytometry. LSD1 inhibition significantly enhanced the percentage of naive (Tn) and stem-like memory cells (T_{SCM}) cells in CD8+ (Figure 1) and CD4+ (Figure 2) T cells relative to controls with scrambled shRNA sequence.

The results are shown for CD8+ T cells in Figure 1. When CD8+ T cells were expanded normally, approximately 10% of the resulting T cells are positive for a T_{SCM} phenotype (CD45RA+CD62L+CCR7+CD27+CD95+). In contrast, when CD8+ T cells transfected with DNA encoding shRNA molecules to LSD1 1A, 1B or 2, the resulting T cell population was enriched in T_{SCM} (e.g., CD45RA+CD62L+CCR7+CD27+CD95+), with nearly 50% of the cells having the T_{SCM} (e.g., CD45RA+CD62L+CCR7+CD27+CD95+) phenotype.

The results are shown for CD4+ T cells in Figure 2. When CD4+ T cells were expanded normally, less than 2% of the resulting T cells are positive for a T_{SCM} phenotype (CD45RA+CD62L+CCR7+CD27+CD95+). In contrast, when CD4+ T cells transfected with DNA encoding shRNA molecules to LSD1 1A, 1B or 2, the resulting T cell population was enriched in T_{SCM} (e.g., CD45RA+CD62L+CCR7+CD27+CD95+), with, in the case of shRNA 1B, up to 20% of the T cells having the T_{SCM} (e.g., CD45RA+CD62L+CCR7+CD27+CD95+) phenotype.

**Table 4**

| **Name** | **SEQ ID NO:** | **Sequence Encoding shRNA** |
|---|---|---|
| Scramble 1 | 123 | GCCGGCAGCTAGCGACGCCATCTCGAGATGGCGTCGCTAGCTGCCGGCTTTTTTGA |
| Scramble 2 | 124 | CCTAAGGTTAAGTCGCCCTCGCTCGAGCGAGGGCGACTTAACCTTAGGTTTTTTGA |
| LSD1 1A | 125 | CGGTTGCTAGAAGCTACATCTGTTAATATTCATAGCAGATGTAGCTTCTAGCAACCGTTTTTTGA |
| LSD1 1B | 126 | CGGTTGCTAGAAGTTACATTTGTTAATATTCATAGCAGATGTAGCTTCTAGCAACCGTTTTTTGA |
| LSD1 2 | 127 | CAGAAGGCCTAGACATTAAACCTCGAGGTTTAATGTCTAGGCCTTCTGTTTTTTGA |
| LSD1 3A | 128 | GCCACATTTCGCAAAGGAAACGTTAATATTCATAGCGTTTCCTTTGCGAAATGTGGCTTTTTTGA |
| LSD1 3B | 129 | GCCACATTTCGTAAAGGAAATGTTAATATTCATAGCGTTTCCTTTGCGAAATGTGGCTTTTTTGA |
| LSD1 4 | 130 | CGGACAAGCTGTTCCTAAAGACTCGAGTCTTTAGGAACAGCTTGTCCGTTTTTTGA |
| LSD1 6A | 131 | CGAGTTGCCACATTTCGCAAAGTTAATATTCATAGCTTTGCGAAATGTGGCAACTCGTTTTTTGA |

### Inhibition of LSD1 by small molecules promotes the expansion of T_{SCM} cells

The ability of the indicated compounds to produce T cells of a given phenotype was assessed. Naive human T cells were isolated by negative selection and expanded with anti-CD3/CD28 beads at a 3:1 ratio for 10 days in the presence of the indicated compounds. Compounds were refreshed every 2 days. Following expansion, T cell phenotypes were determined by FACS staining. LSD1 inhibition significantly enhanced the percentage of T_{SCM} cells and limits the percentage of T_{EM} cells in CD4+ (Figure 3A) and CD8+ (Figure 3B) T cells relative to controls and relative to other molecules believed in the art to affect T_{SCM} proliferation.

### Inhibition of LSD1 by small molecules promotes a superior ratio of T_{SCM} to T_{EM} T cells

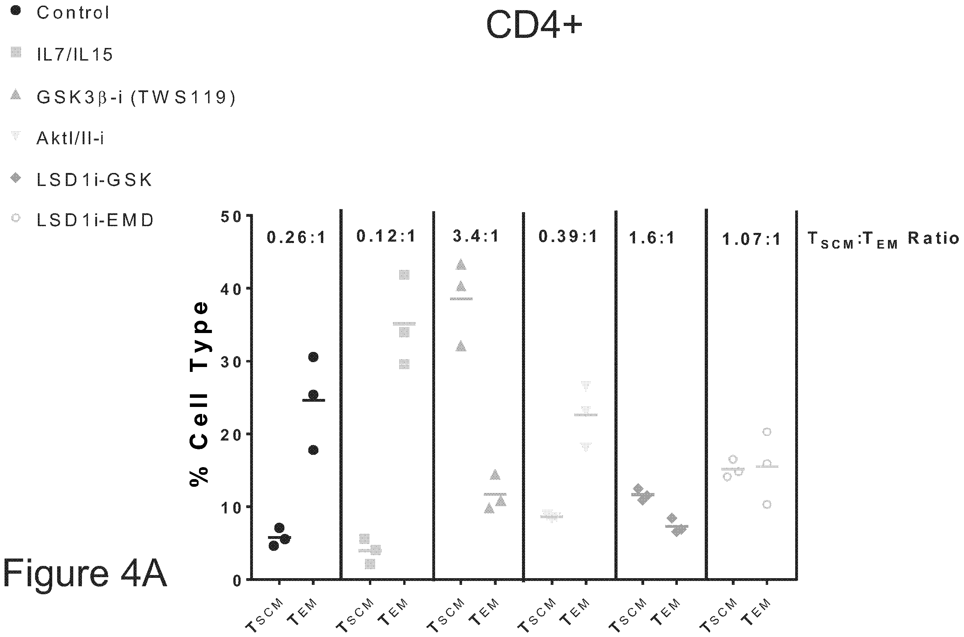

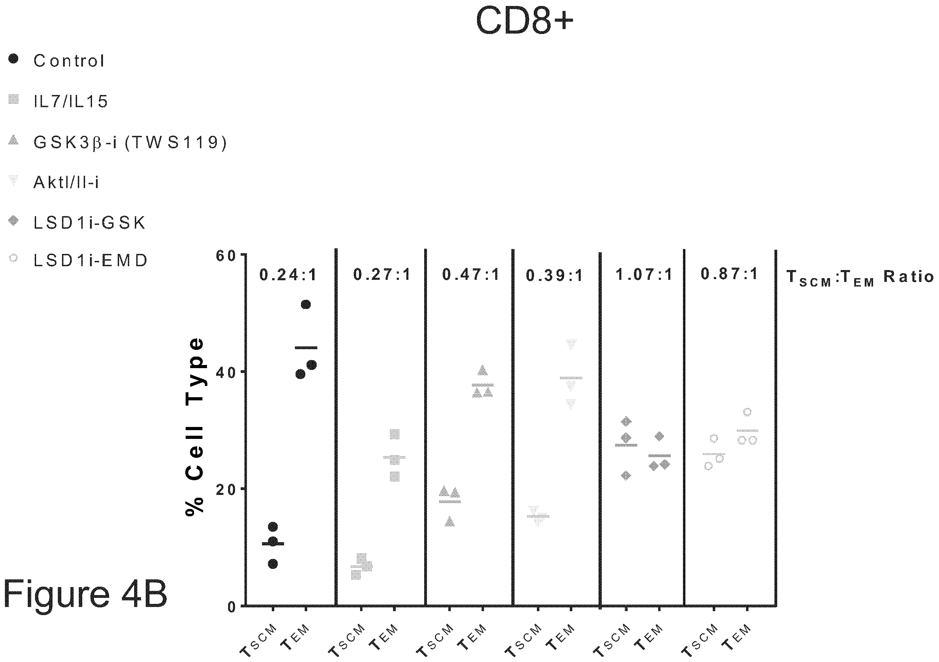

The ability of the indicated compounds to produce T cells of a given phenotype was assessed. Naive human T cells were isolated by negative selection and expanded with anti-CD3/CD28 beads at a 3:1 ratio for 10 days in the presence of the indicated compounds. Compounds were refreshed every 2 days. Following expansion, T cell phenotypes were determined by FACS staining. LSD1 inhibition promotes a ratio of T_{SCM} to T_{EM} consistent with superior T functionality in CD4+ (Figure 4A) and CD8+ (Figure 4B) T cells relative to controls and relative to other molecules believed in the art to affect T_{SCM} proliferation.

### Expansion of T cells in the presence of LSD1 inhibition

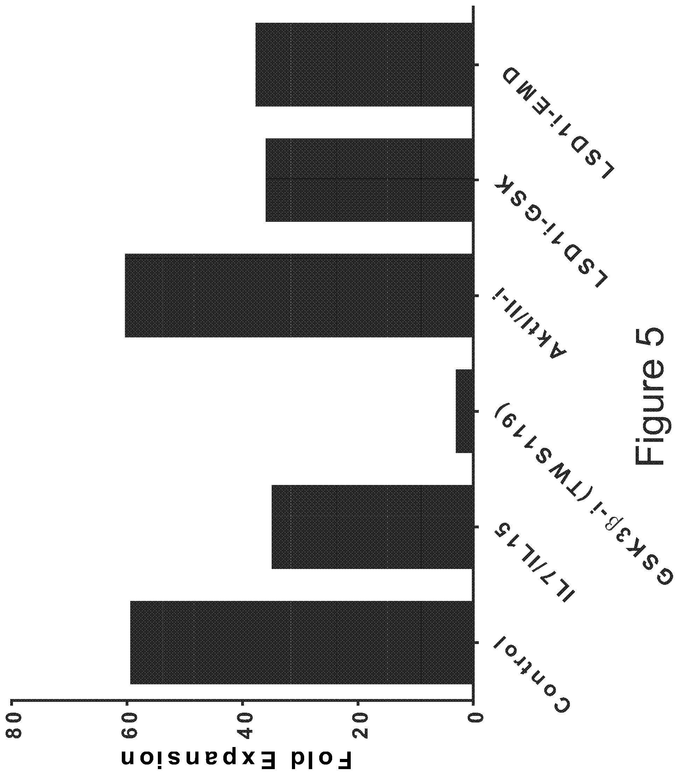

The ability of T cells to expand in the presence of the indicated compounds was assessed. Naive human T cells were isolated by negative selection and expanded with anti-CD3/CD28 beads at a 3:1 ratio for 10 days in the presence of the indicated compounds. Compounds were refreshed every 2 days. Following expansion, T cell numbers were counted and the fold expansion relative to day 1 was assessed. LSD1 inhibition does not significantly impair T cell expansion relative to controls and relative to other molecules believed in the art to affect T_{SCM} proliferation (Figure 5).

### Expression of checkpoint receptors in T cells

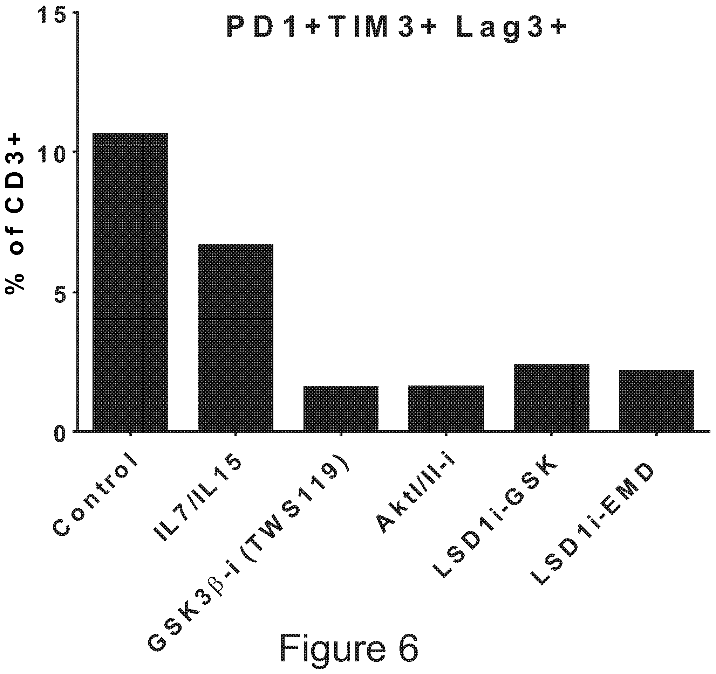

Without being bound by theory, it is believed that co-expression of multiple checkpoint receptors is associated with T cell dysfunction. The ability of the indicated compounds to reduce the expression of checkpoint receptor on T cells was assessed. Naive human T cells were isolated by negative selection and expanded with anti-CD3/CD28 beads at a 3:1 ratio for 10 days in the presence of the indicated compounds. Compounds were refreshed every 2 days. Following expansion, T cell phenotypes were determined by FACS staining. LSD1 inhibition reduces the coexpression of PD1, Tim3, and LAG3 on T cells relative to controls (Figure 6).

### No effect of LSD1 inhibition on CART19 cell expression

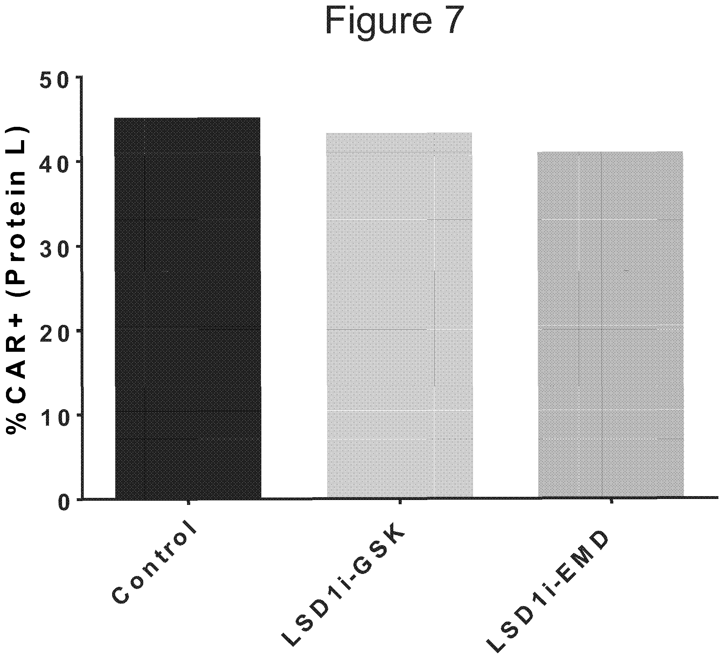

Total human T cells were isolated by negative selection and expanded with anti-CD3/CD28 beads at a 3:1 ratio for 10 days in the presence of the indicated compounds. Compounds were refreshed every 2 days. Following expansion, CAR19 expression was determined by FACS staining with Protein L. LSD1 inhibition has no effect on CAR19 expression (Figure 7).

### No effect of LSD1 inhibition on CD8+ and CD4+ T cell proportions

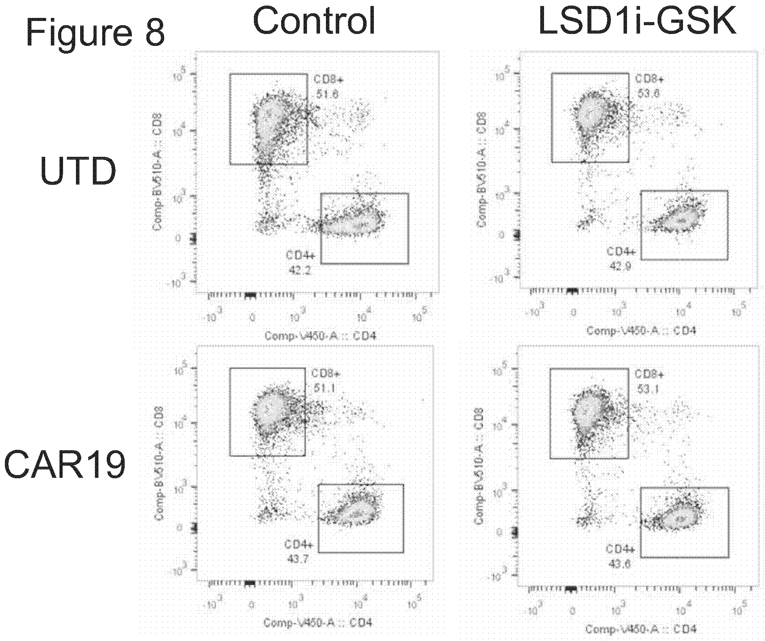

Total human T cells were isolated by negative selection and expanded with anti-CD3/CD28 beads at a 3:1 ratio for 10 days in the presence of the indicated compounds. Compounds were refreshed every 2 days. Following expansion, the percentage of CD4+ and CD8+ T cells were assessed determined by flow cytometry. LSD1 inhibition has no effect on the percentages of CD4+ and CD8+ T cells in either untransduced controls (UTD) or CAR19 transduced T cells (Figure 8).

### No effect of LSD1 inhibition on the expansion of CART19 cells

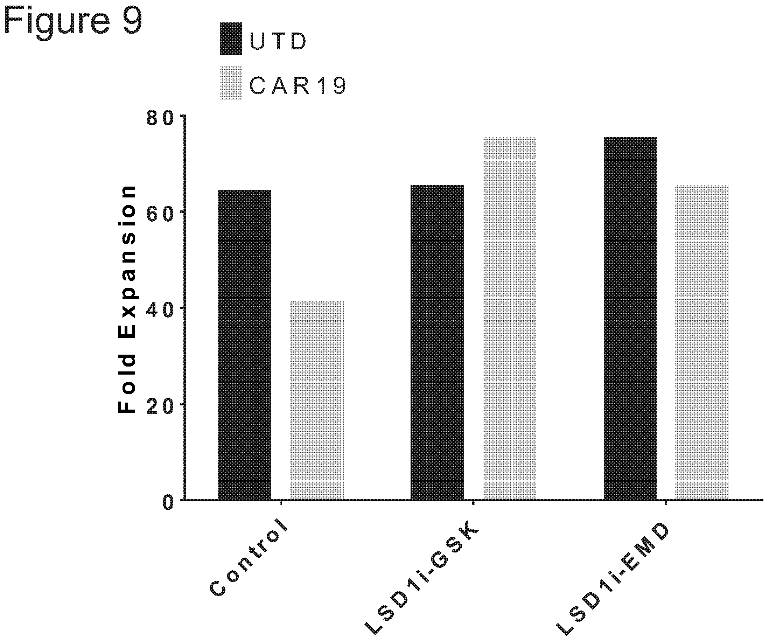

Total human T cells were isolated by negative selection and expanded with anti-CD3/CD28 beads at a 3:1 ratio for 10 days in the presence of the indicated compounds. Compounds were refreshed every 2 days. Following expansion, T cell numbers were counted and the fold expansion relative to day 1 was assessed. CAR19 T cells are able to expand ex vivo in the presence of LSD1 inhibitors, indicating that LSD1 inhibition does not significantly impair T cell expansion in either untransduced controls (UTD) or CAR19 transduced T cells (Figure 9).

### LSD1 inhibition enhances cytokine production from CART19

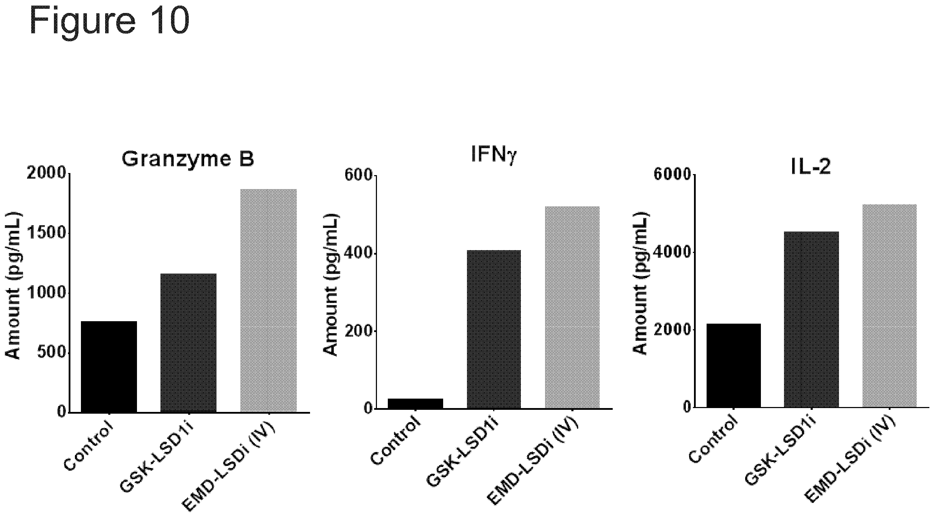

T cells were isolated by negative selection and expanded with anti-CD3/CD28 beads at a 3:1 ratio for 10 days in the presence of the indicated compounds or without treatment (control). T cells were transduced with an anti-CD19 scFV on day 1. Compounds were refreshed every 2 days until day 10 was washed out prior to functional assays, then T cells were frozen. T cells were thawed and incubated with NALM6 cells for 20 hrs. Supernatants were harvested and analyzed by cytokine bead array. LSD1 inhibition significantly enhances cytokine production from CAR19 T cells (Figure 10).

### LSD1 inhibition enhances Proliferation of CART19 cells

T cells were isolated by negative selection and expanded with anti-CD3/CD28 beads at a 3:1 ratio for 10 days in the presence of the indicated compounds or without treatment (control). T cells were transduced with an anti-CD19 scFV on day 1. Compounds were refreshed every 2 days until day 10 was washed out prior to functional assays, then T cells were frozen. T cells were then thawed and mixed with CD19+ tumor cells lines NALM6 and Raji, as well as CD19- tumor cell line K562. Tumor cells were irradiated and T cells and tumor cells were mixed at a 1:1 ratio. On day 4 following incubation, T cells were stained for CAR using Protein L and CAR+ T cell numbers were determined by FACS using countbright beads. Proliferation was measured as the number of FACS positive cells detected in the period of time used to count 2500 beads. Data expressed as fold no target control (K562). LSD1 inhibition enhances the proliferative capacity of CAR19 T cells against CD19+ tumor targets (Figure 11).

### Effect of LSD1 inhibition on killing capacity of CART19 cells

T cells were isolated by negative selection and expanded with anti-CD3/CD28 beads at a 3:1 ratio for 10 days in the presence of the indicated compounds or without treatment (control). T cells were transduced with an anti-CD19 scFV on day 1. Compounds were refreshed every 2 days until day 10 was washed out prior to functional assays, then T cells were frozen. T cells were thawed and incubated with the indicated luciferized cell line targets for 20 hrs. Percent killing was determined by analysis of remaining luciferase activity. These data demonstrate that LSD1 inhibition can increase killing capacity of CAR19 T cells at low E:T ratios, but does not have a significant impact on killing capacity at higher E:T ratios (Figure 12)

### Inhibition of LSD1 promotes enhances CART19 cells efficacy in vivo

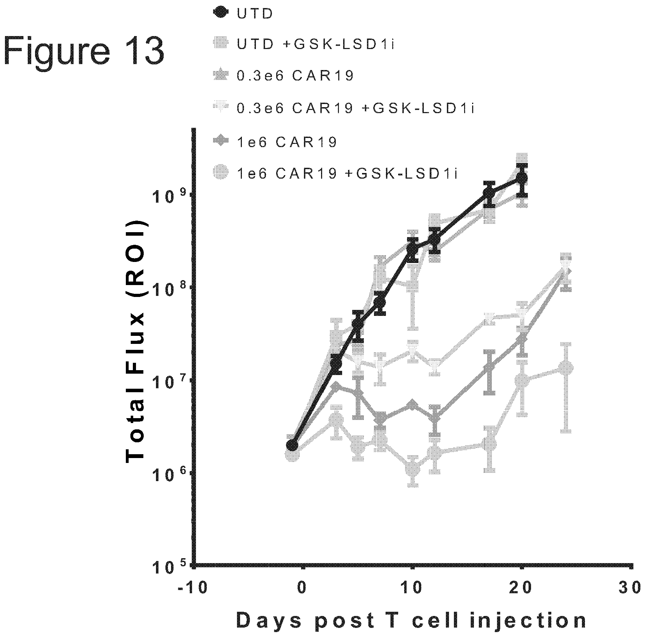

T cells were isolated by negative selection and expanded with anti-CD3/CD28 beads at a 3:1 ratio for 10 days in the presence of the indicated compounds or without treatment (control). T cells were transduced with an anti-CD19 scFV on day 1. Compounds were refreshed every 2 days until day 10 was washed out prior to functional assays, then T cells were frozen. T cells were thawed and injected once into mice bearing established luciferized NALM6 tumors. Doses indicated represent the number of total CAR+ cells injected. These data demonstrate that inhibition of LSD1 during the course of expansion in vitro can significantly enhance the anti-tumor effector function of CAR19 T cells in vivo (Figure 13).

### Multiple LSD1 inhibitors promote the expansion of T_{SCM} cells

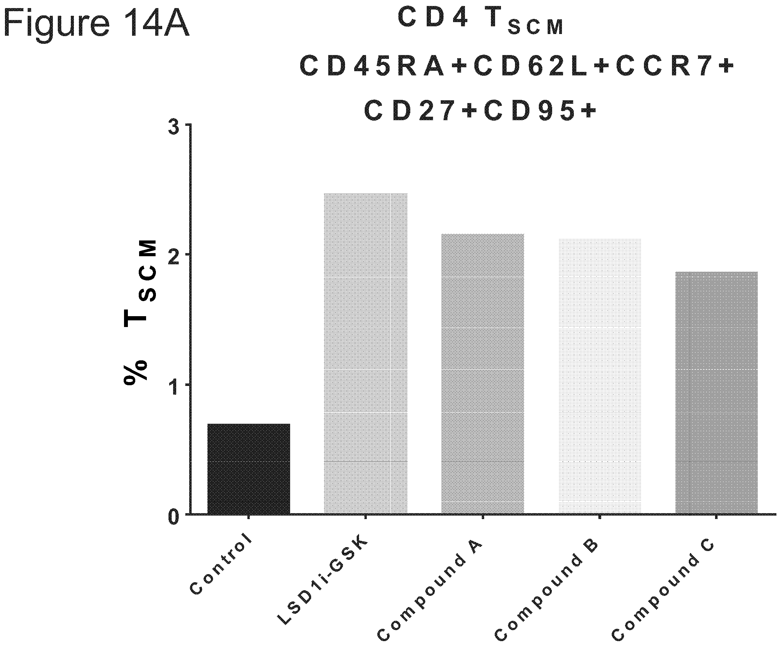

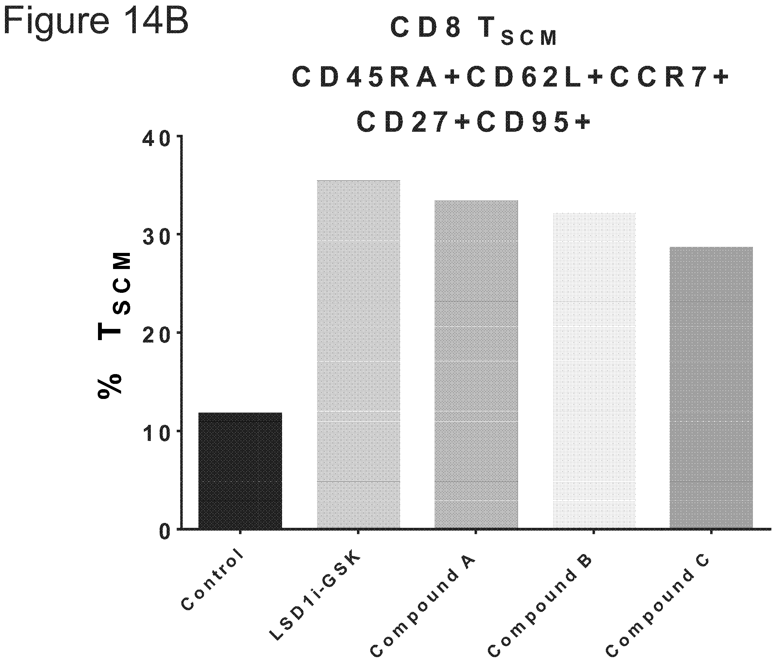

T cells were isolated by negative selection and expanded with anti-CD3/CD28 beads at a 3:1 ratio for 10 days in the presence of the indicated compounds or without treatment (control). Compounds were refreshed every 2 days. Following expansion, T cell phenotypes were determined by flow cytometry. LSD1 inhibition with multiple inhibitors can significantly enhance the percentage of T_{SCM} cells in CD4+ (Figure 14A) and CD8+ (Figure 14B) T cells relative to controls.

### Expression of checkpoint receptors in T cells

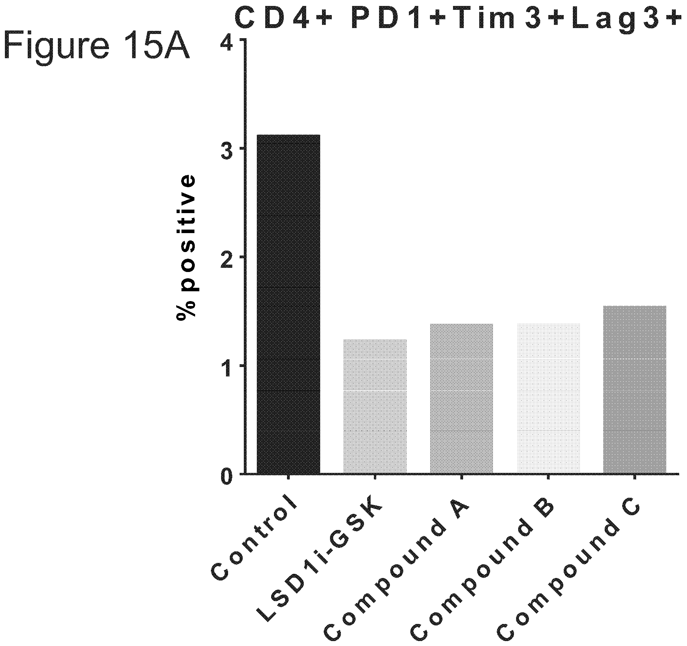

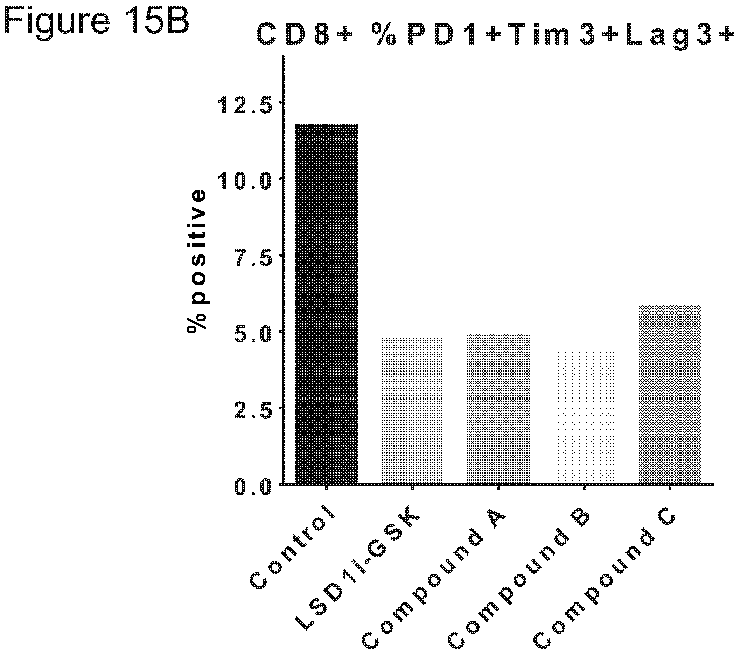

T cells were isolated by negative selection and expanded with anti-CD3/CD28 beads at a 3:1 ratio for 10 days in the presence of the indicated compounds or without treatment (control). Compounds were refreshed every 2 days. Following expansion, T cell checkpoint receptor expression was determined by flow cytometry. Multiple LSD1 inhibitors can reduce the coexpression of PD1, Tim3, and LAG3 on CD4+ (Figure 15A) and CD8+ (Figure 15B) T cells relative to controls.

### Example 5

### Compound 93 ("Example 93"); also referred to herein as "NVS Compound 1"

### Trans-3-(3-amino-2-methylphenyl)-1-(4-hydroxycyclohexyl)-6-methyl-1H-indole-5-carbonitrile Intermediate 93.1: 3-bromo-6-methyl-1-(1,4-dioxaspiro[4.5]decan-8-yl)-1H-indole-5-carbonitrile

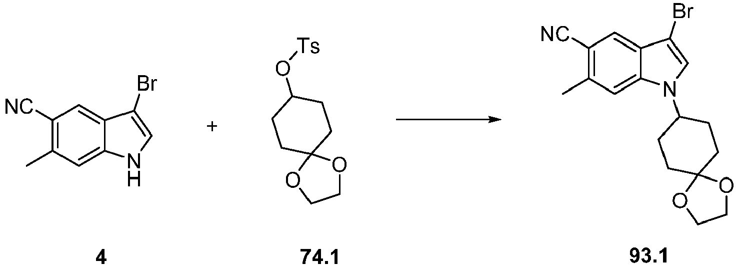

The mixture of compound **74.1** (960 mg, 3.07 mmol), compound **4** (415 mg, 1.77 mmol) and Cs₂CO₃ (1.73 g, 5.31 mmol) in DMF (10 mL) was heated to 100 °C overnight. Then it was diluted with water and extracted with EA for three times. The combined organic phase was washed with brine and dried over Na₂SO₄, filtered and concentrated to give a crude product which was purified by flash column chromatography on silica gel (eluent: PE/EA, EA% = 10^{∼}20%) to give the title compound (726 mg) with 80% purity as a white solid. ¹H NMR (300 MHz, CDCl₃) δ ppm 7.84 (s, 1H), 7.29 (s, 1H), 7.26 (s, 1H), 4.35 - 4.20 (m, 1H), 4.00 (s, 4H), 2.66 (s, 3H), 2.06 (dd, 4H), 1.93 (d, 2H), 1.84 - 1.76 (m, 2H). LC-MS: [M+H]⁺ = 375.29, 377.24.

### Intermediate 93.2: 3-bromo-6-methyl-1-(4-oxocyclohexyl)-1H-indole-5-carbonitrile

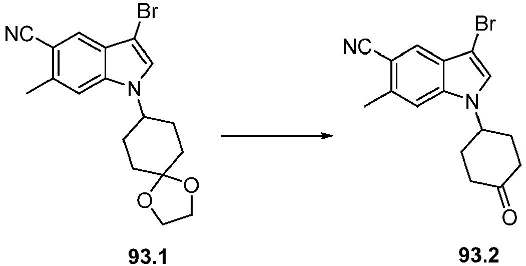

The mixture of compound **93.1** (480 mg, 1.28 mmol) in the co-solvent of AcOH (7.5 mL) and H₂O (1.5 mL) was heated to 60 °C for 3.5 h. After cooling to rt, 10 mL of water was added. Much solid precipitated, which was filtered and washed with water to give the title compound (357 mg, 84%) as a white solid. ¹H NMR (300 MHz, CDCl₃) δ ppm 7.87 (s, 1H), 7.30 (s, 1H), 7.24 (s, 1H), 4.73 (m, 1H), 2.67 (s, 3H), 2.65 - 2.60 (m, 4H), 2.46 - 2.39 (m, 2H), 2.20 (dd, 2H). LC-MS: [M+H]⁺ = 331.2.

### Intermediate 93.3: 3-bromo-1-(4-hydroxycyclohexyl)-6-methyl-1H-indole-5-carbonitrile

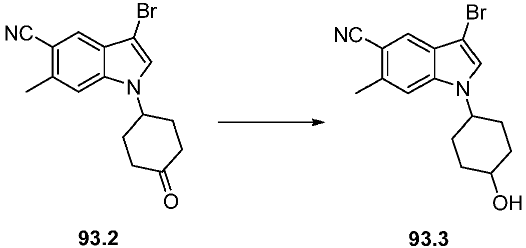

At rt, to a mixture of compound **93.2** (340 mg, 1.03 mmol) in methanol (10 mL) was added NaBH₄ (156 mg, 4.1 mmol) in several portions. The mixture gradually turned clear and was stirred at rt for 1 h. The solvent was removed under vacuum and the residue was dissolved in EA and washed with brine twice. The organic layer was dried over Na₂SO₄, filtered and concentrated to give the title compound (345 mg) as a white solid. ¹H NMR (300 MHz, CDCl₃) δ ppm 7.85 (s, 1H), 7.23 (s, 2H), 4.22 (m, 1H), 3.78 (m, 1H), 2.66 (s, 3H), 2.16 (t, 4H), 1.80 (d, 2H), 1.64 (s, 2H). LC-MS: [M+H]⁺ = 333.2, 335.3.

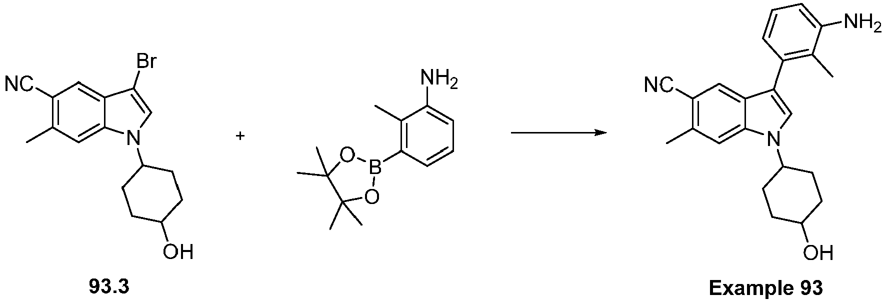

To a mixture of compound **93.3** (200 mg, 0.6 mmol) and 2-methyl-3-(4,4,5,5-tetramethyl-1,3,2-dioxaborolan-2-yl)aniline (168 mg, 0.72 mmol) in the co-solvent of i-PrOH/H₂O (6 mL, 10:1) was added 2N Na₂CO₃ aq. (1.8 mL, 3.6 mmol) and Pd(PPh₃)₂Cl₂ (42 mg, 0.06 mmol). The mixture was stirred at 100 °C for 30 min under N₂ atmosphere by microwave reactor. The reaction mixture was diluted with water and extracted with EA for three times. The combined organic phase was washed with brine and dried over Na₂SO₄, concentrated and purified by column chromatography on silica gel (PE: EA=10:1^{∼}2:1) to give a crude product which contained Ph₃P=O. It was purified by prep-HPLC (0.1% TFA/ACN/H₂O) and lyophilized to give the title compound (73.2 mg, 34%) as a pink solid. ¹H NMR (400 MHz, DMSO-*d*₆) δ ppm 7.76 (s, 1H), 7.69 (d, 1H), 7.64 (s, 1H), 7.21 (t, 1H), 7.07 (d, 2H), 4.47 (d, 2H), 3.84 (s, 1H), 3.58 (t, 2H), 2.59 (s, 3H), 2.13 (d, 3H), 1.94 (t, 6H), 1.53 - 1.44 (m, 2H). LC-MS: [M+H]⁺ = 360.3.

### Example 6

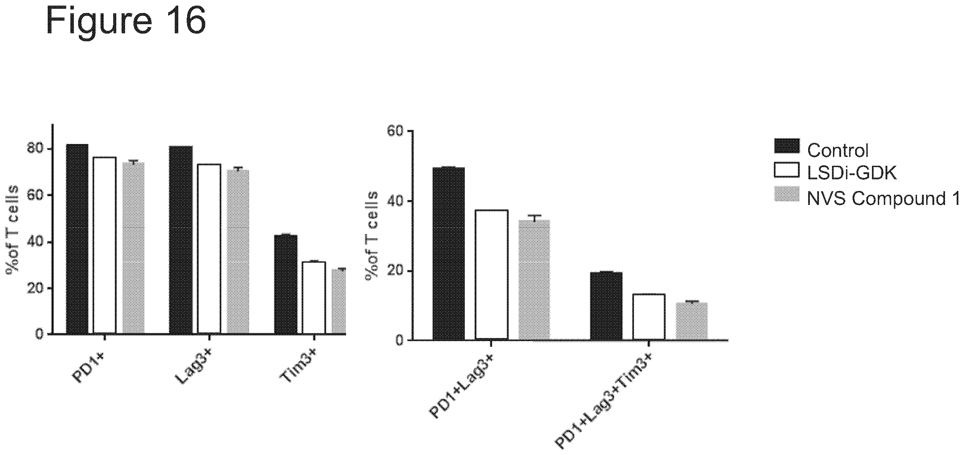

To test the effect of LSD1 inhibition on checkpoint protein expression on T cells, human T cells were isolated by negative selection and expanded with anti-CD3/CD28 beads at a 3:1 ratio for 7 days in the presence of the indicated compounds. Compounds were refreshed every 2 days. Following 7 days of expansion, cells were stained with antibodies against CD4, CD8, PD1, Lag3, and Tim3 and analyzed by flow cytometry. Analysis included assessment of expression of each protein individually (Figure 16, left panel) or the simultaneous expression of PD1 and Lag3 (PD1+Lag3+) or PD1, Lag3, and Tim3 (PD1+Lag3+Tim3+) (Figure 16, right panel). Without being bound by theory, expression of checkpoint markers, and especially, co-expression of multiple checkpoint markers, is thought to be indicative of a more exhausted and less poly-functional T cell. The data indicate that the LSD1 inhibitors can reduce the expression of Tim3 alone, or the co-expression of PD1 and Lag3, or the co-expression of PD1, Tim3, and LAG3 on T cells relative to controls. (Figure 16, left and right panels).

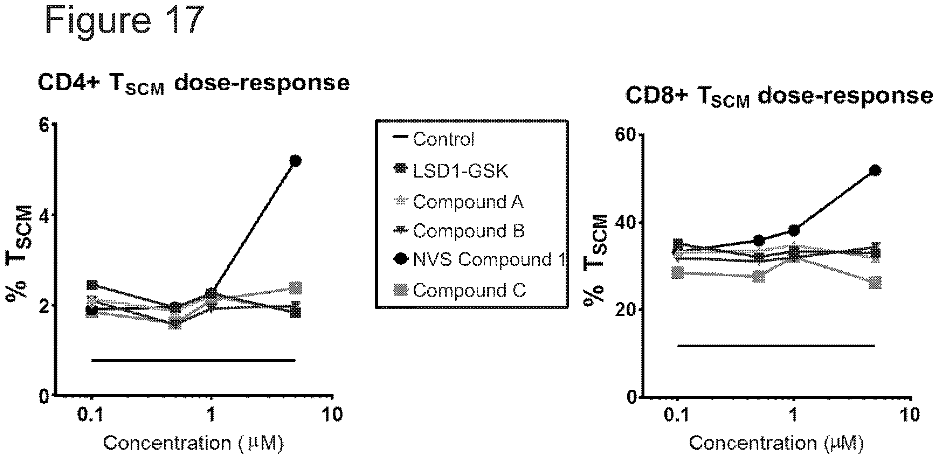

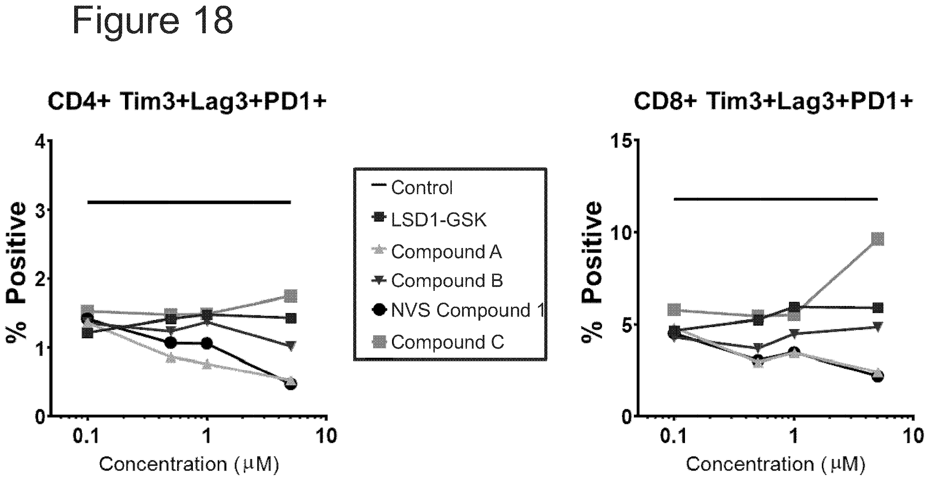

Next, checkpoint marker expression, as well as the fraction of Tscm T cells in either the CD4+ or CD8+ T cell population was assessed in response to culture of T cells in the presence or absence of LSD1 inhibitor. Briefly, T cells were isolated by negative selection and expanded with anti-CD3/CD28 beads at a 3:1 ratio for 10 days in the presence of the indicated compounds at the indicated doses or without treatment (control). Compounds were added the day after bead addition and refreshed every 2 days. Following expansion, the percentage of T_{SCM} cells was determined in CD4+ and CD8+ T cell subsets by flow cytometry using antibodies against CD4, CD8, CD45RA, CD62L, CCR7, CD27, and CD95. These data show that LSD1 inhibition (with any one of a variety of LSD1 inhibitors from multiple molecular classes) can significantly enhance the percentage of T_{SCM} cells in CD4+ (Figure17, left panel) and CD8+ (Figure Figure 17, right panel) T cells relative to controls. As well, following expansion, T cell checkpoint receptor expression was determined in CD4+ and CD8+ T cell subsets by flow cytometry using antibodies against CD4, CD8, Tim3, Lag3, and PD1. These data show that LSD1 inhibition (with any one of a variety of LSD1 inhibitors from multiple molecular classes) can significantly reduce the co-expression of PD1, Tim3, and Lag3 on CD4+ T cells (Figure 18, left panel) and on CD8+ (Figure 18, right panel) T cells relative to controls.

The foregoing written specification is considered to be sufficient to enable one skilled in the art to practice the invention. The present invention is not to be limited in scope by the constructs deposited, since the deposited embodiments are intended to illustrate only certain aspects of the invention. Various modifications of the invention in addition to those shown and described herein will become apparent to those skilled in the art from the foregoing description and fall within the scope of the appended claims.

It is understood that the application of the teachings of the present invention to a specific problem or situation will be within the capabilities of one having ordinary skill in the art in light of the teachings contained herein.

### Example 7 - Investigations of LSD1 inhibitors

### Generation of BCMA-CAR lentiviral constructs

The scFvs used in the BCMA constructs that were assessed in the Examples were independently synthesized based on an anti-BCMA scFV sequence of BCMA-10 (139109) (as described in WO2016/014565)). The scFV was cloned in a light-to-heavy chain direction, with a flexible linker connecting the VL and VH domains, into a vector backbone containing the CD8 hinge region along with the 4-1BB molecule and the CD3zeta molecule.

### Cell lines and cell transduction

Human T cells were transduced by spinoculation with lentiviral supernatant from 293T cells transfected with BCAM-CAR plasmid DNA.

### Cancer Cells

KMS11, NHICI929 and NALM6 cell lines were obtained from the ATCC and maintained in media according to supplier's recommendations. To produce a bioluminescent model for T cell killing, cytokine assays and in vivo efficacy study, all cells were transduced with a luciferase lentiviral construct and kept under antibiotic selection.

### Flow cytometry

Cells were isolated from *in vitro* culture, washed once in MACS+0.5% BSA buffer, and stained on ice using either biotinylated Protein L followed by incubated with a fluorochrome-conjugated streptavidin reagent or an antibody recognizing the given target antigen. In all analyses, the population of interest was gated based on forward vs side scatter characteristics, followed by singlet gating, and live cells were gated. Flow cytometry was performed on a five laser Fortessa (Becton-Dickinson). Data were analysed using R script (Novartis Internal program) and flowjo software. The antibodies used are listed below in Table 5 (for CD19 CAR detection, anti-idiotype CD19 Antibody was included, for BCMA-CAR detection, protein L was included followed by streptavidin-PE staining).

**Table 5. Antibodies used for staining**

| | Differentiation Panel (Panel 2) | | | | **For 10 plates (ul)** | | | | |
|---|---|---|---|---|---|---|---|---|---|
| *Vendor*/*Cat*# | | | uL/sample | uL/ 100 samples | FMO1B | FMO2B | FMO3B | FMO4B | FMO5B |
| *Biolegend*/*423102* | BV510 | Viability | | | **-CD27** | **-CD28** | **-CD45RA** | **-CD62L** | **-CCR7** |
| *Biolegend*/*344822* | AF700 | CD3 | 0.5 | 50 | 5 | 5 | 5 | 5 | 5 |
| Biolegend/344624 | PerCP | CD4 | 1 | 100 | 10 | 10 | 10 | 10 | 10 |
| *BD*/*560179* | APCH7 | CD8 | 0.5 | 50 | 5 | 5 | 5 | 5 | 5 |
| *Biolegend*/*302824* | BV421 | **CD27** | 0.7 | 70 | | 7 | 7 | 7 | 7 |
| *BD*/*562296* | PE-CF594 | **CD28** | 0.7 | 70 | 7 | | 7 | 7 | 7 |
| Biolegend/304210 | ARC | **CD45RA** | 0.1 | 70 | 7 | 7 | | 7 | 7 |
| Biolegend/304822 | PE-Cy7 | **CD62L** | 0.7 | 70 | 7 | 7 | 7 | | 7 |
| Biolegend/359216 | FITC | **CCR7** | 1 | 100 | 10 | 10 | 10 | 10 | |
| Jackson Immunoresearch/016-110-084 | PE | Streptavidin | 0.2 | 20 | 2 | 2 | 2 | 2 | 2 |
| | | Sum | 6 | 600 | 53 | 53 | 53 | 53 | 53 |
| | | FACS Buffer | 34 | 3400 | 347 | 347 | 347 | 347 | 347 |
| | | Final Volume/well | 40 | 4000 | 400 | 400 | 400 | 400 | 400 |

Data were analyzed using R script (novartis internal program) and verified by flowjo software. The following parameters were investigated:
1. Ratio of CD8/CD4 were calculated;
2. % Tscm, Tem, Tcm of CD3+ T cells;
3. Ratios of Tscm/Tem and Tscm/Tcm of CD3+ T cells were calculated;
4. % Tscm, Tem, Tcm of CD8+ or CD4+ T cells were calculated;
5. Ratios of Tscm/Tem and Tscm/Tcm of CD8+ or CD4+ T cells were calculated; and/or
6. In the case of CAR+-T cells, percentage of Tscm, Tem, Tcm of CD19-CAR+T, and BCMA-10-CAR+T cells were evaluated.

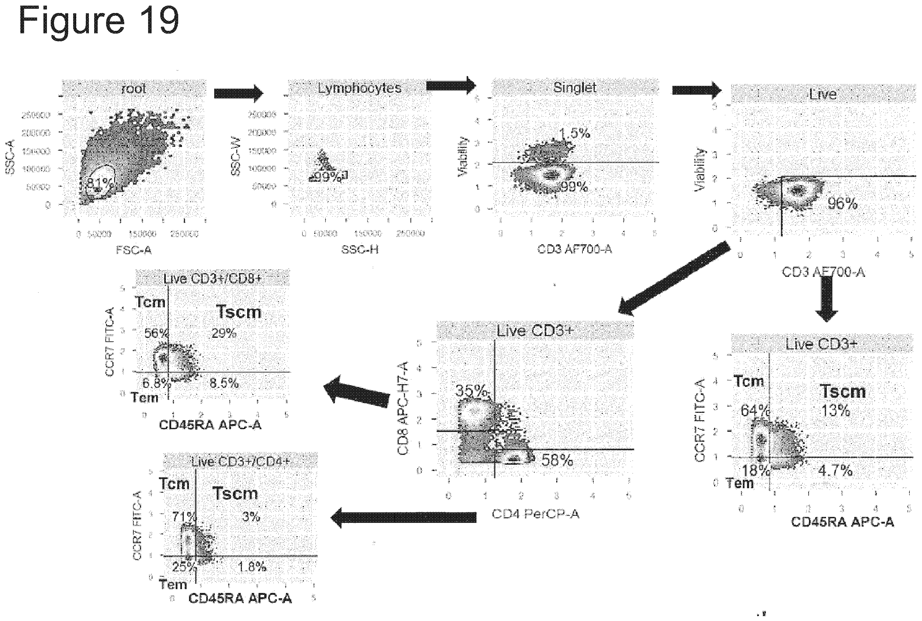

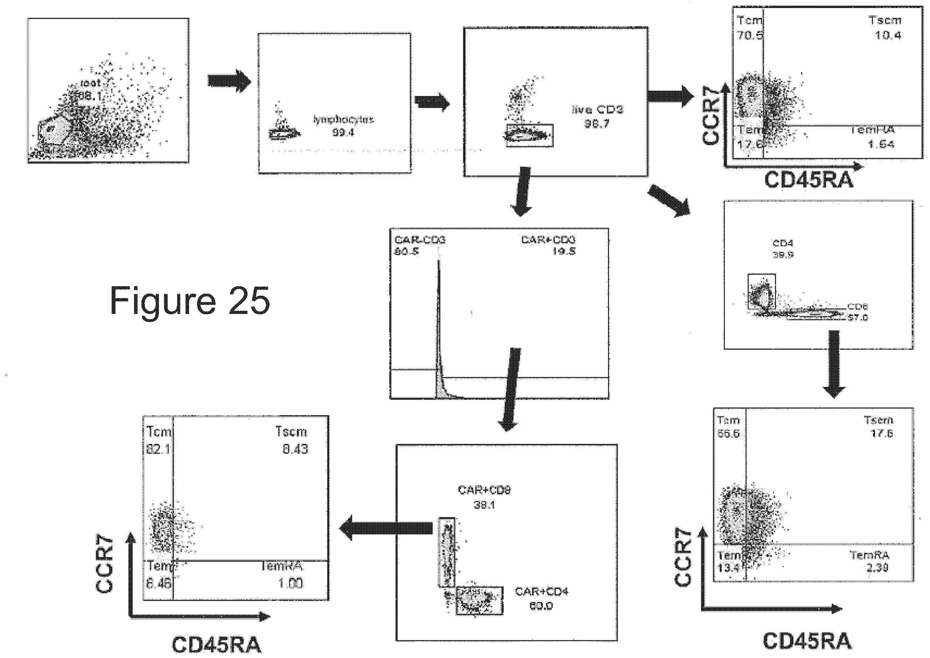

The Tscm population were evaluated as double CD45RA+CCR7+ or triple CD45RA+CCR7+CD62L+ in a given total live CD3+ or subset CD4+ or CD8+ T cell population. Tem were presented as double CD45RA-CCR7- and Tcm as CD45RA-CCR7+ in a given total live CD3+ or subset CD4+ or CD8+ T cell population (figure 19 and figure 25 illustrate the gating strategies).

### Compound treatments

In all instances, LSD inhibitor compounds were either resuspended in water (H₂O) and/or DMSO, depending on compound solubility. In all instances compounds were added throughout the ex vivo T cell culture, and T cells were washed extensively prior to assay. Compounds and concentrations used were 1pM, 10pM, 100pM, 1nM, 10nM, 100 nM LSD1i-GSK (GSK-LSD1 inhibitor, CAS Number 1431368-48-7, Sigma); 1pM, 10pM, 100pM, 1nM, 1.1nM, 3.3nM, 10nM, 100nM NVS Compound 93 (LSD1 inhibitor; Novartis), or 100nM Compound B (LSD1 inhibitor; Novartis), or 100nM Compound A were used. In the figures, "control" refers to vehicle with no LSD inhibitor compound added. For Pan CD3+ T cell expansion experiment, compound treatment was started at 4h or 24h after activation; for CART cell production, compound treatment was started at 48h after viral transduction, and all treatments continued through culture period in all conditions.

### Cytokine secretion

T cell killing was directed towards CAR19-expressing NALM6 cell lines or BCMA-expressing KMS11 or NHICI929 cell lines stably expressing luciferase. Untransduced cells (UTD) and a non-targeting isotype control scFV CART (IsoCAR) were used to determine non-specific background cytokine levels. Effector and target cells were incubated at a 1.25: 1 ratio in RPMI containing 10% FBS for 18 hours. Supernatant was analysed by 3-plex array according the manufacturer's instructions (Invitrogen).

### Proliferation Assay

CAR19 T or BCMA_CAR T cells were tested for their ability to proliferate in response to exposure to antigen on target cells. Target cells included NALM6, KMS11 and NHICI929 cells. Untransduced T cells (UTD) and a non-targeting isotype control scFV CART (IsoCAR) were used as non-specific controls for background T cell effects. On the day of assay (Day 0), target cells were counted and transferred to a 50ml tube in 6 mL of T cell media at 3e6 cells/ml. Target cells were irradiated on ice at 10,000 rad. After irradiation, target cells were washed twice in T cell media, counted, and resuspended to 5e5 cells/ml in T cell media on ice.

Frozen transduced T cells were thawed, washed in 10mL complete T cell media, spun at 300g for 10min, and resuspended gently in 3mL of complete T cell media at RT. T-cells were then counted in a cellometer and resuspended to 2.5e6/mL in 10 mL of media. In a 96 well U-bottom plate, 25,000 irradiated target cells and 25,000 transduced CAR T cells (1:1 ratio) were combined in duplicate wells. In a separate well, 75,000 Anti-CD3/CD28 beads were added in 100µl of medium to 25,000 transduced T cells to create a 1:3 cells-to-beads ratio as positive control; in another well, 100µL of medium was added to 25,000 transduced T cells alone as media-only control. In some cases, 30,000 cells were tested for both target and CART cells. Cells were incubated for 4 days at 37°C, 5% CO2.

On day 4, cells were harvested and duplicates were combined by pipetting and transferring into the same well on the U-bottom plate for staining for FACS of CD3 and CAR using protein L. After staining, cells were resuspended in 120µl MACS+0.5% BSA buffer and 20µl/well countbright beads were added to each well. Proliferation was measured as the number of FACS positive cells detected in the period of time used to count 2500 beads.

### In vivo efficacy study of the LSDli-conditioned BCMA-CART cells

BCMA-CART cells expanded ex vivo either in the presence or absence of LSD1 inhibitor were tested for their ability to eliminate tumor in vivo using the immune compromised NSG mouse bearing established luciferized KMS11 tumors (1e6 cells/mouse); tumor burden are measured by bioluminescent intensity signal generated from the enzyme-substrate (luciferase- luciferin) reaction by injecting the luciferin to the mice.

### Results

### LSD1 inhibitors expand Tscm, reduce Tem at a dose responsive fashion.

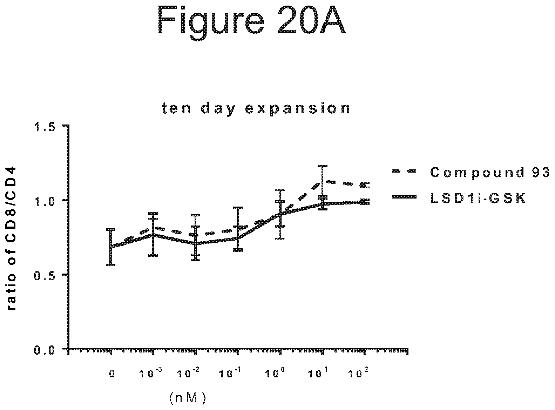

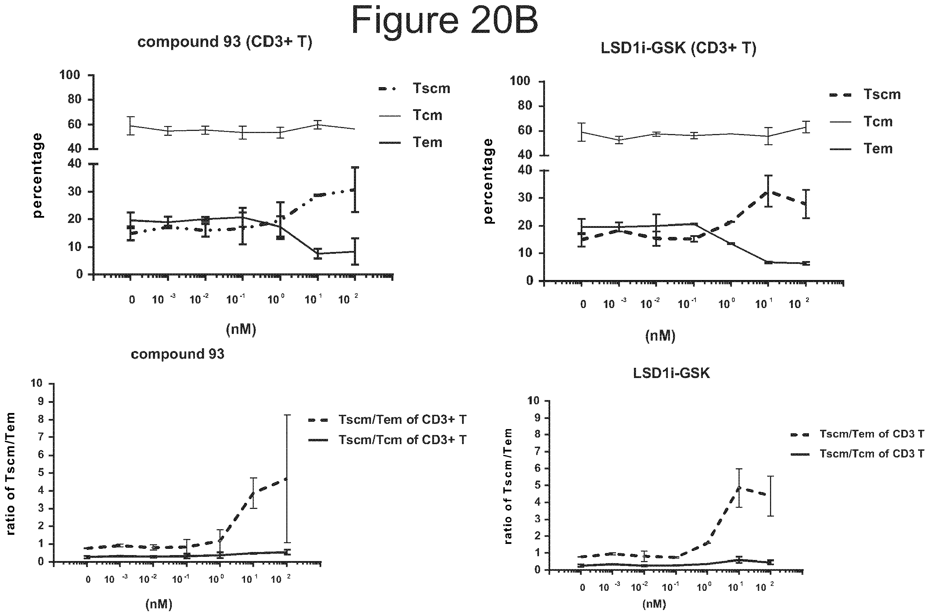

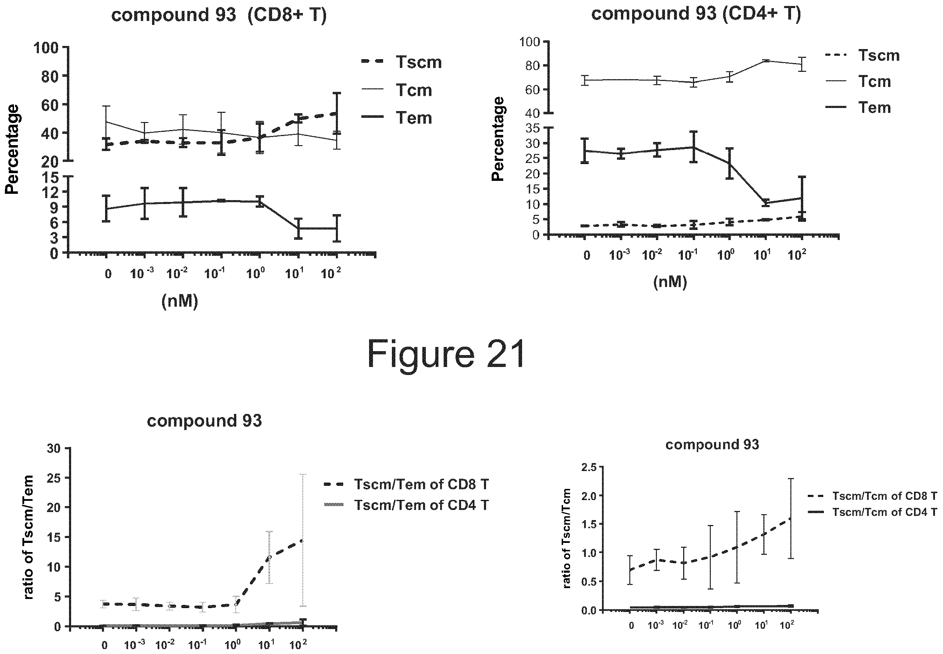

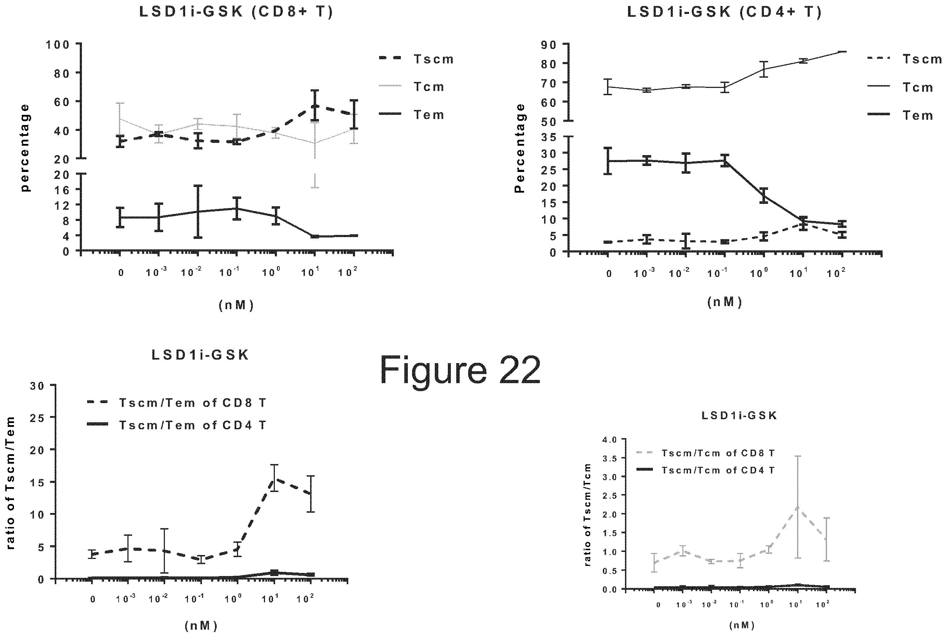

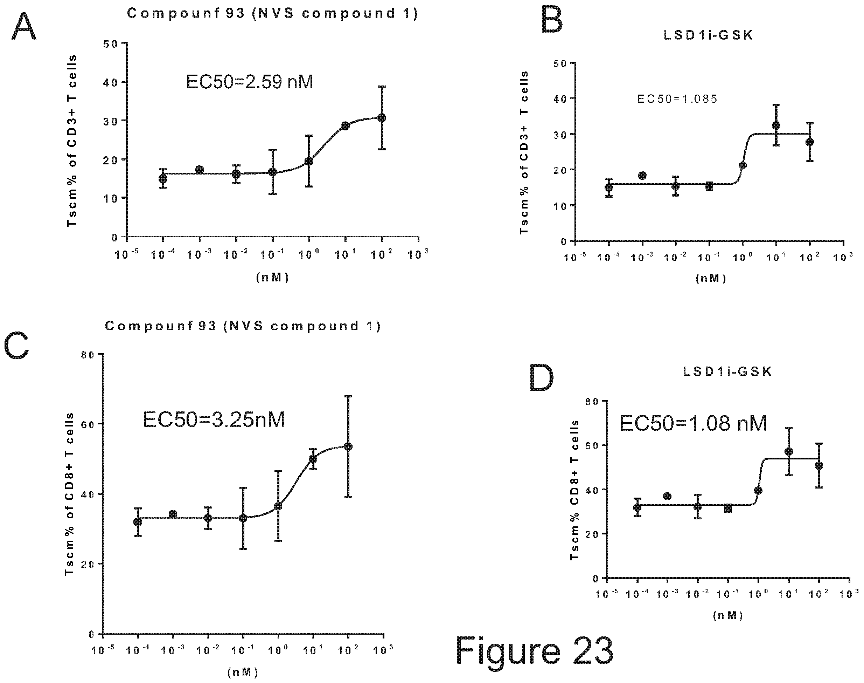

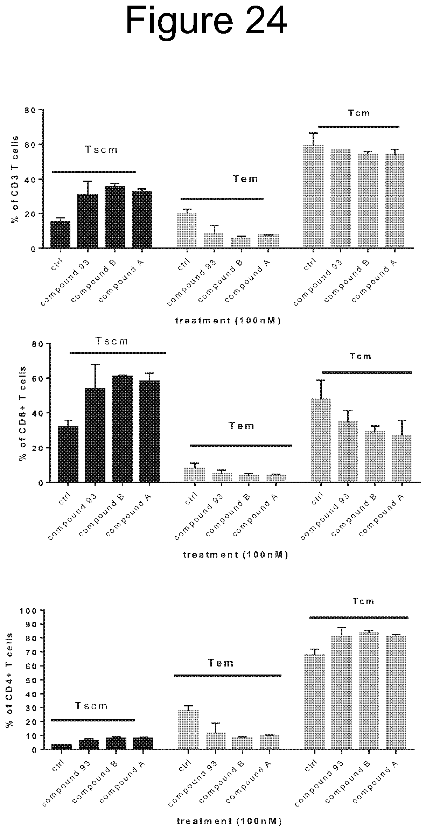

To test the dose responses of LSD1 inhibitors on the T cell phenotypes, human Pan CD3+ T cells from two healthy subjects were isolated by negative selection and expanded with anti-CD3/CD28 beads at a 3:1 ratio for 10 days in the presence of Compound 93 or LSD1i-GSK at different concentrations from 100nM, and subsequent serial 10X dilution to 1pM starting at 4 hours after activation. Meanwhile, other three LSD1 inhibitors Compound A and Compound B at 100nM were also tested for their capability to sustain Tscm cells in some cells starting at 24h after activation. Compounds were refreshed every 2-3 days when fresh media is replenished. Following 10 days of expansion, T cells were harvested and debeaded and their phenotypes were analyzed by flow cytometry using antibodies against CD3, CD4, CD8, CD45RA, CCR7, CD62L ,CD27 and CD28 plus live/dead dye to exclude the dead cell population. Data show that:
1. Both compound 93 and LSD1i-GSK can significantly increase the ratio of CD8/CD4 in response to LSD1i dose escalation, meaning CD8 expand more than CD4 with increased dosage of LSD1 inhibitors during ten day expansion without changing the total expansion rate of total cells. (figure 20A).
2. Both compound 93 and LSD1i-GSK could increase/sustain CD3+ Tscm, decrease Tem, in a dose responsive fashion, observed between 1nM to 100nM; 10nM and 100nM showed similar level of induction, while Tcm remain the same. The EC50 of compound 93 and LSD1i-GSK for inducing Tscm CD3+ T cells are 2.59nM and1.085 nM,respectively (Figure 23A and Figure 23B);
3. Correspondingly, the ratio of Tscm/Tem of CD3+ T cells increases in response to dose elevation, but that of Tscm/Tcm remains unchanged at all doses. (figure 20B).
4. Both compound 93 and LSDli-GSK could increase/sustain CD8+ Tscm and CD4+Tscm, decrease Tem, in a dose responsive fashion, observed between 1nM to 100nM; 10nM and 100nM showed similar level of induction, while Tcm remains unchanged. The majority of Tscm population are from CD8 T cells, while CD4 + Tscm is about 5% at 100nM (which is also increased compared to controls and showing positive dose response) observed from these results (figure 21 and 22).The EC50 of compound 93 and LSD1i-GSK for inducing Tscm CD8+ T cells are 3.25nM and 1.08nM, respectively (Figure 23D and Figure 23C);
5. Correspondingly, the ratio of Tscm/Tem of CD8+ T cells significantly increases in response to dose elevation but it was hardly seen in the CD4+ population; moreover, the ratio of Tscm/Tcm remains largely unchanged relatively to control. (Figure 21 and Figure 22).
6. Compound A and Compound B at 100nM are also able to sustain Tscm and reduce Tern of the total CD3+ and subset of CD8+ T cells, and to much less extent, of the CD4+ T cells, comparable to Compound 93 as shown in Figure 24.

### LSD1 inhibition expands Tscm, reduce Tern in the CART product in a dose dependent manner.

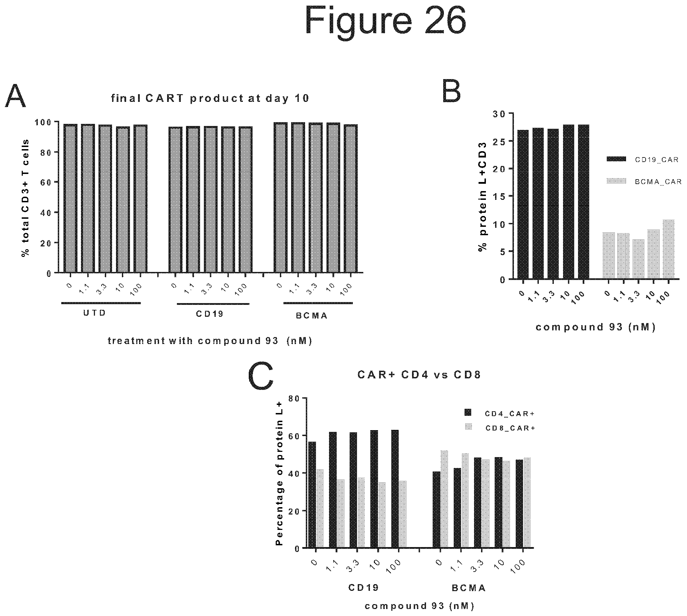

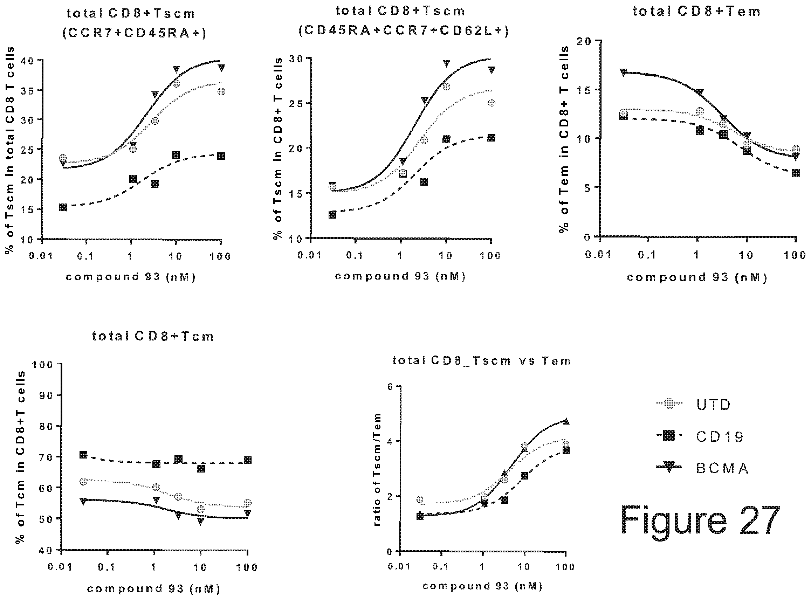

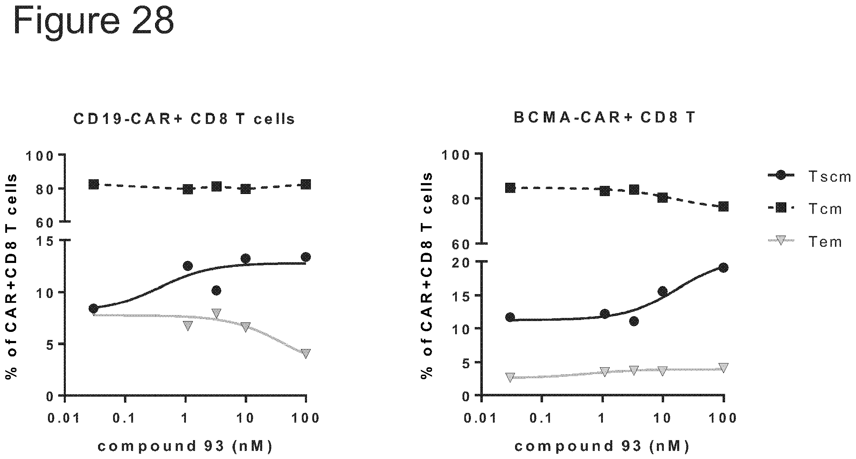

To test the dose responses of LSD1 inhibitors on the CART cell phenotypes, human pan CD3+ T cells from one healthy subject were isolated by negative selection and expanded with anti-CD3/CD28 beads at a 3:1 ratio for 10 days in the presence of Compound 93 at concentrations of 100nM, 10nM, 3.3nM and 1.1nM starting at 48 hours after viral transduction. Different concentrations of the Compound 93 were refreshed every 2-3 days when fresh media is replenished. Following 10 days of expansion, CART product were harvested and de-beaded and their phenotypes were analyzed by flow cytometry using antibodies against CD3, CD4, CD8, CD45RA, CCR7, CD62L ,CD27,CD28, anti-CD19 idiotype or protein L following SA-PE staining plus live/dead dye to exclude the dead cell population. Data show in below bullet points:
- LSD1 inhibition by compound 93 at different tested doses not change the total T cell percentage in all final CART products (UTD, CD19CART and BCMA-CART) (Figure 26A);
- Compound 93 at different tested doses does not remarkably affect CD19-CAR or BCMA-CAR expression (Figure 26B).
- Compound 93 at different tested doses does not affect CD8+ CAR and CD4+CAR T cell proportions. (Figure 26C);
- LSD1 inhibiton by Compound 93 augments CD8+Tscm, reduces CD8+Tem and increases the ratio of Tscm/Tem in the final CD19-CART and BCMA-CART cell populations (which include CAR- and CAR+ population), in a dose dependent manner, but does not change the Tcm (either double CD45RA+CCR7+ or triple CD45RA+CCR7+CD62L+) percentage at all tested doses (Figure 27);
- LSD1 inhibiton by Compound 93 sustains CD19CAR+CD8 Tscm, reduces CAR+ Tern but only increases BCMA-CAR+ CD8+ Tscm in a dose dependent fashion, the CAR+ CD8+ Tcm percentage maintains a similar level at all tested doses (Figure 28).

### LSD1 inhibition by compound 93 enhances cytokine production from CD19-CAR and BCMA-CART

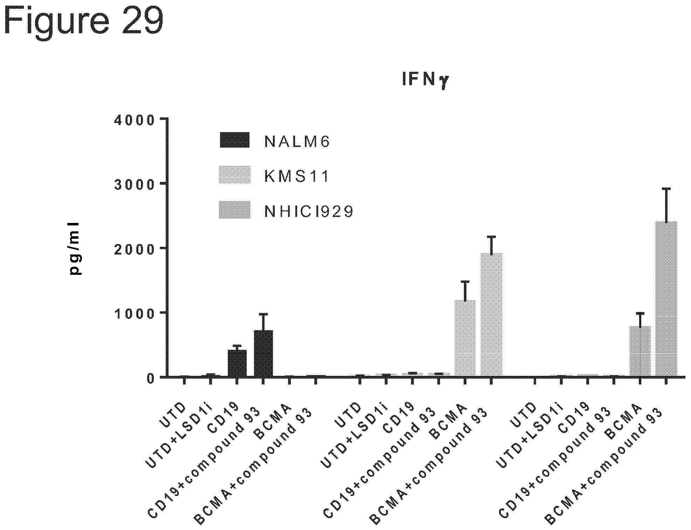

CD19-CART and BCMA-CART cells were incubated with NALM6 (CD19+ but BCMA-), KMS11 and NHICI929 (both CD19- BCMA+) cell lines for 20 hrs at ratio 1.25 to 1. Supernatants were harvested and analyzed by cytokine bead array. LSD1 inhibition by compound 93 at 100nM significantly enhances cytokine production from CD19 CART and BCMA-CART cells in response to their specific targets. (Figure 29).

### LSD1 inhibition by compound 93 enhances proliferation of CD19-CART and BCMA-CART cells

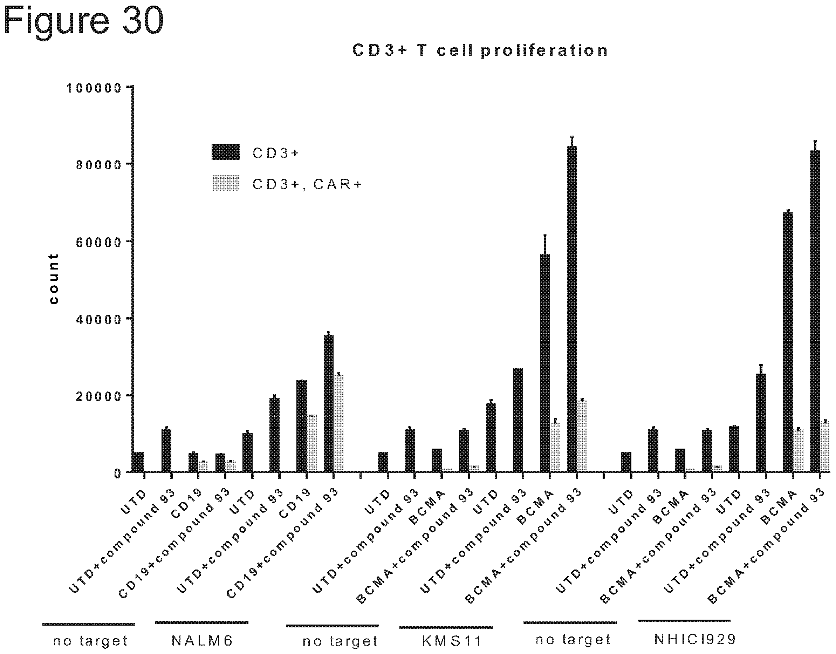

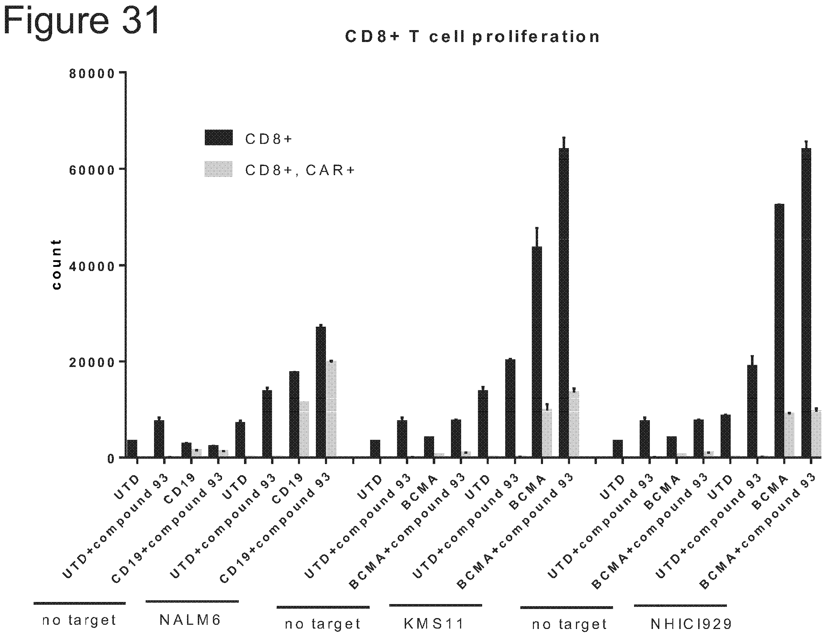

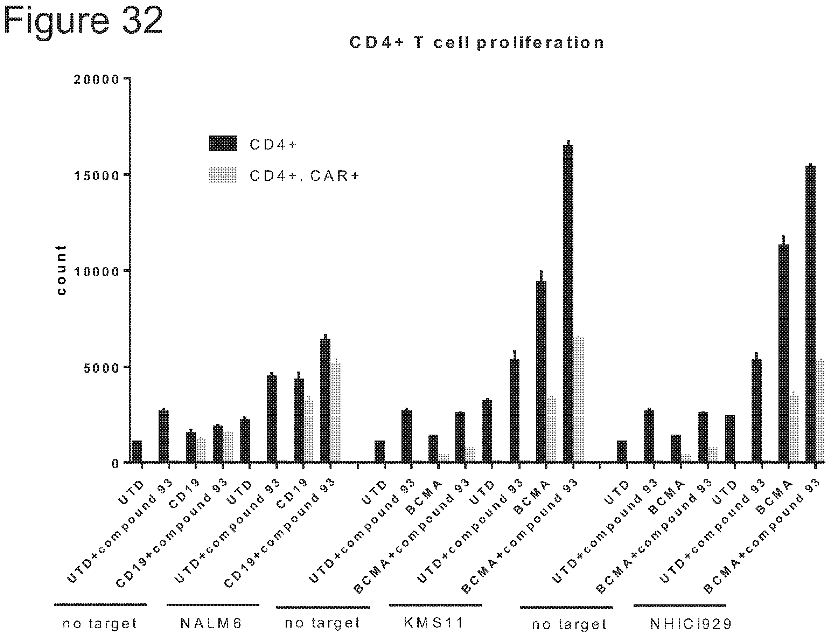

Irradiated tumor lines were mixed with T cells at a 1:1 ratio following 4 day incubation, T cells were stained for CAR using Protein L and CAR+ T cell numbers were determined by FACS using countbright beads. Proliferation was measured as the number of FACS positive cells detected in the period of time used to count 2500 beads. LSD1 inhibition by compound 93 at 100nM enhances the proliferative capacity of CD19-CART and BCMA-CART cells against CD19+ or BCMA+ target cells, respectively, shown in figure 30-32 measured as total CAR+ CD3, CD8 or CD4 T cells.

### Inhibition of LSD1 enhances BCMA-CART cells efficacy in vivo

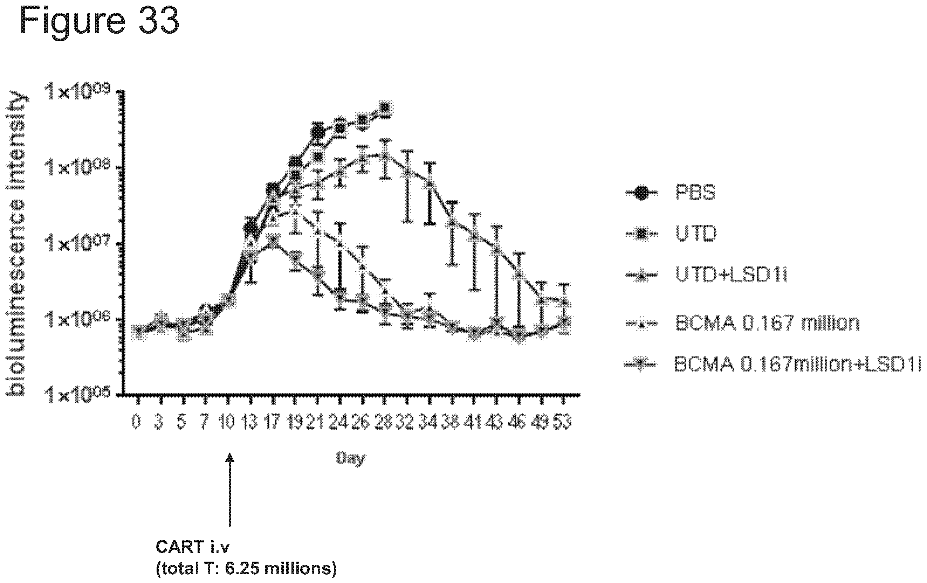

Untransduced T cells and BCMA-CART cells with or without ex vivo LSD1i treatment were injected once into mice bearing established luciferized KMS11 tumors. A Total 6.25 million T cells including 0.167 millions of total CAR+ cells were injected. Interestingly, we found that inhibition of LSD1 increases the anti-tumor activity of CAR negative T cells. Without being bound by theory, this activity may be the result of the increased fraction of Tscm cells as a result of LSD1 inhibition, which results in a population of cells with increased cytokine production and proliferative potential. As well, inhibition of LSD1 enhances the efficacy BCMA-CART cells in vivo. These data demonstrate that compound 93-treated untransduced T cells alone show anti-tumor activity in a slow regression mode over time. As well, this compound can also significantly enhance the antigen-specific anti-tumor function of BCMA CART cells in the acute phase comparing the regular BCMA-CART, while both CART cells (one with, one without compound 93) have durable effects on eliminating tumor growth (Figure 33).

This application is being filed with a sequence listing. To the extent there are any discrepancies between the sequence listing and any sequence recited in the specification, the sequence recited in the specification should be considered the correct sequence.

## Claims

1. A composition comprising a population of immune effector cells engineered to express a CAR for use, in combination with an LSD1 inhibitor, in a method of treating a disease associated with the expression of a tumor antigen in a subject, said method comprising administering to said subject an LSD1 inhibitor and a population of immune effector cells engineered to express a CAR, wherein the population of immune effector cells comprises, e.g., consists of, T cells, wherein the disease associated with expression of a tumor antigen is chosen from a proliferative disease, a precancerous condition, and a cancer.

2. The composition for use of claim 1, wherein:
a) the LSD1 inhibitor is administered before the subject is administered said population of immune effector cells;
b) the LSD1 inhibitor is administered concurrently with said population of immune effector cells;
c) the LSD1 inhibitor is administered after the subject is administered said population of immune effector cells; or
d) any combination of a), b) and c).

3. The composition for use of claim 2, wherein the LSD1 inhibitor is administered before the subject is administered said population of immune effector cells, and wherein said administration of the LSD1 inhibitor is continued for a period of time after the administration of said population of immune effector cells, optionally wherein the administration of the LSD1 inhibitor is in an amount sufficient to increase an anti-tumor effect of said population of immune effector cells relative to an equivalent population of said immune effector cells administered in the absence of said LSD1 inhibitor.

4. A composition comprising a population of immune effector cells engineered to express a CAR for use, in combination with an LSD1 inhibitor, in a method of increasing the therapeutic efficacy of a population of immune effector cells engineered to express a CAR, e.g., a CART19 (e.g., CTL019) in a subject having a disease associated with expression of a tumor antigen, said method comprising a step of decreasing the activity or expression of LSD1 in said cells, transiently or permanently, optionally wherein the step of decreasing the activity or expression of LSD1 in said cells comprises contacting the cells with an LSD1 inhibitor, wherein the population of immune effector cells comprises, e.g., consists of, T cells, wherein the disease associated with expression of a tumor antigen is chosen from a proliferative disease, a precancerous condition, and a cancer.

5. The composition for use of any of claims 1-4, wherein the administration or the contacting of the LSD1 inhibitor results in:
1) an increase in the proportion of naive T cells, e.g., Tscm cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14) an increase in the production of cytokines (e.g., IFNg and/or IL-2) from said population of immune effector cells; or
15) a combination of two or more of the above;
optionally as compared to cells not contacted with the LSD1 inhibitor.

6. A method of making a population of immune effector cells comprising:
a) contacting a population of immune effector cells with an LSD1 inhibitor, ex vivo thereby making a population of immune effector cells, wherein the population of immune effector cells comprises, e.g., consists of, T cells,
wherein the contacting with the LSD1 inhibitor causes one or more of the following to occur:
1) an increase in the proportion of naive T cells, e.g., T_{SCM} cells;
2) an increase in the number of naive T cells, e.g., T_{SCM} cells;
3) a decrease in the number of T_{EM} cells;
4) a decrease in the proportion of T_{EM} cells;
5) an increase in the proportion of CD45RA+CD62L+ T cells;
6) an increase in the number of CD45RA+CD62L+ T cells;
7) an increase in the proportion of CD45RA+CCR7+ T cells;
8) an increase in the number of CD45RA+CCR7+ T cells;
9) a decrease in the proportion of PD-1 positive immune effector cells;
10) an increase in the ratio of PD-1 negative immune effector cells / PD-1 positive immune effector cells;
11) a decrease in the proportion of PD-1+/Lag3+/Tim3+ immune effector cells;
12) an increase in the ratio of PD-1-/Lag3-/Tim3- immune effector cells to PD-1+/Lag3+/Tim3+ immune effector cells;
13) an increase in the proliferation of the immune effector cells;
14) an increase in the production of cytokines (e.g., IFNg and/or IL-2) from said population of immune effector cells; or
15) a combination of two or more of the above;
as compared to a non-contacted population of immune effector cells.

7. The method of claim 6, wherein the method further comprises the step of b) inserting nucleic acid that encodes the CAR into cells of the population of immune effector cells, optionally wherein said contacting of step a) occurs
1) prior to;
2) concurrently with;
3) after; or
4) any combination of two or more of 1) to 3) above;
said inserting of step b).

8. A population of immune effector cells, which is engineered to express a chimeric antigen receptor (CAR), wherein the CAR comprises an antigen-binding domain, a transmembrane domain, and an intracellular signaling domain, and wherein expression and/or function of LSD1 in said cell has been reduced or eliminated, wherein the population of immune effector cells further comprises an LSD1 inhibitor, wherein the population of immune effector cells comprises, e.g., consists of, T cells.

9. The composition for use of any of claims 1 to 5, the method of claim 7, or the population of immune effector cells of claim 8, wherein the CAR comprises an antigen binding domain (which is optionally an antibody or antibody fragment, TCR or TCR fragment), a transmembrane domain, and an intracellular signaling domain (which is optionally an intracellular signaling domain comprising a costimulatory domain and/or a primary signaling domain), optionally wherein the antigen-binding domain binds to a tumor antigen selected from a group consisting of: TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, PRSS21, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-la, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1, *e.g.* wherein the tumor antigen is CD19 or BCMA.

10. The composition for use, the method, or the population of immune effector cells of claim 9, wherein:
(a) the antigen-binding domain is an antibody or antibody fragment comprising:
(i) the amino acid sequence of a CD19 binding domain according to Tables 6-9, e.g., the amino acid sequence of CTL019 scFv domain according to Table 9 or an amino acid sequence according to SEQ ID NO: 957, or an amino acid sequence at least 95% identical thereto;
(ii) the amino acid sequence of a humanized CD19 binding domain according to Tables 6-9, e.g., the amino acid sequence of CAR2 scFv domain according to Table 9 or an amino acid sequence according to SEQ ID NO: 898, or an amino acid sequence at least 95% identical thereto; or
(iii) the amino acid sequence of a BCMA binding domain according to Tables 11A-11B, e.g., the amino acid sequence of 139109 scFv domain according to Table 11A or an amino acid sequence according to SEQ ID NO: 967, or an amino acid sequence at least 95% identical thereto; or
(b) the CAR comprises:
(i) the amino acid sequence of a CD19 CAR according to Tables 6-9, e.g., the amino acid sequence of CTL019 according to Table 9 or an amino acid sequence according to SEQ ID NO: 956 or an amino acid sequence at least 95% identical thereto;
(ii) the amino acid sequence of a humanized CD19 CAR according to Tables 6-9, e.g., the amino acid sequence of CAR2 according to Table 9 or an amino acid sequence according to SEQ ID NO: 902, or an amino acid sequence at least 95% identical thereto; or
(iii) the amino acid sequence of a BCMA CAR according to Tables 11A-11B, e.g., the amino acid sequence of 139109 CAR according to Table 11A or an amino acid sequence according to SEQ ID NO: 971, or an amino acid sequence at least 95% identical thereto.

11. The composition for use, the method, or the population of immune effector cells of claim 9 or 10, wherein:
(a) the transmembrane domain comprises:
(i) an amino acid sequence having at least one, two or three modifications but not more than 20, 10 or 5 modifications of an amino acid sequence of SEQ ID NO: 12, or a sequence with 95-99% identity to an amino acid sequence of SEQ ID NO: 12; or
(ii) the sequence of SEQ ID NO: 12; and/or
(b) the antigen binding domain is connected to the transmembrane domain by a hinge region, wherein said hinge region comprises SEQ ID NO: 2 or SEQ ID NO: 6, or a sequence with 95-99% identity thereto; and/or
(c) the CAR comprises a leader sequence comprising, e.g., consisting of, SEQ ID NO: 2.

12. The composition for use, the method, or the population of immune effector cells of any of claims 9-11, wherein:
(a) the intracellular signaling domain comprises a primary signaling domain and/or a costimulatory signaling domain, wherein the primary signaling domain comprises a functional signaling domain of a protein chosen from CD3 zeta, CD3 gamma, CD3 delta, CD3 epsilon, common FcR gamma (FCER1G), FcR beta (Fc Epsilon R1b), CD79a, CD79b, Fcgamma RIIa, DAP10, or DAP12; and/or
(b) the primary signaling domain comprises:
(i) an amino acid sequence having at least one, two or three modifications but not more than 20, 10 or 5 modifications of an amino acid sequence of SEQ ID NO: 18 or SEQ ID NO: 20, or a sequence with 95-99% identity to an amino acid sequence of SEQ ID NO: 18 or SEQ ID NO: 20; or
(ii) the amino acid sequence of SEQ ID NO: 18 or SEQ ID NO: 20; and/or
(c) the intracellular signaling domain comprises a costimulatory signaling domain, or a primary signaling domain and a costimulatory signaling domain, wherein the costimulatory signaling domain comprises a functional signaling domain of a protein selected from the group consisting of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, a ligand that specifically binds with CD83, CDS, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, and NKG2D; and/or
(d) the costimulatory signaling domain comprises an amino acid sequence having at least one, two or three modifications but not more than 20, 10 or 5 modifications of an amino acid sequence of SEQ ID NO: 14 or SEQ ID NO: 16, or a sequence with 95-99% identity to an amino acid sequence of SEQ ID NO: 14 or SEQ ID NO: 16; and/or
(e) the costimulatory signaling domain comprises a sequence of SEQ ID NO: 14 or SEQ ID NO: 16; and/or
(f) the intracellular domain comprises the sequence of SEQ ID NO: 14 or SEQ ID NO: 16, and the sequence of SEQ ID NO: 18 or SEQ ID NO: 20, wherein the sequences comprising the intracellular signaling domain are expressed in the same frame and as a single polypeptide chain.

13. The composition for use of any of claims 1-5 or 9-12, the method of any of claims 6, 7 or 9-12, or the population of immune effector cells of any of claims 8-12, wherein the LSD1 inhibitor is: (1) a gene editing system targeted to one or more sites of the LSD1 gene, or its corresponding regulatory elements; (2) a nucleic acid (e.g., an siRNA or shRNA, or antisense oligonucleotide) comprising sequence complementary to a target sequence of the LSD1 gene; (3) a protein (e.g., a dominant negative LSD1, e.g., catalytically inactive LSD1, or a dominant negative binding partner of LSD1); (4) a small molecule; (5) a nucleic acid encoding any of (1)-(3); or (6) any combination of (1) -(5),
optionally wherein:
(a) the LSD1 inhibitor is an shRNA or siRNA comprising a sequence complementary to a target sequence of the LSD1 gene listed in Table 1, e.g., selected from SEQ ID NO: 43 to SEQ ID NO: 82;
(b) the LSD1 inhibitor is an shRNA encoded by a nucleic acid comprising any sequence encoding an anti-LSD1 shRNA of Table 1, e.g., encoded by nucleic acid comprising a sequence selected from SEQ ID NO: 83 to SEQ ID NO: 122; or
(c) the LSD1 inhibitor is a nucleic acid comprising any sequence encoding an anti-LSD1 shRNA of Table 1, e.g., a sequence selected from SEQ ID NO: 83 to SEQ ID NO: 122.

14. The composition for use, the method, or the population of immune effector cells of claim 13, wherein said nucleic acid is disposed on a vector, optionally wherein the vector is a retroviral vector, a lentiviral vector, an adenoviral vector, an adeno-associated viral (AAV) vector, a herpes simplex virus (HSV) vector, a plasmid, a minicircle, a nanoplasmid, or an RNA vector, and/or wherein the vector further comprises a sequence encoding a CAR.

15. The composition for use, the method, or the population of immune effector cells of claim 13, wherein the LSD1 inhibitor is a genome editing system specific for a sequence of the LSD1 gene (KDM1A) or its regulatory elements selected from a CRISPR genome editing system, a zinc finger nuclease genome editing system, a TALEN genome editing system and a meganuclease genome editing system, optionally wherein the LSD1 inhibitor is a CRISPR genome editing system comprising a gRNA molecule comprising a targeting domain complementary to a sequence of the LSD1 gene (KDM1A) or its regulatory elements, e.g., comprising any one of SEQ ID NO: 132 to 862.

16. The composition for use, the method, or the population of immune effector cells of claim 13, wherein the LSD1 inhibitor is a small molecule, further wherein the small molecule is a reversible LSD1 inhibitor or an irreversible LSD1 inhibitor, optionally wherein the LSD1 inhibitor is:
a) GSK2699537;
b) rel- 2-[[(1R,2S)-2-[4-[(4-chlorophenyl)methoxy]phenyl]cyclopropyl]amino]-1-(4-methyl-1-piperazinyl)-ethanone;
c) (R)-4-(5-(pyrrolidin-3-ylmethoxy)-2-(p-tolyl)pyridin-3-yl)benzonitrile;
d) (1S,2R)-N-((2-methoxypyridin-3-yl)methyl)-2-phenylcyclopropan-1-amine;
e) N,N-dimethyl-1-((4-(4-(4-(piperidin-4-yl)phenyl)-1H-indazol-1-yl)phenyl)sulfonyl)piperidin-4-amine;
f) 5-(6-chloro-4'-(methylsulfonyl)-[1,1'-biphenyl]-3-yl)-2-(piperazin-1-yl)-1H-pyrrole-3-carbonitrile;
g) rel- N-[(1R,2S)-2-Phenylcyclopropyl]-4-Piperidinamine;
h) 2-(1R,2S)-2-(4-(Benzyloxy)phenyl)cyclopropylamino)-1-(4-methylpiperazin-1-yl)ethanone;
i) Trans-3-(3-amino-2-methylphenyl)-1-(4-hydroxycyclohexyl)-6-methyl-1H-indole-5-carbonitrile; or
j) a pharmaceutically acceptable salt of any of the foregoing.

17. The composition for use, the method, or the population of immune effector cells of claim 13, wherein:
(a) the LSD1 inhibitor is a protein, e.g., is a dominant negative binding partner of LSD1 (e.g., a histone deacetylase (HDAC) that interacts with LSD1 or other member of the Co-REST or AR co-activator complex), or nucleic acid encoding said dominant negative binding partner of LSD1; or
(b) the inhibitor of LSD1 is a protein, e.g., is a dominant negative (e.g., catalytically inactive) LSD1, or nucleic acid encoding said dominant negative LSD1.

18. A population of immune effector cells of claim 8 for use in a method of treating a disease associated with the expression of a tumor antigen in a subject, said method comprising administering to said subject an effective amount of the population of immune effector cells, optionally wherein:
(a) the method further comprises administering to said subject an LSD1 inhibitor;
(b) the subject receives a pre-treatment of an LSD1 inhibitor, prior to the administration of the population of immune effector cells;
(c) the subject receives concurrent treatment with an LSD1 inhibitor and the population of immune effector cells; and/or
(d) the subject receives treatment with an LSD1 inhibitor after administration of the population of immune effector cells;
wherein the disease associated with expression of a tumor antigen is chosen from a proliferative disease, a precancerous condition, and a cancer.

19. The composition for use of any of claims 1-5 or 9-17, or the population of immune effector cells for use of claim 19, wherein the cancer is:
(i) a hematologic cancer chosen from one or more of chronic lymphocytic leukemia (CLL), acute leukemias, acute lymphoid leukemia (ALL), acute myeloid leukemia (AML), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, or pre-leukemia, or
(ii) selected from the group consisting of colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.

20. The composition for use of any of claims 1-5, 9-17 or 19, or the population of immune effector cells for use of claim 18, wherein the subject is a human.

21. A method of manufacturing a population of immune effector cells comprising introducing into said cells nucleic acid encoding a CAR, wherein the nucleic acid integrates into the genome of said cell within the LSD1 gene, such that LSD1 expression and/or function is reduced, wherein the population of immune effector cells comprises, e.g., consists of, T cells.

## Patentansprüche

1. Zusammensetzung, umfassend eine Population von zum Exprimieren eines CAR manipulierten Immuneffektorzellen, zur Verwendung, in Kombination mit einem LSD1-Inhibitor, in einem Verfahren zur Behandlung einer mit der Expression eines Tumorantigens assoziierten Krankheit in einem Subjekt, wobei das Verfahren die Verabreichung eines LSD1-Inhibitors und einer Population von zum Exprimieren eines CAR manipulierten Immuneffektorzellen an das Subjekt umfasst, wobei die Population von Immuneffektorzellen T-Zellen umfasst, z. B. daraus besteht, wobei die mit der Expression eines Tumorantigens assoziierte Krankheit aus einer proliferativen Krankheit, einem präkanzerösen Zustand und einem Krebs ausgewählt ist.

2. Zusammensetzung zur Verwendung nach Anspruch 1, wobei:
a) der LSD1-Inhibitor verabreicht wird, bevor dem Subjekt die Population von Immuneffektorzellen verabreicht wird;
b) der LSD1-Inhibitor gleichzeitig mit der Population von Immuneffektorzellen verabreicht wird;
c) der LSD1-Inhibitor verabreicht wird, nachdem dem Subjekt die Population von Immuneffektorzellen verabreicht wurde; oder
d) jedwede Kombination von a), b) und c).

3. Zusammensetzung zur Verwendung nach Anspruch 2, wobei der LSD1-Inhibitor verabreicht wird, bevor dem Subjekt die Population von Immuneffektorzellen verabreicht wird, und wobei die Verabreichung des LSD1-Inhibitors für einen Zeitraum nach der Verabreichung der Population von Immuneffektorzellen fortgesetzt wird, wobei optional die Verabreichung des LSD1-Inhibitors in einer Menge erfolgt, die ausreicht, um eine Antitumorwirkung der Population von Immuneffektorzellen relativ zu einer äquivalenten Population der Immuneffektorzellen, die in Abwesenheit des LSD1-Inhibitors verabreicht wird, zu erhöhen.

4. Zusammensetzung, umfassend eine Population von zum Exprimieren eines CAR manipulierten Immuneffektorzellen, zur Verwendung, in Kombination mit einem LSD1-Inhibitor, in einem Verfahren zur Erhöhung der therapeutischen Wirksamkeit einer Population von zum Exprimieren eines CAR, z. B. eines CART19 (z. B. CTL019), manipulierten Immuneffektorzellen in einem Subjekt, das eine mit der Expression eines Tumorantigens assoziierte Krankheit aufweist, wobei das Verfahren einen Schritt des vorübergehenden oder dauerhaften Verringerns der Aktivität oder Expression von LSD1 in den Zellen umfasst, wobei optional der Schritt des Verringerns der Aktivität oder Expression von LSD1 in den Zellen das Inkontaktbringen der Zellen mit einem LSD1-Inhibitor umfasst, wobei die Population von Immuneffektorzellen T-Zellen umfasst, z. B. daraus besteht, wobei die mit der Expression eines Tumorantigens assoziierte Krankheit aus einer proliferativen Krankheit, einem präkanzerösen Zustand und einem Krebs ausgewählt ist.

5. Zusammensetzung zur Verwendung nach einem der Ansprüche 1-4, wobei die Verabreichung oder das Inkontaktbringen des LSD1-Inhibitors Folgendes bewirkt:
1) eine Erhöhung des Anteils naiver T-Zellen, z. B. T_{SCM}-Zellen;
2) eine Erhöhung der Anzahl naiver T-Zellen, z. B. T_{SCM}-Zellen;
3) eine Abnahme der Anzahl von T_{EM}-Zellen;
4) eine Abnahme des Anteils an T_{EM}-Zellen;
5) eine Erhöhung des Anteils an CD45RA⁺CD62L⁺-T-Zellen;
6) eine Erhöhung der Anzahl von CD45RA⁺CD62L⁺-T-Zellen;
7) eine Erhöhung des Anteils an CD45RA⁺CCR7⁺-T-Zellen;
8) eine Erhöhung der Anzahl von CD45RA⁺CCR7⁺-T-Zellen;
9) eine Abnahme des Anteils an PD-1-positiven Immuneffektorzellen;
10) eine Erhöhung des Verhältnisses von PD-1-negativen Immuneffektorzellen / PD-1-positiven Immuneffektorzellen;
11) eine Abnahme des Anteils von PD-1⁺/Lag3⁺/Tim3⁺-Immuneffektorzellen;
12) eine Erhöhung des Verhältnisses von PD-1⁻/Lag3⁻/Tim3⁻-Immuneffektorzellen zu PD-1⁺/Lag3⁺/Tim3⁺-Immuneffektorzellen;
13) eine Erhöhung der Proliferation der Immuneffektorzellen;
14) eine Erhöhung der Produktion von Zytokinen (z. B. IFNg und/oder IL-2) aus der Population von Immuneffektorzellen; oder
15) eine Kombination aus zwei oder mehr der oben genannten;
optional im Vergleich zu Zellen, die nicht mit dem LSD1-Inhibitor in Kontakt gebracht wurden.

6. Verfahren zur Herstellung einer Population von Immuneffektorzellen, umfassend:
a) Inkontaktbringen einer Population von Immuneffektorzellen mit einem LSD1-Inhibitor ex vivo, wodurch eine Population von Immuneffektorzellen hergestellt wird, wobei die Population von Immuneffektorzellen T-Zellen umfasst, z. B. daraus besteht,
wobei das Inkontaktbringen mit dem LSD1-Inhibitor das Auftreten eines oder mehrerer der folgenden Fälle verursacht:
1) eine Erhöhung des Anteils naiver T-Zellen, z. B. T_{SCM}-Zellen;
2) eine Erhöhung der Anzahl naiver T-Zellen, z. B. T_{SCM}-Zellen;
3) eine Abnahme der Anzahl von T_{EM}-Zellen;
4) eine Abnahme des Anteils an T_{EM}-Zellen;
5) eine Erhöhung des Anteils an CD45RA⁺CD62L⁺-T-Zellen;
6) eine Erhöhung der Anzahl von CD45RA⁺CD62L⁺-T-Zellen;
7) eine Erhöhung des Anteils an CD45RA⁺CCR7⁺-T-Zellen;
8) eine Erhöhung der Anzahl von CD45RA⁺CCR7⁺-T-Zellen;
9) eine Abnahme des Anteils an PD-1-positiven Immuneffektorzellen;
10) eine Erhöhung des Verhältnisses von PD-1-negativen Immuneffektorzellen / PD-1-positiven Immuneffektorzellen;
11) eine Abnahme des Anteils von PD-1⁺/Lag3⁺/Tim3⁺-Immuneffektorzellen;
12) eine Erhöhung des Verhältnisses von PD-1⁻/Lag3⁻/Tim3--Immuneffektorzellen zu PD-1⁺/Lag3⁺/Tim3⁺-Immuneffektorzellen;
13) eine Erhöhung der Proliferation der Immuneffektorzellen;
14) eine Erhöhung der Produktion von Zytokinen (z. B. IFNg und/oder IL-2) aus der Population von Immuneffektorzellen; oder
15) eine Kombination aus zwei oder mehr der oben genannten;
im Vergleich zu einer nicht in Kontakt gebrachten Population von Immuneffektorzellen.

7. Verfahren nach Anspruch 6, wobei das Verfahren ferner den Schritt b) des Einbringens von Nukleinsäure, die den CAR codiert, in Zellen der Population von Immuneffektorzellen umfasst, wobei optional das Inkontaktbringen von Schritt a)
1) vor;
2) gleichzeitig mit;
3) nach; oder
4) in jedweder Kombination von zwei oder mehreren von obigen 1) bis 3); dem Einbringen von Schritt b) erfolgt.

8. Population von Immuneffektorzellen, die manipuliert ist, um einen chimären Antigenrezeptor (CAR) zu exprimieren, wobei der CAR eine Antigenbindungsdomäne, eine Transmembrandomäne und eine intrazelluläre Signalisierungsdomäne umfasst, und wobei die Expression und/oder Funktion von LSD1 in der Zelle reduziert oder eliminiert wurde, wobei die Population von Immuneffektorzellen ferner einen LSD1-Inhibitor umfasst, wobei die Population von Immuneffektorzellen T-Zellen umfasst, z. B. daraus besteht.

9. Zusammensetzung zur Verwendung nach einem der Ansprüche 1 bis 5, Verfahren nach Anspruch 7 oder Population von Immuneffektorzellen nach Anspruch 8, wobei der CAR eine Antigenbindungsdomäne (die optional ein Antikörper oder Antikörperfragment, TCR oder TCR-Fragment ist), eine Transmembrandomäne und eine intrazelluläre Signalisierungsdomäne (die optional eine intrazelluläre Signalisierungsdomäne, umfassend eine costimulatorische Domäne und/oder eine primäre Signalisierungsdomäne, ist) umfasst, wobei optional die Antigenbindungsdomäne an ein Tumorantigen bindet, ausgewählt aus einer Gruppe bestehend aus: TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-llRa, PSCA, PRSS21, VEGFR2, LewisY, CD24, PDGFR-beta, SSEA-4, CD20, Folatrezeptor alpha, ERBB2 (Her2/neu), MUC1, EGFR, NCAM, Prostase, PAP, ELF2M, Ephrin B2, IGF-I-Rezeptor, CAIX, LMP2, gp100, bcr-abl, Tyrosinase, EphA2, Fucosyl-GM1, sLe, GM3, TGS5, HMWMAA, o-Acetyl-GD2, Folatrezeptor beta, TEM1/CD248, TEM7R, CLDN6, GPRC5D, CXORF61, CD97, CD179a, ALK, Polysialinsäure, PLAC1, GloboH, NY-BR-1, UPK2, HAVCR1, ADRB3, PANX3, GPR20, LY6K, OR51E2, TARP, WT1, NY-ESO-1, LAGE-la, MAGE-A1, Legumain, HPV E6,E7, MAGE Al, ETV6-AML, Spermien-Protein 17, XAGE1, Tie 2, MAD-CT-1, MAD-CT-2, Fos-related Antigen 1, p53, p53-Mutante, Prostein, Survivin und Telomerase, PCTA-1/Galectin 8, MelanA/MART1, Ras-Mutante, hTERT, Sarkom-Translokations-Breakpoints, ML-IAP, ERG (TMPRSS2-ETS-Fusionsgen), NA17, PAX3, Androgenrezeptor, Cyclin B1, MYCN, RhoC, TRP-2, CYP1B1, BORIS, SART3, PAX5, OY-TES1, LCK, AKAP-4, SSX2, RAGE-1, humane Telomerase-Reverse-Transkriptase, RU1, RU2, intestinale Carboxylesterase, mut hsp70-2, CD79a, CD79b, CD72, LAIR1, FCAR, LILRA2, CD300LF, CLEC12A, BST2, EMR2, LY75, GPC3, FCRL5 und IGLL1, z. B. wobei das Tumorantigen CD19 oder BCMA ist.

10. Zusammensetzung zur Verwendung, Verfahren oder Population von Immuneffektorzellen nach Anspruch 9, wobei:
(a) die Antigenbindungsdomäne ein Antikörper oder Antikörperfragment ist, umfassend:
(i) die Aminosäuresequenz einer CD19-Bindungsdomäne gemäß den Tabellen 6-9, z. B. die Aminosäuresequenz der CTL019-scFv-Domäne gemäß Tabelle 9 oder eine Aminosäuresequenz gemäß SEQ ID NO: 957, oder eine Aminosäuresequenz, die mindestens 95% identisch dazu ist;
(ii) die Aminosäuresequenz einer humanisierten CD19-Bindungsdomäne gemäß den Tabellen 6-9, z. B. die Aminosäuresequenz der CAR2-scFv-Domäne gemäß Tabelle 9 oder eine Aminosäuresequenz gemäß SEQ ID NO: 898, oder eine Aminosäuresequenz, die mindestens 95% identisch dazu ist; oder
(iii) die Aminosäuresequenz einer BCMA-Bindungsdomäne gemäß den Tabellen 11A-11B, z. B. die Aminosäuresequenz der 139109-scFv-Domäne gemäß Tabelle 11A oder eine Aminosäuresequenz gemäß SEQ ID NO: 967, oder eine Aminosäuresequenz, die mindestens 95% identisch dazu ist; oder
(b) der CAR umfasst:
(i) die Aminosäuresequenz eines CD19-CAR gemäß den Tabellen 6-9, z. B. die Aminosäuresequenz von CTL019 gemäß Tabelle 9 oder eine Aminosäuresequenz gemäß SEQ ID NO: 956, oder eine Aminosäuresequenz, die mindestens 95% identisch dazu ist;
(ii) die Aminosäuresequenz eines humanisierten CD19-CAR gemäß den Tabellen 6-9, z. B. die Aminosäuresequenz von CAR2 gemäß Tabelle 9 oder eine Aminosäuresequenz gemäß SEQ ID NO: 902, oder eine Aminosäuresequenz, die mindestens 95% identisch dazu ist; oder
(iii) die Aminosäuresequenz eines BCMA-CAR gemäß den Tabellen 11A-11B, z. B. die Aminosäuresequenz von 139109-CAR gemäß Tabelle 11A oder eine Aminosäuresequenz gemäß SEQ ID NO: 971, oder eine Aminosäuresequenz, die mindestens 95% identisch dazu ist.

11. Zusammensetzung zur Verwendung, Verfahren oder Population von Immuneffektorzellen nach Anspruch 9 oder 10, wobei:
(a) die Transmembrandomäne umfasst:
(i) eine Aminosäuresequenz mit mindestens einer, zwei oder drei Modifikationen, aber nicht mehr als 20, 10 oder 5 Modifikationen einer Aminosäuresequenz von SEQ ID NO: 12, oder eine Sequenz mit 95-99% Identität zu einer Aminosäuresequenz von SEQ ID NO: 12; oder
(ii) die Sequenz von SEQ ID NO: 12; und/oder
(b) die Antigenbindungsdomäne mit der Transmembrandomäne durch eine Scharnier-Region verbunden ist, wobei die Scharnier-Region SEQ ID NO: 2 oder SEQ ID NO: 6 oder eine Sequenz mit 95-99% Identität dazu umfasst; und/oder
(c) der CAR eine Leader-Sequenz umfasst, umfassend, z. B. bestehend aus, SEQ ID NO: 2.

12. Zusammensetzung zur Verwendung, Verfahren oder Population von Immuneffektorzellen nach einem der Ansprüche 9-11, wobei:
(a) die intrazelluläre Signalisierungsdomäne eine primäre Signalisierungsdomäne und/oder eine costimulatorische Signalisierungsdomäne umfasst, wobei die primäre Signalisierungsdomäne eine funktionelle Signalisierungsdomäne eines Proteins, ausgewählt aus CD3 zeta, CD3 gamma, CD3 delta, CD3 epsilon, "common FcR gamma" (FCER1G), FcR beta (Fc Epsilon R1b), CD79a, CD79b, Fcgamma RIIa, DAP10 oder DAP12 umfasst; und/oder
(b) die primäre Signalisierungsdomäne umfasst:
(i) eine Aminosäuresequenz mit mindestens einer, zwei oder drei Modifikationen, aber nicht mehr als 20, 10 oder 5 Modifikationen einer Aminosäuresequenz von SEQ ID NO: 18 oder SEQ ID NO: 20, oder eine Sequenz mit 95-99% Identität zu einer Aminosäuresequenz von SEQ ID NO: 18 oder SEQ ID NO: 20; oder
(ii) die Aminosäuresequenz von SEQ ID NO: 18 oder SEQ ID NO: 20; und/oder
(c) die intrazelluläre Signalisierungsdomäne eine costimulatorische Signalisierungsdomäne oder eine primäre Signalisierungsdomäne und eine costimulatorische Signalisierungsdomäne umfasst, wobei die costimulatorische Signalisierungsdomäne eine funktionelle Signalisierungsdomäne eines Proteins umfasst, ausgewählt aus der Gruppe bestehend aus CD27, CD28, 4-1BB (CD137), OX40, CD30, CD40, PD-1, ICOS, Lymphozytenfunktionsassoziiertes Antigen-1 (LFA-1), CD2, CD7, LIGHT, NKG2C, B7-H3, einem Ligand, der spezifisch bindet mit CD83, CDS, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46 und NKG2D; und/oder
(d) die costimulatorische Signalisierungsdomäne eine Aminosäuresequenz mit mindestens einer, zwei oder drei Modifikationen, aber nicht mehr als 20, 10 oder 5 Modifikationen einer Aminosäuresequenz von SEQ ID NO: 14 oder SEQ ID NO: 16, oder eine Sequenz mit 95-99% Identität zu einer Aminosäuresequenz von SEQ ID NO: 14 oder SEQ ID NO: 16 umfasst; und/oder
(e) die costimulatorische Signalisierungsdomäne eine Sequenz von SEQ ID NO: 14 oder SEQ ID NO: 16 umfasst; und/oder
(f) die intrazelluläre Domäne die Sequenz von SEQ ID NO: 14 oder SEQ ID NO: 16 und die Sequenz von SEQ ID NO: 18 oder SEQ ID NO: 20 umfasst, wobei die Sequenzen, die die intrazelluläre Signalisierungsdomäne umfassen, im selben Leserahmen und als Einzel-Polypeptidkette exprimiert werden.

13. Zusammensetzung zur Verwendung nach einem der Ansprüche 1-5 oder 9-12, Verfahren nach einem der Ansprüche 6, 7 oder 9-12 oder Population von Immuneffektorzellen nach einem der Ansprüche 8-12, wobei es sich bei dem LSD1-Inhibitor handelt um: (1) ein Gen-Editierungssystem, das auf eine oder mehrere Stellen des LSD1-Gens oder seiner entsprechenden regulatorischen Elemente abzielt; (2) eine Nukleinsäure (z. B. eine siRNA oder shRNA oder ein Antisense-Oligonukleotid), die eine Sequenz umfasst, die komplementär zu einer Zielsequenz des LSD1-Gens ist; (3) ein Protein (z. B. eine dominant-negative LSD1, z. B. katalytisch inaktive LSD1, oder ein dominant-negativer Bindungspartner von LSD1); (4) ein kleines Molekül; (5) eine Nukleinsäure, die eines von (1)-(3) codiert; oder (6) jedwede Kombination von (1)-(5), wobei optional:
(a) der LSD1-Inhibitor eine shRNA oder siRNA ist, umfassend eine Sequenz, die komplementär zu einer Zielsequenz des in Tabelle 1 aufgeführten LSD1-Gens ist, z. B. ausgewählt aus SEQ ID NO: 43 bis SEQ ID NO: 82;
(b) der LSD1-Inhibitor eine shRNA ist, die von einer Nukleinsäure codiert wird, umfassend eine beliebige Sequenz, die eine Anti-LSD1-shRNA von Tabelle 1 codiert, z. B. codiert von einer Nukleinsäure, die eine Sequenz, ausgewählt aus SEQ ID NO: 83 bis SEQ ID NO: 122, umfasst; oder
(c) der LSD1-Inhibitor eine Nukleinsäure ist, umfassend eine beliebige Sequenz, die eine Anti-LSD1-shRNA von Tabelle 1 codiert, z. B. eine Sequenz, ausgewählt aus SEQ ID NO: 83 bis SEQ ID NO: 122.

14. Zusammensetzung zur Verwendung, Verfahren oder Population von Immuneffektorzellen nach Anspruch 13, wobei die Nukleinsäure auf einem Vektor angeordnet ist, wobei der Vektor optional ein retroviraler Vektor, ein lentiviraler Vektor, ein adenoviraler Vektor, ein Adeno-assoziierter-viraler (AAV) Vektor, ein Herpessimplex-Virus (HSV)-Vektor, ein Plasmid, ein Minicircle, ein Nanoplasmid oder ein RNA-Vektor ist, und/oder wobei der Vektor ferner eine Sequenz umfasst, die einen CAR codiert.

15. Zusammensetzung zur Verwendung, Verfahren oder Population von Immuneffektorzellen nach Anspruch 13, wobei der LSD1-Inhibitor ein für eine Sequenz des LSD1-Gens (KDM1A) oder seiner regulatorischen Elemente spezifisches Genom-Editierungssystem ist, ausgewählt aus einem CRISPR-Genom-Editierungssystem, einem Zinkfinger-Nuklease-Genom-Editierungssystem, einem TALEN-Genom-Editierungssystem und einem Meganuklease-Genom-Editierungssystem, wobei optional der LSD1-Inhibitor ein CRISPR-Genom-Editierungssystem ist, umfassend ein gRNA-Molekül, das eine Targeting-Domäne umfasst, die komplementär zu einer Sequenz des LSD1-Gens (KDM1A) oder seiner regulatorischen Elemente ist, z. B. umfassend eine der SEQ ID NO: 132 bis 862.

16. Zusammensetzung zur Verwendung, Verfahren oder Population von Immuneffektorzellen nach Anspruch 13, wobei der LSD1-Inhibitor ein kleines Molekül ist, wobei ferner das kleine Molekül ein reversibler LSD1-Inhibitor oder ein irreversibler LSD1-Inhibitor ist, wobei es sich optional bei dem LSD1-Inhibitor handelt um:
a) GSK2699537;
b) rel-2-[[(lR,2S)-2-[4-[(4-Chlorphenyl)methoxy]phenyl]cyclopropyl]amino]-1-(4-methyl-1-piperazinyl)-ethanon;
c) (R)-4-(5-(Pyrrolidin-3-ylmethoxy)-2-(p-tolyl)pyridin-3-yl)benzonitril;
d) (1S,2R)-N-((2-Methoxypyridin-3-yl)methyl)-2-phenylcyclopropan-1-amin;
e) N,N-Dimethyl-1-((4-(4-(4-(piperidin-4-yl)phenyl)-1H-indazol-1-yl)phenyl)sulfonyl)piperidin-4-amin;
f) 5-(6-Chlor-4'-(methylsulfonyl)-[1,1'-biphenyl]-3-yl)-2-(piperazin-1-yl)-1H-pyrrol-3-carbonitril;
g) rel-N-[(1R,2S)-2-Phenylcyclopropyl]-4-piperidinamin;
h) 2-(1R,2S)-2-(4-(Benzyloxy)phenyl)cyclopropylamino)-1-(4-methylpiperazin-1-yl)ethanon;
i) trans-3-(3-Amino-2-methylphenyl)-1-(4-hydroxycyclohexyl)-6-methyl-1H-indol-5-carbonitril; oder
j) ein pharmazeutisch akzeptables Salz von einem der vorstehenden.

17. Zusammensetzung zur Verwendung, Verfahren oder Population von Immuneffektorzellen nach Anspruch 13, wobei:
(a) der LSD1-Inhibitor ein Protein, z. B. ein dominant-negativer Bindungspartner von LSD1 (z. B. eine Histon-Deacetylase (HDAC), die mit LSD1 oder einem anderen Mitglied des Co-REST- oder AR-Co-Aktivatorkomplexes interagiert), oder Nukleinsäure, die den dominant-negativen Bindungspartner von LSD1 codiert, ist; oder
(b) der Inhibitor von LSD1 ein Protein ist, z. B. ein dominant-negatives (z. B. katalytisch inaktives) LSD1 ist, oder eine Nukleinsäure, die das dominant-negative LSD1 codiert, ist.

18. Population von Immuneffektorzellen nach Anspruch 8 zur Verwendung in einem Verfahren zum Behandeln einer mit der Expression eines Tumorantigens assoziierten Krankheit in einem Subjekt, wobei das Verfahren das Verabreichen einer wirksamen Menge der Population von Immuneffektorzellen an das Subjekt umfasst, wobei optional:
(a) das Verfahren ferner das Verabreichen eines LSD1-Inhibitors an das Subjekt umfasst;
(b) das Subjekt vor der Verabreichung der Population von Immuneffektorzellen eine Vorbehandlung mit einem LSD1-Inhibitor erhält;
(c) das Subjekt eine gleichzeitige Behandlung mit einem LSD1-Inhibitor und der Population von Immuneffektorzellen erhält; und/oder
(d) das Subjekt eine Behandlung mit einem LSD1-Inhibitor nach Verabreichung der Population von Immuneffektorzellen erhält;
wobei die mit der Expression eines Tumorantigens assoziierte Krankheit aus einer proliferativen Krankheit, einem präkanzerösen Zustand und einem Krebs ausgewählt ist.

19. Zusammensetzung zur Verwendung nach einem der Ansprüche 1-5 oder 9-17 oder Population von Immuneffektorzellen zur Verwendung nach Anspruch 19, wobei der Krebs:
(i) ein hämatologischer Krebs ist, ausgewählt aus einem oder mehreren von chronischer lymphatischer Leukämie (CLL), akuten Leukämien, akuter lymphatischer Leukämie (ALL), akuter myeloischer Leukämie (AML), akuter lymphatischer B-Zell-Leukämie (B-ALL), akuter lymphatischer T-Zell-Leukämie (T-ALL), chronischer myeloischer Leukämie (CML), prolymphozytischer B-Zell-Leukämie, blastischem plasmazytoidem dendritischem Zell-Neoplasma, Burkitt-Lymphom, diffusem großzelligem B-Zell-Lymphom, follikulärem Lymphom, Haarzell-Leukämie, kleinzelligem oder großzelligem follikulärem Lymphom, malignen lymphoproliferativen Erkrankungen, MALT-Lymphom, Mantelzell-Lymphom, Marginalzonen-Lymphom, multiplem Myelom, Myelodysplasie und myelodysplastischem Syndrom, Non-Hodgkin-Lymphom, Hodgkin-Lymphom, plasmablastischem Lymphom, plasmazytoidem dendritischem Zell-Neoplasma, Waldenström-Makroglobulinämie oder Prä-Leukämie, oder
(ii) ausgewählt ist aus der Gruppe bestehend aus Kolonkrebs, Rektalkrebs, Nierenzellkarzinom, Leberkrebs, nicht-kleinzelligem Lungenkarzinom, Dünndarmkrebs, Speiseröhrenkrebs, Melanom, Knochenkrebs, Bauchspeicheldrüsenkrebs, Hautkrebs, Kopf- oder Halskrebs, kutanem oder intraokularem malignen Melanom, Gebärmutterkrebs, Eierstockkrebs, Rektalkrebs, Krebs der Analregion, Magenkrebs, Hodenkrebs, Gebärmutterkrebs, Karzinom der Eileiter, Karzinom der Gebärmutterschleimhaut, Karzinom des Gebärmutterhalses, Karzinom der Vagina, Karzinom der Vulva, Morbus Hodgkin, Non-Hodgkin-Lymphom, Krebs des endokrinen Systems, Krebs der Schilddrüse, Krebs der Nebenschilddrüse, Krebs der Nebenniere, Weichgewebe-Sarkom, Harnröhrenkrebs, Peniskrebs, solide Tumore im Kindesalter, Blasenkrebs, Nieren- oder Harnleiterkrebs, Nierenbeckenkarzinom, Neoplasma des Zentralnervensystems (ZNS), primäres ZNS-Lymphom, Tumorangiogenese, Spinalachsentumor, Hirnstammgliom, Hypophysenadenom, Kaposi-Sarkom, Epidermoidkrebs, Plattenepithelkarzinom, T-Zell-Lymphom, umweltinduzierte Krebsarten, Kombinationen dieser Krebsarten und metastatische Läsionen dieser Krebsarten.

20. Zusammensetzung zur Verwendung nach einem der Ansprüche 1-5, 9-17 oder 19 oder Population von Immuneffektorzellen zur Verwendung nach Anspruch 18, wobei das Subjekt ein Mensch ist.

21. Verfahren zur Herstellung einer Population von Immuneffektorzellen, umfassend das Einbringen von Nukleinsäure, die einen CAR codiert, in die Zellen, wobei die Nukleinsäure in das Genom der Zelle innerhalb des LSD1-Gens integriert wird, so dass die LSD1-Expression und/oder -Funktion verringert wird, wobei die Population von Immuneffektorzellen T-Zellen umfasst, z. B. daraus besteht.

## Revendications

1. Composition comprenant une population de cellules effectrices immunitaires modifiées pour exprimer un CAR pour une utilisation, en combinaison avec un inhibiteur de LSD1, dans un procédé de traitement d'une maladie associée à l'expression d'un antigène tumoral chez un sujet, ledit procédé comprenant l'administration audit sujet d'un inhibiteur de LSD1 et d'une population de cellules effectrices immunitaires modifiées pour exprimer un CAR, dans laquelle la population de cellules effectrices immunitaires comprend, par ex., des cellules T, dans laquelle la maladie associée à l'expression d'un antigène tumoral est choisie parmi une maladie proliférative, un état précancéreux et un cancer.

2. Composition pour une utilisation selon la revendication 1, dans laquelle:
a) l'inhibiteur de LSD1 est administré avant que le sujet ne reçoive ladite population de cellules effectrices immunitaires;
b) l'inhibiteur de LSD1 est administré en même temps que ladite population de cellules effectrices immunitaires;
c) l'inhibiteur de LSD1 est administré après l'administration au sujet de ladite population de cellules effectrices immunitaires; ou
d) toute combinaison de a), b) et c).

3. Composition pour une utilisation selon la revendication 2, dans laquelle l'inhibiteur de LSD1 est administré avant que le sujet ne reçoive ladite population de cellules effectrices immunitaires, et dans laquelle ladite administration de l'inhibiteur de LSD1 est poursuivie pendant une période de temps après l'administration de ladite population de cellules effectrices immunitaires, éventuellement dans laquelle l'administration de l'inhibiteur de LSD1 est en une quantité suffisante pour augmenter un effet antitumoral de ladite population de cellules effectrices immunitaires par rapport à une population équivalente desdites cellules effectrices immunitaires administrée en l'absence dudit inhibiteur de LSD1.

4. Composition comprenant une population de cellules effectrices immunitaires modifiées pour exprimer un CAR pour une utilisation, en combinaison avec un inhibiteur de LSD1, dans un procédé d'augmentation de l'efficacité thérapeutique d'une population de cellules effectrices immunitaires modifiées pour exprimer un CAR, par ex., un CART19 (par ex., CTL019) chez un sujet ayant une maladie associée avec expression d'un antigène tumoral, ledit procédé comprenant une étape de diminution de l'activité ou de l'expression de LSD1 dans lesdites cellules, de manière transitoire ou permanente, éventuellement dans laquelle l'étape de diminution de l'activité ou de l'expression de LSD1 dans lesdites cellules comprend la mise en contact des cellules avec un inhibiteur de LSD1, dans laquelle la population de cellules effectrices immunitaires comprend, par ex. est constituée, de(s) cellules T, dans lequel la maladie associée à l'expression d'un antigène tumoral est choisie parmi une maladie proliférative, un état précancéreux, et un cancer.

5. Composition pour une utilisation selon l'une quelconque des revendications 1 à 4, dans laquelle l'administration ou la mise en contact de l'inhibiteur de LSD1 a pour résultat:
1) une augmentation de la proportion de cellules T naïves, par ex. les cellules T_{SCM};
2) une augmentation du nombre de cellules T naïves, par ex. les cellules T_{SCM};
3) une diminution du nombre de cellules T_{EM};
4) une diminution de la proportion de cellules T_{EM};
5) une augmentation de la proportion de cellules T CD45RA+CD62L+;
6) une augmentation du nombre de cellules T CD45RA+CD62L+;
7) une augmentation de la proportion de cellules T CD45RA+CCR7+;
8) une augmentation du nombre de cellules T CD45RA+CCR7+;
9) une diminution de la proportion de cellules effectrices immunitaires positives PD-1 positives;
10) une augmentation du rapport entre les cellules effectrices immunitaires négatives PD-1 et les cellules effectrices immunitaires positives PD-1;
11) une diminution de la proportion de cellules effectrices immunitaires PD-1 +/Lag3+/Tim3+;
12) une augmentation du rapport de cellules effectrices immunitaires PD-1-/Lag3-/Tim3- sur cellules effectrices immunitaires PD-1+/Lag3+/Tim3+;
13) une augmentation de la prolifération des cellules effectrices du système immunitaire;
14) une augmentation de la production de cytokines (par ex., IFN_{g} et/ou IL-2) par ladite population de cellules effectrices immunitaires; ou
15) une combinaison de deux ou plusieurs des éléments ci-dessus;
éventuellement par rapport à des cellules qui n'ont pas été mises en contact avec l'inhibiteur de LSD1.

6. Procédé de fabrication d'une population de cellules effectrices immunitaires comprenant:
a) mise en contact d'une population de cellules effectrices immunitaires avec un inhibiteur de LSD1, *ex vivo* fabriquant ainsi une population de cellules effectrices immunitaires, dans laquelle la population de cellules effectrices immunitaires comprend, par ex. est constituée, de(s) cellules T,
dans lequel la mise en contact avec l'inhibiteur de LSD1 provoque l'apparition d'un ou plusieurs des éléments suivants:
1) une augmentation de la proportion de cellules T naïves, par ex. les cellules T_{SCM};
2) une augmentation du nombre de cellules T naïves, par ex. les cellules T_{SCM};
3) une diminution du nombre de cellules T_{EM};
4) une diminution de la proportion de cellules T_{EM};
5) une augmentation de la proportion de cellules T CD45RA+CD62L+;
6) une augmentation du nombre de cellules T CD45RA+CD62L+;
7) une augmentation de la proportion de cellules T CD45RA+CCR7+;
8) une augmentation du nombre de cellules T CD45RA+CCR7+;
9) une diminution de la proportion de cellules effectrices immunitaires PD-1 positives;
10) une augmentation du rapport cellules effectrices immunitaires négatives PD-1 / cellules effectrices immunitaires positives PD-1;
11) une diminution de la proportion de cellules effectrices immunitaires PD-1 +/Lag3+/Tim3+;
12) une augmentation du rapport entre les cellules immunitaires effectrices PD-1-/Lag3-/Tim3- et les cellules effectrices immunitaires PD-1 +/Lag3+/Tim3+;
13) une augmentation de la prolifération des cellules effectrices du système immunitaire;
14) une augmentation de la production de cytokines (par ex., IFN_{g} et/ou IL-2) par ladite population de cellules effectrices immunitaires; ou
15) une combinaison de deux ou plusieurs des éléments ci-dessus;
par rapport à une population de cellules effectrices immunitaires sans contact.

7. Procédé selon la revendication 6, dans lequel le procédé comprend en outre l'étape b) d'insertion d'un acide nucléique qui code pour le CAR dans des cellules de la population de cellules effectrices immunitaires, éventuellement dans lequel ladite mise en contact de l'étape a) a lieu
1) avant;
2) en même temps;
3) après; ou
4) toute combinaison de deux ou plus de 1) à 3) ci-dessus;
ladite insertion de l'étape b).

8. Population de cellules effectrices immunitaires, qui est modifiée pour exprimer un récepteur antigénique chimérique (CAR), dans laquelle le CAR comprend un domaine de liaison à l'antigène, un domaine transmembranaire, et un domaine de signalisation intracellulaire, et dans lequel l'expression et/ou la fonction de LSD1 dans ladite cellule a été réduite ou éliminée, dans lequel la population de cellules effectrices immunitaires comprend en outre un inhibiteur de LSD1, dans lequel la population de cellules effectrices immunitaires comprend, par ex., est constituée, de cellules T.

9. Composition pour une utilisation selon l'une quelconque des revendications 1 à 5, procédé selon la revendication 7, ou population de cellules effectrices immunitaires selon la revendication 8, dans laquelle/lequel le CAR comprend un domaine de liaison à l'antigène (qui est éventuellement un anticorps ou un fragment d'anticorps, un TCR ou un fragment de TCR), un domaine transmembranaire, et un domaine de signalisation intracellulaire (qui est éventuellement un domaine de signalisation intracellulaire comprenant un domaine costimulateur et/ou un domaine de signalisation primaire), éventuellement dans laquelle/lequel le domaine de liaison à l'antigène se lie à un antigène tumoral choisi dans un groupe constitué par: TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mésothéline, IL-11Ra, PSCA, PRSS21, VEGFR2, LewisY, CD24, PDGFR-bêta, SSEA-4, CD20, Récepteur de folate alpha, ERBB2 (Her2/neu), MUGI, EGFR, NCAM, Prostase, PAP, ELF2M, Ephrin B2, récepteur d'IGF-1, CAIX, LMP2, gpl 00, bcr-abl, tyrosinase, EphA2, Fucosyl GMI, sLe, GM3, TGS5, HMWMAA, o-acétyl-GD2, Récepteur de folate bêta, TEM1/CD248, TEM7R, CLDN6, GPRC5D, CXORF61, CD97, CDI79a, ALK, acide polysialique, PLACI, GloboH, NY-BR-I, UPK2, HAVCRI, ADRB3, PANX3, GPR20, LY6K, OR51E2, TARP, WTI, NY-ESO-1, LAGE-la, MAGE-Al, légumaine, HPV E6,E7, MAGE Al, ETV6-AML, protéine de sperme 17, XAGEI, Tie 2, MAD-CT-1, MAD-CT-2, antigène 1 lié à Fos, p53, p53 mutant, prostéine, survivine et télomérase, PCTA-1/Galectine 8, MelanA/MARTl, Ras mutant, hTERT, points de rupture de translocation de sarcomes, ML-IAP, ERG (gène de fusion TMPRSS2 ETS), NA17, PAX3, récepteur d'androgène, Cyclin BI, MYCN, RhoC, TRP-2, CYPIBI, BORIS, SART3, PAX5, OYTESI, LCK, AKAP-4, SSX2, RAGE-I, transcriptase inverse de télomérase humaine, RUI, RU2, carboxyl-estérase intestinale, mut hsp70-2, CD79a, CD79b, CD72, LAIR1, FCAR, LILRA2, CD300LF, CLEC12A, BST2, EMR2, LY75, GPC3, FCRL5 et IGLL1, *par ex.* où l'antigène tumoral est CD19 ou BCMA.

10. Composition pour une utilisation, procédé, ou population de cellules effectrices immunitaires selon la revendication 9, dans laquelle/lequel:
(a) le domaine de liaison à l'antigène est un anticorps ou un fragment d'anticorps comprenant:
(i) la séquence d'acides aminés d'un domaine de liaison à CD19 selon les tableaux 6 à 9, par ex., la séquence d'acides aminés du domaine scFv de CTL019 selon le tableau 9 ou une séquence d'acides aminés selon SEQ ID NO: 957, ou une séquence d'acides aminés au moins 95% identique à celle-ci;
(ii) la séquence d'acides aminés d'un domaine de liaison CD19 humanisé selon les tableaux 6 à 9, par ex., la séquence d'acides aminés du domaine CAR2 scFv selon le tableau 9 ou une séquence d'acides aminés selon SEQ ID NO: 898, ou une séquence d'acides aminés au moins 95% identique à celle-ci; ou
(iii) la séquence d'acides aminés d'un domaine de liaison BCMA selon les Tableaux 11A-11B, par ex., la séquence d'acides aminés du domaine 139109 scFv selon le Tableau 11A ou une séquence d'acides aminés selon SEQ ID NO: 967, ou une séquence d'acides aminés au moins 95% identique à celle-ci; ou
(b) le CAR comprend:
(i) la séquence d'acides aminés d'un CAR CD19 selon les tableaux 6 à 9, par ex., la séquence d'acides aminés de CTL019 selon le tableau 9 ou une séquence d'acides aminés selon SEQ ID NO: 956 ou une séquence d'acides aminés au moins 95% identique à celle-ci;
(ii) la séquence d'acides aminés d'un CAR CD19 humanisé selon les tableaux 6 à 9, par ex., la séquence d'acides aminés de CAR2 selon le tableau 9 ou une séquence d'acides aminés selon SEQ ID NO: 902, ou une séquence d'acides aminés au moins 95% identique à celle-ci; ou
(iii) la séquence d'acides aminés d'un CAR BCMA selon les tableaux 11A-11B, par ex., la séquence d'acides aminés du CAR 139109 selon le tableau 11A ou une séquence d'acides aminés selon SEQ ID NO: 971, ou une séquence d'acides aminés au moins 95% identique à celle-ci.

11. Composition pour une utilisation, procédé ou population de cellules effectrices immunitaires selon la revendication 9 ou 10, dans laquelle/lequel:
(a) le domaine transmembranaire comprend:
(xi) une séquence d'acides aminés ayant au moins une, deux ou trois modifications mais pas plus de 20, 10 ou 5 modifications d'une séquence d'acides aminés de SEQ ID NO: 12, ou une séquence ayant 95-99% d'identité avec une séquence d'acides aminés de SEQ ID NO: 12; ou
(xii) la séquence de la SEQ ID NO: 12; et/ou
(b) le domaine de liaison à l'antigène est relié au domaine transmembranaire par une région charnière, dans laquelle ladite région charnière comprend la SEQ ID NO: 2 ou la SEQ ID NO: 6, ou une séquence présentant une identité de 95-99% avec celle-ci; et/ou
(c) le CAR comprend une séquence leader comprenant, par ex., consistant en, SEQ ID NO: 2.

12. Composition pour une utilisation, procédé, ou population de cellules effectrices immunitaires selon l'une quelconque des revendications 9 à 11, dans laquelle/lequel:
(a) le domaine de signalisation intracellulaire comprend un domaine de signalisation primaire et/ou un domaine de signalisation costimulatrice, dans lequel le domaine de signalisation primaire comprend un domaine de signalisation fonctionnel d'une protéine choisie parmi CD3 zêta, CD3 gamma, CD3 delta, CD3 epsilon, FcR gamma commun (FCER1G), FcR bêta (Fc Epsilon R1b), CD79a, CD79b, Fcgamma RIIa, DAPl0, ou DAP12; et/ou
(b) le domaine de signalisation primaire comprend:
(xi) une séquence d'acides aminés ayant au moins une, deux ou trois modifications mais pas plus de 20, 10 ou 5 modifications d'une séquence d'acides aminés de SEQ ID NO: 18 ou SEQ ID NO: 20, ou une séquence ayant 95-99% d'identité avec une séquence d'acides aminés de SEQ ID NO: 18 ou SEQ ID NO: 20; ou
(xii) la séquence d'acides aminés de la SEQ ID NO: 18 ou de la SEQ ID NO: 20; et/ou
(c) le domaine de signalisation intracellulaire comprend un domaine de signalisation costimulatrice, ou un domaine de signalisation primaire et un domaine de signalisation costimulatrice, dans lequel le domaine de signalisation costimulatrice comprend un domaine de signalisation fonctionnel d'une protéine choisie dans le groupe constitué par CD27, CD28, 4-IBB (CD137), OX40, CD30, CD40, PD-1, ICOS, antigène associé à la fonction lymphocytaire-1 (LFA-1), CD2, CD7, LIGHT, NKG2C, B7-H3, un ligand qui se lie spécifiquement à CD83, CDS, ICAM-1, GITR, BAFFR, HVEM (LIGHTR), SLAMF7, NKp80 (KLRF1), CD160, CD19, CD4, CD8alpha, CD8bêta, IL2R bêta, IL2R gamma, IL7Ralpha, ITGA4, VLAI, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGBI, CD29, ITGB2, CD18, LFA-1, ITGB7, TNFR2, TRANCE/RANKL, DNAMI (CD226), SLAMF4 (CD244, 2B4), CD84, CD96 (Tactile), CEACAMI, CRTAM, Ly9 (CD229), CD160 (BY55), PSGLI, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, NKp44, NKp30, NKp46 et NKG2D; et/ou
(d) le domaine de signalisation costimulatrice comprend une séquence d'acides aminés ayant au moins une, deux ou trois modifications mais pas plus de 20, 10 ou 5 modifications d'une séquence d'acides aminés de SEQ ID NO: 14 ou SEQ ID NO: 16, ou une séquence avec 95-99% d'identité à une séquence d'acides aminés de SEQ ID NO: 14 ou SEQ ID NO: 16; et/ou
(e) le domaine de signalisation costimulatrice comprend une séquence de SEQ ID NO: 14 ou SEQ ID NO: 16; et/ou
(f) le domaine intracellulaire comprend la séquence de SEQ ID NO: 14 ou SEQ ID NO: 16, et la séquence de SEQ ID NO: 18 ou SEQ ID NO: 20, où les séquences comprenant le domaine de signalisation intracellulaire sont exprimées dans le même cadre et sous la forme d'une seule chaîne polypeptidique.

13. Composition pour une utilisation selon l'une quelconque des revendications 1-5 ou 9-12, procédé selon l'une quelconque des revendications 6, 7 ou 9-12, ou population de cellules effectrices immunitaires selon l'une quelconque des revendications 8-12, dans laquelle/lequel l'inhibiteur de LSD1 est: (1) un système d'édition de gènes ciblant un ou plusieurs sites du gène LSD1, ou ses éléments régulateurs correspondants; (2) un acide nucléique (par ex., un siRNA ou shRNA, ou un oligonucléotide antisens) comprenant une séquence complémentaire à une séquence cible du gène LSD1; (3) une protéine (par ex., un LSD1 dominant négatif, par ex., un LSD1 catalytiquement inactif, ou un partenaire de liaison dominant négatif du LSD1); (4) une petite molécule; (5) un acide nucléique codant pour l'un quelconque de (1)-(3); ou (6) toute combinaison de (1)-(5),
éventuellement dans laquelle/lequel:
(a) l'inhibiteur de LSD1 est un shRNA ou un siRNA comprenant une séquence complémentaire à une séquence cible du gène LSD1 répertorié dans le tableau 1, par ex., choisi parmi SEQ ID NO: 43 à SEQ ID NO: 82;
(b) l'inhibiteur de LSD1 est un shRNA codé par un acide nucléique comprenant toute séquence codant pour un shRNA anti-LSD1 du tableau 1, par ex., codé par un acide nucléique comprenant une séquence choisie parmi SEQ ID NO: 83 à SEQ ID NO: 122; ou
(c) l'inhibiteur de LSD1 est un acide nucléique comprenant toute séquence codant pour un shRNA anti LSD1 du tableau 1, par ex., une séquence choisie parmi SEQ ID NO: 83 à SEQ ID NO: 122.

14. Composition pour une utilisation, procédé ou population de cellules effectrices immunitaires selon la revendication 13, dans laquelle/lequel ledit acide nucléique est disposé sur un vecteur, éventuellement dans laquelle/lequel le vecteur est un vecteur rétroviral, un vecteur lentiviral, un vecteur adénoviral, un vecteur viral adéno-associé (AAV), un vecteur du virus de l'herpès simplex (HSV), un plasmide, un minicercle, un nanoplasmide ou un vecteur ARN, et/ou dans laquelle le vecteur comprend en outre une séquence codant pour un CAR.

15. Composition pour une utilisation, procédé ou population de cellules effectrices immunitaires selon la revendication 13, dans laquelle l'inhibiteur de LSD1 est un système d'édition de génome spécifique d'une séquence du gène LSD1 (KDM1A) ou de ses éléments régulateurs, choisi parmi un système d'édition de génome CRISPR, un système d'édition de génome à nucléase à doigt de zinc, un système d'édition de génome TALEN et un système d'édition de génome à méganucléase, éventuellement dans laquelle l'inhibiteur de LSD1 est un système d'édition de génome CRISPR comprenant une molécule d'ARNg comprenant un domaine de ciblage complémentaire à une séquence du gène LSD1 (KDM1A) ou de ses éléments régulateurs, par ex., comprenant l'une quelconque des SEQ ID NO: 132 à 862.

16. Composition pour une utilisation, procédé ou population de cellules effectrices immunitaires selon la revendication 13, dans laquelle/lequel l'inhibiteur de LSD1 est une petite molécule, en outre dans laquelle la petite molécule est un inhibiteur de LSD1 réversible ou un inhibiteur de LSD1 irréversible, éventuellement dans laquelle/lequel l'inhibiteur de LSD1 est:
a) GSK2699537;
b) rel-2-[[(1R,2S)-2-[4-[(4-chlorophényl)méthoxy]phényl]cyclopropyl]amino ]-1-(4-méthyl-1-pipérazinyl)-éthanone;
c) (R)-4-(5-(pyrrolidin-3-ylméthoxy)-2-(p-tolyl)pyridin-3-yl)benzonitrile;
d) (1S,2R)-N-((2-méthoxypyridin-3-yl)méthyl)-2-phénylcyclopropan-l-amine;
e) N,N-diméthyl-1-((4-(4-(4-(pipéridin-4-yl)phényl)-1H-indazol-1-yl)phényl)sulfonyl)pipéridin-4-amine;
f) 5-(6-chloro-4'-(méthylsulfonyl)-[1,1'-biphényl]-3-yl)-2-(pipérazin-1-yl)-1H pyrrole-3-carbonitrile;
g) rel-N-[(1R,2S)-2-Phénylcyclopropyl]-4-Pipéridinamine;
h) 2-(1R,2S)-2-(4-(Benzyloxy)phényl)cyclopropylamino)-1-(4-méthylpipérazine-1-yl)éthanone;
i) Trans-3-(3-amino-2-méthylphényl)-1-(4-hydroxycyclohexyl)-6-méthyl-1H-indole-5-carbonitrile; ou
j) un sel pharmaceutiquement acceptable de l'un quelconque des composés précédents.

17. Composition pour une utilisation, procédé ou population de cellules effectrices immunitaires selon la revendication 13, dans laquelle/lequel:
(a) l'inhibiteur de LSD1 est une protéine, par ex., est un partenaire de liaison négatif dominant de LSD1 (par ex., une histone désacétylase (HDAC) qui interagit avec LSD1 ou un autre membre du complexe coactivateur Co-REST ou AR), ou un acide nucléique codant pour ledit partenaire de liaison négatif dominant de LSD1; ou
(b) l'inhibiteur de LSD1 est une protéine, par ex., est un LSD1 négatif dominant (par ex., catalytiquement inactif), ou un acide nucléique codant pour ledit LSD1 négatif dominant.

18. Population de cellules effectrices immunitaires selon la revendication 8 pour une utilisation dans un procédé de traitement d'une maladie associée à l'expression d'un antigène tumoral chez un sujet, ledit procédé comprenant l'administration audit sujet d'une quantité efficace de la population de cellules effectrices immunitaires, éventuellement dans laquelle:
(a) le procédé comprend en outre l'administration audit sujet d'un inhibiteur de LSD1;
(b) le sujet reçoit un prétraitement d'un inhibiteur de LSD1, avant l'administration de la population de cellules effectrices immunitaires;
(c) le sujet reçoit un traitement simultané avec un inhibiteur de LSD1 et la population de cellules effectrices immunitaires; et/ou
(d) le sujet reçoit un traitement avec un inhibiteur de LSD1 après l'administration de la population de cellules effectrices immunitaires;
dans laquelle la maladie associée à l'expression d'un antigène tumoral est choisie parmi une maladie proliférative, un état précancéreux et un cancer.

19. Composition pour une utilisation selon l'une quelconque des revendications 1-5 ou 9-17, ou population de cellules effectrices immunitaires pour une utilisation selon la revendication 19, dans laquelle le cancer est:
(i) un cancer hématologique choisi parmi un ou plusieurs des cancers suivants: leucémie lymphocytaire chronique (CLL), leucémies aiguës, leucémie aiguë lymphoïde (ALL), leucémie aiguë myéloïde (AML), leucémie aiguë lymphoïde à cellules B (B-ALL), leucémie aiguë lymphoïde à cellules T (T-ALL), leucémie myéloïde chronique (LMC), leucémie prolymphocytaire à cellules B, néoplasme à cellules dendritiques plasmacytoïdes blastiques, lymphome de Burkitt, lymphome diffus à grandes cellules B, lymphome folliculaire, leucémie à cellules ciliées, lymphome folliculaire à petites ou grandes cellules, affections lymphoprolifératives malignes, lymphome de MALT, lymphome à cellules du manteau, lymphome de la zone marginale, myélome multiple, myélodysplasie et syndrome myélodysplasique, lymphome non hodgkinien, lymphome hodgkinien, lymphome plasmablastique, néoplasme à cellules dendritiques plasmacytoïdes, macroglobulinémie de Waldenstrom, ou préleucémie, ou
(ii) choisi dans le groupe constitué par le cancer du côlon, cancer du rectum, carcinome des cellules rénales, cancer du foie, carcinome non à petites cellules du poumon, cancer du petit intestin, cancer de l'œsophage, mélanome, cancer des os, cancer du pancréas, cancer de la peau, cancer de la tête ou du cou, mélanome malin cutané ou intraoculaire, cancer de l'utérus, cancer des ovaires, cancer du rectum, cancer de la région anale, cancer de l'estomac, cancer des testicules, cancer de l'utérus, carcinome des trompes de Fallope, carcinome de l'endomètre, carcinome du col de l'utérus, carcinome du vagin, carcinome de la vulve, maladie de Hodgkin, lymphome non hodgkinien, cancer du système endocrinien, cancer de la glande thyroïde, cancer de la glande parathyroïde, cancer de la glande surrénale, sarcome des tissus mous, cancer de l'urètre, cancer du pénis, tumeurs solides de l'enfant, cancer de la vessie, cancer du rein ou de l'uretère, carcinome du bassin rénal, néoplasme du système nerveux central (SNC), lymphome primaire du SNC, angiogenèse tumorale, tumeur de l'axe rachidien, gliome du tronc cérébral, adénome hypophysaire, sarcome de Kaposi, cancer épidermoïde, cancer épidermoïde, lymphome des cellules T, cancers induits par l'environnement, combinaisons desdits cancers et lésions métastatiques desdits cancers.

20. Composition pour une utilisation selon l'une quelconque des revendications 1-5, 9-17 ou 19, ou population de cellules effectrices immunitaires pour une utilisation selon la revendication 18, dans laquelle le sujet est un humain.

21. Procédé de fabrication d'une population de cellules effectrices immunitaires comprenant l'introduction dans lesdites cellules d'un acide nucléique codant pour un CAR, dans lequel l'acide nucléique s'intègre dans le génome de ladite cellule à l'intérieur du gène LSD1, de telle sorte que l'expression et/ou la fonction de LSD1 soit réduite, dans lequel la population de cellules effectrices immunitaires comprend, par ex. est constituée, de(s) cellules T.
